(19) Europäisches Patentamt
European Patent Office
Office européen des brevets

(11) **EP 4 556 480 A1**

(12) **EUROPEAN PATENT APPLICATION**

(43) Date of publication:
**21.05.2025 Bulletin 2025/21**

(21) Application number: **24189896.4**

(22) Date of filing: **05.08.2022**

(51) International Patent Classification (IPC):
***C07D 493/20*** *(2006.01)* ***A61P 35/00*** *(2006.01)*
***A61K 31/35*** *(2006.01)* ***A61K 31/422*** *(2006.01)*
***A61K 31/427*** *(2006.01)* ***A61K 31/4433*** *(2006.01)*

(52) Cooperative Patent Classification (CPC):
**C07D 493/20; A61P 35/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.08.2021 PL 43869621**

(62) Document number(s) of the earlier application(s) in accordance with Art. 76 EPC:
**22764330.1 / 4 380 939**

(71) Applicant: **Fileclo Spolka Z Ograniczona Odpowiedzialnoscia**
**92-318 Lodz (PL)**

(72) Inventors:
- **CZERWONKA, Dominika**
  **61-619 Poznan (PL)**
- **KRZYWIK, Julia**
  **98-290 Grzybki (PL)**
- **SOBIERAJSKI, Tomasz**
  **98-100 Lask (PL)**
- **KLEJBOROWSKA, Greta**
  **60-204 Poznan (PL)**
- **ULLRICH, Malgorzata**
  **93-263 Lodz (PL)**
- **MOZGA, Witold**
  **91-034 Lodz (PL)**
- **HUCZYNSKI, Adam**
  **61-377 Poznan (PL)**
- **PILASZEK, Przemyslaw**
  **95-041 Galkowek-Parcela (PL)**

(74) Representative: **Kancelaria Eupatent.pl Sp. z.o.o**
**Ul. Kilinskiego 185**
**90-348 Lodz (PL)**

Remarks:
•The application is published incomplete as filed (Rule 68(1) EPC).
•This application was filed on 19-07-2024 as a divisional application to the application mentioned under INID code 62.
•The references to parts of the description (parts of originally filed pages 21 and 24) are deemed to be deleted (Rule 56(4)(6) EPC).

(54) **COMPOUNDS CONSTITUTING C20-MODIFIED SALINOMYCIN DERIVATIVES, A METHOD FOR OBTAINING THE SAME, A COMPOSITION CONTAINING THE SAME AND A USE OF SAID COMPOUNDS**

(57) The invention relates to compounds constituting C20-N-modified salinomycin derivatives, a method for obtaining the same, a composition containing the same, and their use as a medicament, particularly as an anti-cancer agent.

EP 4 556 480 A1

**Description**

TECHNICAL FIELD

The invention relates to salinomycin derivatives singly modified at position C20, a method for obtaining the same, a composition containing the same, and a use thereof as a medicament, particularly as an anti-cancer agent. The invention also relates to a method for obtaining intermediate products in a method for obtaining salinomycin derivatives modified at position C-20 as well as such intermediate products themselves.

BACKGROUND

One of the most utilized ways of identifying new cancer drugs is chemical modification of compounds of natural origin that have demonstrated high biological activity. Salinomycin is a natural polyether ionophore antibiotic isolated from *Streptomyces albus* commonly used in veterinary with the formula (FLC-00001):

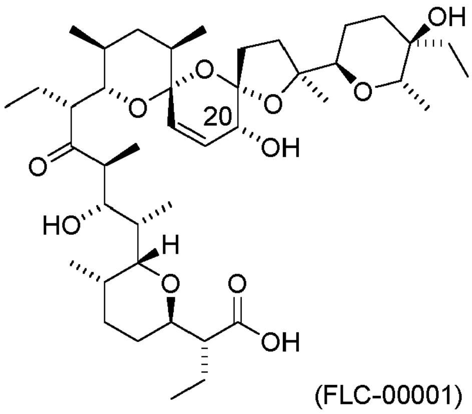

(FLC-00001)

Salinomycin is known for its high anti-microbial activity, but also for its anti-cancer activity. In *in vitro* and *in vivo* tests, salinomycin has shown efficacy against a variety of cancer cells, including drug-resistant cells and cancer stem cells. The mechanism of biological action of salinomycin is related to the ability of this compound to selectively complex metal cations, primarily sodium and potassium cations, and to transport them subsequently across biological membranes. This leads to an imbalance of cations in the cell, changes in intracellular pH, and ultimately results in cell death. The high anti-cancer activity of salinomycin is also related to the effect of said compound on various molecular targets and signalling pathways, including AMPK, MAPK, VEGF or Wnt/β-catenin. Salinomycin has been successfully used on a small group of patients with advanced head, neck, breast and ovarian cancer. Salinomycin therapy resulted in inhibition of cancer progression with no acute side effects, thus demonstrating a high therapeutic potential of this compound.

Patent application EP3191493 and a scientific publication [Mai et al., Nature Chemistry, 9, 2017, 1025-1033] disclose amine derivatives of salinomycin obtained at C-20 position. In *in vitro* studies, some compounds exhibited higher anti-cancer activity and selectivity against CD24 cancer stem cells, as well as the ability to inhibit mammosphere formation compared to the original salinomycin. The use of amine derivatives of salinomycin at C20 position also resulted in a reduction in the volume and weight of tumours in mice with implanted human breast cancer MCF-7. The high anti-cancer activity of said derivatives is related to their ability to induce ferroptosis, i.e. programmed cell death dependent on iron cation content. The findings reported in patent application EP3191493 and the scientific publication [Mai et al., Nature Chemistry, 9, 2017, 1025-1033] refer only to *in vivo* studies conducted in mice. The body of a mouse differs significantly from that of the human, which makes it is impossible to simply translate the results of these studies into the real therapeutic potential of the salinomycin derivatives obtained.

Now, in the scientific publication [Li et al., European Journal of Medicinal Chemistry, 148, 2018, 279-290], the authors disclosed *N*-amide and *N*-carbamate (urethane) derivatives of C20-epi-salinomycin with an inverted absolute configuration (S instead of R absolute configuration) on the asymmetric carbon at the C-20 position. The *in vitro* anti-cancer activity of said compounds was tested versus a series of cancer cell lines: 4T1 (murine mammary carcinoma), A549 (human lung adenocarcinoma), HL-60 (human promyelocytic leukaemia), HeLa (human cervical cancer), MCF-7 (human breast cancer), SMMC-7721 (human liver cancer) and SW480 (human colon adenocarcinoma). Data disclosed in a scientific publication [Li et al., European Journal of Medicinal Chemistry, 148, 2018, 279-290] demonstrate that most of the obtained C20-epi-salinomycin derivatives exhibit higher anti-cancer activity compared to the starting compound. Studies conducted on the normal BEAS-2B cell line (human bronchial epithelial cells) further revealed that the C20-epi-salinomycin with the highest anti-cancer activity are further characterised by high selectivity of action, which in some

cases is several times higher than that exhibited by chemically non-modified salinomycin. The information disclosed in the scientific publication [Li et al., European Journal of Medicinal Chemistry, 148, 2018, 279-290] is limited to *in vitro* studies only, which means that the effect of said compounds "in the living body" (*in vivo* studies) remains unknown. The ability of the resulting C20-*epi*-salinomycin derivatives to overcome drug resistance of cancer cells is also unknown.

Authors of a scientific publication [Versini et al., Chemistry A European Journal, 26 (33), 2020, 7416-7424] disclosed amine derivatives of salinomycin obtained at the C-20 position retaining the absolute configuration R on the asymmetric carbon atom of C20 (as in the original salinomycin). HMLER CSC cell line (human mammary epithelial cancer stem cells) studies revealed that the new derivatives have improved stability, selectivity of activity, as well as potent activity against model breast cancer stem cells.

Present application is a continuation of the scientific concept disclosed in document WO2021156461A1 - application published on 2021-08-12, submitted on the basis of Polish priority PL43285820A dated 2020-02-07 (date of present priority falls before the publication of PL43285820A or WO2021156461A1). WO2021156461A1 discloses salinomycin derivatives modified at C-20 position which are non *epi* analogues and their anti-cancer activity. It was shown for chosen amides and urethanes that in neither case do the *epi* derivatives show higher activity than the corresponding derivatives according to the disclosure.

The anti-cancer activity of biologically active compounds, including salinomycin and derivatives thereof, is closely correlated to the type of cell lines used in the tests. None of the salinomycin derivatives synthesised so far, however, has found a practical medical application, which is due for example to low bioactivity, low selectivity of action, lack of detailed studies on the mechanisms of biological action or pharmacokinetic and pharmacodynamic properties. This is why concentrated efforts are continued that are designed to obtain salinomycin derivatives with a high therapeutic index, which would be applicable in oncology therapy.

SUMMARY OF THE INVENTION

Obtaning new salinomycin derivatives involves a number of synthetic problems that need to be solved. Salinomycin and intermediate products required to obtained derivatives thereof may be unstable in the reaction medium, especially in the presence of acidic and/or basic agents. Salinomycin, as well as its derivatives, are sensitive to high temperatures and may therefore undergo irreversible disintegration. The presence of multiple functional groups presents an additional challenge for selective modification of salinomycin molecule, including any chemo- and regioselective modification of one of the three hydroxyl groups present within its structure. Another problem is the exorbitant price of commercially available salinomycin, which significantly hinders the development of new and efficient methods for chemical modification of the compound.

Accordingly, the invention addresses prior art difficulties regarding the preparation of salinomycin derivatives modified at the C20 position while retaining the absolute configuration *R* on the asymmetric C20 carbon atom (as in the starting salinomycin). The object of the invention was therefore to obtain new salinomycin derivatives modified at C20 position using the method according to the invention. Said method comprises obtaining an intermediate product in a process that has been significantly modified and simplified compared to the methods previously disclosed in the literature, as a result of specific conversions involving selected reactions, reactants and agents as well as conditions of the reaction allowing said derivatives to be obtained while preserving the absolute configuration R at the asymmetric carbon at the C20 position in a relatively uncomplicated and efficient process.

Promising salinomycin derivatives modified at position C20 have not always been easy to prepare using the inventive method, which is why methods using specific intermediates have been developed to obtain some derivatives according to the invention. The object of the invention was therefore to develop a method for preparing said intermediates and to use said intermediates for further conversions towards salinomycin derivatives having biological properties. The newly developed intermediates for synthesis as well as the complete synthesis towards the preparation of the inventive salinomycin derivatives allows for preparing preferable compounds having biological effect.

A further object of the invention was to obtain new derivatives of the natural ionophore salinomycin, both form of acid and salts thereof, modified at the C20 position, which could be used in anti-cancer therapy. It is also an object of the invention to provide such salinomycin derivatives modified at the C20 carbon having an activity against cancer cells with highly advantageous selectivity.

Surprisingly, it was found that the inventive salinomycin derivatives have perfect activity and selectivity against neoplastic diseases, in particular against cancers selected from the group comprising ovarian cancer, melanoma, pancreatic cancer, lung cancer, liver cancer, gastric cancer, malignant primitive neuroectodermal tumour, biphenotypic leukemia or myeloid leukemia.

The invention generally relates to compounds constituting C20-*N*-modified derivatives of salinomycin with the general formula (2):

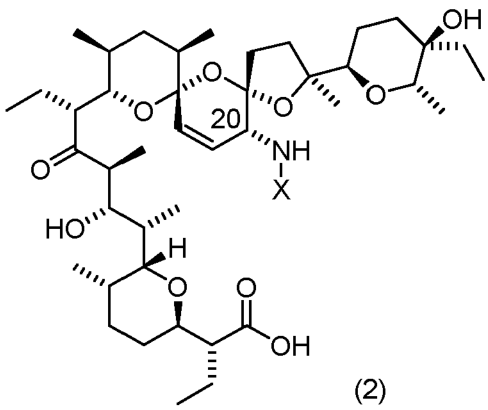

(2)

, wherein:

- X is respectively:

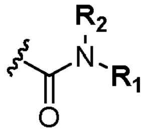 , 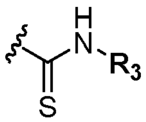 , 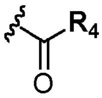 , 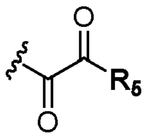 , 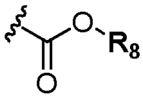 , 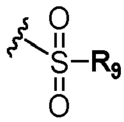 , 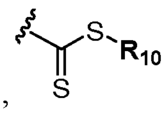

or

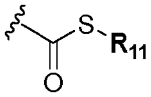 .

The invention relates to compounds constituting C20-*N*-modified derivatives of salinomycin with the general formula (2):

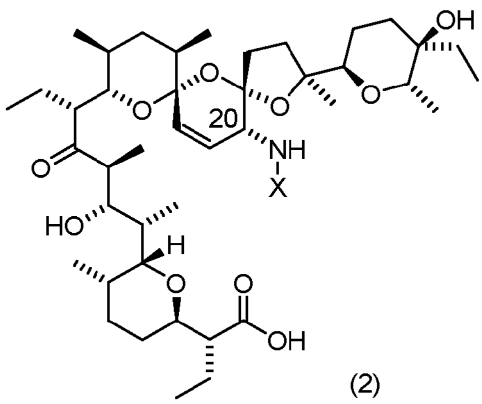

(2)

wherein X is selected from:

X=

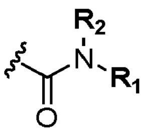 ,

then compounds are C20-N urea salinomycin derivatives with the general formula (3):

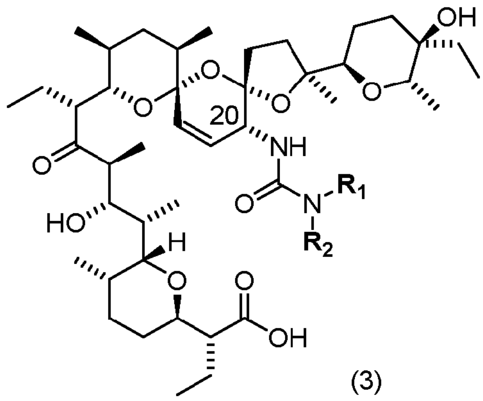

(3)

wherein $R_1$ may be different than $R_2$ or equal to $R_2$ and is:

- when $R_1$ = H, $R_2$ is:

  - a hydrogen;
  - a straight-chain or branched-chain alkyl group comprising 1 to 10 carbons, with the exclusion of n-propyl and n-butyl;
  - a straight-chain or branched-chain alkyl group comprising 1 to 5 carbons which is substituted at any position of the carbon chain by 1 to 3 halogens, which may be both at the same carbon and at different carbons, with the exclusion of 3-chloropropyl,
  - a straight-chain alkyl group comprising 3 to 5 carbons which comprises multiple bonds at any position of the carbon chain;
  - a straight-chain or branched-chain alkyl group comprising 2 to 5 carbons which comprises ether or ester bonds or an amine group at any position of the carbon chain,
  - a monocyclic alkyl group comprising 3 to 6 carbons;
  - a monocyclic alkyl group comprising 3 to 6 carbons, wherein 1 or more carbons are substituted with 1 or more heteroatoms from a group comprising atoms of nitrogen or sulphur, or a sulphonyl group;
  - a monocyclic alkyl group comprising 3 to 6 carbons, substituted with 1 to 3 halogens;
  - an aromatic group, wherein those comprising a six-membered aromatic ring are preferred,
  - an aromatic group substituted with 1 to 5 substituents independently selected from alkyl, haloalkyl, alkoxy or halogen atoms,
  - an heteroaryl group, wherein 1 or more carbons are substituted with 1 or more nitrogen atoms,
  - a monocyclic or aromatic group as defined above linked to the salinomycin moiety by a carbon chain having 1 to 4 carbon atoms or by a sulphonyl bond;

  or salts thereof;

- when $R_1$ and $R_2 \neq$ H, and $R_1$ may be the same as $R_2$ or different than $R_2$, $R_1$ and $R_2$ are:

  - a straight-chain or branched-chain alkyl group comprising 1 to 5 carbons;
  - a straight-chain alkyl group comprising 3 to 5 carbons which comprises multiple bonds at any position of the carbon chain;
  - a straight-chain or branched-chain alkyl group comprising 2 to 5 carbons which comprises an amine group at any position of the carbon chain;
  - a monocyclic alkyl group comprising 3 to 6 carbons;
  - a monocyclic alkyl group comprising 3 to 6 carbons, wherein 1 or more carbons are substituted with 1 or more heteroatoms from a group comprising atoms of nitrogen, oxygen or sulphur, or a sulphonyl group;
  - a monocyclic alkyl group having 3 to 6 carbon atoms substituted with 1 to 3 substituents independently selected from alkyl, haloalkyl groups, or one where at least one of the ring carbons is substituted with sulphonyl or carbonyl;

  or salts thereof;

X =

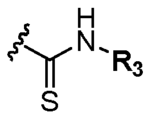

,

then compounds are C20-*N*-thiourea salinomycin derivatives with the general formula (4):

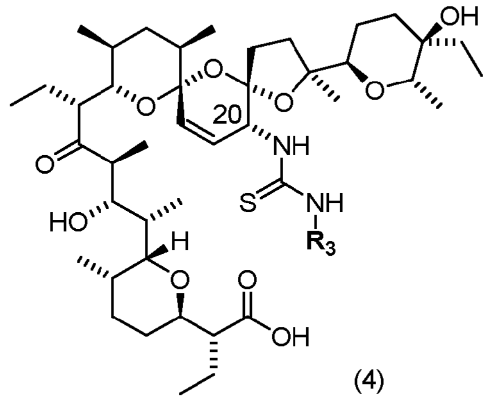

(4)

wherein $R_3$ is:

- a straight-chain or branched-chain alkyl group comprising 1 to 10 carbons;
- a straight-chain or branched-chain alkyl group comprising 2 to 5 carbons which comprises thioether or ester bonds at any position of the carbon chain;
- a monocyclic alkyl group comprising 3 to 6 carbons;
- an aromatic group, wherein those comprising a six-membered aromatic ring are preferred,
- an aromatic group substituted with 1 to 5 substituents independently selected from alkyl, haloalkyl, or halogen atoms,
- a monocyclic or aromatic group as defined above linked to the salinomycin moiety by a carbon chain having 1 to 4 carbon atoms;

or salts thereof;

X =

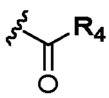

,

then compounds are C20-*N*-amide salinomycin derivatives with the general formula (5):

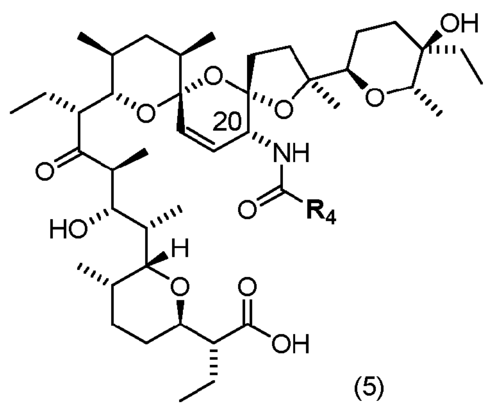

(5)

wherein $R_4$ is:

- a straight-chain or branched-chain alkyl group comprising 2 to 10 carbons, with the exclusion of isopropyl;
- a straight-chain or branched-chain alkyl group comprising 1 to 5 carbons which is substituted at any position of the carbon chain by 1 to 3 halogens, which may be both at the same carbon and at different carbons, with the exclusion of chloromethyl and 3-chloropropyl,
- a straight-chain or branched-chain alkyl group comprising 2 to 5 carbons, comprising a terminal carboxyl group;
- a monocyclic alkyl group comprising 3 to 6 carbons;
- an aromatic group, wherein those comprising a six-membered aromatic ring are preferred, substituted with 1 to 5 substituents independently selected from alkyl, haloalkyl, alkoxy, haloalkoxy groups, or halogen atoms, with the exclusion of 4-(chloromethyl)phenyl,
- an heteroaryl group, wherein 1 or more carbons are substituted with 1 or more nitrogen atoms;

or salts thereof;

X =

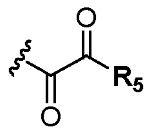

,

then compounds are C20-*N*-modified salinomycin derivatives with the general formula (6):

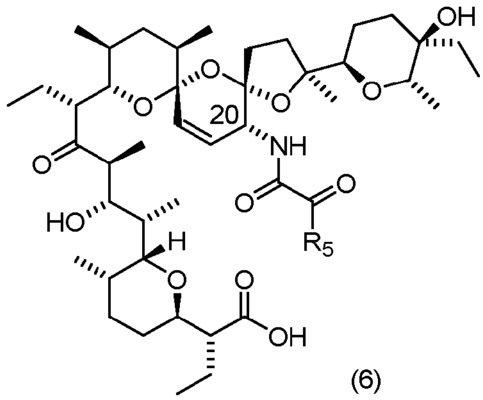

(6)

wherein $R_5$ is:

- -O-$R_6$ or -NH-$R_{7;}$

wherein $R_6$ is:

- a straight-chain alkyl group comprising 1 to 5 carbons;

wherein $R_7$ is:

- a hydrogen;
- a straight-chain or branched-chain alkyl group comprising 1 to 5 carbons;
- a straight-chain or branched-chain alkyl group comprising 1 to 5 carbons which comprises amide bonds at any position of the carbon chain;
- a straight-chain or branched-chain alkyl group comprising 1 to 5 carbons substitutes with a hydroxy or amino group at any position of the carbon chain;
- a monocyclic alkyl group comprising 3 to 6 carbons;
- an aromatic group, wherein those comprising a six-membered aromatic ring are preferred;
- an aromatic group substituted with 1 to 3 substituents independently selected from alkoxy or halogens;

- a monocyclic alkyl group comprising 3 to 6 carbons or an heteroaryl group, wherein 1 or more carbons are substituted with 1 or more atoms from a group comprising atoms of nitrogen or oxygen;
- a monocyclic or aromatic group as defined above linked to the salinomycin moiety by a carbon chain having 1 to 4 carbon atoms;

or salts thereof;

X =

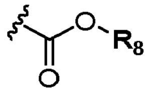

,

then compounds are C20-*N*-urethane salinomycin derivatives with the general formula (7):

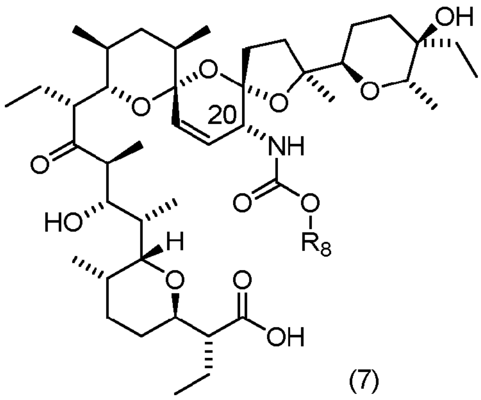

(7)

wherein $R_8$ is:

- a straight-chain or branched-chain alkyl group comprising 3 to 20 carbons, with the exclusion of neopentyl group,
- a straight-chain or branched-chain alkyl group comprising 3 to 10 carbons which comprises multiple bonds, either double or triple bonds, at any position of the carbon chain, with the exclusion of propargyl group;
- a straight-chain or branched-chain alkyl group comprising 1 to 10 carbons which is substituted at any position of the carbon chain by 1 to 6 halogens, which may be both at the same carbon and at different carbons, with the exclusion of trichloroethyl group ;
- a straight-chain or branched-chain alkyl group comprising 1 to 10 carbons substituted at any position of the carbon chain with 1 to 5 substituents independently selected from hydroxy, amino, nitro, sulphonyl groups or any combination thereof;
- a straight-chain or branched-chain alkyl group comprising 2 to 10 carbons, including substituted with halogen atoms, comprising at any position of the carbon chain one or more ether bonds;
- a monocyclic alkyl group comprising 3 to 6 carbons;
- a monocyclic alkyl group comprising 3 to 6 carbons, wherein 1 or more carbons are substituted with 1 or more heteroatoms from a group comprising atoms of oxygen or nitrogen,
- a monocyclic alkyl group comprising 3 to 6 carbons, substituted with an alkyl group at any position,
- an aromatic group, wherein those comprising a six-membered aromatic ring are preferred,
- an aromatic group substituted with 1 to 5 substituents independently selected from alkyl groups or halogen atoms,
- an heteroaryl group, wherein 1 or more carbons are substituted with 1 or more heteroatoms from a group comprising atoms of nitrogen or sulphur,
- a monocyclic or aromatic group as defined above linked to the salinomycin moiety by a carbon chain having 1 to 4 carbon atoms and/or an ether bond;

or salts thereof;

X =

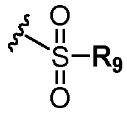

,

then compounds are C20-*N*-sulphonamide salinomycin derivatives with the general formula (8):

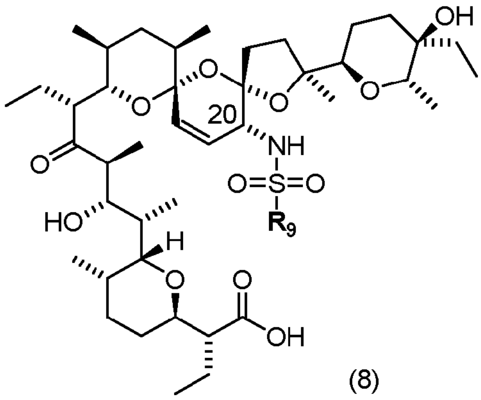

(8)

wherein $R_9$ is:

- a straight-chain or branched-chain alkyl group comprising 1 to 10 carbons;
- an aromatic five- or six-membered group,
- an aromatic five- or six-membered group substituted with 1 to 5 substituents independently selected from alkyl, alkoxy, haloalkyl groups or halogen atoms,
- an heteroaryl group, wherein 1 or more carbons are substituted with 1 or more heteroatoms from a group comprising atoms of oxygen, nitrogen or sulphur;

or salts thereof;

X =

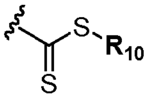

,

then compounds are C20-*N*-dithiocarbamate salinomycin derivatives with the general formula (9):

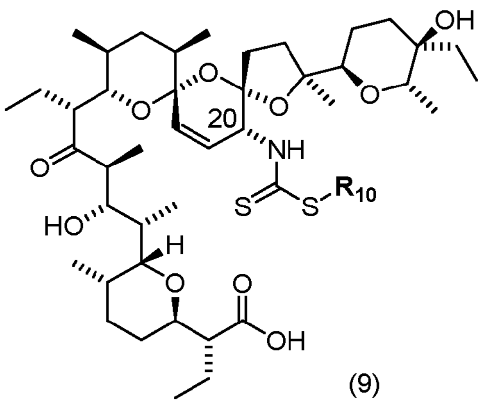

(9)

wherein $R_{10}$ is:

- a straight-chain alkyl group comprising 1 to 10 carbons;

- a benzyl group wherein the atoms in the aromatic ring are hydrogens or from 1 to 3 hydrogens are substituted with halogen or alkyl or alkoxy or haloalkyl;

or salts thereof;

X =

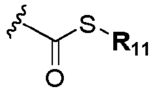

,

then the compounds are S-substituted thiocarbamate derivatives of C20-aminosalinomycin with the general formula (10):

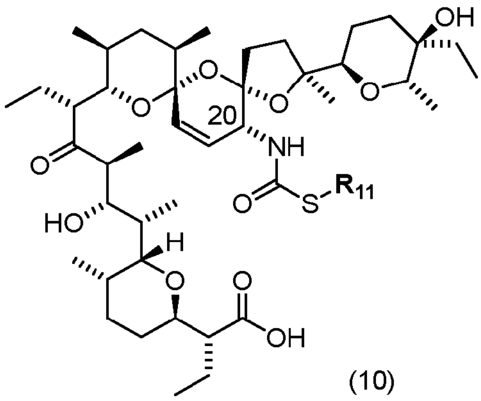

(10)

wherein $R_{11}$ is:

- a phenyl or benzyl group;
- a phenyl or benzyl group substituted at any position with 1 to 3 halogen atoms;

or salts thereof.

Preferably, these compounds are derivatives with the following formulae:

for X =

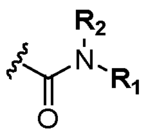

:

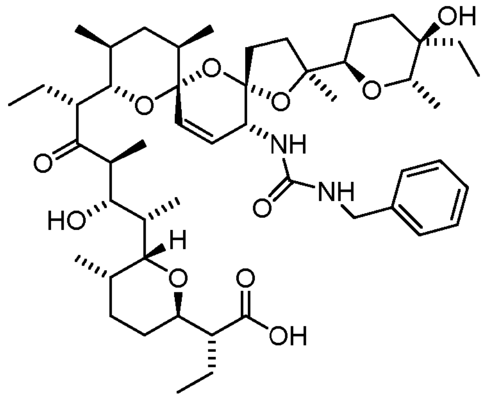

FLC-0337-1

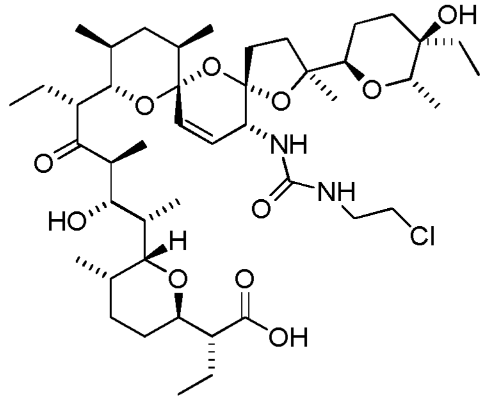

FLC-00338-1

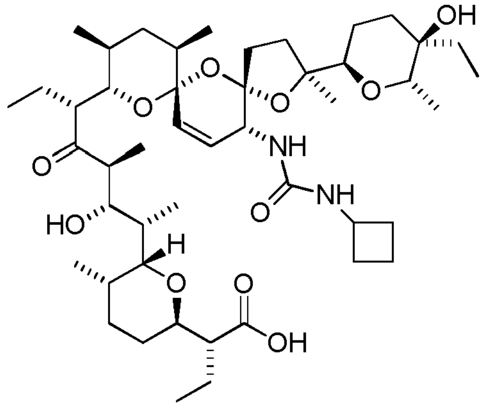

FLC-00341-1

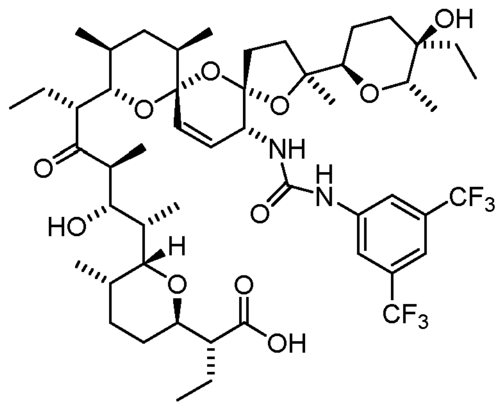

FLC-00344-1

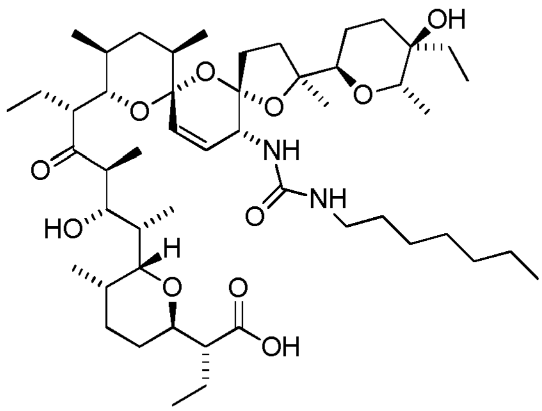

FLC-00350-1

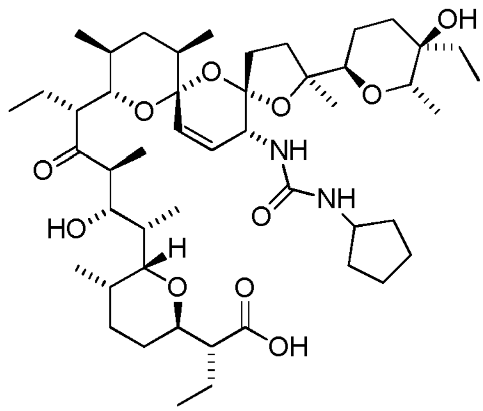

FLC-00351-1

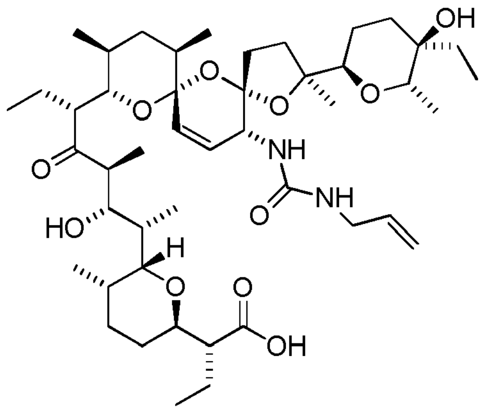

FLC-00353-1

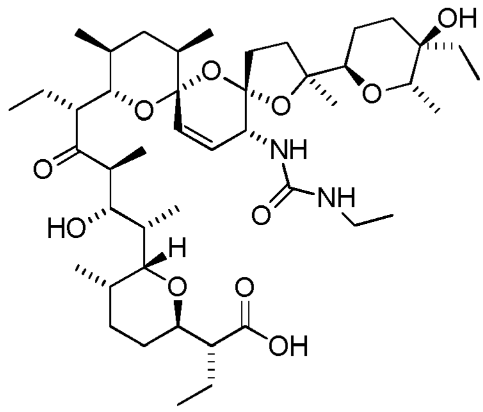

FLC-00358-1

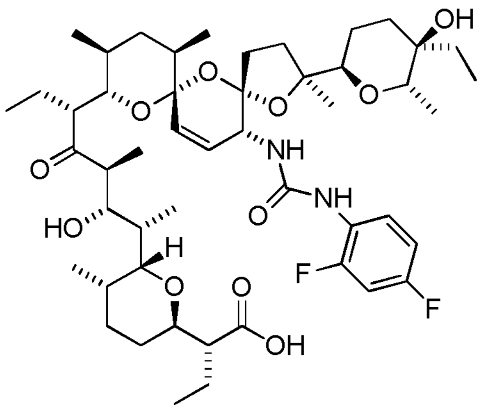

FLC-00359-1

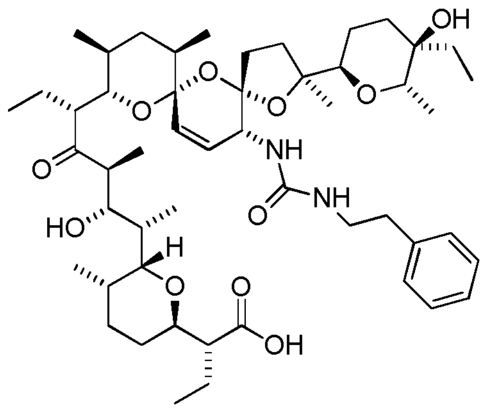

FLC-00362-1

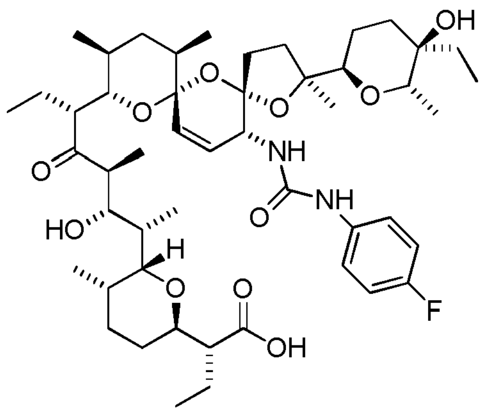

FLC-00363-1

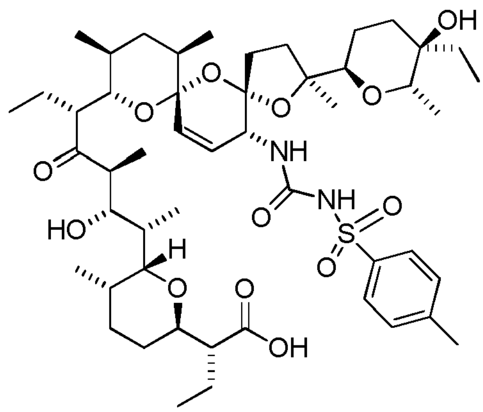

FLC-00364-1

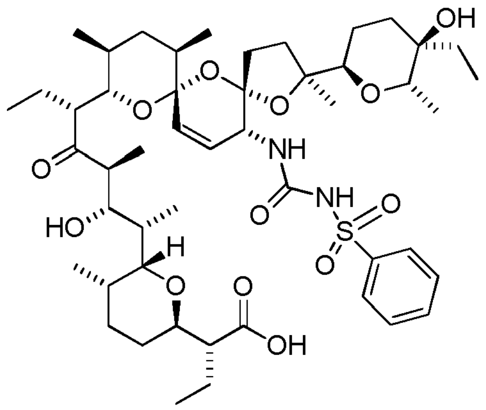

FLC-00365-1

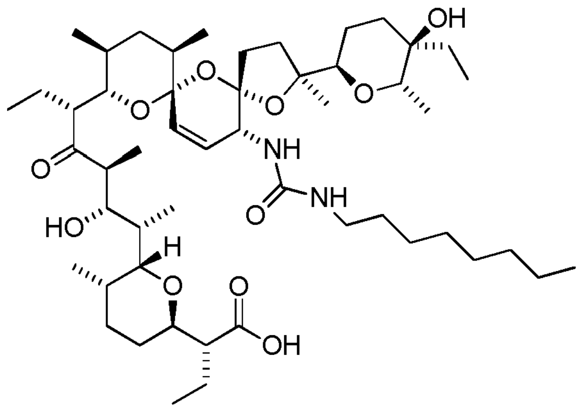

FLC-00366-1

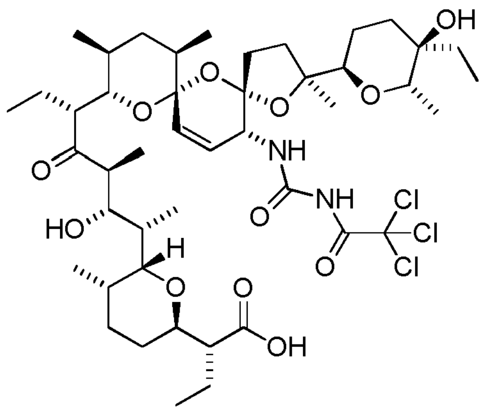

FLC-00367-1

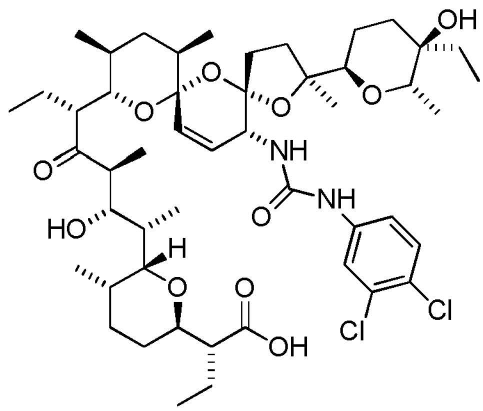

FLC-00370-1

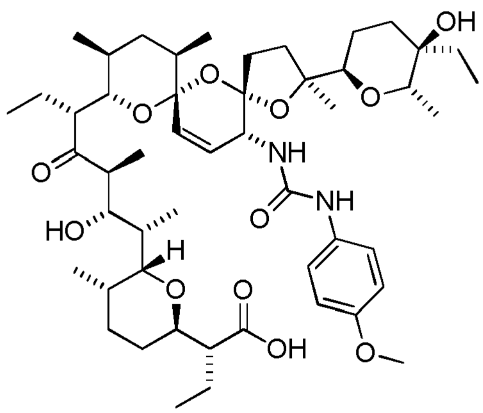

FLC-00371-1

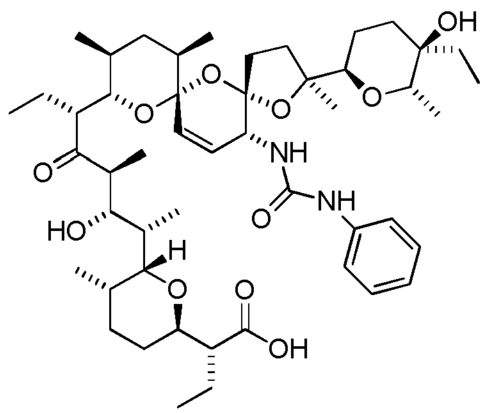

FLC-00374-1

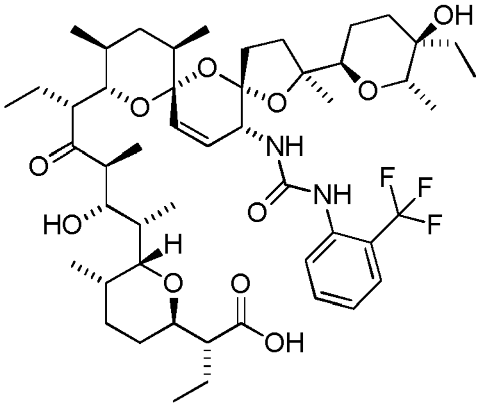

FLC-00381-1

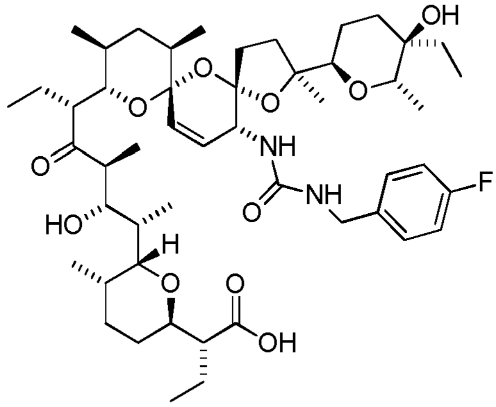

FLC-00383-1

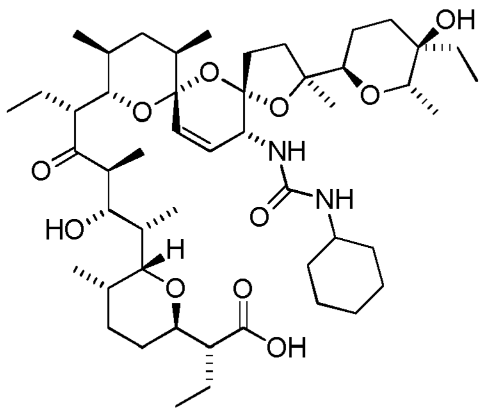

FLC-00384-1

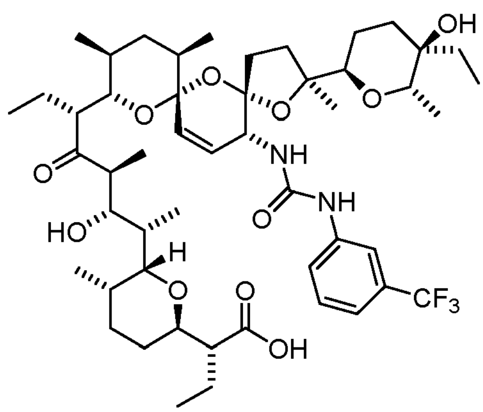

FLC-00385-1

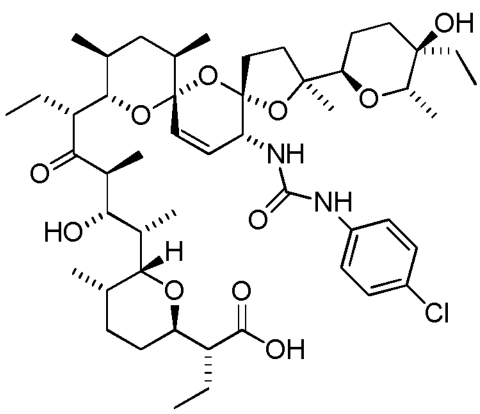

FLC-00388-1

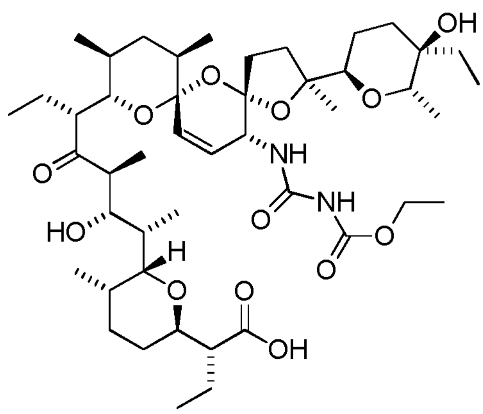

FLC-00389-1

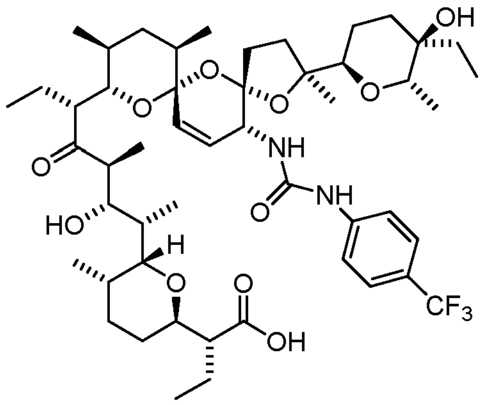

FLC-00390-1

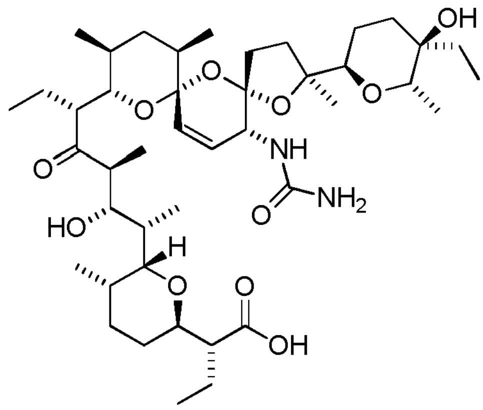

FLC-00391-1

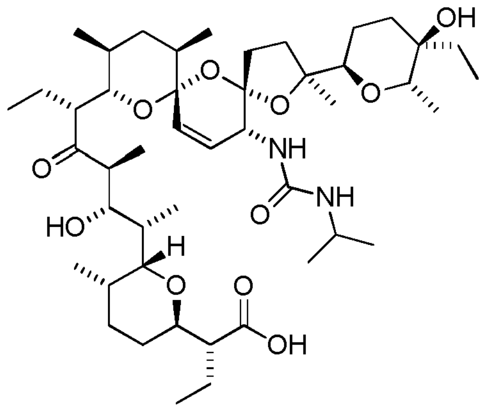

FLC-00396-1

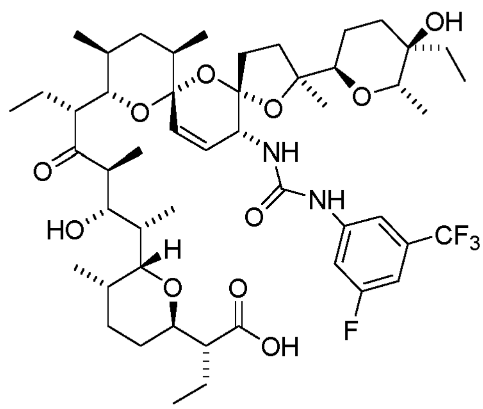

FLC-00427-1

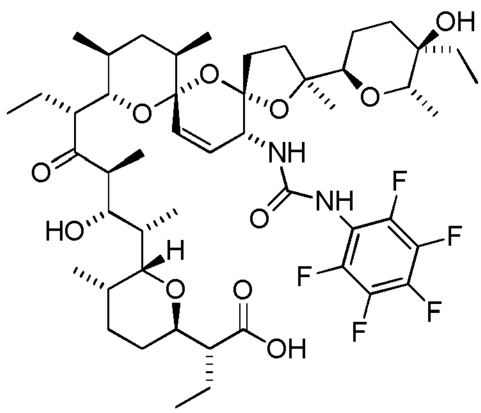

FLC-00428-1

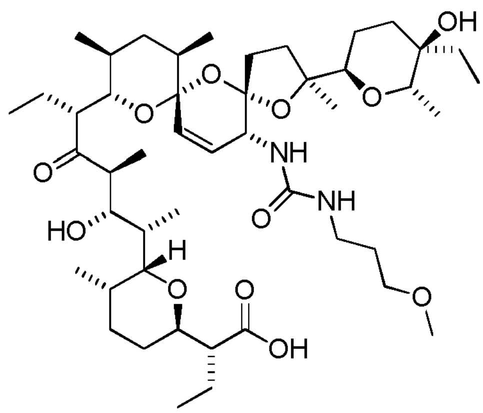

FLC-00539-1

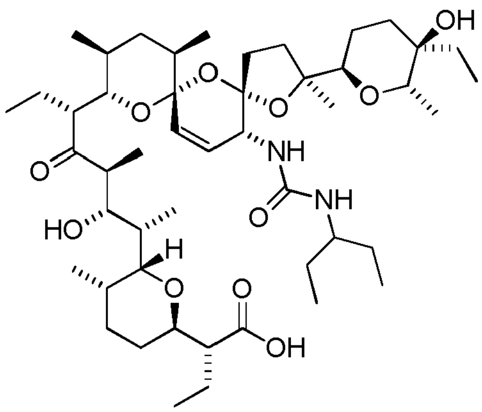

FLC-00541-1

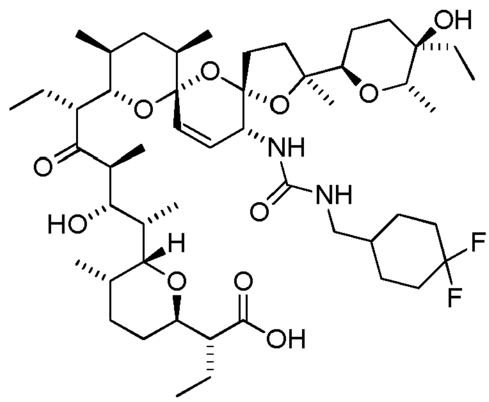

FLC-00545-1

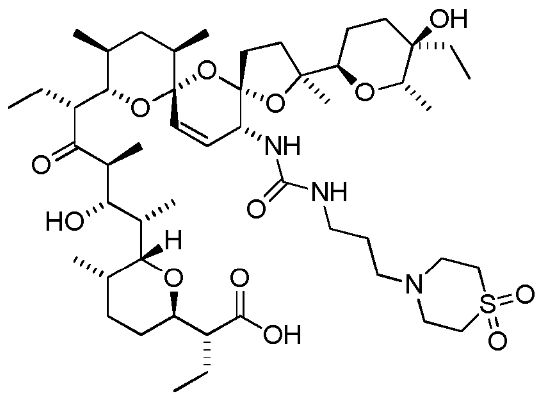

FLC-00546-1

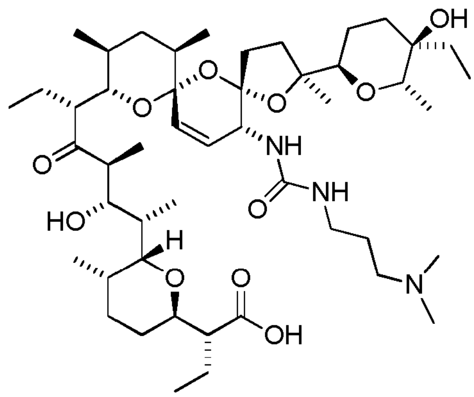

FLC-00562-1

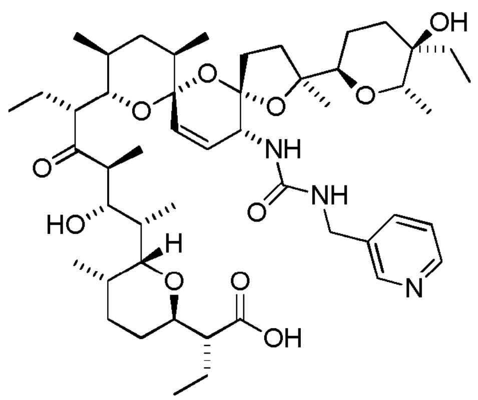

FLC-00566-1

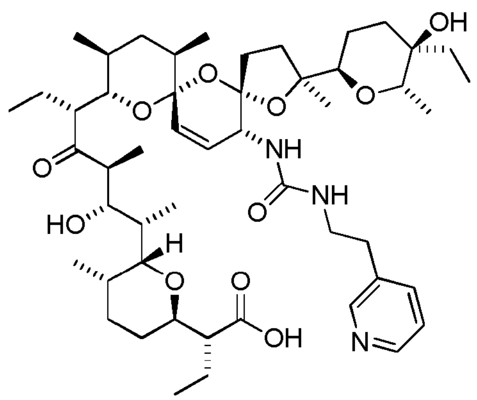

FLC-00567-1

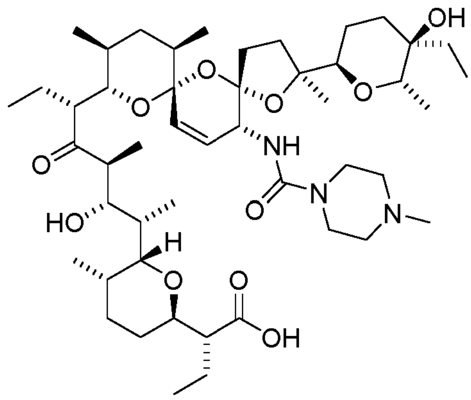

FLC-00515-1

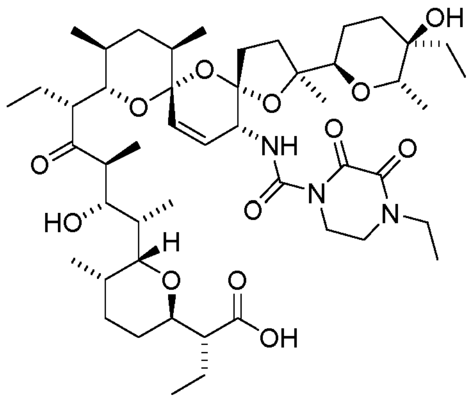

FLC-00518-1

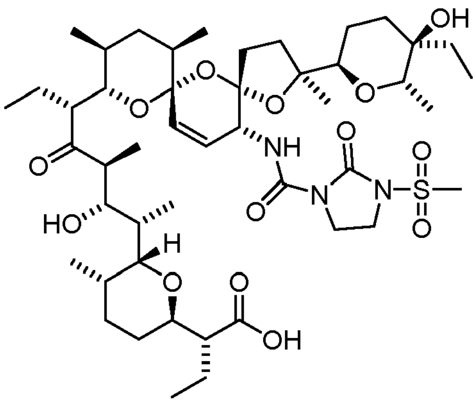

FLC-00519-1

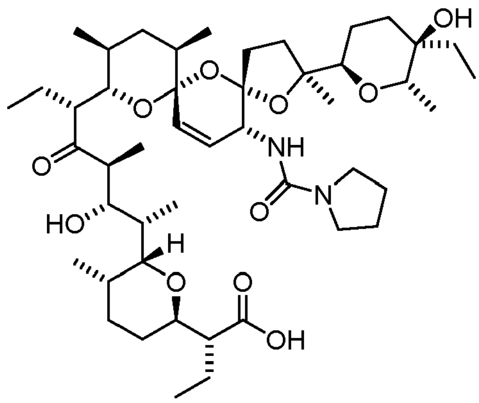

FLC-00537-1

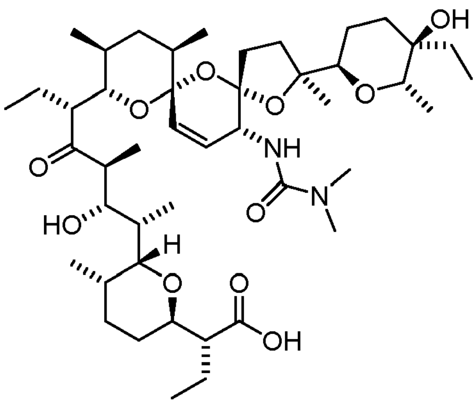

FLC-00538-1

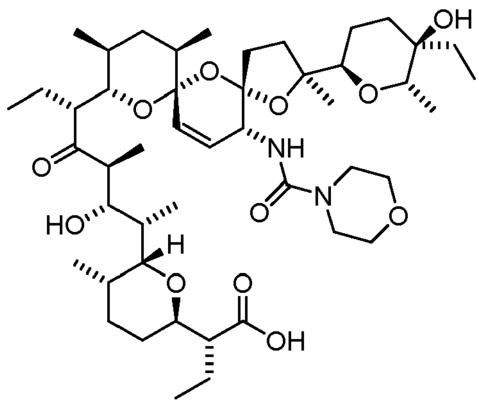

FLC-00540-1

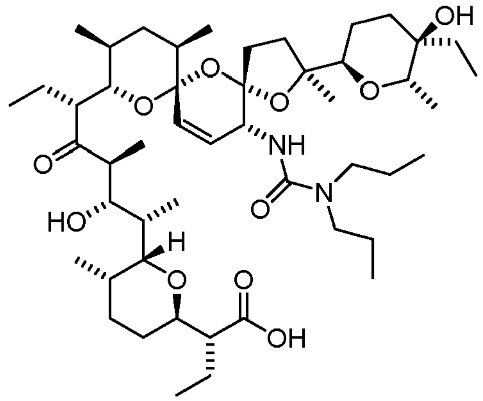

FLC-00542-1

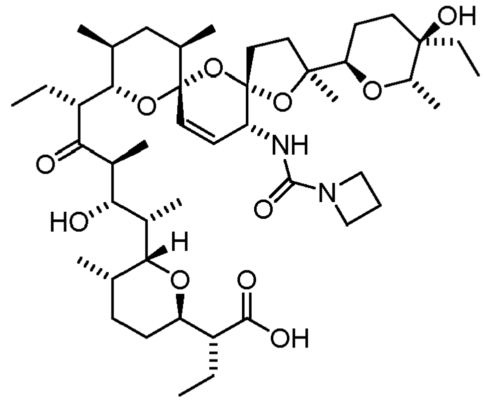

FLC-00543-1

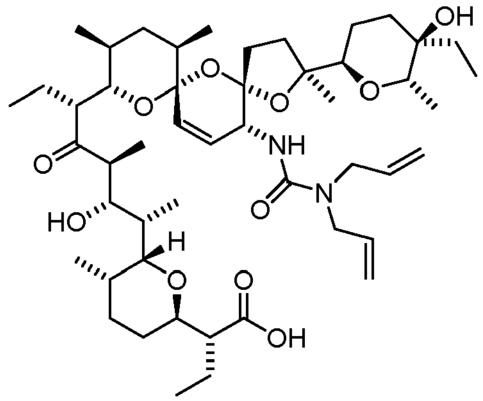

FLC-00544-1

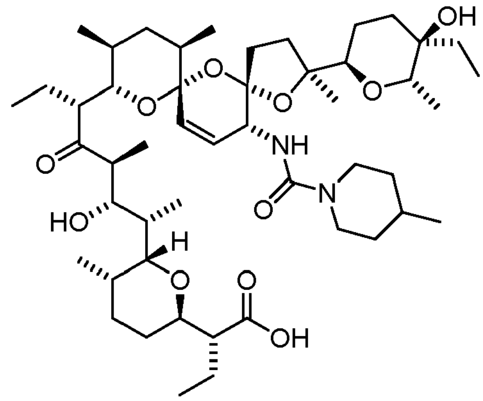

FLC-00557-1

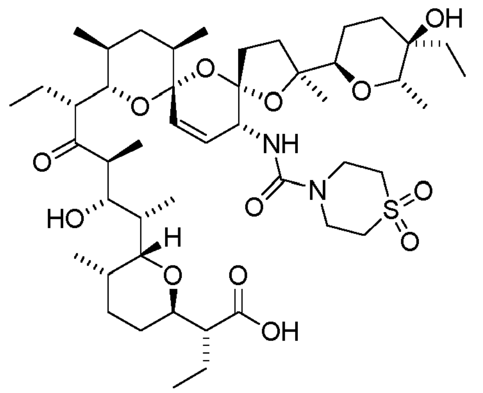

FLC-00560-1

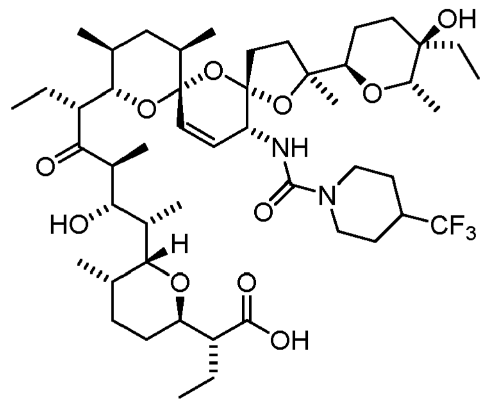

FLC-00561-1

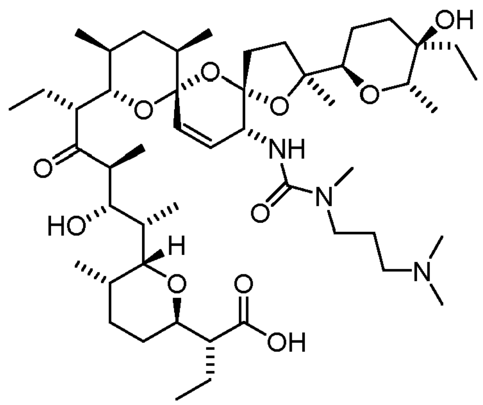

FLC-00563-1

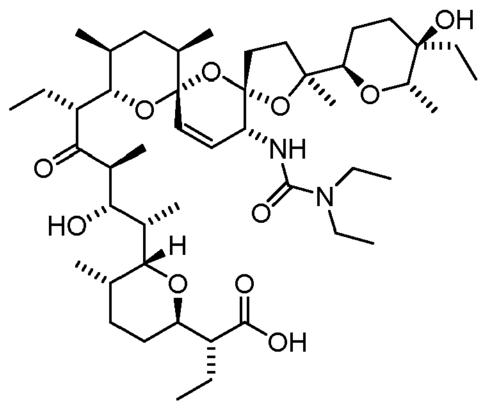

FLC-00564-1

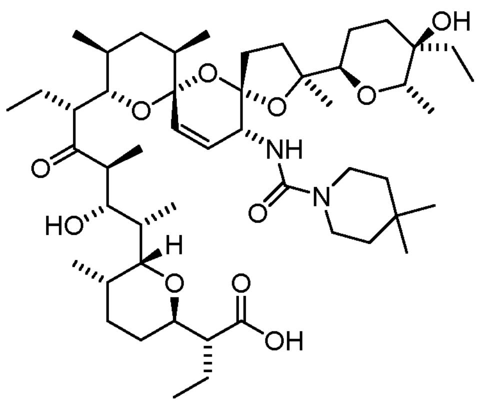

FLC-00565-1

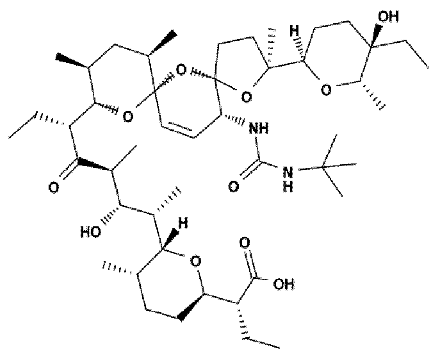

FLC-00593-1

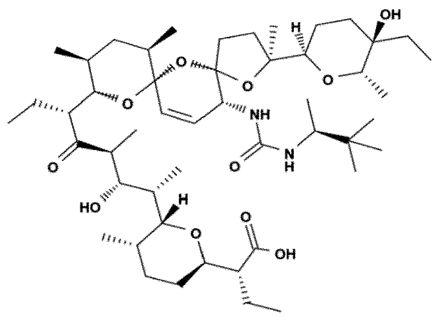

FLC-00606-1

for X =

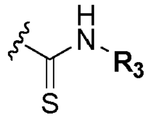

:

FLC-00347-1

FLC-00339-1

FLC-00340-1

FLC-00345-1

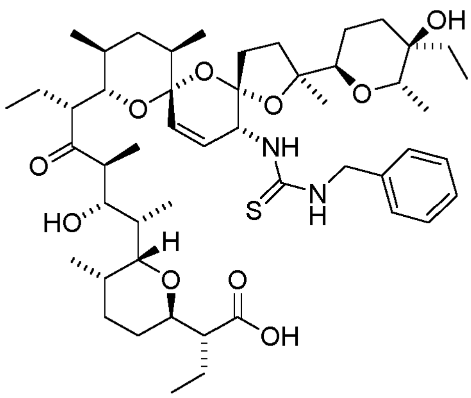

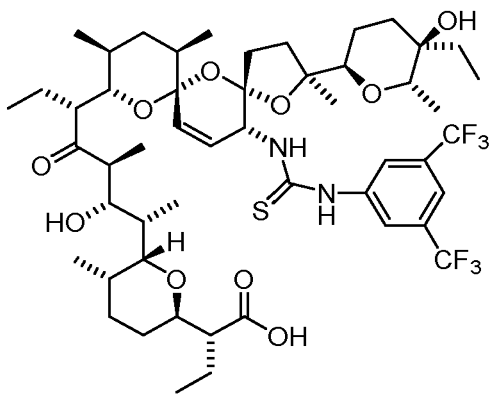

FLC-00346-1

FLC-00348-1

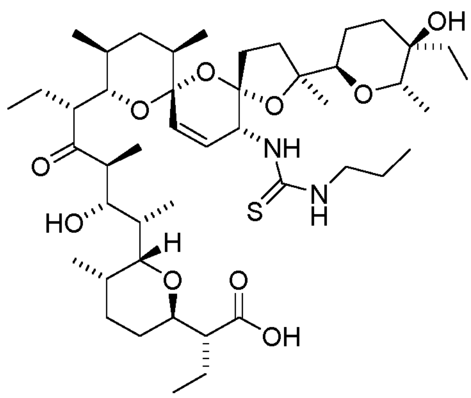

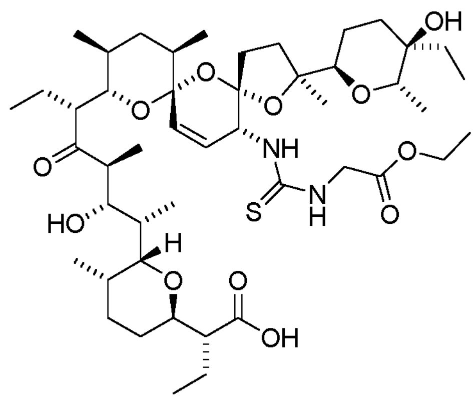

FLC-00349-1

FLC-00352-1

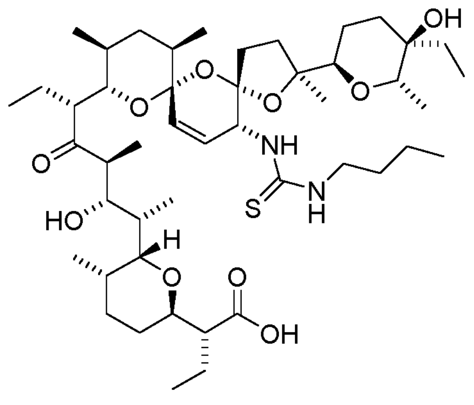

FLC-00368-1

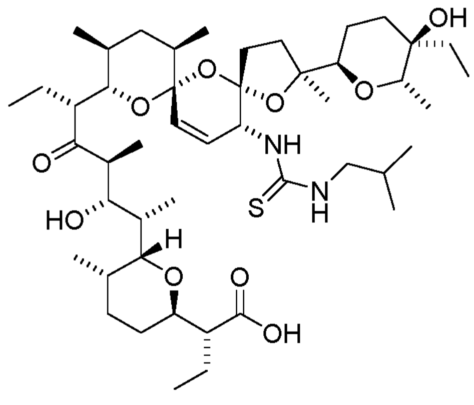

FLC-00369-1

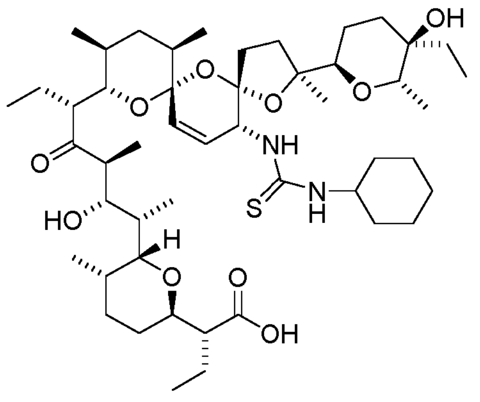

FLC-00377-1

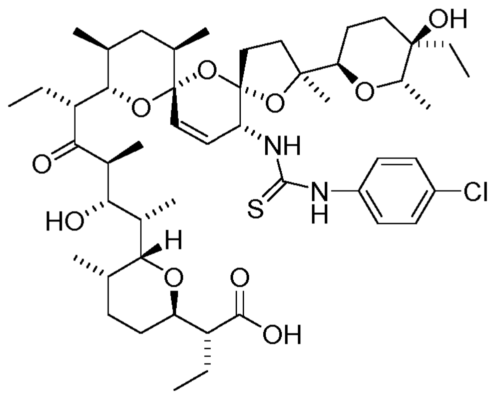

FLC-00378-1

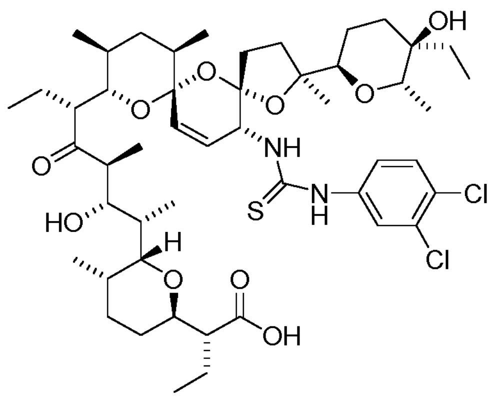

FLC-00379-1

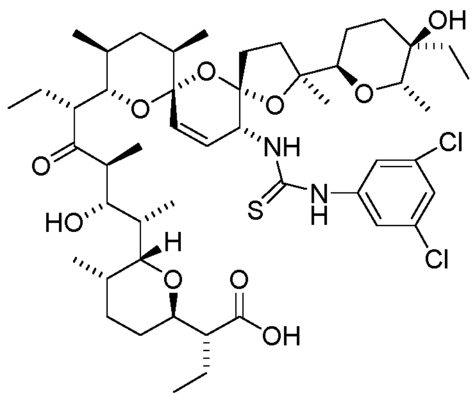

FLC-00380-1

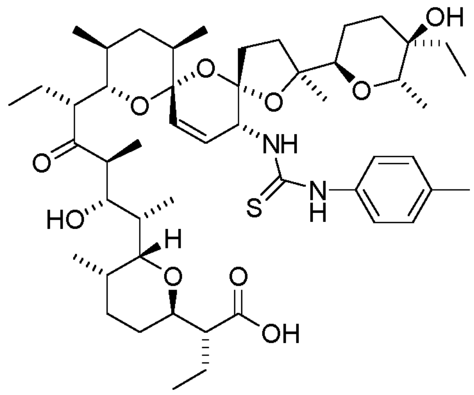

FLC-00382-1

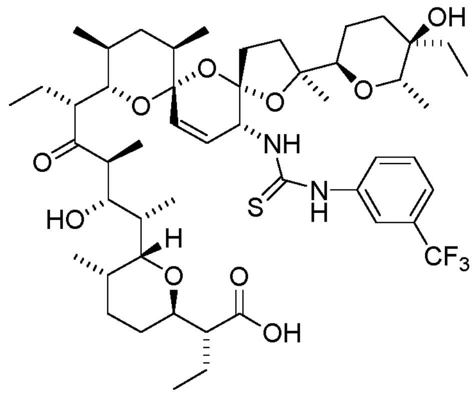

FLC-00386-1

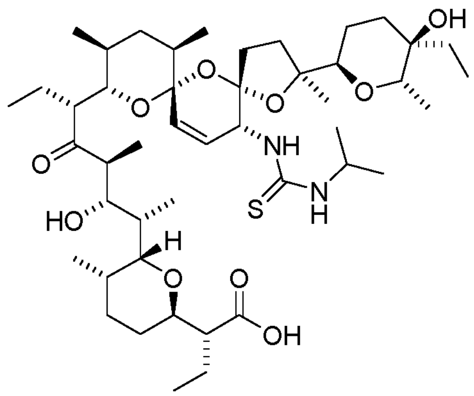

FLC-00387-1

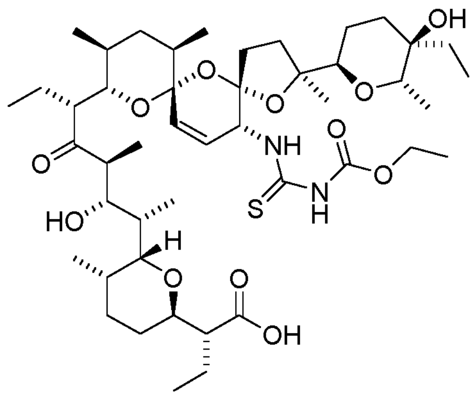

FLC-00393-1

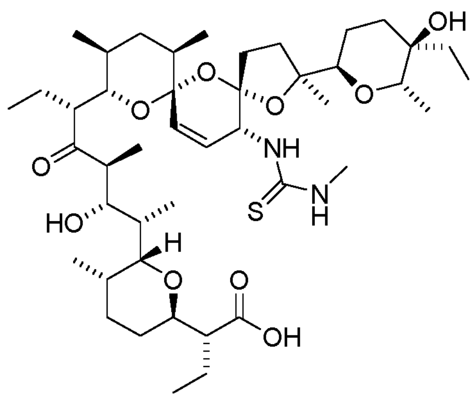

FLC-00394-1

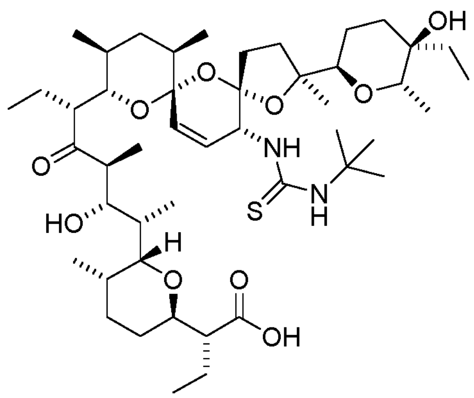

FLC-00467-1

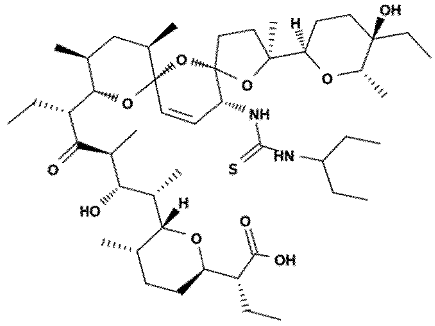

FLC-00771-1

for X =

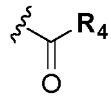

:

FLC-00361-1

FLC-00445-1

FLC-00455-1

FLC-00462-1

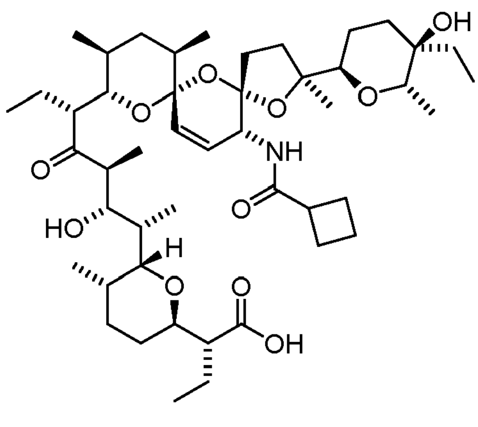

FLC-00463-1

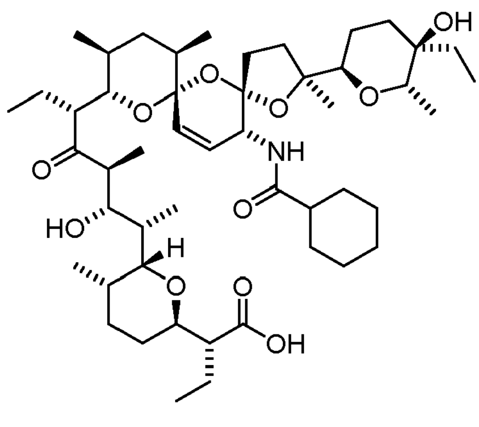

FLC-00464-1

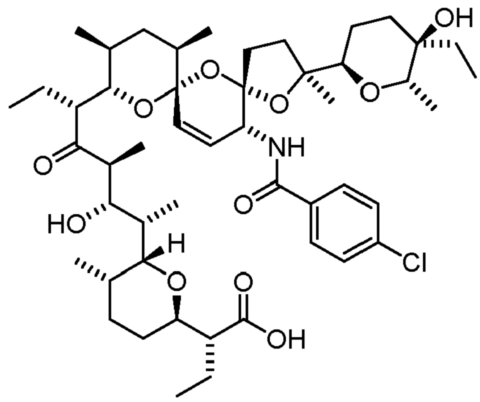

FLC-00469-1

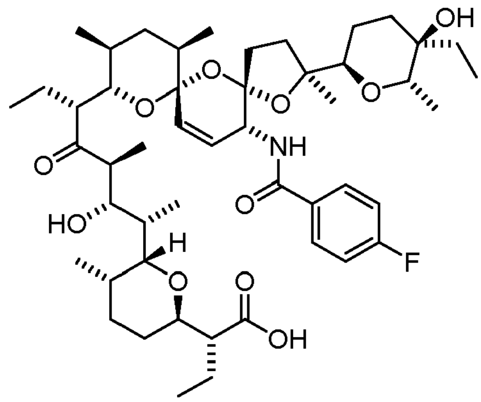

FLC-00470-1

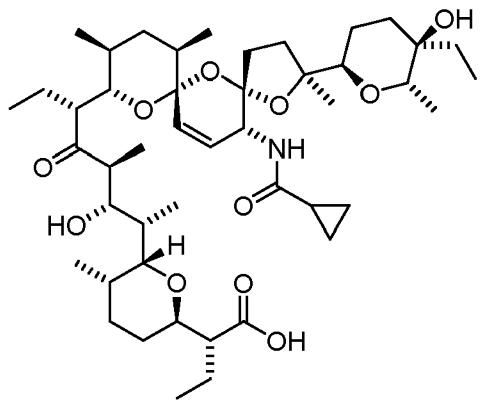

FLC-00472-1

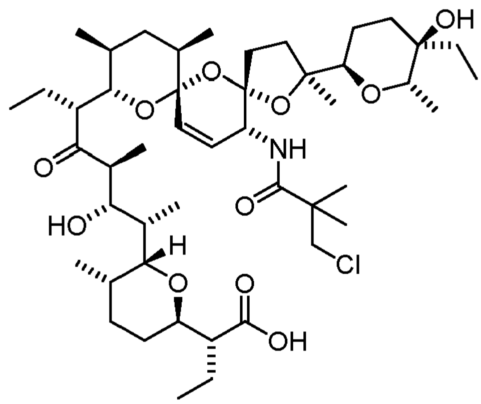

FLC-00473-1

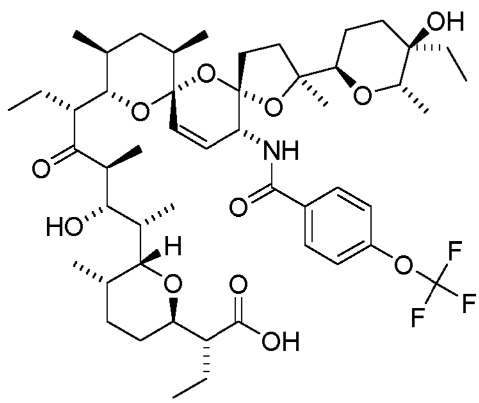

FLC-00486-1

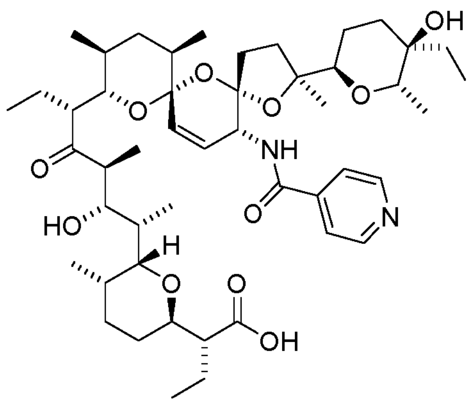

FLC-00487-1

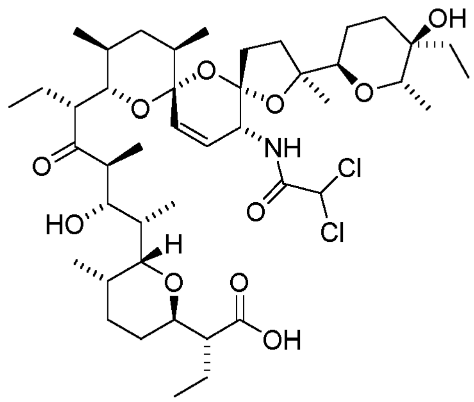

FLC-00493-1

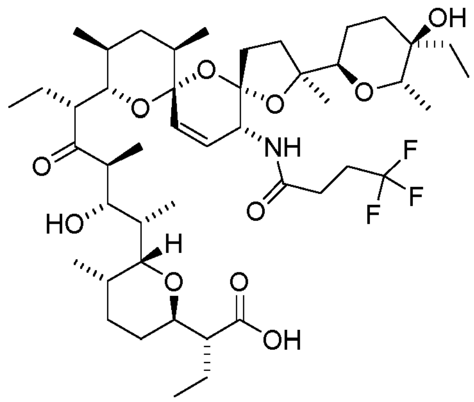

FLC-00494-1

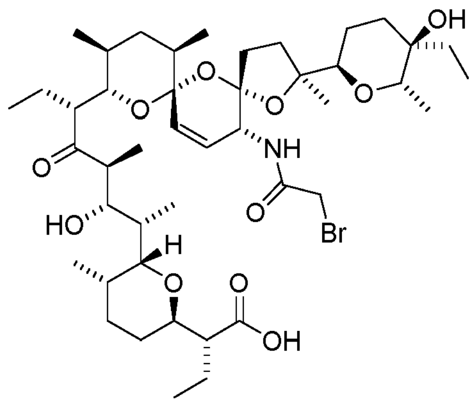

FLC-00501-1

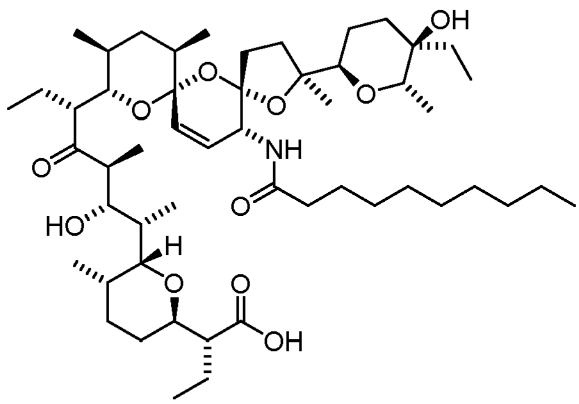

FLC-00528-1

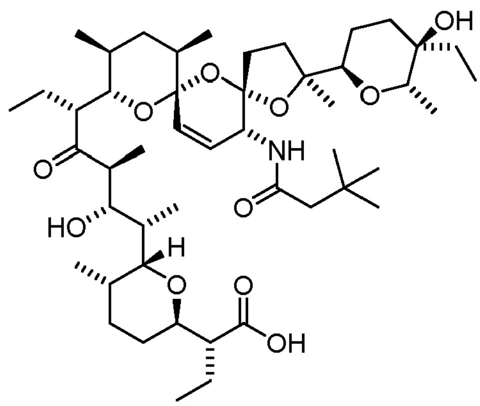

FLC-00534-1

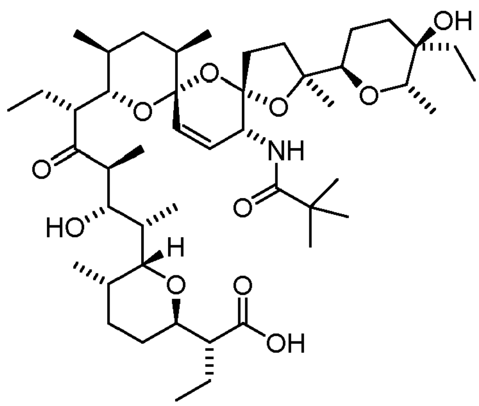

FLC-00550-1

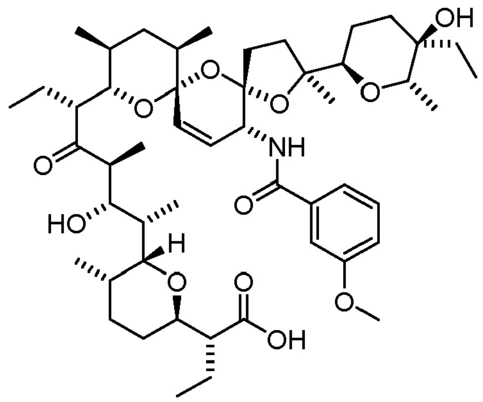

FLC-00569-1

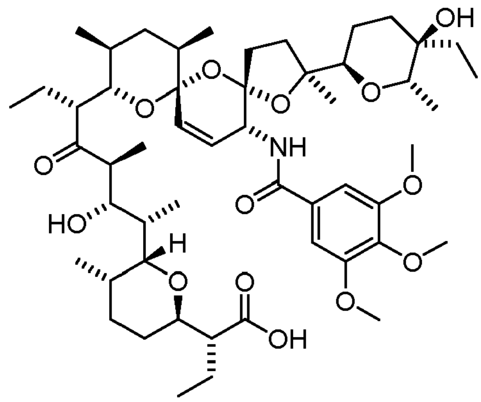

FLC-00573-1

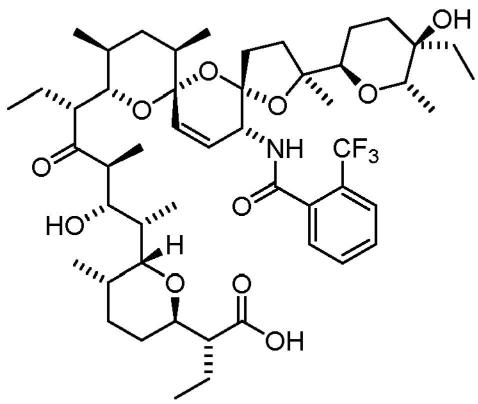

FLC-00578-1

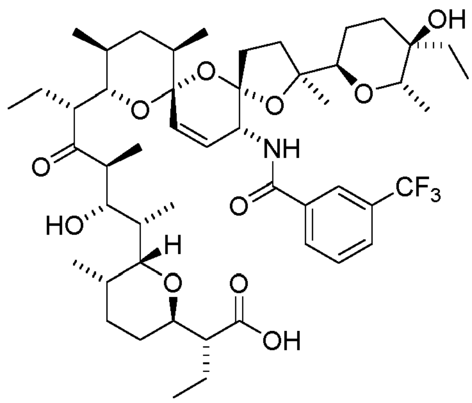

FLC-00579-1

for X =

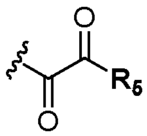

:

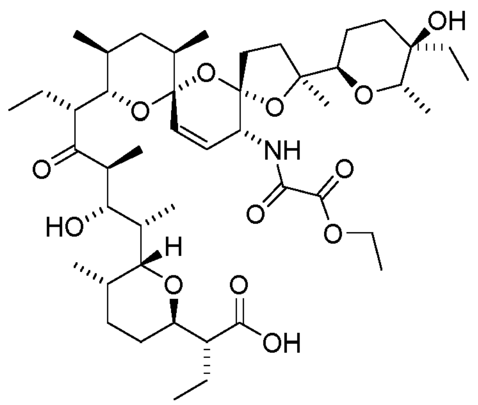

FLC-00373-1

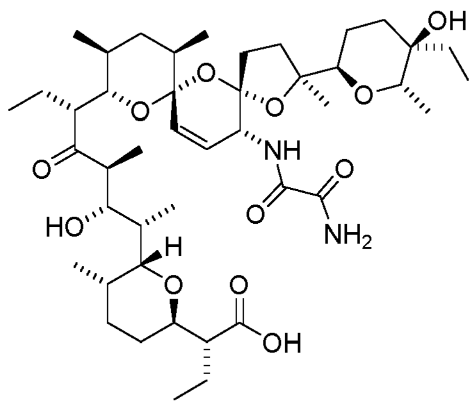

FLC-00376-1

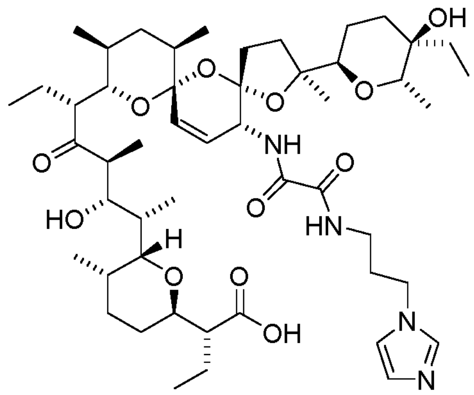

FLC-00392-1

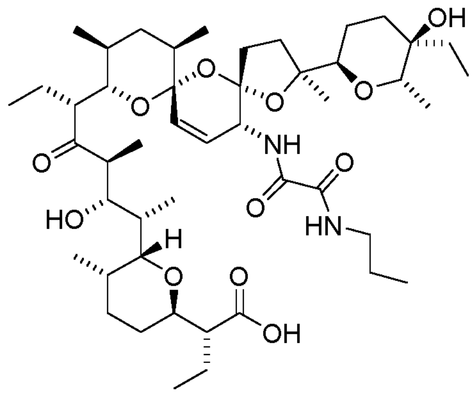

FLC-00423-1

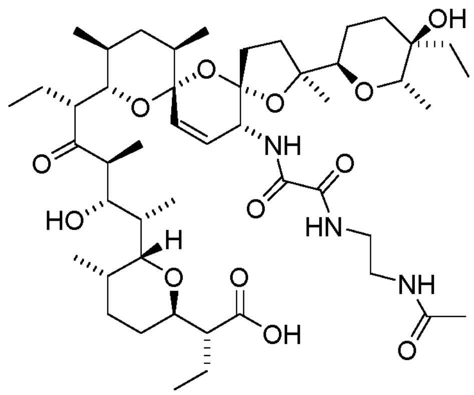

FLC-00430-1

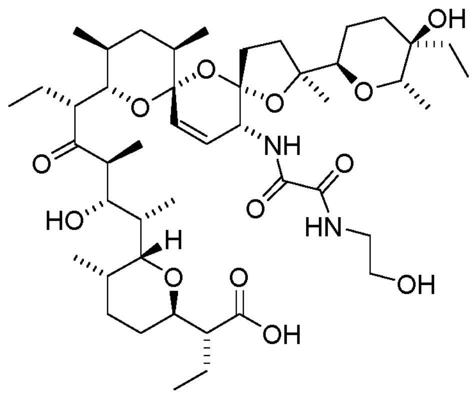

FLC-00438-1

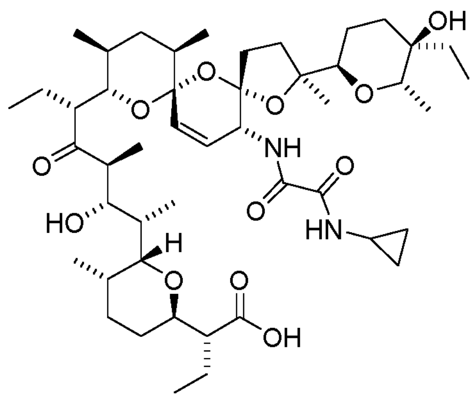

FLC-00441-1

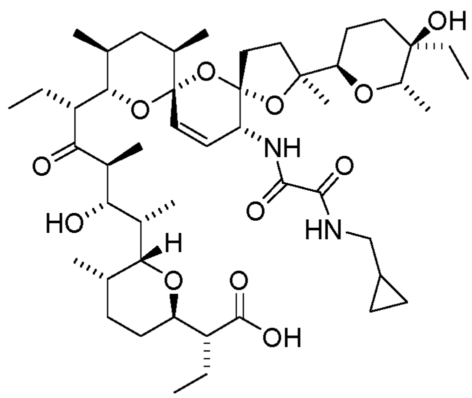

FLC-00442-1

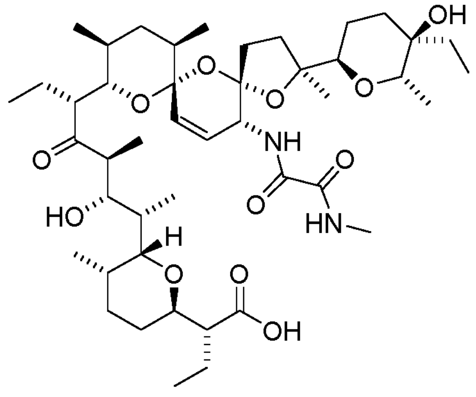

FLC-00443-1

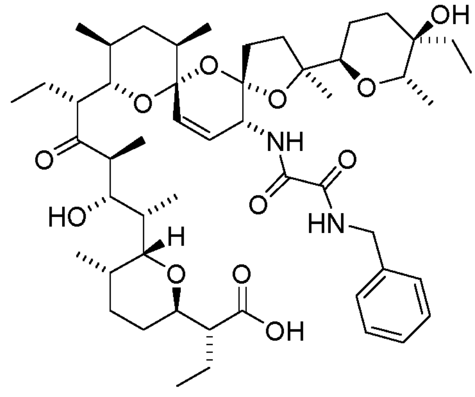

FLC-00452-1

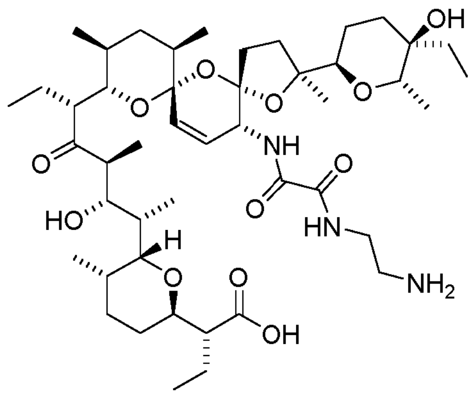

FLC-00456-1

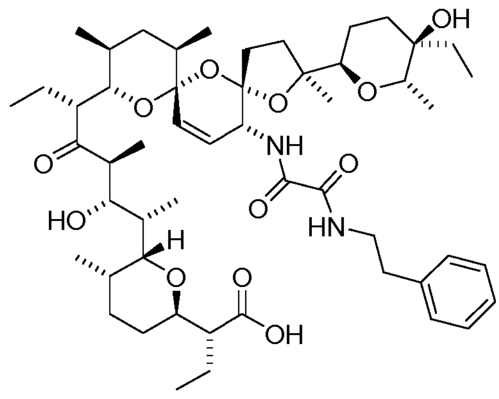

FLC-00457-1

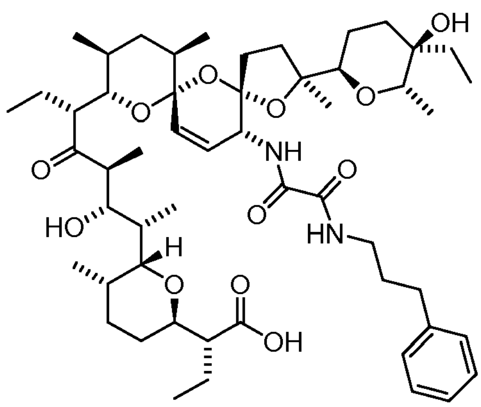

FLC-00458-1

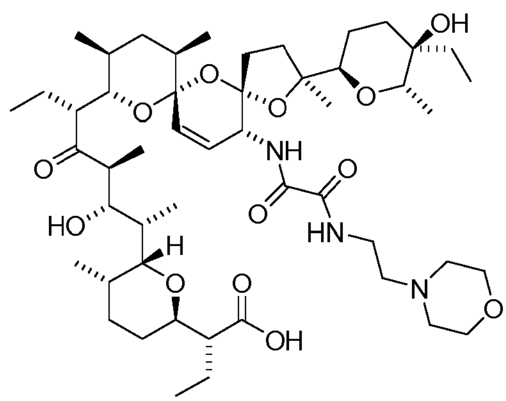

FLC-00465-1

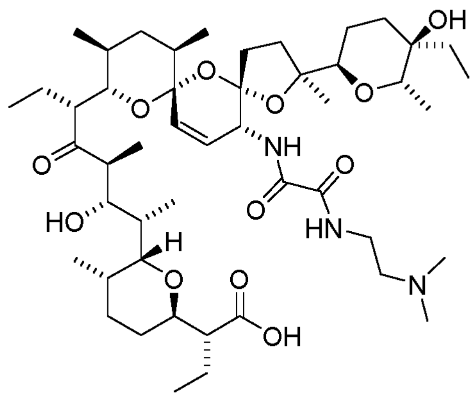

FLC-00471-1

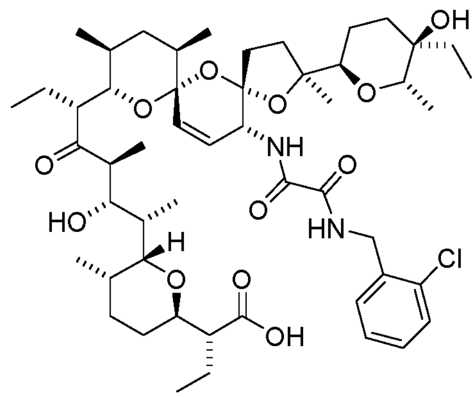

FLC-00477-1

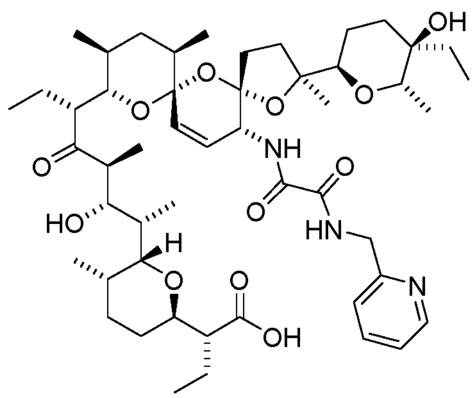

FLC-00478-1

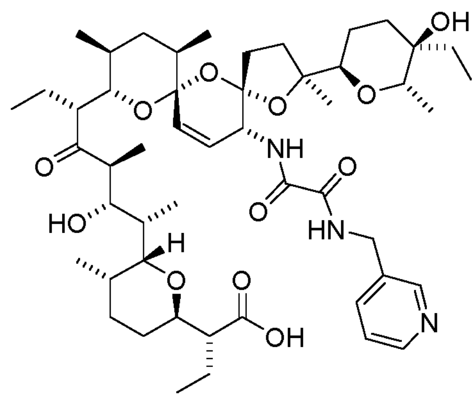

FLC-00479-1

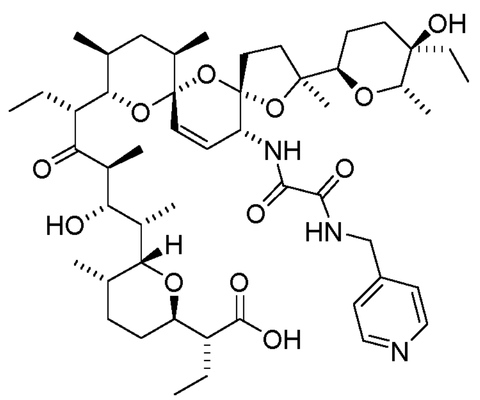

FLC-00489-1

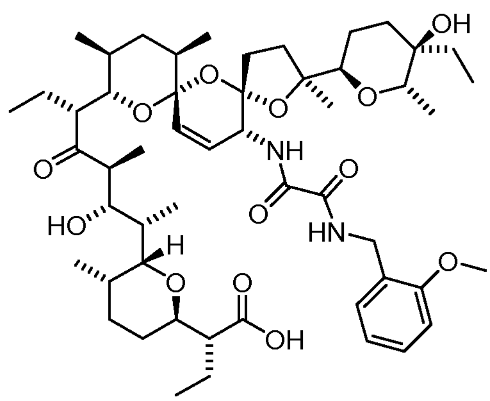

FLC-00491-1

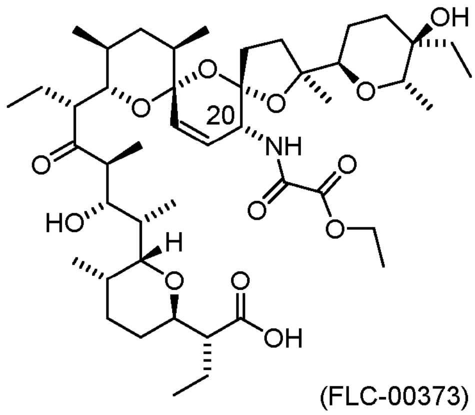

(FLC-00373)

for X =

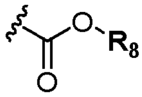

:

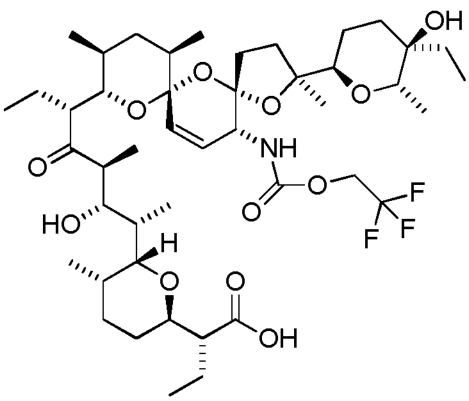

FLC-00395-1

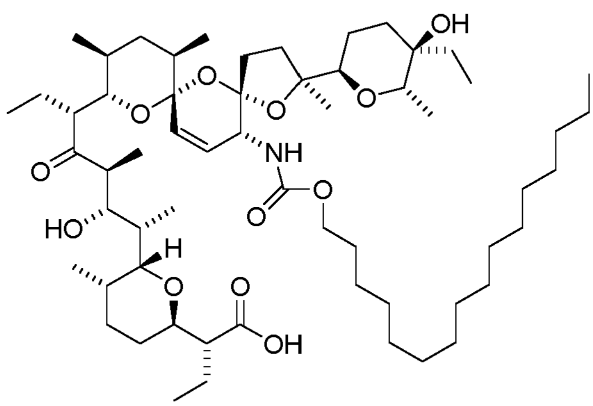

FLC-00410-1

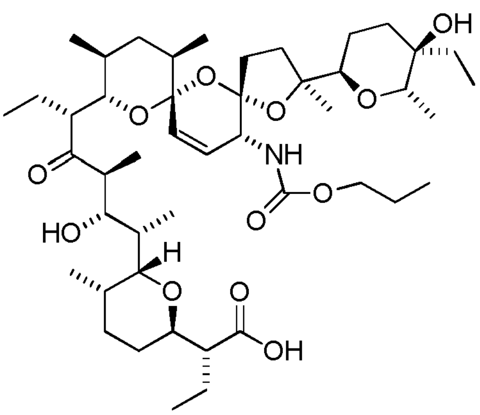

FLC-00424-1

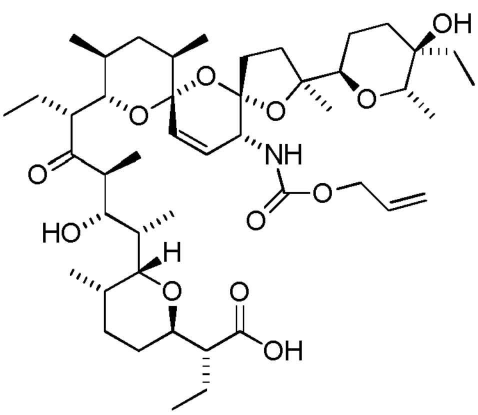

FLC-00425-1

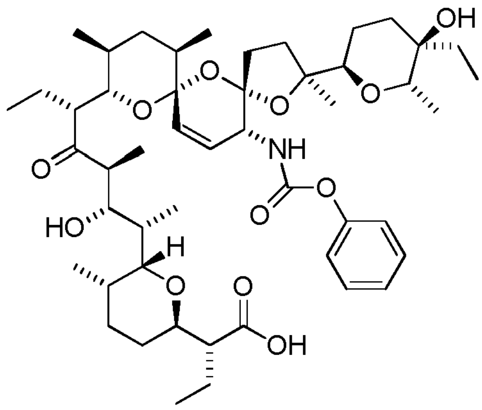

FLC-00429-1

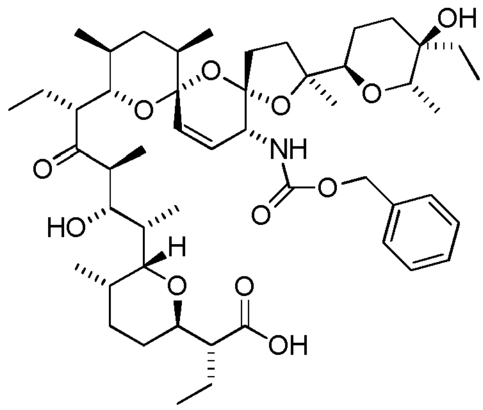

FLC-00431-1

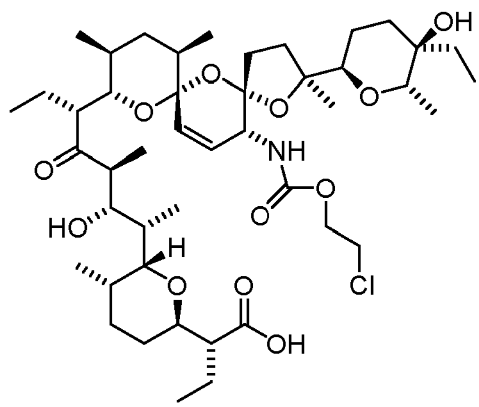

FLC-00436-1

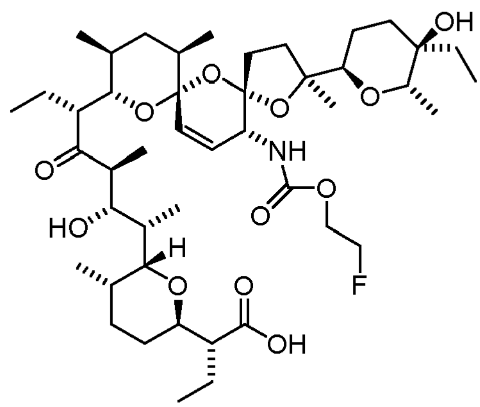

FLC-00437-1

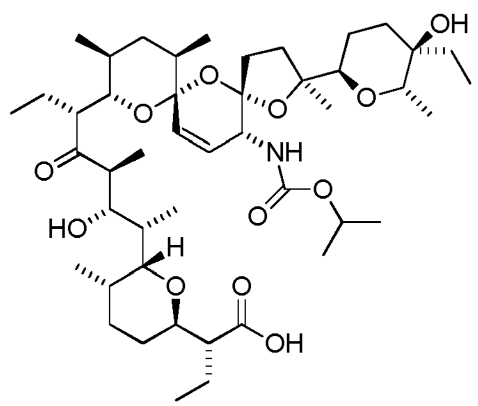

FLC-00439-1

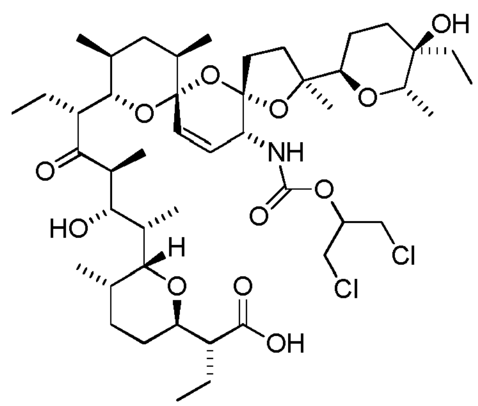

FLC-00440-1

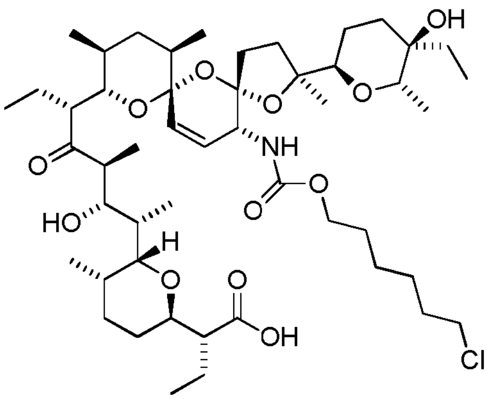

FLC-00444-1

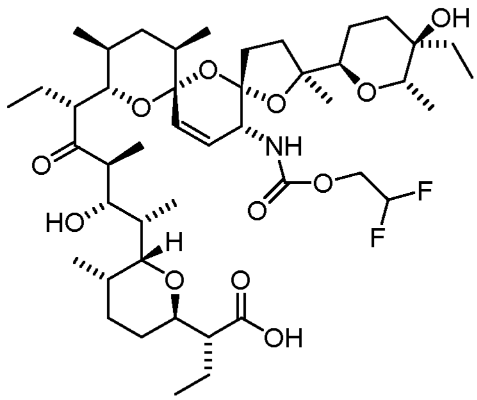

FLC-00446-1

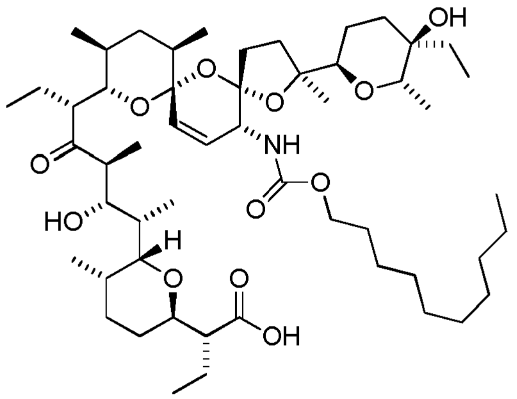

FLC-00447-1

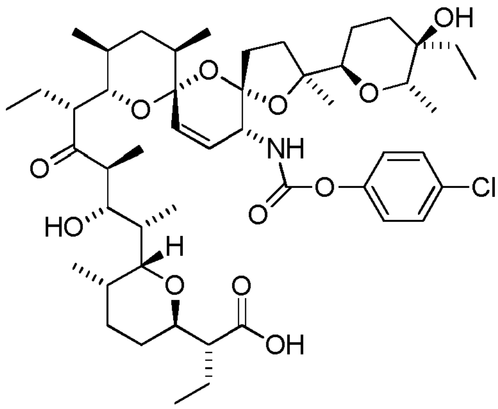

FLC-00450-1

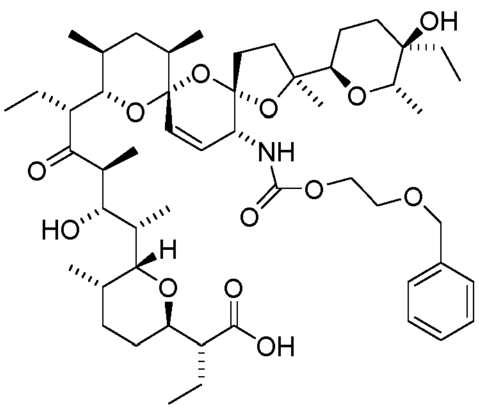

FLC-00451-1

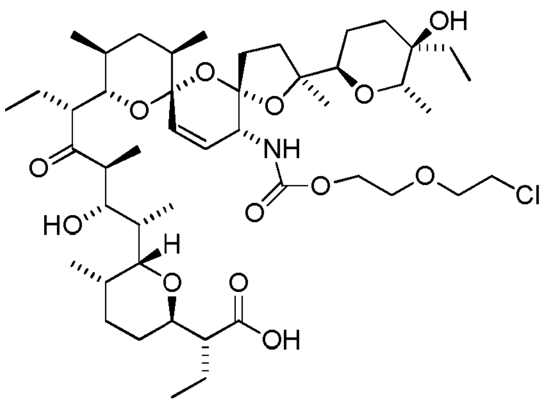

FLC-00454-1

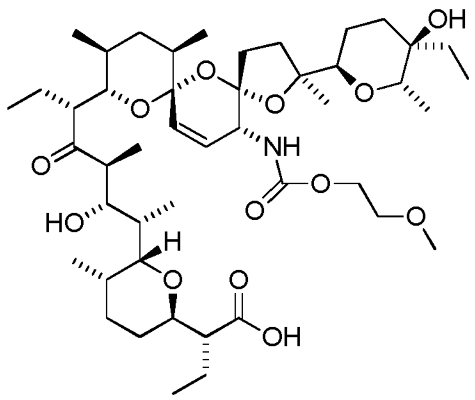

FLC-00459-1

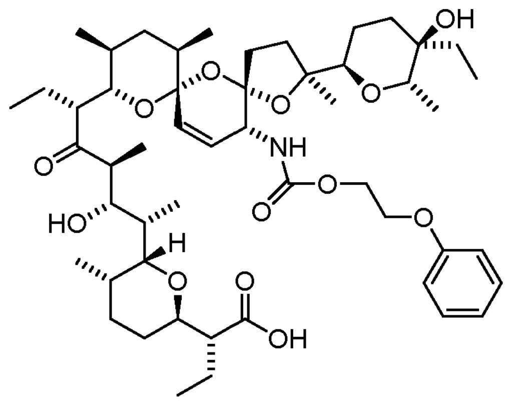

FLC-00460-1

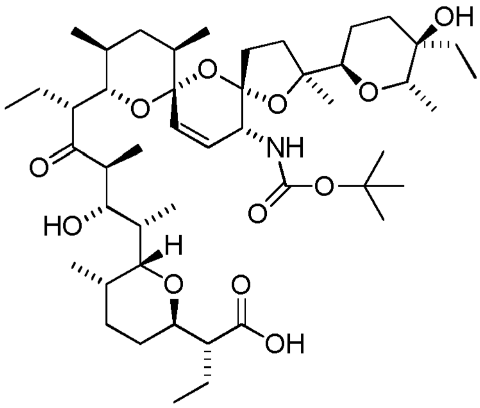

FLC-00461-1

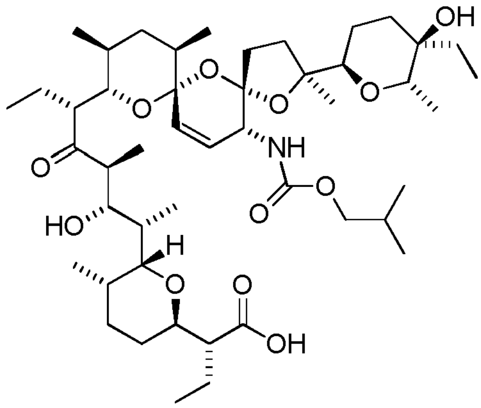

FLC-00466-1

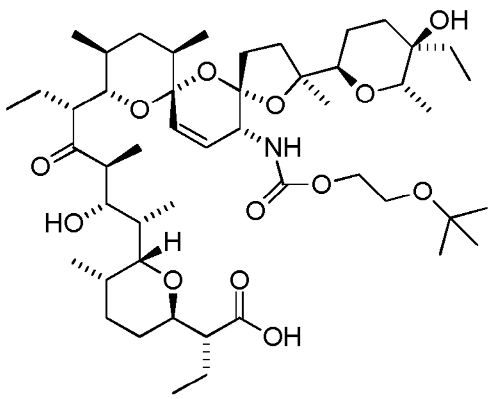

FLC-00480-1

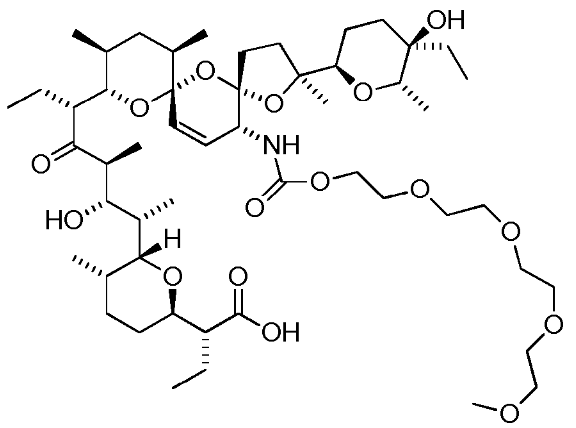

FLC-00481-1

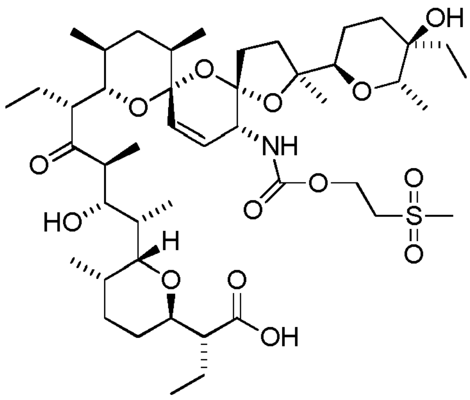

FLC-00483-1

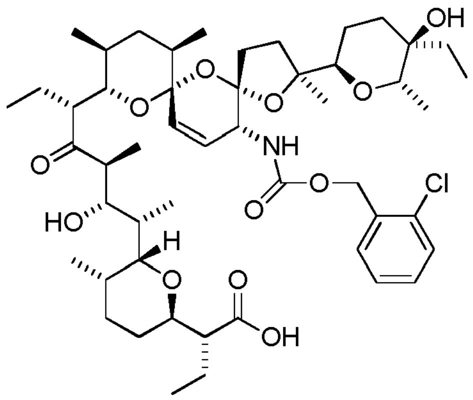

FLC-00484-1

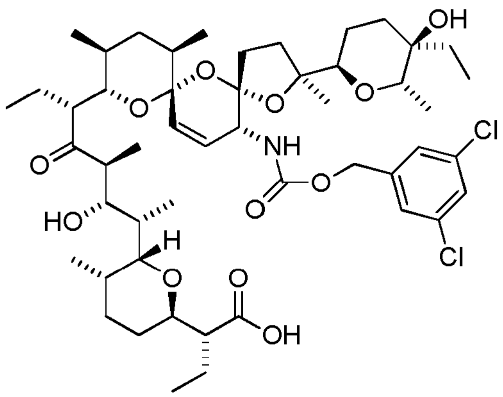

FLC-00485-1

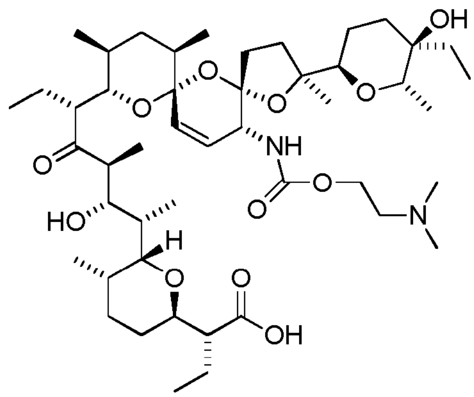

FLC-00497-1

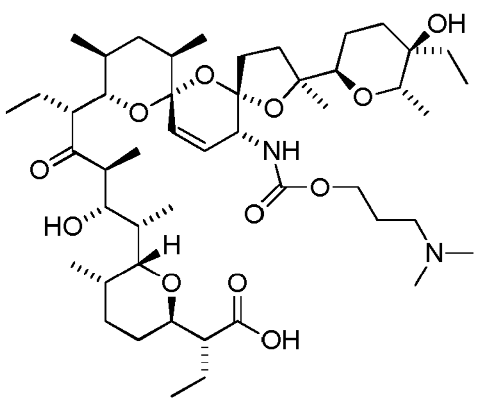

FLC-00498-1

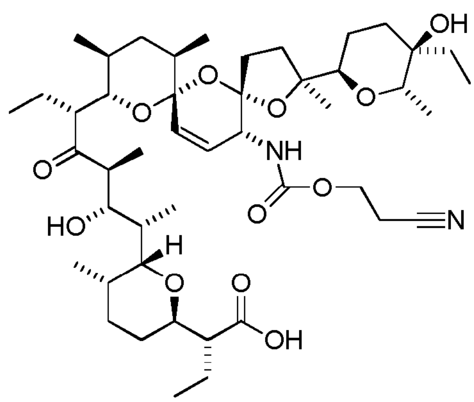

FLC-00509-1

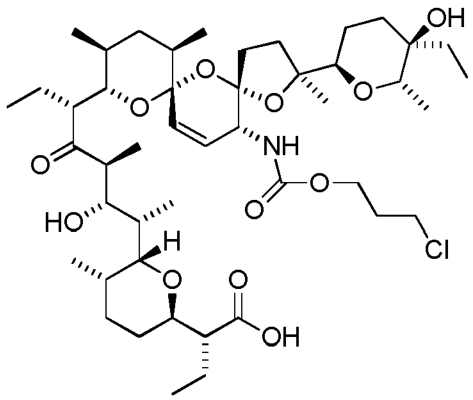

FLC-00530-1

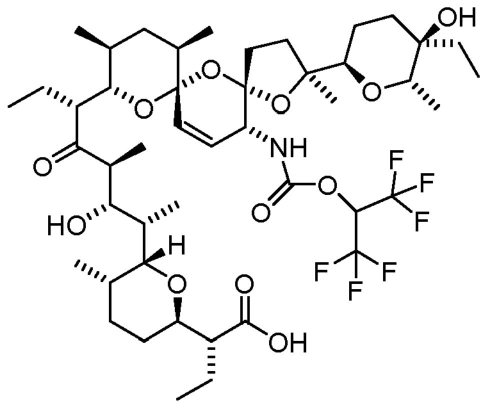

FLC-00531-1

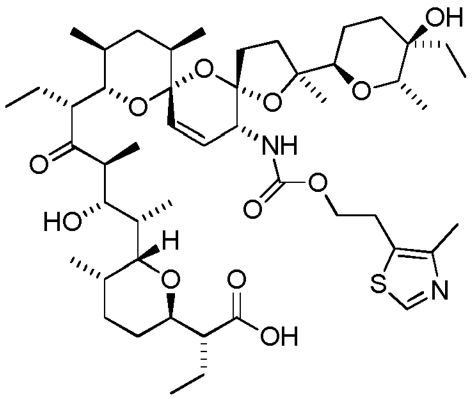

FLC-00547-1

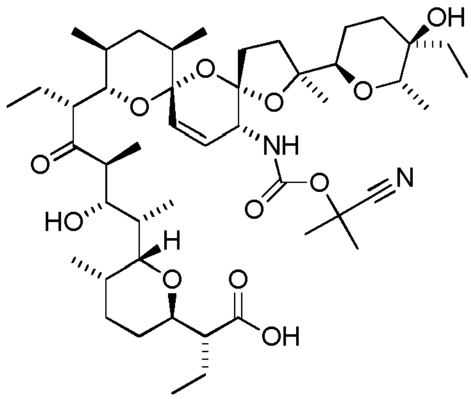

FLC-00551-1

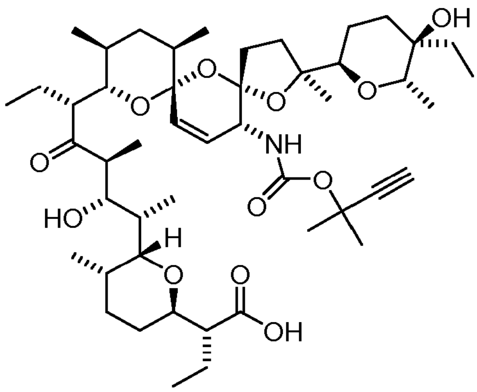

FLC-00552-1

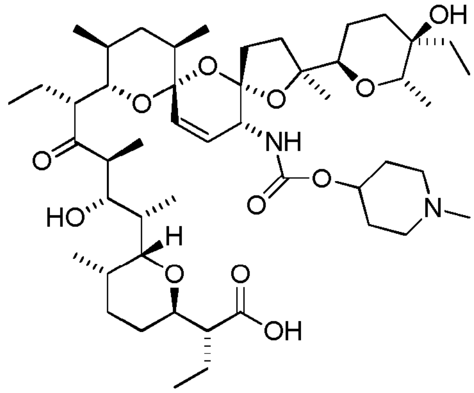

FLC-00559-1

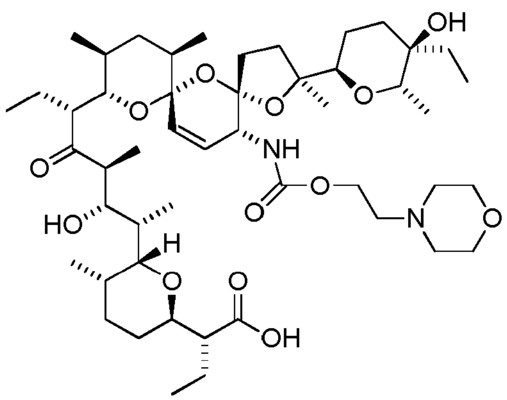

FLC-00570-1

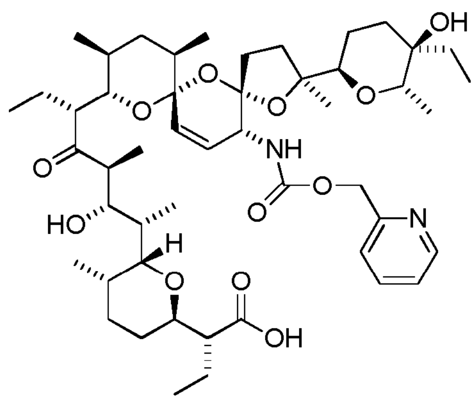

FLC-00571-1

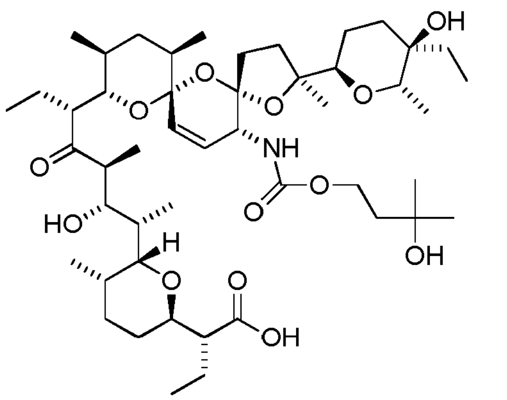

FLC-00577-1

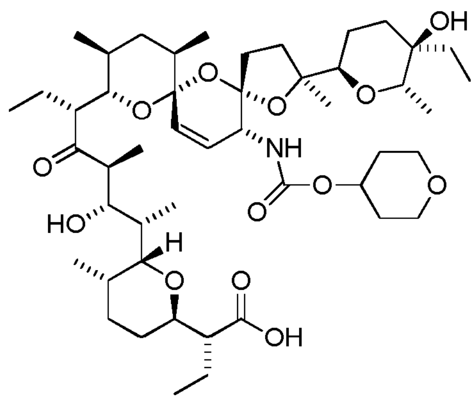

FLC-00580-1

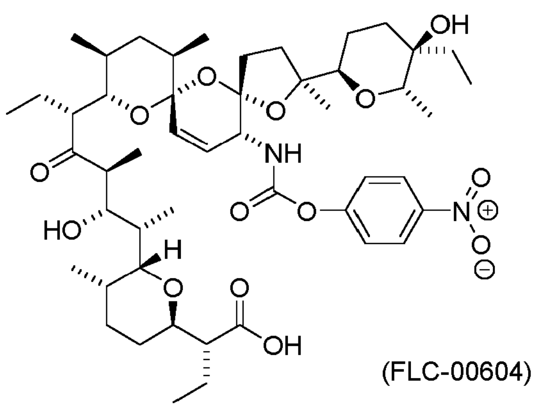

(FLC-00604)

for X =

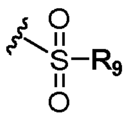

:

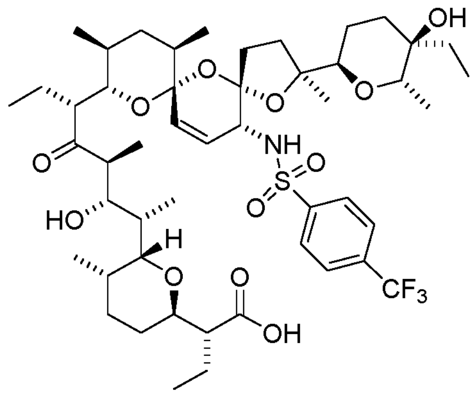

FLC-00468-1

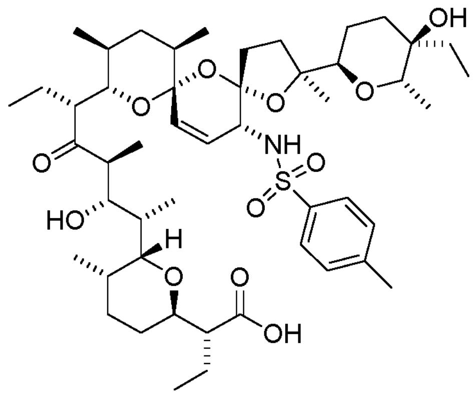

FLC-00502-1

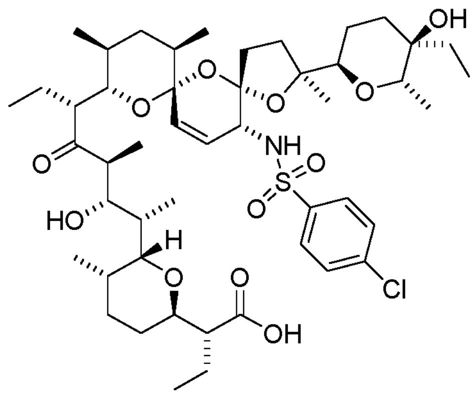

FLC-00503-1

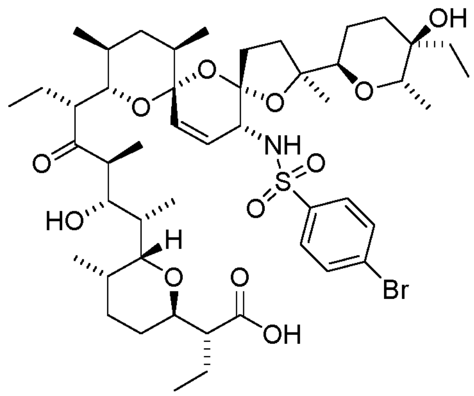

FLC-00504-1

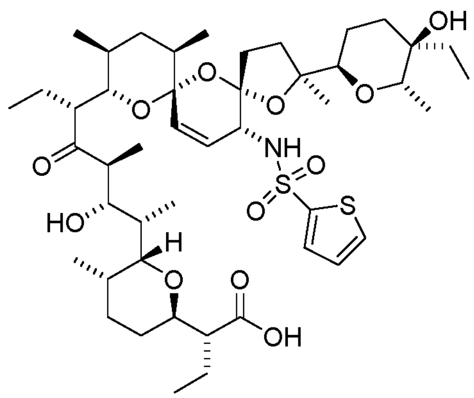

FLC-00505-1

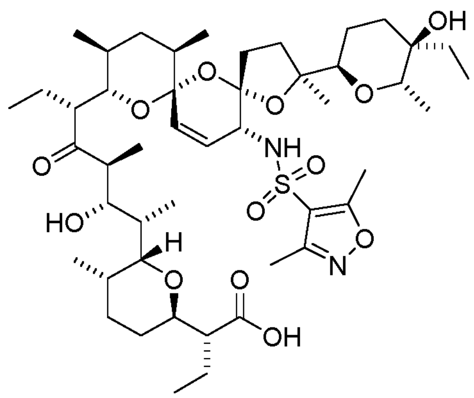

FLC-00506-1

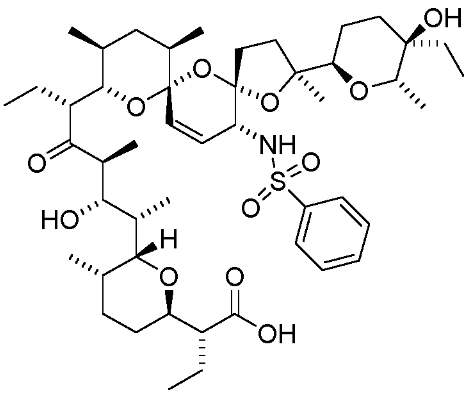

FLC-00514-1

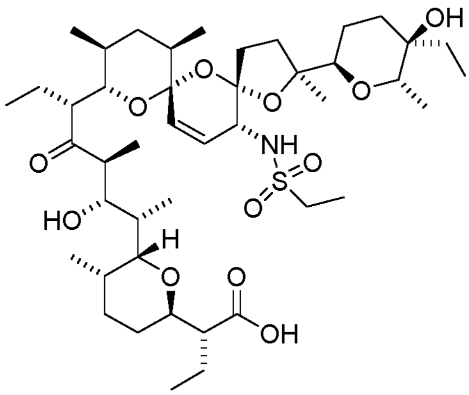

FLC-00536-1

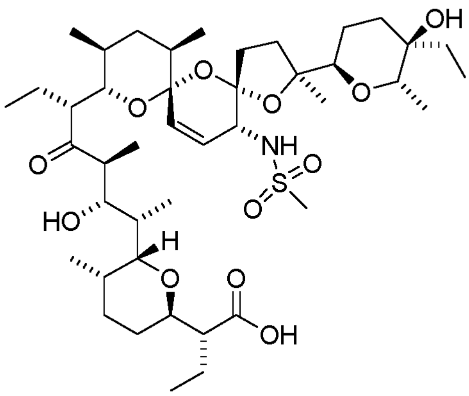

FLC-00548-1

for X =

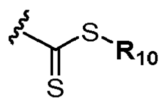

:

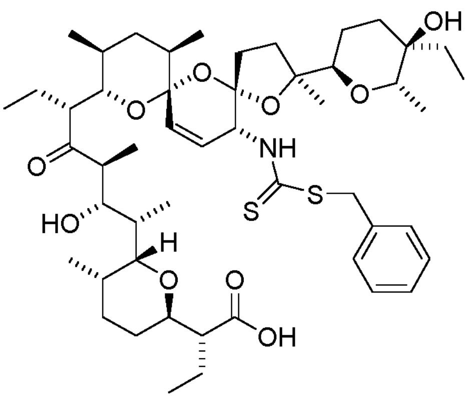

FLC-00474-1

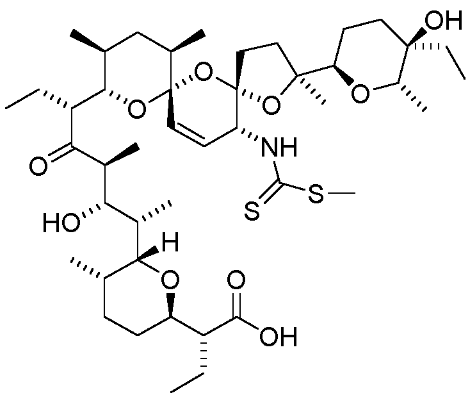

FLC-00475-1

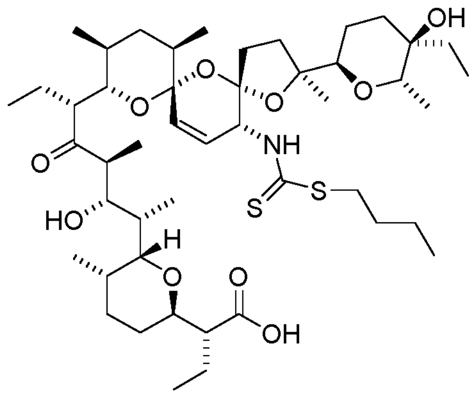

FLC-00476-1

for X =

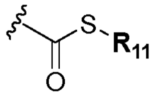

:

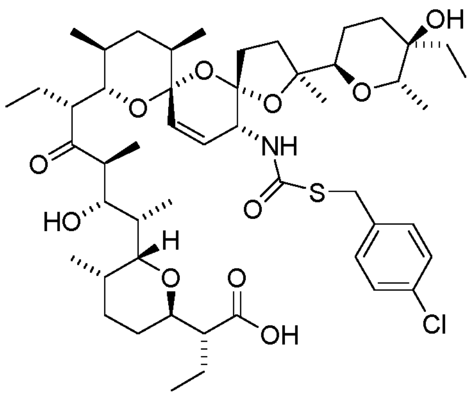

FLC-00553-1

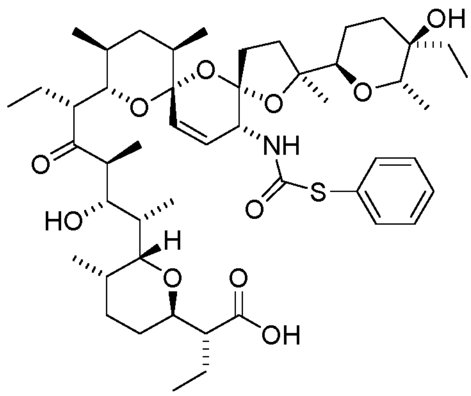

FLC-00554-1

or salts of these compounds.

The following compounds are more preferred:

for X =

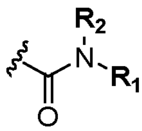

:

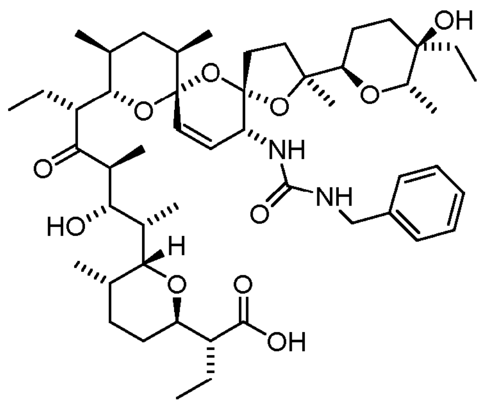

FLC-0337-1

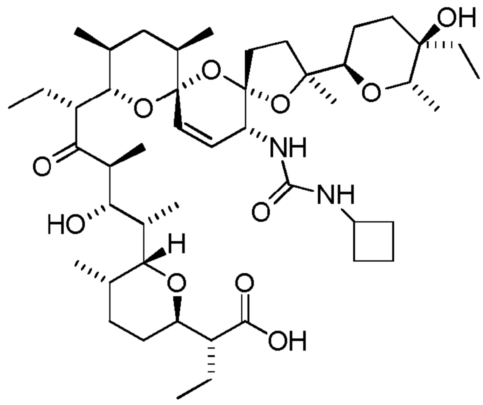

FLC-00341-1

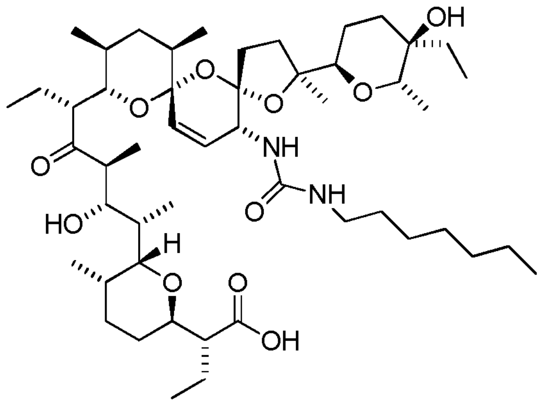

FLC-00350-1

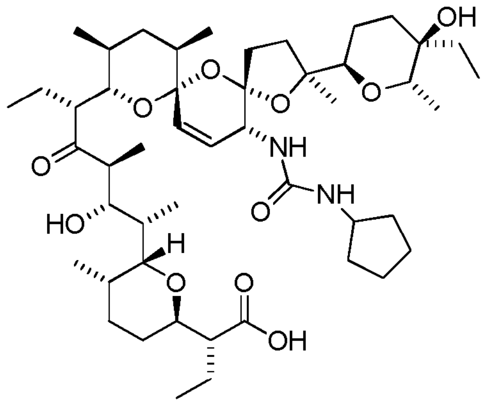

FLC-00351-1

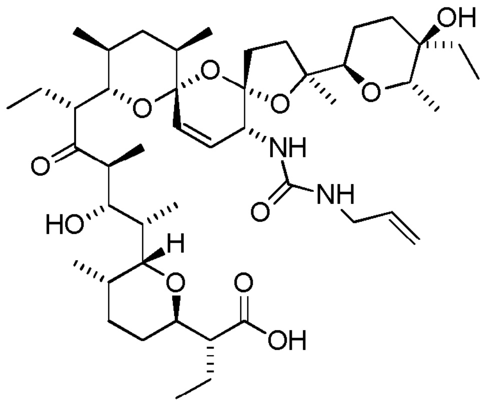

FLC-00353-1

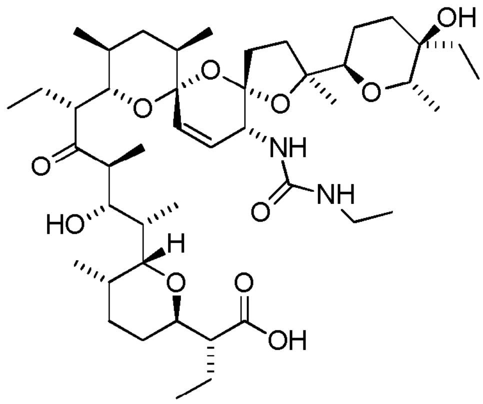

FLC-00358-1

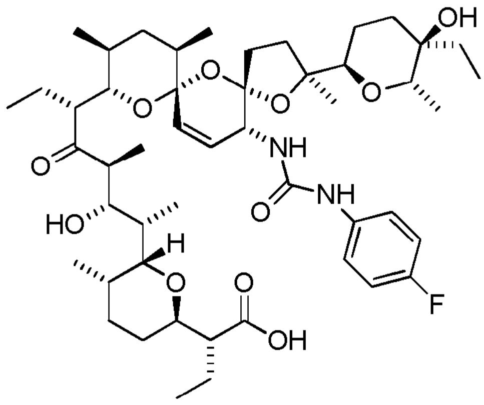

FLC-00363-1

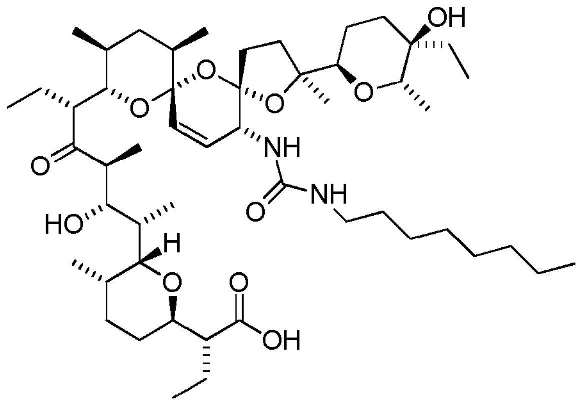

FLC-00366-1

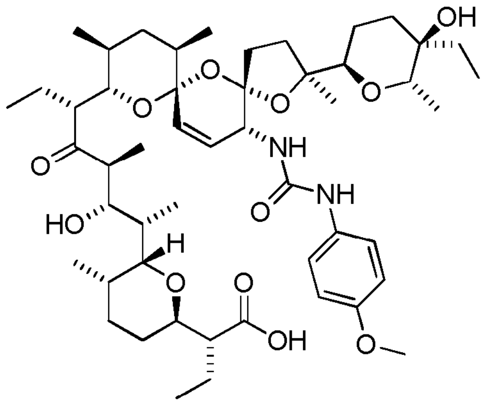

FLC-00371-1

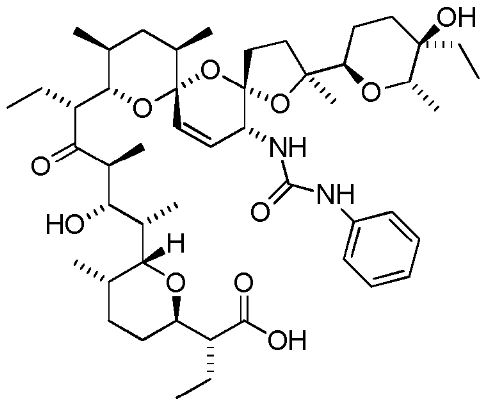

FLC-00374-1

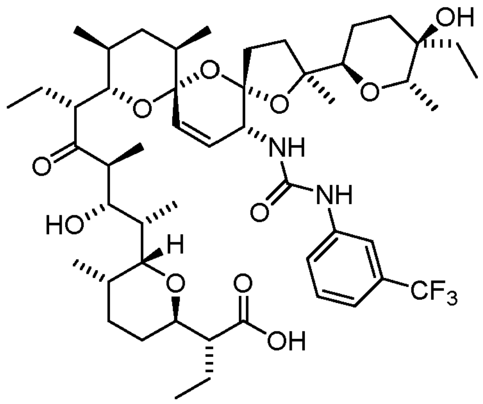

FLC-00385-1

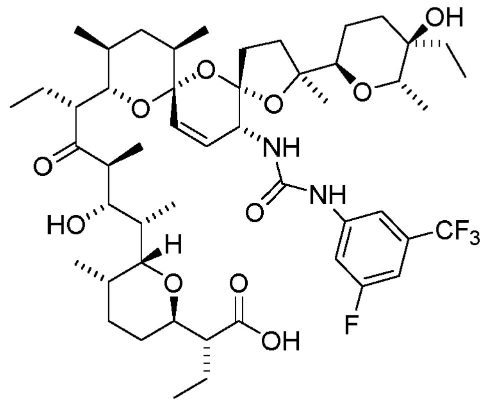

FLC-00427-1

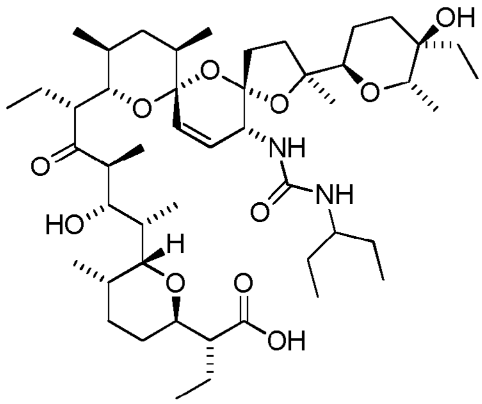

FLC-00541-1

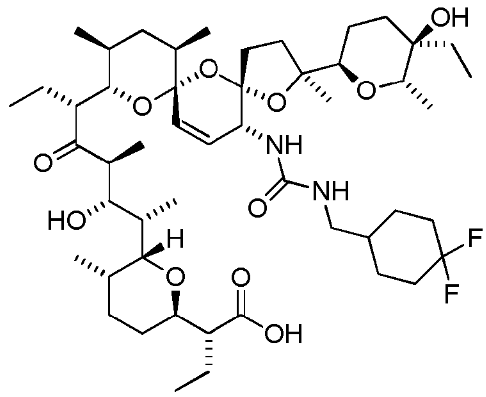

FLC-00545-1

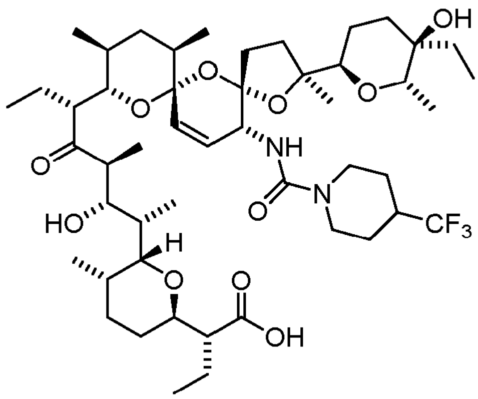

FLC-00561-1

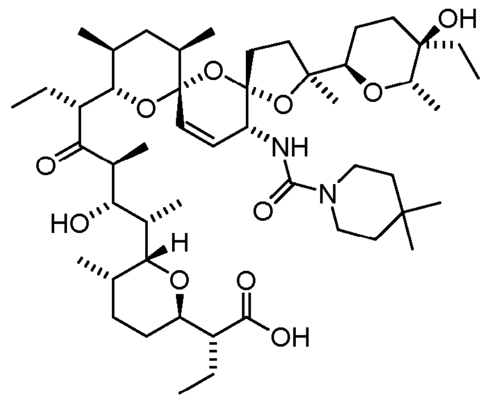

FLC-00565-1

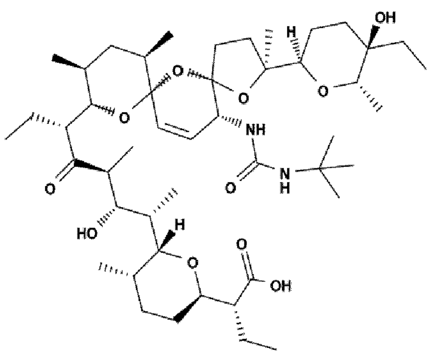

FLC-00593-1

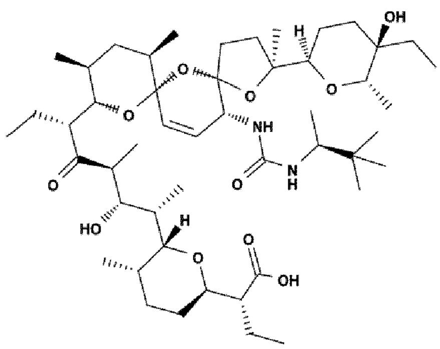

FLC-00606-1

for X =

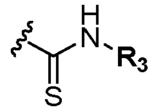

:

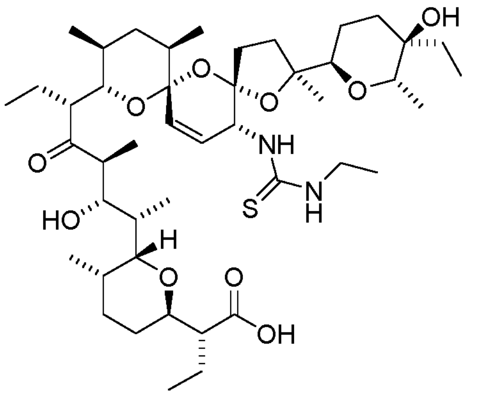

FLC-00339-1

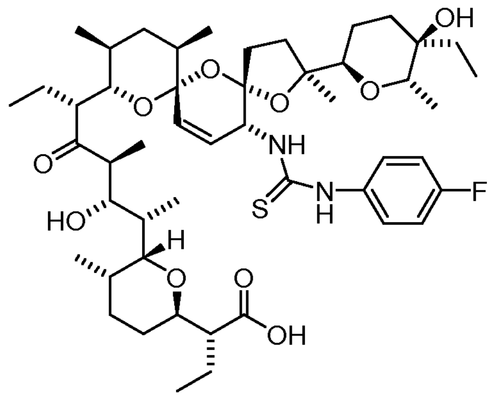

FLC-00340-1

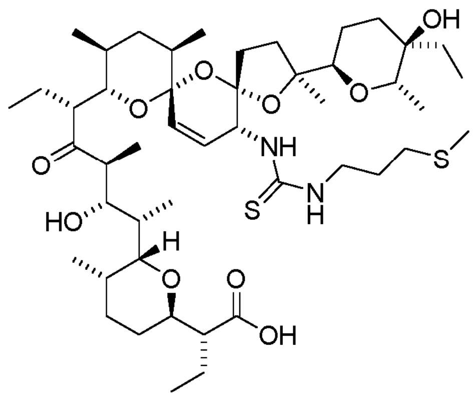

FLC-00345-1

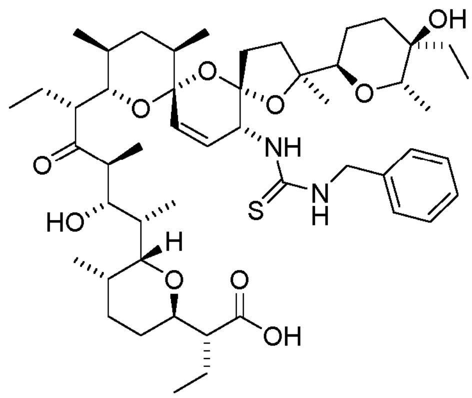

FLC-00346-1

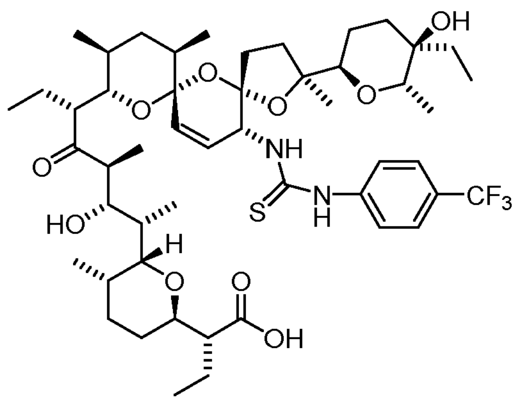

FLC-00347-1

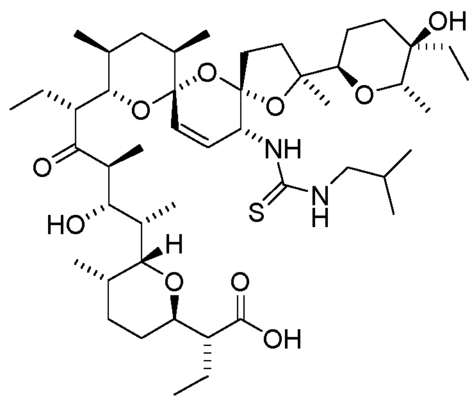

FLC-00369-1

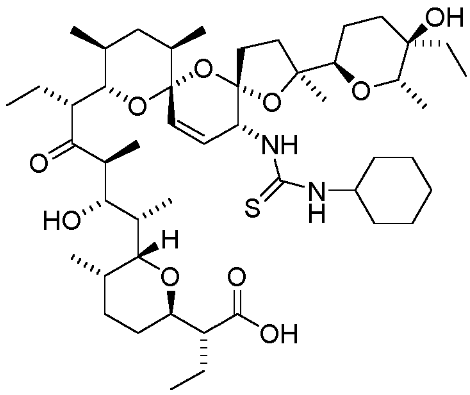

FLC-00377-1

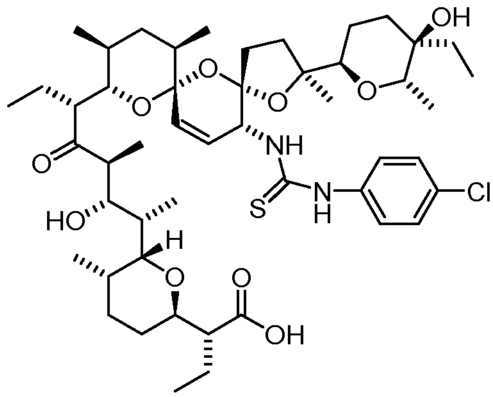

FLC-00378-1

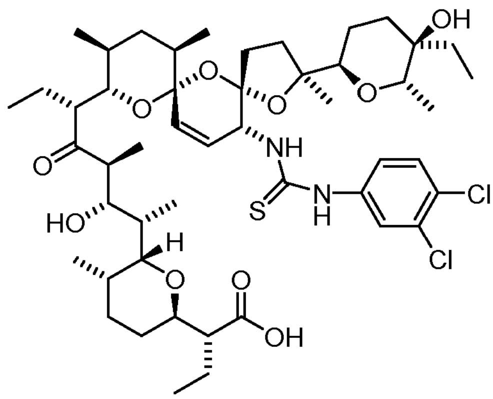

FLC-00379-1

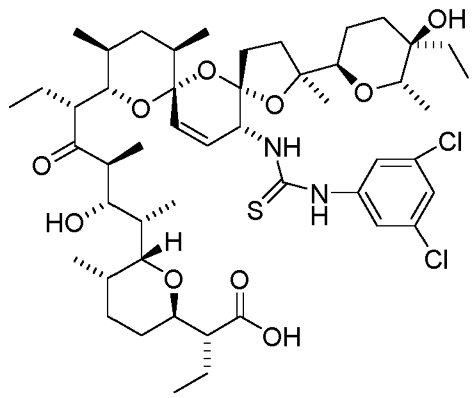

FLC-00380-1

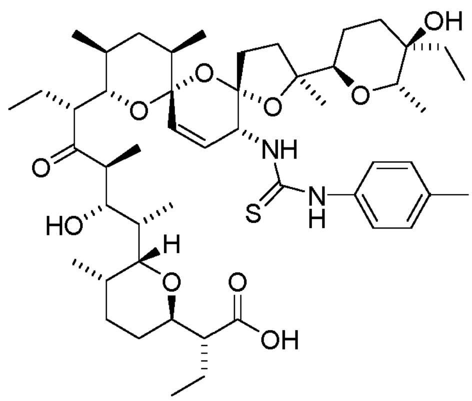

FLC-00382-1

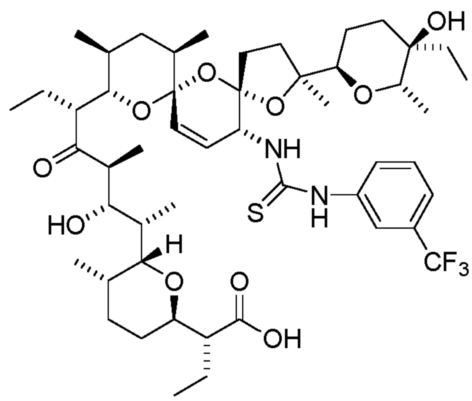

FLC-00386-1

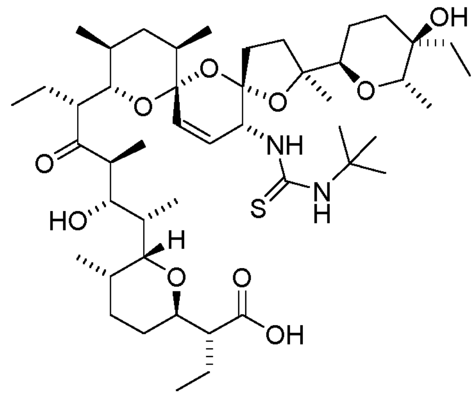

FLC-00467-1

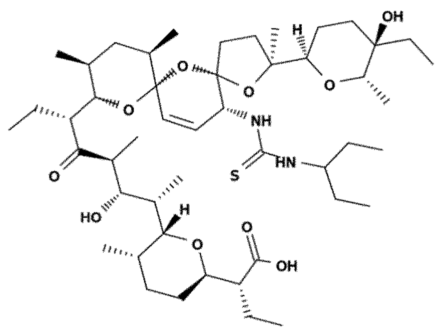

FLC-00771-1

for X =

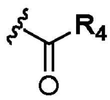

:

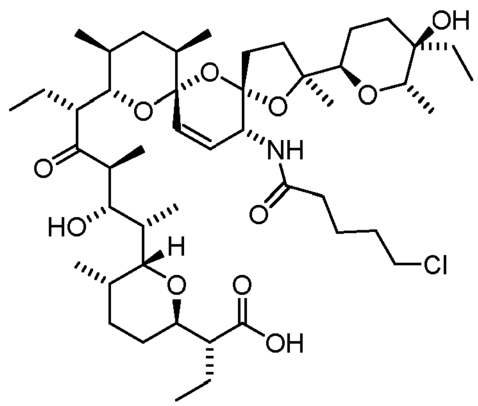

FLC-00445-1

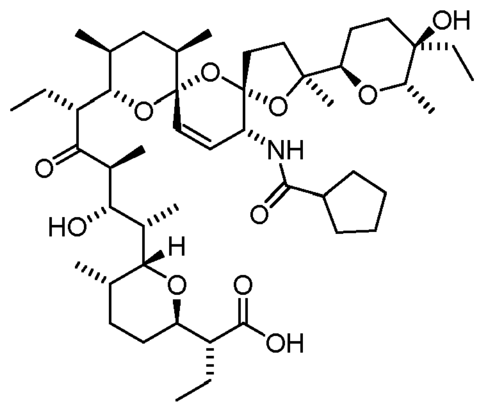

FLC-00455-1

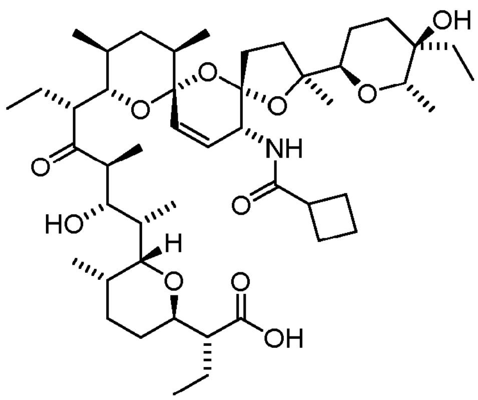

FLC-00463-1

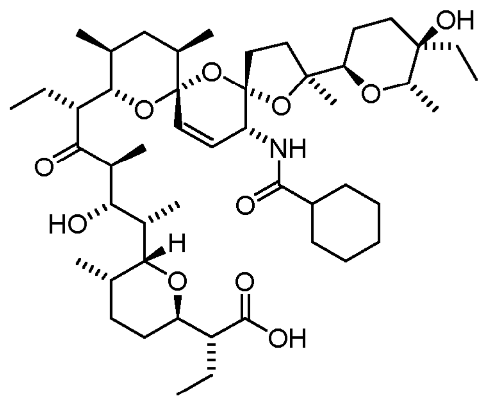

FLC-00464-1

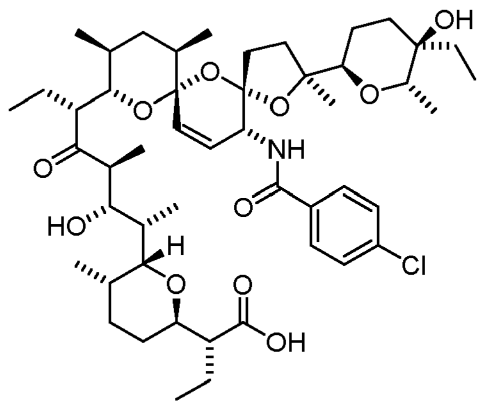

FLC-00469-1

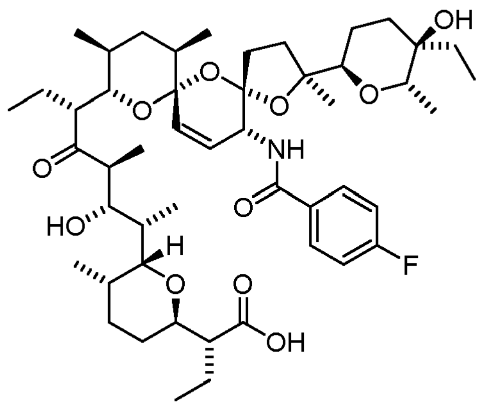

FLC-00470-1

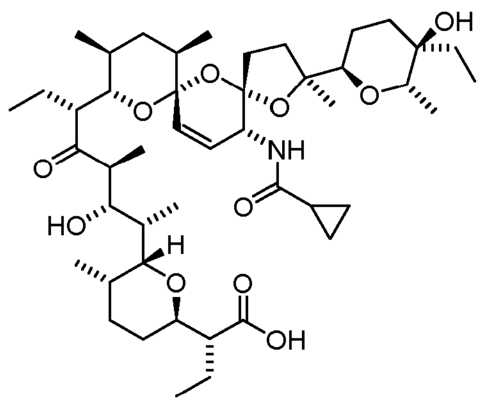

FLC-00472-1

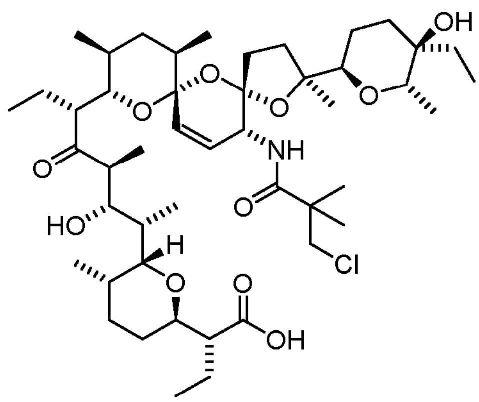

FLC-00473-1

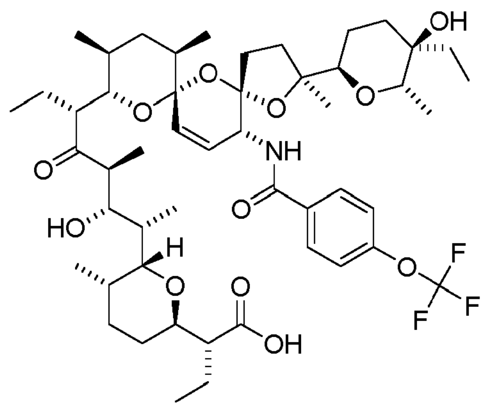

FLC-00486-1

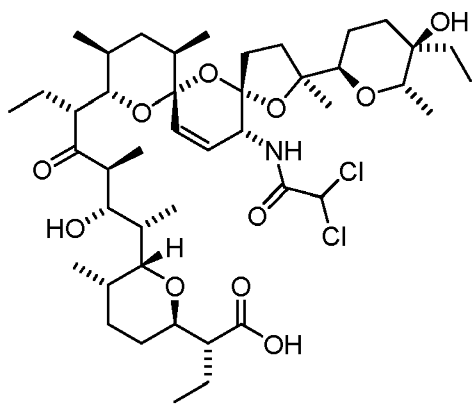

FLC-00493-1

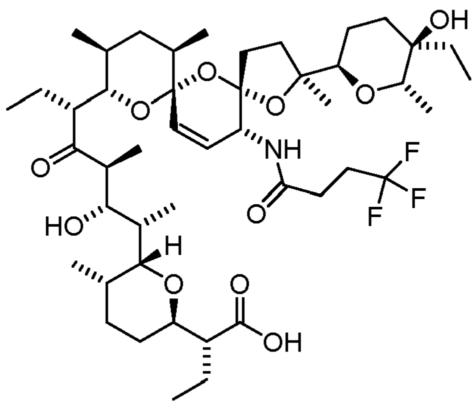

FLC-00494-1

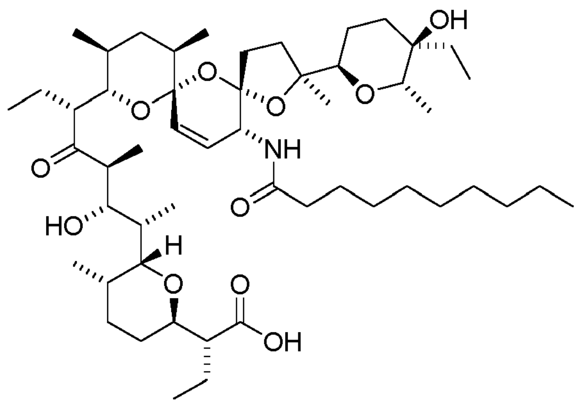

FLC-00528-1

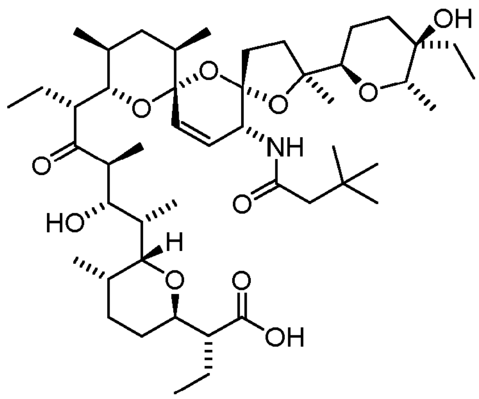

FLC-00534-1

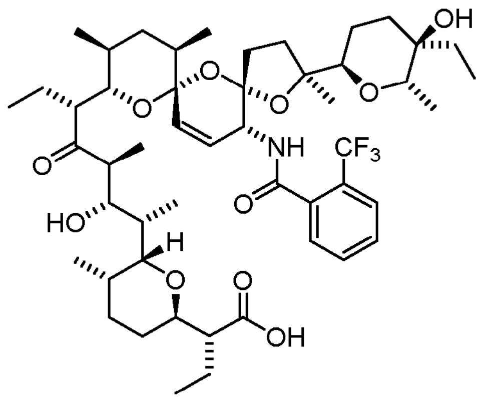

FLC-00578-1

for X =

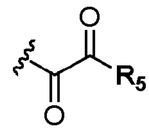

:

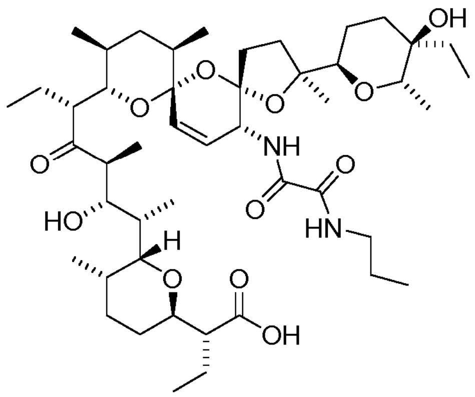

FLC-00423-1

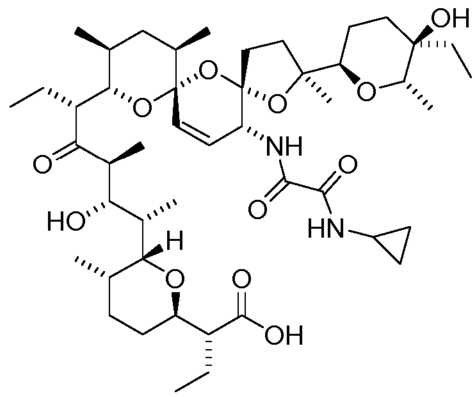

FLC-00441-1

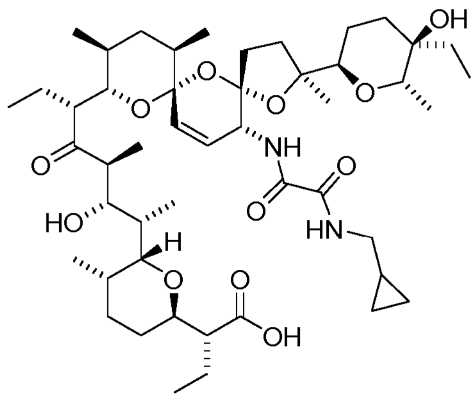

FLC-00442-1

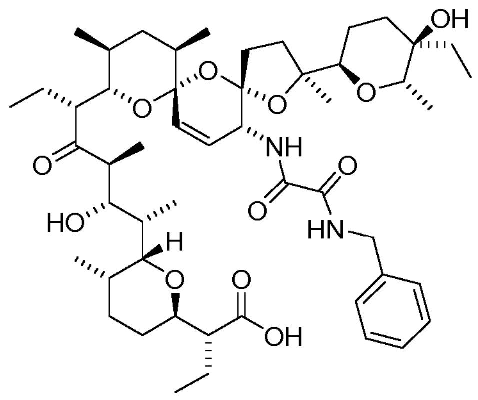

FLC-00452-1

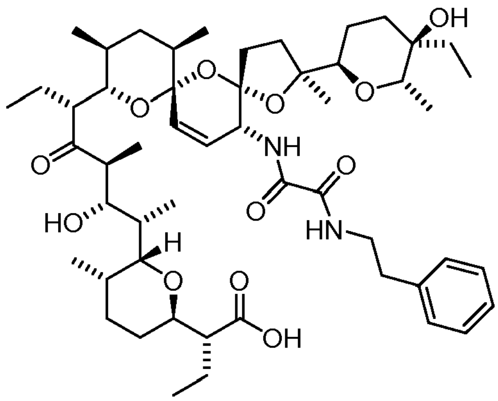

FLC-00457-1

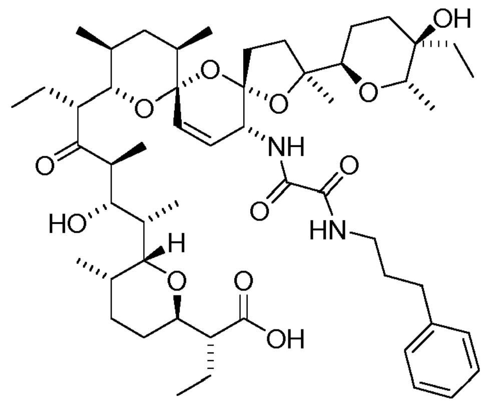

FLC-00458-1

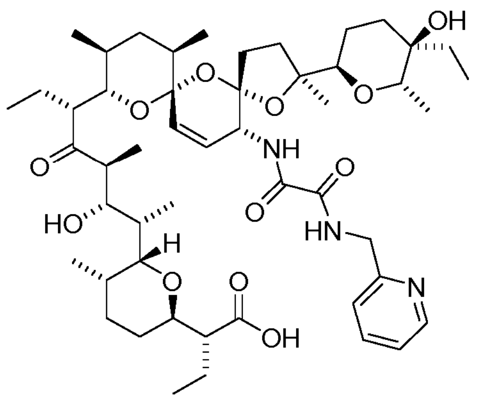

FLC-00478-1

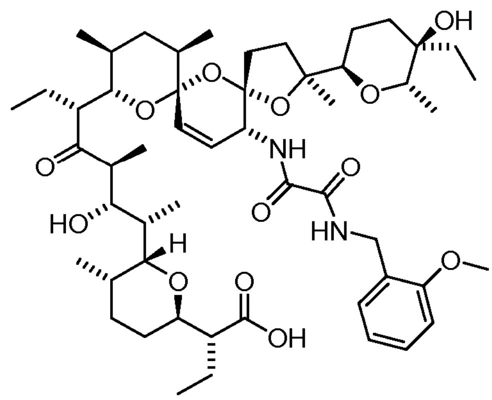

FLC-00491-1

for X =

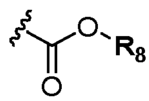

:

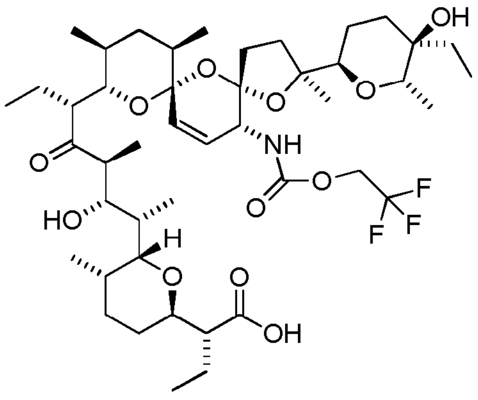

FLC-00395-1

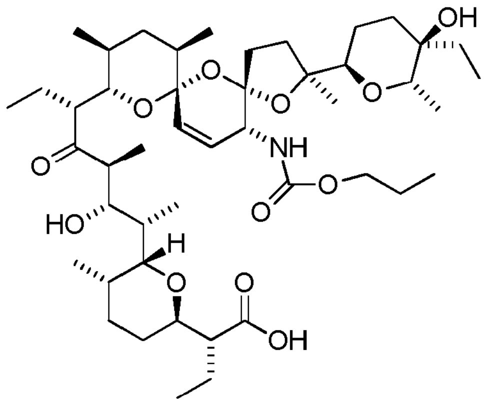

FLC-00424-1

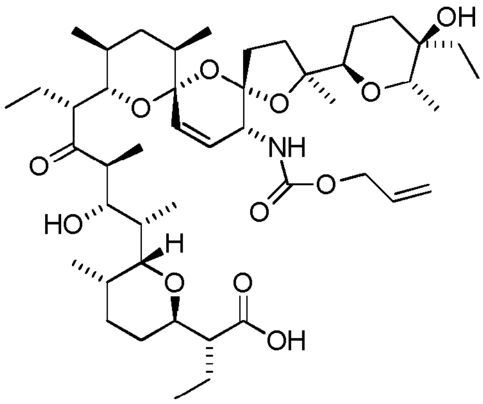

FLC-00425-1

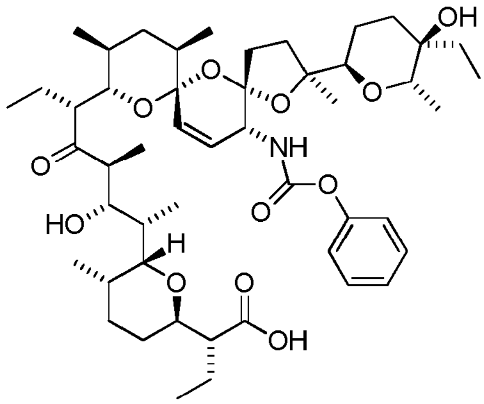

FLC-00429-1

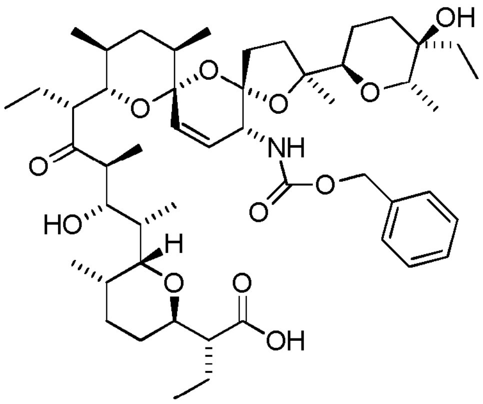

FLC-00431-1

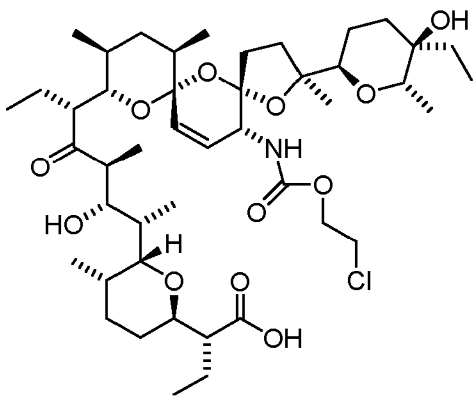

FLC-00436-1

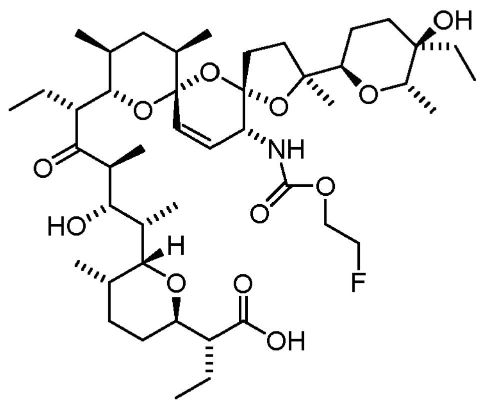

FLC-00437-1

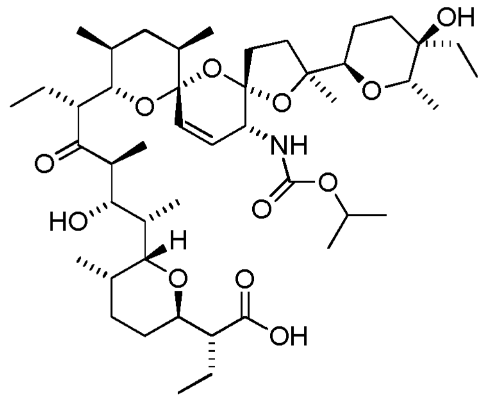

FLC-00439-1

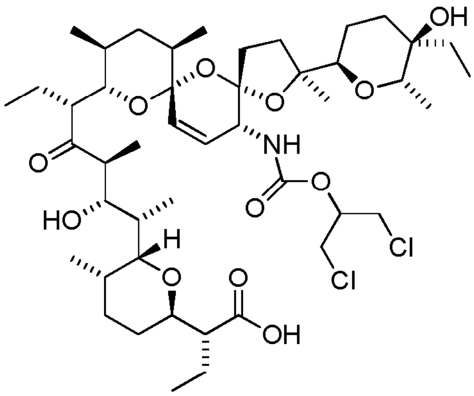

FLC-00440-1

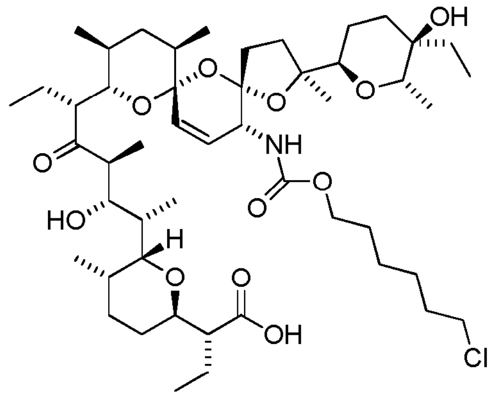

FLC-00444-1

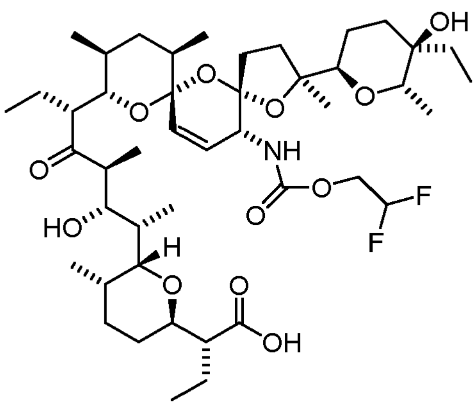

FLC-00446-1

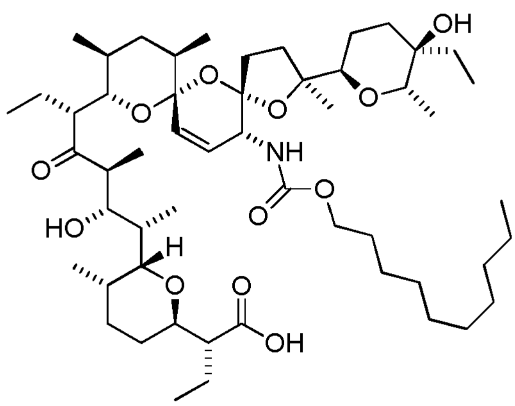

FLC-00447-1

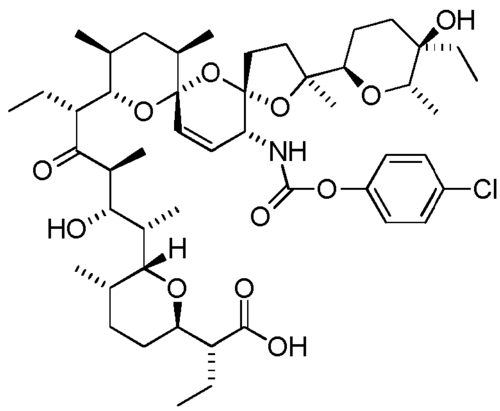

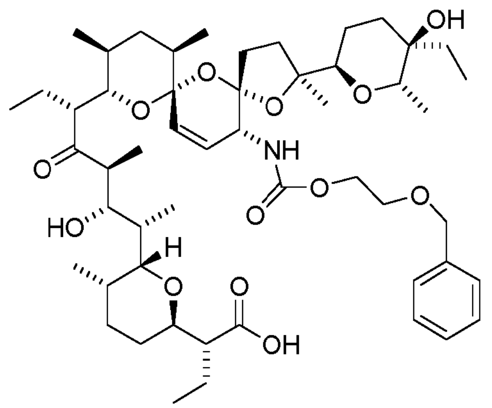

FLC-

FLC-00450-1

FLC-00451-1

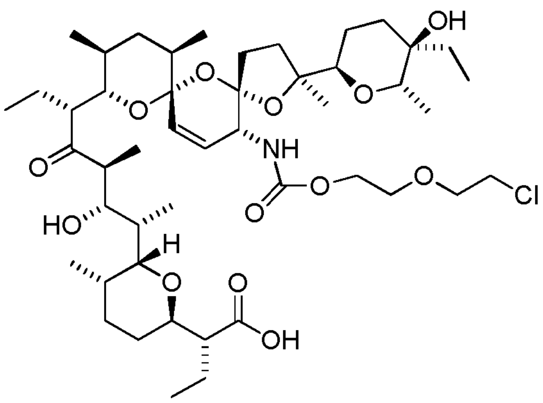

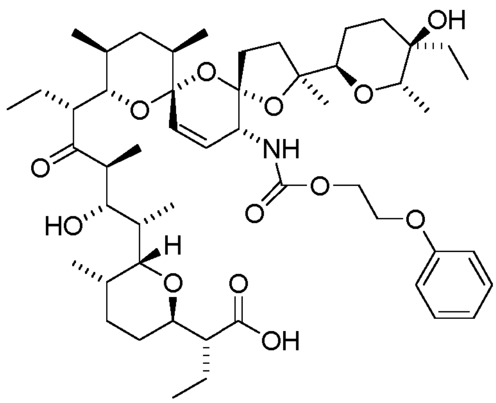

FLC-00454-1

FLC-00460-1

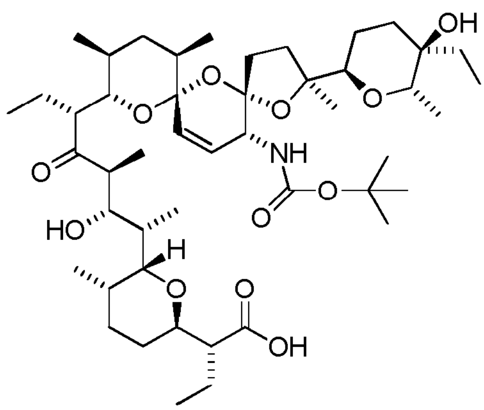

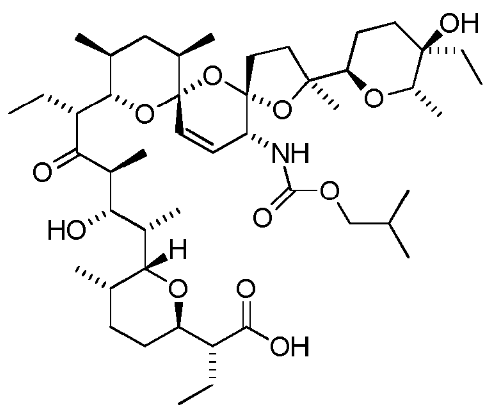

FLC-00461-1

FLC-00466-1

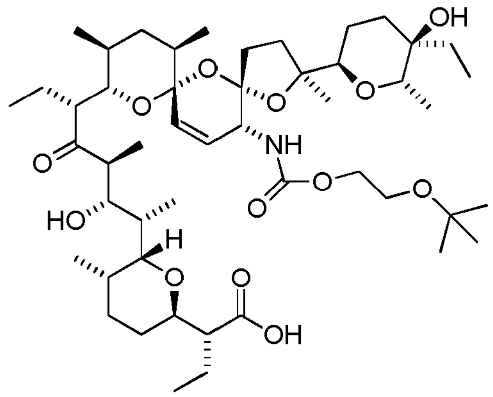

FLC-00480-1

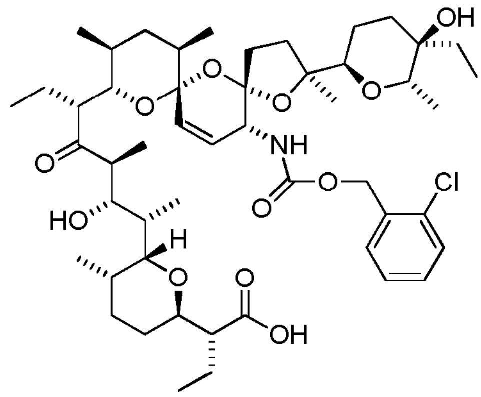

FLC-00484-1

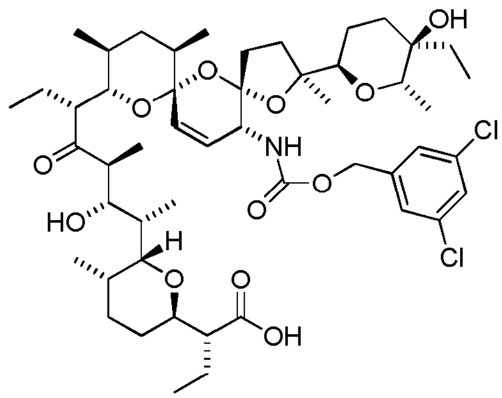

FLC-00485-1

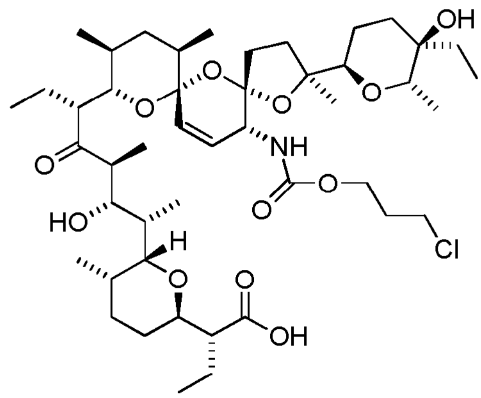

FLC-00530-1

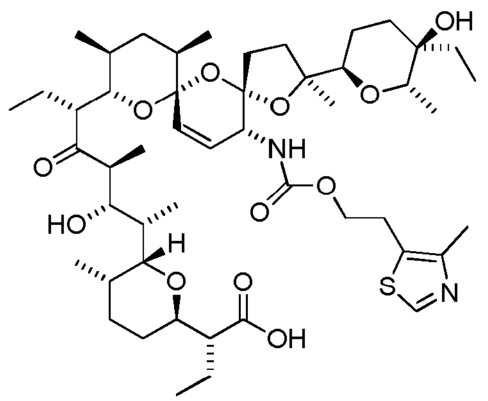

FLC-00547-1

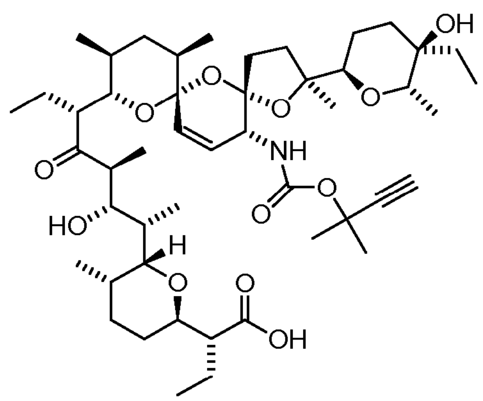

FLC-00552-1

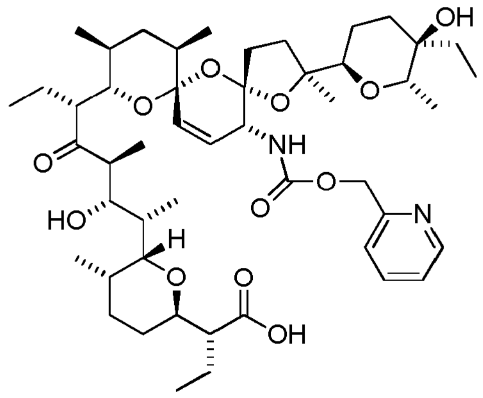

FLC-00571-1

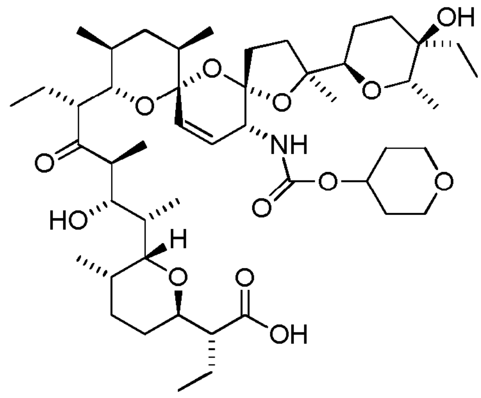

FLC-00580-1

for X =

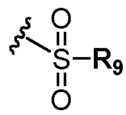

:

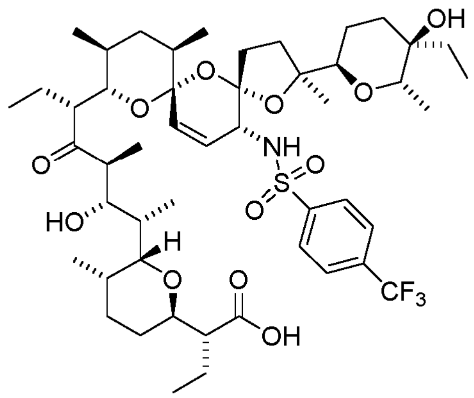

FLC-00468-1

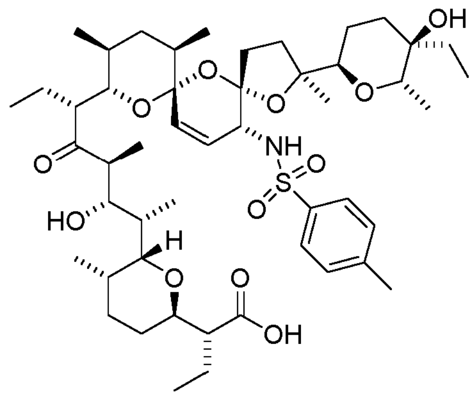

FLC-00502-1

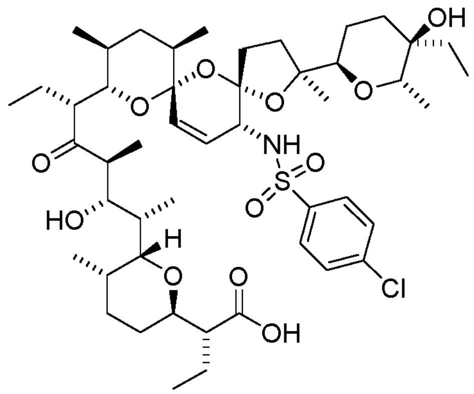

FLC-00503-1

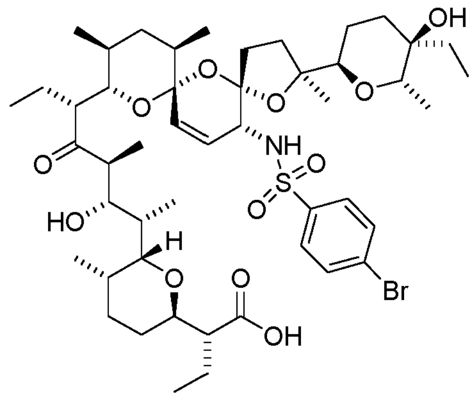

FLC-00504-1

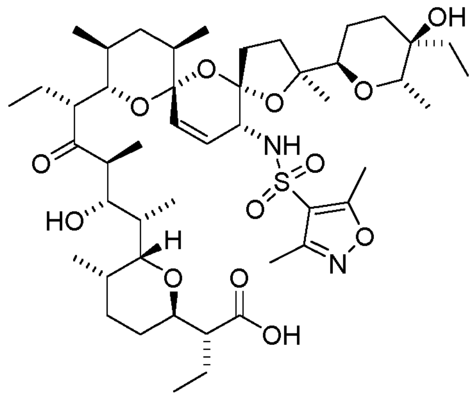

FLC-00506-1

for X =

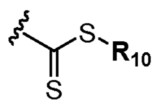

:

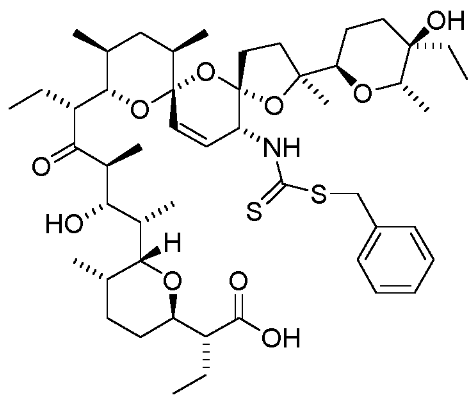

FLC-00474-1

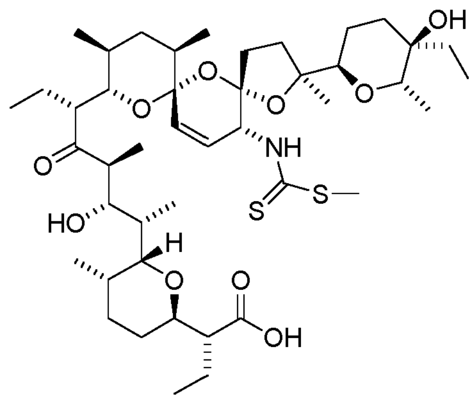

FLC-00475-1

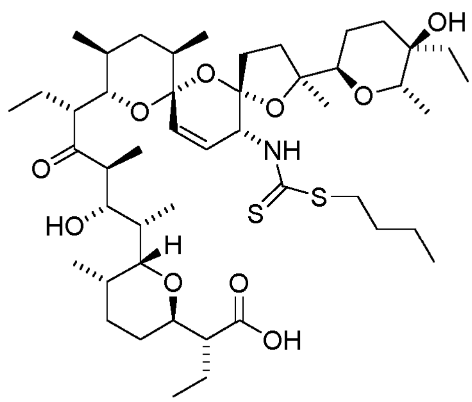

FLC-00476-1

for X =

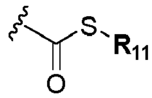

:

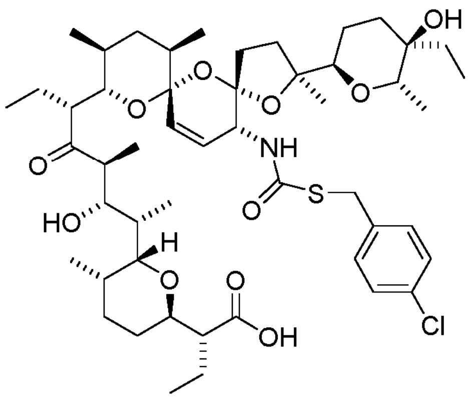

FLC-00553-1

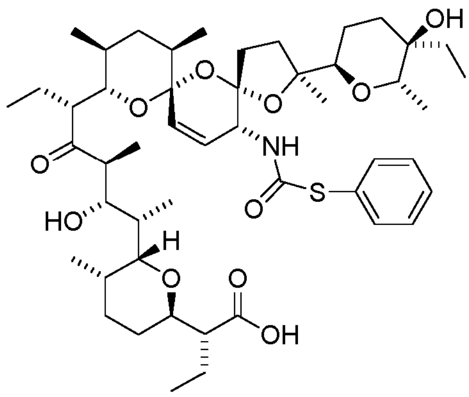

FLC-00554-1

or salts of the compounds listed above.

Of the compounds listed above, the most preferred are as follows:

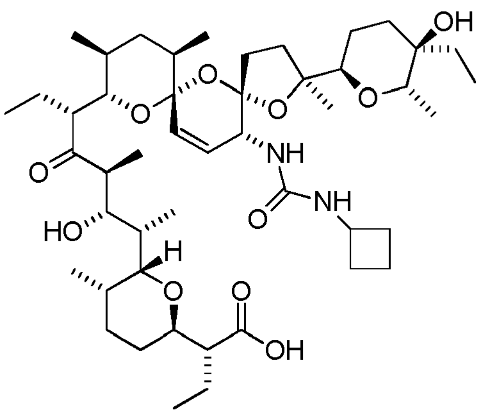

FLC-00341-1

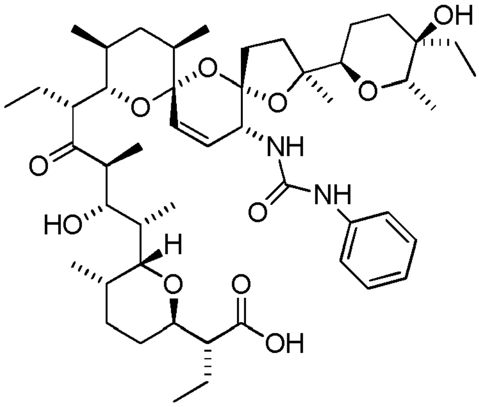

FLC-00374-1

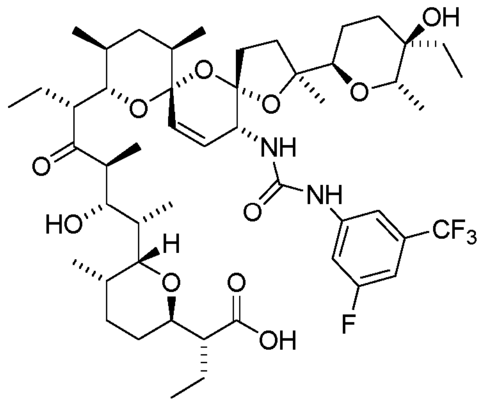

FLC-00427-1

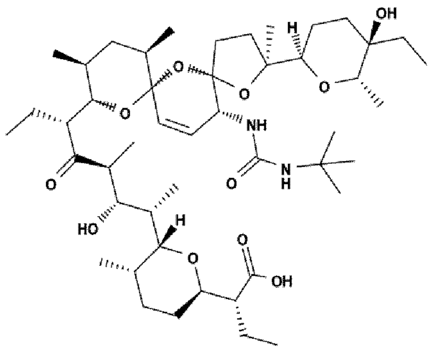

FLC-00593-1

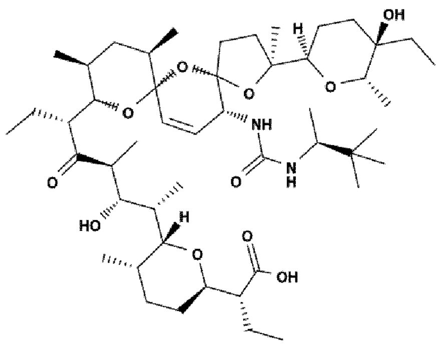

FLC-00606-1

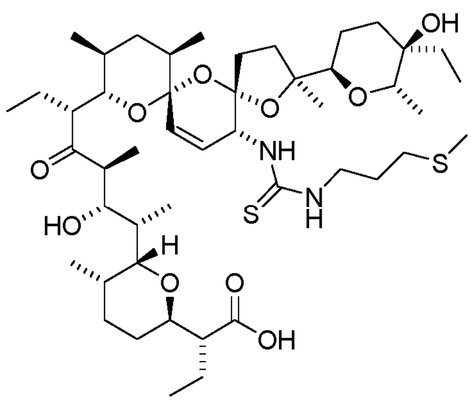

FLC-00345-1

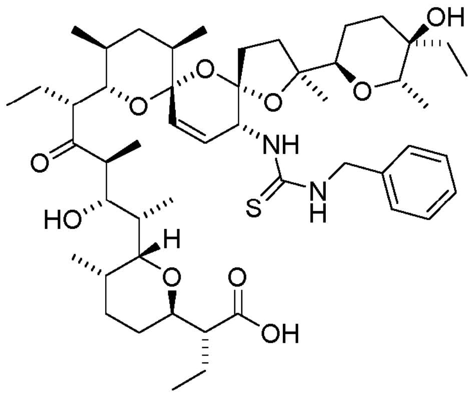

FLC-00346-1

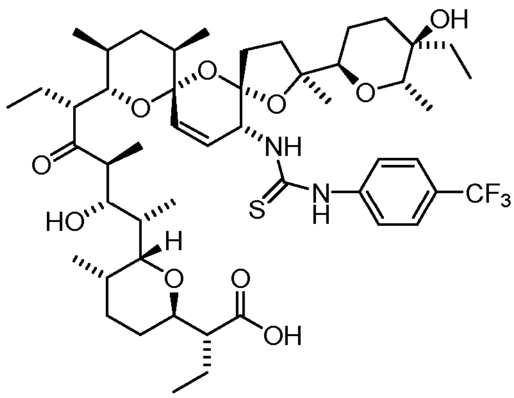

FLC-00347-1

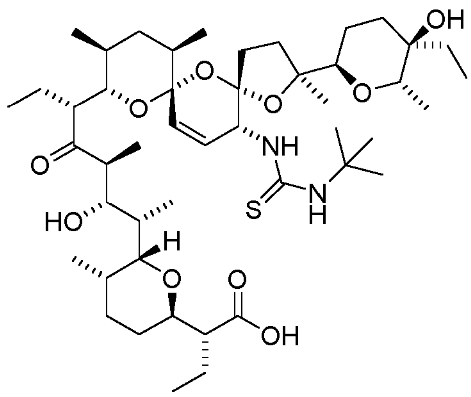

FLC-00467-1

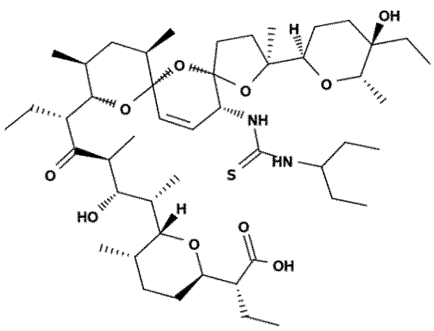

FLC-00771-1

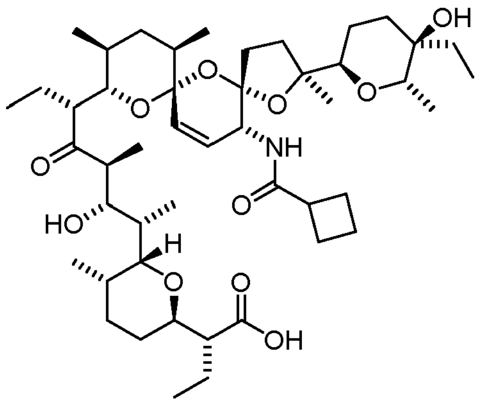

FLC-00463-1

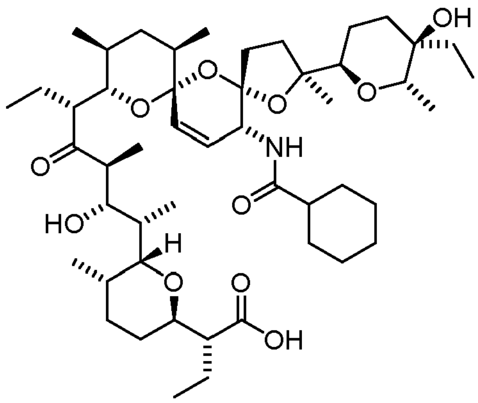

FLC-00464-1

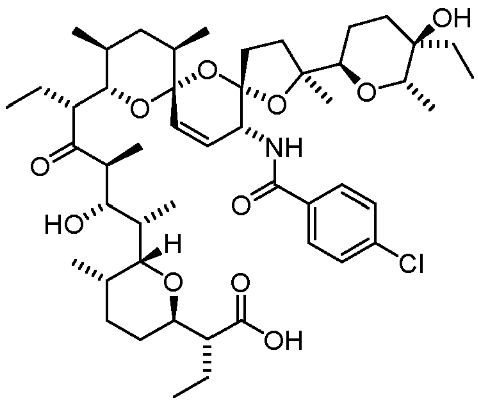

FLC-00469-1

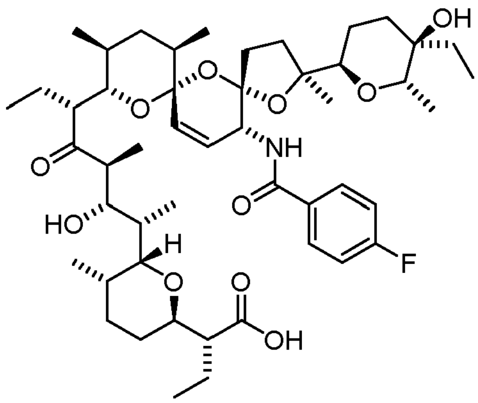

FLC-00470-1

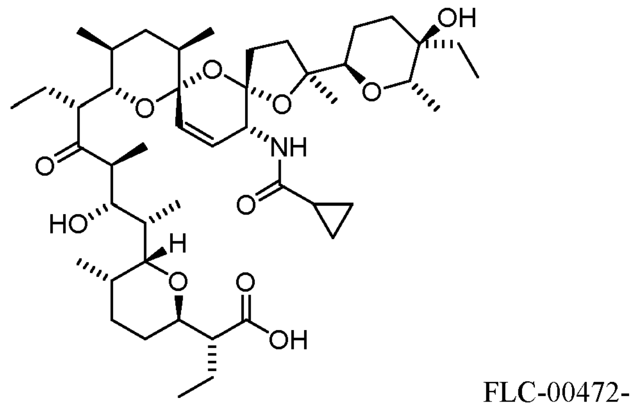

FLC-00472-1

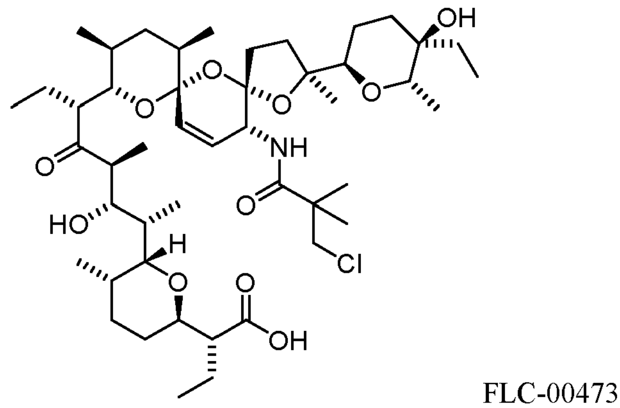

FLC-00473-1

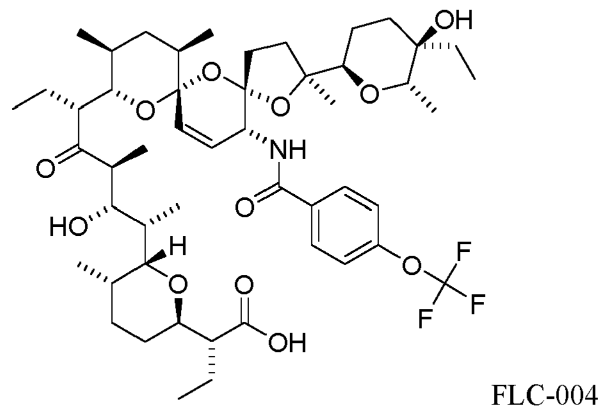

FLC-00486-1

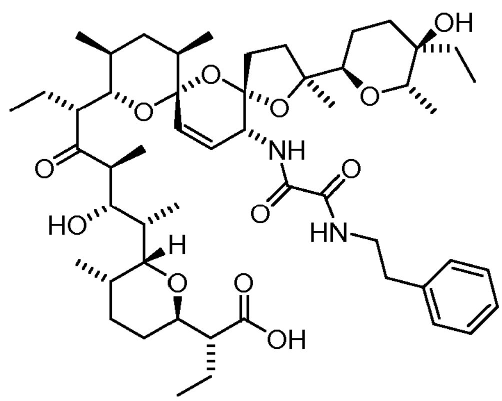

FLC-00457-1

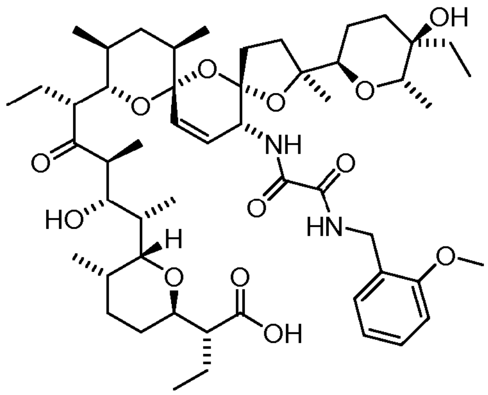

FLC-00491-1

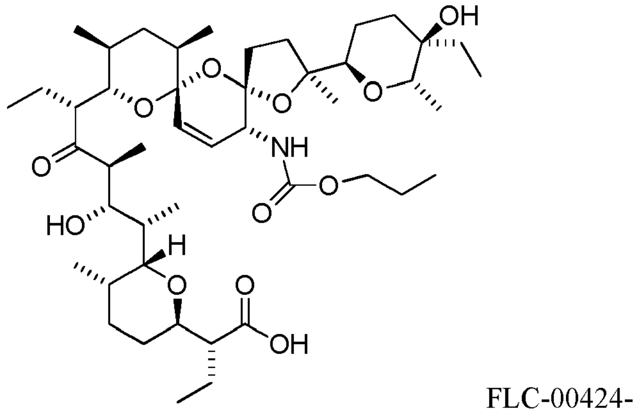

FLC-00424-1

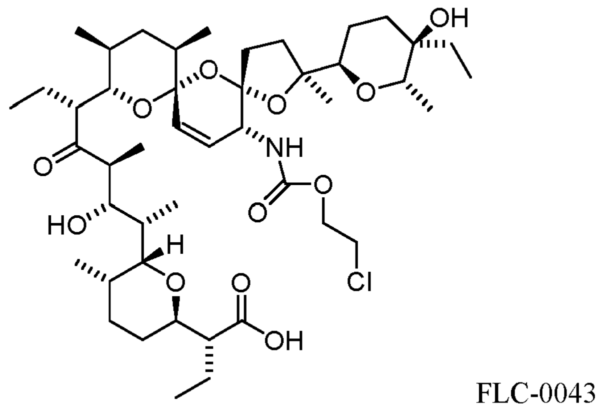

FLC-00436-1

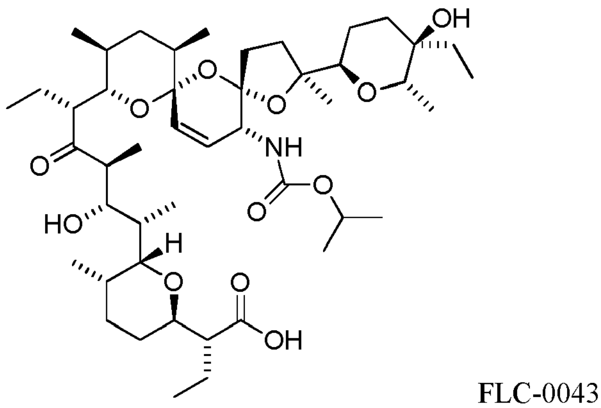

FLC-00439-1

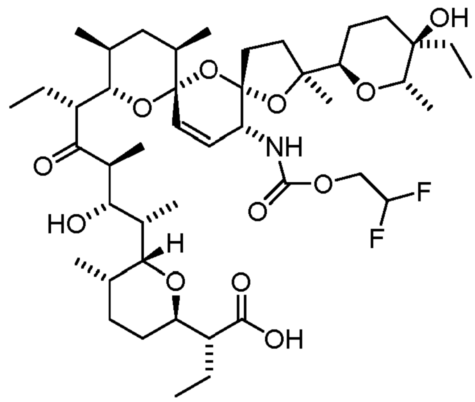

FLC-00446-1

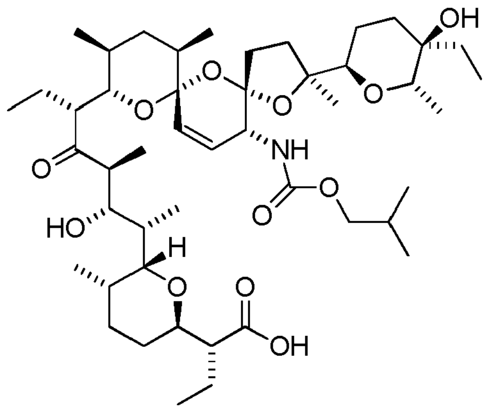

FLC-00466-1

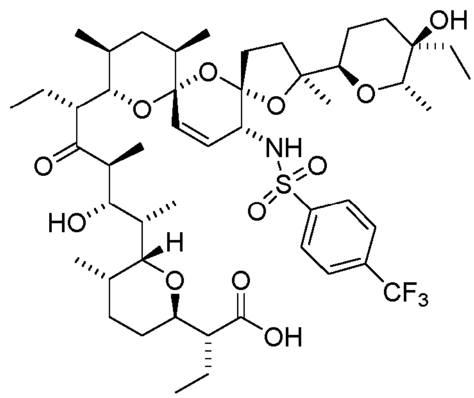

FLC-00468-1

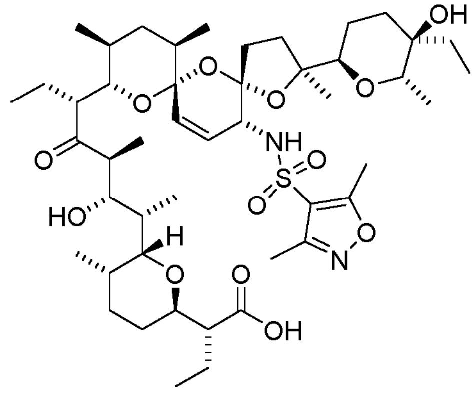

FLC-00506-1

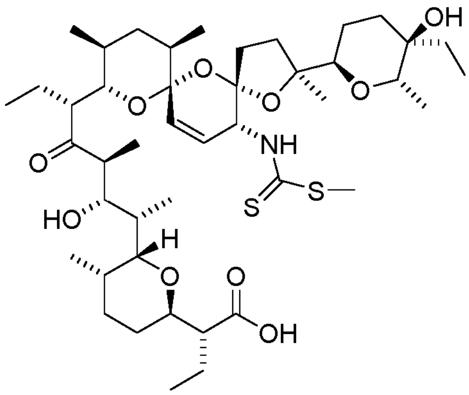

FLC-00475-1

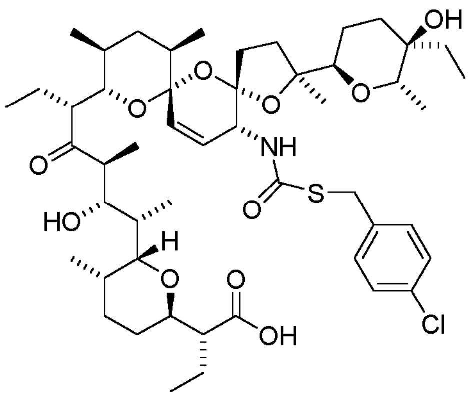

FLC-00553-1

or salts of the compounds listed above.

The invention also relates to a pharmaceutical composition comprising any of the compounds defined above or combinations thereof and at least one pharmaceutically acceptable excipient.

The invention also relates to a method for preparing an intermediate product, C20 ketosalinomycin with the formula FLC-00099 or a salt thereof:

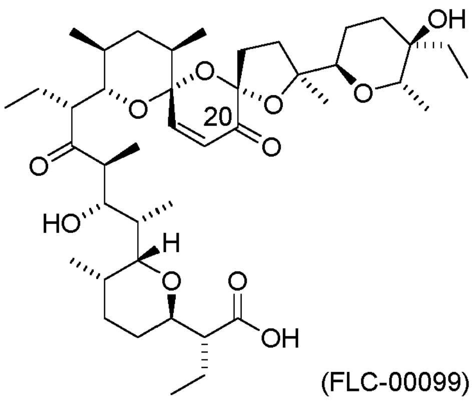

(FLC-00099)

for obtaining the compounds as above, comprising selective oxidation of the C20-hydroxy group of salinomycin with the formula FLC-00001:

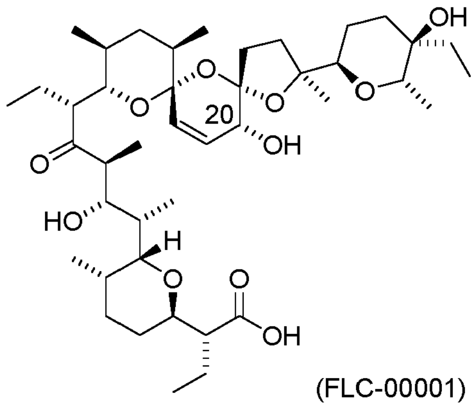

(FLC-00001)

or salts thereof, wherein the oxidising agent is selected from the group consisting of: pyridinium or pyrimidinium salt or derivatives thereof, chlorochromic(VI) or dichromic(VI) acid, pyridinium chlorochromate, pyridinium dichromate, chromium(VI) trioxide $CrO_3$, preferably pyridinium dichromate.

The invention also relates to a method for preparing an intermediate, C20-aminosalinomycin with the formula FLC-00105 or a salt thereof:

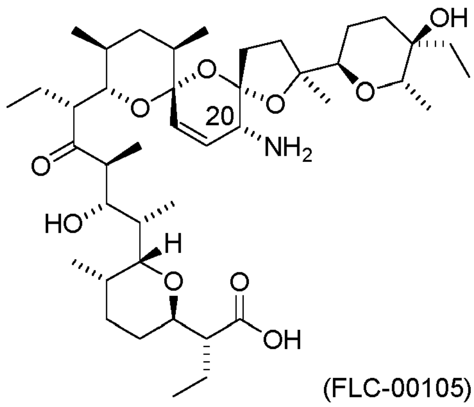

(FLC-00105)

for obtaining the compounds defined above, consisting in stereoselective reductive amination of C20-ketosalinomycin with the formula FLC-00099 or a salt thereof:

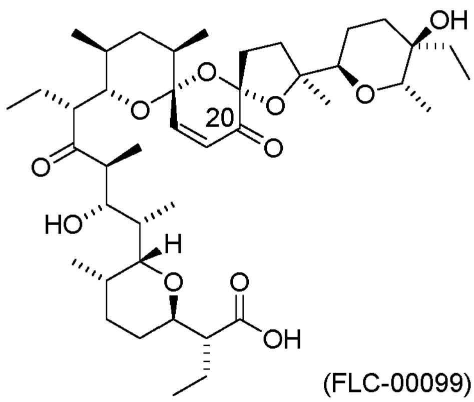

(FLC-00099)

obtained by the method as above, wherein the amine agent used is an alcoholic solution of ammonia and ammonium acetate to obtain *in situ* an imine derivative which is reduced with alkali metal borohydride of sodium, potassium, lithium, or derivatives thereof, such as cyanoborohydride, triacetoxyborohydride, preferably sodium cyanoborohydride.

The invention also relates to a method for preparing an intermediate C20-aminosalinomycin with the formula FLC-00105 or a salt thereof:

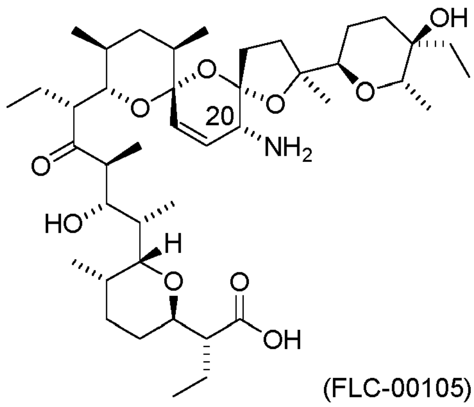

(FLC-00105)

for producing the compounds defined above, comprising the following steps:

a. selective oxidation of the C20-hydroxy group of salinomycin with the formula FLC-00001:

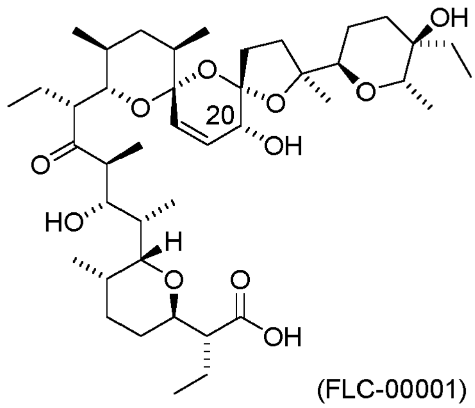

(FLC-00001)

conducted as specified above,

b. stereoselective reductive amination of the resulting C20-ketosalinomycin with the formula FLC-00099:

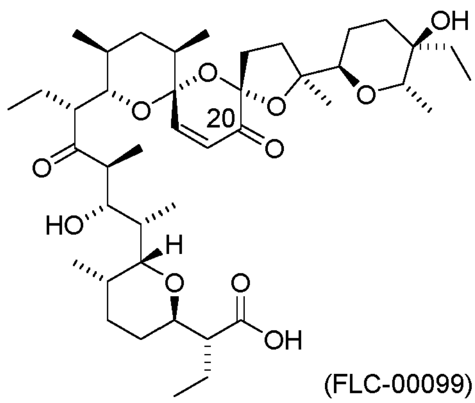

(FLC-00099)

conducted as specified above.

The invention relates to a method for preparing C20-*N*-modified salinomycin derivatives with the general formula (2):

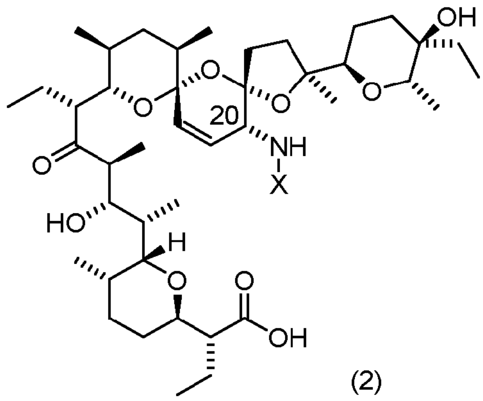

(2)

having the structures as defined above, by reacting C20-aminosalinomycin with the formula FLC-00105 prepared according to the process described above:

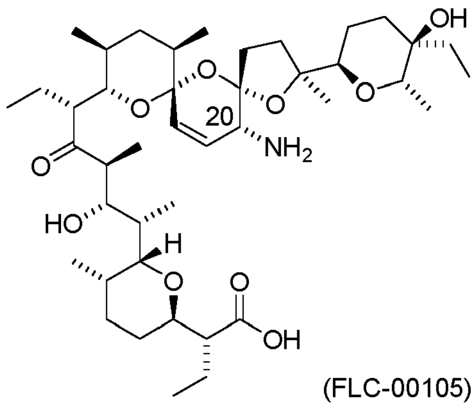

(FLC-00105)

or a salt thereof,
and:

an isocyanate with the general formula (11) to obtain C20-aminosalinomycin ureas

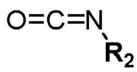

(11),

or

an isothiocyanate with the general formula (12) to obtain C20-aminosalinomycin thioureas

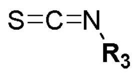

(12),

or

acid chloride with the general formula (13) to obtain C20-aminosalinomycin amides

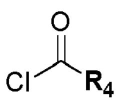

(13),

or

carboxylic acid with the general formula (14) to obtain C20-aminosalinomycin amides

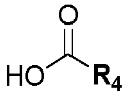 (14),

or

chloroformate with the general formula (15) to obtain C20-aminosalinomycin carbamates

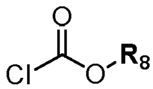 (15),

or

*p*-nitrophenyl carbonate with the general formula (16) to obtain C20-aminosalinomycin carbamates

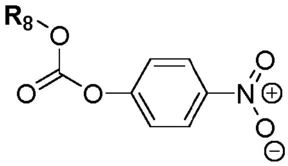 (16),

or

imidazole 1-carboxylate with the general formula (17) to obtain C20-aminosalinomycin carbamates

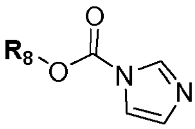 (17),

or

sulphonium chloride with the general formula (18) to obtain C20-aminosalinomycin sulphonamides

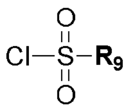 (18),

or

carbon disulphide $CS_2$ and a halide with the general formula $R_{10}$-Y (19), wherein Y = Br or I to obtain C20-aminosalinomycin dithiocarbamates,
or

4-ethyl-2,3-dioxo-1-piperazinecarbonyl chloride,
or

3-(methylsulphonyl)-2-oxoimidazolidine-1-carbonyl chloride,
or

glutaric anhydride, to obtain C20-aminosalinomycin monoglutaramide, or

di-tert-butyl pyrocarbonate to obtain C20-aminosalinomycin tert-butyl carbamate, or

2-bromoacetyl bromide to obtain C20-aminosalinomycin 2-bromoacetamide.

A compound constituting C20-aminosalinomycin p-nitrophenyl urethane with the structure (FLC-00604):

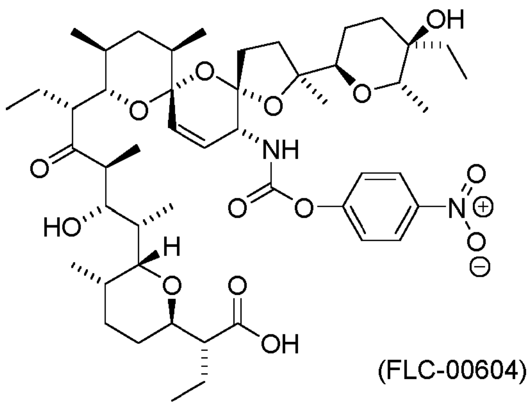

(FLC-00604)

,

is an intermediate for the preparation of C20-*N*-modified salinomycin derivatives with the general formula (2):

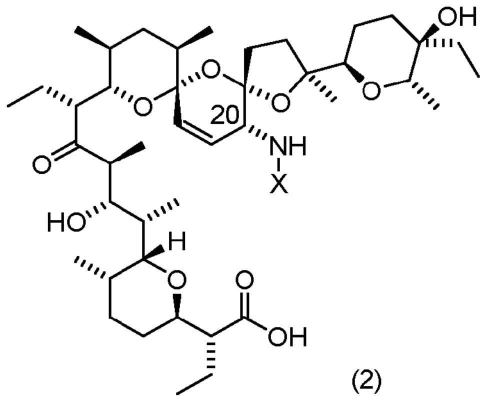

(2)

wherein:

X =

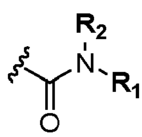

,

then the resulted compounds are *N'*-substituted and *N'*,*N'*-disubstituted C20-aminosalinomycin ureas with the general formula (3),

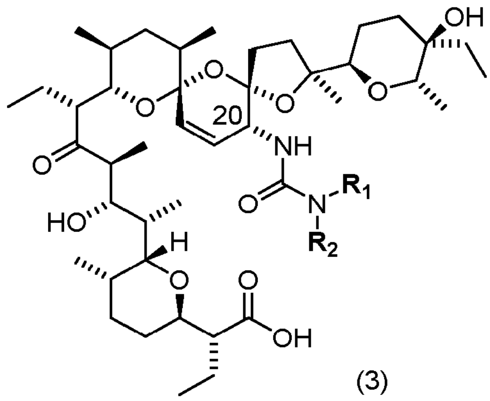

(3)

or

X =

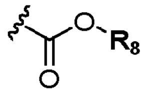

,

then the resulted compounds are C20-aminosalinomycin urethanes (carbamates) with the general formula (7),

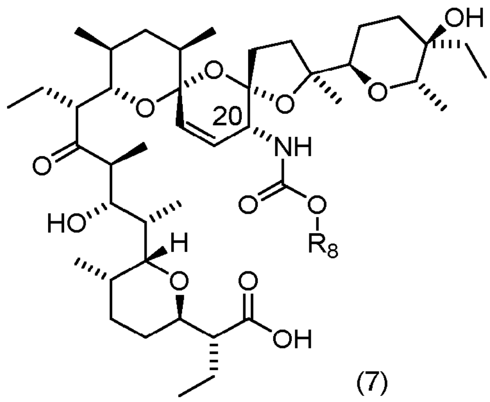

(7)

or

X =

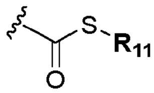

,

then the resulted compounds are S-substituted C20-aminosalinomycin thiocarbamates with the general formula (10).

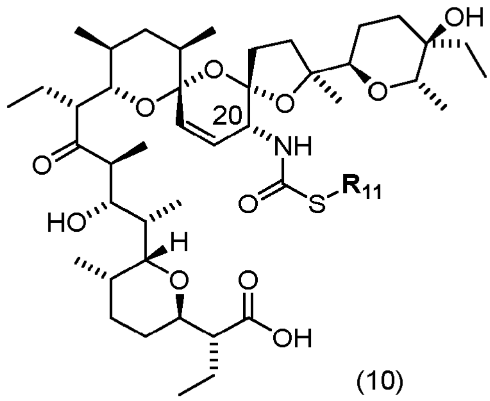

(10)

C20-aminosalinomycin oxalate derivative with the structure (FLC-00373):

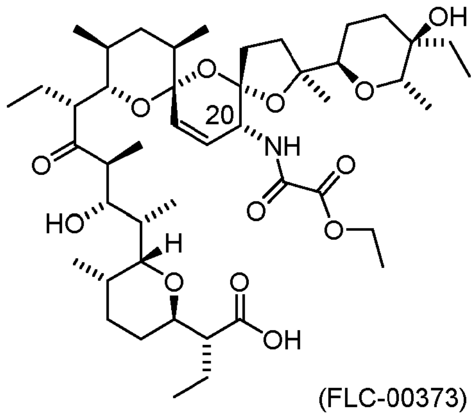

(FLC-00373) ,

or a salt thereof,
is an intermediate for the preparation of C20-*N*-modified salinomycin derivatives with the general formula (2):

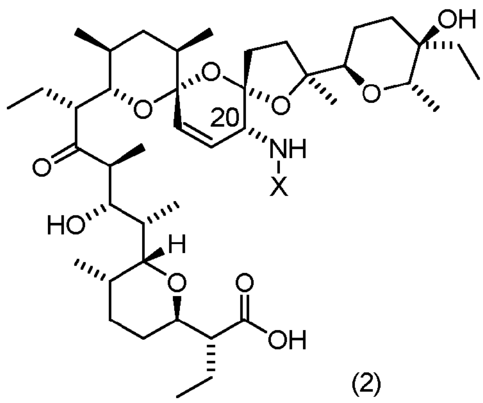

(2)

wherein:

X =

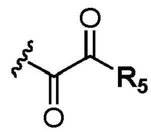

,

then resulted compounds are C20-*N*-modified salinomycin derivatives with the general formula (6),

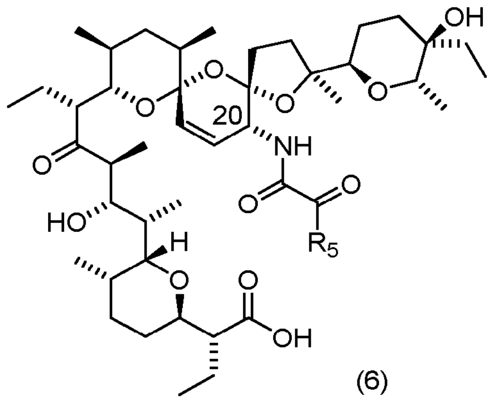

(6)

wherein $R_5$ is $-NH_2$ or $-NH-R_7$;

The invention also relates to the compounds defined above for use as a medicament. Preferably, the use is as an anti-cancer agent.

The compounds defined above are characterised in that they are for use in conditions selected from the group consisting of leukaemia, including without limitation acute myeloid leukaemia, chronic myeloid leukaemia, acute lymphoblastic leukaemia, multiple myeloma; non-small cell lung cancer, including without limitation lung epithelial cell carcinoma, lung adenocarcinoma, human lung squamous cell cancer; large intestine (colon) cancer, including without limitation large intestine adenocarcinoma, colon epithelial cell cancer; tumour of the central nervous system, including without limitation brain tumours such as glioma; melanoma, including without limitation malignant melanoma, epithelial melanoma, non-epithelial melanoma; ovarian cancer, including without limitation epithelial ovarian cancer, ovarian cystadenocarcinoma; kidney cancer, including without limitation renal cell carcinoma; prostate cancer, including without limitation prostate adenocarcinoma; breast cancer, including without limitation adenocarcinoma of the breast, inflammatory breast cancer, metastatic adenocarcinoma; stomach cancer; pancreatic cancer; sarcoma and uterine body cancer, as well as its drug-resistant variant; cervical cancer; bladder cancer.

Preferably, the conditions are selected from the group consisting of ovarian cancer, melanoma, pancreatic cancer, lung cancer, liver cancer, gastric cancer, malignant primitive neuroectodermal tumour, biphenotypic leukaemia or myeloid leukaemia.

## DETAILED DESCRIPTION OF THE INVENTION

Preferred embodiments of the invention are disclosed in the following detailed description and in the accompanying claims. Various embodiments of the invention are defined herein in greater detail. Any of the aspects so defined may be combined with any other aspect or aspects, unless expressly stated otherwise. In particular, any of the features indicated as preferred or preferable may be combined with any other feature or features indicated as preferred or preferable.

Reference throughout the description to an "embodiment" or an "aspect" of the invention is to be understood that a particular feature, structure or characteristic described in connection with such an embodiment is comprised in at least one embodiment of the present invention. Thus, instances of the terms "embodiment" or "aspect" in various places of this description may or may not refer to the same embodiment. Further, particular features, structures or characteristics may be combined in any suitable manner, as would be apparent to a person skilled in the art of this disclosure, in one or more embodiments. Moreover, although some aspects of the invention described herein include some features, not differing from those included in other embodiments, combinations of features from different embodiments are intended to be within the scope of the invention and they form different embodiments, as would be apparent to those skilled in the art. Any of the embodiments claimed may be used in any combination.

The invention relates to compounds constituting C20-*N*-modified salinomycin derivatives with the general formula (2):

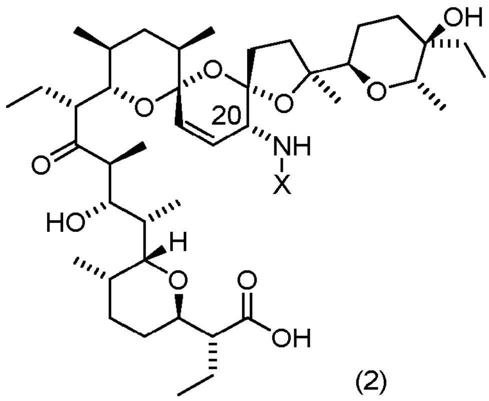

(2)

, wherein:

- X is respectively:

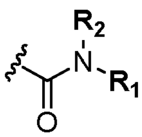 , 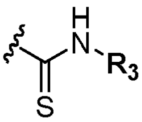 , 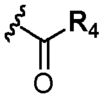 , 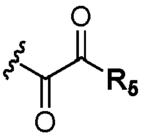 , 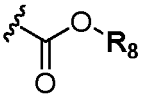 , 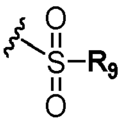 , 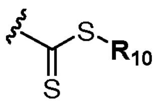

or

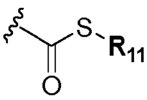 .

The compounds according to the invention have an absolute configuration R on the asymmetric C20 carbon. The absolute configuration of the novel derivatives on the asymmetric C20 carbon is the same as the absolute configuration on the asymmetric C20 carbon of the starting salinomycin. Therefore, the preparation of the novel salinomycin derivatives according to the invention provides a retention of the configuration on the asymmetric C20 carbon of salinomycin. The X moiety in the inventive compounds may be:

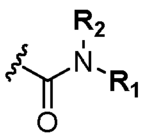 , 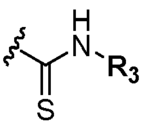 , 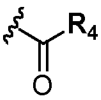 , 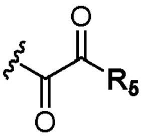 , 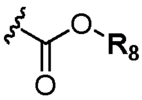 , 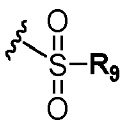 , 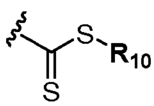 or 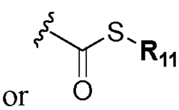

For all types of novel salinomycin derivatives indicated herein, substituent X and further substituents $R_1$ - $R_{11}$ are defined in detail without limitation hereinabove and in the claims. It will be apparent to a person skilled in the art that other $R_1$ - $R_{11}$ substituents similar to those recited below, even to those recited as specific groups, are also within the scope of the invention.

For X as indicated above, new types of biologically active salinomycin derivatives are claimed herein. Te following abbreviated names of the derivatives are used for the purposes of the specification:

| X | Shortened generic name of the derivatives |
|---|---|
| 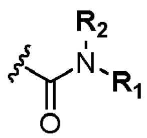 | C20-amino SAL ureas,<br>C20-amino SAL urea derivatives,<br>C20-*N*-urea salinomycin derivatives |

(continued)

| X | Shortened generic name of the derivatives |
|---|---|
| 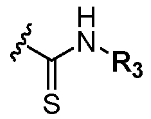 | C20-amino SAL thioureas,<br>C20-amino SAL thiourea derivatives,<br>C20-*N*-thiourea salinomycin derivatives |
| 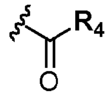 | C20-amino SAL amides,<br>C20-amino SAL amide derivatives,<br>C20-*N*-amide salinomycin derivatives |
| 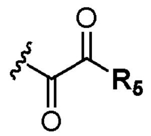 | C20-amino SAL oxalates,<br>C20-amino SAL oxalate derivatives,<br>C20-*N*-oxalate salinomycin derivatives |
| 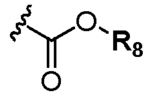 | C20-amino SAL urethanes,<br>C20-amino SAL urethane (carbamate) derivatives,<br>C20-*N*-urethane (carbamate) salinomycin derivatives |
| 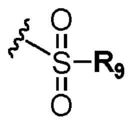 | C20-amino SAL sulphonamides<br>C20-amino SAL sulphonamide derivatives,<br>C20-N-sulphonamide derivatives of salinomycin |
| 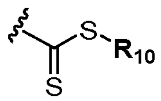 | C20-amino SAL dithiocarbamates,<br>C20-amino SAL dithiocarbamate derivatives,<br>C20-*N*-dithiocarbamate salinomycin derivatives |
| 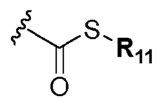 | S-substituted C20-amino SAL thiocarbamates,<br>S-substituted C20-N-thiocarbamate salinomycin derivatives |

Preferably, the compounds according to the invention may also be in the form of salts. The general formula of the salts of salinomycin derivatives according to the invention is illustrated by the general formula (2a):

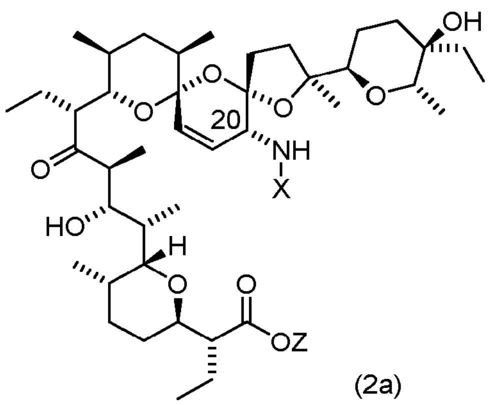

(2a)

Preferred salts of the compounds according to the invention are sodium, potassium, lithium and cesium salt. Z in the general formula (2a) is then respectively: Na, K, Li, Cs. Preferably, Z is Na. The scope of the invention also includes salts with divalent metals, such as e.g. magnesium.

A person skilled in the art will know how to select and adjust the conditions for obtaining an acid derivative or a desired salt thereof. Salinomycin and derivatives thereof, when extracted with an aqueous solution of acid, such as sulphuric(VI) acid, hydrochloric acid, acetic acid, citric acid yields the acid form (Z=H). Extraction of salinomycin and its derivatives with an aqueous solution of a suitable inorganic salt, in turn, yields compounds in the form of salts (Z=Na, K, Li, Cs). Preferably, in order to obtain the sodium, potassium or lithium salt of the inventive compound, extraction with sodium, potassium or lithium carbonate is used.

The invention also relates to a composition comprising the compound according to the invention and at least one pharmaceutically acceptable excipient. Pharmaceutically acceptable excipients are known in the art and are exemplified in Remington: The Science and Practice of Pharmacy 1995, ed. by E. W. Martin, Mack Publishing Company, 19 Edition, Easton, Pa. Preferably, the composition comprises one compound according to the invention and at least one pharmaceutically acceptable excipient.

List of abbreviations used:

AcOH - acetic acid
AcOEt - ethylacetate
$Boc_2O$ - di-*tert*-butyl pyrocarbonate
C20-amino SAL - C20-aminosalinomycin
C20-keto SAL - C20-ketosalinomycin
CDI - 1,1'-carbonyldiimidazole
$CS_2$ - carbon disulphide
DBU - 1,8-Diazabicyclo(5.4.0)undec-7-ene
DCC - *N*,*N*'-dicyclohexylcarbodiimide
DCE - dichloroethane
DCM - dichloromethane
DIC - *N*,*N*'-diisopropylcarbodiimide
DIPEA - *N*,*N*-diisopropyl ethylamine
DMA - dimethylacetamide
DMAP - 4-dimethylaminopyridine
DMF - *N*,*N*-dimethylformamide
EDCI - 1-ethyl-3-(3-dimethylaminopropyl)carbodiimide
ELS - Evaporative Light Scattering detector
ESI-MS - Electrospray Ionisation Mass Spectrometry
eq. - molar equivalent
HATU - 2-(7-aza-1H-benzotriazol-1-yl)-1,1,3,3-tetramethyluronium hexafluorophosphate
i-PrOH - isopropanol
LC-MS - Liquid Chromatography Mass Spectrometry
MeCN - acetonitrile
MeOH - methanol
$NaBH_3CN$ - sodium cyanoborohydride
NMP - *N*-methylpyrrolidone
NMR - Nuclear Magnetic Resonance
PCC - pyridinium chlorochromate
PDC - pyridinium dichromate
p-TsOH - *para*-toluenesulfonyl acid
Py - pyridine
RT - room temperature
SAL - denotes salinomycin residue / fragment
TBTU - O-(benzotriazol-1-yl)-*N*,*N*,*N*',*N*'-tetramethyluronium tetrafluoroborate
TEA - triethylamine
THF - tetrahydrofuran
TLC - thin layer chromatography

The invention relates to a method for preparing compounds constituting the invention, which consists in reactions according to the diagrams shown below.

It was observed that intermediates for the synthesis of C20-*N*-modified salinomycin derivatives are also innovative, since the synthesis where they are used is advantageous in many ways.

One of the intermediates is C20-amino SAL with the formula FLC-00105, as well as the intermediate compound C20-keto SAL with the formula FLC-00099, which is an intermediate for the synthesis of C20-amino SAL.

Method for preparing C20-ketosalinomycin with the formula FLC-00099

The invention relates to a C20-keto SAL intermediate with the formula FLC-00099 or a salt thereof. Said compound is prepared according to Diagram 1.

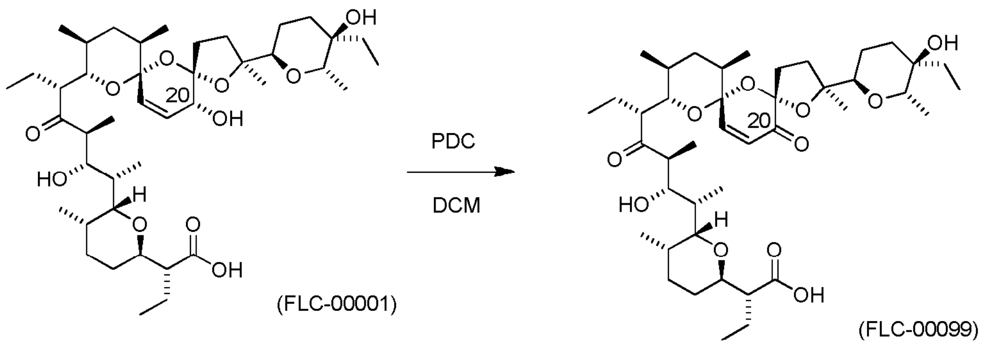


(FLC-00001)

(FLC-00099)

Diagram 1.

The method optionally comprises a step of transforming the resulting compound in acid form into a salt thereof. A person skilled in the art will know how to select the reaction conditions for the conversion of a salinomycin derivative in acid form into a salt thereof.

The method for preparing FLC-00099 comprises selective oxidation of salinomycin C20-hydroxy group with the formula FLC-00001 in a chloroaliphatic solvent such as DCM, DCE, chloroform, preferably in DCM, or in a polar aprotic solvent - simple nitrile (MeCN), THF, simple amide (NMP, DMA), sulfolane, or in an aromatic solvent (benzene, toluene, xylene, pyridine) or in a mixture of solvents, preferably at room temperature. The oxidising agent used is a pyridinium or pyrimidinium salt (or derivatives thereof) of chlorochromic(VI) or dichromic(VI) acid, pyridinium chlorochromate (PCC) or pyridinium dichromate (PDC) or chromium(VI) trioxide $CrO_3$, preferably pyridinium dichromate (PDC). This method differs significantly from the method described in literature that uses activated manganese(IV) oxide [according to: EP3191493 lub Mai et al., Nature Chemistry, 9, 2017, 1025-1033], was developed for the purposes of the invention. The developed method has allowed for a significant reduction in the time to prepare C20-keto SAL, from several days to as little as a few hours. Moreover, it eliminates the use of manganese(IV) oxide used in the prior art method, whose efficiency depends on its crystalline form and on the reagent supplier. This involved various synthetic problems, with the reaction sometimes failing to occur or occurring with low efficiency.

The conversion rate is controlled by TLC and/or LC-MS. The post-reaction mixture is purified by way of column chromatography, preferably combined with an ELS detector, using a column packed with silica and a mixture of organic solvents. The fractions containing the desired product are combined and evaporated under reduced pressure. The residue is dissolved in a chloroaliphatic solvent, preferably in DCM or in chloroform or in AcOEt, followed by washing it with an aqueous solution of a suitable salt (carbonate or bicarbonate, such as sodium or potassium or lithium) or with an aqueous solution of acid, such as sulphuric(VI) ($H_2SO_4$) or hydrochloric acid (HCl). The organic layer is evaporated to dryness under reduced pressure and then evaporated several times with *n*-pentane or MeCN or lyophilised, e.g. with dioxane. TLC, LC-MS, $^{1}$H NMR and $^{13}$C NMR were the analytical methods used to ascertain that the proper compound has been obtained.

Method for preparing C20-aminosalinomycin with the formula FLC-00105

The invention also relates to a C20-amino SAL intermediate with the formula FLC-00105 or a salt thereof. It is prepared according to Diagram 2 from C20-keto SAL with the formula FLC-00099:

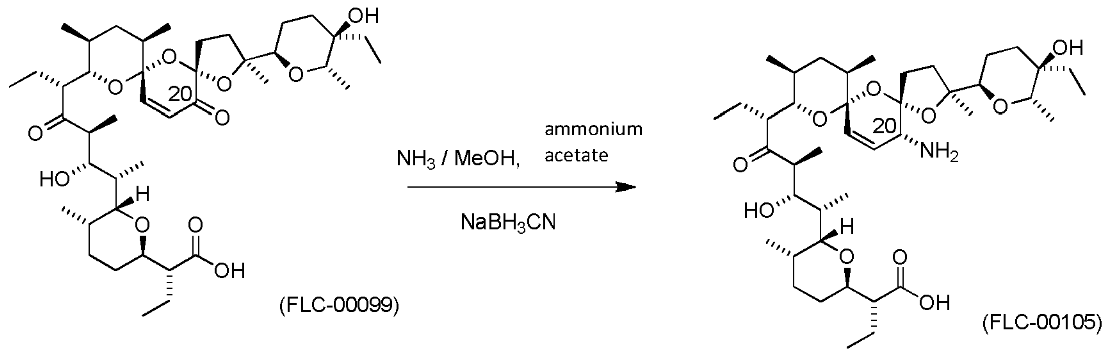


(FLC-00099) (FLC-00105)

Diagram 2.

The method for preparing the intermediate comprises stereoselective reductive amination of C20-keto SAL with the formula FLC-00099, or salts thereof, where the aminating agent used is an alcoholic solution of ammonia and ammonium acetate, which results in the *in situ* formation of an imine derivative which is reduced with alkali metal borohydride (sodium, potassium, lithium) or derivatives thereof, cyanoborohydride, triacetoxyborohydride, preferably sodium cyanoborohydride. C20-ketosalinomycin with the formula FLC-00099 is prepared as above according to Diagram 1.

Literature reports regarding the preparation of C20-aminosalinomycin with the formula FLC-00105 [EP3191493 or Mai et al., Nature Chemistry, 9, 2017, 1025-1033] are based on a typical approach where reduction with cerium salts is used to retain the configuration on the carbon being the reaction centre. Surprisingly, when developing present invention, it was found that it is not necessary to use cerium salts in order to retain the stereochemistry on C20 carbon and allows to obtain C20-amino SAL while retaining the stereochemistry of the starting salinomycin. The inventive reaction is carried out in a polar solvent such as methanol, ethanol, propanol, formamide, DMA, NMP, DMF, AcOH or a mixture thereof, preferably methanol, at room temperature or at reflux of the solvent, preferably at reflux of the solvent.

The conversion rate is controlled by TLC and/or LC-MS. The post-reaction mixture is purified by way of column chromatography, preferably combined with an ELS detector, using a column packed with silica and a mixture of organic solvents. The fractions containing the desired product are combined and evaporated under reduced pressure. The residue is dissolved in a chloroaliphatic solvent, preferably in DCM or in chloroform or in AcOEt, followed by washing it with an aqueous solution of a suitable salt (carbonate or bicarbonate, such as sodium or potassium or lithium) or with an aqueous solution of acid, such as sulphuric(VI) ($H_2SO_4$) or hydrochloric acid (HCl). The organic layer is evaporated to dryness under reduced pressure and then evaporated several times with n-pentane or MeCN or lyophilised, e.g. with dioxane.

Conversion of the compound with the formula FLC-00099 into the compound with the formula FLC-00105 as broadly illustrated in prior art has been enhanced and modified for the purposes of the invention. The modified procedure for preparing the compound with the formula FLC-00105 comprises the use of an alcoholic solution of ammonia and ammonium acetate, where the ammonium acetate has two functions, namely it provides ammonia for the reaction with the acetate form acting as a buffer, so that it is not necessary to generate the buffer by mixing acetic acid with the amine component (here, ammonia solution), as is the case according to the procedure described in the literature: EP3191493 or Mai et al., Nature Chemistry, 9, 2017, 1025-1033. Moreover, the reaction was carried out at reflux of the solvent rather than at room temperature (which greatly accelerates the reaction rate getting it down to several hours), and the cerium salt $CeCl_3*7H_2O$, which was supposed to allow for retaining appropriate stereochemistry [according to the general procedure described in: EP3191493 or Mai et al., Nature Chemistry, 9, 2017, 1025-1033] was eliminated. The amount of cerium salt required for the reaction according to the procedure described in the literature is as much as 1 eq, so it provides both an economic and an environmental advantage to be able not to use it in this conversion. Besides, the efficiency of the novel procedure developed for reductive amination is in the range of 65 to 75%, while the data provided in the literature regarding the prior art procedure indicate efficiencies in the range of 18 to 48% depending on the amine used. TLC, LC-MS, $^1H$ NMR, $^{13}C$ NMR and X-ray analysis were the analytical methods used to ascertain that the proper compound has been obtained. X-ray analysis (data specified in the examples section) further confirms the configuration at the C-20 carbon atom of aminosalinomycin.

The invention also relates to a method for preparing an intermediate C20-amino SAL with the formula FLC-00105 or a salt thereof, comprising two steps:

a) selective oxidation of the C20-hydroxy group of SAL with the formula FLC-00001 or a salt thereof (using the method described above in Diagram 1),

b) and stereoselective reductive amination of the resulting C20-keto SAL with the formula FLC-00099 or a salt thereof (using the method described above in Diagram 2),
according to Diagram 3:

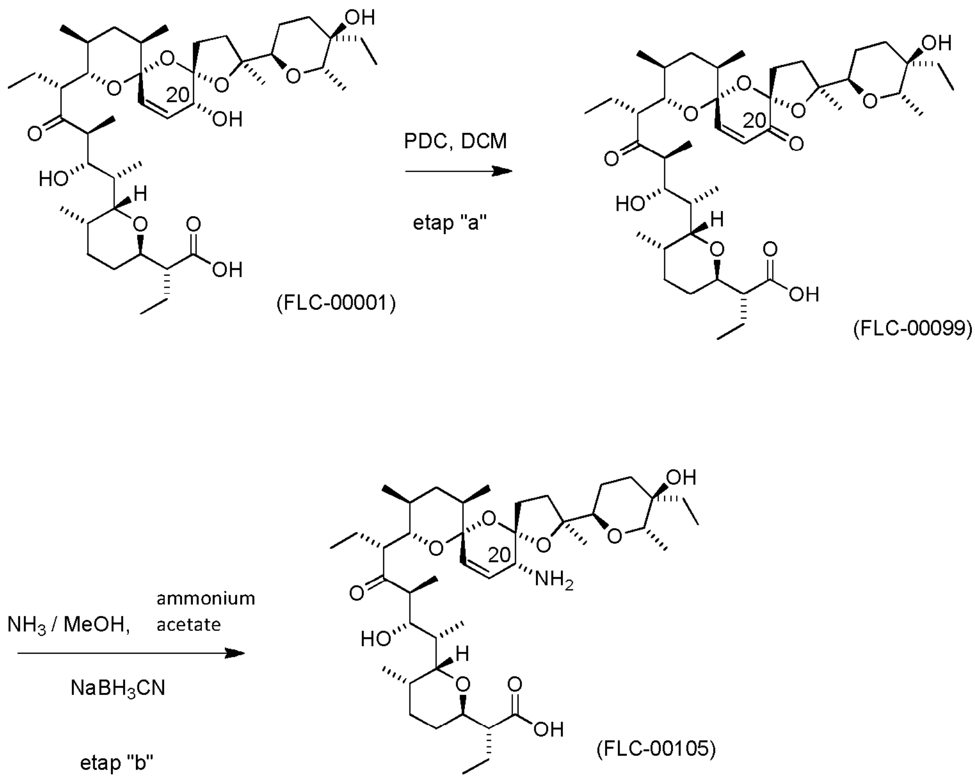


(FLC-00001)

(FLC-00099)

(FLC-00105)

Diagram 3.

This method uses the conversions described above according to Diagram 1 and Diagram 2.

The development of new methods for preparing C20-ketosalinomycin with the formula FLC-00099 and C20-amino SAL with the formula FLC-00105 or salts thereof has allowed to overcome the technical problem of providing an efficient, mild technique that eliminates problematic reactants used in known prior art methods.

The particular methods for synthesising further types of C20-*N*-modified salinomycin derivatives with the general formula (2) are described below:

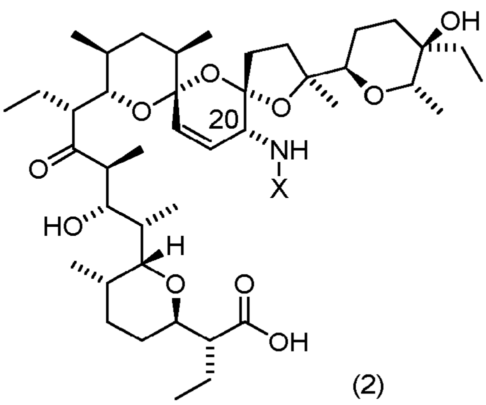

(2)

Method of preparing C20-amino SAL ureas with the general formula (3):

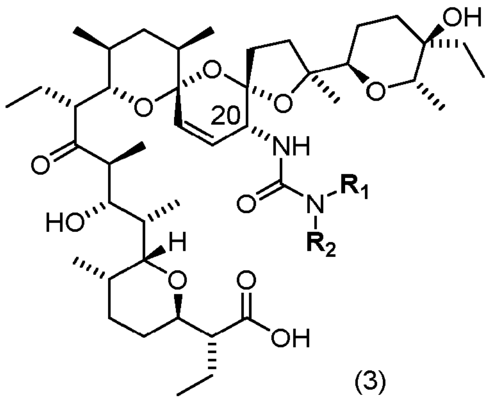

(3)

Salinomycin derivatives with the general formula (3) can be prepared in two methods. The first method involves the reaction according to Diagram 4, reacting C20-amino SAL with the formula FLC-00105 and an isocyanate with the general formula (11):

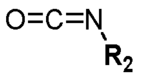

(11) ,

wherein $R_2$ is as defined above.

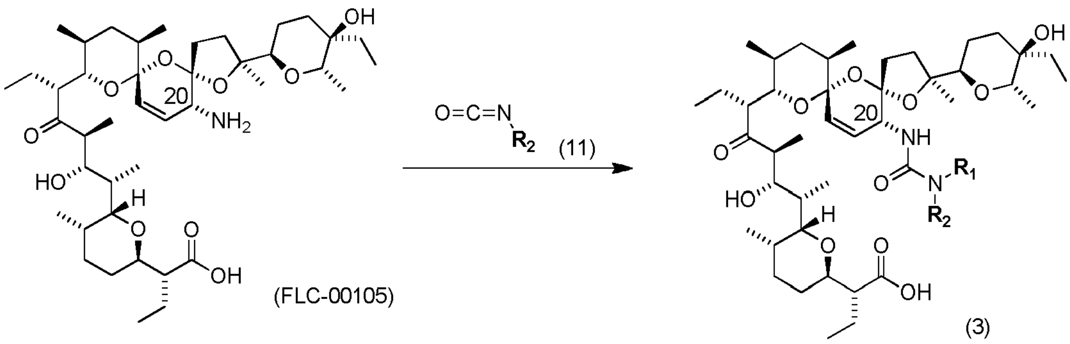

(11)

(FLC-00105)

(3)

gdzie $R_1$ = H

Diagram 4.

The method optionally comprises a step of transforming the resulting compound in acid form into a salt thereof. A person skilled in the art will know how to select the reaction conditions for the conversion of a salinomycin derivative in acid form into a salt thereof.

The reaction of C20-amino SAL with an isocyanate with the general formula (11) is carried out in a chloroaliphatic solvent such as DCM, DCE, chloroform, preferably in DCM, or in a polar aprotic solvent - simple nitrile (MeCN), THF, simple amide (NMP, DMA), DMF, or in an aromatic solvent (benzene, toluene, xylene) or in a mixture of solvents, at room temperature. Also preferably, the reaction is conducted under anhydrous conditions.

The conversion rate is controlled by TLC and/or LC-MS. The post-reaction mixture is purified by way of column chromatography, preferably combined with an ELS detector, using a column packed with silica and a mixture of organic solvents. The fractions containing the desired product are combined and evaporated under reduced pressure. The residue is dissolved in a chloroaliphatic solvent, preferably in DCM or in chloroform or in AcOEt, followed by washing it with an aqueous solution of a suitable salt (carbonate or bicarbonate, such as sodium or potassium or lithium) or with an aqueous solution of acid, such as sulphuric(VI) ($H_2SO_4$) or hydrochloric acid (HCl). The organic layer is evaporated to dryness under reduced pressure and then evaporated several times with *n*-pentane or MeCN or lyophilised, e.g. with dioxane.

When carrying out the synthesis as above, by converting C20-amino SAL with the formula FLC-00105 into the inventive compounds with the general formula (3), using isocyanates with the general formula (11), it was observed that, in order to obtain some of the derivatives, this method was found not to be viable given that respective isocyanates necessary to obtain the preferable intended derivatives were not commercially available. Also, the method described above does not

yield derivatives with the general formula (3), wherein $R_1 \neq H$ and $R_2 \neq H$, i.e. *N*',*N*'-disubstituted C20-amino SAL ureas. Normally, *N*',*N*'-disubstituted ureas are prepared by reacting the amino group with the corresponding carbamoyl chloride, but with C20-amino SAL such conversion was found to be poorly efficient or impossible to implement due to the instability of the salinomycin derivative under the conditions of said conversion. In these cases, it was found that in order to achieve the conversion into the intended final derivative, C20-amino SAL first needs to be converted into the intermediate:

- C20-amino SAL *p*-nitrophenyl urethane with the formula (FLC-00604):

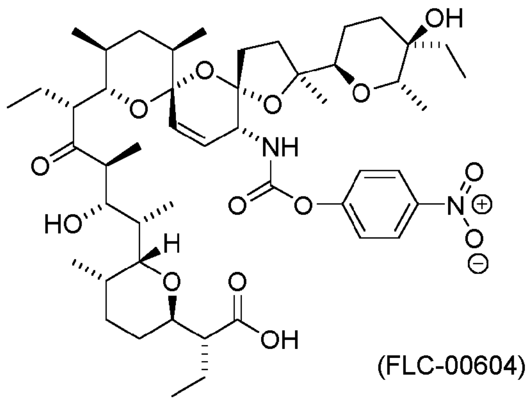

(FLC-00604)

and then convert it under mild conditions into the desired C20-*N*-urea SAL derivatives with the general formula (3).

Accordingly, another preferable example for the preparation of C20-*N*-urea salinomycin derivatives is to react said p-nitrophenyl urethane of C20-amino SAL with the formula FLC-00604 (prepared from C20-amino SAL with the formula FLC-00105 using the method described below),
and:

a primary amine with the general formula (20):

$R_2$-$NH_2$ (20)

wherein $R_2$ is as defined above;
or
a secondary amine with the general formula (21):

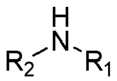

(21)

wherein $R_1$ and $R_2$ are as defined above.

C20-amino SAL prepared as described above is converted to urethane FLC-00604, which is used for the synthesis of salinomycin derivatives with the general formula (3).

Derivatives with the general formula (3) are prepared according to Diagram 5.

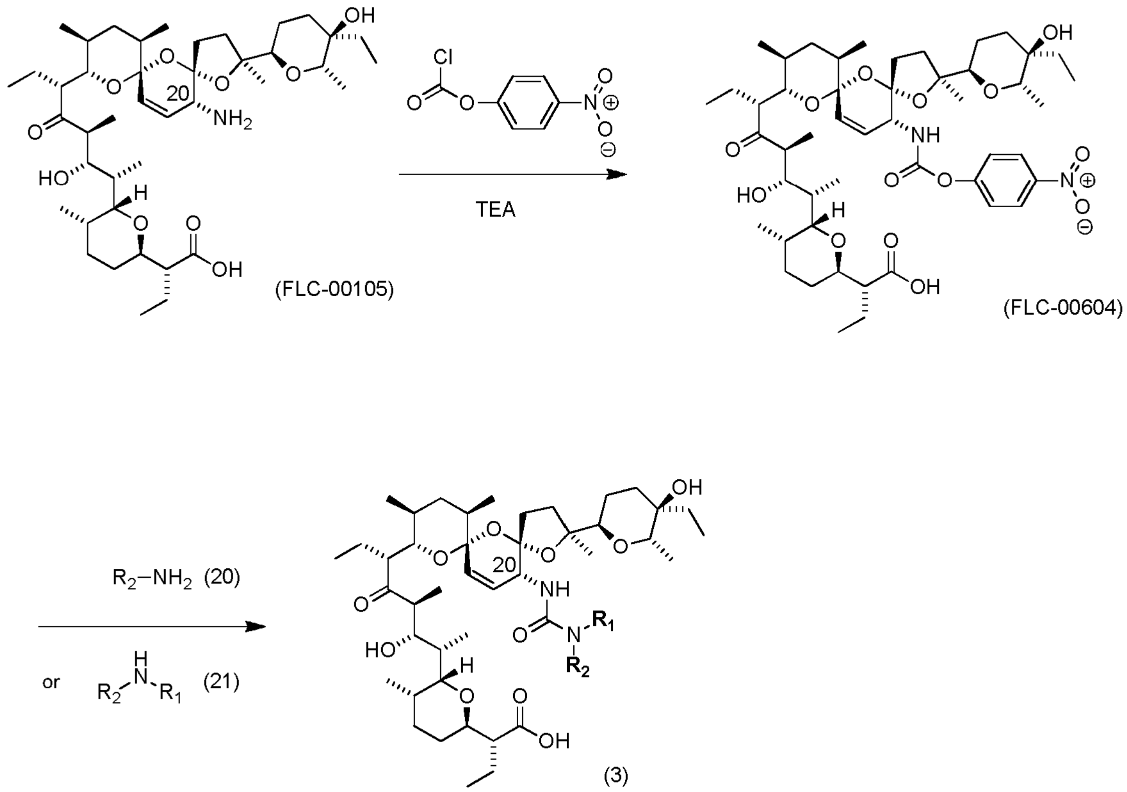


(FLC-00105)

(FLC-00604)

(3)

Diagram 5.

The method optionally comprises a step of transforming the resulting compound in acid form into a salt thereof. A person skilled in the art will know how to select the reaction conditions for the conversion of a salinomycin derivative in acid form into a salt thereof.

In the first step, C20-amino SAL *p*-nitrophenyl urethane with the formula (FLC-00604) is obtained from C20-amino SAL, and then a compound (3) is obtained by reacting the urethane with a substituted amine with the general formula (20) or (21).

C20-amino SAL *p*-nitrophenyl urethane with the formula (FLC-00604) is a novel salinomycin derivative which is an intermediate for the preparation of the final inventive compounds. Similar derivatives were known (phenyl urethane or *p*-nitrobenzyl urethane of C20-*epi*-aminosalinomycin - analogues with an inverted configuration at the asymmetric C20 carbon atom [Li et al., European Journal of Medicinal Chemistry, 148, 2018, 279-290]); to date, however, no such derivative has been prepared for C20-amino SAL. This derivative solves the technical problem of the difficulty or even impossibility of obtaining the inventive compounds having biological properties. Conversion to the final derivatives using said intermediate is convenient and occurs under mild conditions that do not result in the degradation of C20-amino SAL derivatives.

*Preparation of C20-amino SAL p-nitrophenyl urethane:*

C20-amino SALp-nitrophenyl urethane with the formula (FLC-00604) is prepared from C20-amino SAL by reacting it with *p*-nitrophenyl chloroformate in the presence of a base. The reaction is carried out in a chloroaliphatic solvent, in DCM, DCE, chloroform, preferably in DCM, or in a polar solvent - simple nitrile (MeCN), THF, simple amide (NMP, DMA), DMF, or in an aromatic solvent (benzene, toluene, xylene) or in a mixture of solvents, at reduced temperature or room temperature, preferably at reduced temperature. An aliphatic amine or inorganic salt such as carbonates, (sodium, potassium, caesium) bicarbonates or TEA, tripropylamine, tributylamine, diisopropylamine, triisopropylamine, triisobutylamine, *tert*-butylamine, Py, pyridine derivatives (picoline, colidine), DIPEA, DBU, preferably TEA or DIPEA are used as the base.

The conversion rate is controlled by TLC and/or LC-MS. The post-reaction mixture is diluted with a chloroaliphatic solvent, DCM, DCE, chloroform, preferably DCM and washed several times with water. The organic layer is evaporated to dryness under reduced pressure and then evaporated several times with n-pentane or MeCN or lyophilised, e.g. with

dioxane, and used in the next step to obtain the desired inventive derivatives.

The conversion of C20-amino SAL using the step of preparing C20-amino SAL p-nitrophenyl urethane with the formula (FLC-00604) is used to produce a number of other inventive derivatives, C20-amino SAL urea derivatives which were not obtainable with the use of direct conversion from C20-amino SAL, as well as other inventive derivatives, as will be discussed in more detail below.

Thus, the second method for preparing C20-amino SAL urea derivatives consists in mixing a urethane with the formula FLC-00604 with a primary amine with the general formula (20) or a secondary amine with the general formula (21) in the presence of a base (Diagram 5). The reaction is carried out in a chloroaliphatic solvent, in DCM, DCE, chloroform, or in a polar aprotic solvent - simple nitrile (MeCN), THF, simple amide (NMP, DMA), DMF, or in an aromatic solvent (benzene, toluene, xylene) preferably in THF or in a mixture of solvents, at room temperature. An aliphatic amine or inorganic salt such as carbonates bicarbonates, (sodium, potassium, caesium) or TEA, tripropylamine, tributylamine, diisopropylamine, triisopropylamine, triisobutylamine, *tert*-butylamine, Py, pyridine derivatives (picoline, colidine), DIPEA, DBU, preferably TEA or DIPEA are used as the base.

After the reaction, the procedure is as in the example above describing the synthesis of C20-N-urea derivatives by reacting C20-amino SAL with an isocyanate with the general formula (11).

A further preferred method for preparing salinomycin C20-*N*-urea derivatives is by reacting C20-amino SAL (FLC-00105) with 4-ethyl-2,3-dioxo-1-piperazinecarbonyl chloride to obtain the derivative FLC-00518, or 3-(methylsulphonyl)-2-oxoimidazolidine-1-carbonyl chloride, to obtain the derivative FLC-00519, using conditions as in the reaction of C20-amino SAL (FLC-00105) with acid chlorides with the general formula (13), as described below.

<u>Method of preparing C20-amino SAL thioureas with the general formula</u> (4):

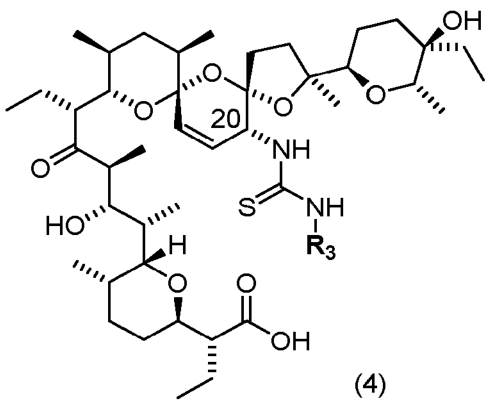

(4)

Salinomycin derivatives with the general formula (4) are prepared according to the conversion in Diagram 6 by reacting a C20-amino SAL with the formula FLC-00105 and an isothiocyanate with the general formula (12):

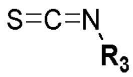

(12),

wherein $R_3$ is as defined above.

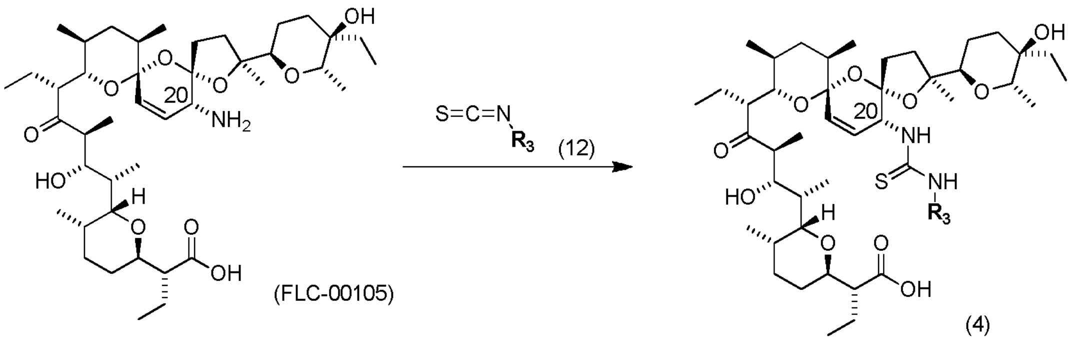


(FLC-00105)

(4)

Diagram 6.

The method optionally comprises a step of transforming the resulting compound in acid form into a salt thereof. A person skilled in the art will know how to select the reaction conditions for the conversion of a salinomycin derivative in acid form into a salt thereof.

The reaction of C20-amino SAL with an isothiocyanate with the general formula (12) is carried out as in the reaction of C20-amino SAL with the formula FLC-00105 with an isocyanate with the general formula (11) as described above. Preferably, the solvent used is THF.

Method of preparing C20-amino SAL amides with the general formula (5):

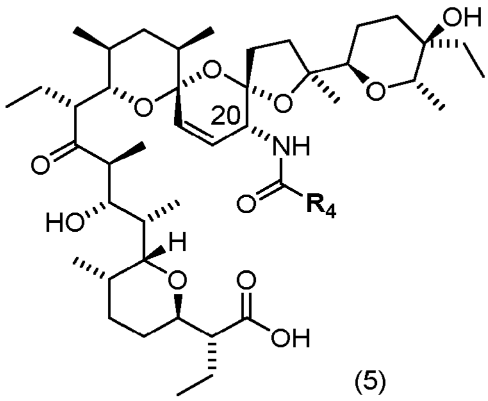

(5)

Salinomycin derivatives with the general formula (5) can be prepared using various methods.

The first method for preparing C20-amino SAL amide derivatives involves the reaction according to Diagram 7, reacting C20-amino SAL with the formula FLC-00105 and an acid chloride with the general formula (13):

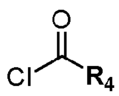

(13),

wherein $R_4$ is as defined above.

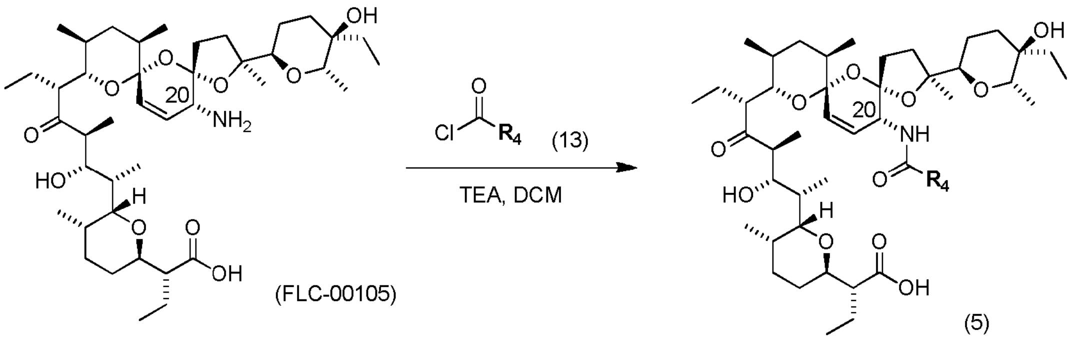


(FLC-00105) (13) (5)

Diagram 7.

The method optionally comprises a step of transforming the resulting compound in acid form into a salt thereof. A person skilled in the art will know how to select the reaction conditions for the conversion of a salinomycin derivative in acid form into a salt thereof.

The reaction of C20-amino SAL with an isocyanate with the general formula (13) is carried out in a chloroaliphatic solvent, in DCM, DCE, chloroform, preferably in DCM, or in a polar aprotic solvent - simple nitrile (MeCN), THF, simple amide (NMP, DMA), DMF, or in an aromatic solvent (benzene, toluene, xylene) or in a mixture of solvents, at room temperature, in the presence of a base. Also preferably, the reaction is conducted under anhydrous conditions. An aliphatic amine or inorganic salt such as carbonates, bicarbonates (sodium, potassium, caesium) or TEA, tripropylamine, tributylamine, diisopropylamine, triisopropylamine, triisobutylamine, tert-butylamine, Py, pyridine derivatives (picoline, colidine), DIPEA, DBU, preferably TEA or DIPEA are used as the base.

The conversion rate is controlled by TLC and/or LC-MS. If a side product with an additional fragment derived from the acid chloride with the general formula (13) used is present in the reaction mixture, the organic layer is concentrated under reduced pressure, and MeCN is added to the residue with 1M NaOH and stirred at room temperature. After hydrolysis, the degree of hydrolysis is preferably controlled by LC-MS, the post-reaction mixture is diluted with water and extracted twice with DCM. The post-reaction mixture is purified by way of column chromatography, preferably combined with an ELS detector, using a column packed with silica and a mixture of organic solvents. The fractions containing the desired product are combined and evaporated under reduced pressure. The residue is dissolved in a chloroaliphatic solvent, preferably in DCM or in chloroform or in AcOEt, followed by washing it with an aqueous solution of a suitable salt (carbonate or bicarbonate, such as sodium or potassium or lithium) or with an aqueous solution of acid, such as sulphuric(VI) ($H_2SO_4$) or hydrochloric acid (HCl). The organic layer is evaporated to dryness under reduced pressure and then evaporated several times with *n*-pentane or MeCN or lyophilised, e.g. with dioxane.

A further method for preparing C20-*N*-amide SAL derivatives involves the reaction according to Diagram 8, reacting C20-amino SAL with the formula FLC-00105 and a carboxylic acid with the general formula (14):

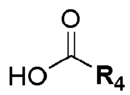

(14),

wherein $R_4$ is as defined above.

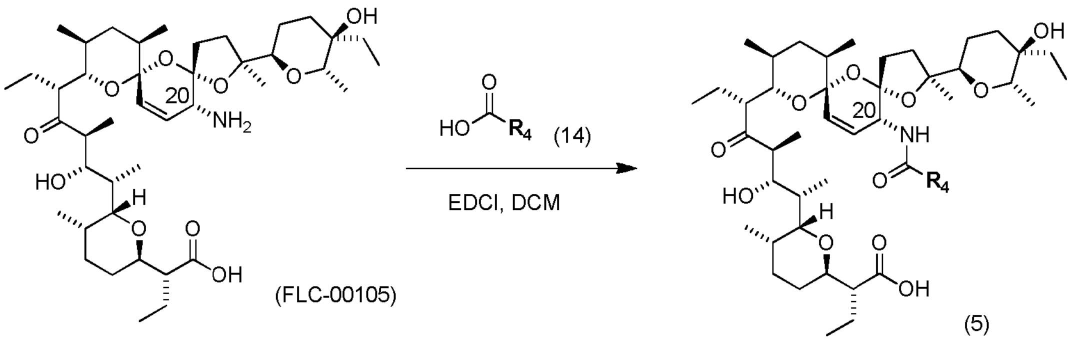


(FLC-00105)

(5)

Diagram 8.

The method optionally comprises a step of transforming the resulting compound in acid form into a salt thereof. A person skilled in the art will know how to select the reaction conditions for the conversion of a salinomycin derivative in acid form into a salt thereof.

For the reaction shown in Diagram 8 in order to carry out the reaction it is necessary to activate the carboxylic acid with the general formula (14) with appropriate activating reagents according to the procedure described in detail below.

The reaction is carried out in a chloroaliphatic solvent, in DCM, DCE, chloroform, preferably in DCM, or in a polar aprotic solvent - simple nitrile (MeCN), THF, simple amide (NMP, DMA), DMF, or in an aromatic solvent (benzene, toluene, xylene) or in a mixture of solvents, at room temperature. Also preferably, the reaction is conducted under anhydrous conditions. The reagent activating the carboxylic acid used is DCC, DIC, CDI, EDCI, HATU, TBTU, preferably EDCI in the presence of an aliphatic amine such as: TEA, tripropylamine, tributylamine, diisopropylamine, triisopropylamine, triisobutylamine, *tert*-butylamine, Py, pyridine derivatives (picoline, colidine), DIPEA, DBU, preferably DIPEA or TEA. It is preferable to also use *p*-TsOH, DMAP or 4-pyrrolidinopyridine, most preferably DMAP.

The conversion rate is controlled by TLC and/or LC-MS. The post-reaction mixture is purified by column chromatography, and the subsequent procedure is as in the example above describing the synthesis of C20-*N*-urea derivatives by reacting C20-amino SAL with an isocyanate with the general formula (11).

A further preferred method for preparing C20-amino SAL amides with the general formula (5) is to react C20-amino SAL with the formula FLC-00105: with glutaric anhydride (FLC-00436), as shown in Diagram 9, or with 2-bromoacetyl bromide (FLC-00501), as shown in Diagram 10. Unique reaction conditions were used for each of the reactants, as described in detail in the examples.

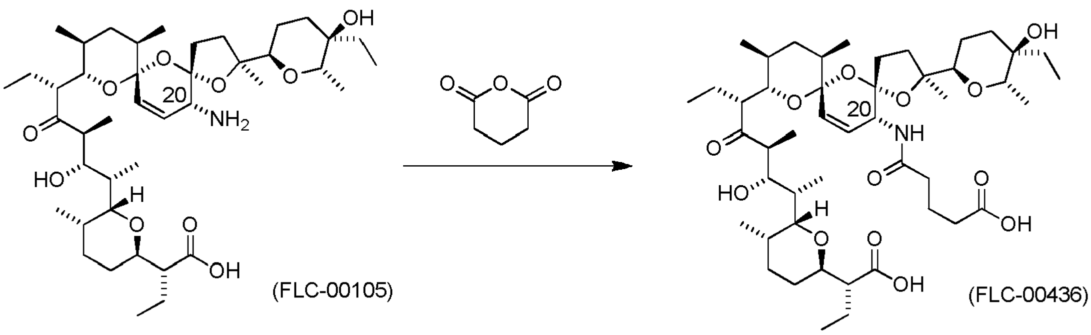

(FLC-00105)

(FLC-00436)

Diagram 9.

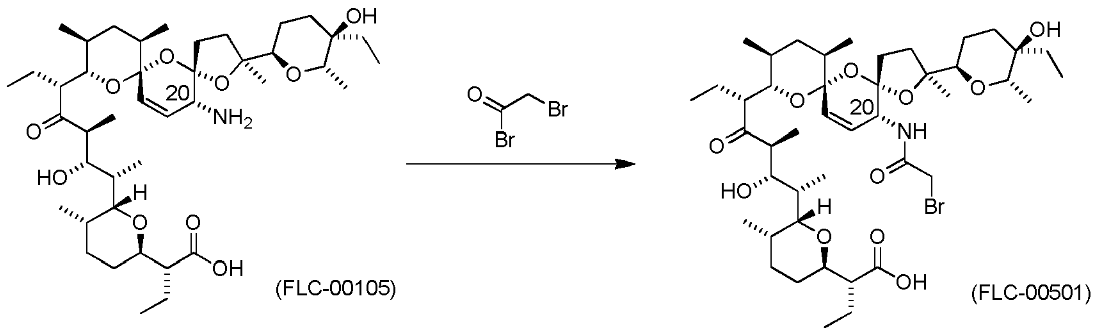

(FLC-00105) (FLC-00501)

Diagram 10.

Method of preparing C20-amino SAL urethanes (carbamates) with the general formula (7):

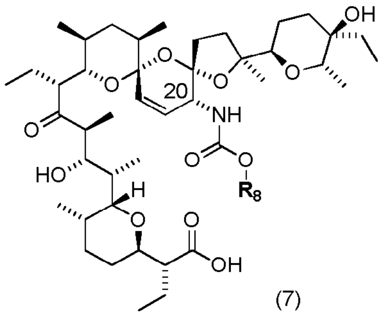

(7)

Salinomycin derivatives with the general formula (7) can be prepared using various methods. The first method involves the reaction according to Diagram 11, reacting C20-amino SAL with the formula FLC-00105 and a chloroformate with the general formula (15):

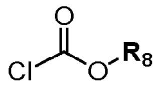

(15),

wherein $R_8$ is as defined above.

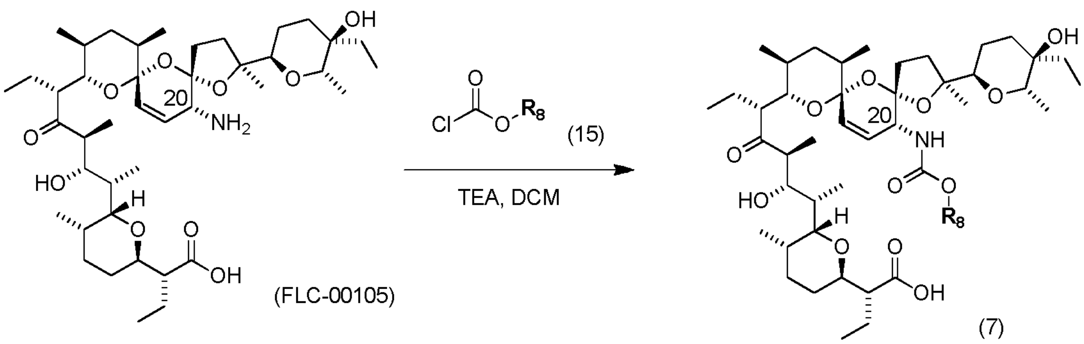


(FLC-00105) (7)

Diagram 11.

The method optionally comprises a step of transforming the resulting compound in acid form into a salt thereof. A person skilled in the art will know how to select the reaction conditions for the conversion of a salinomycin derivative in acid form into a salt thereof.

The reaction of C20-amino SAL with a chlorformate with the general formula (15) is carried out as in the reaction

of C20-amino SAL with the formula FLC-00105 with an acid chloride with the general formula (13) as described above.

A further preferred method for preparing C20-amino SAL carbamates is the reaction shown in Diagram 12 between C20-amino SAL
and:

*p*-nitrophenyl carbonate with the general formula (16):

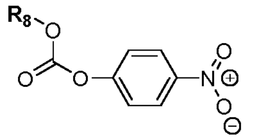
(16),

or
imidazole 1-carboxylate with the general formula (17):

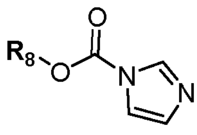
(17),

wherein $R_8$ is as defined above.

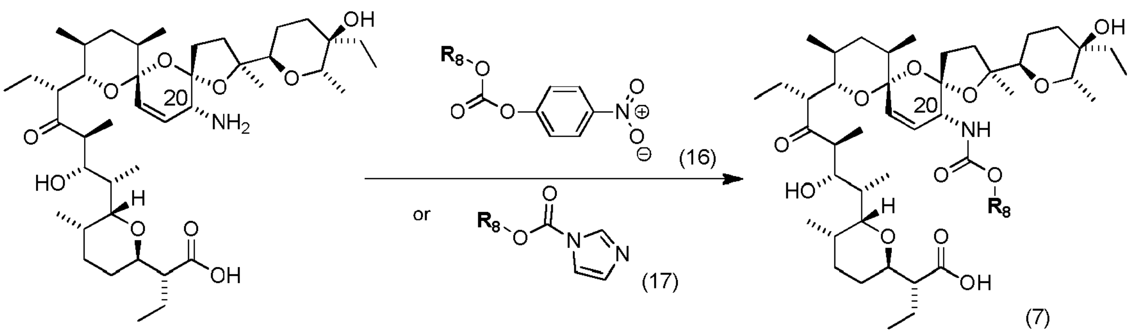

(FLC-00105)

Diagram 12.

This is another innovative synthesis method for preparing the inventive compounds. It uses alcohol activation by means other than the use of phosgene or an equivalent thereof (such as diphosgene, triphosgene, etc.) yielding chloroformates which are substrates for the synthesis of C20-amino SAL carbamates using the method described above. The method using compounds with the structures (16) and (17) according to Diagram 12 solves the technical problem of the difficulty or even impossibility of preparing the inventive compounds having biological properties. It yields derivatives whose synthesis is impossible due to the commercial unavailability of some of the chloroformates or the impossibility of synthesising said chloroformates. The "active" form of alcohol is formed under mild conditions, which is necessary due to the presence in the structures of activated alcohols of groups that are sensitive to acid conditions generated by using phosgene, diphosgene or triphosgene.

The method optionally comprises a step of transforming the resulting compound in acid form into a salt thereof. A person skilled in the art will know how to select the reaction conditions for the conversion of a salinomycin derivative in acid form into a salt thereof.

The reaction of C20-amino SAL with the formula FLC-00105 with *p*-nitrophenyl carbonate with the general formula (16) or with imidazole 1-carboxylate with the general formula (17) is carried out in the chloroaliphatic solvent DCM, DCE, chloroform or in THF, preferably in THF in the presence of a base. An aliphatic amine or inorganic salt such as carbonates, bicarbonates (sodium, potassium, caesium) or TEA, tripropylamine, Py, pyridine derivatives (picoline, colidine), DIPEA, preferably TEA or DIPEA are used as the base.

The conversion rate is controlled by TLC and/or LC-MS. The post-reaction mixture is purified by column chromatography, and the subsequent procedure is as in the example above describing the synthesis of C20-*N*-urea

derivatives by reacting C20-amino SAL with an isocyanate with the general formula (11).

A further preferred method for preparing the inventive compound, C20-amino SAL carbamate with the formula FLC-00461, consists in reacting C20-amino SAL with the formula FLC-00105 with di-*tert*-butyl pyrocarbonate $Boc_2O$, shown in Diagram 13.

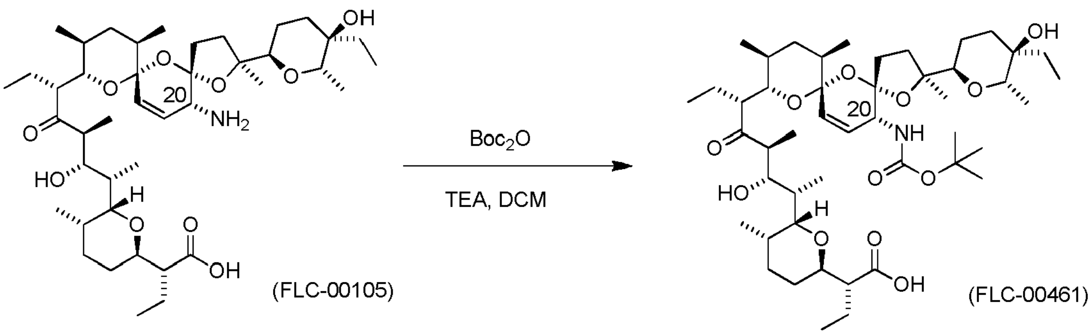

(FLC-00105) (FLC-00461)

Diagram 13.

The method optionally comprises a step of transforming the resulting compound in acid form into a salt thereof. A person skilled in the art will know how to select the reaction conditions for the conversion of a salinomycin derivative in acid form into a salt thereof.

The reaction of C20-amino SAL with $Boc_2O$ with the general formula is carried out in a chloroaliphatic solvent, in DCM, DCE, chloroform, preferably in DCM, or in a polar aprotic solvent - simple nitrile (MeCN), THF, simple amide (NMP, DMA), DMF, or in an aromatic solvent (benzene, toluene, xylene) or in a mixture of solvents, at room temperature, in the presence of a base. An aliphatic amine or inorganic salt such as carbonates, bicarbonates (sodium, potassium, caesium) or TEA, tripropylamine, tributylamine, diisopropylamine, triisopropylamine, triisobutylamine, *tert*-butylamine, Py, pyridine derivatives (picoline, colidine), DIPEA, DBU, preferably TEA or DIPEA are used as the base.

The conversion rate is controlled by TLC and/or LC-MS. The post-reaction mixture is purified by column chromatography, and the subsequent procedure is as in the example above describing the synthesis of C20-*N*-urea derivatives by reacting C20-amino SAL with an isocyanate with the general formula (11).

A further preferred method for preparing C20-amino SAL carbamates is the reaction shown in Diagram 14 between said C20-amino SAL *p*-nitrophenyl urethane (obtained using the method described above from C20-amino SAL) and an alcohol with the formula:

$$HO\text{-}R_8 \qquad (23)$$

wherein $R_8$ is as defined above

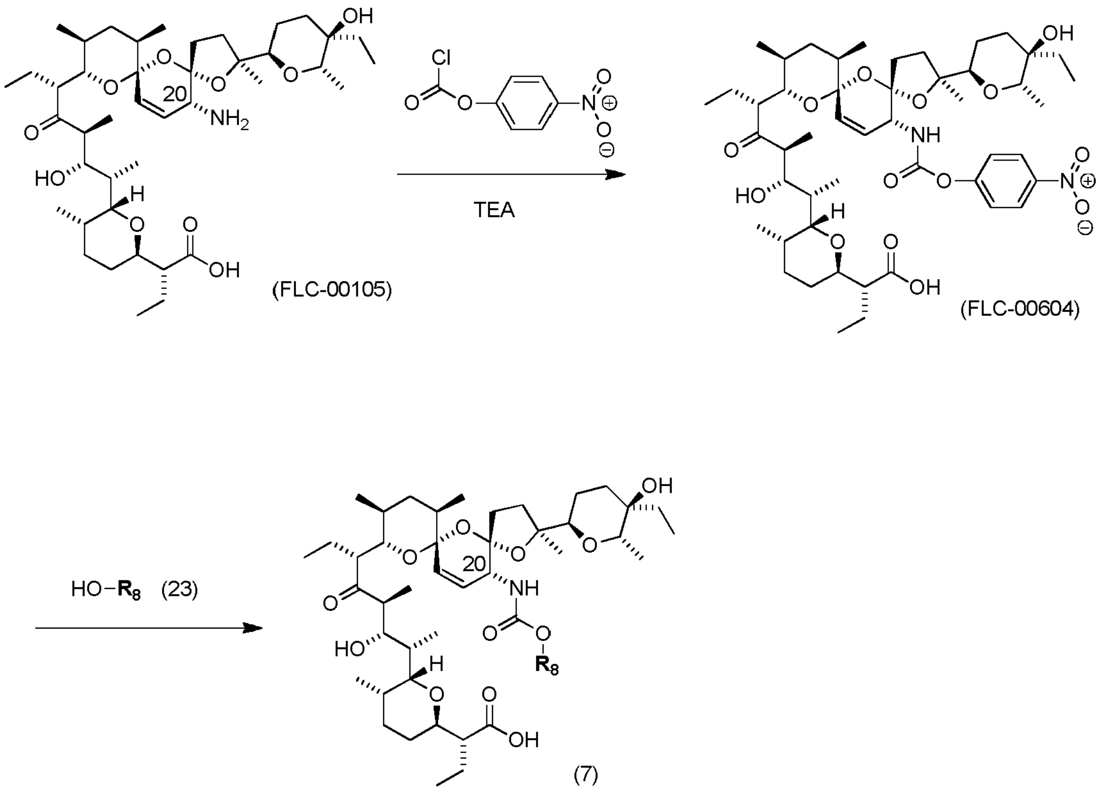


(FLC-00105)

(FLC-00604)

(7)

Diagram 14.

This is another innovative use of compound FLC-00604 to obtain the inventive compounds having biological properties.

The method optionally comprises a step of transforming the resulting compound in acid form into a salt thereof. A person skilled in the art will know how to select the reaction conditions for the conversion of a salinomycin derivative in acid form into a salt thereof.

The reaction is carried out as in the reaction for preparing C20-*N*-urea salinomycin derivatives from C20-amino SAL *p*-nitrophenyl urethane with the formula (FLC-00604) as described above, except that instead of the amine with the general formula (20) or (21), an alcohol with the formula HO-Rs is added to the reaction mixture.

<u>Method of preparing C20-amino SAL sulphonamides with the general formula (8):</u>

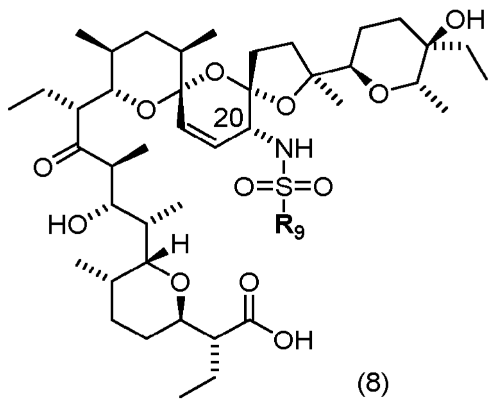

(8)

Salinomycin derivatives with the general formula (8) can be prepared using various reaction conditions. The first

method involves the reaction according to Diagram 15, reacting C20-amino SAL with the formula FLC-00105 and a sulfonyl chloride with the general formula (18):

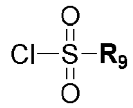

(18),

wherein $R_9$ is as defined above.

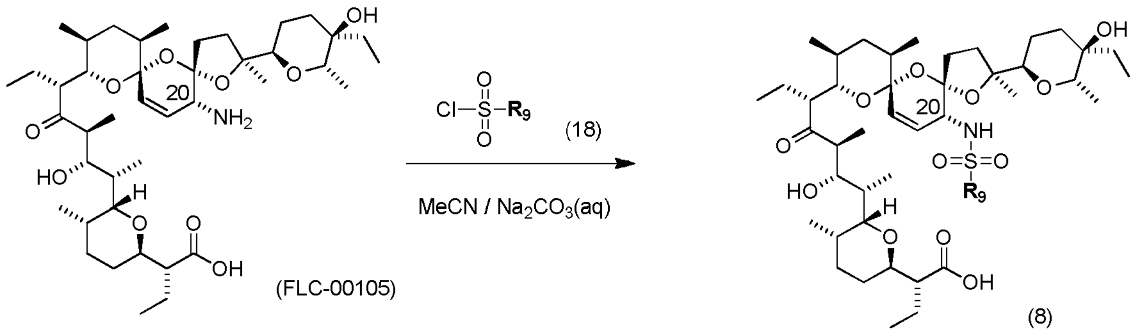


(FLC-00105) (18) (8)

Diagram 15.

The method optionally comprises a step of transforming the resulting compound in acid form into a salt thereof. A person skilled in the art will know how to select the reaction conditions for the conversion of a salinomycin derivative in acid form into a salt thereof.

The reaction is carried out in a mixture of an organic solvent, MeCN, dioxane, THF, preferably MeCN, and an aqueous solution of a base, carbonate or bicarbonate of sodium, potassium, lithium, preferably sodium carbonate. A sulfonyl chloride with the general formula (18) in an organic solvent, in MeCN, dioxane, THF, preferably in MeCN, is added dropwise to C20-amino SAL FLC-00105 in said solvent mixture at room temperature or at reduced temperature, preferably at reduced temperature.

The conversion rate is controlled by TLC and/or LC-MS. The post-reaction mixture is diluted with water and extracted twice with DCM. The residue is purified by column chromatography, and the subsequent procedure is as in the example above describing the synthesis of C20-*N*-urea derivatives by reacting C20-amino SAL with an isocyanate with the general formula (11).

A further preferred method for preparing C20-amino SAL sulphonamides is the reaction shown in Diagram 16 between C20-amino SAL and a sulfonyl chloride with the general formula (18):

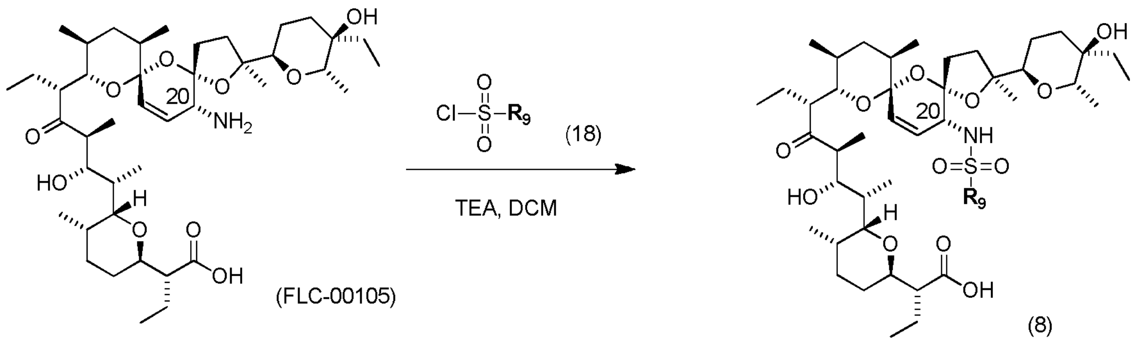


(FLC-00105) (18) (8)

Diagram 16.

The method optionally comprises a step of transforming the resulting compound in acid form into a salt thereof. A person skilled in the art will know how to select the reaction conditions for the conversion of a salinomycin derivative in acid form into a salt thereof.

The reaction is carried out in a chloroaliphatic solvent, in DCM, DCE, chloroform, preferably in DCM, at room

temperature or reduced temperature, preferably at reduced temperature in the presence of a base. A sulfonyl chloride with the general formula (18) in a chloroaliphatic solvent, in DCM, DCE, chloroform, preferably in DCM, is added dropwise to C20-amino SAL in said solvent or a mixture thereof. An aliphatic amine, TEA, tripropylamine, tributylamine, triisopropylamine, DIPEA, preferably DIPEA or TEA, is used as the base.

The conversion rate is controlled by TLC and/or LC-MS. After the reaction, the procedure is as in the example above describing the synthesis of C20-*N*-urea derivatives by reacting C20-amino SAL with an isocyanate with the general formula (11).

<u>Method of preparing C20-amino SAL dithiocarbamates with the general formula</u> (9):

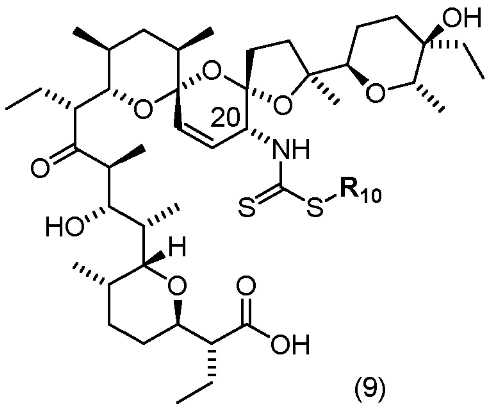

(9)

Salinomycin derivatives with the general formula (9) are prepared according to Diagram 17, by reacting C20-amino SAL with the formula FLC-00105 and carbon disulphide $CS_2$ and halide with the general formula (19):

$Y\text{-}R_{10}$ (19)

wherein $R_{10}$ is as defined above, and Y = Br or I.

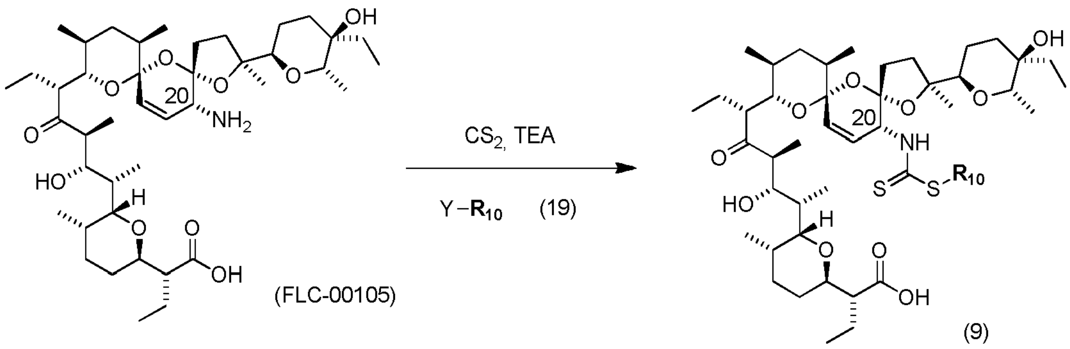


(FLC-00105)

(9)

Diagram 17.

The method optionally comprises a step of transforming the resulting compound in acid form into a salt thereof. A person skilled in the art will know how to select the reaction conditions for the conversion of a salinomycin derivative in acid form into a salt thereof.

The reaction yielding salinomycin derivatives with the general formula (9) is carried out in a chloroaliphatic solvent, in DCM, DCE, chloroform, or in a polar aprotic solvent, simple nitrile MeCN or THF, preferably in MeCN or in a mixture of solvents, at reduced temperature or at room temperature, preferably at reduced temperature, in the presence of an aliphatic amine. An aliphatic amine such as TEA, tripropylamine, tributylamine, diisopropylamine, triisopropylamine, triisobutylamine, DIPEA, preferably DIPEA or TEA is used as the base.

The conversion rate is controlled by TLC and/or LC-MS. The post-reaction mixture is purified by column chromatography, and the subsequent procedure is as in the example above describing the synthesis of C20-*N*-urea derivatives by reacting C20-amino SAL with an isocyanate with the general formula (11).

Method of preparing S-substituted C20-amino SAL thiocarbamates with the general formula (10):

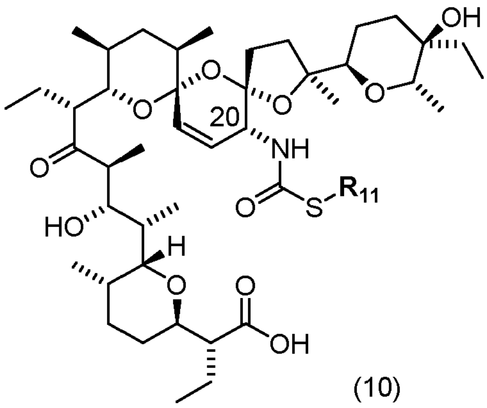

(10)

S-substituted C20-amino SAL thiocarbamates are obtained by reacting C20-amino SAL p-nitrophenyl urethane with the structure FLC-00604:

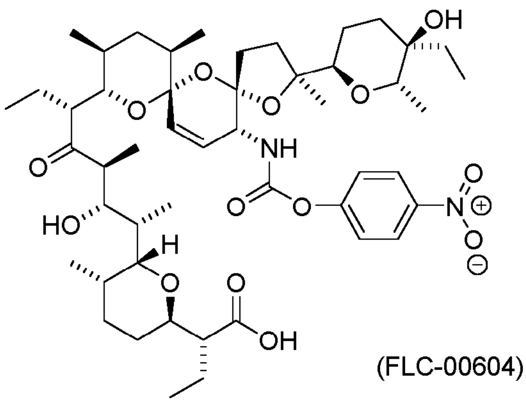

(FLC-00604)

with a thiol with the general formula (22):

**HS-$R_{11}$** (22)

wherein $R_{11}$ is as defined above.

This is another innovative example of using the intermediate FLC-00604 to prepare the final inventive compounds. Derivatives with the general formula 10 are obtained according to Diagram 18:

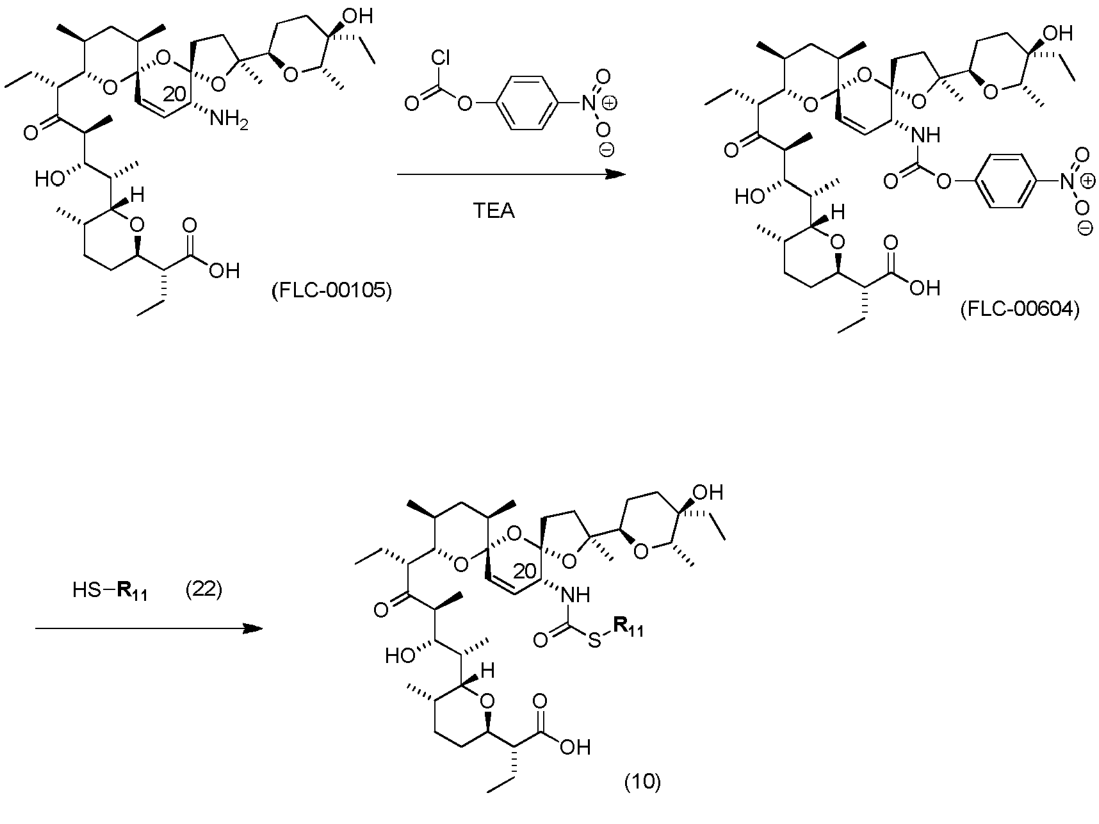


(FLC-00105)

(FLC-00604)

(10)

Diagram 18.

The method optionally comprises a step of transforming the resulting compound in acid form into a salt thereof. A person skilled in the art will know how to select the reaction conditions for the conversion of a salinomycin derivative in acid form into a salt thereof.

The reaction is carried out as in the reaction for preparing C20-*N*-urea salinomycin derivatives from C20-amino SAL *p*-nitrophenyl urethane with the formula (FLC-00604) as described above, except that instead of the amine with the general formula (20) or (21), a thiol with the general formula (22) is added to the reaction mixture.

<u>Method of preparing C20-amino SAL oxalate derivatives with the general formula (6):</u>

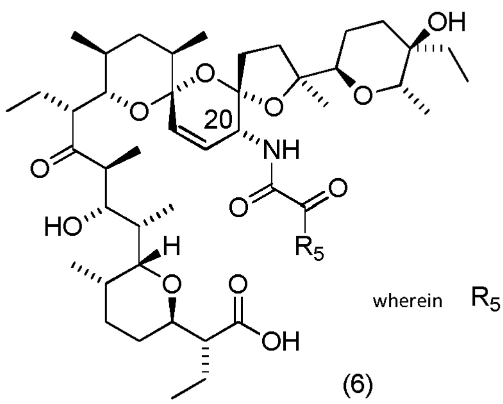

wherein $R_5$ = -O-$R_6$

(6)

wherein $R_6$ is as defined above.

Salinomycin derivatives with the general formula (6) are prepared according to Diagram 19, by reacting C20-amino SAL with the formula FLC-00105 and alkyl chlorooxoacetate:

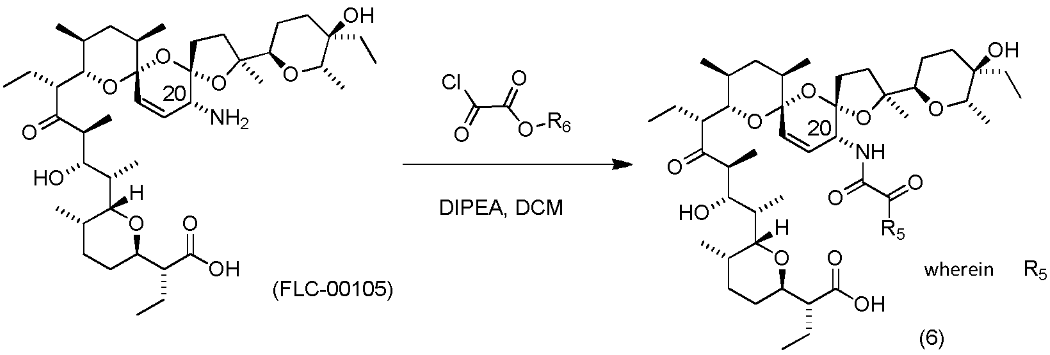


(FLC-00105)

wherein $R_5$ = -O-$R_6$

(6)

Diagram 19.

The method optionally comprises a step of transforming the resulting compound in acid form into a salt thereof. A person skilled in the art will know how to select the reaction conditions for the conversion of a salinomycin derivative in acid form into a salt thereof.

The reaction of C20-amino SAL with alkyl chlorooxoacetate is carried out in a chloroaliphatic solvent, in DCM, DCE, chloroform, preferably in DCM, or in a polar aprotic solvent - simple nitrile (MeCN), THF, simple amide (NMP, DMA), DMF or in an aromatic solvent (benzene, toluene, xylene) or in a mixture of solvents, at reduced temperature or at room temperature, preferably at reduced temperature, in the presence of a base. An aliphatic amine or inorganic salt such as carbonates, bicarbonates (sodium, potassium, caesium) or TEA, tripropylamine, tributylamine, diisopropylamine, triisopropylamine, triisobutylamine, *ter*t-butylamine, Py, pyridine derivatives (picoline, colidine), DIPEA, DBU, preferably TEA or DIPEA are used as the base.

The conversion rate is controlled by TLC and/or LC-MS. The post-reaction mixture is purified by column chromatography, and the subsequent procedure is as in the example above describing the synthesis of C20-*N*-urea derivatives by reacting C20-amino SAL with an isocyanate with the general formula (11).

<u>Method of preparing C20-*N*-modified compounds with the general formula (6):</u>

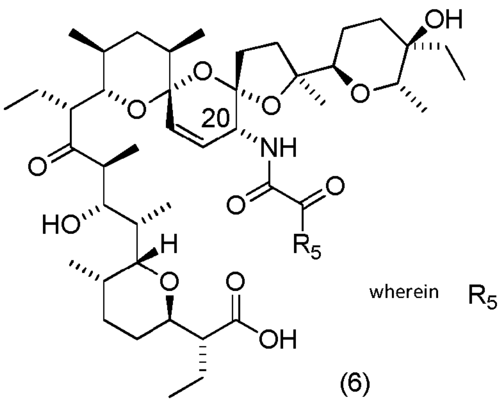

wherein $R_5$ = -NH-$R_7$

(6)

wherein $R_7$ is as defined above.

It was observed that the preparation of derivatives with the formula (6), wherin $R_5$ = -NH-$R_7$ is efficiently carried out via an intermediate with the structure FLC-00373:

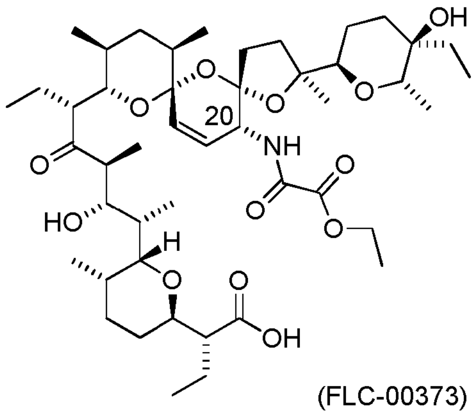

(FLC-00373)

C20-amino SAL derivatives with the general formula (6), where $R_5$ = -NH-$R_7$ are prepared according to Diagram 20:

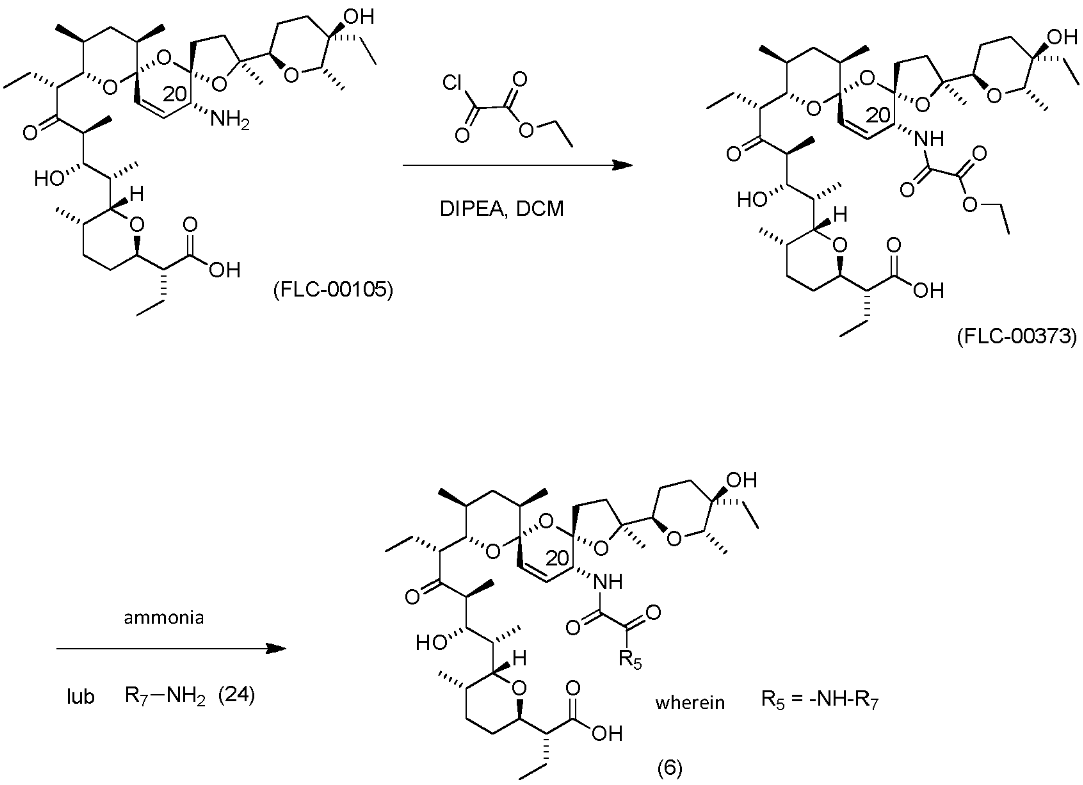


(FLC-00105) (FLC-00373) (6)

Diagram 20.

The method optionally comprises a step of transforming the resulting compound in acid form into a salt thereof. A person skilled in the art will know how to select the reaction conditions for the conversion of a salinomycin derivative in acid form into a salt thereof.

C20-amino SAL prepared as described above is converted to a compound with the structure FLC-00373, which is used for the synthesis of salinomycin derivatives with said general formula (6).

In the first step, the derivative FLC-00373 is obtained from C20-amino SAL, and then a compound (6) is obtained by way of reaction with an amine with the general formula:

$R_7$-$NH_2$ (24)

The compound with the formula FLC-00373 is a novel salinomycin derivative (C20-aminosalinomycin oxalate

derivative), which is an object of the invention as an intermediate for preparing the final inventive compounds.

Conversion to the final derivatives using this intermediate is efficient and convenient and takes place under mild conditions. Said compound overcomes the technical problem in that salinomycin derivatives having biological properties now can be prepared that could not be prepared otherwise. Once synthesised, the intermediate allows for performing simple conversions with commercially available amines to obtain various salinomycin final derivatives.

The reaction of the intermediate compound with the formula FLC-00373 with a methanolic ammonia solution or with an amine with the general formula (24) is carried out in a chloroaliphatic solvent, in DCM, DCE, chloroform, preferably in DCM, or in a polar aprotic solvent - simple nitrile (MeCN), THF, simple amide (NMP, DMA), DMF, or in an aromatic solvent (benzene, toluene, xylene) or in a mixture of solvents, at room temperature. Also preferably, the reaction is conducted under anhydrous conditions.

After the reaction, the procedure is as in the example above describing the synthesis of C20-*N*-urea derivatives by reacting C20-amino SAL with an isocyanate with the general formula (11).

The invention also relates to the use of the novel salinomycin derivatives according to the invention for use as a medicament. In a preferred embodiment, the compounds according to the invention are for use as anti-cancer agents.

The compound according to the invention is suitable for use in conditions selected without limitations from the group consisting of leukaemia, including without limitation acute myeloid leukaemia, chronic myeloid leukaemia, acute lymphoblastic leukaemia, biphenotypic leukaemia, multiple myeloma; non-small cell lung cancer, including without limitation lung epithelial cell carcinoma, lung adenocarcinoma, human lung squamous cell cancer; large intestine (colon) cancer, including without limitation large intestine adenocarcinoma, colon epithelial cell cancer; tumour of the central nervous system, including without limitation brain tumours such as glioma; melanoma, including without limitation malignant melanoma, epithelial melanoma, non-epithelial melanoma; ovarian cancer, including without limitation epithelial ovarian cancer, ovarian cystadenocarcinoma; kidney cancer, including without limitation renal cell carcinoma; prostate cancer, including without limitation prostate adenocarcinoma; breast cancer, including without limitation adenocarcinoma of the breast, inflammatory breast cancer, metastatic adenocarcinoma; stomach cancer; pancreatic cancer; sarcoma and uterine body cancer, as well as its drug-resistant variant; cervical cancer; bladder cancer.

In this description, the terms "cancer" and "carcinoma" are used interchangeably.

Preliminary studies indicate that the compounds according to the invention have anti-cancer activity against the cell lines recited below:

HL-60(TB), K-562, MOLT-4, RPMI-8226, SR, A549/ATCC, EKVX, HOP-62, HOP-92, NCI-H226, NCI-H23, NCI-H460, NCI-H522, COLO 205, HCC-2998, HCT-116, HCT-15, HT29, KM12, SW-620, SF-268, SF-295, SF-539, SNB-19, SNB-75, U251, LOX IMVI, MALME-3M, M14, MDA-MB-435, SK-MEL-2, SK-MEL-28, SK-MEL-5, UACC-257, UACC-62, IGROV1, OVCAR-3, OVCAR-4, OVCAR-5, OVCAR-8, NCI/ADR-RES, SK-OV-3, 786-0, A498, ACHN, CAKI-1, RXF 393, SN12C, TK-10, UO-31, PC-3, DU-145, MCF7, MDA-MB-231/ATCC, HS 578T, BT-549, T-47D, MDA-MB-468, KATOIII, NCI-N87, SNU-16, SNU-5, AGS, SNU-1, Capan-2, ATCC HTB-80, Panc 10.05, CFPAC-1, HPAF-II, SW 1990, BxPC-3, AsPC-1, MES-SA, MES-SA/DXS, KLE, HEC-1-A, AN3 CA, Ca-Ski, DoTc2 4510, SiHa, C-33-A, 5637, KU-19-19, MBT-2, HCV29T, Hu1703He. For some of these cell lines, the specific associated conditions have already been recited in the list above.

Anti-cancer activity is understood as:

- preventing cancer development,
- inhibiting cancer cell growth (inhibiting proliferation),
- inducing cancer cell death (pro-apoptotic effect),
- preventing or inhibiting cancer cell metastasis,
- inhibiting the phenomenon of multi-drug resistance,
- inhibiting the angiogenesis process,
- eliminating or minimising the impact of neoplastic disease.

*In vitro* studies conducted on a number of cancer cell lines confirmed high cytotoxic activity of the new compounds, which greatly exceeded the bioactivity of a chemically unmodified salinomycin. The salinomycin derivatives obtained not only demonstrate high anti-cancer activity, but also high selectivity of action against cancer cells. Moreover, comparison of the anti-cancer activity and toxicity of the newly synthesized compounds with those exhibited by the salinomycin derivatives known from prior art, including the structurally similar C20-*N*-amide and C20-*N*-carbamate (urethane) derivatives of C20-*epi*-salinomycin with an inverted absolute configuration (*S* instead of *R* absolute configuration) on the asymmetric carbon at the C-20 position [Li et al., European Journal of Medicinal Chemistry, 148, 2018, 279-290], clearly demonstrated the superiority of the compounds that are the object of the present invention in the context of their potential therapeutic use.

The following cancer cell lines were used in *in vitro* studies:

- A2780 (human ovarian cancer) and A2780Cis (cisplatin-resistant human ovarian cancer),
- A375 (human melanoma),
- Hs294T (human melanoma),
- KG-1 (human acute myelogenous leukaemia)
- MIA PaCa-2 (human pancreatic cancer),
- MV-4-11 (human biphenotypic leukaemia),
- NCI-H23 (human lung cancer),
- NCI-H1581 (human lung cancer),
- NCI-H358 (human lung cancer),
- PFSK-1 (malignant primitive neuroectodermal tumour),
- PLC/PRF/5 (human liver cancer),
- SNU-16 (human gastric cancer).

Normal murine BALB/3T3 fibroblasts were also used in the tests for determining the selectivity coefficients of the compounds used in the assays. These values allow for predicting the direction of action of salinomycin derivatives by answering the question whether these compounds will first destroy cancer cells or attack normal body cells.

Table 1 summarises the data for all cell lines (both cancer and normal) used in the *in vitro* studies.

Table 1.

| Cell line symbol | Cell line name | Source of origin of the cell line | Number of cells per well in the test |
|---|---|---|---|
| A2780 | human ovary cancer | ECACC | $2\times10^3$ |
| A2780Cis | cisplatin-resistant human ovary cancer | ECACC | $2\times10^3$ |
| A375 | human melanoma | ATCC | $2\times10^3$ |
| Hs294T | human melanoma | ATCC | $2\times10^3$ |
| KG-1 | human acute myelogenous leukaemia | ATCC | $2\times10^3$ |
| MIA PaCa-2 | human pancreatic cancer | ATCC | $2\times10^3$ |
| MV-4-11 | human biphenotypic leukaemia | ATCC | $2\times10^3$ |
| NCI-H23 | human lung cancer | ATCC | $2\times10^3$ |
| NCI-H358 | human lung cancer | ATCC | $2\times10^3$ |
| NCI-H1581 | human lung cancer | ATCC | $2{,}5\times10^3$ |
| PFSK-1 | malignant primitive neuroectodermal tumour | ATCC | $2{,}5\times10^3$ |
| PLC/PRF/5 | human liver cancer | ATCC | $2\times10^3$ |
| SNU-16 | human gastric cancer | ATCC | $2\times10^3$ |
| BALB/3T3 | normal murine fibroblasts | ATCC | $2\times10^3$ |

During the *in vitro* cytotoxicity tests, the culture media and reagents summarised in Table 2 were used.

Table 2.

| Name | Reagent (Manufacturer) |
|---|---|
| Culture medium for **BALB/3T3, Hs294T** lines | • Dulbecco medium (Gibco)<br>• 10 % FBS HyClone (GE Healthcare, USA)<br>• 2 mM L-glutamine (Sigma-Aldrich, Germany),<br>• 0.1 mg streptomycin, 100 U/mL penicillin (Sigma-Aldrich, Germany and Polfa Tarchomin, Poland) |

(continued)

| Name | Reagent (Manufacturer) |
|---|---|
| Culture medium for **A2780, A2780Cis** lines | • RPMI1640 + GlutaMax (Gibco)<br>• 10% FBS HyClone, (GE Healthcare, USA)<br>• 0.1 mg streptomycin, 100 U/mL penicillin (Sigma-Aldrich, Germany and Polfa Tarchomin, Poland)<br>Medium for A2780C is supplemented with 1 pM cisplatin |
| Culture medium for **A-375** line | • DMEM (ATCC)<br>• 10% FBS (ATCC)<br>• 0.1 mg streptomycin, 100 U/mL penicillin (Sigma-Aldrich, Germany and Polfa Tarchomin, Poland) |
| Culture medium for **KG-1** line | • IMDM (ATCC)<br>• 20% FBS (ATCC)<br>• 0.1 mg streptomycin, 100 U/mL penicillin (Sigma-Aldrich, Germany and Polfa Tarchomin, Poland) |
| Culture medium for **MIA PaCa-2** line | • DMEM (ATCC)<br>• 10% FBS (ATCC)<br>• 2.5% horse serum (Gibco)<br>• 0.1 mg streptomycin, 100 U/mL penicillin (Sigma-Aldrich, Germany, Germany and Polfa Tarchomin, Poland, Poland) |
| Culture medium for **MV4-11** line | • RPMI1640 + GlutaMax (Gibco)<br>• 10% FBS (Sigma-Aldrich, Germany)<br>• 1mM pyruvate (Sigma-Aldrich, Germany)<br>• 0.1 mg streptomycin, 100 U/mL penicillin (Sigma-Aldrich, Germany and Polfa Tarchomin, Poland) |
| Culture medium for **NCI-H23, NCI-H358** lines | • RPMI1640 + GlutaMax (Gibco)<br>• 10% FBS HyClone, (GE Healthcare, USA)<br>• 1mM pyruvate (Sigma-Aldrich, Germany)<br>• 4.5 g/L glucose (Sigma-Aldrich, Germany)<br>• 0.1 mg streptomycin, 100 U/mL penicillin (Sigma-Aldrich, Germany and Polfa Tarchomin, Poland) |
| Culture medium for **NCI-1581** line | • RPMI1640 + GlutaMax (Gibco)<br>• 5% FBS HyClone, (GE Healthcare, USA)<br>• 0.1 mg streptomycin, 100 U/mL penicillin (Sigma-Aldrich, Germany and Polfa Tarchomin, Poland) |
| Culture medium for **PLC/ PRF/5** line | • EMEM (ATCC)<br>• 10% FBS (ATCC)<br>• 0.1 mg streptomycin, 100 U/mL penicillin (Sigma-Aldrich, Germany and Polfa Tarchomin, Poland) |
| Culture medium for **PFSK-1, SNU-16** lines | • RPMI-1640 (ATCC)<br>• 10% FBS (ATCC)<br>• 0.1 mg streptomycin, 100 U/mL penicillin (Sigma-Aldrich, Germany and Polfa Tarchomin, Poland) |
| Test medium for dilutions | • RPMI1640 + HEPES (PChO, IITD)<br>• 10% FBS HyClone (GE Healthcare)<br>• 2mM L-glutamine (Sigma-Aldrich, Germany)<br>• 0.1 mg streptomycin, 100 U/mL penicillin (Sigma-Aldrich, Germany and Polfa Tarchomin, Poland) |
| 10 mM TRIS | 1. Deionised water for general laboratory purposes, non-sterile<br>2. TRIZMA BASE (Sigma-Aldrich, Germany) |

(continued)

| Name | Reagent (Manufacturer) |
|---|---|
| 1% acetic acid | 1. Deionised water for general laboratory purposes, non-sterile<br>2. Acetic acid 80%, analytical grade (POCh, Poland) |
| 0,1 % sulforhodamine B (SRB) solution | 1. Sulforhodamine B sodium salt (Sigma-Aldrich, Germany)<br>2. 1% acetic acid |
| 50% trichloroacetic acid (TCA) | 1. Deionised water for general laboratory purposes, non-sterile<br>2. Trichloroacetic acid, analytical grade (POCh, Poland) |
| 5 mg/ml MTT | 1. MTT (Sigma-Aldrich, Germany)<br>2. PBS (PChO, IITD) |
| DMSO | 1. DMSO (POCh, Poland) |
| cisplatin | • concentrate for solution for infusion, 1 mg/mL (10 mg/10 mL), 110 mL vial (Accord Healthcare) |

*In vitro* cytotoxicity tests were performed according to the procedure described below. Stock solutions of the test compounds with the concentration of 7 mM were prepared for each experiment *ex tempore* by dissolving an weighed amount of the preparation in a suitable amount of dimethylsulfoxide (DMSO). The solvent for further dilutions was a culture medium. The compounds were tested at concentrations starting at 10 μM in a 10-fold dilution series. Weighed amount of preparations for the preparation of stock solutions were prepared using a Mettler Toledo XP26 analytical balance with an accuracy of 0.001 mg.

The assays of the cytotoxic effects of the tested compounds and the reference compound cisplatin (a commonly used anti-cancer drug) were carried out in 96-hour *in vitro* cultures. For cells growing in suspension, such as MV-4-11 (human biphenotypic leukaemia), KG-1 (human acute myeloid leukaemia), SNU-16 (human gastric cancer), the MTT tetrazolium salt reduction assay was performed [according to: Wietrzyk et al., Anti-cancer drugs, 2007, 18, 447-457] to evaluate the metabolic activity of cancer cells. Now, in order to determine the anti-proliferation activity of all tested compounds versus adherent cell lines: A2780, A2780 Cis (human ovarian cisplatin-sensitive and cisplatin-resistant cancer), A375, Hs294T (human melanoma), MIA PaCa-2 (human pancreatic cancer), NCI-H23, NCI-H358, NCI-H1581 (human lung cancer), PFSK-1 (malignant primitive neuroectodermal tumour), PLC/PRF/5 (human liver cancer), and BALB/3T3 (normal mouse fibroblasts) SRB colorimetric assay was used [according to: Skehan et al., Journal of the National Cancer Institute, 1990, 82, 1107-1112] to measure the inhibition of target cell proliferation based on the determined level of cellular protein. In each experiment, samples containing specific concentrations of the test compound were applied to 384-well plates in triplicate. The experiments were repeated at least 3 times. *In vitro* cytotoxicity tests were performed according to the following procedure:

(a) Cells (derived from an *in vitro* culture) were plated into the wells of a plate at a density of $2.5\times10^3$ or $2\times10^3$ cells in 50 μL culture medium, followd by incubation at 37°C in a humid atmosphere (85-95% humidity) saturated with 5% carbon dioxide ($CO_2$),
(b) after 24 hours, 20 μL of the medium (cell growth control) or the medium containing the test compounds at the specified concentrations was added to the wells,
(c) the plates were incubated for another 72 hours in an incubator (37°C, humid atmosphere (85-95% humidity) saturated with 5% $CO_2$),
(d) after 72 hours of incubation of the cells with the test compounds, the MTT or SRB assay was performed,
(e) in each experiment, samples containing determined concentrations of the test compound were applied in triplicate, and the experiments were reproduced at least 3 times.

MTT reading: 20 μL of MTT solution was added to each well of the 384-well plate. After 4 hours of incubation at 37°C, the medium was aspirated from each well together with MTT, and 80 μl of DMSO was added to the resulting formazan at the bottom of the wells. After 15 minutes of incubation at room temperature, optical density of individual samples was read at 570 nm using a Synergy H4 (universal) plate reader (BioTek Instruments, USA).

SRB reading: 30 μL of cold 50% trichloroacetic acid was added to each well of the 384-well plate. After 60 minutes of incubation at room temperature, the plates were washed 4 times with water and then dried on paper towels. 25 μL of a 0.1% solution of sulforhodamine B (SRB) in 1% acetic acid was then added to each well to stain the cell protein precipitated in the well. After a 30 min. incubation with SRB at room temperature, the plates were washed 4 times with 1% acetic acid and dried again on paper towels. In the next step, 70 μL of 10 mM TRIS buffer was added to each well to dissolve the dye

bound with the cell protein. The optical density of individual samples was read at 540 nm using a Synergy H4 (universal) plate reader (BioTek Instruments, USA).

The inhibition of proliferation was calculated as a percentage for each test compound at a given concentration based on the measurement of the absorbance of individual wells using the following equation:

$$percentage\ of\ inhibition\ of\ proliferation = \left(\left(\frac{A_t - A_m}{A_k - A_m}\right) x100\right) - 100$$

, wherein:

$A_m$ - mean absorbance value of the control medium,
$A_k$ - mean absorbance value of control cells, i.e. untreated cells,
At - mean absorbance value of cells treated with test compounds at a particular concentration.

When calculating the mean absorbance value for a set of wells (untreated cells, cells treated with a specific compound at a specific concentration, control of medium alone), outlier values were rejected on the basis of a 10% CV volatility coefficient. The percentage inhibition of proliferation data was used to determine $IC_{50}$ values, i.e. the concentration of the test compound required to inhibit cell growth by 50% [as per: Nevozhay, PLoS One, 2014, 9, e106186]. Mean $IC_{50}$ values were then calculated based on another 3 to 5 (or more) replicates of the test, together with standard deviation values.

For comparative purposes, analogous studies were conducted using a commonly used anti-cancer drug, i.e. cisplatin. The results of the *in vitro* cytotoxic activity of the test compounds are presented as $IC_{50}$ values expressed in nanomole concentrations (nM), which have been summarised in Table 3.1., 3.2., 4 and 5. All the newly obtained salinomycin derivatives have a very high anti-cancer activity, which in most cases significantly exceeds that of the chemically unmodified salinomycin FLC-00001 (SAL), as well as the reference oncology drug cisplatin. Moreover, most of the newly synthesised salinomycin derivatives have been identified as anti-cancer agents capable of effectively overcoming the drug resistance of the A2780Cis cell line with RI drug resistance index values of <1.0. The majority of the newly obtained salinomycin derivatives are characterised by high selectivity of action towards cancer cells, which is manifested by selectivity coefficients SI >3.0, while some of them have been identified as extremely highly selective compounds (SI >1000.0). This clearly indicates that the effect of the newly synthesised salinomycin derivatives against cancer cells compared to their toxicity is by far superior to that of normal body cells.

The value of the selectivity coefficient SI was calculated using the following equationF:

$$\mathrm{SI} = \frac{IC_{50} for\ normal\ cell\ line\ BALB/3T3}{IC_{50}\ for\ individual\ cancer\ cell\ lines}$$

The value of drug resistance coefficient RI was calculated using the following equation:

$$\mathrm{RI} = \frac{IC_{50}\ for\ drug\ resistant\ cancer\ cell\ line}{IC_{50}\ for\ drug\ sensitive\ cancer\ cell\ line}$$

Table 3.1. Examples of use of compounds according to the invention.

| Test compound | Cancer cells<br>**NCI-H1581** | | **NCI-H23** | | **PFSK-1** | | **A2780** | | **A2780Cis** | | | **A375** | |
|---|---|---|---|---|---|---|---|---|---|---|---|---|---|
| | $IC_{50}$ (nM) | SI | $IC_{50}$ (nM) | SI | $IC_{50}$ (nM) | SI | $IC_{50}$ (nM) | SI | $IC_{50}$ (nM) | SI | RI | $IC_{50}$ (nM) | SI |
| FLC-00001 (SAL) | 68.93 | 9.7 | 355.54 | 1.9 | 335.8 | 2.0 | 119.63 | 5.6 | 67.12 | 10.0 | **0.6** | 574 | 1.2 |
| FLC-00337-1 | **0.18** | **675.6** | 444.91 | 0.3 | 19.37 | 6.3 | **3.10** | 39.2 | **1.98** | 61.5 | **0.6** | 295.66 | 0.4 |
| FLC-00338-1 Na | **2.37** | 69.4 | 620.41 | 0.3 | 53.06 | 3.1 | **5.40** | 30.4 | 10.14 | 16.2 | 1.9 | 354.40 | 0.5 |
| FLC-00339-1 Na | **1.73** | 31.5 | **4.38** | **12.4** | 16.10 | 3.4 | **2.27** | 24.0 | **2.04** | 26.7 | **0.9** | **26.85** | 2.0 |
| FLC-00340-1 Na | **1.76** | 32.8 | **8.64** | 6.7 | **9.37** | 6.2 | **2.41** | 24.0 | **1.84** | 31.4 | **0.8** | **31.24** | 1.8 |
| FLC-00341-1 Na | **0.03** | **2854.0** | 318.21 | 0.3 | 51.56 | 1.9 | **1.62** | 59.2 | **1.30** | 73.9 | **0.8** | 292.69 | 0.3 |
| FLC-00344-1 Na | **3.30** | **322.2** | 284.64 | 3.7 | 112.15 | 9.5 | 21.03 | **50.5** | 24.30 | 43.8 | 1.2 | 2308.32 | 0.5 |
| FLC-00345-1 | **0.03** | **2321.2** | 46.54 | 1.5 | 12.27 | 5.6 | **0.84** | **81.2** | **0.71** | 96.6 | **0.8** | 400.44 | 0.2 |
| FLC-00346-1 | **0.00** | **14980.8** | 60.27 | 1.1 | 25.11 | 2.7 | 0.71 | **96.4** | **0.14** | **487.1** | **0.2** | 392.70 | 0.2 |
| FLC-00347-1 | **0.09** | **788.9** | **5.51** | **13.3** | **4.54** | **16.1** | **0.93** | **79.0** | **0.28** | **264.7** | **0.3** | 91.15 | 0.8 |
| FLC-00348-1 | **8.00** | **353.5** | 971.80 | 2.9 | 379.49 | 7.4 | 57.01 | 49.6 | 46.65 | 60.6 | **0.8** | 2593.54 | 1.1 |
| FLC-00349-1 | **2.88** | 23.5 | 75.15 | 0.9 | 27.60 | 2.4 | **4.50** | 15.0 | **4.56** | 14.8 | 1.0 | 112.76 | 0.6 |
| FLC-00350-1 | **0.18** | **577.2** | 100.68 | 1.0 | **7.25** | **14.2** | **2.46** | 42.0 | **2.30** | 44.9 | 0.9 | 251.07 | 0.4 |
| FLC-00351-1 | **1.92** | 34.7 | 111.98 | 0.6 | 10.73 | 6.2 | **0.45** | **148.7** | **0.53** | **125.6** | 1.2 | 103.91 | 0.6 |
| FLC-00352-1 | **0.06** | **1914.3** | 233.70 | 0.5 | 45.16 | 2.5 | **2.14** | **53.8** | **3.77** | 30.4 | 1.8 | 458.68 | 0.3 |
| FLC-00353-1 | **0.11** | **2107.9** | 409.90 | 0.6 | 13.38 | **16.9** | **1.14** | **199.8** | **1.26** | 180.6 | 1.1 | 106.67 | 2.1 |
| FLC-00358-1 | **0.59** | **377.5** | 112.95 | 2.0 | 32.28 | 6.9 | **3.32** | **67.0** | **2.27** | 98.1 | 0.7 | 207.72 | 1.1 |
| FLC-00359-1 | **0.03** | **2631.9** | 147.86 | 0.5 | 19.37 | 3.8 | **1.62** | 45.1 | **1.12** | 65.3 | 0.7 | 228.50 | 0.3 |
| FLC-00361-1 | n/a | * | n/a | * | n/a | * | n/a | * | n/a | * | * | n/a | * |
| FLC-00362-1 | **3.94** | 39.0 | 62.41 | 2.5 | 38.39 | 4.0 | 11.29 | 13.6 | 11.91 | 12.9 | 1.1 | 281.19 | 0.5 |
| FLC-00363-1 | 0.11 | **583.6** | **2.78** | **22.4** | 4.06 | **15.3** | **2.00** | 31.1 | **1.41** | 44.3 | 0.7 | **37.31** | 1.7 |
| FLC-00364-1 | 2574.58 | * | 4790.99 | * | 5601.69 | * | 2602.87 | * | 1126.26 | * | **0.4** | n/a | * |
| FLC-00365-1 | 696.23 | * | n/a | * | 6203.05 | * | 3481.58 | * | 3017.50 | * | **0.9** | n/a | * |
| FLC-00366-1 | 0.33 | **347.1** | **5.78** | **19.8** | **4.53** | **25.3** | **0.90** | **128.0** | **0.21** | **550.8** | **0.2** | 69.37 | 1.7 |
| FLC-00367-1 | 41.17 | **123.3** | 682.18 | 7.4 | 688.85 | 7.4 | 322.30 | 15.7 | 265.53 | 19.1 | **0.8** | 5924.72 | 0.9 |
| FLC-00368-1 | **3.22** | 40.7 | 18.36 | 7.1 | 18.62 | 7.0 | 3.54 | 37.1 | **2.97** | 44.2 | **0.8** | 89.12 | 1.5 |
| FLC-00369-1 | **0.23** | **357.9** | **5.98** | **13.8** | **5.84** | **14.1** | **5.08** | 16.2 | **1.84** | 44.8 | **0.4** | **49.65** | 1.7 |
| FLC-00371-1 | **< 0.25** | **≈ 369.3** | **4.54** | **20.4** | **5.13** | **18.0** | **2.35** | 39.3 | **0.22** | **413.4** | **0.1** | **43.30** | 2.1 |
| FLC-00373-1 | 3482.13 | * | n/a | * | n/a | * | 6693.62 | * | n/a | * | * | n/a | * |
| FLC-00374-1 | **0.0022** | **31454.6** | **2.92** | **24.1** | **3.83** | **18.4** | **0.45** | **156.6** | 0.11 | **671.0** | **0.2** | **34.18** | 2.1 |
| FLC-00377-1 | **< 0.12** | **≈611.1** | **5.70** | **12.9** | **7.82** | 9.4 | 3.55 | 20.6 | **1.56** | 47.2 | **0.4** | 65.54 | 1.1 |
| FLC-00378-1 | **< 0.21** | ≈ 48.2 | **4.12** | 2.5 | **3.28** | 3.1 | 0.55 | 18.3 | **0.18** | 57.0 | **0.3** | **36.56** | 0.3 |

(continued)

| Test compound | Cancer cells | | | | | | | | | | | | |
|---|---|---|---|---|---|---|---|---|---|---|---|---|---|
| | **NCI-H1581** | | **NCI-H23** | | **PFSK-1** | | **A2780** | | **A2780Cis** | | | **A375** | |
| | $IC_{50}$ (nM) | SI | $IC_{50}$ (nM) | SI | $IC_{50}$ (nM) | SI | $IC_{50}$ (nM) | SI | $IC_{50}$ (nM) | SI | RI | $IC_{50}$ (nM) | SI |
| FLC-00379-1 | **< 1.70** | 57.8 | **8.96** | **11.0** | **6.60** | **14.9** | **4.31** | 22.8 | **0.81** | **121.2** | **0.2** | 68.07 | 1.4 |
| FLC-00380-1 | **0.04** | ≈ **4772.2** | **5.22** | **36.6** | 6.05 | **31.6** | **2.23** | **85.7** | **0.40** | **483.3** | **0.2** | 194.87 | 1.0 |
| FLC-00381-1 | **< 3.27** | ≈ 48.7 | **46.29** | 3.4 | 50.42 | 3.2 | 13.40 | 11.9 | **7.34** | 21.7 | **0.5** | 189.05 | 0.8 |
| FLC-00382-1 | **1.84** | 28.0 | **3.92** | **13.2** | 3.75 | **13.8** | **0.84** | **61.4** | **0.33** | **157.5** | **0.4** | **44.16** | 1.2 |
| FLC-00383-1 | **3.32** | 52.3 | 24.60 | 7.0 | 25.96 | 6.7 | **6.38** | 27.2 | **4.45** | 39.0 | **0.7** | 205.05 | 0.8 |
| FLC-00384-1 | **< 2.37** | ≈ 44.1 | 27.66 | 3.8 | 10.00 | **10.5** | **4.04** | 25.9 | **1.31** | 80.0 | **0.3** | 120.45 | 0.9 |
| Cisplatin | 2865.41 | 0.5 | 2323.13 | 0.6 | 1514.80 | 0.9 | 178.43 | 7.8 | 2607.54 | 0.5 | **14.6** | 409.53 | 3.4 |

NCI-H1581 (human lung cancer), NCI-H23 (human lung cancer), PFSK-1 (malignant primitive neuroectodermal tumour), A2780 (human ovarian cancer), A2780Cis (cisplatin-resistant human ovarian cancer), A375 (human melanoma); n/a - non-active compound, *cannot be calculated;

$IC_{50}$ value - concentration of a compound corresponding to 50% inhibition of growth of cells used in the tests; SI > 1.0 compound has a stronger cytotoxic effect against cancer cells than against normal cells; RI < 2 cells sensitive to the tested compound, RI of 2-10 cells have moderate sensitivity to the test compound, RI > 10 denotes a strong resistance to the test compound;

Table 3.2. Examples of use of compounds according to the invention.

| | Cancer cells | | | | | | | | | | Normal cells |
|---|---|---|---|---|---|---|---|---|---|---|---|
| Test compound | **Hs294T** | | **PLC/PRF/5** | | **SNU-16** | | **KG-1** | | **MIA PaCa-2** | | **BALB/3T3** |
| | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) |
| FLC-00001 (SAL) | 2158.74 | 0.3 | 503.97 | 1.3 | 144.91 | 4.6 | 429.19 | 1.6 | 303.91 | 2.2 | 671.01 |
| FLC-00337-1 | 549.40 | 0.2 | 41.44 | 2.9 | 21.38 | 5.7 | 122.30 | 1.0 | 56.04 | 2.2 | 121.62 |
| FLC-00338-1 Na | 1867.31 | 0.1 | 61.57 | 2.7 | 39.20 | 4.2 | 221.60 | 0.7 | 58.60 | 2.8 | 164.34 |
| FLC-00339-1 Na | nd | * | **10.81** | 5.0 | 17.48 | 3.1 | **11.06** | 4.9 | **17.13** | 3.2 | 54.47 |
| FLC-00340-1 Na | nd | * | 19.10 | 3.0 | 16.69 | 3.5 | 24.37 | 2.4 | 33.89 | 1.7 | 57.72 |
| FLC-00341-1 Na | 1637.89 | 0.1 | 54.54 | 1.8 | 27.11 | 3.5 | 45.88 | 2.1 | 55.44 | 1.7 | 96.17 |
| FLC-00344-1 Na | 2569.61 | 0.4 | 1286.90 | 0.8 | 106.09 | 10.0 | 286.33 | 3.7 | 382.51 | 2.8 | 1063.18 |
| FLC-00345-1 | 396.18 | 0.2 | 27.99 | 2.4 | 26.02 | 2.6 | 39.17 | 1.7 | 111.06 | 0.6 | 68.36 |
| FLC-00346-1 | 388.27 | 0.2 | **5.80** | 11.9 | **4.52** | **15.2** | 20.85 | 3.3 | 43.30 | 1.6 | 68.81 |
| FLC-00347-1 | 102.62 | 0.7 | **5.87** | 12.5 | 13.50 | 5.4 | **14.13** | 5.2 | 44.29 | 1.7 | 73.24 |
| FLC-00348-1 | 3317.75 | 0.9 | 1149.23 | 2.5 | 537.45 | 5.3 | 188.17 | **15.0** | 2320.87 | 1.2 | 2826.77 |
| FLC-00349-1 | 107.50 | 0.6 | 24.07 | 2.8 | 20.68 | 3.3 | 24.66 | 2.7 | 66.34 | 1.0 | 67.51 |
| FLC-00350-1 | 418.86 | 0.2 | 23.03 | 4.5 | 18.18 | 5.7 | 35.85 | 2.9 | 37.40 | 2.8 | 103.30 |
| FLC-00351-1 | 359.63 | 0.2 | 22.29 | 3.0 | 24.49 | 2.7 | 23.67 | 2.8 | 22.04 | 3.0 | 66.59 |
| FLC-00352-1 | 415.53 | 0.3 | 32.86 | 3.5 | 52.83 | 2.2 | 30.04 | 3.8 | 50.88 | 2.3 | 114.86 |
| FLC-00353-1 | 612.16 | 0.4 | 31.49 | 7.2 | 88.60 | 2.6 | 34.32 | 6.6 | 28.93 | 7.8 | 226.74 |
| FLC-00358-1 | 1644.29 | 0.1 | 52.79 | 4.2 | 67.89 | 3.3 | 67.73 | 3.3 | 45.60 | 4.9 | 222.75 |
| FLC-00359-1 | 211.65 | 0.3 | 38.64 | 1.9 | 9.57 | 7.6 | **13.09** | 5.6 | 37.70 | 1.9 | 72.97 |
| FLC-00361-1 | n/a | * | n/a | * | n/a | * | n/a | * | n/a | * | n/a |
| FLC-00362-1 | 473.03 | 0.3 | 32.05 | 4.8 | **16.97** | 9.0 | 36.83 | 4.2 | 73.83 | 2.1 | 153.47 |
| FLC-00363-1 | **27.31** | 2.3 | 13.02 | 4.8 | **4.46** | 14.0 | **11.72** | 5.3 | **19.31** | 3.2 | 62.25 |
| FLC-00364-1 | n/a | * | 4477.45 | * | 5383.75 | * | 3704.19 | * | n/a | * | n/a |
| FLC-00365-1 | n/a | * | n/a | * | 5833.00 | * | 7997.98 | * | n/a | * | n/a |
| FLC-00366-1 | 213.76 | 0.5 | 5.62 | **20.4** | **8.64** | 13.3 | 40.61 | 2.8 | 44.97 | 2.5 | 114.56 |
| FLC-00367-1 | n/a | * | 939.71 | 5.4 | 367.91 | 13.8 | 665.56 | 7.6 | 3222.50 | 1.6 | 5075.50 |
| FLC-00368-1 | 152.31 | 0.9 | 74.24 | 1.8 | 16.02 | 8.2 | 28.20 | 4.7 | 88.09 | 1.5 | 131.18 |
| FLC-00369-1 | 105.62 | 0.8 | **12.43** | 6.6 | 16.00 | 5.1 | **11.66** | 7.1 | 44.72 | 1.8 | 82.32 |
| FLC-00371-1 | 67.71 | 1.4 | **12.83** | 7.2 | **14.85** | 6.2 | 22.05 | 4.2 | 31.29 | 3.0 | 92.34 |
| FLC-00373-1 | n/a | * | n/a | * | n/a | * | 9061.69 | * | n/a | * | n/a |
| FLC-00374-1 | 73.13 | 1.0 | **8.83** | 8.0 | 4.03 | **17.5** | **12.50** | 5.6 | 28.69 | 2.5 | 70.46 |
| FLC-00377-1 | 94.80 | 0.8 | 18.76 | 3.9 | 51.77 | 1.4 | 18.33 | 4.0 | 41.60 | 1.8 | 73.33 |
| FLC-00378-1 | **29.38** | 0.3 | **2.58** | 3.9 | **3.21** | 3.2 | 19.08 | 0.5 | 18.43 | 0.5 | 10.12 |

(continued)

| Test compound | Cancer cells | | | | | | | | | | Normal cells |
|---|---|---|---|---|---|---|---|---|---|---|---|
| | **Hs294T** | | **PLC/PRF/5** | | **SNU-16** | | **KG-1** | | **MIA PaCa-2** | | **BALB/3T3** |
| | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) |
| FLC-00379-1 | 107.89 | 0.9 | 22.05 | 4.5 | 17.97 | 5.5 | 39.02 | 2.5 | 52.57 | 1.9 | 98.18 |
| FLC-00380-1 | 177.68 | 1.1 | 19.89 | 9.6 | **6.60** | **28.9** | **6.44** | **29.6** | 48.22 | 4.0 | 190.89 |
| FLC-00381-1 | 138.21 | 1.2 | 62.44 | 2.5 | 63.76 | 2.5 | 36.40 | 4.4 | 69.63 | 2.3 | 159.12 |
| FLC-00382-1 | 61.13 | 0.8 | **5.15** | 10.0 | **5.15** | 10.0 | **9.84** | 5.2 | 36.52 | 1.4 | 51.59 |
| FLC-00383-1 | 243.57 | 0.7 | **3.35** | **51.7** | 20.26 | 8.6 | 30.62 | 5.7 | 47.66 | 3.6 | 173.25 |
| FLC-00384-1 | 141.24 | 0.7 | 28.22 | 3.7 | 21.11 | 5.0 | 19.72 | 5.3 | 62.65 | 1.7 | 104.74 |
| Cisplatin | 735.80 | 1.9 | 1578.23 | 0.9 | 2326.60 | 0.6 | 325.53 | 4.3 | 2361.58 | 0.6 | 1393.73 |

Hs294T (human melanoma), PLC/PRF/5 (human liver cancer), SNU-16 (human gastric cancer), KG-1 (human acute myeloid leukaemia), MIA PaCa-2 (human pancreatic cancer), BALB/3T3 (normal mouse fibroblasts); n/a -non-active compound, nt. - compound not tested/not tested with a particular cell line, * cannot be calculated; $IC_{50}$ value - concentration of a compound corresponding to 50% inhibition of growth of cells used in the tests; SI > 1.0 compound has a stronger cytotoxic effect against cancer cells than against normal cells;

Table 4. Examples of use of compounds according to the invention.

| Test compounds | Cancer cells | | | | | | | | | | | | | | Normal cells |
|---|---|---|---|---|---|---|---|---|---|---|---|---|---|---|---|
| | **A2780Cis** | | **Hs294T** | | **PLC/PRF/5** | | **SNU-16** | | **MV4-11** | | **MIA PaCa-2** | | **NCI-H358** | | **BALB3T3** |
| | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) |
| FLC-00001 (SAL) | 67.12 | 10.0 | 2158.74 | 0.3 | 503.97 | 1.3 | 144.91 | 4.6 | 69.10 | 9.7 | 303.91 | 2.2 | 600.34 | 1.12 | 671.01 |
| FLC-00370-1 · Na | 32.71 | **134.5** | 1859.05 | 2.4 | 144.09 | **30.5** | 1522.60 | 2.9 | 41.16 | **106.9** | 489.38 | 9.0 | 826.30 | **5.33** | 4400.85 |
| FLC-00376-1 · Na | 436.43 | 10.6 | n/a | * | 1361.60 | 3.4 | n/a | * | 662.82 | 7.0 | 1846.32 | 2.5 | 5308.57 | 0.87 | 4643.88 |
| FLC-00385-1 | **< 0.1** | **≈ 219.0** | 161.68 | 0.1 | 0.55 | **39.7** | 20.87 | 1.0 | 0.17 | **125.5** | **5.93** | 3.7 | **36.20** | 0.60 | 21.90 |
| FLC-00386-1 | **< 0.3** | **~187.3** | 147.04 | 0.4 | 10.20 | 5.5 | **10.08** | 5.6 | **< 0.31** | **≈ 181.3** | 11.31 | 5.0 | 51.30 | 1.10 | 56.20 |
| FLC-00387-1 | **< 0.3** | ≈ 81.5 | 161.96 | 0.2 | 0.70 | **34.8** | **10.16** | 2.4 | **< 0.55** | ~- 44.4 | **9.71** | 2.5 | **44.43** | 0.55 | 24.44 |
| FLC-00388-1 · Na | 18.32 | 36.0 | 1544.89 | 0.4 | 47.45 | 13.9 | 125.62 | 5.3 | 41.01 | 16.1 | 246.44 | 2.7 | 533.22 | 1.24 | 660.37 |
| FLC-00389-1 · Na | 69.43 | 12.7 | 655.63 | 1.3 | 276.48 | 3.2 | 607.62 | 1.5 | 32.66 | 27.1 | 438.61 | 2.0 | 1091.97 | 0.81 | 885.07 |
| FLC-00390-1 | 0.27 | **153.4** | 218.62 | 0.2 | 7.57 | 5.4 | 16.21 | 2.5 | **0.18** | **224.8** | 26.62 | 1.5 | 51.38 | 0.79 | 40.65 |
| FLC-00391-1 | 108.06 | 33.0 | 5874.58 | 0.6 | 584.29 | 6.1 | 1325.70 | 2.7 | 348.77 | 10.2 | 3087.80 | 1.2 | 5152.86 | 0.69 | 3569.49 |
| FLC-00392-1 · Na | 3746.67 | * | n/a | * | 8326.12 | * | n/a | * | 1963.89 | * | n/a | * | n/a | * | n/a |
| FLC-00393-1 | 15.03 | 23.7 | 293.81 | 1.2 | 44.83 | 7.9 | 86.47 | 4.1 | 24.60 | 14.5 | 165.84 | 2.1 | 319.55 | 1.11 | 356.03 |
| FLC-00394-1 | **1.24** | 58.8 | 86.01 | 0.8 | 16.89 | 4.3 | 23.92 | 3.0 | **3.31** | 22.0 | 36.55 | 2.0 | 132.04 | 0.55 | 72.83 |

(continued)

| Test compounds | Cancer cells | | | | | | | | | | | | | | Normal cells |
|---|---|---|---|---|---|---|---|---|---|---|---|---|---|---|---|
| | **A2780Cis** | | **Hs294T** | | **PLC/PRF/5** | | **SNU-16** | | **MV4-11** | | **MIA PaCa-2** | | **NCI-H358** | | **BALB3T3** |
| | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) |
| FL-C-00395-1 · Na | **1.78** | 43.8 | **47.29** | 1.6 | 37.53 | 2.1 | 103.66 | 0.8 | **0.44** | **177.2** | 33.12 | 2.4 | 120.80 | 0.65 | 77.95 |
| FLC-00396-1 | **<0.16** | ≈ **1343.2** | 345.84 | 0.6 | 30.74 | 7.0 | 39.34 | 5.5 | **2.96** | 72.7 | 31.77 | 6.8 | 131.64 | 1.63 | 214.92 |
| FLC-00410-1 · Na | 7704.50 | * | n/a | * | n/a | * | n/a | * | 3741.80 | * | n/a | * | n/a | * | n/a |
| FLC-00423-1 · Na | 37.50 | 25.1 | 1296.25 | 0.7 | 91.22 | 10.3 | 1758.51 | 0.5 | 22.07 | 42.6 | 249.27 | 3.8 | 439.53 | 2.14 | 940.55 |
| FLC-00424-1 · Na | **< 0.46** | ≈ **249.0** | 684.93 | 0.2 | 5.01 | 22.9 | 219.60 | 0.5 | **< 1.03** | ≈ **111.2** | 38.35 | 3.0 | **48.68** | 2.35 | 114.53 |
| FLC-00425-1 · Na | **6.41** | 30.7 | 362.21 | 0.5 | 26.46 | 7.4 | 419.77 | 0.5 | **0.71** | **276.1** | 268.70 | 0.7 | 233.96 | 0.84 | 196.97 |
| FLC-00427-1 · Na | 0.80 | **1121.5** | 549.15 | 1.6 | 68.96 | 13.1 | 115.31 | 7.8 | **1.36** | 664.3 | 82.44 | 10.9 | 406.73 | 2.22 | 901.73 |
| FLC-00428-1 · Na | **4.36** | 56.0 | 172.74 | 1.4 | 7.85 | 31.1 | 114.47 | 2.1 | 1.34 | **182.9** | 36.89 | 6.6 | 57.30 | 4.27 | 244.42 |
| FLC-00429-1 · Na | **6.66** | 14.7 | 115.27 | 0.8 | 26.98 | 3.6 | 168.27 | 0.6 | 4.05 | 24.2 | 39.76 | 2.5 | 76.10 | 1.29 | 97.95 |
| FLC-00430-1 · Na | n/a | * | n/a | * | n/a | * | n/a | * | n/a | * | n/a | * | n/a | * | n/a |
| FLC-00431-1 · Na | **4.74** | 14.4 | 115.10 | 0.6 | **5.80** | 11.8 | 143.22 | 0.5 | **0.51** | **133.8** | 28.89 | 2.4 | 56.11 | 1.22 | 68.24 |

(continued)

| Test compounds | Cancer cells | | | | | | | | | | | | | | Normal cells |
|---|---|---|---|---|---|---|---|---|---|---|---|---|---|---|---|
| | **A2780Cis** | | **Hs294T** | | **PLC/PRF/5** | | **SNU-16** | | **MV4-11** | | **MIA PaCa-2** | | **NCI-H358** | | **BALB3T3** |
| | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) |
| FL-C-00436-1 · Na | 21.71 | 32.0 | 83.21 | 8.3 | 39.62 | 17.5 | 736.73 | 0.9 | **0.45** | **1535.0** | 35.52 | **19.6** | 306.17 | 2.27 | 694.57 |
| FLC-00437-1 · Na | 4.70 | 40.8 | 264.47 | 0.7 | 42.32 | 4.5 | 342.40 | 0.6 | 5.11 | 37.5 | 45.66 | 4.2 | 184.23 | 1.04 | 191.98 |
| FLC-00438-1 · Na | 2896.54 | * | n/a | * | 7276.53 | * | n/a | * | 2497.81 | * | 6464.79 | * | n/a | * | n/a |
| FLC-00439-1 · Na | < 0.3 | ≈ **582.6** | 131.25 | 1.3 | 10.14 | 17.2 | 208.52 | 0.8 | 0.67 | 260.9 | 31.72 | 5.5 | 40.12 | 4.36 | 174.77 |
| FLC-00440-1 · Na | 4.41 | 26.1 | 130.10 | 0.9 | 9.50 | 12.1 | 400.47 | 0.3 | 3.52 | 32.6 | 43.60 | 2.6 | 60.16 | 1.91 | 114.93 |
| FLC-00441-1 · Na | 43.83 | 25.7 | 969.24 | 1.2 | 207.96 | 5.4 | 2599.72 | 0.4 | 26.47 | 42.5 | 372.99 | 3.0 | 929.80 | 1.21 | 1124.96 |
| FLC-00442-1 · Na | 23.32 | 32.3 | 1714.84 | 0.4 | 53.85 | 14.0 | 1116.82 | 0.7 | 19.37 | 38.9 | 102.48 | 7.4 | 340.29 | 2.22 | 754.13 |
| FLC-00443-1 · Na | 176.38 | 23.3 | 3969.64 | 1.0 | 476.86 | 8.6 | 6190.00 | 0.7 | 175.37 | 23.4 | 553.92 | 7.4 | 2619.32 | 1.57 | 4110.90 |
| FLC-00444-1 · Na | 1.50 | 98.9 | 627.16 | 0.2 | **4.00** | **36.9** | 105.27 | 1.4 | **1.50** | 98.9 | 21.34 | 6.9 | 53.69 | 2.75 | 147.89 |
| FLC-00445-1 · Na | 2.97 | **232.8** | 762.53 | 0.9 | 15.78 | **43.8** | 191.90 | 3.6 | **2.60** | **265.9** | 33.55 | **20.6** | 103.98 | 6.65 | 691.40 |
| FLC-00446-1 · Na | 0.71 | **294.5** | 189.11 | 1.1 | 37.91 | 5.5 | 261.59 | 0.8 | **0.70** | **298.7** | 26.77 | 7.8 | 50.80 | 4.12 | 209.07 |

(continued)

| Test compounds | Cancer cells | | | | | | | | | | | | | | Normal cells |
|---|---|---|---|---|---|---|---|---|---|---|---|---|---|---|---|
| | **A2780Cis** | | **Hs294T** | | **PLC/PRF/5** | | **SNU-16** | | **MV4-11** | | **MIA PaCa-2** | | **NCI-H358** | | **BALB3T3** |
| | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) |
| FL-C-00447-1 · Na | 11.58 | 9.2 | 245.02 | 0.4 | 15.42 | 6.9 | 617.22 | 0.2 | **1.86** | 57.1 | 40.34 | 2.6 | 59.46 | 1.78 | 106.13 |
| FLC-00450-1 · Na | 4.71 | 31.8 | 401.88 | 0.4 | 29.04 | 5.2 | 355.16 | 0.4 | **4.18** | 35.9 | 36.12 | 4.2 | 196.15 | 0.76 | 149.98 |
| FLC-00451-1 · Na | 2.13 | 96.0 | 542.36 | 0.4 | 41.95 | 4.9 | 125.73 | 1.6 | **1.60** | **127.9** | 14.68 | 13.9 | 306.87 | 0.67 | 204.21 |
| FLC-00452-1 · Na | 25.66 | 31.9 | 2464.39 | 0.3 | 119.45 | 6.9 | 438.73 | 1.9 | 27.68 | 29.6 | 106.31 | 7.7 | 538.53 | 1.52 | 818.95 |
| FLC-00454-1 · Na | 5.32 | 58.9 | 1037.86 | 0.3 | 63.13 | 5.0 | 103.40 | 3.0 | 17.61 | 17.8 | 45.17 | 6.9 | 434.13 | 0.72 | 313.30 |
| FLC-00455-1 · Na | 1.39 | 232.6 | 140.33 | 2.3 | 60.74 | 5.3 | 165.32 | 2.0 | **2.87** | **112.3** | 32.95 | 9.8 | 54.54 | 5.91 | 322.52 |
| FLC-00456-1 · Na | 2848.04 | * | 8693.18 | * | 7293.23 | * | n/a | * | 3425.91 | * | n/a | * | n/a | * | n/a |
| FLC-00457-1 · Na | 9.66 | 76.0 | 2532.60 | 0.3 | 122.11 | 6.0 | 551.27 | 1.3 | 18.04 | 40.7 | 53.66 | 13.7 | 446.00 | 1.65 | 734.06 |
| FLC-00458-1 · Na | 24.51 | 29.6 | 1588.47 | 0.5 | 84.39 | 8.6 | 323.26 | 2.2 | 23.25 | 31.3 | 61.88 | 11.7 | 368.32 | 1.97 | 726.78 |
| FLC-00459-1 · Na | 34.70 | 30.7 | 786.45 | 1.4 | 92.23 | 11.6 | 535.35 | 2.0 | 33.58 | 31.8 | 78.27 | 13.6 | 530.70 | 2.01 | 1066.80 |
| FLC-00460-1 · Na | **0.98** | 94.4 | 309.41 | 0.3 | 17.56 | 5.3 | 130.17 | 0.7 | **1.86** | 50.0 | 13.58 | 6.8 | 281.83 | 0.33 | 92.83 |

(continued)

| Test compounds | Cancer cells | | | | | | | | | | | | | | Normal cells |
|---|---|---|---|---|---|---|---|---|---|---|---|---|---|---|---|
| | **A2780Cis** | | **Hs294T** | | **PLC/PRF/5** | | **SNU-16** | | **MV4-11** | | **MIA PaCa-2** | | **NCI-H358** | | **BALB3T3** |
| | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) | SI | $IC_{50}$ (nM) |
| FL-C-00461-1 · Na | **0.21** | **459.1** | 173.91 | 0.5 | 13.73 | 6.9 | 28.27 | 3.4 | **1.92** | 49.5 | 13.66 | 6.9 | 62.73 | 1.51 | 94.87 |
| FLC-00462-1 · Na | 22.50 | 24.5 | 1807.58 | 0.3 | 63.83 | 8.6 | 339.30 | 1.6 | 13.65 | 40.4 | 52.02 | 10.6 | 397.01 | 1.39 | 551.79 |
| Cisplatin | 2607.54 | 0.5 | 735.80 | 1.9 | 1578.23 | 0.9 | 2326.60 | 0.6 | 165.55 | 8.4 | 2361.58 | 0.6 | 2545.24 | 0.55 | 1393.73 |

A2780Cis (human cisplatin-resistant ovarian cancer), Hs294T (human melanoma), PLC/PRF/5 (human liver cancer), SNU-16 (human gastric cancer), MV-4-11 (human biphenotypic leukemia), MIA PaCa-2 (human pancreatic cancer), NCI-H358 (human lung cancer), BALB/3T3 (normal mouse fibroblasts), n/a - non-active compound, * cannot be calculated;

$IC_{50}$ value - concentration of a compound corresponding to 50% inhibition of growth of cells used in the tests; SI > 1.0 compound has a stronger cytotoxic effect against cancer cells than against normal cells;

Table 5. Examples of use of compounds according to the invention.

| Test compound | Cancer cells A2780Cis IC$_{50}$ (nM) | A2780Cis SI | MV4-11 IC$_{50}$ (nM) | MV4-11 SI | NCI-H1581 IC$_{50}$ (nM) | NCI-H1581 SI | Normal cells BALB3T3 IC$_{50}$ (nM) |
|---|---|---|---|---|---|---|---|
| FLC-00001 (SAL) | 67.12 | 10.0 | 69.10 | 9.7 | 68.93 | 9.7 | 671.01 |
| FLC-00366-1 | **0.21** | **550.8** | nd | * | **0.33** | **347.1** | 114.56 |
| FLC-00524-1 · Na | 38.9 | 20.6 | 34.0 | 23.5 | 55.0 | 14.5 | 800.0 |
| FLC-00374-1 | **0.11** | **671.0** | nd | * | **0.0022** | **31454.6** | 70.46 |
| FLC-00521-1 · Na | 600.0 | 6.2 | 300.0 | 12.3 | 500.0 | 7.4 | 3700.0 |
| FLC-00463-1 · Na | **0.72** | **266.3** | **1.98** | **96.5** | **0.11** | **1694.6** | 190.64 |
| FLC-00464-1 · Na | **< 0.28** | **≈ 305.7** | **0.46** | **187.9** | **< 0.03** | **≈ 2853.1** | 85.59 |
| FLC-00465-1 · Na | 3071.09 | * | 3168.02 | * | 4832.61 | * | n/a |
| FLC-00466-1 · Na | **< 1.84** | ≈ 58.2 | **< 3.10** | ≈ 34.6 | **< 0.08** | **≈ 1338.9** | 107.11 |
| FLC-00467-1 · Na | **<0.0001** | **≈ 853700** | **<0.0001** | **≈ 853700** | **<0.0001** | **≈ 853700** | 85.37 |
| FLC-00468-1 · Na | **1.01** | 48.1 | **2.60** | 18.6 | **0.33** | **145.4** | 48.34 |
| FLC-00469-1 · Na | **0.0027** | **14630.8** | **0.012** | **3330.5** | **<0.01** | **≈ 3896.7** | 38.97 |
| FLC-00470-1 · Na | **0.03** | **982.4** | **0.27** | **115.3** | **0.31** | 97.3 | 30.55 |
| FLC-00471-1 · Na | 4292.63 | * | 4729.32 | * | 5035.05 | * | n/a |
| FLC-00472-1 · Na | **0.40** | **158.4** | **2.47** | 25.3 | **0.05** | **1360.6** | 62.59 |
| FLC-00473-1 · Na | **0.72** | **179.1** | **2.05** | 62.7 | **0.11** | **1169.4** | 128.64 |
| FLC-00474-1 · Na | **0.46** | 80.3 | **0.73** | 50.6 | **0.03** | **1227.4** | 36.82 |
| FLC-00475-1 · Na | **< 0.29** | **≈ 216.3** | **< 0.47** | **≈ 133.5** | **< 0.03** | **≈ 2090.9** | 62.73 |
| FLC-00476-1 · Na | **0.48** | 44.8 | **0.44** | 48.7 | **0.11** | **197.6** | 21.38 |
| FLC-00477-1 · Na | **3.54** | 40.4 | **3.11** | 46.1 | **4.49** | 31.9 | 143.21 |
| FLC-00478-1 · Na | 63.65 | 25.9 | 180.43 | 9.2 | 50.95 | 32.4 | 1651.03 |
| FLC-00479-1 · Na | 272.34 | 15.8 | 342.83 | 12.6 | 337.00 | 12.8 | 4303.62 |
| FLC-00480-1 · Na | **4.25** | 43.6 | 19.05 | 9.7 | **3.37** | 55.1 | 185.41 |
| FLC-00481-1 · Na | 219.64 | 17.4 | 319.70 | 12.0 | 275.39 | 13.9 | 3825.86 |
| FLC-00483-1 · Na | 250.27 | 21.2 | 338.62 | 15.7 | 341.24 | 15.6 | 5308.10 |
| FLC-00484-1 · Na | **0.25** | 95.4 | **0.59** | 40.7 | **0.12** | **195.5** | 23.86 |
| FLC-00485-1 · Na | **4.62** | 23.0 | 20.04 | 5.3 | **3.90** | 27.3 | 106.41 |
| FLC-00486-1 · Na | **0.05** | **873.6** | **1.27** | 36.8 | **0.06** | **832.0** | 46.59 |
| FLC-00487-1 · Na | 45.91 | 79.6 | 132.73 | 27.5 | 46.01 | 79.4 | 3652.57 |
| FLC-00489-1 · Na | 175.46 | 28.0 | 337.98 | 14.6 | 323.25 | 15.2 | 4919.19 |
| FLC-00491-1 · Na | **5.19** | 79.2 | **6.30** | 65.3 | **4.75** | 86.5 | 411.12 |
| FLC-00493-1 · Na | **2.14** | 26.9 | **5.43** | 10.6 | **4.68** | 12.3 | 57.71 |
| FLC-00494-1 · Na | **4.45** | 29.2 | **3.40** | 38.3 | **3.98** | 32.7 | 130.12 |
| FLC-00497-1 · Na | 277.55 | 8.2 | 278.48 | 8.2 | 450.16 | 5.1 | 2281.33 |
| FLC-00498-1 · Na | 757.57 | 6.6 | 1562.39 | 3.2 | 687.53 | 7.3 | 5008.36 |
| FLC-00501-1 · Na | 2231.47 | 1.3 | 1743.87 | 1.6 | 1174.58 | 2.4 | 2828.27 |
| FLC-00502-1 · Na | **1.76** | 52.7 | 3.30 | 28.1 | **1.43** | 64.8 | 92.72 |
| FLC-00503-1 · Na | **2.49** | 31.7 | 3.65 | 21.6 | **1.40** | 56.3 | 78.77 |
| FLC-00504-1 · Na | 2.99 | 30.5 | **4.40** | 20.7 | 2.69 | 34.0 | 91.28 |
| FLC-00505-1 · Na | 7.61 | 15.2 | 14.04 | 8.3 | 4.63 | 25.1 | 116.05 |
| FLC-00506-1 · Na | 0.92 | 100.3 | 2.62 | 35.3 | **1.32** | 70.0 | 92.48 |
| FLC-00509-1 · Na | 16.71 | 14.7 | 22.64 | 10.9 | 19.69 | 12.5 | 246.29 |
| FLC-00514-1 · Na | 8.51 | 15.7 | 11.43 | 11.7 | 6.17 | 21.7 | 133.83 |
| FLC-00515-1 · Na | 356.87 | 10.6 | 399.57 | 9.5 | 1028.93 | 3.7 | 3789.14 |
| FLC-00518-1 · Na | 3217.97 | * | 3493.56 | * | 7640.22 | * | n/a |
| FLC-00519-1 · Na | 1191.08 | 5.3 | 1589.19 | 4.0 | 1406.26 | 4.5 | 6365.28 |
| FLC-00528-1 · Na | 0.30 | 220.3 | 0.65 | 102.4 | < 0.25 | ≈ **266.8** | 66.69 |
| FLC-00530-1 · Na | 2.60 | 24.0 | **6.42** | 9.7 | 2.12 | 29.5 | 62.52 |

(continued)

| Test compound | Cancer cells A2780Cis | | Cancer cells MV4-11 | | Cancer cells NCI-H1581 | | Normal cells BALB3T3 |
|---|---|---|---|---|---|---|---|
| | $IC_{50}$ (nM) | SI | $IC_{50}$ (nM) | SI | $IC_{50}$ (nM) | SI | $IC_{50}$ (nM) |
| FLC-00531-1 · Na | 11.01 | 13.2 | 23.31 | 6.2 | 7.84 | 18.5 | 144.95 |
| FLC-00534-1 · Na | **1.28** | 50.7 | **1.53** | 42.4 | 5.05 | 12.8 | 64.89 |
| FLC-00536-1 · Na | 5.04 | 50.0 | 28.04 | 9.0 | 5.24 | 48.1 | 252.15 |
| FLC-00537-1 · Na | 19.90 | 23.9 | 26.04 | 18.3 | 63.90 | 7.4 | 475.39 |
| FLC-00538-1 · Na | 42.16 | 22.4 | 102.98 | 9.2 | 165.83 | 5.7 | 942.88 |
| FLC-00539-1 · Na | 7.06 | 46.1 | 27.26 | 11.9 | 1.53 | 212.7 | 325.73 |
| FLC-00540-1 · Na | 36.98 | 24.1 | 53.86 | 16.5 | 202.97 | 4.4 | 890.82 |
| FLC-00541-1 · Na | < 0.22 | **≈ 393.3** | **< 0.28** | **≈ 309.0** | **0.001** | **86528.3** | 86.53 |
| FLC-00542-1 · Na | 18.22 | 46.1 | 24.76 | 33.9 | 0.96 | 875.7 | 839.26 |
| FLC-00543-1 · Na | 67.02 | 15.2 | 173.97 | 5.9 | 29.54 | 34.5 | 1017.78 |
| FLC-00544-1 · Na | 3.98 | 60.1 | 13.68 | 17.5 | 10.18 | 23.5 | 239.33 |
| FLC-00545-1 · Na | 0.57 | 186.6 | 1.68 | 62.8 | 0.11 | **983.4** | 105.72 |
| FLC-00546-1 · Na | 111.85 | 19.5 | 257.76 | 8.5 | 56.99 | 38.3 | 2180.46 |
| FLC-00547-1 · Na | < 1.38 | ≈ 56.2 | 2.21 | 35.2 | < 0.15 | ≈ 517.2 | 77.59 |
| FLC-00548-1 · Na | 42.47 | 17.0 | 64.50 | 11.2 | 35.18 | 20.5 | 722.61 |
| FLC-00550-1 · Na | 10.63 | 42.1 | 16.86 | 26.6 | 3.87 | 115.8 | 447.78 |
| FLC-00551-1 · Na | 63.64 | 12.5 | 80.96 | 9.8 | 34.85 | 22.7 | 792.37 |
| FLC-00552-1 · Na | 4.02 | 46.7 | 5.84 | 32.1 | **2.44** | 76.7 | 187.48 |
| FLC-00553-1 · Na | 5.16 | 52.6 | 13.54 | 20.1 | **1.87** | 145.0 | 271.44 |
| FLC-00554-1 · Na | 417.94 | 13.9 | 380.11 | 15.3 | 262.89 | 22.1 | 5818.56 |
| FLC-00557-1 · Na | 21.02 | 64.2 | 82.66 | 16.3 | 33.54 | 40.2 | 1349.45 |
| FLC-00559-1 · Na | 329.78 | 16.2 | 299.93 | 17.8 | 335.69 | 15.9 | 5342.08 |
| FLC-00560-1 · Na | 282.84 | 18.8 | 338.59 | 15.7 | 251.89 | 21.2 | 5329.82 |
| FLC-00561-1 · Na | **3.23** | **119.2** | **6.09** | 63.2 | **0.58** | **659.8** | 384.87 |
| FLC-00562-1 · Na | 3006.61 | * | 3067.17 | * | 2714.96 | * | n/a |
| FLC-00563-1 · Na | 3118.68 | * | 3245.04 | * | 2227.57 | * | n/a |
| FLC-00564-1 · Na | 38.39 | 56.0 | 76.52 | 28.1 | 25.59 | 84.1 | 2151.74 |
| FLC-00565-1 · Na | **1.70** | 185.7 | **5.44** | 58.2 | 0.17 | **1861.1** | 316.39 |
| FLC-00566-1 · Na | 122.38 | 47.6 | 254.82 | 22.8 | 38.25 | **152.2** | 5821.03 |
| FLC-00567-1 · Na | 15.66 | 54.2 | 33.96 | 25.0 | 6.32 | **134.1** | 848.21 |
| FLC-00569-1 · Na | 19.45 | 41.3 | 28.85 | 27.8 | 4.25 | **189.1** | 802.95 |
| FLC-00570-1 · Na | 30.04 | 21.2 | 43.68 | 14.6 | 23.24 | 27.4 | 636.30 |
| FLC-00571-1 · Na | 6.28 | 84.3 | 22.12 | 23.9 | 2.57 | **206.0** | 529.34 |
| FLC-00573-1 · Na | 48.66 | 38.7 | 158.20 | 11.9 | 29.82 | 63.2 | 1883.30 |
| FLC-00577-1 · Na | 29.12 | 30.0 | 42.76 | 20.4 | 24.19 | 36.1 | 873.82 |
| FLC-00578-1 · Na | **1.94** | **184.9** | **5.99** | 59.8 | **0.72** | **497.0** | 357.87 |
| FLC-00579-1 · Na | 71.42 | 22.3 | 187.98 | 8.5 | 27.90 | 57.1 | 1594.11 |
| FLC-00580-1 · Na | **2.24** | 94.4 | **6.51** | 32.5 | **1.36** | **156.0** | 211.36 |
| FLC-00593-1 · Na | **<0.01** | **≈ 7220** | **<0.01** | **≈ 7220** | **<0.01** | **≈ 7220** | 72.2 |
| FLC-00606-1 · Na | **<0.01** | **≈ 5240** | **<0.01** | **≈ 5240** | **<0.01** | **≈ 5240** | 52.4 |
| FLC-00771-1 · Na | **<0.01** | **≈ 8000** | **<0.01** | **≈ 8000** | **<0.01** | **≈ 8000** | 80.0 |
| Cisplatin | 2865.41 | 0.5 | 165.55 | 8.4 | 2865.41 | 0.5 | 1393.73 |

A2780Cis (human cisplatin-resistant ovarian cancer), MV-4-11 (human biphenotypic leukaemia), NCI-H1581 (human lung cancer), BALB/3T3 (normal mouse fibroblasts), n/a - non-active compound, nt. - compound not tested/not tested with a particular cell line, * cannot be calculated;
$IC_{50}$ value - concentration of a compound corresponding to 50% inhibition of growth of cells used in the tests; SI > 1.0 compound has a stronger cytotoxic effect against cancer cells than against normal cells;

Tables 3.1, 3.2, 4 and 5 clearly show that the compounds according to the invention have an anti-cancer activity against cancer cells from various tissues and organs. Moreover, the derivatives obtained have an anti-proliferative effect that is several times more potent compared to that of the parent compound, e.g. compounds with the formula FLC-00341, FLC-00374, FLC-00427, FLC-00345, FLC-00346, FLC-00347, FLC-00467, FLC-00463, FLC-00464, FLC-00469, FLC-00470, FLC-00472, FLC-00473, FLC-00486, FLC-00457, FLC-00491, FLC-00424, FLC-00436, FLC-00439, FLC-00446, FLC-00466, FLC-00468, FLC-00506, FLC-00475, FLC-00553, FLC-00593, FLC-00606, FLC-00771. The results of activity tests carried out on drug-sensitive and drug-resistant human ovary cancer cells indicate that the compounds according to the invention can overcome drug resistance of cancer cells. Furthermore, the compounds according to the invention are characterised by an excellent selectivity of action, which demonstrates their extensive therapeutic potential. A person skilled in the art will know how to select a particular derivative to treat the appropriate type of cancer.

The inventive compounds, i.e. derivatives with a retained configuration on the C20 carbon versus the starting salinomycin have improved biological properties compared to their counterparts with a configuration conversion, i.e. the so-called epi-salinomycin derivatives referred to in prior art. For example, table 5 shows comparative examples:

- inventive compound FLC-00366-1 and the corresponding *epi* derivative designated FLC-00524-1 · Na
- inventive compound FLC-00374-1 and the corresponding *epi* derivative designated FLC-00521-1 · Na

| | |
|---|---|
| 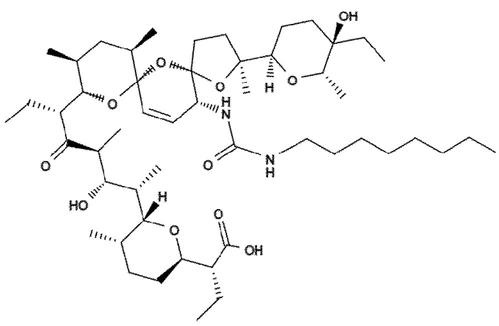<br>**FLC-00366-1** | 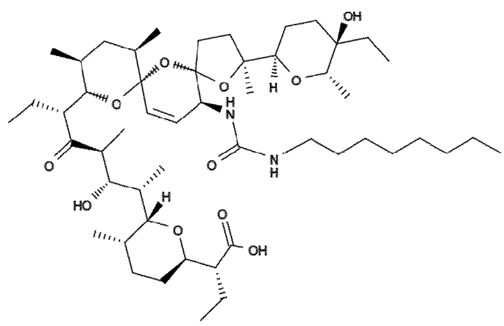<br>**FLC-00524-1 Na** |
| 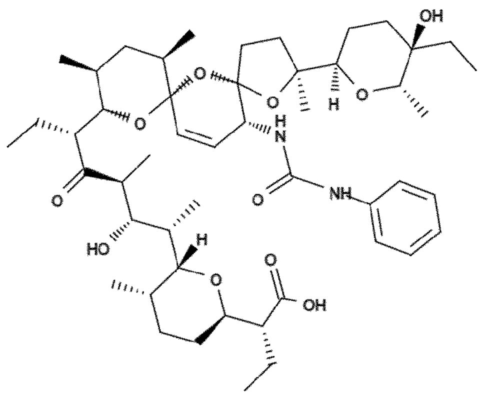<br>**FLC-00374-1** | 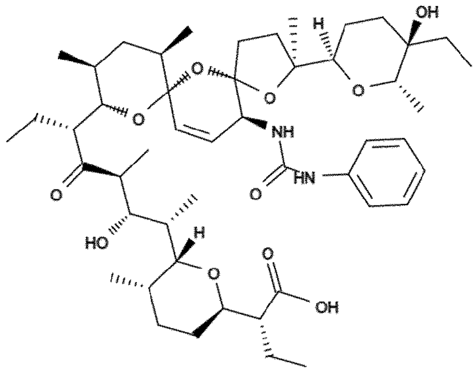<br>**FLC-00521-1 Na** |

The inventive compounds have lower $IC_{50}$ values and higher SI values compared to the corresponding *epi* compounds. Thus, the inventive compounds have a superior activity profile in accordance with the object of the invention regarding known salinomycin derivatives.

Higher anti-cancer activity of non epiC20-salinomycin derivatives (some examples of amides and urethanes) is disclosed in WO2021156461A1. It was disclosed on p. 53 and Table 4 on p. 54 (Example 19) of WO2021156461A1 test

results for C20-*epi*-salinomycin derivatives versus corresponding derivatives disclosed therein. The results presented clearly show that in neither case did the *epi* derivatives show higher activity than the corresponding derivatives according to the disclosure. For some tested cell lines (see Li *et al.*), salinomycin derivatives with the substituent at the C-20 position located at the *epi* configuration show a complete lack of activity, thus presenting a difference of orders of magnitude compared to the derivatives according to the disclosure.

The comparison for other pairs of compounds: C20-epi salinomycines known from the closest prior art, see Li et al., European Journal of Medicinal Chemistry, 148, 2018, 279-290 and their non-epi analogues known from WO2021156461A1 is presented in relation to their activity against MCF-7 cell line in Table 6 below.

Table 6

| WO2021156461A1 | MCF-7 $IC_{50}$ [μM] | Publication by Li *et al.* | MCF-7 $IC_{50}$ [μM] |
|---|---|---|---|
| Comp. no. 9 | 2.94 | Comp. no. 15 | > 20 |
| Comp. no. 17 | 0.49 | Comp. no. 50 | 16 |
| Comp. no. 15 | 0.28 | Comp. no. 27 | 8.52 |
| Comp. no. 20 | 2.38 | Comp. no. 7 | 8.83 |

The inventive compounds with the formula 5 and 7 are different derivatives than those disclosed in WO2021156461A1, even though their core is the same. Therefore, based on the results indicated above, a general conclusion may be made. All compounds according to the present invention which are non C20-*epi* derivatives of salinomycin, including compounds 5 and 7, have higher activity against cancer cells in comparison to their C-20-*epi* analogues.

The compounds according to the invention and the methods for preparing the same are illustrated in the following examples. Said examples, rather than being intended to limit the scope of protection, only constitute selected, representative examples of embodiments of the invention. It will be within the knowledge of a person skilled in the art to select reactants and adjust reaction conditions to obtain other derivatives within the scope of protection as defined in the patent claims. Any suitable organic synthesis technique may be used to prepare the compounds. The methods illustrate the nature of such processes and are not intended to limit the range of usable methods.

**General**

All solvents, substrates, and reagents were obtained from TriMen Chemicals (Poland) or Merck and were used without further purification. Spectral grade solvents were stored over 3 Å molecular sieves for several days. Thin-layer chromatography (TLC) analysis was performed using aluminum-backed plates (200 μm thickness, F-254 indicator) from SiliCycle Inc., and spots were visualized by UV light followed by treatment with phosphomolybdic acid (PMA, 5% in absolute EtOH) and gentle heating.

Products were purified by flash chromatography using high-purity grade silica gel (pore size 60 Å, 230-400 mesh particle size) from SiliCycle, Inc or products of the reactions were purified using CombiFlash® $Rf^+$ Lumen Flash Chromatography System (Teledyne Isco) with integrated ELS and UV detectors.

Preparative HPLC was performed on LC-20AP Shimadzu with ELSD-LTII detector equipped with Phenomenex Luna C18 250 × 21 mm, 5 μm column eluted with 20 mL/min flow over 20 min of acetonitrile in water.

Solvents were removed using a rotary evaporator.

NMR spectra were recorded on Bruker Avance DRX 500 ($^1$H NMR at 500 MHz and $^{13}$C NMR at 126 MHz), Varian 400 ($^1$H NMR at 400 MHz, $^{13}$C NMR at 101 MHz) or AVANCE II PLUS ($^1$H NMR at 700 MHz and $^{13}$C NMR at 176 MHz) magnetic resonance spectrometers, respectively. $^1$H NMR spectra are reported in chemical shifts downfield from tetramethylsilane (TMS) using the respective residual solvent peak as internal standard ($CDCl_3$ δ 7.26 ppm, $CD_2Cl_2$ δ 5.32 ppm). $^1$H NMR spectra are reported as follows: chemical shift (δ, ppm), multiplicity (s = singlet, d = doublet, t = triplet, q = quartet, dd = doublet of doublets, dt = doublet of triplets, dq = doublet of quartets, m = multiplet), coupling constant (J) in hertz, and integration. $^{13}$C NMR spectra are reported in chemical shifts downfield from TMS using the respective residual solvent peak as internal standard ($CDCl_3$ δ 77.2 ppm, $CD_2Cl_2$δ 53.5 ppm).

Electrospray ionization (ESI) mass spectra were obtained on a Waters Alliance 2695 separation module with a PDA 2996 UV detector and a Waters Micromass ZQ 2000 mass detector equipped with a Kinetex Biphenyl 50 × 2.1 mm2, 2.6 μm column eluted with 0.3 mL/min flow of 3-100% gradient (over 6 min) of acetonitrile in water (mobile phases contained an addition of 0.04% of formic acid).

Example FLC-00099-2

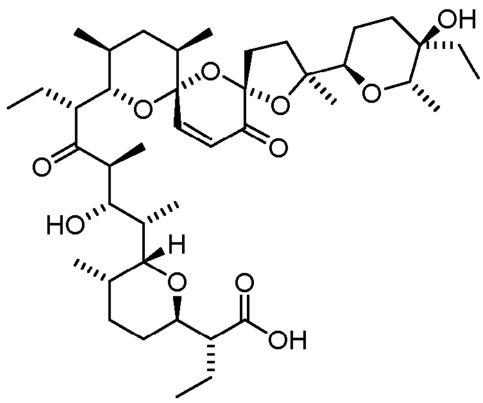

100 mg salinomycin (1 eq) was dissolved in DCM and PDC (1 eq) was added. Reactions were carried out at RT for 4 hours (TLC and LC-MS control). The post-reaction mixture was suspended on silica gel and purified using a chromatograph with an ELS detector using a column packed with silica gel. The respective fractions were combined and concentrated, and the residue was dissolved in DCM and washed twice with 0.06 M $H_2SO_4$. The yield of the obtained product was -70%.

ESI-MS for $C_{42}H_{68}O_{11}$ (*m/z*): $[M+Na]^+$ 771.9; $[M-H]^-$ 747.9.

$^1$H NMR (500 MHz, $CD_2Cl_2$) δ 7.15 (d, *J* = 10.8 Hz, 1H), 6.11 (d, *J* = 10.8 Hz, 1H), 4.10 (d, *J* = 6.6 Hz, 1H), 3.95 (d, *J* = 10.3 Hz, 1H), 3.89 (dd, *J* = 10.9, 5.8 Hz, 1H), 3.75 (d, *J* = 10.2 Hz, 1H), 3.54 (dd, *J* = 9.9, 1.9 Hz, 1H), 3.44 (t, *J* = 5.0 Hz, 1H), 3.28 - 3.08 (m, 1H), 2.84 (d, *J* = 3.9 Hz, 1H), 2.71 (dd, *J* = 10.3, 7.2 Hz, 1H), 2.60 (dd, *J* = 10.7, 2.1 Hz, 1H), 2.51 - 2.41 (m, 1H), 2.11 - 0.51 (m, 55H) ppm.

13C NMR (126 MHz, $CD_2Cl_2$) δ 213.7, 190.5, 177.5, 144.1, 126.1, 105.9, 97.6, 91.1, 75.8, 75.0, 74.7, 72.5, 71.5, 68.1, 54.8, 49.3, 48.5, 40.0, 38.7, 36.3, 35.1, 34.4, 32.7, 30.4, 28.0, 26.2, 24.2, 22.7, 21.5, 19.7, 17.6, 15.7, 13.7, 12.9, 12.7, 11.8, 10.5, 6.6, 6.5 ppm.

Example FLC-00105-2

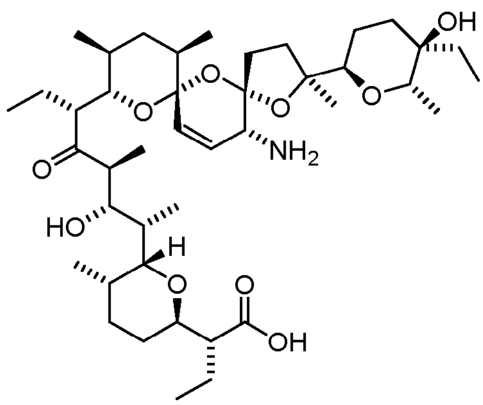

A $NaBH_3CN$ (2 eq) solution in MeOH was added dropwise to a mixture of 100 mg of ketone FLC-00099-2 (1 eq), ammonium acetate (6 eq), 7-10 M ammonia in MeOH (4eq) and MeOH. This was stirred at reflux until there was no ketone remaining (TLC and LC-MS control). The post-reaction mixture was suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was -70%.

ESI-MS for $C_{42}H_{71}NO_{10}$ (*m/z*): $[M+H]^+$ 751, $[M-H]^-$ 749.

$^1$H NMR (500 MHz, $CD_2Cl_2$) δ 6.43 (t, J = 9.1 Hz, 2H), 4.32 (d, J = 9.8 Hz, 1H), 4.05 (m, 1H), 3.79 (dd, J = 10.9, 4.5

Hz, 1H), 3.64 (dd, J = 10.1, 2.0 Hz, 2H), 3.63 - 3.48 (m, 2H), 2.72 (td, J = 11.0, 3.1 Hz, 1H), 2.63 (d, J = 10.7 Hz, 1H), 2.55 (dd, J = 16.5, 7.5 Hz, 1H), 2.22 - 2.09 (m, 1H), 2.05 - 0.85(m, 46H), 0.76 (dt, J = 10.1, 5.6 Hz, 9H), 0.63 (d, J = 6.9 Hz, 3H) ppm.

$^{13}C$ NMR (176 MHz, $CD_2Cl_2$) δ 216.1, 181.7, 132.1, 128.2, 107.0, 99.5, 86.8, 76.9, 76.2, 75.5, 72.5, 71.4, 70.7, 67.5, 65.6, 54.8, 50.8, 50.1, 47.9, 38.5, 37.2, 37.1, 36.1, 32.1, 31.0, 29.2, 27.9, 26.6, 24.4, 23.7, 21.9, 20.1, 16.9, 16.1, 15.1, 14.5, 12.5, 12.1, 10.6, 6.7, 6.0 ppm.

The crystallographic structure was published in the CSD database: DOI: 10.5517/ccdc.csd.cc28d584
CCDC Number: 2097065.

Example FLC-0337-1

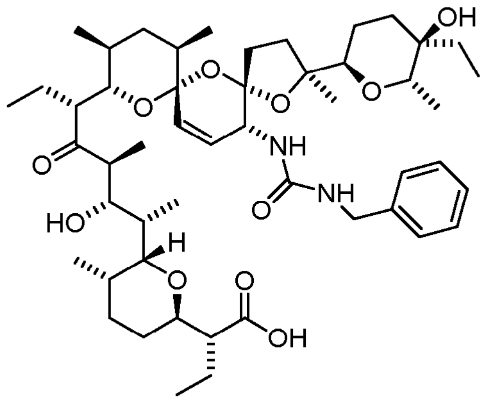

100 mg of C20-amino SAL (1 eq) was mixed with benzyl isocyanate (1.2 eq) in DCM. The reaction was carried out at RT (TLC and LC-MS control). The post-reaction mixture was suspended on silica gel and purified using a chromatograph with an ELS detector using a column packed with silica gel. The respective fractions were combined and concentrated, and the residue was dissolved in DCM and washed twice with 0.06 M $H_2SO_4$. The yield of the obtained product was 32%.

ESI-MS for $C_{50}H_{78}N_2O_{11}$ (*m/z*): $[M+H]^+$ 883.9, $[M+Na]^+$ 909.9.

$^1H$ NMR (700 MHz, $CD_2Cl_2$) δ 7.39 (dd, *J* = 7.9, 0.8 Hz, 2H), 7.32 - 7.29 (m, 2H), 7.21 (t, *J* = 7.4 Hz, 1H), 6.77 (s, 1H), 6.35 (dd, *J* = 9.8, 6.6 Hz, 1H), 6.14 (d, *J* = 10.0 Hz, 1H), 5.80 (d, *J* = 10.0 Hz, 1H), 4.48 (ddd, *J* = 20.9, 12.4, 6.3 Hz, 2H), 4.34 (dd, *J* = 15.0, 5.0 Hz, 1H), 4.14 (dd, *J* = 10.1, 1.7 Hz, 1H), 3.94 (dd, *J* = 11.0, 5.5 Hz, 1H), 3.73 (q, *J* = 6.8 Hz, 1H), 3.69 - 3.65 (m, 2H), 3.45 (dd, *J* = 11.1, 2.5 Hz, 1H), 2.97 (td, *J* = 11.1, 4.1 Hz, 1H), 2.66 (dq, *J* = 10.0, 7.1 Hz, 1H), 2.62 - 2.59 (m, 1H), 1.97 - 0.53 (m, 57H) ppm.

$^{13}C$ NMR (176 MHz, $CD_2Cl_2$) δ 217.6, 178.3, 160.8, 140.2, 133.3, 129.0, 128.8, 128.3, 127.3, 109.4, 100.4, 86.8, 77.7, 75.9, 75.5, 74.0, 71.7, 71.4, 68.6, 56.3, 51.0, 49.5, 48.1, 44.8, 39.8, 38.5, 38.2, 36.9, 34.2, 32.9, 30.9, 29.9, 28.7, 26.3, 23.9, 22.5, 21.6, 20.3, 17.7, 16.7, 16.0, 14.8, 13.3, 13.2, 12.4, 12.0, 7.5, 6.8 ppm.

Example FLC-00338-1

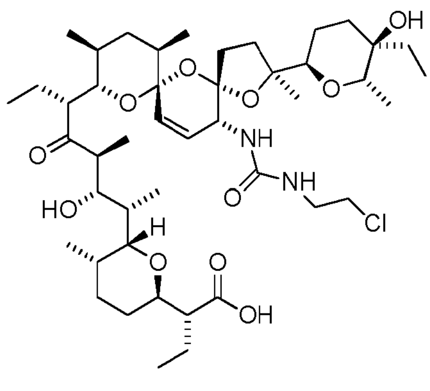

The compound was prepared according to the procedure described in example FLC-0337-1, except that 2-chloroethyl isocyanate (1.2 eq) was added to the reaction mixture instead of benzyl isocyanate. Following purification, the compound was washed with 0.25 M $Na_2CO_3$ instead of 0.06 M $H_2SO_4$. The yield of the obtained product was 64 %.

ESI-MS for $C_{45}H_{75}ClN_2O_{11}$ (*m*/*z*): $[M+H]^+$ 855.7, $[M+Na]^+$ 877.8.

$^1H$ NMR (700 MHz, $CD_2Cl_2$) δ 6.25 (t, *J* = 5.8 Hz, 1H), 6.08 (dd, *J* = 10.9, 3.0 Hz, 1H), 5.85 (d, *J* = 9.9 Hz, 1H), 5.68 (dd, *J* = 10.8, 1.9 Hz, 1H), 4.60 (ddd, *J* = 9.9, 2.9, 2.0 Hz, 1H), 4.30 (q, *J* = 6.7 Hz, 1H), 4.08 (dd, *J* = 10.5, 1.4 Hz, 1H), 3.82 (dd, *J* = 11.0, 4.9 Hz, 1H), 3.64 (dd, *J* = 10.2, 2.2 Hz, 1H), 3.61 - 3.58 (m, 2H), 3.52 - 3.45 (m, 4H), 3.39 - 3.36 (m, 2H), 2.83 (td, *J* = 11.1, 3.3 Hz, 1H), 2.74 - 2.68 (m, 2H), 2.14 - 0.67 (m, 55H) ppm.

$^{13}C$ NMR (176 MHz, $CD_2Cl_2$) δ 219.6, 185.0, 157.9, 129.8, 123.0, 106.9, 99.2, 88.9, 76.3, 76.2, 75.9, 74.2, 71.6, 71.4, 68.4, 56.5, 50.8, 50.0, 47.2, 45.1, 44.2, 42.6, 41.0, 39.0, 37.5, 36.2, 32.9, 32.9, 32.5, 28.4, 28.1, 28.1, 27.1, 23.9, 20.1, 17.6, 16.2, 15.9, 14.7, 13.3, 12.7, 12.4, 11.0, 6.8, 6.6 ppm.

Example FLC-00341-1

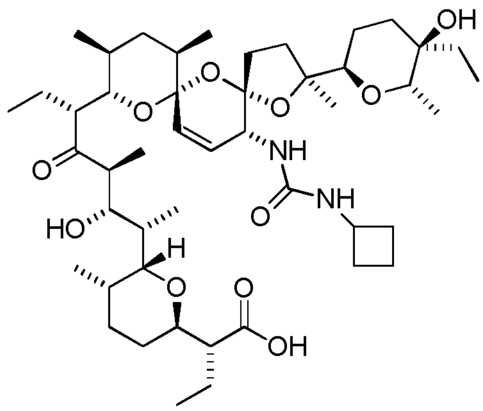

The compound was prepared according to the procedure described in example FLC-0337-1, except that cyclobutyl isocyanate (1.2 eq) was added to the reaction mixture instead of benzyl isocyanate. Following purification, the compound was washed with 0.25 M $Na_2CO_3$ instead of 0.06 M $H_2SO_4$. The yield of the obtained product was 47 %.

ESI-MS for $C_{47}H_{78}N_2O_{11}$ (*m*/*z*): $[M+H]^+$ 848.1, $[M+Na]^+$ 870.9.

$^1H$ NMR (500 MHz, $CD_2Cl_2$) δ 6.06 (dd, *J* = 10.8, 2,9 Hz, 2H), 5.67 (dd, *J* = 10.8, 1.8 Hz, 1H), 5.58 (d, *J* = 9.9 Hz, 1H), 4.84 (s, 1H), 4.62 - 4,56 (m, 1H), 4.32 (q, *J* = 6.7 Hz, 1H), 4.18 - 4.05 (m, 2H), 3.83 (dd, *J* = 11.0, 4.5 Hz, 1H), 3.64 (dd, *J* = 10.1, 1.9 Hz, 1H), 3.59 (d, *J* = 10.1 Hz, 1H), 3.47 (dd, *J* = 12.4, 2.8 Hz, 1H), 2.83 (td, *J* = 11.1, 3.2 Hz, 1H), 2.75 - 2.66 (m, 2H), 2.25 - 0.65 (m, 62H) ppm.

$^{13}C$ NMR (126 MHz, $CD_2Cl_2$) δ 219.5, 185.0, 156.9, 130.0, 122.8, 107.0, 99.1, 88.7, 76.2, 76.1, 75.8, 74.3, 71.5, 71.4, 68.3, 56.5, 50.9, 50.0, 46.8, 45.8, 40.9, 39.0, 37.3, 36.2, 32.9, 32.8, 32.7, 32.2, 31.8, 28.4, 28.1, 28.0, 27.1, 24.0, 20.3, 20.0, 17.6, 16.1, 15.9, 15.1, 14.7, 13.2, 13.2, 12.3, 10.9, 6.8 ppm.

Example FLC-00344-1

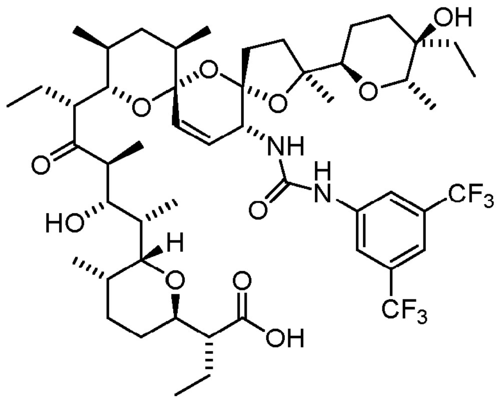

The compound was prepared according to the procedure described in example FLC-0337-1, except that 3,5-bis(trifluoromethyl)phenyl isocyanate (1.2 eq) was added to the reaction mixture instead of benzyl isocyanate. Following purification, the compound was washed with 0.25 M $Na_2CO_3$ instead of 0.06 M $H_2SO_4$. The yield of the obtained product was 22 %.

ESI-MS for $C_{51}H_{74}F_6N_2O_{11}$ (*m*/*z*): $[M+H]^+$ 1005.8, $[M+Na]^+$ 1027.8.

$^1H$ NMR (500 MHz, $CD_2Cl_2$) δ 9.02 (s, 1H), 8.04 (s, 2H), 7.38 (s, 1H), 6.53 (d, *J* = 9.6 Hz, 1H), 6.22 (s, 1H), 6.15 (dd, *J* = 10.8, 2.9 Hz, 1H), 5.73 (dd, *J* = 10.8, 1.8 Hz, 1H), 4.77 - 4.70 (m, 1H), 4.57 (s, 1H), 4.41 (q, *J* = 6.7 Hz, 1H), 4.11 (d, *J* = 10.3 Hz, 1H), 3.86 (dd, *J* = 11.0, 5.0 Hz, 1H), 3.62 (dd, *J* = 16.9, 6.3 Hz, 2H), 3.49 (dd, *J* = 12.3, 2.6 Hz, 1H), 2.84 (td, *J* = 11.0, 3.1 Hz, 1H), 2.79 - 2.70 (m, 2H), 2.18 - 0.61 (m, 55H) ppm.

$^{13}C$ NMR (126 MHz, $CD_2Cl_2$) δ 219.6, 185.7, 154.9, 142.8, 131.85 (q), 128.4, 125.1, 123.9, 122.9, 117.7, 114.3, 106.7, 99.1, 89.0, 76.2, 76.1, 75.8, 74.2, 72.2, 71.5, 68.5, 56.5, 50.6, 49.8, 46.9, 40.9, 38.9, 37.8, 36.2, 32.9, 32.8, 32.8, 28.6, 28.4, 28.1, 26.9, 23.9, 20.4, 20.2, 17.6, 16.1, 15.8, 14.7, 13.2, 12.4, 11.0, 6.8, 6.6 ppm.

Example FLC-00350-1

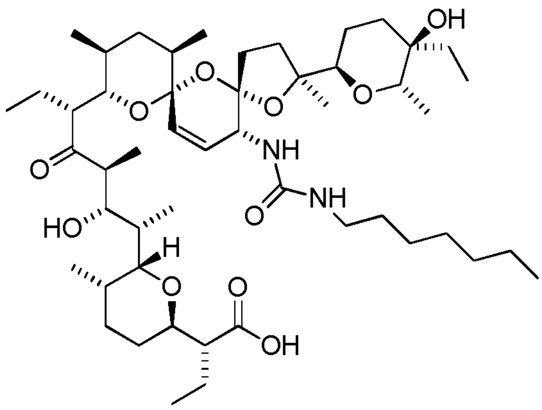

The compound was prepared according to the procedure described in example FLC-0337-1, except that heptyl isocyanate (1.2 eq) was added to the reaction mixture instead of benzyl isocyanate. The yield of the obtained product was 37 %.

ESI-MS for $C_{50}H_{86}N_2O_{11}$ (*m*/*z*): $[M+H]^+$ 891.9, $[M+Na]^+$ 913.9.

$^1H$ NMR (500 MHz, $CD_2Cl_2$) δ 6.42 (s, 1H), 6.27 (dd, *J* = 9.8, 6.4 Hz, 1H), 6.10 (d, *J* = 10.0 Hz, 1H), 5.61 (d, *J* = 9.9 Hz, 1H), 4.42 (dd, *J* = 9.9, 6.4 Hz, 1H), 4.07 (dd, *J* = 10.1, 9.1 Hz, 1H), 3.91 (dd, *J* = 10.9, 5.2 Hz, 1H), 3.74 (q, *J* = 6.8 Hz, 1H), 3.66 (d, *J* = 10.2 Hz, 1H), 3.61 (dd, *J* = 10.0, 1.7 Hz, 1H), 3.48 - 3.41 (m. 1H), 3.21 (td, *J* = 12.9, 6.6 Hz, 1H), 3.10 (td, *J* = 11.9, 6.5 Hz, 1H), 2.94 (td, *J* = 10.8, 3.7 Hz, 1H), 2.73 - 2.61 (m, 2H), 2.03 - 0.60 (m, 60H) ppm.

$^{13}C$ NMR (126 MHz, $CD_2Cl_2$) δ 217.4, 178.2, 160.5, 133.1, 126.4, 109.1, 100.2, 86.6, 77.5, 75.7, 75.5, 73.8, 71.3, 71.2, 68.3, 56.0, 50.7, 49.1, 47.9, 40.6, 39.7, 38.3, 37.9, 36.8, 33.8, 32.8, 32.2, 30.6, 30.5, 29.7, 29.6, 28.4, 27.5, 26.1, 23.9, 23.1, 22.3, 21.3, 20.2, 17.6, 16.5, 15.8, 14.6, 14.3, 13.1, 13.1, 12.2, 11.8, 7.3, 6.7 ppm.

Example FLC-00351-1

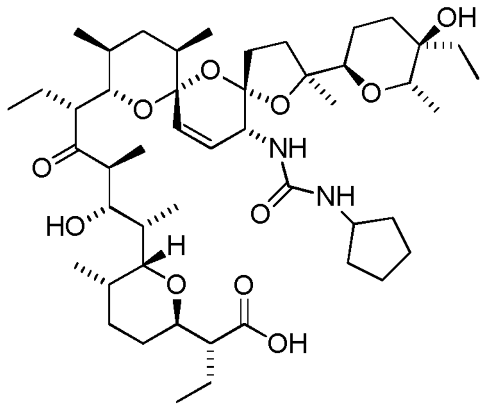

The compound was prepared according to the procedure described in example FLC-0337-1, except that cyclopentyl isocyanate (1.2 eq) was added to the reaction mixture instead of benzyl isocyanate. The yield of the obtained product was 44 %.

ESI-MS for $C_{48}H_{80}N_2O_{11}$ (*m/z*): $[M+H]^+$ 862.2, $[M+Na]^+$ 885.1.

$^1H$ NMR (500 MHz, $CD_2Cl_2$) δ 6.31 (s, 1H), 6.24 (dd, *J* = 9.9, 6.1 Hz, 1H), 6.10 (d, *J* = 10.0 Hz, 1H), 5.59 (d, *J* = 9.9 Hz, 1H), 4.40 (dd, *J* = 9.9, 6.1 Hz, 1H), 4.05 (h, *J* = 6.4 Hz, 2H), 3.91 (dd, *J* = 10.9, 5.2 Hz, 1H), 3.76 (q, *J* = 6.8 Hz, 1H), 3.69 (d, *J* = 10.2 Hz, 1H), 3.62 (dd, *J* = 10.0, 1.7 Hz, 1H), 3.52 - 3.45 (m, 1H), 2.95 (td, *J* = 11.0, 4.0 Hz, 1H), 2.72 - 2.61 (m, 2H), 1.96 - 0.62 (m, 65H) ppm.

$^{13}C$ NMR (126 MHz, $CD_2Cl_2$) δ 217.3, 178.2, 159.8, 133.0, 126.1, 109.0, 100.2, 86.7, 77.4, 75.7, 75.5, 74.0, 71.3, 71.2, 68.4, 56.1, 52.1, 50.6, 49.0, 48.0, 39.9, 38.3, 37.8, 36.8, 34.1, 33.8, 33.2, 32.8, 30.5, 29.7, 28.4, 26.1, 24.0, 23.9, 22.4, 21.3, 20.2, 17.6, 16.5, 15.8, 14.7, 13.2, 13.1, 12.3, 11.8, 7.3, 6.7 ppm.

Example FLC-00353-1

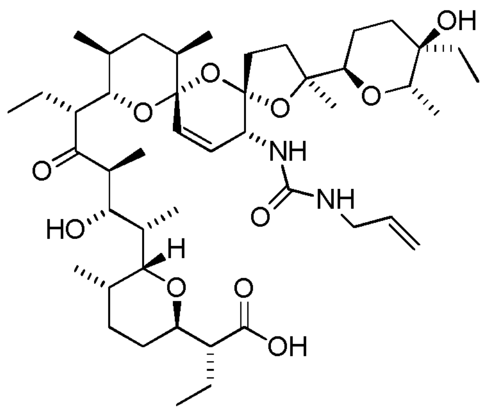

The compound was prepared according to the procedure described in example FLC-0337-1, except that allyl isocyanate (1.2 eq) was added to the reaction mixture instead of benzyl isocyanate. The yield of the obtained product was 92 %.

ESI-MS for $C_{46}H_{76}N_2O_{11}$ (*m/z*): $[M+H]^+$ 834.2, $[M+Na]^+$ 857.0.

$^1H$ NMR (500 MHz, $CD_2Cl_2$) δ 6.39 (s, 1H), 6.23 (dd, *J* = 9.9, 6.0 Hz, 1H), 6.11 (d, *J* = 10.0 Hz, 1H), 5.90 (ddd, *J* = 22.6, 10.7, 5.5 Hz, 1H), 5.74 (d, *J* = 9.9 Hz, 1H), 5.23 (dd, *J* = 17.2, 1.6 Hz, 1H), 5.06 (dd, *J* = 10.3, 1.4 Hz, 1H), 4.43 (dd, *J* = 9.8, 6.0 Hz, 1H), 4.09 - 4.04 (m, 1H), 3.92 (dd, *J* = 10.9, 5.3 Hz, 1H), 3.80 (t, *J* = 5.5 Hz, 2H), 3.75 (q, *J* = 6.8 Hz, 1H), 3.68 (d, *J* = 10.2 Hz, 1H), 3.62 (dd, *J* = 10.0, 1.7 Hz, 1H), 3.50 - 3.44 (m, 1H), 2.95 (td, *J* = 11.0, 4.0 Hz, 1H), 2.73 - 2.66 (m, 1H), 2.63 (d, *J* = 10.2 Hz, 1H), 2.02 - 0.66 (m, 57H) ppm.

$^{13}C$ NMR (126 MHz, $CD_2Cl_2$) δ 217.5, 178.3, 160.2, 136.3, 132.7, 126.2, 115.4, 108.8, 100.1, 86.8, 77.5, 75.7,

75.5, 73.8, 71.4, 71.3, 68.4, 56.1, 50.6, 49.2, 48.0, 43.1, 39.9, 38.3, 37.9, 36.8, 33.6, 32.8, 30.8, 29.6, 28.4, 26.1, 24.1, 22.4, 21.3, 20.2, 17.6, 16.6, 15.8, 14.6, 13.2, 13.1, 12.2, 11.7, 7.3, 6.6 ppm.

Example FLC-00358-1

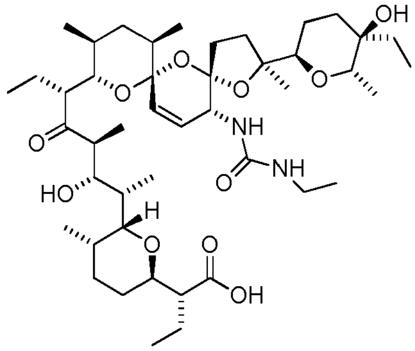

The compound was prepared according to the procedure described in example FLC-0337-1, except that ethyl isocyanate (1.2 eq) was added to the reaction mixture instead of benzyl isocyanate. The yield of the obtained product was 72 %.

ESI-MS for $C_{45}H_{76}N_2O_{11}$ (*m/z*): $[M+H]^+$ 821.9, $[M+Na]^+$ 843.9.

$^1H$ NMR (500 MHz, $CD_2Cl_2$) δ 6.22 (dd, *J*= 10.0, 5.9 Hz, 2H), 6.10 (d, *J*= 10.0 Hz, 1H), 5.63 (d, *J* = 9.9 Hz, 1H), 4.41 (dd, *J* = 9.9, 5.9 Hz, 1H), 4.05 (dd, *J* = 10.1, 1.2 Hz, 1H), 3.91 (dd, *J* = 11.0, 5.2 Hz, 1H), 3.75 (q, *J* = 6.8 Hz, 1H), 3.69 (d, *J* = 10.2 Hz, 1H), 3.63 - 3.60 (m, 1H), 3.49 - 3.44 (m, 1H), 3.24 - 3.15 (m, 2H), 2.95 (td, *J* = 11.1, 4.0 Hz, 1H), 2.72 - 2.66 (m, 1H), 2.63 (d, *J*= 10.0 Hz, 1H), 2.02 - 0.65 (m, 50H) ppm.

$^{13}C$ NMR (151 MHz, $CD_2Cl_2$) δ 217.4, 178.3, 160.2, 132.9, 126.0, 108.9, 100.1, 86.8, 77.5, 75.7, 75.6, 73.8, 71.4, 71.2, 68.4, 67.4, 56.1, 50.6, 49.1, 48.0, 39.9, 38.3, 37.9, 36.8, 35.3, 33.6, 32.8, 30.7, 29.6, 28.4, 26.1, 24.2, 22.4, 21.3, 20.2, 17.7, 16.6, 15.8, 15.5, 14.6, 13.2, 12.2, 11.8, 7.3, 6.6 ppm.

Example FLC-00359-1

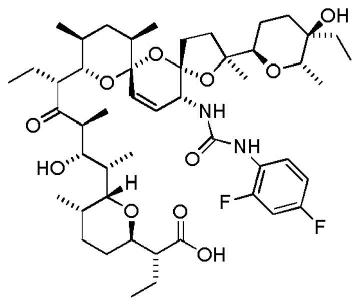

The compound was prepared according to the procedure described in example FLC-0337-1, except that 2.4-difluorophenyl isocyanate (1.2 eq) was added to the reaction mixture instead of benzyl isocyanate. The yield of the obtained product was 37 %.

ESI-MS for $C_{49}H_{74}F_2N_2O_{11}$ (*m/z*): $[M+Na]^+$ 927.9.

$^1H$ NMR (500 MHz, $CD_2Cl_2$) δ 8.44 (s, 1H), 7.89 (td, *J* = 8.8, 6.2 Hz, 1H), 6.90 - 6.78 (m, 2H), 6.25 (d, *J* = 9.6 Hz, 1H), 6.22 - 6.16 (m, 2H), 4.51 (dd, *J* = 9.6, 4.7 Hz, 1H), 4.19 (dd, *J*= 10.1, 1.1 Hz, 1H), 3.80 (q, *J* = 6.7 Hz, 1H), 3.70 (p, *J* = 6.2 Hz, 2H), 3.63 - 3.59 (m, 1H), 3.52 (dd, *J* = 10.1, 7.7 Hz, 1H), 2.91 (td, *J* = 10.9, 4.3 Hz, 1H), 2.76 - 2.66 (m, 2H), 2.06 - 0.64 (m, 57H) ppm.

$^{13}C$ NMR (151 MHz, $CD_2Cl_2$) δ 217.8, 178.3, 158.95 (d) 157.4, 157.34 (d) 154.53 (d) 152.89 (d) 131.8, 124.58 (dd) 123.52 (d) 126.5, 110.81 (dd) 108.3, 103.62 (dd) 100.1, 87.2, 77.4, 75.8, 75.4, 73.6, 67.4, 56.0, 50.6, 49.3, 47.9, 40.0, 38.4, 38.0, 36.7, 33.2, 32.8, 31.0, 29.6, 28.4, 26.1, 24.4, 22.3, 21.4, 20.1, 17.6, 16.6, 15.8, 14.7, 13.1, 13.0, 12.1, 11.7, 7.2, 6.5 ppm.

Example FLC-00362-1

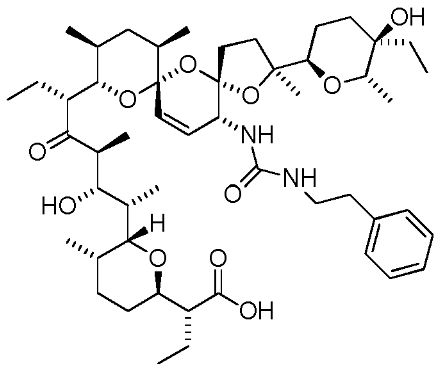

The compound was prepared according to the procedure described in example FLC-0337-1, except that 2-phenylethyl isocyanate (1.2 eq) was added to the reaction mixture instead of benzyl isocyanate. The yield of the obtained product was 25 %.

ESI-MS for $C_{51}H_{80}N_2O_{11}$ (*m/z*): $[M+Na]^+$ 920.1.

$^1H$ NMR (500 MHz, $CD_2Cl_2$) δ 7.30 - 7.24 (m, 4H), 7.22 - 7.14 (m, 1H), 6.44 (s, 1H), 6.25 (dd, *J* = 9.9, 6.1 Hz, 1H), 6.12 (d, *J* = 10.0 Hz, 1H), 5.70 (d, *J* = 9.9 Hz, 1H), 4.45 (dd, *J* = 9.8, 6.1 Hz, 1H), 4.05 (dd, *J* = 10.0, 1.3 Hz, 1H), 3.94 (dd, *J* = 11.0, 5.2 Hz, 1H), 3.72 (p, *J* = 6.9 Hz, 1H), 3.68 - 3.61 (m, 1H), 3.44 (ddd, *J* = 18.0, 11.7, 4.9 Hz, 3H), 2.97 (td, *J* = 11.1, 4.0 Hz, 1H), 2.83 (td, *J* = 7.9, 2.6 Hz, 2H), 2.68 - 2.60 (m, 1H), 2.00 - 0.64 (m, 59H) ppm.

$^{13}C$ NMR (126 MHz, $CD_2Cl_2$) δ 217.2, 178.3, 160.3, 140.3, 132.8, 129.2, 128.7, 126.4, 126.3, 108.9, 100.1, 86.8, 77.5, 75.7, 75.6, 73.8, 71.5, 71.2, 68.2, 55.9, 50.6, 49.2, 48.0, 42.0, 39.8, 38.3, 37.9, 36.8, 36.8, 33.6, 32.8, 30.7, 29.7, 28.4, 26.1, 24.2, 22.4, 21.4, 20.2, 17.6, 16.5, 15.8, 14.6, 13.1, 13.0, 12.3, 11.8, 7.3, 6.7 ppm.

Example FLC-00363-1

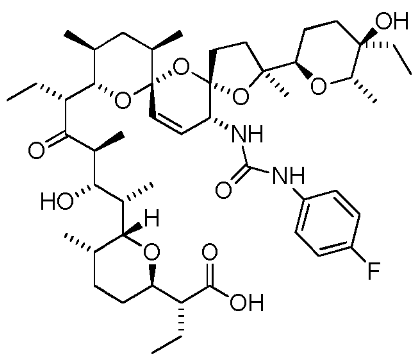

The compound was prepared according to the procedure described in example FLC-0337-1, except that 4-fluorophenyl isocyanate (1.2 eq) was added to the reaction mixture instead of benzyl isocyanate. The yield of the obtained product was 48 %.

ESI-MS for $C_{49}H_{75}FN_2O_{11}$ (*m/z*): $[M-H]^-$ 886.1, $[M+Na]^+$ 910.2.

$^1H$ NMR (500 MHz, $CD_2Cl_2$) δ 8.50 (s, 1H), 7.39 - 7.31 (m, 2H), 7.00 - 6.90 (m, 2H), 6.22 - 6.13 (m, 2H), 6.03 (d, *J* = 9.9 Hz, 1H), 4.52 (dd, *J* = 9.8, 4.0 Hz, 1H), 4.19 (d, *J* = 10.1 Hz, 1H), 3.83 (q, *J* = 6.8 Hz, 1H), 3.73 - 3.66 (m, 2H), 3.62 (dd, *J* = 10.0, 2.2 Hz, 1H), 3.51 (dd, *J* = 11.6, 2.4 Hz, 1H), 2.90 (td, *J* = 10.9, 4.4 Hz, 1H), 2.79 - 2.72 (m, 1H), 2.69 (d, *J* = 9.9 Hz, 1H), 2.06 - 0.67 (m, 57H) ppm.

$^{13}C$ NMR (126 MHz, $CD_2Cl_2$) δ 218.1, 178.5, 159.8, 157.9, 157.8, 136.2, 132.0, 126.3, 122.14 (d) 115.4, 115.2, 108.4, 100.1, 87.0, 77.3, 75.9, 75.4, 73.7, 71.5, 71.5, 68.5, 56.0, 50.4, 48.9, 47.8, 40.0, 38.4, 38.0, 36.7, 33.5, 32.9, 31.1, 29.5, 28.4, 26.1, 24.6, 22.2, 21.3, 20.2, 17.7, 16.5, 15.8, 14.6, 13.2, 13.1, 12.2, 11.8, 7.3, 6.6 ppm.

Example FLC-00364-1

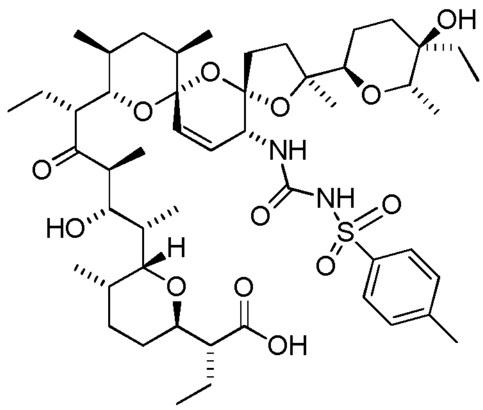

The compound was prepared according to the procedure described in example FLC-0337-1, except that *p*-toluenesulfonyl isocyanate (1.2 eq) was added to the reaction mixture instead of benzyl isocyanate. The yield of the obtained product was 77 %.

ESI-MS for $C_{50}H_{78}N_2O_{13}S$ (*m/z*): $[M-H]^-$ 946.2, $[M+Na]^+$ 970.0.

$^1H$ NMR (500 MHz, $CD_2Cl_2$) δ 7.87 (d, *J* = 8.3 Hz, 2H), 7.31 (d, *J* = 8.1 Hz, 2H), 6.45 (d, *J* = 9.7 Hz, 1H), 6.12 (dd, *J* = 10.4, 1.5 Hz, 1H), 5.85 (dd, *J* = 10.3, 3.3 Hz, 1H), 4.31 (dd, *J* = 9.7, 2.1 Hz, 1H), 4.09 (d, *J* = 10.2 Hz, 1H), 3.93 (dd, *J* = 10.8, 5.6 Hz, 1H), 3.84 (q, *J* = 6.7 Hz, 1H), 3.72 (d, *J* = 9.5 Hz, 1H), 3.61 (d, *J* = 9.6 Hz, 1H), 3.45 (d, *J* = 11.2 Hz, 1H), 2.94 (td, *J* = 10.8, 4.0 Hz, 1H), 2.81 - 2.73 (m, 1H), 2.64 (d, *J* = 10.2 Hz, 1H), 2.43 (s, 3H), 2.01 - 0.68 (m, 58H) ppm.

$^{13}C$ NMR (126 MHz, $CD_2Cl_2$) δ 217.2, 178.6, 152.4, 144.4, 137.6, 130.2, 129.7, 128.3, 125.2, 107.0, 99.6, 87.6, 77.1, 76.4, 75.3, 73.6, 72.1, 71.9, 68.9, 55.8, 49.7, 49.1, 48.5, 40.6, 38.5, 37.7, 36.6, 33.0, 31.3, 28.8, 28.6, 26.4, 25.3, 22.8, 21.7, 20.8, 20.3, 17.7, 16.3, 15.8, 14.6, 13.3, 13.3, 12.2, 11.5, 7.4, 6.5 ppm.

Example FLC-00365-1

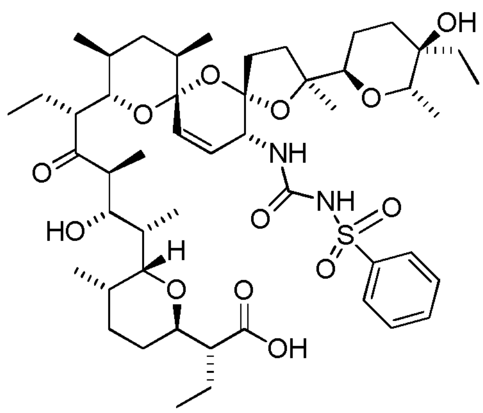

The compound was prepared according to the procedure described in example FLC-0337-1, except that benzenesulfonyl isocyanate (1.2 eq) was added to the reaction mixture instead of benzyl isocyanate. The yield of the obtained product was 62 %.

ESI-MS for $C_{49}H_{76}N_2O_{13}S$ (*m/z*): $[M-H]^-$ 932.1, $[M+Na]^+$ 956.0.

$^1H$ NMR (500 MHz, $CD_2Cl_2$) δ 8.05 - 7.98 (m, 1H), 7.61 (t, *J* = 7.4 Hz, 1H), 7.52 (t, *J* = 7.7 Hz, 2H), 6.46 (d, *J* = 9.7 Hz, 1H), 6.13 (dd, *J* = 10.4, 1.2 Hz, 1H), 5.87 (dd, *J* = 10.3, 3.7 Hz, 1H), 4.32 (dd, *J* = 9.7, 2.8 Hz, 1H), 4.09 (d, *J* = 10.1 Hz, 1H), 3.93 (dd, *J* = 10.7, 5.6 Hz, 1H), 3.84 (q, *J* = 6.7 Hz, 1H), 3.71 (d, *J* = 11.0 Hz, 1H), 3.63 - 3.60 (m, 1H), 3.45 - 3.40 (m, 1H), 2.95 (td, *J* = 10.8, 4.1 Hz, 1H), 2.77 (dq, *J* = 14.4, 7.2 Hz, 1H), 2.64 (d, *J* = 10.4 Hz, 1H), 2.03 - 0,67 (m, 59H) ppm.

$^{13}C$ NMR (126 MHz, $CD_2Cl_2$) δ 217.4, 178.5, 152.4, 140.6, 133.4, 130.3, 129.0, 128.3, 125.4, 107.0, 99.6, 87.6, 77.1, 76.5, 75.3, 73.5, 72.1, 71.9, 68.9, 55.8, 49.7, 49.2, 48.4, 40.5, 38.5, 37.8, 36.6, 32.9, 32.9, 31.3, 28.9, 28.6, 26.4, 25.2,

22.7, 20.8, 20.3, 17.7, 16.3, 15.8, 14.5, 13.3, 12.2, 11.5, 7.5, 6.5 ppm.

Example FLC-00366-1

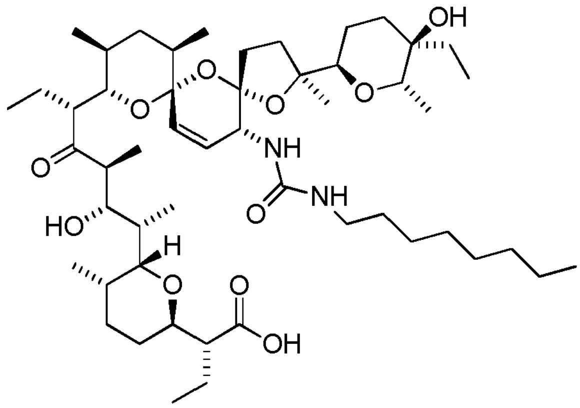

The compound was prepared according to the procedure described in example FLC-0337-1, except that octyl isocyanate (1.2 eq) was added to the reaction mixture instead of benzyl isocyanate. The yield of the obtained product was 75 %.

ESI-MS for $C_{51}H_{88}N_2O_{11}$ (*m/z*): $[M+H]^+$ 906.1, $[M+Na]^+$ 928.2.

$^1H$ NMR (500 MHz, $CDCl_3$) δ 6.29 - 6.22 (m, 1H), 6.14 (s, 1H), 6.09 (d, *J* = 10.1 Hz, 1H), 5.67 (d, *J* = 9.7 Hz, 1H), 4.44 (dd, *J* = 9.8, 5.7 Hz, 1H), 4.09 (d, *J* = 10.0 Hz, 1H), 3.94 (dd, *J* = 10.9, 5.3 Hz, 1H), 3.82 - 3.75 (m, 1H), 3.72 - 3.64 (m, 1H), 3.52 (d, *J* = 10.6 Hz, 1H), 3.22 (td, *J* = 12.8, 6.6 Hz, 1H), 3.13 (td, *J* = 12.5, 6.9 Hz, 1H), 2.93 (td, *J* = 11.0, 3.9 Hz, 1H), 2.65 (dq, *J* = 14.4, 7.1 Hz, 1H), 2.56 (d, *J* = 10.2 Hz, 1H), 2.13 - 0.63 (m, 75H) ppm.

$^{13}C$ NMR (126 MHz, $CDCl^3$) δ 216.3, 178.0, 159.9, 132.6, 125.3, 108.3, 99.6, 86.0, 75.2, 75.2, 73.9, 71.3, 71.1, 68.1, 56.0, 50.3, 48.6, 47.8, 40.4, 39.5, 38.2, 37.4, 36.5, 33.9, 32.5, 32.0, 30.3, 29.6, 29.4, 29.3, 28.1, 27.2, 26.0, 23.8, 22.8, 22.2, 21.2, 20.0, 17.8, 16.4, 15.8, 14.6, 14.2, 13.2, 13.0, 12.2, 11.7, 7.2, 6.6 ppm.

Example FLC-00367-1

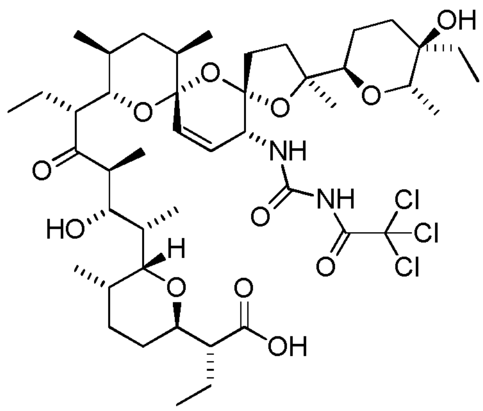

The compound was prepared according to the procedure described in example FLC-0337-1, except that 3,3,3-trichloropropionyl isocyanate (1.2 eq) was added to the reaction mixture instead of benzyl isocyanate. The yield of the obtained product was 21 %.

ESI-MS for $C_{45}H_{71}Cl_3N_2O_{12}$ (*m/z*): $[M-H]^-$ 936.0, $[M+Na]^+$ 962.1.

$^1H$ NMR (500 MHz, $CDCl_3$) δ 9.24 (s, 1H), 7.71 (s, 1H), 6.21 (dd, *J* = 10.5, 1.9 Hz, 1H), 5.96 (d, *J* = 10.4 Hz, 1H), 4.57 (dt, *J* = 9.3, 2.4 Hz, 1H), 4.17 (d, *J* = 10.1 Hz, 1H), 3.97 (dd, *J* = 10.9, 5.7 Hz, 1H), 3.79 - 3.74 (m, 2H), 3.73 - 3.66 (m, 1H), 3.63 (d, *J* = 9.9 Hz, 1H), 2.90 (td, *J* = 10.9, 3.7 Hz, 1H), 2.77 (dq, *J* = 14.5, 7.1 Hz, 1H), 2.63 (d, *J* = 9.3 Hz, 1H), 2.19 - 0.67 (m, 57H) ppm.

$^{13}C$ NMR (126 MHz, $CDCl_3$) δ 216.1, 178.0, 160.5, 152.2, 128.8, 125.1, 106.0, 99.7, 92.0, 88.3, 76.2, 74.8, 72.9, 72.5, 71.6, 71.3, 68.4, 56.1, 50.0, 49.2, 48.6, 40.7, 38.7, 36.6, 36.5, 32.7, 30.5, 29.8, 29.3, 28.1, 26.3, 24.4, 22.6, 21.9, 20.0, 17.9, 17.0, 15.8, 14.4, 13.3, 13.0, 12.1, 11.2, 7.0, 6.6 ppm.

Example FLC-00370-1

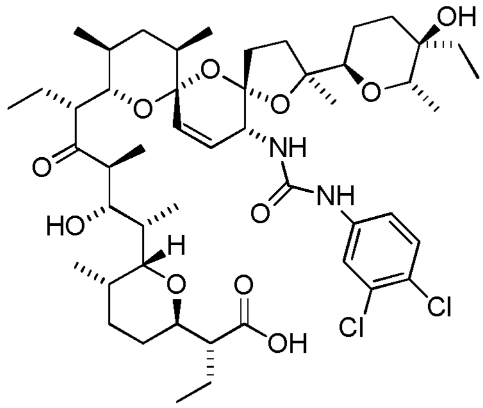

The compound was prepared according to the procedure described in example FLC-0337-1, except that 3,4-dichlorophenyl isocyanate (1.2 eq) was added to the reaction mixture instead of benzyl isocyanate. Following purification, the compound was washed with 0.25 M $Na_2CO_3$ instead of 0.06 M $H_2SO_4$. The yield of the obtained product was 76%.
ESI-MS for $C_{49}H_{74}Cl_2N_2O_{11}$ (*m/z*): $[M-H]^-$ 936.1, $[M+Na]^+$ 960.0.
$^1H$ NMR (500 MHz, $CD_2Cl_2$) δ 8.57 (s, 1H), 7.74 (dd, *J* = 9.3, 2.4 Hz, 1H), 7.32 (dd, *J* = 8.8, 2.4 Hz, 1H), 7.24 (dd, *J* = 8.8, 5.2 Hz, 1H), 6.34 (d, *J* = 9.7 Hz, 1H), 6.13 (dd, *J* = 10.8, 2.9 Hz, 1H), 5.72 (dd, *J* = 10.8, 1.6 Hz, 1H), 4.70 (d, *J* = 9.6 Hz, 1H), 4.36 (q, *J* = 6.7 Hz, 1H), 4.09 (d, *J* = 10.4 Hz, 1H), 3.86 (dd, *J* = 11.0, 5.1 Hz, 1H), 3.63 - 3.59 (m, 2H), 3.48 (dd, *J* = 12.3, 2.6 Hz, 1H), 2.83 (td, *J* = 11.0, 3.1 Hz, 1H), 2.79 - 2.68 (m, 2H), 2.26 - 0.58 (m, 57H) ppm.
$^{13}C$ NMR (126 MHz, $CD_2Cl_2$) δ 219.7, 185.5, 154.9, 142.2, 141.0, 132.2, 130.3, 128.7, 123.9, 123.6, 120.9, 119.6, 117.7, 106.8, 99.1, 88.9, 76.2, 76.0, 75.8, 74.1, 72.0, 71.5, 68.5, 56.5, 50.6, 49.9, 46.9, 40.9, 38.9, 37.7, 36.1, 32.9, 32.8, 28.4, 28.0, 27.0, 23.7, 20.2, 17.6, 16.1, 15.8, 14.7, 13.2, 12.5, 12.4, 11.0, 6.9, 6.8 ppm.

Example FLC-00371-1

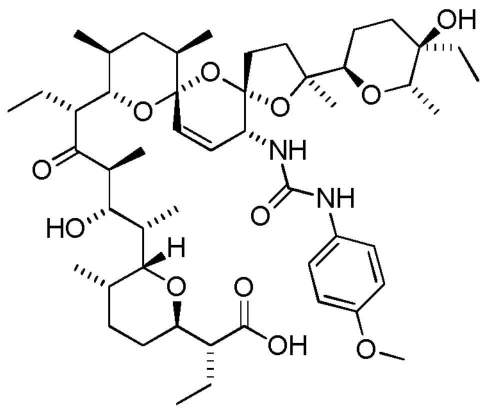

The compound was prepared according to the procedure described in example FLC-0337-1, except that 4-metoxyphenyl isocyanate (1.2 eq) was added to the reaction mixture instead of benzyl isocyanate. The yield of the obtained product was 43%.
ESI-MS for $C_{50}H_{78}N_2O_{12}$ (*m/z*): $[M+H]^+$ 902.2, $[M+Na]^+$ 922.2.
$^1H$ NMR (500 MHz, $CDCl_3$) δ 8.14 (s, 1H), 7.28 (d, *J* = 8.4 Hz, 2H), 6.79 (d, *J* = 8.5 Hz, 2H), 6.22 - 6.10 (m, 2H), 5.97 (d, *J* = 9.5 Hz, 1H), 4.57 - 4.49 (m, 1H), 4.23 (d, *J* = 9.6 Hz, 1H), 3.85 (d, *J* = 6.3 Hz, 1H), 3.79 - 3.72 (m, 5H), 3.55 (d, *J* = 10.6

Hz, 1H), 2.87 (t, *J* = 8.5 Hz, 1H), 2.75 - 2.67 (m, 1H), 2.60 (d, *J* = 10.5 Hz, 1H), 2.13 - 0.59 (m, 58H) ppm.

$^{13}C$ NMR (126 MHz, $CDCl_3$) δ 216.6, 178.2, 157.6, 155.3, 132.9, 131.8, 125.2, 122.1, 113.9, 107.8, 99.5, 86.3, 75.5, 75.0, 73.7, 71.5, 71.2, 68.3, 55.9, 55.6, 50.1, 48.5, 47.6, 39.7, 38.3, 37.5, 36.3, 33.6, 32.5, 30.8, 29.1, 28.2, 26.0, 24.2, 22.0, 21.1, 20.1, 17.8, 16.3, 15.8, 14.6, 13.2, 13.1, 12.2, 11.8, 7.2, 6.6 ppm.

Example FLC-00374-1

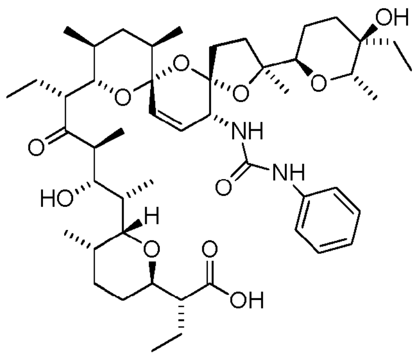

The compound was prepared according to the procedure described in example FLC-0337-1, except that phenyl isocyanate (1.2 eq) was added to the reaction mixture instead of phenyl isocyanate. The yield of the obtained product was 80 %.

ESI-MS for $C_{49}H_{76}N_2O_{12}$ (*m/z*): $[M-H]^-$ 868.0, $[M+Na]^+$ 891.9.

$^1H$ NMR (500 MHz, $CDCl_3$) δ 8.27 (s, 1H), 7.40 (d, *J* = 7.7 Hz, 2H), 7.23 (t, *J* = 7.9 Hz, 2H), 6.95 (t, *J*= 7.4 Hz, 1H), 6.18 - 6.12 (m, 2H), 6.04 (d, *J*= 9.9 Hz, 1H), 4.55 (dd, *J* = 9.9, 3.1 Hz, 1H), 4.21 (d, *J* = 10.1 Hz, 1H), 3.88 (q, *J* = 6.7 Hz, 1H), 3.77 - 3.72 (m, 2H), 3.65 (dd, *J* = 10.0, 2.2 Hz, 1H), 3.57 (dd, *J* = 11.8, 1.9 Hz, 1H), 2.87 (td, *J* = 10.9, 3.9 Hz, 1H), 2.72 (dq, *J* = 14.4, 7.1 Hz, 1H), 2.61 (d, *J* = 10.1 Hz, 1H), 2.10 - 0.64 (m, 57H) ppm.

$^{13}C$ NMR (126 MHz, $CDCl_3$) δ 216.5, 178.2, 157.1, 139.9, 131.6, 128.7, 125.2, 122.2, 119.6, 107.6, 99.4, 86.3, 75.5, 75.0, 73.8, 71.6, 71.2, 68.3, 55.9, 50.0, 48.4, 47.7, 39.8, 38.3, 37.6, 36.3, 33.5, 32.5, 30.9, 29.1, 28.2, 26.1, 24.4, 22.1, 21.1, 20.2, 17.8, 16.2, 15.8, 14.6, 13.3, 13.2, 12.1, 11.8, 7.3, 6.6 ppm.

Example FLC-00381-1

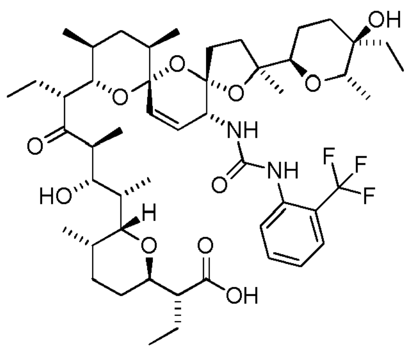

The compound was prepared according to the procedure described in example FLC-0337-1, except that 2-trifluoromethylphenyl isocyanate (1.2 eq) and, additionally, TEA (2 eq), was added to the reaction mixture instead of benzyl isocyanate. The yield of the obtained product was 69 %.

ESI-MS for $C_{50}H_{75}F_3N_2O_{11}$ (*m/z*): $[M-H]^-$ 936.3, $[M+Na]^+$ 959.9.

$^1H$ NMR (500 MHz, $CDCl_3$) δ 8.01 (d, *J* = 8.0 Hz, 1H), 7.59 (s, *J* = 18.1 Hz, 1H), 7.54 (d, *J* = 7.4 Hz, 1H), 7.46 (t, *J* = 7.4 Hz, 1H), 7.10 (t, *J* = 7.1 Hz, 1H), 6.36 (d, *J* = 8.1 Hz, 1H), 6.16 (s, 2H), 4.49 (d, *J* = 6.6 Hz, 1H), 4.20 (d, *J* = 9.7 Hz, 1H), 3.95 (d, *J* = 4.3 Hz, 1H), 3.76 (t, *J* = 9.6 Hz, 2H), 3.70 - 3.63 (m, 2H), 2.92 (t, *J* = 8.7 Hz, 1H), 2.72 - 2.64 (m, 1H), 2.60 (d, *J* = 10.6 Hz, 1H), 2.11 - 0.59 (m, 57H) ppm.

$^{13}C$ NMR (126 MHz, $CDCl_3$) δ 215.4, 177.7, 156.5, 137.2, 132.4, 131.0, 126.0, 125.3, 124.7, 123.2, 122.9, 121.9,

107.1, 99.6, 87.1, 75.5, 74.9, 73.7, 71.5, 71.2, 68.2, 55.7, 50.2, 49.6, 48.1, 40.2, 38.5, 37.1, 36.4, 32.6, 30.6, 29.8, 29.0, 28.2, 26.1, 24.3, 22.4, 21.5, 20.1, 17.8, 16.5, 15.8, 14.7, 13.1, 12.8, 12.0, 11.6, 7.2, 6.4 ppm.

Example FLC-00383-1

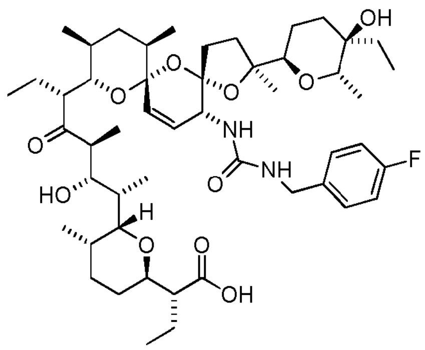

The compound was prepared according to the procedure described in example FLC-0337-1, except that 4-fluorobenzyl isocyanate (1.2 eq) was added to the reaction mixture instead of benzyl isocyanate. The yield of the obtained product was 36 %.

ESI-MS for $C_{50}H_{77}FN_2O_{11}$ (*m/z*): $[M-H]^-$ 900.0, $[M+Na]^+$ 923.9.

$^1$H NMR (500 MHz, $CD_2Cl_2$) δ 7.37 (dd, *J* = 8.5, 5.6 Hz, 2H), 7.02 - 6.97 (m, 2H), 6.72 (s, 1H), 6.14 (d, *J* = 10.0 Hz, 1H), 5.83 (d, *J* = 10.0 Hz, 1H), 4.51 - 4.45 (m, 2H), 4.27 (dd, *J*= 14.9, 4.5 Hz, 1H), 4.10 (dd, *J* = 10.0, 1.3 Hz, 1H), 3.92 (dd, *J* = 11.0, 5.4 Hz, 1H), 3.76 - 3.71 (m, 1H), 3.69 - 3.66 (m, 2H), 3.44 (dd, *J* = 10.9, 2.4 Hz, 1H), 2.97 (td, *J* = 11.0, 4.1 Hz, 1H), 2.69 - 2.59 (m, 2H), 2.01 - 0.56 (m, 58H) ppm.

$^{13}$C NMR (126 MHz, $CD_2Cl_2$) δ 217.5, 178.2, 162.31 (d) 160.4, 135.9, 132.9, 129.9, 129.8, 126.7, 115.3, 115.2, 109.2, 100.2, 86.7, 77.5, 75.7, 75.3, 73.8, 71.5, 71.2, 68.4, 56.0, 50.7, 49.3, 48.0, 43.8, 39.7, 38.3, 37.9, 36.8, 33.9, 32.7, 30.7, 29.6, 28.4, 26.1, 24.0, 22.4, 21.3, 20.1, 17.6, 16.5, 15.8, 14.6, 13.1, 13.0, 12.2, 11.8, 7.3, 6.6 ppm.

Example FLC-00384-1

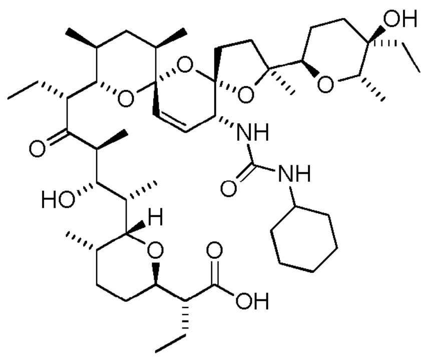

The compound was prepared according to the procedure described in example FLC-0337-1, except that cyclohexyl isocyanate (1.2 eq) was added to the reaction mixture instead of benzyl isocyanate. The yield of the obtained product was 21 %.

ESI-MS for $C_{49}H_{82}N_2O_{11}$ (*m/z*): $[M+H]^+$ 876.2, $[M+Na]^+$ 898.0.

$^1$H NMR (500 MHz, $CD_2Cl_2$) δ 6.24 (dd, *J* = 9.6, 6.2 Hz, 2H), 6.10 (d, *J*= 10.0 Hz, 1H), 5.59 (d, *J*= 9.9 Hz, 1H), 4.41 (dd, *J* = 9.8, 6.2 Hz, 1H), 4.09 (d, *J* = 9.9 Hz, 1H), 3.88 (dd, *J* = 10.8, 5.0 Hz, 1H), 3.75 (q, *J* = 6.6 Hz, 1H), 3.68 (d, *J* = 10.2 Hz, 1H), 3.62 (d, *J* = 9.7 Hz, 1H), 3.56 (dd, *J* = 13.3, 9.3 Hz, 1H), 3.47 (d, *J* = 7.1 Hz, 1H), 2.95 (td, *J* = 10.9, 3.6 Hz, 1H), 2.70 - 2.61 (m, 2H), 2.03 - 0.64 (m, 67H) ppm.

$^{13}$C NMR (126 MHz, $CD_2Cl_2$) δ 217.2, 178.2, 159.6, 133.0, 126.1, 109.0, 100.2, 86.7, 77.5, 75.7, 75.5, 73.9, 71.3, 71.2, 68.4, 56.1, 50.7, 49.0, 47.9, 39.8, 38.3, 37.8, 36.8, 34.5, 33.9, 33.4, 32.8, 30.6, 29.7, 28.4, 26.1, 26.1, 25.4, 25.2, 23.8, 22.4, 21.3, 20.2, 17.6, 16.5, 15.8, 14.7, 13.2, 12.3, 11.8, 7.3, 6.6 ppm.

Example FLC-00385-1

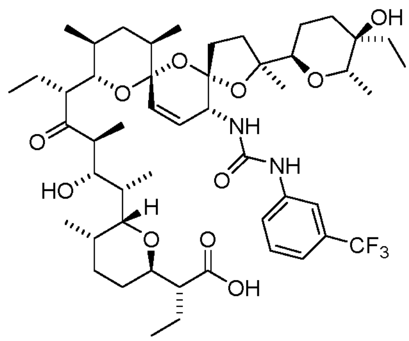

The compound was prepared according to the procedure described in example FLC-0337-1, except that 3-trifluoromethylphenyl isocyanate (1.2 eq) and, additionally, TEA (2 eq) was added to the reaction mixture instead of benzyl isocyanate. The yield of the obtained product was 14 %.

ESI-MS for $C_{50}H_{75}F_3N_2O_{11}$ (*m/z*): $[M-H]^-$ 936.1, $[M+H]^+$ 938.2, $[M+Na]^+$ 959.9.

$^1H$ NMR (500 MHz, $CD_2Cl_2$) δ 8.85 (s, 1H), 7.70 (s, 1H), 7.59 (d, *J* = 8.3 Hz, 1H), 7.38 (t, *J*= 7.9 Hz, 1H), 7.23 (d, *J* = 7.7 Hz, 1H), 6.22 - 6.16 (m, 2H), 6.11 (d, *J* = 9.9 Hz, 1H), 4.54 (dd, *J* = 9.9, 4.4 Hz, 1H), 4.20 (dd, *J* = 10.1, 1.3 Hz, 1H), 3.83 (q, *J* = 6.8 Hz, 1H), 3.70 (d, *J* = 10.2 Hz, 1H), 3.59 (dd, *J* = 10.0, 2.2 Hz, 1H), 3.52 (dd, *J* = 11.6, 2.7 Hz, 2H), 2.87 (td, *J* = 10.7, 4.7 Hz, 1H), 2.79 - 2.73 (m, 1H), 2.70 (d, *J* = 10.0 Hz, 1H), 2.07 - 0.64 (m, 57H) ppm.

$^{13}C$ NMR (126 MHz, $CD_2Cl_2$) δ 218.6, 178.5, 157.6, 140.7, 131.8, 130.94 (d) 129.4, 126.5, 125.8, 123.4, 119.0, 116.7, 108.4, 100.1, 86.9, 77.4, 76.0, 75.3, 73.8, 71.6, 71.4, 68.5, 56.0, 50.5, 48.9, 47.6, 39.9, 38.3, 38.1, 36.6, 33.6, 32.8, 31.1, 29.4, 28.4, 26.1, 24.3, 22.1, 21.3, 20.1, 17.6, 16.5, 15.8, 14.6, 13.2, 13.1, 12.1, 11.7, 7.4, 6.6 ppm.

Example FLC-00388-1

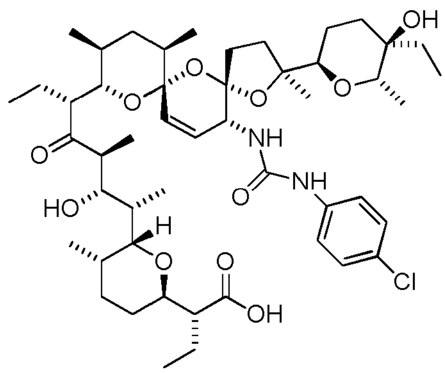

The compound was prepared according to the procedure described in example FLC-0337-1, except that 4-chlorophenyl isocyanate (1.1 eq) was added to the reaction mixture instead of benzyl isocyanate. Following purification, the compound was washed with 0.25 M $Na_2CO_3$ instead of 0.06 M $H_2SO_4$. The yield of the obtained product was 22 %.

ESI-MS for $C_{49}H_{75}ClN_2O_{11}$ (*m/z*): $[M+H]^+$ 904.0, $[M+Na]^+$ 926.0, $[M-H]^-$ 902.0.

$^1H$ NMR (500 MHz, $CD_2Cl_2$) δ 8.41 (s, 1H), 7.43 (d, *J* = 8.9 Hz, 2H), 7.14 (d, *J* = 8.9 Hz, 2H), 6.22 (d, *J* = 9.7 Hz, 1H), 6.12 (dd, *J* = 10.8, 2.9 Hz, 1H), 5.72 (dd, *J* = 10.8, 1.6 Hz, 1H), 4.70 (d, *J* = 9.6 Hz, 1H), 4.35 (q, *J* = 6.5 Hz, 1H), 4.08 (d, *J* = 10.4 Hz, 1H), 3.85 (dd, *J* = 10.9, 5.0 Hz, 1H), 3.63 - 3.57 (m, 2H), 3.47 (dd, *J* = 12.3, 2.7 Hz, 1H), 2.83 (td, *J* = 11.1, 3.2 Hz, 1H), 2.75 - 2.66 (m, 2H), 2.12 - 0.69 (m, 56H) ppm.

$^{13}C$ NMR (126 MHz, $CD_2Cl_2$) δ 219.2, 185.0, 154.7, 139.5, 128.6, 128.3, 125.6, 123.0, 119.1, 106.4, 98.7, 88.5, 75.8, 75.5, 75.4, 73.7, 71.6, 71.1, 68.1, 56.1, 50.2, 49.5, 46.5, 40.5, 38.5, 37.2, 35.7, 32.5, 32.4, 29.7, 28.0, 27.6, 26.6, 23.3, 19.8, 17.2, 15.7, 15.4, 14.3, 12.8, 12.1, 12.0, 10.6, 6.4 ppm.

Example FLC-00389-1

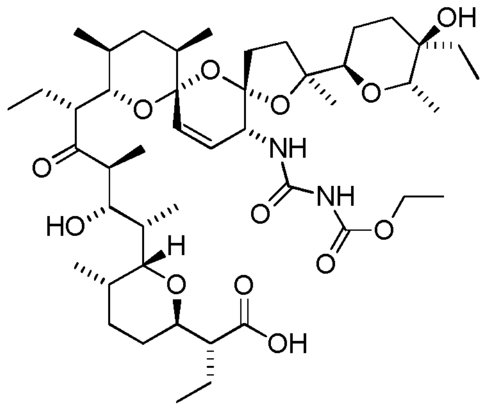

The compound was prepared according to the procedure described in example FLC-0337-1, except that etoxycarbonyl isocyanate (1.2 eq) was added to the reaction mixture instead of benzyl isocyanate. Following purification, the compound was washed with 0.25 M $Na_2CO_3$ instead of 0.06 M $H_2SO_4$. The yield of the obtained product was 30 %.

ESI-MS for $C_{46}H_{76}N_2O_{13}$ (m/z): $[M+Na]^+$ 888.0, $[M-H]^-$ 864.2.

$^1H$ NMR (500 MHz, $CD_2Cl_2$) δ 9.21 (s, 1H), 6.35 (d, *J* = 9.7 Hz, 1H), 6.11 (dd, *J* = 10.9, 3.0 Hz, 1H), 5.70 (dd, *J* = 10.8, 1.8 Hz, 1H), 4.74 (s, 1H), 4.72 - 4.66 (m, 1H), 4.29 (q, *J* = 6.6 Hz, 1H), 4.10 - 4.04 (m, 3H), 3.83 (dd, *J* = 11.0, 4.7 Hz, 1H), 3.63 - 3.59 (m, 2H), 3.50 (dd, *J* = 12.3, 3.1 Hz, 1H), 2.82 (td, *J* = 11.0, 3.4 Hz, 1H), 2.77 - 2.67 (m, 2H), 2.08 - 0.66 (m, 50H) ppm.

$^{13}C$ NMR (126 MHz, $CD_2Cl_2$) δ 219.8, 185.4, 152.6, 152.4, 128.5, 123.6, 106.4, 99.2, 89.1, 76.3, 76.2, 75.9, 73.8, 71.9, 71.5, 68.5, 61.1, 56.5, 50.7, 50.0, 46.9, 40.9, 38.9, 37.6, 36.1, 32.8, 32.8, 32.2, 28.4, 28.0, 27.0, 26.8, 23.8, 20.2, 20.2, 17.6, 16.1, 15.8, 14.6, 14.5, 13.2, 12.3, 12.3, 10.9, 6.8, 6.7 ppm.

Example FLC-00390-1

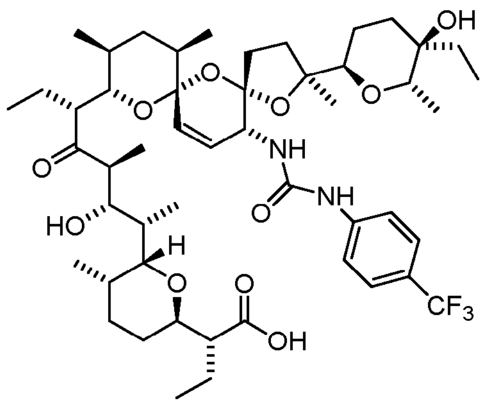

The compound was prepared according to the procedure described in example FLC-0337-1, except that 4-trifluoromethylphenyl isocyanate (1.2 eq) was added to the reaction mixture instead of benzyl isocyanate. The yield of the obtained product was 33 %.

ESI-MS for $C_{50}H_{75}F_3N_2O_{11}$ (*m/z*): $[M-H]^-$ 935.9, $[M+Na]^+$ 960.3.

$^1H$ NMR (500 MHz, $CD_2Cl_2$) δ 8.78 (s, 1H), 7.55 (d, *J* = 8.7 Hz, 2H), 7.50 (d, *J* = 8.7 Hz, 2H), 6.20 - 6.11 (m, *J* = 21.0, 10.1, 5.2 Hz, 3H), 4.54 (dd, *J* = 9.8, 4.8 Hz, 1H), 4.17 - 4.12 (m, 1H), 3.85 (q, *J* = 6.8 Hz, 1H), 3.73 (d, *J* = 10.2 Hz, 1H), 3.67 - 3.63 (m, 1H), 3.60 (dd, *J* = 10.0, 2.2 Hz, 1H), 3.51 (dd, *J* = 11.8, 2.4 Hz, 1H), 2.89 (td, *J* = 10.9, 4.3 Hz, 1H), 2.76 (dq, *J* = 14.4, 7.2 Hz, 1H), 2.69 (d, *J* = 9.9 Hz, 1H), 2.04 - 0.64 (m, 57H) ppm.

$^{13}C$ NMR (126 MHz, $CD_2Cl_2$) δ 218.2, 178.6, 156.9, 143.8, 131.5, 126.2, 126.2, 123.73 (m) 118.9, 108.1, 100.0, 87.1, 77.2, 76.1, 75.4, 73.8, 73.1, 71.7, 71.5, 68.5, 56.0, 50.3, 48.8, 47.9, 40.1, 38.4, 38.0, 36.6, 33.4, 32.9, 31.3, 29.4, 28.5,

26.1, 24.9, 22.3, 21.2, 20.2, 17.7, 16.4, 15.9, 14.6, 13.3, 13.2, 12.1, 11.7, 7.3, 6.7 ppm.

Example FLC-00391-1

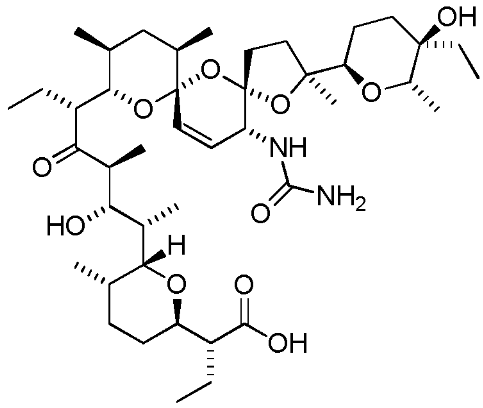

The compound was prepared according to the procedure described in example FLC-0337-1, except that trimethylsilyl isocyanate (1.2 eq) was added to the reaction mixture instead of benzyl isocyanate. Following purification, the compound was washed with 0.25 M $Na_2CO_3$ instead of 0.06 M $H_2SO_4$. The yield of the obtained product was 81 %.

ESI-MS for $C_{43}H_{72}N_2O_{11}$ (*m/z*): $[M-H]^-$ 792.0, $[M+H]^+$ 794.0, $[M+Na]^+$ 816.8.

$^1H$ NMR (500 MHz, $CDCl_3$) δ 6,06 (dd, *J* = 10.9, 2.9 Hz, 1H), 5.97 (d, *J* = 10.0 Hz, 1H), 5.78 (dd, *J* = 10.8, 1.7 Hz, 1H), 5.42 (s, 1H), 5.03 (s, 1H), 4.85 (d, *J* = 3.8 Hz, 1H), 4.64 - 4.60 (m, 1H), 4.33 (q, *J* = 6.7 Hz, 1H), 4.13 (d, *J* = 10.0 Hz, 1H), 3.91 (dd, *J* = 11.0, 5.1 Hz, 1H), 3.66 (dd, *J* = 10.1, 1.7 Hz, 1H), 3.56 (d, *J* = 10.1 Hz, 1H), 3.43 (dd, *J* = 12.3, 2.8 Hz, 1H), 2.85 (td, *J* = 11.0, 3.2 Hz, 1H), 2.70 - 2.60 (m, 2H), 2.33 (s, 1H), 2.23 - 0.63 (m, 55H) ppm.

$^{13}C$ NMR (126 MHz, $CDCl_3$) δ 218.6, 185.0, 158.8, 129.5, 122.4, 106.6, 98.8, 88.5, 76.0, 75.5, 75.4, 74.0, 71.4, 71.1, 68.0, 56.1, 50.8, 50.0, 47.0, 40.6, 38.7, 37.1, 35.9, 32.7, 32.5, 32.2, 28.1, 28.0, 26.9, 23.6, 20.0, 19.9, 17.6, 16.0, 15.7, 14.6, 13.2, 12.4, 12.1, 10.8, 6.8, 6.5 ppm.

Example FLC-00396-1

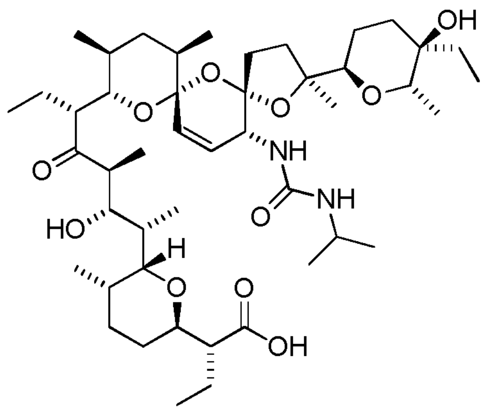

The compound was prepared according to the procedure described in example FLC-0337-1, except that isopropyl isocyanate (1.2 eq) was added to the reaction mixture instead of benzyl isocyanate. The yield of the obtained product was 38 %.

ESI-MS for $C_{46}H_{78}N_2O_{11}$ (*m/z*): $[M-H]^-$ 834.0, $[M+H]^+$ 836.2, $[M+Na]^+$ 858.9.

$^1H$ NMR (500 MHz, $CDCl_3$) δ 6.19 (dd, *J* = 9.6, 4.9 Hz, 1H), 6.08 (d, *J* = 10.1 Hz, 1H), 5.83 (s, 1H), 5.63 (d, *J* = 9.8 Hz, 1H), 4.43 (dd, *J* = 9.7, 5.2 Hz, 1H), 4.10 (d, *J*= 10.1 Hz, 1H), 3.98 - 3.86 (m, 2H), 3.81 (q, *J* = 6.8 Hz, 1H), 3.73 (d, *J* = 10.2 Hz, 1H), 3.66 (dd, *J* = 10.1, 1.5 Hz, 1H), 3.59 - 3.54 (m, 1H), 2.93 (td, *J* = 11.0, 3.8 Hz, 1H), 2.66 (dq, *J* = 14.1, 7.0 Hz, 1H), 2.56 (d, *J* = 10.1 Hz, 1H), 2.08 - 0.64 (m, 63H) ppm.

$^{13}C$ NMR (126 MHz, $CDCl_3$) δ 216.0, 177.9, 158.8, 132.3, 124.8, 108.0, 99.5, 86.1, 77.0, 75.2, 75.1, 73.8, 71.2, 71.1, 68.2, 56.0, 50.1, 48.5, 47.8, 41.8, 39.7, 38.2, 37.1, 36.3, 33.5, 32.4, 30.2, 29.7, 29.1, 28.1, 26.0, 23.9, 23.0, 22.2, 21.1, 20.0, 17.7, 16.3, 15.7, 14.5, 13.2, 13.0, 12.1, 11.7, 7.1, 6.5 ppm.

Example FLC-00427-1

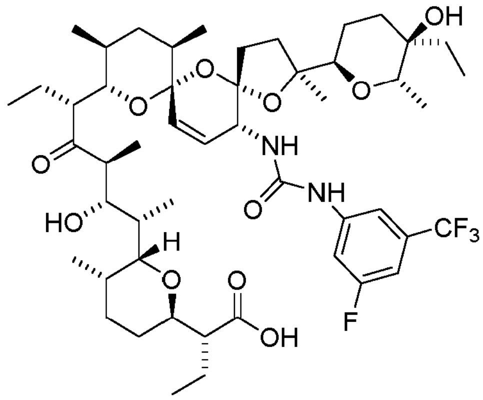

The compound was prepared according to the procedure described in example FLC-0337-1, except that 2-fluoro-5-trifluoromethylphenyl isocyanate (1.2 eq) was added to the reaction mixture instead of benzyl isocyanate. Following purification, the compound was washed with 0.25 M $Na_2CO_3$ instead of 0.06 M $H_2SO_4$. The yield of the obtained product was 50 %.

ESI-MS for $C_{50}H_{74}F_4N_2O_{11}$ (*m/z*): $[M-H]^-$ 954.2, $[M+Na]^+$ 978.0.

$^1H$ NMR (500 MHz, $CDCl_3$) δ 8.76 (s, 1H), 7.63 (d, *J*= 11.3 Hz, 1H), 7.44 (s, *J* = 11.4 Hz, 1H), 6.83 (d, *J* = 8.2 Hz, 1H), 6.38 (d, *J* = 9.7 Hz, 1H), 6.13 (dd, *J* = 10.9, 2.9 Hz, 1H), 5.96 (s, 1H), 5.82 (dd, *J* = 10.8, 1.8 Hz, 1H), 4.78 - 4.72 (m, 1H), 4.51 (d, *J* = 4.1 Hz, 1H), 4.44 (q, *J* = 6.7 Hz, 1H), 4.16 (dd, *J* = 10.3, 2.2 Hz, 1H), 3.95 (dd, *J* = 11.1, 5.4 Hz, 1H), 3.64 (dd, *J* = 10.1, 1.7 Hz, 1H), 3.57 (d, *J* = 10.1 Hz, 1H), 3.46 (dd, *J* = 12.3, 2.6 Hz, 1H), 2.87 (td, *J* = 11.0, 3.1 Hz, 1H), 2.72 - 2.64 (m, 2H), 2.20 - 0.64 (m, 55H) ppm.

$^{13}C$ NMR (126 MHz, $CDCl_3$) δ 218.7, 185.6, 163.9, 162.0, 154.9, 143.1, 143.0, 128.5, 123.2, 110.5, 108.2, 106.4, 105.0, 98.8, 88.6, 75.9, 75.7, 75.4, 74.0, 72.0, 71.5, 68.3, 56.2, 50.8, 49.7, 46.6, 40.6, 38.7, 37.5, 35.9, 32.7, 32.6, 32.5, 28.4, 28.1, 28.0, 26.9, 23.8, 20.2, 19.9, 17.6, 16.0, 15.6, 14.6, 13.2, 12.2, 12.2, 10.8, 6.8, 6.7 ppm.

Example FLC-00428-1

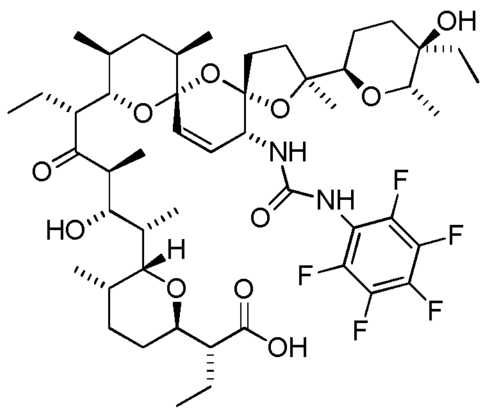

The compound was prepared according to the procedure described in example FLC-0337-1, except that pentafluorophenyl isocyanate (1.2 eq) was added to the reaction mixture instead of phenyl isocyanate. Following purification, the compound was washed with 0.25 M $Na_2CO_3$ instead of 0.06 M $H_2SO_4$. The yield of the obtained product was 70 %.

ESI-MS for $C_{49}H_{71}F_5N_2O_{11}$ (*m/z*): $[M-H]^-$ 958.3, $[M+Na]^+$ 982.1.

$^1H$ NMR (500 MHz, $CDCl_3$) δ 8,19 (s, 1H), 6.55 (d, *J* = 9.8 Hz, 1H), 6.12 (dd, *J* = 10.8, 2.6 Hz, 1H), 5.83 (d, *J* = 10.8 Hz, 1H), 4.92 (s, 1H), 4.71 (d, *J* = 9.8 Hz, 1H), 4.36 (q, *J* = 6.6 Hz, 1H), 4.13 (d, *J* = 10.4 Hz, 1H), 3.93 (dd, *J*= 10.9, 5.1 Hz, 1H), 3.72 - 3.67 (m, 1H), 3.60 (d, *J* = 10.1 Hz, 1H), 3.49 (dd, *J* = 12.3, 2.4 Hz, 1H), 2.87 (td, *J* = 11.1, 2.9 Hz, 1H), 2.69 (dt, *J* = 18.5, 7.9 Hz, 2H), 2.29 - 0.63 (m, 56H) ppm.

$^{13}C$ NMR (126 MHz, $CDCl_3$) δ 218.9, 185.2, 154.8, 144.2, 140.3, 138.8, 136.8, 129.0, 122.7, 114.4, 106.3, 98.9, 88.7, 76.2, 75.5, 75.5, 74.0, 71.7, 71.4, 68.2, 56.2, 50.5, 50.0, 47.5, 40.6, 38.7, 37.3, 35.8, 32.7, 32.5, 32.1, 28.1, 28.0, 27.4, 26.8, 23.3, 20.1, 20.0, 17.6, 16.0, 15.7, 14.5, 13.3, 12.1, 12.0, 10.9, 6.8, 6.4 ppm.

Example FLC-00539-1

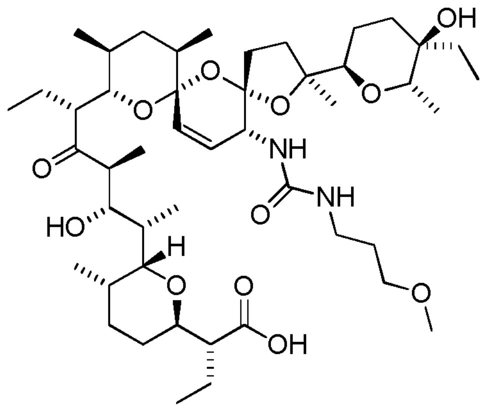

100 mg FLC-00604-1 was mixed with 3-methoxypropylamine (2 eq) and DIPEA (2 eq) in THF. The reaction was carried out at RT (TLC and LC-MS control). The post-reaction mixture was suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was 64%.

ESI-MS for $C_{47}H_{80}N_2O_{12}$ (*m/z*): $[M+H]^+$ 866.0, $[M+Na]^+$ 888.1, $[M-H]^-$ 864.2.

$^1H$ NMR (500 MHz, $CD_2Cl_2$) δ 6.05 (dd, *J* = 10.9, 3.0 Hz, 1H), 5.82 (t, *J* = 5.6 Hz, 1H), 5.69 - 5.62 (m, 2H), 4.84 (s, 1H), 4.63 - 4.56 (m, 1H), 4.30 (q, *J* = 6.7 Hz, 1H), 4.06 (d, *J* = 10.4 Hz, 1H), 3.80 (dd, *J* = 11.0, 4.5 Hz, 1H), 3.60 (dd, *J* = 15.8, 6.0 Hz, 2H), 3.45 (dd, *J* = 12.3, 2.8 Hz, 1H), 3.33 (t, *J* = 6.5 Hz, 2H), 3.25 (s, 3H), 3.09 (dt, *J* = 13.3, 6.8 Hz, 2H), 2.81 (td, *J* = 11.1, 3.2 Hz, 1H), 2.73 - 2.65 (m, 2H), 2.14 - 0.66 (m, 57H) ppm.

$^{13}C$ NMR (126 MHz, $CD_2Cl_2$) δ 219.1, 184.5, 157.7, 129.6, 122.4, 106.6, 98.7, 88.3, 75.8, 75.6, 75.4, 73.8, 71.1, 70.9, 70.4, 67.9, 58.2, 56.0, 50.4, 49.6, 46.6, 40.5, 38.6, 37.1, 36.9, 35.8, 32.5, 32.4, 32.2, 30.5, 28.0, 27.8, 27.7, 26.7, 23.5, 19.9, 19.6, 17.2, 15.8, 15.5, 14.3, 12.9, 12.4, 11.9, 10.5, 6.4, 6.2 ppm.

Example FLC-00541-1

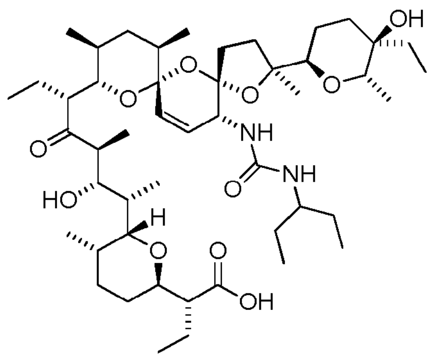

The compound was prepared according to the procedure described in example FLC-00539-1, except that 3-pentylamine (2 eq) was added to the reaction mixture instead of 3-methoxypropylamine. The yield of the obtained product was 53%.

ESI-MS for $C_{48}H_{82}N_2O_{11}$ (*m/z*): $[M+H]^+$ 864.2, $[M+Na]^+$ 886.2, $[M-H]^-$ 862.1.

$^1H$ NMR (500 MHz, $CD_2Cl_2$) δ 6.05 (dd, *J* = 10.9, 3.0 Hz, 1H), 5.70 (dd, *J* = 10.8, 1.7 Hz, 1H), 5.43 (d, *J* = 10.1 Hz, 1H), 5.27 (d, *J* = 9.1 Hz, 1H), 4.67 - 4.62 (m, 1H), 4.33 (q, *J* = 6.6 Hz, 1H), 4.07 (d, *J* = 10.4 Hz, 1H), 3.80 (dd, *J* = 11.0, 4.5 Hz, 1H), 3.65 (d, *J* = 1.8 Hz, 1H), 3.59 (d, *J* = 10.2 Hz, 1H), 3.46 (ddd, *J* = 13.2, 9.8, 3.0 Hz, 2H), 2.81 (td, *J* = 11.0, 3.3 Hz, 1H), 2.74 - 2.65 (m, 2H), 2.20 - 0.66 (m, 66H) ppm.

$^{13}C$ NMR (126 MHz, $CD_2Cl_2$) δ 219.1, 184.2, 157.3, 130.3, 122.1, 106.6, 98.9, 88.3, 75.8, 75.6, 75.3, 73.8, 71.2, 70.9, 67.8, 56.0, 52.7, 50.4, 49.8, 46.6, 40.6, 38.6, 36.5, 35.8, 32.5, 32.4, 32.1, 28.5, 28.1, 28.0, 27.8, 27.3, 26.7, 23.5, 19.9, 19.8, 17.2, 15.7, 15.6, 14.3, 12.9, 12.8, 11.9, 10.6, 10.5, 10.4, 6.4, 6.3 ppm.

Example FLC-00545-1

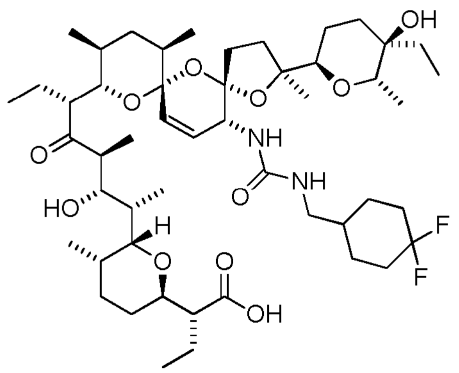

The compound was prepared according to the procedure described in example FLC-00539-1, except that (4,4-difluorocyclohexyl)methylamine*HCl (1 eq) was added to the reaction mixture instead of 3-methoxypropylamine. The yield of the obtained product was 51%.

ESI-MS for $C_{50}H_{82}F_2N_2O_{11}$ (*m/z*): $[M+H]^+$ 926.0, $[M+Na]^+$ 948.2, $[M-H]^-$ 924.1.

$^1H$ NMR (500 MHz, $CD_2Cl_2$) δ 6.06 (dd, *J* = 10.9, 3.0 Hz, 1H), 5.86 (t, *J* = 5.8 Hz, 1H), 5.68 - 5.63 (m, 2H), 4.84 (s, 1H), 4.63 - 4.56 (m, 1H), 4.31 (q, *J* = 6.6 Hz, 1H), 4.07 (d, *J* = 10.4 Hz, 1H), 3.81 (dd, *J* = 11.0, 4.5 Hz, 1H), 3.62 (d, *J* = 1.8 Hz, 1H), 3.58 (d, *J* = 10.1 Hz, 1H), 3.46 (dd, *J* = 12.3, 2.8 Hz, 1H), 3.01 (dt, *J* = 12.9, 6.3 Hz, 1H), 2.96 - 2.89 (m, 1H), 2.81 (td, *J* = 11.0, 3.2 Hz, 1H), 2.74 - 2.67 (m, 2H), 2.16 - 0.67 (m, 64H) ppm.

$^{13}C$ NMR (126 MHz, $CD_2Cl_2$) δ 219.1, 184.5, 157.8, 129.6, 122.4, 106.6, 98.8, 88.3, 75.8, 75.6, 75.4, 73.8, 71.1, 70.9, 67.9, 56.0, 50.4, 49.6, 46.7, 44.9, 40.5, 38.6, 36.9, 36.8, 35.8, 33.3, 33.1, 32.9, 32.5, 32.4, 32.2, 29.7, 28.0, 27.8, 27.7, 26.8, 26.8, 26.7, 26.6, 23.4, 19.9, 19.6, 17.2, 15.7, 15.5, 14.3, 12.8, 12.4, 11.9, 10.5, 6.4, 6.2 ppm.

Example FLC-00546-1

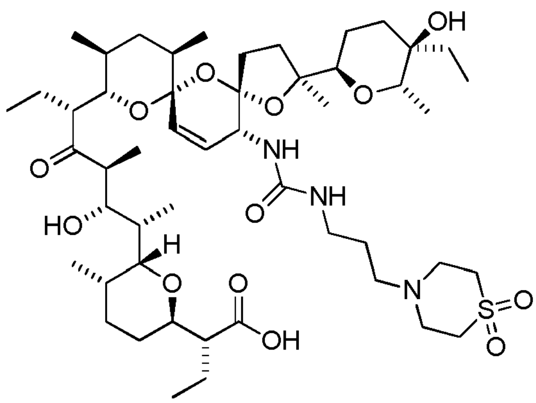

The compound was prepared according to the procedure described in example FLC-00539-1, except that (4-(3-aminopropylo)thiomorpholine) 1,1-dioxide (1 eq) was added to the reaction mixture instead of 3-methoxypropylamine. The yield of the obtained product was 14%.

ESI-MS for $C_{50}H_{85}N_3O_{13}S$ (*m/z*): $[M+H]^+$ 969.2, $[M+Na]^+$ 991.0, $[M-H]^-$ 967.1.

$^1$H NMR (500 MHz, $CD_2Cl_2$) δ 6.06 (dd, *J*= 10.9, 3.0 Hz, 1H), 5.86 (t, *J*= 5.6 Hz, 1H), 5.73 - 5.63 (m, 2H), 4.62 - 4.56 (m, 1H), 4.29 (q, *J* = 6.6 Hz, 1H), 4.07 (d, *J* = 10.3 Hz, 1H), 3.81 (dd, *J* = 11.0, 4.6 Hz, 1H), 3.60 (dd, *J* = 16.7, 6.0 Hz, 2H), 3.46 (dd, *J* = 12.3, 2.7 Hz, 1H), 3.16 - 3.02 (m, 2H), 3.02 - 2.96 (m, 4H), 2.91 (dd, *J* = 6.5, 3.4 Hz, 4H), 2.82 (td, *J* = 11.1, 3.3 Hz, 1H), 2.74 - 2.67 (m, 2H), 2.48 (dd, *J* = 12.6, 7.0 Hz, 2H), 2.11 - 0.67 (m, 58H) ppm.

$^{13}$C NMR (126 MHz, $CD_2Cl_2$) δ 219.6, 185.0, 158.2, 129.9, 123.0, 107.1, 99.2, 88.8, 76.3, 76.2, 75.9, 74.3, 71.6, 71.3, 68.4, 56.5, 54.7, 51.9, 51.2, 50.9, 50.0, 47.1, 41.0, 39.0, 38.3, 37.4, 36.2, 32.9, 32.9, 32.7, 30.2, 28.6, 28.4, 28.3, 28.1, 27.1, 23.9, 20.3, 20.1, 17.7, 16.2, 16.0, 14.8, 13.3, 12.9, 12.4, 11.0, 6.9, 6.7 ppm.

Example FLC-00562-1

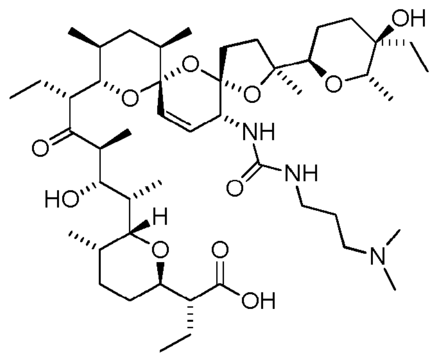

The compound was prepared according to the procedure described in example FLC-00539-1, except that 3-dimethylaminopropylamine (2 eq) was added to the reaction mixture instead of 3-methoxypropylamine. The yield of the obtained product was 70%.

ESI-MS for $C_{48}H_{83}N_3O_{11}$ (*m/z*): $[M+H]^+$ 878.9, $[M+Na]^+$ 901.0, $[M-H]^-$ 877.1.

$^1$H NMR (500 MHz, $CDCl_3$) δ 6.05 (dd, *J* = 10.8, 2.9 Hz, 1H), 5.92 (t, *J* = 5.6 Hz, 1H), 5.78 (dd, *J*= 10.8, 1.7 Hz, 1H), 5.63 (d, *J* = 10.0 Hz, 1H), 4.81 (d, *J* = 4.4 Hz, 1H), 4.71 - 4.62 (m, 1H), 4.34 (t, *J* = 6.6 Hz, 1H), 4.13 (dd, *J* = 10.1, 3.0 Hz, 1H), 3.91 (dd, *J* = 11.0, 5.1 Hz, 1H), 3.66 (dd, *J* = 10.1, 1.6 Hz, 1H), 3.54 (t, *J* = 8.8 Hz, 1H), 3.48 - 3.40 (m, 2H), 3.21 - 3.13 (m, 1H), 3.06 (td, *J* = 13.0, 7.2 Hz, 1H), 2.85 (td, *J* = 11.1, 3.2 Hz, 1H), 2.66 (dd, *J* = 10.4, 7.8 Hz, 2H), 2.26 (dd, *J* = 8.3, 5.8 Hz, 2H), 2.19 (s, 6H), 2.18 - 0.67 (m, 56H) ppm.

$^{13}$C NMR (126 MHz, $CDCl_3$) δ 218.4, 184.8, 158.1, 129.9, 122.1, 106.6, 98.7, 88.3, 75.9, 75.5, 75.3, 73.9, 71.3, 71.0, 67.9, 57.5, 56.0, 50.8, 49.9, 46.6, 45.5, 40.5, 38.6, 38.5, 36.9, 35.8, 32.6, 32.4, 32.3, 29.7, 28.6, 28.0, 27.9, 26.8, 23.6, 19.9, 19.7, 17.5, 16.0, 15.6, 14.5, 13.2, 12.5, 12.0, 10.7, 6.7, 6.5 ppm.

Example FLC-00566-1

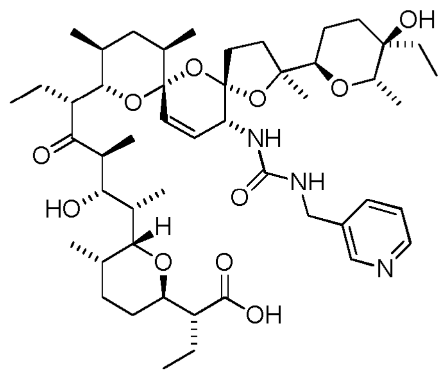

The compound was prepared according to the procedure described in example FLC-00539-1, except that 3-(aminomethyl)pirydine (1.5 eq) was added to the reaction mixture instead of 3-methoxypropylamine. The yield of the obtained product was 52%.

ESI-MS for $C_{49}H_{77}N_3O_{11}$ (*m/z*): $[M+H]^+$ 885.1, $[M+Na]^+$ 907.0, $[M-H]^-$ 883.1.

$^1H$ NMR (500 MHz, $CDCl_3$) δ 8.46 (d, J = 1.8 Hz, 1H), 8.40 (dd, J = 4.8, 1.5 Hz, 1H), 7.60 (d, J = 7.8 Hz, 1H), 7.13 (dd, J = 7.7, 4.8 Hz, 1H), 6.42 (t, J = 5.8 Hz, 1H), 6.02 (dd, J = 10.9, 2.9 Hz, 1H), 5.82 - 5.71 (m, 2H), 5.32 (s, 1H), 4.67 (dd, J = 7.5, 3.6 Hz, 2H), 4.34 - 4.23 (m, 2H), 4.19 (dd, J = 15.0, 5.1 Hz, 1H), 4.08 (dd, J = 10.3, 3.3 Hz, 1H), 3.84 (dd, J = 11.0, 4.9 Hz, 1H), 3.59 (dd, J = 10.1, 1.6 Hz, 1H), 3.51 (d, J = 10.2 Hz, 1H), 3.36 (dd, J = 12.3, 2.7 Hz, 1H), 2.78 (td, J = 11.1, 3.1 Hz, 1H), 2.65 - 2.58 (m, 2H), 2.02 - 0.62 (m, 54H) ppm.

$^{13}C$ NMR (126 MHz, $CDCl_3$) δ 218.5, 184.9, 158.0, 149.5, 148.3, 136.0, 135.8, 129.5, 123.2, 122.4, 106.5, 98.7, 88.4, 75.8, 75.6, 75.4, 73.9, 71.3, 71.0, 68.0, 56.0, 50.8, 49.8, 46.8, 41.6, 40.5, 38.6, 37.1, 35.8, 32.6, 32.4, 32.1, 28.0, 27.4, 26.8, 23.6, 19.9, 19.8, 17.6, 16.0, 15.6, 14.4, 13.2, 12.2, 12.0, 10.7, 6.7, 6.4 ppm.

Example FLC-00567-1

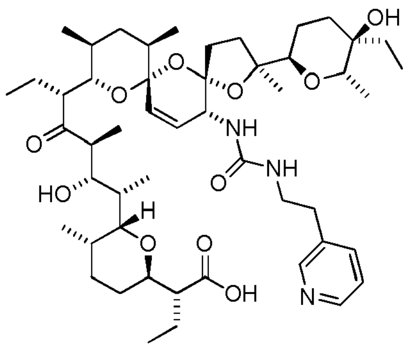

The compound was prepared according to the procedure described in example FLC-00539-1, except that 3-(2-aminoethyl)pyridine (1.5 eq) was added to the reaction mixture instead of 3-methoxypropylamine. The yield of the obtained product was 61%.

ESI-MS for $C_{50}H_{79}N_3O_{11}$ (*m/z*): $[M+H]^+$ 899.1, $[M-H]^-$ 896.9.

$^1H$ NMR (500 MHz, $CDCl_3$) δ 8,38 (dd, J = 4.6, 1.5 Hz, 2H), 7.49 (d, J = 7.8 Hz, 1H), 7.14 (dd, J = 7.7, 4.9 Hz, 1H), 6.10 (t, J = 5.6 Hz, 1H), 6.02 (dd, J = 10.9, 2.9 Hz, 1H), 5.76 - 5.69 (m, 2H), 5.39 (s, 1H), 4.73 (d, J = 4.5 Hz, 1H), 4.66 - 4.59 (m, 1H), 4.30 (q, J = 6.6 Hz, 1H), 4.10 (dd, J = 10.3, 3.5 Hz, 1H), 3.87 (dd, J = 11.0, 5.0 Hz, 1H), 3.63 - 3.59 (m, 1H), 3.52 (d, J = 10.0 Hz, 1H), 3.39 (dd, J = 12.3, 2.7 Hz, 1H), 3.32 - 3.25 (m, 2H), 2.84 - 2.58 (m, 6H), 2.08 - 0.63 (m, 53H) ppm.

$^{13}C$ NMR (126 MHz, $CDCl_3$) δ 218.4, 184.8, 158.0, 150.2, 147.6, 136.1, 135.1, 129.6, 123.3, 122.2, 106.5, 98.7, 88.3, 75.8, 75.6, 75.3, 73.8, 71.3, 71.0, 67.9, 55.9, 50.8, 49.8, 46.6, 41.3, 40.5, 38.6, 37.0, 35.8, 34.0, 32.6, 32.4, 32.2, 27.9, 27.9, 27.7, 26.8, 23.6, 19.8, 19.6, 17.5, 15.9, 15.6, 14.4, 13.1, 12.3, 12.0, 10.7, 6.6, 6.5 ppm.

Example FLC-00593-1

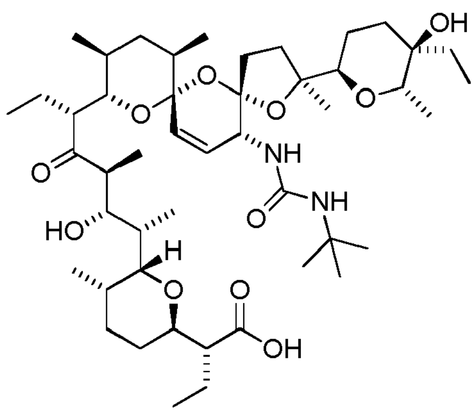

The compound was prepared according to the procedure described in example FLC-00539-1, except that tert-butylamine (2 eq) was added to the reaction mixture instead of 3-methoxypropylamine. The yield of the obtained product was 48%.

ESI-MS for $C_{47}H_{80}N_2O_{11}$ (*m*/*z*): $[M+H]^+$ 850.0, $[M+Na]^+$ 872.0, $[M-H]^-$ 848.0.

$^1$H NMR (500 MHz, $CD_2Cl_2$) δ 6.04 (dd, J = 10.9, 3.0 Hz, 1H), 5.67 (dd, J = 10.8, 1.5 Hz, 1H), 5.58 (s, 1H), 5.49 (s, 1H), 5.41 (d, J = 9.9 Hz, 1H), 4.91 (d, J = 3.5 Hz, 1H), 4.66 - 4.57 (m, 1H), 4.31 (d, J = 6.8 Hz, 1H), 4.06 (d, J = 10.1 Hz, 1H), 3.81 (dd, J = 11.0, 4.4 Hz, 1H), 3.62 (s, 1H), 3.59 (d, J = 10.1 Hz, 1H), 3.46 (dd, J = 12.3, 2.6 Hz, 1H), 2.81 (td, J = 11.1, 3.2 Hz, 1H), 2.70 (dd, J = 10.1, 7.7 Hz, 2H), 2.19 - 0.67 (m, 63H) ppm.

$^{13}$C NMR (126 MHz, $CD_2Cl_2$) δ 219.1, 184.4, 156.5, 130.0, 122.1, 106.7, 98.8, 88.2, 75.8, 75.4, 75.3, 73.9, 71.1, 70.9, 67.8, 56.1, 50.4, 49.8, 49.8, 46.0, 40.5, 38.6, 36.7, 35.8, 32.4, 29.1, 28.1, 28.0, 27.7, 26.7, 23.5, 19.9, 19.6, 17.2, 15.7, 15.5, 14.3, 12.9, 12.9, 11.9, 10.5, 6.4, 6.4 ppm.

Example FLC-00606-1

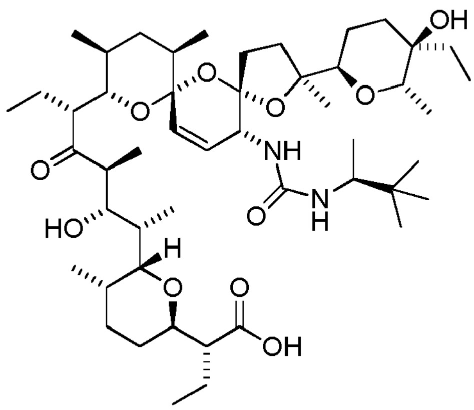

The compound was prepared according to the procedure described in example FLC-00539-1, except that S-3,3-dimethyl-2-butylamine (3 eq) was added to the reaction mixture instead of 3-methoxypropylamine. The yield of the obtained product was 38%.

ESI-MS for $C4_9H_{84}N_2O_{11}$ *(m/z):* $[M+H]^+$ 878.0, $[M+Na]^+$ 900.0, $[M-H]^-$ 876.1.

$^1$H NMR (500 MHz, $CD_2Cl_2$) δ 6.04 (dd, J = 10.8, 3.0 Hz, 1H), 5.68 (dd, J = 10.8, 1.7 Hz, 1H), 5.54 (d, J = 9.9 Hz, 1H), 5.09 (s, 1H), 5.00 (d, J = 9.8 Hz, 1H), 4.66 - 4.61 (m, 1H), 4.30 (q, J = 6.7 Hz, 1H), 4.07 (d, J = 10.4 Hz, 1H), 3.81 (dd, J = 10.9, 4.6 Hz, 1H), 3.67 (dd, J = 12.0, 4.6 Hz, 2H), 3.60 (d, J = 10.1 Hz, 1H), 3.47 (dd, J = 12.3, 2.8 Hz, 1H), 2.80 (td, J = 11.0, 3.3 Hz, 1H), 2.74 - 2.65 (m, 2H), 2.26. - 0.67 (m, 67H) ppm.

$^{13}$C NMR (126 MHz, $CD_2Cl_2$) δ 219.1, 184.1, 156.9, 130.3, 121.9, 106.5, 99.0, 88.3, 75.9, 75.5, 75.4, 73.9, 71.1, 70.8, 67.9, 56.0, 53.3, 50.2, 49.8, 47.0, 40.6, 38.6, 36.4, 35.8, 34.4, 32.5, 32.4, 32.1, 28.0, 27.8, 27., 26.7, 26.1, 23.4, 20.0, 19.7, 17.3, 16.3, 15.7, 15.6, 14.2, 13.3, 12.9, 11.9, 10.6, 6.5, 6.3 ppm.

Example FLC-00515-1

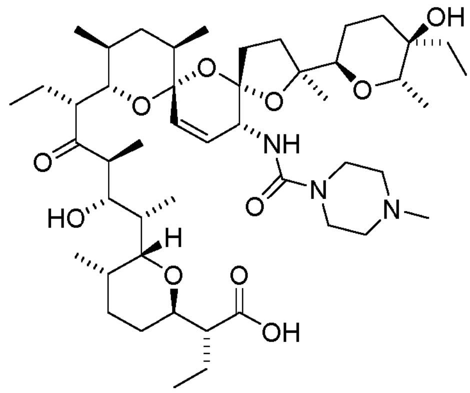

The compound was prepared according to the procedure described in example FLC-00539-1, except that N-methylpiperazine (2 eq) was added to the reaction mixture instead of N-methoxypropylamine. The yield of the obtained product was 73%.

ESI-MS for $C_{48}H_{81}N_3O_{11}$ (*m/z*): $[M+H]^+$ 877.0, $[M+Na]^+$ 899.0, $[M-H]^-$ 875.1.

$^1$H NMR (500 MHz, $CD_2Cl_2$) δ 6.08 (dd, J = 10.8, 3.1 Hz, 1H), 5.69 (dd, J = 10.8, 1.4 Hz, 1H), 5.33 - 5.31 (m, 1H), 4.73 (d, J = 9.4 Hz, 1H), 4.21 (q, J = 6.7 Hz, 1H), 4.13 (d, J = 10.4 Hz, 1H), 3.85 (dd, J = 11.0, 4.4 Hz, 1H), 3.58 (dd, J = 14.1, 5.9 Hz, 2H), 3.45 - 3.31 (m, 5H), 2.79 (td, J = 10.9, 3.5 Hz, 1H), 2.75 - 2.66 (m, 2H), 2.26 (t, J = 5.0 Hz, 4H), 2.20 (s, 3H), 2.11 - 0,67 (m, 56H) ppm.

$^{13}$C NMR (126 MHz, $CD_2Cl_2$) δ 218.3, 184.3, 157.2, 129.6, 122.8, 106.8, 99.4, 88.2, 76.4, 75.7, 75.6, 74.0, 71.1, 69.8, 68.0, 55.8, 55.1, 50.2, 49.2, 48.3, 45.9, 43.6, 40.8, 38.7, 35.8, 32.4, 32.3, 32.1, 29.7, 28.2, 28.1, 27.4, 26.7, 23.5, 19.9, 19.7, 17.3, 15.5, 15.5, 14.3, 12.9, 12.4, 12.0, 10.6, 6.4, 6.3 ppm.

Example FLC-00518-1

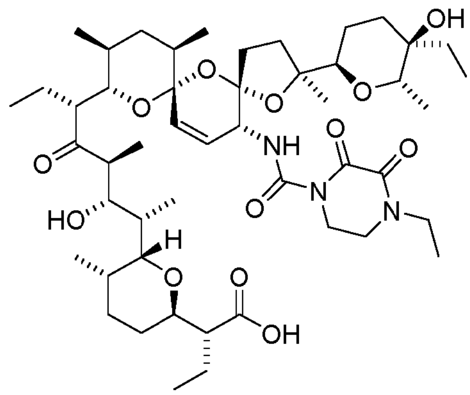

The compound was prepared according to the procedure described in example FLC-00445-1, except that 4-ethyl-2,3-dioxo-1-piperazinecarbonyl chloride (1 eq) was added to the reaction mixture instead of 5-chloropentanoyl chloride. The yield of the obtained product was 87%.

ESI-MS for $C_{49}H_{79}N_3O_{13}$ (*m/z*): $[M+Na]^+$ = 941. $[M-H]^-$ = 917.

1H NMR (500 MHz, $CD_2Cl_2$) δ 8,90 (d, J = 7.9 Hz, 1H), 6.14 (dd, J = 10.8, 3.0 Hz, 1H), 5.92 (dd, J = 10.8, 1.2 Hz, 1H), 4.37 - 4.31 (m, 1H), 4.31 - 4.24 (m, 1H), 4.23 - 4.15 (m, 1H), 4.11 (dd, J = 10.4, 1.0 Hz, 1H), 3.84 (dd, J = 10.8, 4.3 Hz, 1H), 3.81 - 3.74 (m, 1H), 3.71 (dd, J = 10.1, 1.4 Hz, 1H), 3.62 (d, J = 10.1 Hz, 1H), 3.59 - 3.35 (m, 5H), 2.76 - 2.65 (m, 3H), 2.20 - 2.06 (m, 2H), 2.00 - 0.70 (m, 53H), 0.68 (d, J = 6.9 Hz, 3H) ppm.

$^{13}$C NMR (126 MHz, $CD_2Cl_2$) δ 217.3, 182.1, 158.9, 155.7, 153.6, 128.5, 121.8, 106.2, 99.8, 88.7, 77.0, 76.4, 75.8, 73.3, 70.9, 69.9, 67.3, 54.9, 51.3, 50.5, 49.5, 43.7, 42.4, 41.1, 40.8, 38.6, 36.2, 35.7, 32.5, 32.3, 31.8, 29.7, 28.2, 27.9, 26.8, 22.8, 20.3, 19.7, 17.4, 16.0, 15.4, 14.0, 13.0, 12.2, 12.1, 11.9, 10.9, 6.7, 6.4 ppm.

Example FLC-00519-1

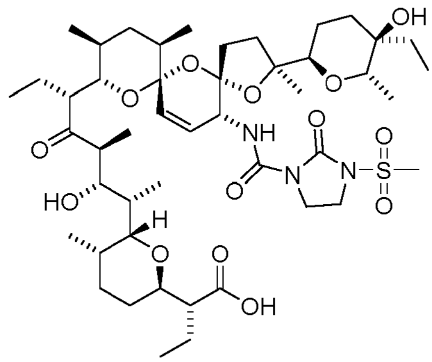

The compound was prepared according to the procedure described in example FLC-00445-1, except that 3-(methylsulphonyl)-2-oxoimidazolidine-1-carbonyl chloride (2eq) was added to the reaction mixture instead of 5-chloropentanoyl chloride. The yield of the obtained product was 82%.

ESI-MS for $C_{47}H_{78}N_3O_{14}S$ (*m/z*): $[M+Na]^+$ = 963, $[M-H]^-$ = 939.

$^1H$ NMR (500 MHz, $CD_2Cl_2$) δ 7,83 (d, *J* = 8.9 Hz, 1H), 6.16 (dd, *J* = 10.8, 3.1 Hz, 1H), 5.84 (dd, *J* = 10.7, 1.3 Hz, 1H), 4.56 - 4.44 (m, 1H), 4.17 (m, 1H), 4.08 (dd, *J* = 10.5, 1.4 Hz, 1H), 3.95 - 3.73 (m, 5H), 3.68 (dd, *J* = 13.3, 5.5 Hz, 2H), 3.45 (dd, *J* = 12.2, 2.0 Hz, 1H), 3.32 (s, 3H), 2.76 - 2.64 (m, 3H), 2.12 (t, *J* = 7.4 Hz, 2H), 2.05 - 0.71 (m,51H), 0.68 (d, *J* = 6.9 Hz, 3H) ppm.

$^{13}C$ NMR (126 MHz, $CD_2Cl_2$) δ 218.2, 182.6, 152.2, 128.7, 122.8, 106.1, 100.0, 88.6, 76.8, 76.2, 75.7, 73.2, 70.9, 69.8, 67.7, 55.5, 50.4, 50.0, 49.6, 40.9, 40.4, 40.3, 39.9, 38.7, 36.1, 35.6, 32.5, 32.4, 32.0, 29.7, 28.5, 28.2, 26.9, 26.0, 22.6, 20.3, 20.0, 17.4, 15.8, 15.4, 14.0, 12.9, 12.2, 12.0, 10.9, 6.7, 6.4 ppm.

Example FLC-00537-1

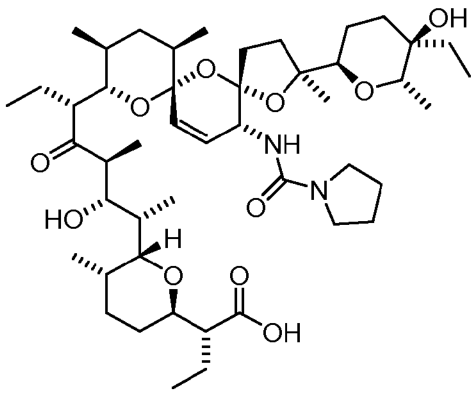

The compound was prepared according to the procedure described in example FLC-00539-1, except that pyrrolidine (2 eq) was added to the reaction mixture instead of 3-methoxypropylamine. The yield of the obtained product was 55%.

ESI-MS for $C_{47}H_{78}N_2O_{11}$ (*m/z*): $[M+H]^+$ 848.0, $[M+Na]^+$ 870.0, $[M-H]^-$ 846.0.

$^1H$ NMR (500 MHz, $CD_2Cl_2$) δ 6.09 (dd, J = 10.8, 3.1 Hz, 1H), 5.68 (dd, J = 10.8, 1.6 Hz, 1H), 4.99 (d, J= 9.7 Hz, 1H), 4.68 - 4.61 (m, 1H), 4.20 (q, J = 6.7 Hz, 1H), 4.14 (d, J = 10.4 Hz, 1H), 3.83 (dd, J = 11.0, 4.6 Hz, 1H), 3.62 - 3.54 (m, 2H), 3.47 (d, J = 5.9 Hz, 2H), 3.41 (dd, J = 12.1, 2.1 Hz, 1H), 3.27 - 3.20 (m, 2H), 2.78 (td, J = 11.1, 3.5 Hz, 1H), 2.74 - 2.65 (m, 2H), 2.18 - 0.66 (m, 60H) ppm.

$^{13}C$ NMR (126 MHz, $CD_2Cl_2$) δ 218.1, 184.2, 156.2, 129.7, 122.8, 106.9, 99.3, 88.2, 76.5, 75.7, 75.6, 74.2, 71.1, 69.7, 67.8, 55.7, 50.3, 49.2, 48.0, 45.6, 40.8, 38.8, 35.9, 35.8, 32.5, 32.4, 32.1, 28.6, 27.9, 27.5, 26.7, 25.5, 23.6, 19.9, 19.8, 17.3, 15.6, 15.5, 14.4, 12.9, 12.2, 12.1, 10.5, 6.4, 6.2 ppm.

Example FLC-00538-1

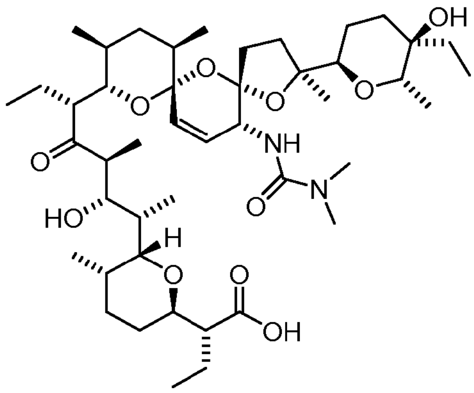

The compound was prepared according to the procedure described in example FLC-00539-1, except that N,N-dimethylamine (2 eq) was added to the reaction mixture instead of 3-methoxypropylamine. The yield of the obtained product was 46%.

ESI-MS for $C_{45}H_{76}N_2O_{11}$ (*m/z*): $[M+Na]^+$ 844.1, $[M-H]^-$ 819.9.

$^1$H NMR (500 MHz, $CD_2Cl_2$) δ 6.08 (dd, J = 10.8, 3.1 Hz, 1H), 5.67 (dd, J = 10.8, 1.6 Hz, 1H), 5.21 (d, J = 9.6 Hz, 1H), 4.67 - 4.60 (m, 1H), 4.22 (q, J = 6.7 Hz, 1H), 4.13 (d, J = 10.4 Hz, 1H), 3.82 (dd, J = 11.0, 4.6 Hz, 1H), 3.59 (dd, J = 15.2, 6.0 Hz, 2H), 3.41 (dd, J = 12.1, 2.1 Hz, 1H), 2.86 (s, 6H), 2.80 - 2.74 (m, 1H), 2.72 - 2.64 (m, 2H), 2.14 - 0.67 (m, 56H) ppm.

$^{13}$C NMR (126 MHz, $CD_2Cl_2$) δ 218.2, 184.2, 157.9, 129.7, 122.7, 106.8, 99.3, 88.2, 76.3, 75.8, 75.7, 74.2, 71.1, 69.8, 67.9, 55.7, 50.4, 49.1, 48.5, 40.7, 38.7, 36.2, 35.9, 35.8, 32.5, 32.3, 32.1, 29.7, 28.8, 27.9, 27.4, 26.7, 23.5, 19.9, 19.9, 17.3, 15.6, 15.5, 14.4, 12.9, 12.2, 12.1, 10.6, 6.4, 6.2 ppm.

Example FLC-00540-1

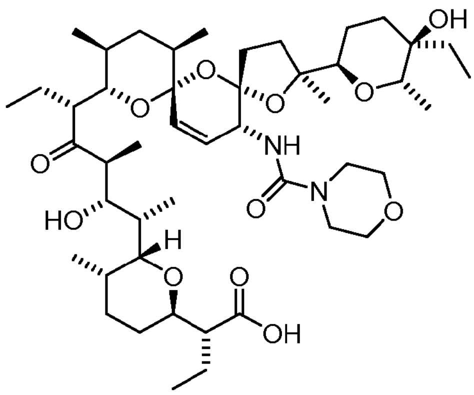

The compound was prepared according to the procedure described in example FLC-00539-1, except that morpholine (2 eq) was added to the reaction mixture instead of 3-methoxypropylamine. The yield of the obtained product was 48%.

ESI-MS for $C_{47}H_{78}N_2O_{12}$ (*m/z*): $[M+H]^+$ 864.0, $[M+Na]^+$ 886.1, $[M-H]^-$ 862.0.

$^1$H NMR (500 MHz, $CD_2Cl_2$) δ 6.09 (dd, J = 10.8, 3.2 Hz, 1H), 5.68 (dd, J = 10.8, 1.6 Hz, 1H), 5.36 (d, J = 9.5 Hz, 1H), 4.80 - 4.70 (m, 1H), 4.21 (q, J = 6.7 Hz, 1H), 4.14 (d, J = 10.5 Hz, 1H), 3.85 (dd, J = 11.1, 4.5 Hz, 1H), 3.62 - 3.52 (m, 6H), 3.45 - 3.28 (m, 5H), 2.87 - 2.75 (m, 1H), 2.69 (q, J = 7.1 Hz, 2H), 2.05 - 0.66 (m, 56H) ppm.

$^{13}$C NMR (126 MHz, $CD_2Cl_2$) δ 218.2, 184.4, 157.3, 129.2, 123.0, 106.7, 99.3, 88.3, 76.4, 75.8, 75.4, 74.2, 71.1, 69.9, 66.9, 55.7, 50.1, 49.1, 48.1, 43.9, 40.7, 38.7, 35.8, 35.7, 32.4, 32.3, 32.2, 29.7, 28.4, 28.0, 27.6, 26.6, 23.5, 19.9, 19.7, 17.2, 15.5, 15.5, 14.3, 12.9, 12.2, 12.0, 10.5, 6.4, 6.3 ppm.

Example FLC-00542-1

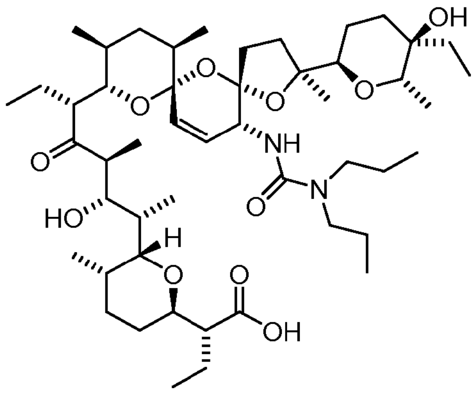

The compound was prepared according to the procedure described in example FLC-00539-1, except that N,N-dipropylamine (2 eq) was added to the reaction mixture instead of 3-methoxypropylamine. The yield of the obtained product was 51%.

ESI-MS for $C_{49}H_{84}N_2O_{11}$ (*m/z*): $[M+H]^+$ 878.2, $[M+Na]^+$ 900.0, $[M-H]^-$ 876.0.

$^1$H NMR (500 MHz, $CD_2Cl_2$) δ 6.07 (dd, J = 10.8, 3.1 Hz, 1H), 5.72 (dd, J = 10.7, 1.6 Hz, 1H), 5.25 (d, J = 9.1 Hz, 1H), 4.68 - 4.60 (m, 1H), 4.25 (q, J = 6.7 Hz, 1H), 4.14 (dd, J = 10.5, 0.9 Hz, 1H), 3.83 (dd, J = 11.0, 4.5 Hz, 1H), 3.65 (d, J = 1.8 Hz, 1H), 3.56 (d, J = 10.1 Hz, 1H), 3.43 (dd, J = 12.1, 2.3 Hz, 1H), 3.29 (dt, J = 14.4, 7.2 Hz, 2H), 3.04 (dt, J = 14.3, 7.1 Hz, 2H), 2.77 (td, J = 10.4, 3.6 Hz, 1H), 2.68 (ddd, J = 14.9, 9.3, 5.4 Hz, 2H), 2.23 - 0.67 (m, 66H) ppm. $^{13}$C NMR (126 MHz, $CD_2Cl_2$) δ 218.1, 183.8, 157.4, 130.4, 122.5, 107.1, 99.6, 88.1, 76.2, 75.8, 75.7, 73.8, 71.1, 69.9, 67.7, 55.6, 50.4, 49.4, 49.1, 47.9, 40.9, 38.7, 35.8, 35.5, 32.5, 32.4, 32.1, 29.7, 28.4, 28.0, 27.3, 26.7, 23.6, 21.4, 20.1, 19.8, 17.3, 15.7, 15.5, 14.3, 12.9, 12.5, 12.0, 11.1, 10.6, 6.4, 6.2 ppm.

Example FLC-00543-1

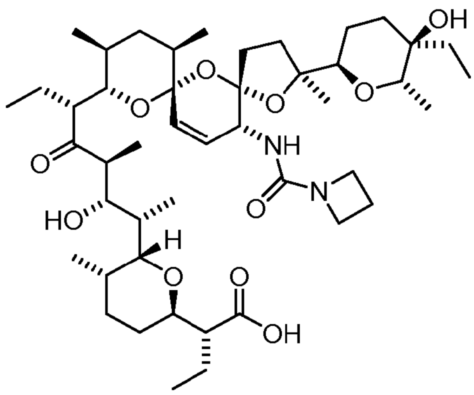

The compound was prepared according to the procedure described in example FLC-00539-1, except that azetidine (2 eq) was added to the reaction mixture instead of 3-methoxypropylamine. The yield of the obtained product was 48%.

ESI-MS for $C_{46}H_{76}N_2O_{11}$ (*m/z*): $[M+Na]^+$ 856.1, $[M-H]^-$ 832.1.

$^1$H NMR (500 MHz, $CD_2Cl_2$) δ 6.08 (dd, J = 10.8, 3.1 Hz, 1H), 5.66 (dd, J = 10.8, 1.5 Hz, 1H), 4.96 (d, J = 10.0 Hz, 1H), 4.57 - 4.49 (m, 1H), 4.20 (q, J = 6.7 Hz, 1H), 4.11 (dd, J = 10.5, 1.0 Hz, 1H), 3.99 (q, J = 7.7 Hz, 2H), 3.88 (q, J = 7.7 Hz, 2H), 3.83 (dd, J = 11.0, 4.4 Hz, 1H), 3.62 (d, J = 2.0 Hz, 1H), 3.58 (d, J = 10.1 Hz, 1H), 3.44 (dd, J = 12.1, 2.2 Hz, 1H), 2.77 (td, J = 10.6, 3.5 Hz, 1H), 2.73 - 2.62 (m, 2H), 2.14 - 0.67 (m, 58H) ppm.

$^{13}$C NMR (126 MHz, $CD_2Cl_2$) δ 218.4, 184.0, 158.9, 129.6, 122.7, 106.6, 99.4, 88.3, 76.4, 75.9, 75.8, 73.9, 71.0, 69.9, 68.0, 55.8, 50.4, 49.2, 49.1, 48.2, 40.7, 38.7, 36.1, 35.7, 32.4, 32.4, 32.0, 29.7, 28.5, 28.0, 27.1, 26.7, 23.4, 20.0, 19.9, 17.3, 15.6, 15.5, 15.3, 14.3, 12.9, 12.2, 12.1, 10.6, 6.4, 6.2 ppm.

Example FLC-00544-1

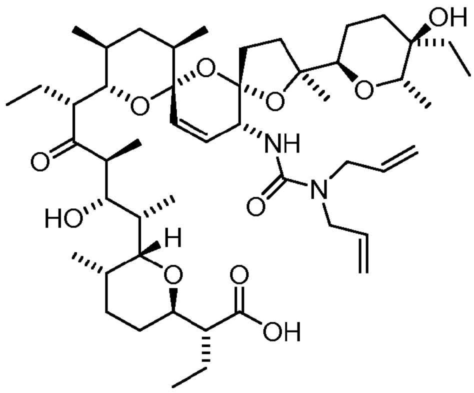

The compound was prepared according to the procedure described in example FLC-00539-1, except that N,N-diallylamine (2 eq) was added to the reaction mixture instead of 3-methoxypropylamine. The yield of the obtained product was 57%.

ESI-MS for $C_{49}H_{80}N_2O_{11}$ (*m/z*): $[M+H]^+$ 874.0, $[M+Na]^+$ 896.1, $[M-H]^-$ 872.0,

$^1$H NMR (500 MHz, $CD_2Cl_2$) δ 6,08 (dd, J = 10.8, 3.1 Hz, 1H), 5.78 - 5.65 (m, 3H), 5.26 (d, J = 9.3 Hz, 1H), 5.12 - 5.08 (m, 2H), 5.07 (s, 2H), 4.68 - 4.59 (m, 1H), 4.23 (q, J = 6.7 Hz, 1H), 4.15 - 4.11 (m, 1H), 3.90 (dd, J = 16.7, 5.4 Hz, 2H), 3.85 - 3.77 (m, 3H), 3.61 (d, J = 2.0 Hz, 1H), 3.57 (d, J = 10.1 Hz, 1H), 3.42 (dd, J = 12.1, 2.3 Hz, 1H), 2.77 (td, J = 10.8, 3.7 Hz, 1H), 2.72 - 2.65 (m, 2H), 2.20 - 0.67 (m, 56H) ppm,

$^{13}$C NMR (126 MHz, $CD_2Cl_2$) δ 218.2, 184.1, 157.3, 134.2, 129.8, 122.6, 115.7, 106.8, 99.5, 88.3, 76.2, 75.7, 73.9, 71.1, 69.9, 67.8, 55.7, 50.2, 49.3, 49.1, 48.1, 40.8, 38.7, 35.8, 35.8, 32.5, 32.3, 32.1, 29.7, 28.1, 28.0, 27.4, 26.7, 23.6, 20.0, 19.8, 17.3, 15.6, 15.5, 14.3, 12.9, 12.5, 12.0, 10.6, 6.4, 6.2 ppm.

Example FLC-00557-1

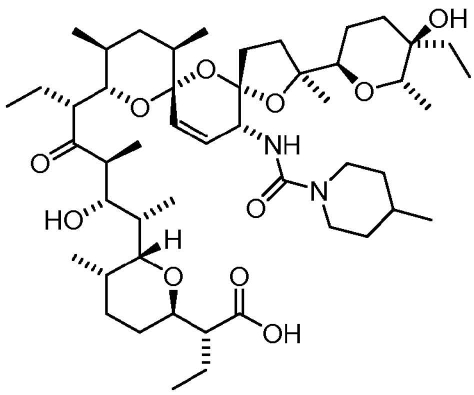

The compound was prepared according to the procedure described in example FLC-00539-1, except that 4-methylpiperidine (2 eq) was added to the reaction mixture instead of 3-methoxypropylamine. The yield of the obtained product was 60%.

ESI-MS for $C_{49}H_{82}N_2O_{11}$ (*m/z*): $[M+H]^+$ 875.9, $[M+Na]^+$ 898.1, $[M-H]^-$ 874.0.

$^1$H NMR (500 MHz, $CD_2Cl_2$) δ 6.07 (dd, J = 10.8, 2.9 Hz, 1H), 5.68 (d, J = 10.7 Hz, 1H), 5.23 (d, J= 9.5 Hz, 1H), 4.71 (d, J = 9.3 Hz, 1H), 4.21 (q, J = 6.6 Hz, 1H), 4.12 (d, J = 10.4 Hz, 1H), 4.05 (d, J = 13.0 Hz, 1H), 3.95 (d, J = 12.8 Hz, 1H), 3.84 (dd, J = 10.8, 3.7 Hz, 1H), 3.60 (dd, J = 12.5, 5.6 Hz, 2H), 3.46 - 3,40 (m, 1H), 2.82 - 2,56 (m, 6H), 2.11 - 0.67 (m, 63H) ppm.

$^{13}$C NMR (126 MHz, $CD_2Cl_2$) δ 218.3, 184.2, 157.2, 129.9, 122.5, 106.8, 99.4, 88.2, 76.4, 75.7, 73.9, 71.0, 69.7, 67.9, 55.8, 50.3, 49.3, 48.5, 44.4, 44.1, 40.8, 38.8, 35.8, 34.3, 34.0, 32.5, 32.3, 32.1, 30.9, 29.7, 28.2, 28.0, 27.4, 26.7, 23.5, 21.7, 20.0, 19.7, 17.3, 15.5, 14.3, 12.9, 12.4, 12.0, 10.6, 6.4, 6.3 ppm.

Example FLC-00560-1

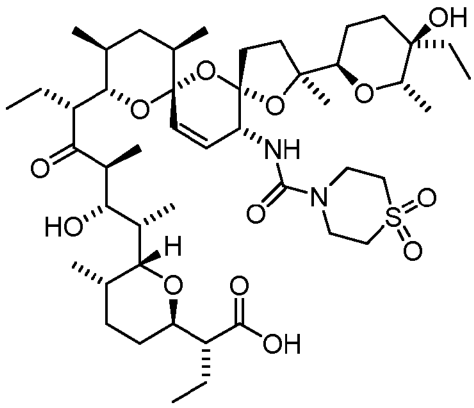

The compound was prepared according to the procedure described in example FLC-00539-1, except that thiomorpholine 1,1-dioxide (2 eq) was added to the reaction mixture instead of 3-methoxypropylamine. The yield of the obtained product was 62%.

ESI-MS for $C_{47}H_{78}N_2O_{13}S$ (*m/z*): $[M+Na]^+$ 934.0, $[M-H]^-$ 910.0.

$^1H$ NMR (500 MHz, $CD_2Cl_2$) δ 6.09 (dd, J = 10.8, 3.1 Hz, 1H), 5.70 (d, J = 9.6 Hz, 1H), 5.64 (dd, J = 10.8, 1.6 Hz, 1H), 4.73 - 4.65 (m, 1H), 4.26 - 4.22 (m, 1H), 4.14 (d, J = 10.2 Hz, 1H), 3.97 - 3.91 (m, 2H), 3.88 - 3.79 (m, 3H), 3.57 (dd, J = 10.7, 2.1 Hz, 2H), 3.43 (dd, J = 12.0, 2.1 Hz, 1H), 3.00 - 2.94 (m, 2H), 2.93 - 2.86 (m, 2H), 2.84 - 2.76 (m, 1H), 2.74 - 2.65 (m, 2H), 2.03 - 0.66 (m, 56H) ppm.

$^{13}C$ NMR (126 MHz, $CD_2Cl_2$) δ 218.3, 184.7, 156.7, 128.7, 123.0, 106.2, 99.3, 88.4, 76.2, 76.1, 75.3, 74.3, 71.1, 70.1, 68.2, 55.7, 52.0, 50.0, 48.8, 48.7, 43.2, 40.7, 38.6, 36.1, 35.7, 32.4, 32.2, 32.1, 29.7, 28.9, 27.9, 27.7, 26.6, 23.6, 19.9, 17.2, 15.6, 15.4, 14.3, 12.8, 12.2, 12.1, 10.6, 6.4, 6.2 ppm.

Example FLC-00561-1

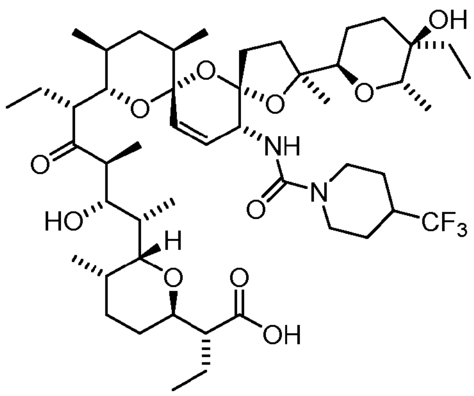

The compound was prepared according to the procedure described in example FLC-00539-1, except that 4-(trifluoromethyl)piperidine*HCl (1.5 eq) was added to the reaction mixture instead of 3-methoxypropylamine. The yield of the obtained product was 59%.

ESI-MS for $C_{49}H_{79}F_3N_2O_{11}$ (*m/z*): $[M+H]^+$ 929.9, $[M+Na]^+$ 952.1, $[M-H]^-$ 928.1.

$^1H$ NMR (500 MHz, $CD_2Cl_2$) δ 6.09 (dd, J = 10.8, 3.1 Hz, 1H), 5.68 (dd, J = 10.8, 1.6 Hz, 1H), 5.37 (d, J = 9.5 Hz, 1H), 4.75 - 4.70 (m, 1H), 4.65 (s, 1H), 4.24 (dt, J = 13.6, 10.2 Hz, 2H), 4.13 (t, J = 9.3 Hz, 2H), 3.85 (dd, J = 11.0, 4.4 Hz, 1H), 3.62 - 3.54 (m, 2H), 3.43 (dd, J = 12.1, 2.2 Hz, 1H), 2.84 - 2.76 (m, 1H), 2.72 - 2.57 (m, 4H), 2.14 - 0.67 (m, 60H) ppm.

$^{13}C$ NMR (126 MHz, $CD_2Cl_2$) δ 218.3, 184.5, 157.0, 129.5, 122.7, 106.6, 99.4, 88.3, 76.4, 75.8, 75.5, 74.1, 71.0, 69.8, 68.0, 55.8, 50.2, 49.2, 48.4, 43.1, 42.7, 40.7, 40.5, 40.3, 38.7, 35.8, 35.7, 32.4, 32.3, 32.1, 28.3, 28.0, 27.5, 26.7, 24.8, 24.4, 23.5, 19.9, 19.7, 17.2, 15.5, 15.5, 14.3, 12.9, 12.3, 12.0, 10.5, 6.4, 6.2 ppm.

Example FLC-00563-1

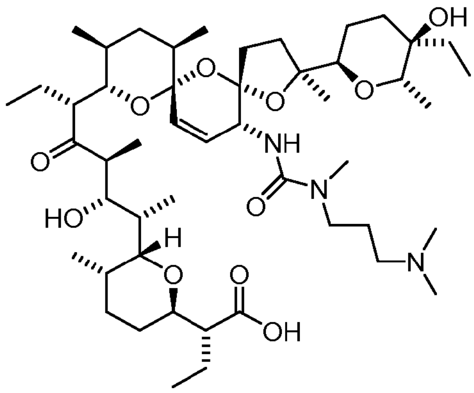

The compound was prepared according to the procedure described in example FLC-00539-1, except that N,N,N'-trimethyl-1,3-propanodiamine (2 eq) was added to the reaction mixture instead of 3-methoxypropylamine. The yield of the obtained product was 56%.

ESI-MS for $C_{49}H_{85}N_3O_{11}$ (*m/z*): $[M+H]^+$ 892.9, $[M+Na]^+$ 915.1, $[M-H]^-$ 891.1.

$^1H$ NMR (500 MHz, $CDCl_3$) δ 6.09 (dd, J = 10.9, 3.1 Hz, 1H), 5.80 (dd, J = 10.8, 1.6 Hz, 1H), 5.28 (d, J = 9.6 Hz, 1H), 4.78 - 4.70 (m, 1H), 4.49 (s, 1H), 4.36 - 4.29 (m, 1H), 4.25 (d, J = 10.3 Hz, 1H), 3.91 (dd, J = 11.0, 4.6 Hz, 1H), 3.65 (dd, J = 10.2, 1.7 Hz, 1H), 3.48 (dd, J = 20.2, 6.7 Hz, 2H), 3.40 - 3.33 (m, 2H), 3.27 (dt, J = 14.0, 7.1 Hz, 1H), 2.96 (s, 3H), 2.85 (td, J = 10.8, 3.2 Hz, 1H), 2.65 (dd, J = 10.3, 7.3 Hz, 2H), 2.29 - 2.18 (m, 8H), 1.96 - 0.67 (m, 56H) ppm.

$^{13}C$ NMR (126 MHz, $CDCl_3$) δ 217.0, 184.8, 157.7, 129.6, 122.8, 106.9, 99.3, 88.2, 76.1, 75.7, 75.4, 74.6, 4, 69.9, 57.0, 55.5, 50.8, 49.3, 48.2, 47.0, 45.5, 40.8, 38.8, 35.9, 34.4, 32.5, 32.4, 32.3, 29.7, 29.7, 29.5, 28.1, 27.9, 26.7, 26.3, 23.9, 20.0, 19.9, 17.5, 15.7, 15.6, 14.7, 13.2, 12.2, 12.1, 10.7, 6.6, 6.5 ppm.

Example FLC-00564-1

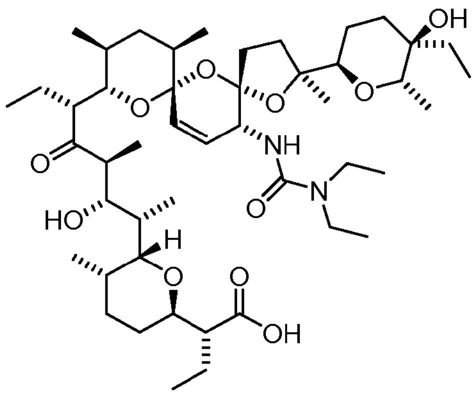

The compound was prepared according to the procedure described in example FLC-00539-1, except that N,N-diethylamine (2 eq) was added to the reaction mixture instead of 3-methoxypropylamine. The yield of the obtained product was 62%.

ESI-MS for $C_{47}H_{80}N_2O_{11}$ (*m/z*): $[M+H]^+$ 850.2, $[M+Na]^+$ 872.1, $[M-H]^-$ 848.0.

$^1H$ NMR (500 MHz, $CD_2Cl_2$) δ 6.08 (dd, J = 10.8, 3.1 Hz, 1H), 5.71 (dd, J = 10.7, 1.6 Hz, 1H), 5.04 (d, J= 9.6 Hz, 1H), 4.93 (s, 1H), 4.65 - 4.59 (m, 1H), 4.24 (q, J = 6.7 Hz, 1H), 4.13 (d, J = 10.4 Hz, 1H), 3.83 (dd, J = 11.0, 4.5 Hz, 1H), 3.59 (dd, J = 18.9, 6.0 Hz, 2H), 3.43 (dd, J = 12.1, 2.3 Hz, 1H), 3.32 - 3.17 (m, 4H), 2.77 (td, J = 10.6, 3.6 Hz, 1H), 2.71 - 2.66 (m, 2H), 2.23 - 2.13 (m, 1H), 2.01 - 0.67 (m, 60H) ppm.

$^{13}C$ NMR (126 MHz, $CD_2Cl_2$) δ 218.2, 184.0, 156.9, 130.1, 122.5, 106.9, 99.5, 88.3, 76.2, 75.7, 75.7, 74.0, 71.0, 69.7, 67.8, 55.7, 50.3, 49.3, 48.8, 40.8, 40.4, 38.8, 35.8, 32.5, 32.4, 32.1, 29.7, 28.5, 28.0, 27.4, 26.7, 23.6, 20.0, 19.7, 17.3, 15.6, 15.5, 14.3, 13.4, 12.9, 12.4, 12.0, 10.6, 6.4, 6.2 ppm.

Example FLC-00565-1

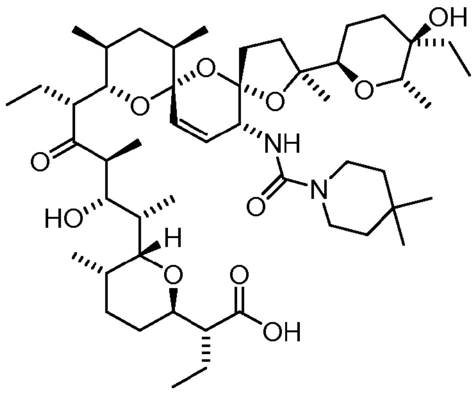

The compound was prepared according to the procedure described in example FLC-00539-1, except that 4,4-dimethylpiperidine*HCl (1.5 eq) was added to the reaction mixture instead of 3-methoxypropylamine. The yield of the obtained product was 49%.

ESI-MS for $C_{50}H_{84}N_2O_{11}$ (*m/z*): $[M+H]^+$ 889.9, $[M+Na]^+$ 911.9, $[M-H]^-$ 887.9.

$^1H$ NMR (500 MHz, $CD_2Cl_2$) δ 6.08 (dd, J = 10.8, 3.1 Hz, 1H), 5.68 (dd, J = 10.8, 1.6 Hz, 1H), 5.23 (d, J = 9.6 Hz, 1H), 4.80 (s, 1H), 4.75 - 4.70 (m, 1H), 4.21 (q, J = 6.8 Hz, 1H), 4.13 (d, J = 10.5 Hz, 1H), 3.84 (dd, J = 11.0, 4.5 Hz, 1H), 3.59 (dd, J = 13.4, 6.0 Hz, 2H), 3.43 (dd, J = 12.1, 2.3 Hz, 1H), 3.37 - 3.26 (m, 4H), 2.78 (td, J = 10.8, 3.5 Hz, 1H), 2.73 - 2.65 (m, 2H), 1.98 - 0.67 (m, 65H) ppm.

$^{13}C$ NMR (126 MHz, $CD_2Cl_2$) δ 218.2, 184.2, 157.3, 129.8, 122.6, 106.8, 99.4, 88.2, 76.4, 75.7, 74.0, 71.0, 69.7, 67.9, 55.8, 50.4, 49.3, 48.4, 40.8, 40.4, 38.8, 38.4, 35.8, 32.5, 32.3, 32.2, 29.7, 28.5, 28.2, 28.0, 27.4, 26.7, 23.5, 20.0, 19.7, 17.3, 15.5, 15.5, 14.3, 12.9, 12.4, 12.0, 10.6, 6.4, 6.3 ppm.

Example FLC-00347-1

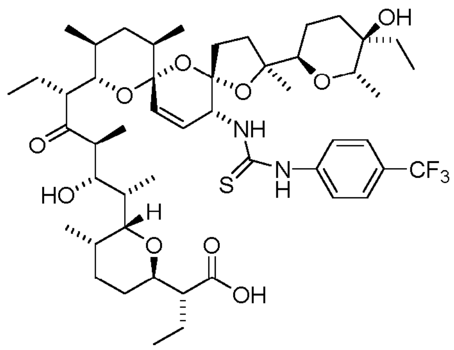

100 mg of C20-amino SAL (1 eq) was mixed with 4-trifluoromethylphenyl isothiocyanate (1.2 eq) in THF. The reaction was carried out at RT (TLC and LC-MS control). The post-reaction mixture was suspended on silica gel and purified using a chromatograph with an ELS detector using a column packed with silica gel. The respective fractions were combined and concentrated, and the residue was dissolved in DCM and washed twice with 0.06 M $H_2SO_4$.

The yield of the obtained product was 30%.

ESI-MS for $C_{50}H_{75}F_3N_2O_{10}S$ (*m/z*): $[M+H]^+$ 954.0, $[M+Na]^+$ 976.0, $[M-H]^-$ 952.0.

$^1H$ NMR (500 MHz, $CDCl_3$) δ 9.31 (s, 1H), 7.72 (d, J = 8.4 Hz, 2H), 7.54 (d, J = 8.5 Hz, 2H), 7.43 (d, J = 9.4 Hz, 1H), 6.21 (dd, J = 10.4, 1.2 Hz, 1H), 6.04 (dd, J = 10.3, 3.8 Hz, 1H), 5.45 (d, J = 6.5 Hz, 1H), 4.19 (d, J = 9.2 Hz, 1H), 3.95 - 3.90 (m, 1H), 3.82 (q, J = 6.7 Hz, 1H), 3.74 (d, J = 10.1 Hz, 1H), 3.63 (ddd, J = 14.0, 10.9, 2.1 Hz, 2H), 2.86 (td, J = 10.7, 3.8 Hz, 1H), 2.74 (dq, J = 14.5, 7.2 Hz, 1H), 2.61 (d, J = 10.2 Hz, 1H), 2.05 - 0.72 (m, 56H) ppm.

$^{13}C$ NMR (126 MHz, $CDCl_3$) δ 216.1, 180.8, 178.1, 142.9, 129.1, 125.6, 125.6, 124.9, 123.2, 106.5, 99.1, 87.0, 76.6, 75.9, 74.8, 73.6, 71.9, 71.7, 68.5, 55.3, 52.8, 49.3, 48.2, 40.1, 38.3, 37.7, 36.0, 33.1, 32.5, 31.1, 29.7, 28.7, 28.3, 26.0, 25.1, 22.3, 20.7, 20.3, 17.7, 15.9, 15.9, 14.6, 13.3, 13.1, 11.9, 11.6, 7.3, 6.5 ppm.

Example FLC-00339-1

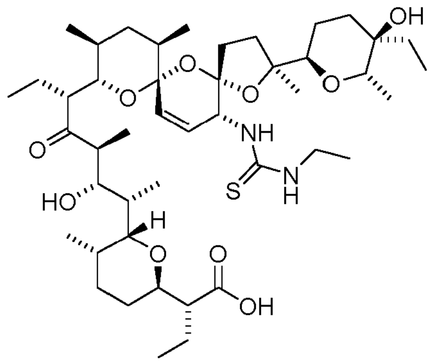

The compound was prepared according to the procedure described in example FLC-00347-1, except that ethyl isothiocyanate (3 eq) and, additionally, TEA (3 eq) was added to the reaction mixture instead of 4-trifluoromethylphenyl isothiocyanate. Following purification, the compound was washed with 0.25 M $Na_2CO_3$ instead of 0.06 M $H_2SO_4$. The yield of the obtained product was 36%.
ESI-MS for $C_{45}H_{76}N_2O_{10}S$ (*m/z*): $[M+H]^+$ 837.8, $[M+Na]^+$ 859.8, $[M-H]^-$ 835.8.
$^1H$ NMR (500 MHz, $CD_2Cl_2$) δ 7.46 (s, 1H), 6.69 (d, J = 9.8 Hz, 1H), 6.08 (dd, J = 10.8, 3.0 Hz, 1H), 5.69 (dd, J = 10.8, 1.6 Hz, 1H), 5.58 - 5.48 (m, 1H), 4.86 - 4.69 (m, 1H), 4.31 (q, J = 6.8 Hz, 1H), 4.07 (d, J = 10.3 Hz, 1H), 3.82 (dd, J = 11.0, 4.5 Hz, 1H), 3.61 (ddd, J = 10.9, 8.1, 2.7 Hz, 2H), 3.52 (dd, J = 7.2, 5.6 Hz, 1H), 3.49 - 3.38 (m, 2H), 2.82 (dd, J = 11.0, 7.8 Hz, 1H), 2.77 - 2.67 (m, 2H), 2.20 (t, J = 10.8 Hz, 1H), 2.00 - 0.67 (m, 57H) ppm.
$^{13}C$ NMR (126 MHz, $CD_2Cl_2$) δ 219.4, 184.7, 182.5, 128.6, 122.5, 106.5, 98.9, 88.4, 75.8, 75.5, 73.5, 71.2, 71.0, 68.1, 56.1, 50.8, 50.3, 49.6, 40.6, 39.6, 38.5, 36.8, 35.7, 32.4, 32.2, 29.7, 28.0, 27.6, 27.5, 26.6, 23.4, 19.9, 19.5, 17.3, 15.8, 15.5, 14.2, 14.2, 13.9, 12.8, 12.4, 12.0, 10.5, 6.4, 6.3 ppm.

Example FLC-00340-1

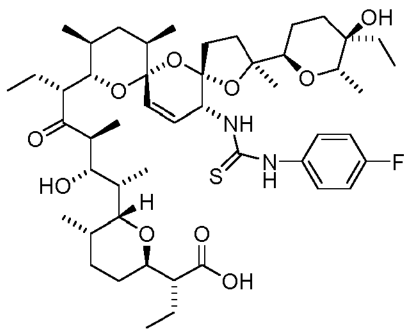

The compound was prepared according to the procedure described in example FLC-00347-1, except that 4-fluorophenyl isothiocyanate (1.2 eq) and, additionally, TEA (1 eq) was added to the reaction mixture instead of 4-trifluoromethylphenyl isothiocyanate. Following purification, the compound was washed with 0.25 M $Na_2CO_3$ instead of 0.06 M $H_2SO_4$. The yield of the obtained product was 36%.
ESI-MS for $C_{49}H_{75}FN_2O_{10}S$ (*m/z*): $[M+H]^+$ 903.8, $[M+Na]^+$ 925.7, $[M-H]^-$ 901.8.
$^1H$ NMR (500 MHz, $CD_2Cl_2$) δ 9.32 (s, 1H), 7.49 (dt, J = 7.0, 4.2 Hz, 2H), 7.40 (d, J = 9.6 Hz, 1H), 6.99 - 6.90 (m, 2H), 6.15 (dd, J = 10.8, 2.9 Hz, 1H), 5.76 (dd, J = 10.8, 1.7 Hz, 1H), 5.67 - 5.58 (m, 1H), 4.65 (s, 1H), 4.34 (q, J = 6.8 Hz, 1H), 4.09 (d, J = 10.3 Hz, 1H), 3.82 (dd, J = 10.9, 4.9 Hz, 1H), 3.67 - 3.59 (m, 2H), 3.51 (dd, J = 12.3, 2.8 Hz, 1H), 2.85 - 2.77 (m, 1H), 2.77 - 2.68 (m, 2H), 2.33 - 2.22 (m, 1H), 2.04 - 0.67 (m, 54H) ppm.
$^{13}C$ NMR (126 MHz, $CD_2Cl_2$) δ 219.5, 185.1, 181.9, 160.5, 158.6, 136.3, 127.9, 125.9, 125.8, 123.0, 114.6, 114.4, 106.4, 98.9, 88.5, 75.8, 75.7, 75.6, 73.7, 71.6, 71.0, 68.2, 56.2, 50.9, 50.2, 49.4, 40.5, 38.5, 37.1, 35.7, 32.4, 32.2, 28.0, 27.8, 27.6, 26.6, 23.3, 19.8, 19.7, 17.3, 15.8, 15.4, 14.2, 12.8, 12.1, 12.0, 10.6, 6.4, 6.3 ppm.

Example FLC-00345-1

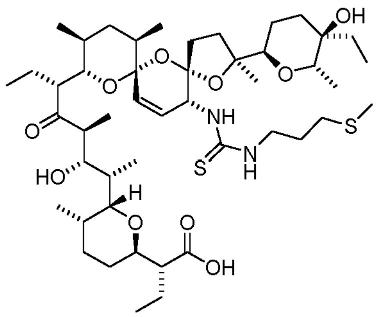

The compound was prepared according to the procedure described in example FLC-00347-1, except that 3-(methylthio)propyl isothiocyanate (3 eq) and, additionally, TEA (3 eq) was added to the reaction mixture instead of 4-trifluoromethylphenyl isothiocyanate. The yield of the obtained product was 34%.

ESI-MS for $C_{47}H_{80}N_2O_{10}S_2$ (*m/z*): $[M+H]^+$ 897.8, $[M+Na]^+$ 918.8, $[M-H]^-$ 895.8.

$^1$H NMR (500 MHz, $CD_2Cl_2$) δ 7.31 (s, 1H), 6.82 (s, 1H), 6.19 - 6.10 (m, 1H), 6.01 (dd, J = 10.2, 4.1 Hz, 1H), 5.25 (s, 1H), 4.06 (d, J = 10.1 Hz, 1H), 3.93 (dd, J = 10.8, 5.4 Hz, 1H), 3.82 (d, J = 6.6 Hz, 1H), 3.75 - 3.66 (m, 2H), 3.63 - 3.55 (m, 3H), 2.91 (td, J = 10.9, 4.1 Hz, 1H), 2.72 (dd, J = 10.0, 7.2 Hz, 1H), 2.63 (d, J = 9.8 Hz, 1H), 2.54 (td, J = 7.3, 2.3 Hz, 2H), 2.07 (s, 3H), 1.96 - 0.69 (m, 58H) ppm.

$^{13}$C NMR (126 MHz, $CD_2Cl_2$) δ 216.5, 182.3, 177.6, 130.2, 125.0, 107.2, 99.4, 86.9, 77.0, 75.7, 74.9, 73.4, 71.4, 71.3, 68.3, 55.4, 49.5, 47.9, 43.8, 40.1, 38.1, 37.4, 36.1, 32.8, 32.6, 31.4, 30.9, 29.7, 28.9, 28.6, 28.2, 25.9, 24.4, 22.3, 20.8, 20.0, 17.4, 16.0, 15.5, 15.0, 14.4, 12.9, 12.8, 11.8, 11.2, 6.9, 6.2 ppm.

Example FLC-00346-1

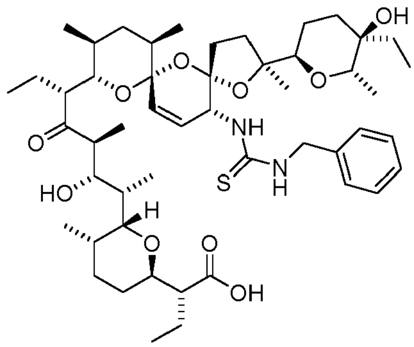

The compound was prepared according to the procedure described in example FLC-00347-1, except that benzyl isothiocyanate (3 eq) and, additionally, TEA (2 eq) was added to the reaction mixture instead of 4-trifluoromethylphenyl isothiocyanate. The yield of the obtained product was 29%.

ESI-MS for $C_{50}H_{78}N_2O_{10}S$ (*m/z*): $[M+H]^+$ 899.8, $[M+Na]^+$ 921.8, $[M-H]^-$ 897.8.

$^1$H NMR (500 MHz, $CD_2Cl_2$) δ 7.58 (s, 1H), 7.34 (d, J = 7.4 Hz, 2H), 7.30 (t, J = 7.5 Hz, 2H), 7.23 (t, J = 7.2 Hz, 1H), 6.96 (d, J = 8.0 Hz, 1H), 6.17 - 6.13 (m, 1H), 6.05 (dd, J = 10.2, 4.0 Hz, 1H), 5.33 (s, 1H), 4.88 (dd, J= 14.7, 5.6 Hz, 1H), 4.69 (dd, J = 14.7, 4.5 Hz, 1H), 4.06 (d, J = 10.1 Hz, 1H), 3.95 (dd, J = 10.8, 5.6 Hz, 1H), 3.78 - 3.73 (m, 1H), 3.72 - 3.68 (m, 1H), 3.61 (dd, J = 14.4, 6.5 Hz, 2H), 2.90 (td, J = 10.8, 4.1 Hz, 1H), 2.69 (dq, J = 14.4, 7.2 Hz, 1H), 2.60 (d, J = 10.0 Hz, 1H), 2.03 - 0.69 (m, 56H) ppm.

$^{13}$C NMR (126 MHz, $CD_2Cl_2$) δ 216.2, 182.4, 177.6, 138.5, 130.1, 128.4, 127.9, 127.1, 124.9, 107.1, 99.3, 87.0, 76.8, 75.8, 74.8, 73.4, 71.5, 71.3, 68.3, 55.3, 49.5, 48.8, 47.9, 40.1, 38.2, 37.5, 36.1, 32.8, 32.5, 30.8, 29.7, 28.6, 28.2, 26.0, 24.5, 22.3, 20.7, 20.0, 17.3, 16.0, 15.5, 14.4, 12.9, 12.7, 11.7, 11.2, 7.0, 6.1 ppm.

Example FLC-00348-1

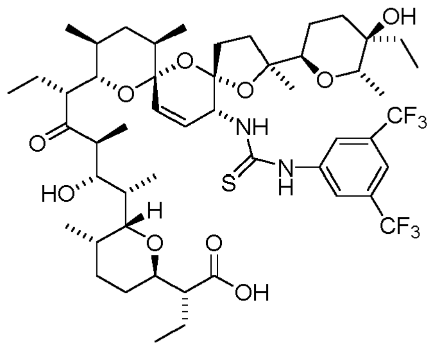

The compound was prepared according to the procedure described in example FLC-00347-1, except that 3,5-bis(trifluoromethyl)phenyl isothiocyanate (1.2 eq) was added to the reaction mixture instead of 4-trifluoromethylphenyl isothiocyanate. The yield of the obtained product was 66%.

ESI-MS for $C_{51}H_{74}F_6N_2O_{10}S$ (*m/z*): $[M+H]^+$ 1021.9, $[M+Na]^+$ 1044.0, $[M-H]^-$ 1019.9.

$^1H$ NMR (500 MHz, $CD_2Cl_2$) δ 9.83 (s, 1H), 8.21 (s, 2H), 7.60 (s, 1H), 7.49 (d, J = 9.4 Hz, 1H), 6.25 (d, J = 10.3 Hz, 1H), 6.05 (dd, J = 10.2, 4.4 Hz, 1H), 5.40 (dd, J = 9.2, 4.3 Hz, 1H), 4.19 (d, J = 10.0 Hz, 1H), 3.92 (q, J = 6.7 Hz, 1H), 3.82 (dd, J = 10.5, 5.3 Hz, 1H), 3.70 (d, J = 10.2 Hz, 1H), 3.61 (dd, J = 6.1, 3.5 Hz, 2H), 2.85 (td, J = 10.5, 4.8 Hz, 1H), 2.78 (dq, J = 14.6, 7.3 Hz, 1H), 2.68 (d, J = 10.5 Hz, 1H), 2.03 - 0.72 (m, 56H) ppm.

$^{13}C$ NMR (126 MHz, $CD_2Cl_2$) δ 217.2, 180.8, 177.8, 141.6, 131.2, 131.0, 128.9, 125.7, 124.5, 123.6, 122.3, 117.3, 117.3, 106.6, 99.1, 86.9, 76.6, 76.3, 74.8, 73.6, 72.0, 71.7, 68.6, 55.0, 52.6, 49.1, 47.8, 39.8, 38.1, 37.9, 35.8, 33.4, 32.5, 31.1, 28.7, 28.4, 25.9, 24.4, 22.0, 20.6, 20.1, 17.2, 15.9, 15.6, 14.4, 13.0, 12.9, 11.5, 11.4, 7.2, 6.1 ppm.

Example FLC-00349-1

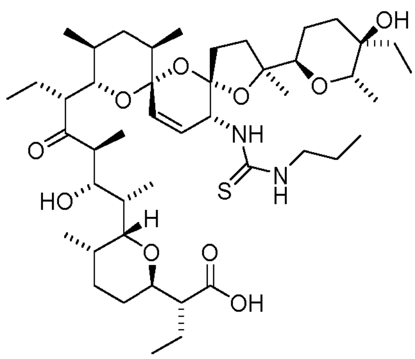

The compound was prepared according to the procedure described in example FLC-00347-1, except that propyl isothiocyanate (5 eq) and, additionally, TEA (5 eq) was added to the reaction mixture instead of 4-trifluoromethylphenyl isothiocyanate. The yield of the obtained product was 74%.

ESI-MS for $C_{46}H_{78}N_2O_{10}S$ (*m/z*): $[M+H]^+$ 851.8, $[M+Na]^+$ 873.8, $[M-H]^-$ 849.9.

$^1H$ NMR (500 MHz, $CD_2Cl_2$) δ 7.05 (s, 1H), 6.78 (d, J = 9.0 Hz, 1H), 6.12 (dd, J = 10.4, 1.5 Hz, 1H), 5.96 (dd, J = 10.3, 3.7 Hz, 1H), 5.26 (s, 1H), 4.10 - 4.02 (m, 1H), 3.91 (dd, J = 10.7, 5.5 Hz, 1H), 3.83 (q, J = 6.6 Hz, 1H), 3.72 (d, J = 10.7 Hz, 1H), 3.60 (dd, J = 7.4, 2.3 Hz, 2H), 3.51 - 3.41 (m, 2H), 2.90 (td, J = 10.8, 4.1 Hz, 1H), 2.73 (dq, J = 9.9, 7.2 Hz, 1H), 2.62 (d, J = 9.7 Hz, 1H), 2.02 - 0.69 (m, 61H) ppm.

$^{13}C$ NMR (126 MHz, $CD_2Cl_2$) δ 216.5, 182.6, 178.1, 130.4, 124.7, 107.2, 99.7, 87.5, 77.3, 76.2, 75.3, 73.8, 71.8, 71.7, 68.7, 55.8, 49.7, 48.4, 47.0, 40.6, 38.6, 37.6, 36.5, 33.0, 31.3, 30.1, 30.1, 29.2, 28.6, 26.4, 25.2, 22.9, 22.8, 21.1, 20.4, 17.8, 16.4, 15.9, 14.8, 13.3, 13.2, 12.1, 11.7, 11.6, 7.3, 6.6 ppm.

Example FLC-00352-1

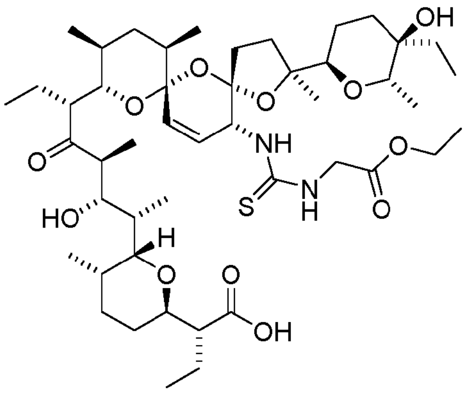

The compound was prepared according to the procedure described in example FLC-00347-1, except that ethyl isothiocyanatoacetate (3 eq) and, additionally, TEA (3 eq) was added to the reaction mixture instead of 4-trifluoromethylphenyl isothiocyanate. The yield of the obtained product was 77%.

ESI-MS for $C_{47}H_{78}N_2O_{12}S$ (*m/z*): $[M+H]^+$ 895.9, $[M+Na]^+$ 917.9, $[M-H]^-$ 893.9.

$^1H$ NMR (500 MHz, $CD_2Cl_2$) δ 7.41 (d, J = 7.1 Hz, 1H), 6.89 (s, 1H), 6.10 (dd, J = 10.6, 2.2 Hz, 1H), 5.86 (dd, J = 10.5, 2.5 Hz, 1H), 5.14 (s, 1H), 4.47 (dd, J = 18.3, 5.4 Hz, 1H), 4.31 (d, J = 5.0 Hz, 1H), 4.22 - 4.15 (m, 4H), 4.08 (d, J = 10.1 Hz, 1H), 3.99 - 3.89 (m, 2H), 3.76 (t, J = 12.3 Hz, 2H), 3.59 (dd, J = 10.0, 1.3 Hz, 1H), 2.89 (td, J = 10.8, 4.0 Hz, 1H), 2.79 - 2.70 (m, 1H), 2.63 (d, J = 9.4 Hz, 1H), 2.03 - 0.70 (m, 56H) ppm.

$^{13}C$ NMR (126 MHz, $CD_2Cl_2$) δ 216.2, 183.1, 177.7, 170.4, 129.4, 123.8, 106.1, 99.2, 87.6, 76.9, 75.8, 74.9, 73.5, 71.6, 71.3, 68.5, 61.4, 55.5, 49.3, 48.3, 46.3, 40.7, 38.4, 37.2, 36.2, 32.8, 31.4, 31.2, 29.7, 28.6, 28.1, 26.1, 25.2, 22.6, 21.2, 19.9, 17.5, 16.2, 15.5, 14.6, 13.9, 12.9, 12.8, 11.7, 10.9, 6.8, 6.1 ppm.

Example FLC-00368-1

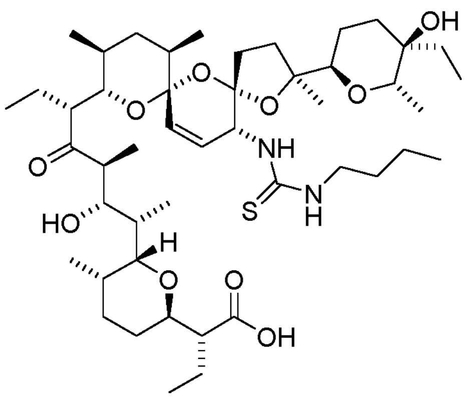

The compound was prepared according to the procedure described in example FLC-00347-1, except that propyl isothiocyanate (3 eq) and, additionally, TEA (3 eq) was added to the reaction mixture instead of 4-trifluoromethylphenyl isothiocyanate. The yield of the obtained product was 27%.

ESI-MS for $C_{47}H_{80}N_2O_{10}S$ (*m/z*): $[M+H]^+$ 865.9, $[M+Na]^+$ 888.0, $[M-H]^-$ 864.0.

$^1H$ NMR (500 MHz, $CDCl_3$) δ 6.86 (d, J = 8.1 Hz, 2H), 6.10 (dd, J = 10.5, 1.9 Hz, 1H), 5.94 (dd, J = 10.4, 3.0 Hz, 1H), 5.28 (s, 1H), 4.11 (d, J = 10.1 Hz, 1H), 3.96 (dd, J = 10.5, 5.5 Hz, 1H), 3.87 (d, J = 6.6 Hz, 1H), 3.74 (d, J = 10.1 Hz, 1H), 3.65 - 3.59 (m, 2H), 3.57 - 3.46 (m, 2H), 2.88 (td, J = 10.8, 3.8 Hz, 1H), 2.70 (dt, J = 14.4, 7.2 Hz, 1H), 2.57 (d, J = 9.8 Hz, 1H), 2.08 - 0.69 (m, 63H) ppm.

$^{13}C$ NMR (126 MHz, $CDCl_3$) δ 215.5, 182.0, 178.2, 129.8, 123.6, 106.5, 99.1, 87.0, 76.8, 75.7, 74.9, 73.7, 71.6, 71.4, 68.3, 55.5, 53.0, 49.4, 48.3, 44.6, 40.6, 38.4, 37.1, 36.2, 32.7, 32.6, 31.4, 31.0, 29.7, 28.8, 28.2, 26.2, 25.6, 22.5, 20.7, 20.1, 17.8, 16.0, 15.9, 14.6, 13.9, 13.3, 13.0, 11.9, 11.4, 7.2, 6.4 ppm.

Example FLC-00369-1

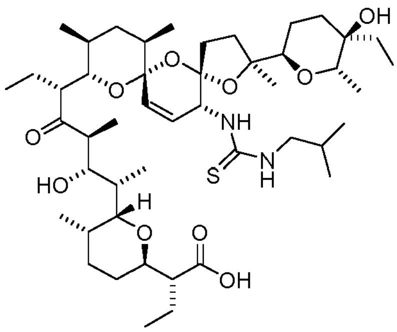

The compound was prepared according to the procedure described in example FLC-00347-1, except that isobuthyl isothiocyanate (3 eq) and, additionally, TEA (3 eq) was added to the reaction mixture instead of 4-trifluoromethylphenyl isothiocyanate. The yield of the obtained product was 45%.

ESI-MS for $C_{47}H_{80}N_2O_{10}S$ (*m/z*): $[M+H]^+$ 866.0, $[M+Na]^+$ 888.0, $[M-H]^-$ 864.0.

$^1H$ NMR (500 MHz, $CDCl_3$) δ 6.90 (s, 2H), 6.10 (dd, J = 10.5, 1.8 Hz, 1H), 5.94 (dd, J = 10.4, 2.7 Hz, 1H), 5.30 (s, 1H), 4.11 (d, J = 10.1 Hz, 1H), 3.95 (dd, J = 10.6, 5.5 Hz, 1H), 3.86 (d, J = 6.5 Hz, 1H), 3.73 (d, J = 10.2 Hz, 1H), 3.63 (d, J = 10.5 Hz, 2H), 3.36 (d, J = 4.4 Hz, 2H), 2.88 (td, J = 10.8, 3.8 Hz, 1H), 2.70 (dq, J = 14.4, 7.1 Hz, 1H), 2.57 (d, J = 9.8 Hz, 1H), 2.09 - 0.69 (m, 63H) ppm.

$^{13}C$ NMR (126 MHz, $CDCl_3$) δ 215.4, 182.3, 178.2, 129.8, 123.7, 106.6, 99.2, 87.0, 75.7, 74.9, 73.7, 71.6, 71.4, 68.3, 55.5, 53.1, 52.3, 49.4, 48.3, 40.5, 38.4, 37.0, 36.2, 32.6, 31.0, 29.7, 28.8, 28.3, 28.2, 26.1, 25.5, 22.5, 20.7, 20.3, 20.3, 20.1, 17.8, 16.1, 15.8, 14.6, 13.3, 13.0, 11.9, 11.4, 7.1, 6.4 ppm.

Example FLC-00377-1

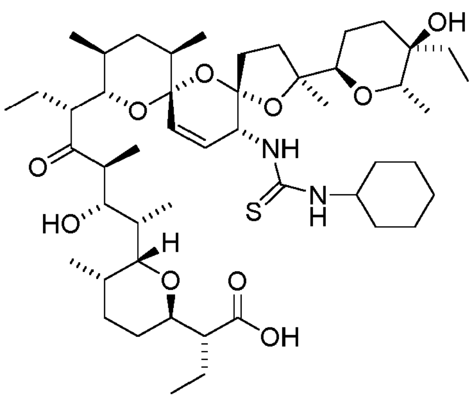

The compound was prepared according to the procedure described in example FLC-00347-1, except that cyclohexyl isothiocyanate (5 eq) and, additionally, TEA (5 eq) was added to the reaction mixture instead of 4-trifluoromethylphenyl isothiocyanate. The yield of the obtained product was 81%.

ESI-MS for $C_{49}H_{82}N_2O_{10}S$ (*m/z*): $[M+H]^+$ 892.0, $[M+Na]^+$ 914.0, $[M-H]^-$ 890.0.

$^1H$ NMR (500 MHz, $CD_2Cl_2$) δ 6.90 (s, 1H), 6.76 (d, J = 6.4 Hz, 1H), 6.12 (dd, J = 10.4, 1.5 Hz, 1H), 5.95 (dd, J = 10.3, 3.6 Hz, 1H), 5.28 (s, 1H), 4.20 - 4.08 (m, 1H), 4.07 (dd, J = 10.1, 1.0 Hz, 1H), 3.91 (dd, J = 10.6, 5.3 Hz, 1H), 3.84 (dd, J = 13.3, 6.5 Hz, 1H), 3.75 - 3.69 (m, 1H), 3.61 (dd, J = 10.3, 1.6 Hz, 2H), 2.91 (td, J = 10.8, 4.1 Hz, 1H), 2.72 (dq, J = 9.9, 7.2 Hz, 1H), 2.62 (d, J = 9.9 Hz, 1H), 2.01 - 0.69 (m, 66H) ppm.

$^{13}C$ NMR (126 MHz, $CD_2Cl_2$) δ 215.9, 180.8, 177.7, 130.1, 124.3, 106.9, 99.3, 86.9, 77.0, 75.8, 74.9, 73.6, 71.5, 71.3, 68.4, 55.3, 52.8, 49.3, 48.0, 40.2, 38.2, 37.2, 36.1, 33.0, 32.8, 32.6, 32.6, 31.0, 29.7, 28.8, 28.2, 26.0, 25.7, 25.0, 24.9, 24.7, 22.4, 20.7, 20.1, 17.4, 16.0, 15.5, 14.5, 13.0, 12.9, 11.8, 11.3, 7.0, 6.3 ppm.

Example FLC-00378-1

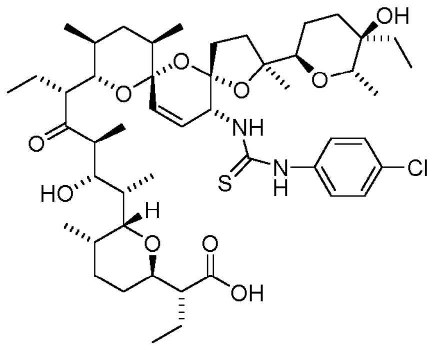

The compound was prepared according to the procedure described in example FLC-00347-1, except that 4-chlorophenyl isothiocyanate (3 eq) and, additionally, TEA (2 eq) was added to the reaction mixture instead of 4-trifluoromethylphenyl isothiocyanate. The yield of the obtained product was 67%.
ESI-MS for $C_{49}H_{75}ClN_2O_{10}S$ (*m/z*): $[M+H]^+$ 920.0, $[M+Na]^+$ 942.0, $[M-H]^-$ 918.0.
$^1H$ NMR (500 MHz, $CD_2Cl_2$) δ 9.28 (s, 1H), 7.47 (d, J = 8.7 Hz, 2H), 7.30 - 7.22 (m, 3H), 6.21 (d, J = 10.2 Hz, 1H), 6.07 (dd, J = 10.2, 4.5 Hz, 1H), 5.40 (dd, J = 8.8, 4.0 Hz, 1H), 4.18 - 4.13 (m, 1H), 3.89 (dd, J = 10.6, 5.4 Hz, 1H), 3.78 (q, J = 6.7 Hz, 1H), 3.71 (d, J = 10.2 Hz, 1H), 3.63 - 3.56 (m, 2H), 2.89 (td, J = 10.8, 4.2 Hz, 1H), 2.76 (dq, J = 14.5, 7.2 Hz, 1H), 2.66 (d, J = 10.2 Hz, 1H), 2.01 - 0.72 (m, 56H) ppm.
$^{13}C$ NMR (126 MHz, $CD_2Cl_2$) δ 217.0, 181.3, 177.7, 138.4, 129.8, 129.5, 128.3, 126.0, 125.6, 107.0, 99.3, 86.9, 76.9, 76.0, 74.9, 73.3, 71.5, 71.5, 68.4, 55.2, 49.3, 47.8, 39.9, 38.1, 37.7, 36.0, 33.1, 32.5, 30.9, 29.7, 28.7, 28.2, 25.9, 24.2, 22.1, 20.7, 20.1, 17.3, 16.0, 15.5, 14.4, 12.9, 12.8, 11.7, 11.3, 7.1, 6.3 ppm.

Example FLC-00379-1

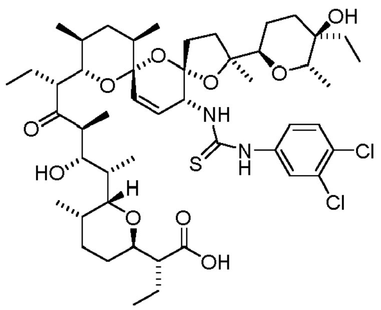

The compound was prepared according to the procedure described in example FLC-00347-1, except that 3,4-dichlorophenyl isothiocyanate (1.2 eq) was added to the reaction mixture instead of 4-trifluoromethylphenyl isothiocyanate. The yield of the obtained product was 70%.
ESI-MS for $C_{49}H_{74}Cl_2N_2O_{10}S$ (*m/z*): $[M+Na]^+$ 976.0, $[M-H]^-$ 952.0.
$^1H$ NMR (500 MHz, $CD_2Cl_2$) δ 9.58 (s, 1H), 7.75 (d, J = 2.3 Hz, 1H), 7.46 (dd, J = 8.7, 2.3 Hz, 1H), 7.37 (d, J = 8.7 Hz, 1H), 7.28 (d, J = 9.5 Hz, 1H), 6.24 (d, J = 10.1 Hz, 1H), 6.12 (dd, J = 10.0, 5.2 Hz, 1H), 5.39 (dd, J = 9.3, 5.3 Hz, 1H), 4.18 (dd, J = 10.0, 1.0 Hz, 1H), 3.90 (dd, J = 10.6, 5.2 Hz, 1H), 3.84 (q, J = 6.7 Hz, 1H), 3.67 (d, J = 10.1 Hz, 1H), 3.59 (dd, J = 11.2, 1.7 Hz, 2H), 2.89 (td, J = 10.6, 4.4 Hz, 1H), 2.76 (dq, J = 14.6, 7.2 Hz, 1H), 2.68 (d, J = 10.5 Hz, 1H), 2.00 - 0.72 (m, 56H) ppm.
$^{13}C$ NMR (126 MHz, $CD_2Cl_2$) δ 217.4, 181.1, 177.4, 139.6, 131.6, 129.6, 127.8, 126.4, 126.3, 124.3, 107.3, 99.3, 86.7, 76.9, 76.2, 74.8, 73.3, 71.6, 71.5, 68.5, 55.0, 49.3, 47.6, 39.5, 38.0, 37.9, 35.9, 33.5, 32.4, 30.9, 28.8, 28.3, 25.8, 23.7, 21.9, 20.7, 20.2, 17.2, 16.0, 15.5, 14.4, 12.9, 12.8, 11.7, 11.6, 7.2, 6.3 ppm.

Example FLC-00380-1

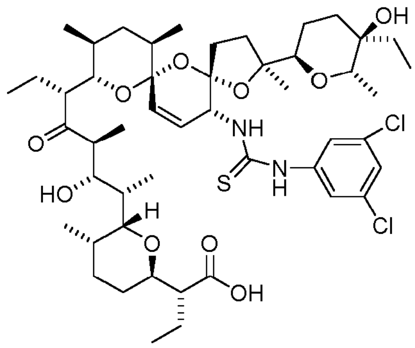

The compound was prepared according to the procedure described in example FLC-00347-1, except that 3,5-dichlorophenyl isothiocyanate (1.2 eq) was added to the reaction mixture instead of 4-trifluoromethylphenyl isothiocyanate. The yield of the obtained product was 59%.

ESI-MS for $C_{49}H_{74}Cl_2N_2O_{10}S$ (*m/z*): $[M+Na]^+$ 976.0, $[M-H]^-$ 952.0.

$^1$H NMR (500 MHz, $CD_2Cl_2$) δ 9.55 (s, 1H), 7.60 (d, J = 1.6 Hz, 2H), 7.35 (d, J = 9.4 Hz, 1H), 7.11 (t, J = 1.8 Hz, 1H), 6.24 (d, J = 10.2 Hz, 1H), 6.08 (dd, J = 10.1, 4.9 Hz, 1H), 5.39 (dd, J = 9.3, 4.9 Hz, 1H), 4.18 (dd, J = 10.0, 1.2 Hz, 1H), 3.95 - 3.83 (m, 2H), 3.67 (d, J = 10.3 Hz, 1H), 3.63 - 3.57 (m, 2H), 2.88 (td, J = 10.5, 4.5 Hz, 1H), 2.76 (dq, J = 14.6, 7.3 Hz, 1H), 2.67 (d, J = 10.5 Hz, 1H), 2.02 - 0.71 (m, 56H) ppm.

$^{13}$C NMR (126 MHz, $CD_2Cl_2$) δ 217.1, 180.9, 177.4, 142.0, 134.0, 129.4, 126.1, 124.2, 122.7, 107.0, 99.1, 86.8, 76.8, 76.3, 74.7, 73.4, 71.8, 71.6, 68.6, 54.9, 52.8, 49.1, 47.7, 39.6, 38.1, 37.9, 35.8, 33.5, 32.4, 30.9, 29.7, 28.7, 28.4, 25.9, 24.0, 22.0, 20.7, 20.2, 17.2, 15.9, 15.5, 14.4, 13.0, 12.9, 11.6, 11.6, 7.3, 6.4 ppm.

Example FLC-00382-1

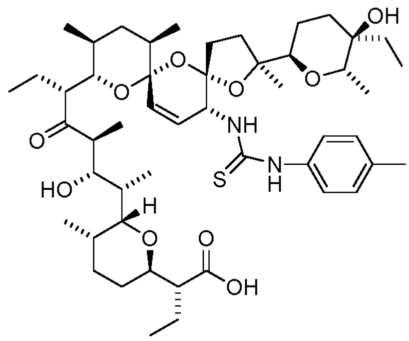

The compound was prepared according to the procedure described in example FLC-00347-1, except that 4-methylphenyl isothiocyanate (3 eq) and, additionally, TEA (3 eq) was added to the reaction mixture instead of 4-trifluoromethylphenyl isothiocyanate. The yield of the obtained product was 80%.

ESI-MS for $C_{50}H_{78}N_2O_{10}S$ (*m/z*): $[M+H]^+$ 900.0, $[M+Na]^+$ 922.0, $[M-H]^-$ 898.0.

$^1$H NMR (500 MHz, $CD_2Cl_2$) δ 9.07 (s, 1H), 7.28 (d, J = 8.3 Hz, 2H), 7.13 (d, J = 8.2 Hz, 2H), 7.08 (d, J = 9.4 Hz, 1H), 6.20 (d, J = 10.3 Hz, 1H), 6.10 (dd, J = 10.2, 4.5 Hz, 1H), 5.41 (d, J = 4.7 Hz, 1H), 4.23 - 4.10 (m, 1H), 3.93 (dd, J = 10.7, 5.4 Hz, 1H), 3.75 - 3.65 (m, 3H), 3.59 (d, J = 10.0 Hz, 1H), 2.92 (td, J = 10.8, 4.1 Hz, 1H), 2.76 (dq, J = 9.9, 7.2 Hz, 1H), 2.66 (d, J = 9.8 Hz, 1H), 2.32 (s, 3H), 2.04 - 0.72 (m, 56H) ppm.

$^{13}$C NMR (126 MHz, $CD_2Cl_2$) δ 216.9, 181.5, 177.6, 136.8, 135.0, 129.8, 129.1, 125.6, 125.0, 107.1, 99.5, 86.9, 76.9, 75.8, 74.9, 73.1, 71.4, 71.3, 68.4, 55.4, 49.6, 47.8, 39.9, 38.1, 37.4, 36.1, 33.1, 32.6, 30.7, 29.7, 28.8, 28.2, 25.9, 23.8, 22.2, 20.9, 20.7, 20.0, 17.4, 16.1, 15.5, 14.4, 12.9, 12.8, 11.8, 11.3, 7.0, 6.2 ppm.

Example FLC-00386-1

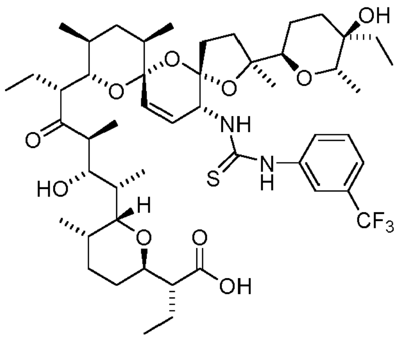

The compound was prepared according to the procedure described in example FLC-00347-1, except that 3-trifluoromethylphenyl isothiocyanate (3 eq) and, additionally, TEA (3 eq) was added to the reaction mixture instead of 4-trifluoromethylphenyl isothiocyanate. The yield of the obtained product was 38%.

ESI-MS for $C_{50}H_{75}F_3N_2O_{10}S$ (*m/z*): $[M+H]^+$ 953.8, $[M+Na]^+$ 975.8, $[M-H]^-$ 951.8.

$^1H$ NMR (500 MHz, $CD_2Cl_2$) δ 9.47 (s, 1H), 7.91 - 7.80 (m, 2H), 7.43 (t, J = 7.9 Hz, 1H), 7.37 (d, J = 8.0 Hz, 2H), 6.22 (dd, J = 10.3, 1.0 Hz, 1H), 6.04 (dd, J = 10.3, 4.2 Hz, 1H), 5.40 (dd, J = 9.0, 3.7 Hz, 1H), 4.17 (dd, J = 10.1, 0.9 Hz, 1H), 3.91 - 3.83 (m, 2H), 3.74 - 3.69 (m, 1H), 3.64 - 3.60 (m, 2H), 2.87 (td, J = 10.7, 4.4 Hz, 1H), 2.77 (tt, J = 14.5, 7.2 Hz, 1H), 2.67 (d, J = 10.2 Hz, 1H), 2.02 - 0.68 (m, 58H) ppm.

$^{13}C$ NMR (126 MHz, $CD_2Cl_2$) δ 217.0, 181.2, 177.9, 140.5, 131.7, 130.2, 129.2, 128.7, 127.6, 125.3, 123.1, 121.0, 120.9, 106.7, 99.2, 86.9, 76.8, 76.0, 74.9, 73.6, 71.8, 71.5, 68.5, 55.2, 52.7, 49.2, 47.9, 38.1, 37.8, 36.0, 33.2, 32.5, 31.0, 28.7, 28.3, 25.9, 24.6, 22.1, 20.6, 20.0, 17.3, 15.9, 15.6, 14.4, 12.9, 12.9, 11.6, 11.3, 7.1, 6.1 ppm.

Example FLC-00387-1

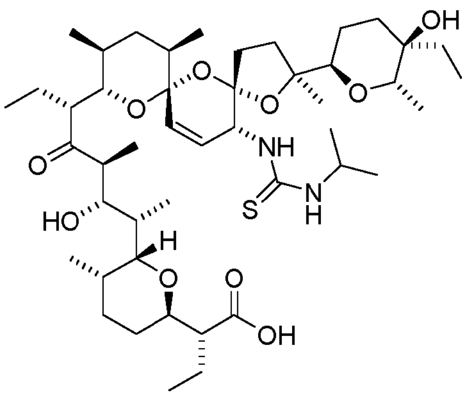

The compound was prepared according to the procedure described in example FLC-00347-1, except that isopropyl isothiocyanate (5 eq) and, additionally, TEA (5 eq) was added to the reaction mixture instead of 4-trifluoromethylphenyl isothiocyanate. The yield of the obtained product was 61%.

ESI-MS for $C_{46}H_{78}N_2O_{10}S$ (*m/z*): $[M+H]^+$ 851.8, $[M+Na]^+$ 873.8, $[M-H]^-$ 849.9.

$^1H$ NMR (500 MHz, $CD_2Cl_2$) δ 6.88 (s, 1H), 6.71 (d, J = 9.0 Hz, 1H), 6.11 (dd, J = 10.4, 1.5 Hz, 1H), 5.95 (dd, J = 10.3, 3.7 Hz, 1H), 5.27 (s, 1H), 4.53 - 4.37 (m, 1H), 4.10 - 4.02 (m, 1H), 3.91 (dd, J = 10.7, 5.4 Hz, 1H), 3.83 (q, J = 6.6 Hz, 1H), 3.76 - 3.70 (m, 1H), 3.63 - 3.58 (m, 2H), 2.91 (td, J = 10.8, 4.1 Hz, 1H), 2.72 (dq, J = 10.0, 7.2 Hz, 1H), 2.62 (d, J = 9.8 Hz, 1H), 2.00 - 0.69 (m, 62H) ppm.

$^{13}C$ NMR (126 MHz, $CD_2Cl_2$) δ 216.1, 180.9, 177.7, 130.1, 124.2, 106.8, 99.3, 87.0, 76.8, 75.8, 74.9, 73.5, 71.5, 71.3, 68.4, 55.4, 52.8, 49.3, 48.0, 46.1, 40.2, 38.2, 37.2, 36.1, 32.6, 31.0, 29.7, 28.8, 28.2, 26.0, 24.7, 22.5, 22.4, 22.1, 20.7, 20.1, 17.4, 16.0, 15.5, 14.4, 13.0, 12.9, 11.8, 11.2, 6.9, 6.3 ppm.

Example FLC-00393-1

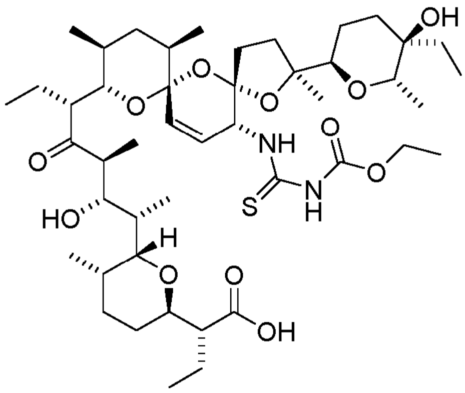

The compound was prepared according to the procedure described in example FLC-00347-1, except that ethoxycarbonyl isothiocyanate (1.5 eq) was added to the reaction mixture instead of 4-trifluoromethylphenyl isothiocyanate. The yield of the obtained product was 28%.

ESI-MS for $C_{46}H_{76}N_2O_{12}S$ (*m/z*): $[M+H]^+$ 881.8, $[M+Na]^+$ 903.9, $[M-H]^-$ 879.9.

$^1H$ NMR (500 MHz, $CD_2Cl_2$) δ 9,69 (d, J = 9.0 Hz, 1H), 8.20 (s, 1H), 6.18 (dd, J = 10.7, 2.8 Hz, 1H), 5.81 (dd, J = 10.7, 1.4 Hz, 1H), 5.19 (d, J = 8.9 Hz, 1H), 4.27 - 4.14 (m, 2H), 4.06 (d, J = 10.1 Hz, 1H), 3.93 (dd, J = 10.7, 5.7 Hz, 1H), 3.79 (d, J = 10.4 Hz, 1H), 3.76 - 3.71 (m, 1H), 3.59 (dd, J = 9.9, 1.5 Hz, 1H), 3.53 (d, J = 9.8 Hz, 1H), 2.94 - 2.79 (m, 2H), 2.68 - 2.60 (m, 1H), 2.05 - 0.71 (m, 59H) ppm.

$^{13}C$ NMR (126 MHz, $CD_2Cl_2$) δ 215.8, 180.1, 177.8, 152.7, 127.7, 124.1, 105.6, 99.9, 88.5, 77.6, 77.3, 75.2, 72.8, 72.1, 71.4, 69.1, 63.2, 56.6, 54.5, 49.7, 49.2, 41.6, 39.2, 36.8, 36.3, 33.2, 33.0, 31.0, 29.7, 28.6, 26.7, 24.6, 23.1, 21.7, 20.4, 18.0, 17.7, 15.9, 14.6, 14.4, 13.5, 13.5, 12.1, 11.3, 7.2, 6.7 ppm.

Example FLC-00394-1

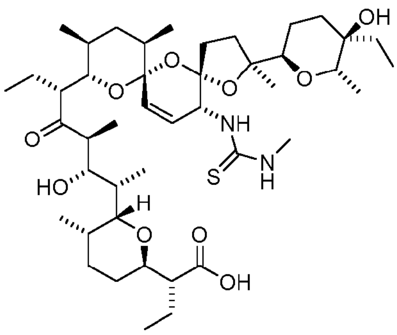

The compound was prepared according to the procedure described in example FLC-00347-1, except that methyl isothiocyanate (5 eq) and, additionally, TEA (5 eq) was added to the reaction mixture instead of 4-trifluoromethylphenyl isothiocyanate. Following purification, the compound was washed with 0.25 M $Na_2CO_3$ instead of 0.06 M $H_2SO_4$. The yield of the obtained product was 38%.

ESI-MS for $C_{44}H_{74}N_2O_{10}S$ (*m/z*): $[M+H]^+$ 823.9, $[M+Na]^+$ 845.9, $[M-H]^-$ 822.0.

$^1H$ NMR (500 MHz, $CD_2Cl_2$) δ 7.63 (d, J = 3.5 Hz, 1H), 6.79 (d, J = 9.9 Hz, 1H), 6.09 (dd, J = 10.8, 2.9 Hz, 1H), 5.69 (dd, J = 10.8, 1.7 Hz, 1H), 5.53 - 5.42 (m, 1H), 4.28 (q, J = 6.7 Hz, 1H), 4.07 (d, J = 10.5 Hz, 1H), 3.81 (dd, J = 11.0, 4.7 Hz, 1H), 3.63 - 3.57 (m, 3H), 3.47 (dd, J = 12.4, 3.0 Hz, 1H), 2.92 (d, J = 4.4 Hz, 3H), 2.81 (td, J = 11.1, 3.3 Hz, 1H), 2.75 - 2.66 (m, 2H), 2.25 - 2.14 (m, 1H), 1.97 - 0.68 (m, 54H) ppm.

$^{13}C$ NMR (126 MHz, $CD_2Cl_2$) δ 219.4, 184.8, 183.6, 128.4, 122.7, 106.5, 98.8, 88.4, 75.8, 75.6, 73.5, 71.2, 71.1, 68.1, 56.1, 50.9, 50.4, 49.5, 40.5, 38.5, 37.0, 35.8, 32.4, 32.0, 30.8, 29.7, 28.0, 27.6, 27.2, 26.6, 23.4, 19.8, 19.5, 17.2, 15.8, 15.4, 14.2, 12.8, 12.0, 11.8, 10.5, 6.4, 6.1 ppm.

Example FLC-00467-1

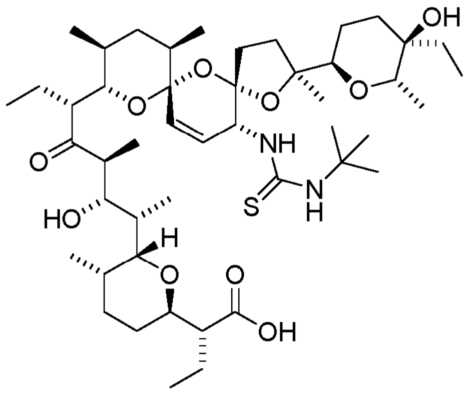

The compound was prepared according to the procedure described in example FLC-00347-1, except that tert-butyl isothiocyanate (5 eq) and, additionally, TEA (5 eq) was added to the reaction mixture instead of 4-trifluoromethylphenyl isothiocyanate. Following purification, the compound was washed with 0.25 M $Na_2CO_3$ instead of 0.06 M $H_2SO_4$. The yield of the obtained product was 70%.

ESI-MS for $C_{47}H_{80}N_2O_{10}S$ (*m/z*): $[M+H]^+$ 866.0, $[M+Na]^+$ 888.0, $[M-H]^-$ 864.0.

$^1H$ NMR (700 MHz, $CD_2Cl_2$) δ 7.21 (s, 1H), 6.64 (d, J = 9.7 Hz, 1H), 6.11 (dd, J = 10.9, 3.1 Hz, 1H), 5.72 (dd, J = 10.8, 1.8 Hz, 1H), 5.68 (ddd, J = 9.7, 2.9, 1.9 Hz, 1H), 5.17 (s, 1H), 4.33 (q, J = 6.8 Hz, 1H), 4.09 (dd, J = 10.4, 1.5 Hz, 1H), 3.89 (dd, J = 11.0, 5.5 Hz, 1H), 3.72 - 3.69 (m, 2H), 3.53 (dd, J = 12.3, 3.1 Hz, 1H), 2.85 (td, J = 11.0, 3.4 Hz, 1H), 2.81 - 2.72 (m, 2H), 2.41 - 2.32 (m, 1H), 2.03 - 0.73 (m, 63H) ppm.

$^{13}C$ NMR (176 MHz, $CD_2Cl_2$) δ 219.6, 184.4, 181.0, 129.3, 122.0, 106.7, 99.0, 88.3, 76.0, 75.4, 75.2, 73.3, 71.2, 71.1, 68.1, 56.3, 53.3, 50.1, 50.0, 49.9, 40.6, 38.6, 36.7, 35.7, 32.5, 32.3, 32.3, 29.0, 28.2, 28.0, 27.4, 26.7, 23.0, 20.0, 19.4, 17.3, 15.8, 15.6, 14.1, 13.1, 12.9, 11.9, 10.7, 6.5, 6.3 ppm.

Example FLC-00771-1

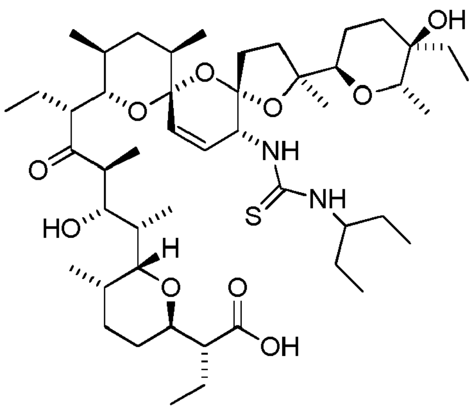

The compound was prepared according to the procedure described in example FLC-00347-1, except that 3-pentyl isothiocyanate (3 eq) and, additionally, TEA (3 eq) was added to the reaction mixture instead of 4-trifluoromethylphenyl isothiocyanate. Following purification, the compound was washed with 0.25 M $Na_2CO_3$ instead of 0.06 M $H_2SO_4$. The yield of the obtained product was 36%.

ESI-MS for $C_{48}H_{82}N_2O_{10}S$ (*m/z*): $[M+H]^+$ 879.9, $[M+Na]^+$ 901.9, $[M-H]^-$ 878.2.

$^1H$ NMR (400 MHz, $CD_2Cl_2$) δ 6.97 (d, J = 8.8 Hz, 1H), 6.62 (d, J = 9.8 Hz, 1H), 6.12 (d, J = 10.8 Hz, 1H), 5.74 (dd, J = 25.0, 10.3 Hz, 3H), 5.12 (s, 1H), 4.38 (d, J = 6.3 Hz, 2H), 4.12 (d, J = 10.4 Hz, 1H), 3.88 (d, J = 7.7 Hz, 1H), 3.71 (s, 1H), 3.66 (s, 1H), 3.54 (d, J = 11.7 Hz, 1H), 2.85 (t, J = 10.1 Hz, 1H), 2.75 (d, J = 10.3 Hz, 2H), 2.28 - 0.72 (m, 64H) ppm.

$^{13}C$ NMR (101 MHz, $CD_2Cl_2$) δ 219.5, 184.2, 182.8, 129.3, 122.2, 106.4, 99.1, 88.4, 75.9, 75.4, 73.4, 71.3, 71.1, 68.0, 57.5, 56.1, 51.3, 50.0, 49.8, 40.6, 38.6, 36.3, 35.7, 32.4, 32.4, 32.0, 29.7, 28.0, 27.8, 27.7, 27.6, 27.1, 26.6, 23.3, 20.0, 19.6, 17.3, 15.7, 15.6, 14.2, 12.9, 12.9, 11.9, 10.7, 10.6, 10.5, 6.5, 6.4 ppm.

Example FLC-00361-1

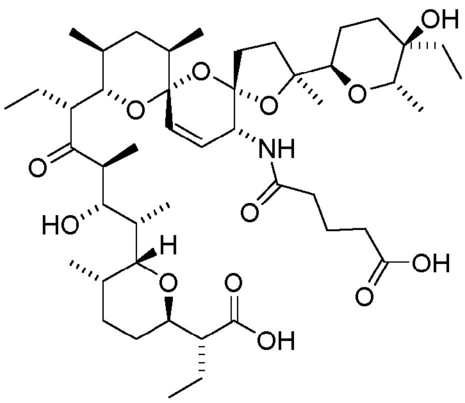

100 mg C20-amino-SAL (1 eq) was diluted in DCM, TEA (1 eq) and glutaric anhydride (1 eq) were added. The reaction was carried out at RT (TLC and LC-MS control). The post-reaction mixture was then suspended on silica gel and purified using a chromatograph with an ELS detector using a column packed with silica gel. The respective fractions were combined and concentrated, and the residue was dissolved in DCM and washed twice with 0.06 M $H_2SO_4$. The yield of the obtained product was 9%.

ESI-MS for $C_{47}H_{77}NO_{13}$ (*m/z*): $[M+Na]^+$ 887.1, $[M-H]^-$ 862.9.

$^1H$ NMR (500 MHz, $CD_2Cl_2$) δ 7.59 (d, *J* = 8.5 Hz, 1H), 5.81 - 5.70 (m, 2H), 4.49 (d, *J* = 8.4 Hz, 1H), 4.15 (d, *J* = 10.0 Hz, 1H), 3.87 (dd, *J* = 17.9, 5.9 Hz, 2H), 3.70 (d, *J* = 10.4 Hz, 1H), 3.64 - 3.54 (m, 2H), 2.90 (d, *J* = 3.6 Hz, 1H), 2.68 (dd, *J* = 10.1, 7.8 Hz, 2H), 2.37 - 0.61 (m, 64H) ppm.

$^{13}C$ NMR (126 MHz, $CD_2Cl_2$) δ 213.4, 178.3, 176.0, 173.2, 132.9, 131.0, 108.6, 96.4, 86.3, 77.0, 74.7, 73.4, 73.1, 71.7, 71.5, 67.0, 50.6, 49.5, 48.8, 39.1, 36.7, 36.5, 36.0, 35.0, 32.7, 32.5, 31.0, 29.7, 29.6, 29.0, 27.9, 26.1, 24.9, 23.0, 22.3, 20.8, 19.8, 17.9, 16.9, 15.7, 15.0, 12.9, 12.9, 11.9, 10.7, 6.8, 6.1 ppm.

Example FLC-00445-1

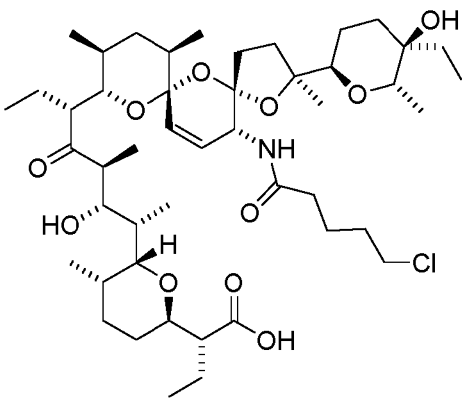

100 mg of C20-amino SAL (1 eq) was mixed with 5-chloropentanoyl chloride (2.2 eq) and TEA (8 eq) in DCM. The reaction was carried out at RT (TLC and LC-MS control). The DCM was then concentrated, MeCN supplemented with 1M NaOH was added to the residue followed by stirring at RT (LC-MS control). The post-reaction mixture was diluted with $H_2O$ and extracted twice with DCM. The organic layers were combined, suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was 51%.

ESI-MS for $C_{47}H_{78}ClNO_{11}$ (*m/z*): $[M+Na]^+$ 890.9, $[M-H]^-$ 867.0.

$^1H$ NMR (700 MHz, $CDCl_3$) δ 7.08 (d, *J* = 9.6 Hz, 1H), 6.11 (dd, *J* = 10.9, 3.1 Hz, 1H), 5.69 (dd, *J* = 10.8, 1.8 Hz, 1H),

4.85 (ddd, $J$ = 9.6, 2.9, 2.0 Hz, 1H), 4.29 (q, $J$ = 6.7 Hz, 1H), 4.22 (dd, $J$ = 10.4, 1.3 Hz, 1H), 3.90 (dd, $J$ = 11.0, 4.8 Hz, 1H), 3.62 (dd, $J$ = 10.2, 2.0 Hz, 1H), 3.54 - 3.50 (m, 3H), 3.37 (dd, $J$ = 12.1, 2.3 Hz, 1H), 2.86 (td, $J$ = 10.7, 3.3 Hz, 1H), 2.66 (ddd, $J$ = 18.6, 10.8, 5.6 Hz, 2H), 2.49 (ddd, $J$ = 15.1, 9.0, 6.0 Hz, 1H), 2.23 (ddd, $J$ = 15.3, 9.4, 5.8 Hz, 1H), 2.09 - 0.65 (m, 61H) ppm.

$^{13}C$ NMR (176 MHz, $CDCl_3$) δ 217.7, 185.1, 173.4, 128.0, 123.4, 106.3, 98.9, 88.5, 76.5, 76.0, 75.6, 74.8, 71.5, 70.0, 68.3, 55.9, 51.2, 49.2, 46.2, 44.9, 40.6, 38.8, 37.2, 36.0, 35.1, 32.6, 32.5, 32.4, 32.3, 29.2, 28.0, 28.0, 26.9, 24.3, 22.6, 20.1, 20.0, 17.6, 16.0, 15.5, 14.7, 13.2, 12.7, 12.2, 10.8, 6.7, 6.58 ppm.

Example FLC-00455-1

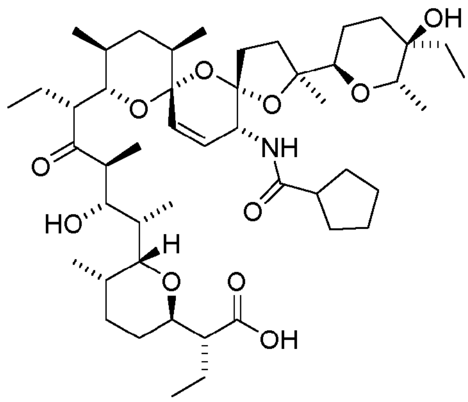

The compound was prepared according to the procedure described in example FLC-00445-1, except that cyclopentanecarbonyl chloride (2.2 eq) was added to the reaction mixture instead of 5-chloropentanoyl chloride. The yield of the obtained product was 80%.

ESI-MS for $C_{48}H_{79}NO_{11}$ *(m/z):* $[M+Na]^+$ 869.0.

$^{1}H$ NMR (700 MHz, $CDCl_3$) δ 7.00 (d, $J$ = 9.4 Hz, 1H), 6.13 (dd, $J$ = 10.9, 3.1 Hz, 1H), 5.75 (dd, $J$ = 10.8, 1.8 Hz, 1H), 4.88 (ddd, $J$ = 9.7, 2.9, 1.9 Hz, 1H), 4.31 (q, $J$ = 6.6 Hz, 1H), 4.24 (dd, $J$ = 10.4, 1.3 Hz, 1H), 3.93 (dd, $J$ = 11.0, 4.9 Hz, 1H), 3.67 (dd, $J$ = 10.2, 2.1 Hz, 1H), 3.56 (d, $J$ = 9.9 Hz, 1H), 3.40 (dd, $J$ = 12.2, 2.4 Hz, 1H), 2.88 (td, $J$ = 10.7, 3.4 Hz, 1H), 2.77 (p, $J$= 8.0 Hz, 1H), 2.71 - 2.64 (m, 2H), 2.12 - 0.68 (m, 65H) ppm.

$^{13}C$ NMR (176 MHz, $CDCl_3$) δ 217.6, 184.9, 177.8, 128.4, 123.0, 106.3, 99.0, 88.4, 76.4, 75.7, 75.6, 74.6, 71.4, 69.8, 68.1, 67.1, 55.8, 51.2, 49.3, 46.3, 45.3, 40.6, 38.7, 36.8, 35.9, 32.4, 32.2, 31.5, 30.3, 28.7, 27.9, 27.9, 26.8, 26.2, 26.2, 24.1, 20.0, 17.5, 15.8, 15.5, 14.6, 13.1, 12.2, 12.1, 10.7, 6.6, 6.4 ppm.

Example FLC-00462-1

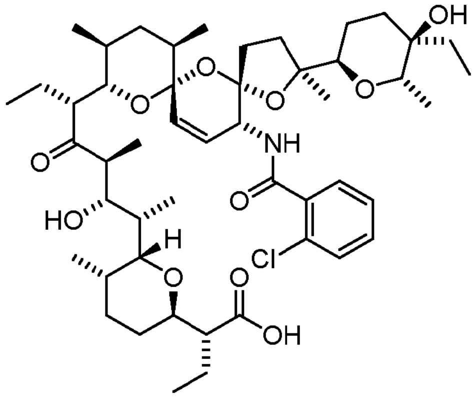

The compound was prepared according to the procedure described in example FLC-00445-1, except that 2-chlorobenzoyl chloride (2 eq) was added to the reaction mixture instead of 5-chloropentanoyl chloride. The yield of the obtained product was 74%.

ESI-MS for $C_{49}H_{74}ClNO_{11}$ *(m/z):* [M+Na]$^+$ 911.0, [M-H]$^-$ 886.0.

$^1$H NMR (700 MHz, $CD_2Cl_2$) δ 7.54 (dd, J = 7.5, 1.7 Hz, 1H), 7.39 (dd, J = 8.0, 1.1 Hz, 1H), 7.35 - 7.28 (m, 3H), 6.23 (dd, J = 10.8, 3.2 Hz, 1H), 5.87 (dd, J = 10.8, 1.8 Hz, 1H), 5.07 (ddd, J = 9.6, 2.9, 1.9 Hz, 1H), 4.20 - 4.11 (m, 2H), 3.87 - 3.77 (m, 1H), 3.67 - 3.62 (m, 2H), 3.49 (dd, J = 12.2, 2.6 Hz, 1H), 2.76 - 2.72 (m, 3H), 2.37 - 2.27 (m, 1H), 2.12 - 0.62 (m, 55H) ppm. $^{13}$C NMR (176 MHz, $CD_2Cl_2$) δ 218.6, 184.2, 167.5, 135.8, 130.9, 130.5, 129.8, 128.9, 128.2, 127.1, 123.5, 106.2, 99.5, 88.7, 76.5, 75.8, 75.6, 73.9, 71.1, 69.7, 68.0, 55.9, 50.2, 49.5, 47.7, 40.8, 38.7, 36.4, 35.8, 32.5, 32.3, 31.9, 28.0, 27.7, 27.5, 26.7, 23.3, 20.1, 19.9, 17.3, 15.6, 15.6, 14.3, 12.9, 12.2, 12.0, 10.5, 6.4, 6.2 ppm.

Example FLC-00463-1

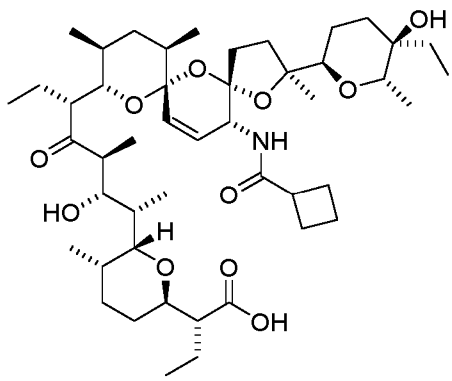

The compound was prepared according to the procedure described in example FLC-00445-1, except that cyclobutanecarbonyl chloride (2.2 eq) was added to the reaction mixture instead of 5-chloropentanoyl chloride. The yield of the obtained product was 78%.

ESI-MS for $C_{47}H_{77}NO_{11}$ *(m/z):* [M+Na]$^+$ 855.0, [M-H]$^-$ 831.0,

$^1$H NMR (700 MHz, $CDCl_3$) δ 6.96 (d, *J* = 9.6 Hz, 1H), 6.11 (dd, *J* = 10.9, 3.1 Hz, 1H), 5.70 (dd, *J* = 10.8, 1.8 Hz, 1H), 4.86 (ddd, *J* = 9.6, 2.9, 2.0 Hz, 1H), 4.30 (q, *J* = 6.7 Hz, 1H), 4.24 (dd, *J* = 10.4, 1.2 Hz, 1H), 3.94 (dd, *J* = 11.0, 4.8 Hz, 1H), 3.66 (dd, *J* = 10.2, 2.1 Hz, 1H), 3.55 (d, *J* = 10.0 Hz, 1H), 3.37 (dd, *J* = 12.2, 2.3 Hz, 1H), 3.24 (p, *J* = 8.3 Hz, 1H), 2.88 (td, *J* = 10.8, 3.3 Hz, 1H), 2.72 - 2.62 (m, 2H), 2.32 - 0.67 (m, 63H) ppm.

$^{13}$C NMR (176 MHz, $CDCl_3$) δ 217.6, 184.9, 175.8, 128.2, 123.1, 106.3, 98.9, 88.3, 76.5, 75.8, 75.7, 74.7, 71.4, 69.8, 68.2, 67.1, 55.7, 51.3, 49.2, 46.3, 40.6, 39.8, 38.7, 37.0, 35.9, 32.4, 32.2, 29.0, 27.9, 27.8, 26.7, 25.3, 25.0, 24.1, 20.0, 19.8, 18.4, 17.5, 15.9, 15.4, 14.6, 13.1, 12.2, 12.1, 10.7, 6.6, 6.5 ppm.

Example FLC-00464-1

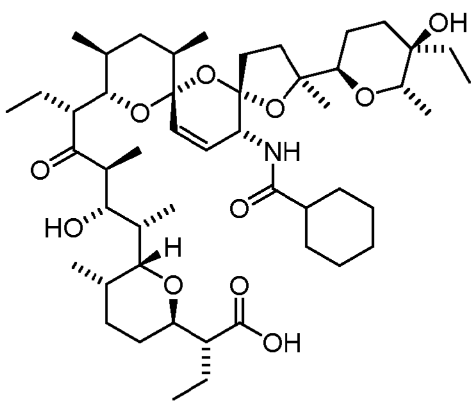

The compound was prepared according to the procedure described in example FLC-00445-1, except that cyclohexanecarbonyl chloride (2.2 eq) was added to the reaction mixture instead of 5-chloropentanoyl chloride. The yield of the obtained product was 79%.

ESI-MS for $C_{49}H_{81}NO_{11}$ (*m/z*): $[M+Na]^+$ 883.1, $[M-H]^-$ 859.1.

$^1H$ NMR (700 MHz, $CDCl_3$) δ 6.65 (d, *J* = 9.9 Hz, 1H), 6.13 (dd, *J* = 10.9, 3.1 Hz, 1H), 5.78 (dd, *J* = 10.8, 1.8 Hz, 1H), 4.88 (ddd, *J* = 9.9, 3.0, 1.9 Hz, 1H), 4.33 (t, *J* = 6.7 Hz, 1H), 4.23 (dd, *J* = 10.4, 1.0 Hz, 1H), 3.96 (dd, *J* = 11.0, 4.8 Hz, 1H), 3.69 - 3.67 (m, 1H), 3.57 (d, *J* = 10.0 Hz, 1H), 3.43 (dd, *J* = 12.2, 2.7 Hz, 1H), 2.91 - 2.86 (m, 1H), 2.71 - 2.64 (m, 2H), 2.41 (tt, *J* = 11.5, 3.3 Hz, 1H), 2.12 - 0.67 (m, 67H) ppm.

$^{13}C$ NMR (176 MHz, $CDCl_3$) δ 217.8, 184.8, 177.3, 128.8, 122.8, 106.2, 99.2, 88.5, 76.3, 75.6, 75.5, 74.2, 71.4, 69.8, 68.0, 67.1, 55.8, 50.9, 49.8, 46.1, 44.6, 40.7, 38.8, 36.4, 35.9, 32.4, 32.2, 29.8, 29.1, 28.0, 27.9, 27.8, 26.8, 25.8, 25.4, 25.3, 23.9, 20.0, 19.9, 17.5, 15.8, 15.6, 14.5, 13.2, 12.7, 12.0, 10.7, 6.6 ppm.

Example FLC-00469-1

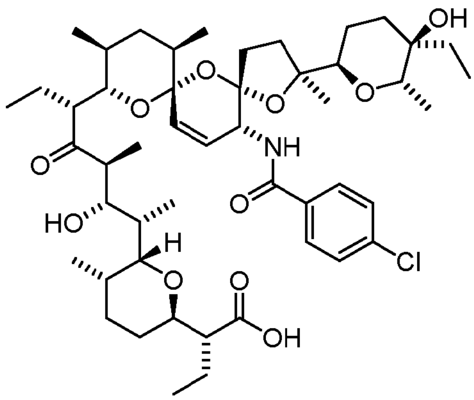

The compound was prepared according to the procedure described in example FLC-00445-1, except that 4-chlorobenzoyl chloride (2 eq) was added to the reaction mixture instead of 5-chloropentanoyl chloride. The yield of the obtained product was 85%.

ESI-MS for $C_{49}H_{74}ClNO_{11}$ *(m/z):* $[M+Na]^+$ 910.9, $[M-H]^-$ 887.0.

$^1H$ NMR (700 MHz, $CD_2Cl_2$) δ 8.16 - 8.04 (m, 2H), 7.46 (d, J = 9.4 Hz, 1H), 7.43 - 7.38 (m, 2H), 6.22 (dd, J= 10.8, 3.2 Hz, 1H), 5.81 (dd, J = 10.8, 1.8 Hz, 1H), 5.14 - 5.10 (m, 1H), 4.25 (q, J = 6.7 Hz, 1H), 4.18 (dd, J= 10.5, 1.7 Hz, 1H), 3.98 (dd, J = 11.0, 5.9 Hz, 1H), 3.71 - 3.69 (m, 1H), 3.67 - 3.64 (m, 1H), 3.49 (dd, J = 12.2, 2.6 Hz, 1H), 2.82 - 2.73 (m, 3H), 2.23 - 0.72 (m, 56H) ppm.

$^{13}C$ NMR (176 MHz, $CD_2Cl_2$) δ 219.3, 185.2, 166.4, 138.0, 132.3, 130.3, 129.2, 128.8, 123.8, 107.0, 100.1, 89.3, 77.3, 76.6, 76.4, 74.0, 71.6, 70.6, 69.0, 56.6, 50.6, 49.6, 48.5, 41.3, 39.3, 37.1, 36.2, 33.1, 32.7, 32.6, 28.7, 28.3, 27.4, 27.3, 23.6, 20.6, 20.4, 17.9, 16.2, 16.0, 14.7, 13.5, 12.8, 12.7, 11.3, 7.1, 7.0 ppm.

Example FLC-00470-1

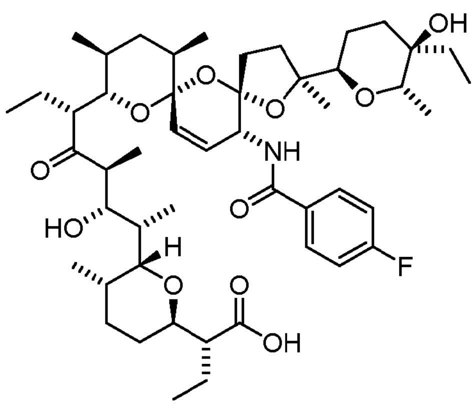

The compound was prepared according to the procedure described in example FLC-00445-1, except that 4-fluorobenzoyl chloride (2 eq) was added to the reaction mixture instead of 5-chloropentanoyl chloride. The yield of the obtained product was 72%.

ESI-MS for $C_{49}H_{74}FNO_{11}$ *(m/z):* $[M+Na]^+$ 895.0, $[M-H]^-$ 871.0.

$^1$H NMR (700 MHz, $CD_2Cl_2$) δ 8.23 - 8.12 (m, 2H), 7.37 (d, J = 9.5 Hz, 1H), 7.15 - 7.08 (m, 2H), 6.23 - 6.20 (m, 1H), 5.81 (dd, J = 10.8, 1.8 Hz, 1H), 5.13 (ddd, J = 9.5, 3.1, 1.8 Hz, 1H), 4.25 (q, J = 6.7 Hz, 1H), 4.18 (dd, J = 10.5, 1.7 Hz, 1H), 3.98 (dd, J = 11.0, 5.9 Hz, 1H), 3.72 - 3.63 (m, 2H), 3.49 (dd, J = 12.2, 2.6 Hz, 1H), 2.80 - 2.73 (m, 3H), 2.25 - 0.73 (m, 56H) ppm. $^{13}$C NMR (176 MHz, $CD_2Cl_2$) δ 218.7, 184.6, 165.7, 165.5, 164.1, 130.7, 130.7, 129.4, 129.4, 128.2, 123.2, 115.3, 115.2, 106.5, 99.5, 88.7, 76.8, 76.0, 75.8, 73.5, 71.0, 70.0, 68.4, 56.0, 50.1, 49.1, 47.8, 40.7, 38.7, 36.5, 35.7, 32.5, 32.1, 32.1, 28.1, 27.7, 26.8, 26.7, 23.1, 20.0, 19.8, 17.4, 15.6, 15.4, 14.2, 12.9, 12.2, 12.1, 10.7, 6.5, 6.4 ppm.

Example FLC-00472-1

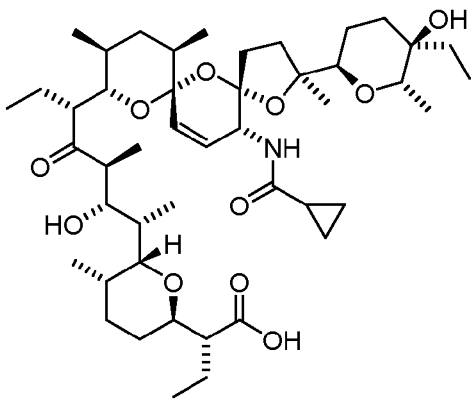

The compound was prepared according to the procedure described in example FLC-00445-1, except that cyclopropanecarbonyl chloride (2.2 eq) was added to the reaction mixture instead of 5-chloropentanoyl chloride. The yield of the obtained product was 68%.

ESI-MS for $C_{46}H_{75}NO_{11}$ *(m/z):* $[M+Na]^+$ 841.2, $[M-H]^-$ 817.0,

$^1$H NMR (700 MHz, $CDCl_3$) δ 6.13 (dd, *J* = 10.9, 3.1 Hz, 1H), 5.78 (dd, *J* = 10.8, 1.9 Hz, 1H), 4.88 (ddd, *J* = 9.8, 2.9, 2.0 Hz, 1H), 4.29 (q, *J* = 6.7 Hz, 1H), 4.23 (dd, *J* = 10.4, 1.4 Hz, 1H), 3.94 (dd, *J* = 11.0, 4.8 Hz, 1H), 3.70 - 3.67 (m, 1H), 3.60 (d, *J* = 10.1 Hz, 1H), 3.42 (dd, *J* = 12.2, 2.4 Hz, 1H), 2.88 (td, *J* = 10.9, 3.3 Hz, 1H), 2.73 - 2.64 (m, 2H), 2.24 - 0.60 (m, 63H) ppm.

$^{13}$C NMR (176 MHz, $CDCl_3$) δ 217.9, 184.8, 174.4, 128.3, 122.9, 106.4, 98.9, 88.5, 76.4, 75.8, 75.7, 74.4, 71.3, 69.9, 68.2, 67.1, 55.9, 51.0, 49.3, 46.5, 40.5, 38.7, 37.1, 35.8, 32.4, 32.4, 32.2, 28.9, 28.0, 27.5, 26.8, 23.8, 19.9, 19.9, 17.5, 15.9, 15.4, 14.6, 14.5, 13.1, 12.1, 12.1, 10.8, 7.2, 7.1, 6.7, 6.4 ppm.

Example FLC-00473-1

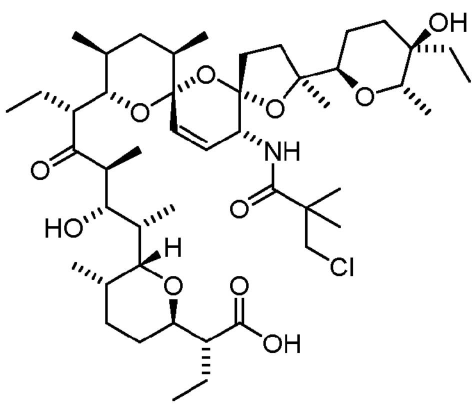

The compound was prepared according to the procedure described in example FLC-00445-1, except that 3-chloropivaloyl chloride (2.2 eq) was added to the reaction mixture instead of 5-chloropentanoyl chloride. The yield of the obtained product was 66%.

ESI-MS for $C_{47}H_{78}ClNO_{11}$ *(m/z):* $[M+Na]^+$ 891.2, $[M-H]^-$ 867.0.

$^1H$ NMR (700 MHz, $CD_2Cl_2$) δ 6.69 (d, *J* = 9.5 Hz, 1H), 6.18 (dd, *J* = 10.8, 3.2 Hz, 1H), 5.70 (dd, *J* = 10.8, 1.7 Hz, 1H), 4.88 (ddd, *J* = 9.5, 3.0, 1.8 Hz, 1H), 4.29 (q, *J* = 6.6 Hz, 1H), 4.21 - 4.19 (m, 1H), 3.89 (dd, *J* = 11.0, 4.8 Hz, 1H), 3.67 - 3.64 (m, 2H), 3.61 (d, *J* = 10.1 Hz, 1H), 3.47 (dd, *J* = 12.1, 2.3 Hz, 1H), 2.87 - 2.83 (m, 1H), 2.75 (ddd, *J* = 12.1, 7.6, 4.5 Hz, 2H), 2.17 - 0.69 (m, 64H) ppm.

$^{13}C$ NMR (176 MHz, $CD_2Cl_2$) δ 218.2, 184.3, 175.3, 128.5, 123.4, 106.3, 99.6, 88.5, 76.4, 76.0, 75.7, 74.1, 71.1, 70.0, 68.1, 67.1, 55.7, 50.3, 49.1, 47.6, 44.4, 40.8, 38.7, 35.9, 35.8, 32.5, 32.2, 32.1, 29.7, 28.7, 28.0, 27.5, 26.7, 23.6, 23.4, 23.1, 20.4, 20.0, 17.2, 15.5, 15.5, 14.4, 12.9, 12.5, 12.1, 10.6, 6.4, 6.2 ppm.

Example FLC-00486-1

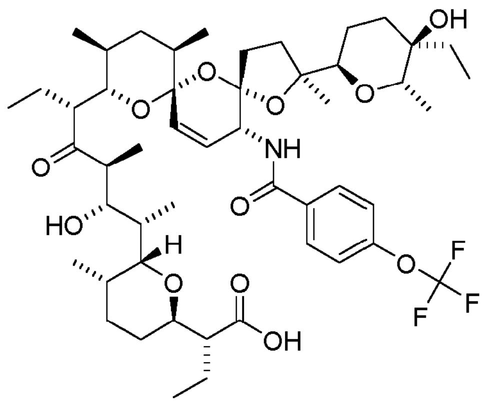

The compound was prepared according to the procedure described in example FLC-00445-1, except that 4-trifluoromethoxybenzoyl chloride (2 eq) was added to the reaction mixture instead of 5-chloropentanoyl chloride. The yield of the obtained product was 81%.

ESI-MS for $C_{50}H_{74}F_3NO_{12}$ (m/z): $[M+Na]^+$ 960.9, $[M-H]^-$ 937.0.

$^1H$ NMR (500 MHz, $CD_2Cl_2$) δ 8.19 (d, J = 8.8 Hz, 2H), 7.42 (d, J = 9.5 Hz, 1H), 7.24 (d, J = 8.1 Hz, 2H), 6.18 (dd, J = 10.8, 3.1 Hz, 1H), 5.75 (dd, J = 10.8, 1.6 Hz, 1H), 5.13 - 5.03 (m, 1H), 4.21 (q, J = 6.8 Hz, 1H), 4.14 (dd, J = 10.5, 1.1 Hz, 1H), 3.93 (dd, J = 11.0, 5.5 Hz, 1H), 3.65 (s, 1H), 3.60 (d, J = 10.2 Hz, 1H), 3.44 (dd, J = 12.2, 2.3 Hz, 1H), 2.78 - 2.67 (m, 3H), 2.17 - 0.69 (m, 56H) ppm.

$^{13}C$ NMR (126 MHz, $CD_2Cl_2$) δ 218.7, 184.7, 165.5, 151.4, 131.7, 130.3, 128.0, 123.3, 121.4, 120.5, 106.4, 99.4, 88.7, 76.8, 76.0, 75.6, 73.5, 70.9, 69.9, 68.5, 56.0, 49.9, 49.0, 47.7, 40.7, 38.7, 36.57, 35.7, 32.4, 32.1, 32.1, 28.0, 27.6, 26.9, 26.6, 23.0, 20.0, 19.7, 17.3, 15.6, 15.3, 14.2, 12.9, 12.1, 12.0, 10.6, 6.5, 6.3 ppm.

Example FLC-00487-1

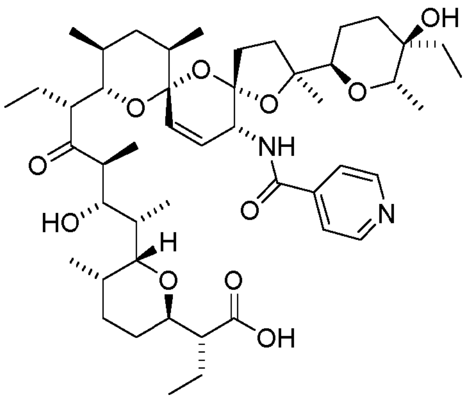

100 mg of C20-amino SAL (1 eq) was mixed with isonicotinic acid (1.2 eq), EDCI (1.2 eq), TEA (3 eq) and DMAP (0.1 eq) in DCM. The reaction was carried out at RT (TLC and LC-MS control). The post-reaction mixture was suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was 24%.

ESI-MS for $C_{48}H_{74}N_2O_{11}$ (*m/z*): $[M+Na]^+$ 878.0, $[M-H]^-$ 854.0.

$^1H$ NMR (500 MHz, $CD_2Cl_2$) δ 8.66 (s, 2H), 7.96 (d, J = 3.7 Hz, 2H), 7.65 (d, J = 8.7 Hz, 1H), 6.19 (dd, J = 10.8, 2.9 Hz, 1H), 5.76 (d, J = 10.7 Hz, 1H), 5.09 (d, J = 9.1 Hz, 1H), 4.24 - 4.07 (m, 2H), 3.93 (dd, J = 10.5, 5.4 Hz, 1H), 3.69 - 3.53 (m, 2H), 3.44 (d, J = 10.5 Hz, 1H), 2.73 (ddd, J = 26.1, 15.0, 8.7 Hz, 3H), 2.16 - 0.67 (m, 56H) ppm.

$^{13}C$ NMR (126 MHz, $CD_2Cl_2$) δ 218.7, 184.7, 165.3, 150.5, 140.0, 127.6, 123.5, 121.7, 106.3, 99.4, 88.8, 76.8, 76.1, 75.6, 73.5, 71.0, 70.1, 68.5, 56.0, 49.9, 49.0, 47.9, 40.7, 38.6, 36.6, 35.6, 32.4, 32.1, 32.0, 29.7, 28.1, 27.7, 26.8, 26.6, 23.1, 20.0, 19.8, 17.3, 15.6, 15.3, 14.2, 12.9, 12.2, 12.1, 10.7, 6.5, 6.4 ppm.

Example FLC-00493-1

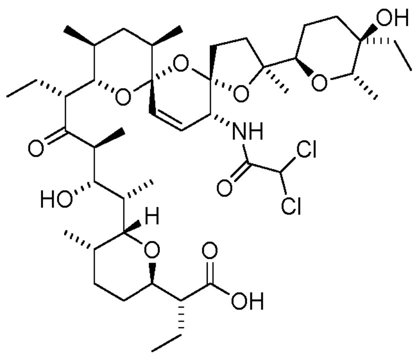

The compound was prepared according to the procedure described in example FLC-00487-1, except that 2,2-dichloroacetic acid (1.2 eq) was added to the reaction mixture instead of isonicotinic acid. The yield of the obtained product was 64%.

ESI-MS for $C_{44}H_{71}Cl_2NO_{11}$ *(m/z):* $[M+Na]^+$ 882.8, $[M-H]^-$ 858.9.

$^1H$ NMR (500 MHz, $CD_2Cl_2$) δ 7.83 (d, J = 9.2 Hz, 1H), 6.36 (s, 1H), 6.16 (dd, J = 10.8, 2.9 Hz, 1H), 5.64 (d, J = 10.8 Hz, 1H), 4.78 (d, J = 9.2 Hz, 1H), 4.25 - 4.20 (m, 1H), 4.13 (d, J = 10.4 Hz, 1H), 3.83 (dd, J = 10.5, 3.7 Hz, 1H), 3.61 - 3.55 (m, 2H), 3.44 (dd, J = 12.1, 2.1 Hz, 1H), 2.84 (td, J = 10.6, 3.0 Hz, 1H), 2.75 - 2.64 (m, 2H), 2.04 - 0.68 (m, 56H) ppm.

$^{13}C$ NMR (126 MHz, $CD_2Cl_2$) δ 218.6, 185.1, 164.6, 126.3, 124.1, 105.7, 98.9, 88.8, 76.3, 76.1, 75.7, 74.1, 71.1, 70.3, 68.4, 67.1, 55.9, 50.3, 48.8, 47.5, 40.5, 38.5, 37.3, 35.7, 32.4, 32.2, 32.0, 27.9, 27.7, 27.4, 26.5, 23.8, 20.1, 19.9, 17.2, 15.6, 15.2, 14.3, 12.8, 12.3, 12.1, 10.6, 6.3, 6.1 ppm.

Example FLC-00494-1

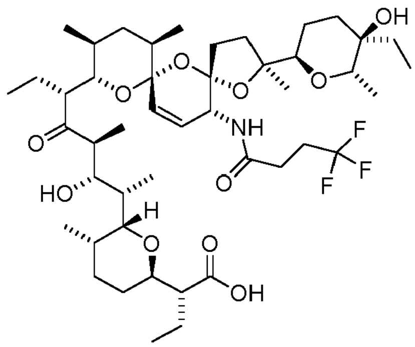

The compound was prepared according to the procedure described in example FLC-00487-1, except that 4,4,4-trifluorobutanoic acid (1.2 eq) was added to the reaction mixture instead of isonicotinic acid. The yield of the obtained product was 49%.

ESI-MS for $C_{46}H_{74}F_3NO_{11}$ *(m/z):* $[M+Na]^+$ 897.1, $[M-H]^-$ 873.0.

$^1H$ NMR (500 MHz, $CD_2Cl_2$) δ 7.21 (d, J = 9.3 Hz, 1H), 6.13 (dd, J = 10.8, 2.9 Hz, 1H), 5.62 (d, J= 10.8 Hz, 1H), 4.81 (d, J = 9.4 Hz, 1H), 4.22 (d, J = 6.8 Hz, 1H), 4.13 (d, J = 10.4 Hz, 1H), 3.82 (dd, J = 10.8, 4.0 Hz, 1H), 3.58 (dd, J = 15.3, 5.7 Hz, 2H), 3.42 (dd, J = 12.0, 2.0 Hz, 1H), 2.80 (d, J = 10.0 Hz, 2H), 2.75 - 2.66 (m, 2H), 2.45 - 2.37 (m, 3H), 2.03 - 0.67 (m, 56H) ppm.

$^{13}C$ NMR (126 MHz, $CD_2Cl_2$) δ 218.5, 184.8, 170.2, 128.3, 127.5, 123.6, 106.1, 98.8, 88.5, 76.5, 75.9, 75.6, 74.4, 71.2, 69.9, 68.1, 55.8, 50.7, 49.0, 46.4, 40.5, 38.5, 37.1, 35.8, 32.4, 32,3. 32.1, 29.1, 28.9, 28.3, 28.0, 27.9, 27.6, 26.6, 23.9, 19.8, 17.2, 15.6, 15.3, 14.4, 12.8, 12.3, 12.1, 10.5, 6.3, 6.1 ppm.

Example FLC-00501-1

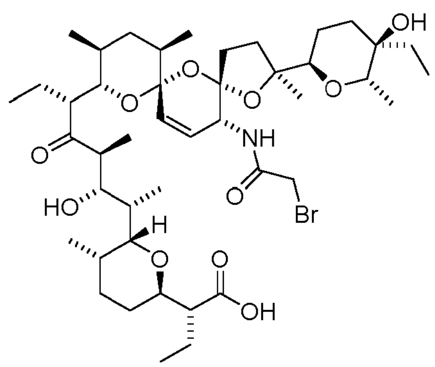

100 mg C20-amino SAL (1 eq) in MeCN with 0.5 M $Na_2CO_3$ (5 eq) was cooled in an ice/water bath and 2-bromoacetyl bromide (5 eq) diluted with MeCN was added dropwise. Afterwards, the reaction was conducted at RT (TLC and LC-MS control). The post-reaction mixture was diluted with $H_2O$ and extracted twice with DCM. The organic layers were combined, suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was 78%.

ESI-MS for $C_{44}H_{72}BrNO_{11}$ (*m/z*): $[M+Na]^+$ 892.8, $[M-H]^-$ 868.7.

$^1H$ NMR (500 MHz, $CD_2Cl_2$) δ 7.57 (d, J = 9.4 Hz, 1H), 6.13 (dd, J = 10.8, 3.0 Hz, 1H), 5.62 (dd, J = 10.8, 1.7 Hz, 1H), 4.83 - 4.74 (m, 1H), 4.38 (s, 1H), 4.23 (q, J = 6.6 Hz, 1H), 4.11 (d, J = 10.4 Hz, 1H), 4.02 (d, J = 11.7 Hz, 1H), 3.87 - 3.79 (m, 2H), 3.64 - 3.55 (m, 2H), 3.42 (dd, J = 12.1, 2.2 Hz, 1H), 2.82 (td, J = 10.6, 3.4 Hz, 1H), 2.74 - 2.65 (m, 2H), 2.09 - 0.67 (m, 55H) ppm.

$^{13}C$ NMR (126 MHz, $CD_2Cl_2$) δ 218.6, 184.8, 166.6, 126.9, 123.7, 105.9, 98.8, 88.6, 76.2, 76.0, 75.7, 74.2, 71.1, 70.1, 68.2, 55.8, 50.5, 48.9, 47.1, 40.5, 38.5, 37.4, 35.8, 32.4, 32.3, 32,1. 30,0. 28,4. 27,9. 27,4. 26,6, 23.8, 20.0, 19.9, 17.2, 15.7, 15.3, 14.4, 12.8, 12.4, 12.1, 10.6, 6.4, 6.1 ppm.

Example FLC-00528-1

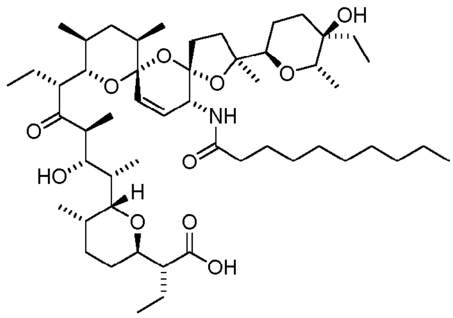

The compound was prepared according to the procedure described in example FLC-00445-1, except that decanoyl chloride (3 eq) was added to the reaction mixture instead of 5-chloropentanoyl chloride. The yield of the obtained product was 57%.

ESI-MS for $C_{52}H_{89}NO_{11}$ (*m/z*): $[M+Na]^+$ 927.

$^1H$ NMR (500 MHz, $CD_2Cl_2$) δ 6.90 (d, *J* = 9.7 Hz, 1H), 6.11 (dd, *J* = 10.8, 3.1 Hz, 1H), 5.60 (dd, *J* = 10.8, 1.8 Hz, 1H), 4.79 (ddd, *J* = 9.7, 2.9, 2.0 Hz, 1H), 4.53 (d, *J* = 3.6 Hz, 1H), 4.22 (dd, *J* = 13.6, 6.8 Hz, 1H), 4.12 (d, *J* = 10.0 Hz, 1H), 3.81 (dd, *J* = 11.0, 4.6 Hz, 1H), 3.59 (dd, *J* = 14.6, 6.3 Hz, 2H), 3.41 (dd, *J* = 12.1, 2.3 Hz, 1H), 2.84 - 2.75 (m, 1H), 2.73 - 2.64 (m, 2H), 2.32 (ddd, *J* = 15.3, 9.4, 6.0 Hz, 1H), 2.20 - 0.62 (m, 74H) ppm.

$^{13}C$ NMR (126 MHz, $CD_2Cl_2$) δ δ 218.9, 184.9, 173.7, 128.4, 123.5, 106.6, 99.3, 88.7, 76.8, 76.2, 76.1, 74.7, 71.5, 70.1, 68.4, 56.2, 51.1, 49.4, 46.5, 40.9, 38.9, 37.2, 36.3, 36.2, 32.8, 32.7, 32.5, 32.3, 29.9, 29.9, 29.7, 29.6, 29.0, 28.3, 27.8, 27.0, 25.7, 24.2, 23.0, 20.3, 20.2, 17.6, 16.0, 15.7, 14.8, 14.2, 13.2, 13.0, 12.4, 10.8, 6.7, 6.6 ppm.

Example FLC-00534-1

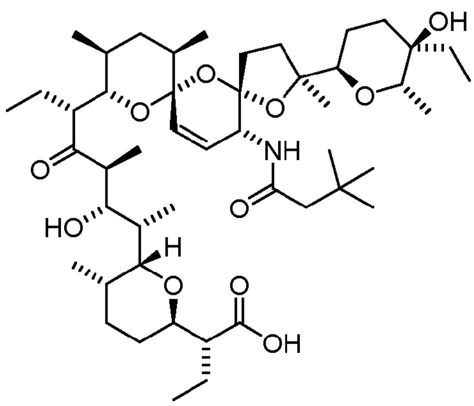

The compound was prepared according to the procedure described in example FLC-00445-1, except that 3,3-dimethylbutanoic acid (1.5 eq) was added to the reaction mixture instead of 5-chloropentanoyl chloride. The yield of the obtained product was 82%.

ESI-MS for $C_{48}H_{81}NO_{11}$ (*m/z*): $[M+H]^+$ 848.9, $[M+Na]^+$ 870.9, $[M-H]^-$ 847.0.

$^1H$ NMR (500 MHz, $CD_2Cl_2$) δ 6.65 (d, *J* = 9.7 Hz, 1H), 6.10 (dd, *J* = 10.8, 3.1 Hz, 1H), 5.62 (dd, *J* = 10.8, 1.6 Hz, 1H), 4.96 (s, 1H), 4.86 - 4.75 (m, 1H), 4.62 (d, *J* = 4.3 Hz, 1H), 4.25 (d, *J* = 6.8 Hz, 1H), 4.13 (dd, *J* = 10.4, 3.3 Hz, 1H), 3.81 (dd, *J* = 11.0, 4.7 Hz, 1H), 3.67 - 3.51 (m, 2H), 3.42 (dd, J= 12.2, 2.3 Hz, 1H), 2.80 (td, *J* = 10.6, 3.5 Hz, 1H), 2.70 (dd, *J* = 10.1, 7.6 Hz, 2H), 2.29 - 0.46 (m, 65H) ppm.

$^{13}C$ NMR (126 MHz, $CD_2Cl_2$) δ 218.4, 184.5, 172.1, 128.4, 123.1, 106.2, 99.1, 88.4, 76.3, 75.7, 75.7, 74.3, 71.1, 69.9, 67.9, 55.8, 50.7, 49.3, 48.2, 46.0, 40.6, 38.6, 36.6, 35.9, 32.4, 32.3, 32.2, 30.4, 29.4, 29.3, 28.4, 27.9, 27.9, 27.7, 26.7, 24.0, 19.9, 19.9, 17.2, 15.6, 15.4, 14.4, 13.1, 12.8, 12.0, 10.4, 6.3, 6.2 ppm.

Example FLC-00550-1

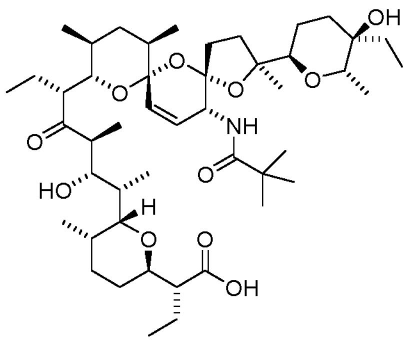

The compound was prepared according to the procedure described in example FLC-00445-1, except that pivaloyl chloride (1.5 eq) was added to the reaction mixture instead of 5-chloropentanoyl chloride. The yield of the obtained product was 50%.

ESI-MS for $C_{47}H_{79}NO_{11}$ (*m/z*): $[M+H]^+$ 834.9, $[M+Na]^+$ 856.8, $[M-H]^-$ 832.9.

$^1$H NMR (500 MHz, $CD_2Cl_2$) δ 6.48 (d, J = 9.3 Hz, 1H), 6.13 (dd, J = 10.7, 2.9 Hz, 1H), 5.65 (d, J = 10.7 Hz, 1H), 4.79 (d, J = 9.3 Hz, 2H), 4.30 - 4.20 (m, 1H), 4.15 (d, J = 10.4 Hz, 1H), 3.83 (dd, J= 10.9, 4.2 Hz, 1H), 3.59 (dd, J = 25.5, 10.1 Hz, 2H), 3.41 (d, J = 11.1 Hz, 1H), 2.85 - 2.74 (m, 1H), 2.68 (dd, J = 12.3, 6.1 Hz, 2H), 2.10 - 0.61 (m, 64H) ppm.

$^{13}$C NMR (126 MHz, $CD_2Cl_2$) δ 218.1, 184.3, 178.8, 128.7, 123.3, 106.5, 99.6, 88.3, 76.3, 75.8, 75.7, 74.2, 71.1, 69.8, 67.8, 55.6, 50.4, 49.2, 47.2, 40.8, 38.7, 38.6, 35.8, 32.4, 32.2, 32.1, 28.9, 27.9, 27.5, 27.4, 26.7, 23.6, 20.2, 19.9, 17.2, 15.5, 15.4, 14.4, 12.9, 12.6, 12.0, 10.5, 6.4, 6.2 ppm.

Example FLC-00569-1

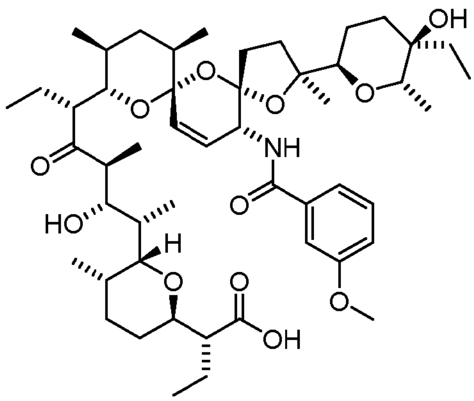

The compound was prepared according to the procedure described in example FLC-00445-1, except that 3-methoxybenzoyl chloride (1.5 eq) was added to the reaction mixture instead of 5-chloropentanoyl chloride. The yield of the obtained product was 72%.

ESI-MS for $C_{50}H_{77}NO_{12}$ (m/z): $[M+Na]^+$ 906.8, $[M-H]^-$ 882.8.

$^1$H NMR (500 MHz, $CDCl_3$) δ 7.82 (d, *J* = 7.7 Hz, 1H), 7.59 - 7.47 (m, 1H), 7.27 (dt, *J* = 9.6, 7.4 Hz, 2H), 6.98 (dd, *J* = 8.2, 2.5 Hz, 1H), 6.17 (dd, *J* = 10.8, 3.1 Hz, 1H), 5.86 (dd, *J* = 10.8, 1.5 Hz, 1H), 5.14 (dd, *J* = 7.1, 1.8 Hz, 2H), 4.72 (s, 1H), 4.24 (dd, *J* = 13.5, 8.5 Hz, 2H), 3.99 (dd, *J* = 11.0, 5.5 Hz, 1H), 3.82 (s, 3H), 3.68 - 3.55 (m, 2H), 3.39 (dd, *J* = 12.1, 2.2 Hz, 1H), 2.78 (td, *J* = 10.8, 3.2 Hz, 1H), 2.74 - 2.60 (m, 2H), 2.23 - 0.58 (m, 54H) ppm.

$^{13}$C NMR (126 MHz, $CDCl_3$) δ 217.7, 184.8, 167.3, 159.5, 134.2, 129,9. 128,4. 123.2, 120.4, 118.4, 112.3, 106.5, 99.5, 88.7, 75.7, 75.5, 73.8, 71.2, 69.8, 68.2, 55.8, 55.4, 50.4, 49.5, 47.6, 40.8, 38.8, 36.2, 35.8, 32.4, 32.3, 32.2, 28.0, 27.9, 27.5, 26.8, 23.4, 19.9, 19.8, 17.6, 15.8, 15.6, 14.4, 13.2, 12.2, 12.1, 10.8, 6.7 ppm.

Example FLC-00573-1

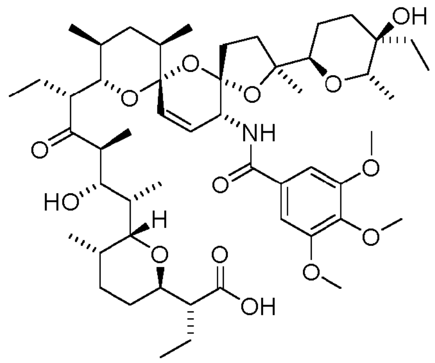

The compound was prepared according to the procedure described in example FLC-00445-1, except that 3.4.5-trimethoxybenzoyl chloride(1.5 eq) was added to the reaction mixture instead of 5-chloropentanoyl chloride. The yield of the obtained product was 72%.

ESI-MS for $C_{52}H_{81}NO_{14}$ (*m/z*): $[M+H]^+$ 944.8, $[M+Na]^+$ 966.8, $[M-H]^-$ 942.9.

$^1H$ NMR (500 MHz, $CDCl_3$) δ 7.69 (d, J = 9.3 Hz, 1H), 7.19 (s, 2H), 6.14 (dd, J = 10.8, 3.1 Hz, 1H), 5.86 (dd, J = 10.7, 1.4 Hz, 1H), 5.09 (d, J = 9.0 Hz, 1H), 4.24 (dd, J = 22.7, 8.6 Hz, 2H), 3.94 (dd, J = 11.0, 5.2 Hz, 1H), 3.91 (s, 6H), 3.84 (s, 3H), 3.72 - 3.67 (m, 1H), 3.60 (d, J = 10.1 Hz, 1H), 3.42 (dd, J = 12.1, 2.2 Hz, 1H), 2.81 - 2.61 (m, 3H), 2.31 - 0.61 (m, 56H) ppm,

$^{13}C$ NMR (126 MHz, $CDCl_3$) δ 218.0, 183.9, 167.7, 153.1, 141.0, 129.5, 129.2, 122.7, 106.7, 105.2, 99.8, 88.3, 76.4, 76.0, 75.8, 73.5, 71.2, 70.4, 68.1, 60.8, 56.7, 55.6, 50.9, 49.5, 49.0, 40.9, 38.7, 35.8, 35.8, 32.4, 32.3, 31.9, 28.3, 28.1, 26.9, 26.7, 23.3, 19.8, 17.8, 15.8, 15.7, 14.3, 13.3, 12.4, 12.2, 10.9, 6.8, 6.5 ppm.

Example FLC-00578-1

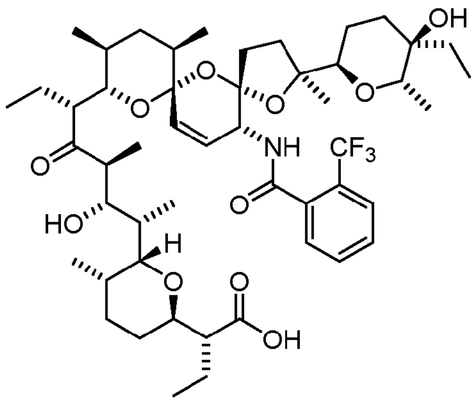

The compound was prepared according to the procedure described in example FLC-00445-1, except that 2-trifluoromethylbenzoyl chloride (1.5 eq) was added to the reaction mixture instead of 5-chloropentanoyl chloride. The yield of the obtained product was 75%.

ESI-MS for $C_{50}H_{74}F_3NO_{11}$ (*m/z*): $[M+Na]^+$ 944.7, $[M-H]^-$ 920.8.

$^1H$ NMR (500 MHz, $CDCl_3$) δ 7.78 (d, *J* = 7.5 Hz, 1H), 7.64 (d, *J* = 7.7 Hz, 1H), 7.45 (ddd, *J* = 25.6, 16.4, 8.4 Hz, 3H), 6.16 (dd, *J* = 10.8, 3.1 Hz, 1H), 5.85 (dd, *J* = 10.8, 1.1 Hz, 1H), 5.08 (d, *J* = 9.3 Hz, 1H), 4.65 (s, 2H), 4.18 (dd, *J* = 12.1, 8.9 Hz, 2H), 3.88 (dd, *J* = 11.0, 4.8 Hz, 1H), 3.65 - 3.52 (m, 2H), 3.42 (dd, *J* = 12.0, 2.0 Hz, 1H), 2.69 (ddd, *J* = 28.0, 15.0, 5.2 Hz, 3H), 2.29 - 0.35 (m, 55H) ppm.

$^{13}C$ NMR (126 MHz, $CDCl_3$) δ 217.8, 184.7, 168.7, 134.9, 132.3, 129.6, 129.1, 128.2, 126.5, 123.4, 106.2, 99.4, 88.8, 76.4, 75.6, 75.3, 73.9, 71.2, 69.8, 68.0, 55.8, 50.2, 49.7, 47.8, 40.8, 38.8, 36.4, 35.8, 32.4, 32.3, 32.0, 27.9, 27.7, 26.8, 23.3, 20.0, 19.8, 17.5, 15.8, 15.7, 14.5, 13.2, 12.0, 10.7, 6.6, 6.5 ppm.

Example FLC-00579-1

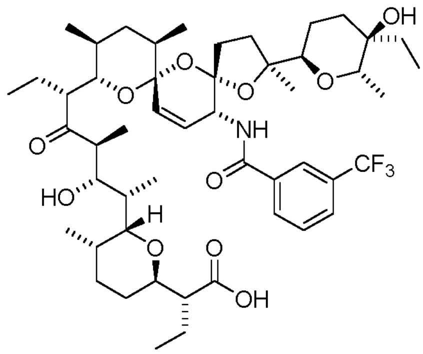

The compound was prepared according to the procedure described in example FLC-00445-1, except that 3-trifluoromethylbenzoyl chloride (1.5 eq) was added to the reaction mixture instead of 5-chloropentanoyl chloride. The yield of the obtained product was 70%.

ESI-MS for $C_{50}H_{74}F_3NO_{11}$ (*m/z*): $[M+Na]^+$ 944.8, $[M-H]^-$ 920.8.

$^1H$ NMR (500 MHz, $CDCl_3$) δ 8.46 (d, *J* = 7.8 Hz, 1H), 8.28 (s, 1H), 7.67 (d, *J* = 7.6 Hz, 1H), 7.52 (dd, *J* = 15.9, 8.6 Hz, 2H), 6.18 (dd, *J* = 10.8, 3.0 Hz, 1H), 5.84 (dd, *J*= 10.8, 1.5 Hz, 1H), 5.16 (d, *J* = 9.1 Hz, 1H), 4.25 (dd, *J* = 18.3, 8.8 Hz, 2H), 4.01 (dd, *J* = 11.0, 5.7 Hz, 1H), 3.64 - 3.55 (m, 2H), 3.40 (dd, *J* = 12.1, 2.0 Hz, 1H), 2.79 (t, *J* = 8.6 Hz, 1H), 2.72 - 2.64 (m, 2H), 2.21 - 0.62 (m, 56H) ppm.

$^{13}C$ NMR (126 MHz, $CDCl_3$) δ 217.8, 185.0, 166.2, 133.8, 131.2, 129.5, 127.9, 127.8, 125.7, 123.4, 106.3, 99.5, 88.7, 76.6, 75.9, 75.3, 73.8, 71.2, 70.0, 68.4, 55.8, 50.1, 49.3, 47.6, 40.7, 38.8, 36.4, 35.7, 32.4, 32.2, 32.2, 28.0, 27.6, 26.7, 23.3, 19.9, 19.8, 17.6, 15.8, 15.5, 14.4, 13.2, 12.2, 12.1, 10.8, 6.7 ppm.

Example FLC-00373-1

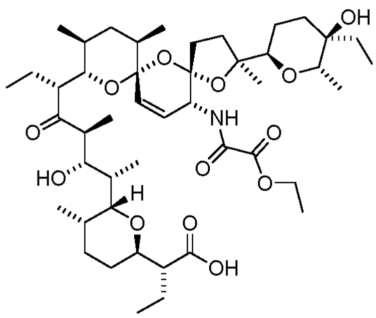

100 mg C20-amino-SAL (1 eq) was diluted in DCM, DIPEA (1 eq) and ethyl chlorooxoacetate (1 eq) were added. Reactions were carried out at 0 °C for 30 minutes (TLC and LC-MS control). The post-reaction mixture was then suspended on silica gel and purified using a chromatograph with an ELS detector using a column packed with silica gel. The respective fractions were combined and concentrated, and the residue was dissolved in DCM and washed twice with 0.06 M $H_2SO_4$. The yield of the obtained product was 40%.

ESI-MS for $C_{46}H_{75}NO_{13}$ (m/z): $[M+Na]^+$ 873, $[M-H]^-$ 849.

$^1H$ NMR (500 MHz, $CDCl_3$) δ 8.09 (d, *J* = 7.7 Hz, 1H), 6.17 (d, *J* = 10.3 Hz, 1H), 5.81 (d, *J* = 10.6 Hz, 1H), 4.52 (d, *J* = 7.4 Hz, 1H), 4.31 (dd, *J* = 13.9, 6.8 Hz, 2H), 4.16 (d, *J* = 9.7 Hz, 1H), 4.04 - 3.96 (m, 1H), 3.90 (t, *J* = 13.4 Hz, 2H), 3.63 (d, *J* = 9.4 Hz, 1H), 2.88 (d, *J* = 3.5 Hz, 1H), 2.76 (s, 1H), 2.61 (d, *J* = 10.3 Hz, 1H), 2.27 - 0.62 (m, 60H) ppm.

$^{13}C$ NMR (126 MHz, $CDCl_3$) δ 215.6, 177.6, 160.5, 157.5, 128.0, 123.6, 104.9, 99.6, 89.2, 75.8, 74.8, 72.8, 71.8, 71.1, 68.6, 62.9, 56.2, 49.8, 48.8, 41.1, 38.6, 36.7, 36.4, 32.7, 30.5, 30.2, 29.1, 28.0, 26.3, 25.9, 22.7, 22.3, 20.0, 17.9, 16.6, 15.8, 14.4, 14.0, 13.3, 13.0, 11.9, 11.1, 6.8, 6.4 ppm.

Example FLC-00376-1

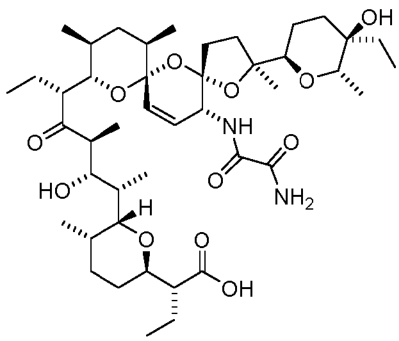

100 mg FLC-00373-1 (1 eq) was mixed with $NH_3$/MeOH (excess) in DCM. The reaction was carried out at RT (TLC and LC-MS control). The post-reaction mixture was then suspended on silica gel and purified using a chromatograph with an ELS detector using a column packed with silica gel. The respective fractions were combined and concentrated, and the residue was dissolved in DCM and washed twice with 0.5 M $Na_2CO_3$. The yield of the obtained product was 55%.
ESI-MS for $C_{44}H_{72}N_2O_{12}$ (m/z): $[M+Na]^+$ 843.8, $[M-H]^-$ 819.8.
$^1H$ NMR (500 MHz, $CD_2Cl_2$) δ 8.27 (d, *J* = 10.0 Hz, 1H), 7.40 (s, 1H), 6.86 - 6.68 (m, 1H), 6.18 (dd, *J* = 10.8, 3.0 Hz, 1H), 5.73 (dd, *J* = 10.8, 1.3 Hz, 1H), 5.46 - 5.32 (m, 1H), 4.64 (d, *J* = 9.9 Hz, 1H), 4.14 (d, *J* = 6.9 Hz, 1H), 4.08 (d, *J* = 10.2 Hz, 1H), 3.90 (dd, *J* = 10.7, 4.8 Hz, 1H), 3.68 (t, *J* = 9.2 Hz, 2H), 3.46 (dd, *J* = 12.2, 1.8 Hz, 1H), 2.86 - 2.63 (m, 6H), 2.26 - 0.50 (m, 52H) ppm.
$^{13}C$ NMR (126 MHz, $CD_2Cl_2$) δ 219.0, 183.7, 161.3, 160.2, 127.5, 123.3, 106.0, 99.7, 88.6, 76.9, 76.4, 75.9, 73.2, 71.0, 70.3, 68.3, 55.9, 50.8, 49.2, 49.1, 40.7, 38.6, 36.7, 35.6, 32.5, 32.3, 31.8, 28.2, 26.8, 25.6, 22.8, 20.2, 19.9, 17.5, 15.6, 15.5, 14.0, 12.9, 12.2, 12.1, 10.9, 6.7, 6.4 ppm.

Example FLC-00392-1

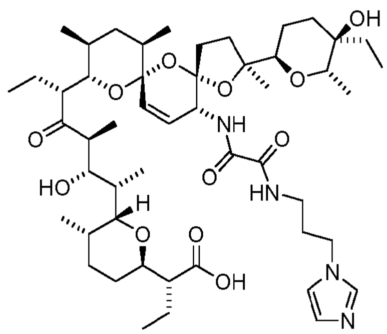

The compound was prepared according to the procedure described in example FLC-00376-1, except that 1-(3-aminopropyl)imidazole (excess) was added to the reaction mixture instead of $NH_3$/MeOH. The yield of the obtained product was 66%.
ESI-MS for $C_{50}H_{80}N_4O_{12}$ (*m/z*): $[M+H]^+$ 930.1, $[M-H]^-$ 928.1.
$^1H$ NMR (500 MHz, $CDCl_3$) δ 8.38 (d, *J* = 9.3 Hz, 1H), 7.74 (t, *J* = 6.2 Hz, 1H), 7.46 (s, 1H), 7.01 (s, 1H), 6.94 (s, 1H), 6.16 (dd, *J* = 10.8, 3.0 Hz, 1H), 5.84 (dd, *J* = 10.8, 1.5 Hz, 1H), 5.25 (d, *J* = 23.0 Hz, 1H), 4.56 - 4.48 (m, 1H), 4.28 (d, *J* = 6.8 Hz, 1H), 4.22 (d, *J* = 10.2 Hz, 1H), 3.99 - 3.90 (m, 3H), 3.70 (dd, *J* = 10.1, 1.6 Hz, 1H), 3.56 (d, *J* = 10.1 Hz, 1H), 3.40 - 3.33 (m, 1H), 3.26 (ddt, *J* = 20.2, 13.7, 6.8 Hz, 3H), 2.87 - 2.78 (m, 1H), 2.65 (dd, *J* = 10.2, 7.6 Hz, 2H), 2.14 - 0.58 (m, 56H) ppm,
**[0671]** $^{13}C$ NMR (126 MHz, $CDCl_3$) δ 217.3, 184.0, 161.0, 159.8, 137.1, 129.5, 127.2, 123.2, 119.0, 116.5, 106.1, 99.5, 88.7, 76.3, 75.9, 75.8, 74.0, 71.2, 70.1, 67.7, 55.4, 50.5, 49.6, 49.1, 44.4, 40.7, 38.6, 36.7, 36.6, 35.8, 32.4, 32.3, 32.2, 31.2, 29.5, 28.1, 27.1, 26.8, 23.2, 20.1, 19.9, 17.6, 15.7, 14.5, 13.2, 12.3, 12.1, 10.9, 6.9, 6.6 ppm.

Example FLC-00423-1

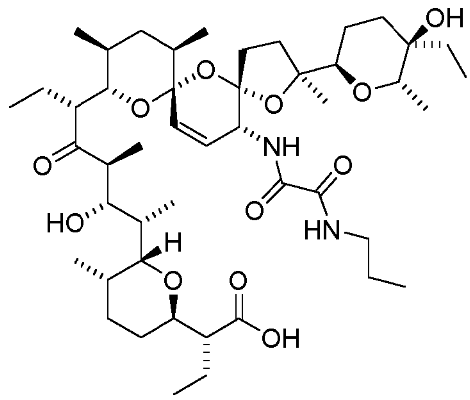

The compound was prepared according to the procedure described in example FLC-00376-1, except that propylamine (excess) was added to the reaction mixture instead of $NH_3$/MeOH. The yield of the obtained product was 85%.

ESI-MS for $C_{47}H_{78}N_2O_{12}$ (m/z): $[M+Na]^+$ 886, $[M-H]^-$ 862.

$^1H$ NMR (500 MHz, $CD_2Cl_2$) δ 8.15 (d, *J* = 9.9 Hz, 1H), 7.40 (s, 1H), 6.18 (dd, *J* = 10.8, 3.1 Hz, 1H), 5.74 (dd, *J* = 10.8, 1.6 Hz, 1H), 4.61 (ddd, *J* = 9.9, 2.8, 1.8 Hz, 1H), 4.18 - 3.98 (m, 2H), 3.91 (dd, *J* = 10.5, 4.9 Hz, 1H), 3.75 - 3.63 (m, 2H), 3.47 (dd, *J* = 12.4, 2.3 Hz, 1H), 3.22 (dt, *J* = 13.6, 7.0 Hz, 2H), 2.76 (dd, *J* = 10.5, 7.2 Hz, 1H), 2.67 (dt, *J* = 10.7, 5.3 Hz, 2H), 2.26 - 0.60 (m, 61H) ppm.

$^{13}C$ NMR (126 MHz, $CD_2Cl_2$) δ 218.9, 183.2, 160.6, 159.0, 127.7, 123.1, 106.0, 99.8, 88.6, 77.0, 76.7, 75.9, 72.9, 70.9, 70.1, 68.3, 55.9, 50.9, 49.6, 49.2, 41.2, 40.7, 38.7, 36.7, 35.6, 32.5, 32.3, 31.9, 28.3, 28.0, 26.9, 25.2, 22.8, 22.6, 20.2, 19.9, 17.5, 15.5, 15.4, 13.9, 12.9, 12.2, 12.1, 11.1, 10.9, 6.7, 6.4 ppm.

Example FLC-00430-1

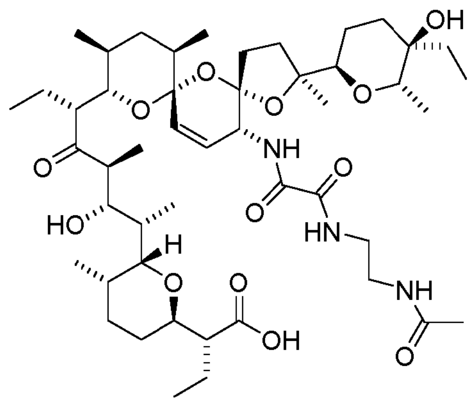

The compound was prepared according to the procedure described in example FLC-00376-1, except that *N*-acetyl ethylene diamine (excess) was added to the reaction mixture instead of $NH_3$/MeOH. The yield of the obtained product was 69%.

ESI-MS for $C_{48}H_{79}N_3O_{13}$ (m/z): $[M+Na]^+$ 929.0, [M-H]-905.1.

$^1H$ NMR (500 MHz, $CD_2Cl_2$) δ 8.20 (d, *J* = 9.7 Hz, 1H), 8.07 (t, *J* = 5.3 Hz, 1H), 7.14 (s, 1H), 6.18 (dd, *J* = 10.8, 3.0 Hz, 1H), 5.73 (dd, *J* = 10.8, 1.4 Hz, 1H), 5.49 - 5.31 (m, 1H), 4.60 (d, *J* = 9.4 Hz, 1H), 4.23 - 4.02 (m, 2H), 3.90 (dd, *J* = 10.4, 4.7 Hz, 1H), 3.69 (t, *J* = 9.7 Hz, 2H), 3.49 - 3.41 (m, 2H), 3.41 - 3.34 (m, 2H), 3.27 (dd, *J* = 7.9, 5.3 Hz, 1H), 2.81 - 2.64 (m, 4H), 2.28 - 0.48 (m, 57H) ppm.

$^{13}C$ NMR (126 MHz, $CD_2Cl_2$) δ 218.8, 183.4, 171.1, 160.3, 159.9, 127.5, 123.1, 106.0, 99.7, 88.6, 76.8, 76.6, 76.0, 73.1, 71.2, 70.3, 68.3, 55.8, 51.0, 49.5, 49.1, 40.8, 40.7, 39.3, 38.6, 36.8, 35.5, 32.5, 32.3, 31.8, 28.4, 28.3, 26.8, 25.4,

22.8, 22.7, 20.4, 19.9, 17.5, 15.5, 15.4, 14.0, 12.9, 12.4, 12.1, 11.1, 6.8, 6.4 ppm.

Example FLC-00438-1

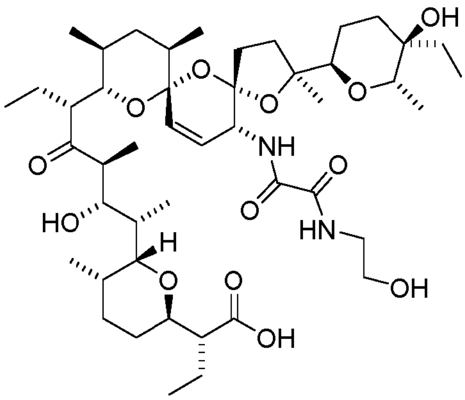

The compound was prepared according to the procedure described in example FLC-00376-1, except that etanolamine (excess) was added to the reaction mixture instead of $NH_3$/MeOH. The yield of the obtained product was 81%.

ESI-MS for $C_{46}H_{76}N_2O_{13}$ (m/z): $[M+Na]^+$ 887.9, $[M-H]^-$ 863.9.

$^1H$ NMR (500 MHz, $CDCl_3$) δ 8.44 (d, $J$ = 8.8 Hz, 1H), 7.77 (s, 1H), 6.12 (dd, $J$ = 10.8, 2.9 Hz, 1H), 5.83 (d, $J$ = 10.9 Hz, 1H), 5.14 - 4.83 (m, 1H), 4.38 (d, $J$ = 8.4 Hz, 1H), 4.31 (d, $J$ = 6.8 Hz, 1H), 4.22 (d, $J$ = 10.1 Hz, 1H), 3.91 (dd, $J$ = 10.7, 4.0 Hz, 1H), 3.72 (dd, $J$ = 10.6, 5.8 Hz, 1H), 3.71 - 3.64 (m, 1H), 3.57 (dd, $J$ = 11.3, 5.8 Hz, 1H), 3.49 (d, $J$ = 10.1 Hz, 1H), 3.38 - 3.27 (m, 3H), 3.03 - 2.92 (m, 1H), 2.79 (s, 1H), 2.66 - 2.54 (m, 2H), 2.19 - 0.45 (m, 55H) ppm.

$^{13}C$ NMR (126 MHz, $CDCl_3$) δ 215.0, 182.2, 159.2, 158.1, 125.2, 121.0, 104.2, 97.4, 86.6, 73.8, 73.8, 72.4, 68.8, 65.6, 58.8, 53.2, 47.8, 47.2, 41.1, 38.8, 36.6, 34.8, 33.6, 30.5, 30.4, 30.1, 28.1, 27.8, 26.5, 25.5, 24.9, 21.0, 18.1, 15.6, 14.0, 13.8, 12.7, 11.3, 10.3, 10.2, 5.2, 4.5 ppm.

Example FLC-00441-1

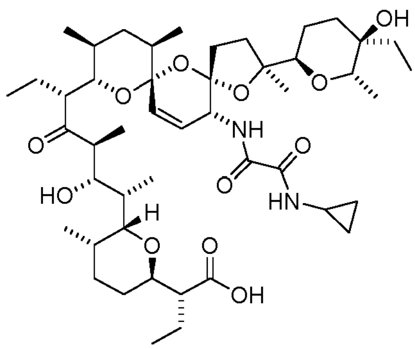

The compound was prepared according to the procedure described in example FLC-00376-1, except that cyclopropylamine (excess) was added to the reaction mixture instead of $NH_3$/MeOH. The yield of the obtained product was 38%.

ESI-MS for $C_{47}H_{76}N_2O_{12}$ (m/z): $[M+Na]^+$ 884.0.

$^1H$ NMR (500 MHz, $CDCl_3$) δ 8.19 (d, $J$ = 9.1 Hz, 1H), 7.76 (d, $J$ = 3.6 Hz, 1H), 6.16 (dd, $J$= 10.8, 3.0 Hz, 1H), 5.90 (dd, $J$ = 10.8, 1.1 Hz, 1H), 5.87 - 5.73 (m, 1H), 5.52 (s, 1H), 4.40 (d, $J$ = 9.0 Hz, 1H), 4.30 (d, $J$ = 6.8 Hz, 1H), 4.24 (d, $J$ = 10.2 Hz, 1H), 3.96 (dd, $J$ = 10.9, 4.7 Hz, 1H), 3.73 (dd, $J$ = 10.1, 1.2 Hz, 1H), 3.58 (d, $J$ = 10.1 Hz, 1H), 3.37 (d, $J$ = 10.6 Hz, 1H), 2.81 (td, $J$ = 10.6, 3.2 Hz, 1H), 2.73 (dt, $J$ = 11.1, 3.7 Hz, 1H), 2.69 - 2.59 (m, 2H), 2.05 (dd, $J$ = 11.6, 4.9 Hz, 2H), 2.00 - 0.44 (m, 56H) ppm.

$^{13}C$ NMR (126 MHz, $CDCl_3$) δ 216.9, 183.5, 161.2, 160.8, 127.6, 122.8, 106.2, 99.7, 88.7, 76.2, 76.1, 75.7, 73.8,

71.2, 70.2, 67.5, 55.2, 50.7, 49.9, 49.6, 40.8, 38.7, 36.4, 35.9, 32.4, 32.3, 32.2, 29.3, 28.1, 27.0, 26.9, 23.3, 22.6, 20.1, 19.9, 17.6, 15.9, 15.7, 14.4, 13.3, 12.4, 12.1, 10.9, 6.9, 6.7, 6.2, 6.1 ppm.

Example FLC-00442-1

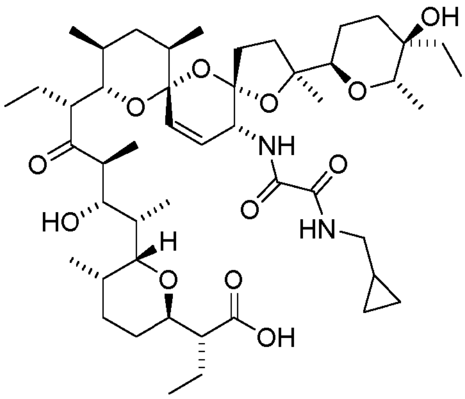

The compound was prepared according to the procedure described in example FLC-00376-1, except that cyclopropylmethylamine (excess) was added to the reaction mixture instead of $NH_3$/MeOH. The yield of the obtained product was 76%.

ESI-MS for $C_{48}H_{78}N_2O_{12}$ (m/z): $[M+Na]^+$ 898.1, $[M-H]^-$ 874.0.

$^1H$ NMR (500 MHz, $CDCl_3$) δ 8.29 (d, *J* = 9.6 Hz, 1H), 7.51 (t, *J* = 5.7 Hz, 1H), 6.17 (dd, *J*= 10.8, 3.0 Hz, 1H), 5.84 (dd, *J* = 10.8, 1.3 Hz, 1H), 5.70 - 5.37 (m, 1H), 4.57 (d, *J* = 9.1 Hz, 1H), 4.23 (dd, *J* = 16.8, 8.8 Hz, 2H), 3.99 (dd, *J* = 10.8, 4.9 Hz, 1H), 3.73 (dd, *J* = 10.1, 1.4 Hz, 1H), 3.63 (d, *J* = 10.1 Hz, 1H), 3.45 - 3.34 (m, 1H), 3.20 - 3.02 (m, 2H), 2.79 (td, *J* = 10.6, 3.4 Hz, 1H), 2.73 - 2.56 (m, 2H), 2.08 (dd, *J* = 11.4, 5.3 Hz, 2H), 2.03 - 0.11 (m, 58H) ppm.

$^{13}C$ NMR (126 MHz, $CDCl_3$) δ 217.6, 183.7, 161.2, 158.9, 127.7, 123.0, 106.1, 99.7, 88.6, 76.4, 76.2, 75.7, 73.7, 71.2, 70.0, 67.8, 55.5, 50.7, 49.7, 49.3, 44.4, 40.8, 38.7, 36.5, 35.8, 32.4, 32.3, 32,1. 29.1, 28.1, 26.9, 26.6, 23.2, 20.1, 19.9, 17.7, 15.8, 15.7, 14.4, 13.3, 12.3, 12.1, 11.0, 10.6, 6.9, 6.7, 3.6, 3.5 ppm.

Example FLC-00443-1

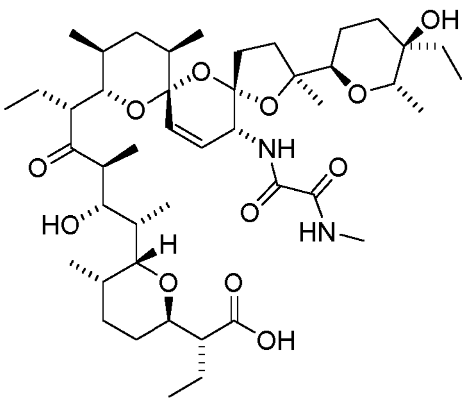

The compound was prepared according to the procedure described in example FLC-00376-1, except that methylamine (excess) was added to the reaction mixture instead of $NH_3$/MeOH. The yield of the obtained product was 38%.

ESI-MS for $C_{45}H_{74}N_2O_{12}$ (m/z): $[M+Na]^+$ 858.0, $[M-H]^-$ 834.1.

$^1H$ NMR (500 MHz, $CDCl_3$) δ 8.07 (d, *J*= 9.1 Hz, 1H), 7.75 (d, *J* = 4.9 Hz, 1H), 6.15 (dd, *J*= 10.8, 3.0 Hz, 1H), 5.86 (dd, *J* = 10.8, 1.2 Hz, 1H), 5.52 - 5.19 (m, 1H), 4.51 (d, *J* = 8.9 Hz, 1H), 4.28 (q, *J* = 6.7 Hz, 1H), 4.22 (d, *J* = 10.2 Hz, 1H), 3.98 (dd, *J* = 10.9, 4.9 Hz, 1H), 3.71 (dd, *J* = 10.1, 1.3 Hz, 1H), 3.38 (d, *J* = 10.5 Hz, 1H), 2.83 - 2.76 (m, 4H), 2.71 - 2.61 (m, 2H),

2.11 - 0.57 (m, 56H) ppm.

$^{13}C$ NMR (126 MHz, $CDCl_3$) δ 217.3, 183.8, 161.4, 160.3, 127.6, 122.8, 106.2, 99.6, 88.7, 76.3, 76.0, 75.7, 73.8, 71.2, 70.1, 67.7, 55.4, 50.5, 49.8, 49.3, 40.8, 38.7, 36.5, 35.8, 32.4, 32.3, 32.1, 28.9, 28.1, 27.0, 26.9, 26.2, 23.2, 20.1, 19.9, 17.6, 15.8, 15.8, 14.4, 13.3, 12.1, 10.9, 6.9, 6.7 ppm.

Example FLC-00452-1

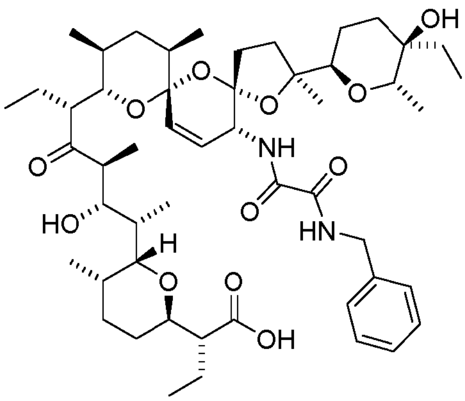

The compound was prepared according to the procedure described in example FLC-00376-1, except that benzylamine (excess) was added to the reaction mixture instead of $NH_3$/MeOH. The yield of the obtained product was 51%.

ESI-MS for $C_{51}H_{78}N_2O_{12}$ (m/z): $[M+Na]^+$ 934.1, $[M-H]^-$ 910.1.

$^1H$ NMR (700 MHz, $CD_2Cl_2$) δ 8.26 - 8.21 (m, 1H), 7.76 - 7.71 (m, 1H), 7.36 (s, 2H), 7.32 (d, *J*= 7.2 Hz, 2H), 6.25 - 6.21 (m, 1H), 5.82 - 5.78 (m, 1H), 5.75 - 5.49 (m, 1H), 4.73 - 4.65 (m, 1H), 4.58 - 4.41 (m, 2H), 4.24 - 4.16 (m, 1H), 4.16 - 4.10 (m, 1H), 3.99 - 3.93 (m, 1H), 3.81 - 3.70 (m, 2H), 3.57 - 3.46 (m, 1H), 2.85 - 2.78 (m, 1H), 2.78 - 2.67 (m, 2H), 2.25 - 0.69 (m, 56H) ppm,

$^{13}C$ NMR (176 MHz, $CD_2Cl_2$) δ 220.8, 185.2, 162.4, 160.9, 139.6, 130.5, 129.7, 129.5, 129.4, 125.1, 107.9, 101.7, 90.6, 78.9, 78.6, 77.8, 75.0, 72.9, 72.0, 70.2, 57.8, 52.7, 51.5, 51.1, 45.3, 42.6, 40.6, 38.7, 37.5, 34.4, 34.2, 33.8, 30.2, 30.0, 28.8, 27.3, 24.8, 22.1, 21.8, 19.4, 17.5, 17.3, 15.8, 14.8, 14.2, 14.0, 12.8, 8.6, 8.3 ppm.

Example FLC-00456-1

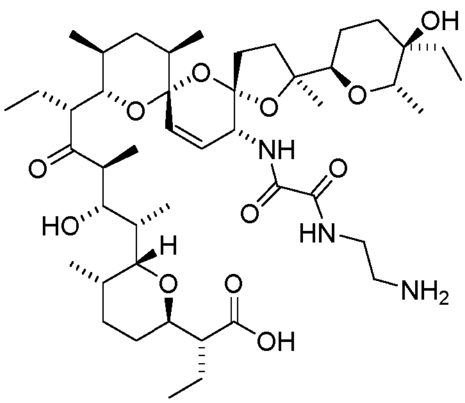

The compound was prepared according to the procedure described in example FLC-00376-1, except that ethylenediamine (excess) was added to the reaction mixture instead of $NH_3$/MeOH. The yield of the obtained product was 7%.

ESI-MS for $C_{46}H_{77}N_3O_{12}$ (m/z): $[M+H]^+$ 865.1, $[M-H]^-$ 863.0.

$^1H$ NMR (700 MHz, $CDCl_3$) δ 8.45 - 8.36 (m, 1H), 7.80 - 7.71 (m, 1H), 6.18 (dd, *J* = 10.8, 3.1 Hz, 1H), 5.86 (dd, *J* =

10.8, 1.6 Hz, 1H), 4.62 - 4.55 (m, 1H), 4.29 (d, $J$ = 6.8 Hz, 1H), 4.24 (d, $J$ = 10.2 Hz, 1H), 4.04 - 3.95 (m, 1H), 3.74 (dd, $J$ = 10.2, 2.1 Hz, 1H), 3.63 (d, $J$ = 10.1 Hz, 1H), 3.40 (dd, $J$ = 12.2, 2.2 Hz, 1H), 3.34 (dd, $J$ = 5.9, 3.1 Hz, 2H), 2.83 (ddd, $J$ = 13.7, 11.0, 6.6 Hz, 3H), 2.72 - 2.63 (m, 2H), 2.21 - 0.54 (m, 58H) ppm.

$^{13}C$ NMR (176 MHz, $CDCl_3$) δ 217.5, 183.8, 161.1, 159.7, 127.5, 123.1, 106.1, 99.6, 88.6, 76.4, 76.1, 75.7, 73.8, 71.2, 70.2, 67.8, 55.5, 50.6, 49.7, 49.2, 42.6, 41.4, 40.7, 38.7, 36.6, 35.8, 32.4, 32.3, 32.1, 29.3, 28.1, 26.9, 26.8, 23.1, 20.2, 19.9, 17.6, 15.8, 15.7, 14.4, 13.2, 12.3, 12.1, 11.0, 6.9, 6.6 ppm.

Example FLC-00457-1

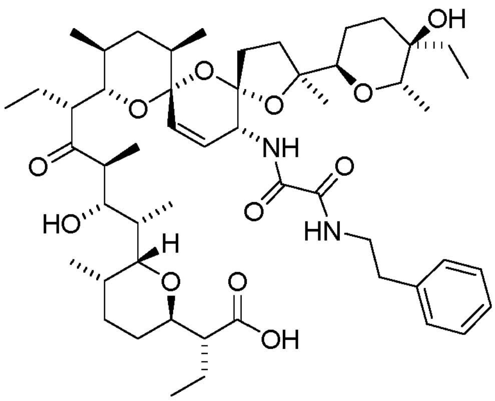

The compound was prepared according to the procedure described in example FLC-00376-1, except that 2-phenylethylamine (excess) was added to the reaction mixture instead of $NH_3$/MeOH. The yield of the obtained product was 29%.

ESI-MS for $C_{52}H_{80}N_2O_12$ ($m/z$): $[M+Na]^+$ 948.0, $[M-H]^-$ 924.1.

$^1H$ NMR (700 MHz, $CDCl_3$) δ 8.39 - 8.33 (m, 1H), 7.53 (s, 1H), 7.30 (dd, $J$ = 12.1, 4.5 Hz, 2H), 7.23 - 7.18 (m, 3H), 6.20 (dd, $J$ = 10.8, 3.1 Hz, 1H), 5.87 (dd, $J$ = 10.8, 1.7 Hz, 1H), 4.64 - 4.52 (m, 1H), 4.31 - 4.27 (m, 1H), 4.27 - 4.23 (m, 1H), 4.05 - 4.00 (m, 1H), 3.78 - 3.74 (m, 1H), 3.66 (d, $J$ = 10.1 Hz, 1H), 3.56 (s, 1H), 3.50 (s, 1H), 3.42 (d, $J$ = 10.1 Hz, 1H), 2.83 (dd, $J$ = 9.1, 5.5 Hz, 3H), 2.74 - 2.65 (m, 2H), 2.22 - 0.61 (m, 56H) ppm.

$^{13}C$ NMR (176 MHz, $CDCl_3$) δ 217.7, 183.8, 161.1, 159.1, 138.7, 128.7, 128.6, 127.6, 126.5, 123.1, 106.1, 99.6, 88.6, 76.5, 76.2, 75.7, 73.7, 71.2, 70.1, 67.9, 55.6, 50.7, 49.7, 49.2, 40.9, 40.8, 38.7, 36.6, 35.8, 35.6, 32.4, 32.3, 32.1, 29.1, 28.1, 26.9, 26.6, 23.2, 20.2, 19.9, 17.7, 15.7, 14.4, 13.2, 12.4, 12.1, 11.0, 6.9, 6.7 ppm.

Example FLC-00458-1

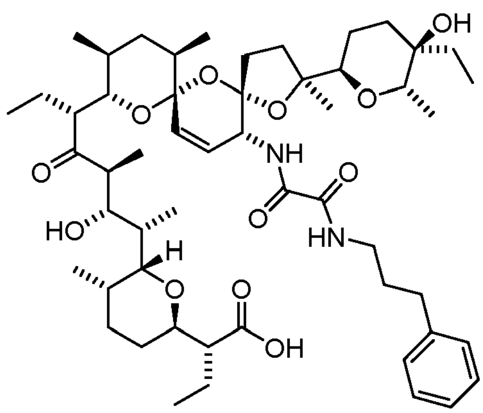

The compound was prepared according to the procedure described in example FLC-00376-1, except that 3-phenylpropylamine (excess) was added to the reaction mixture instead of $NH_3$/MeOH. The yield of the obtained product was 15%.

ESI-MS for $C_{53}H_{82}N_2O_{12}$ (*m/z*): $[M+Na]^+$ 962.1.

$^1H$ NMR (700 MHz, $CDCl_3$) δ 8.35 - 8.28 (m, 1H), 7.55 - 7.49 (m, 1H), 7.28 (t, *J* = 3.6 Hz, 2H), 7.19 (dd, *J* = 6.2, 5.1 Hz, 3H), 6.23 - 6.17 (m, 1H), 5.89 (dd, *J* = 10.8, 1.7 Hz, 1H), 5.70 - 5.35 (m, 1H), 4.66 - 4.53 (m, 1H), 4.32 - 4.23 (m, 2H), 4.07 - 3.99 (m, 1H), 3.79 - 3.75 (m, 1H), 3.69 - 3.63 (m, 1H), 3.45 - 3.40 (m, 1H), 3.35 - 3.29 (m, 2H), 2.87 - 2.80 (m, 1H), 2.73 - 2.67 (m, 2H), 2.64 (t, *J* = 7.8 Hz, 2H), 2.17 - 0.62 (m, 57H) ppm.

$^{13}C$ NMR (176 MHz, $CDCl_3$) δ 217.6, 183.8, 161.2, 159.2, 141.3, 128.4, 128.4, 127.6, 126.0, 123.1, 106.2, 99.7, 88.7, 76.9, 76.5, 76.2, 75.7, 73.7, 71.2, 70.0, 67.9, 55.5, 50.7, 49.7, 49.3, 40.8, 39.2, 38.7, 36.6, 35.8, 33.1, 32.4, 32.3, 32.1, 30.9, 29.1, 28.1, 26.9, 26.7, 23.2, 20.2, 19.9, 17.7, 15.8, 14.4, 13.2, 12.4, 12.1, 11.0, 6.9, 6.7 ppm.

Example FLC-00465-1

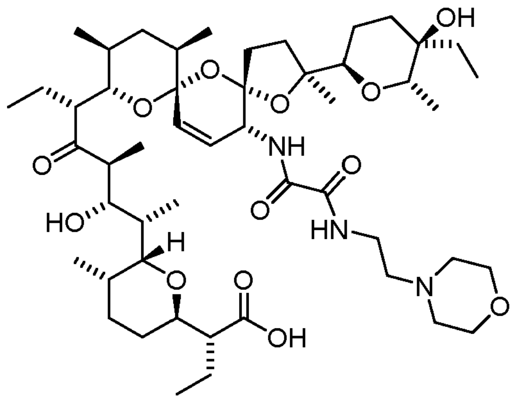

The compound was prepared according to the procedure described in example FLC-00376-1, except that 4-(2-aminethyl)morpholine (excess) was added to the reaction mixture instead of $NH_3$/MeOH. The yield of the obtained product was 38%.

ESI-MS for $C_{50}H_{83}N_3O_{13}$ (m/z): $[M+H]^+$ 934.9, $[M-H]^-$ 933.0.

$^1H$ NMR (700 MHz, $CDCl_3$) δ 8.57 - 8.51 (m, 1H), 7.73 - 7.68 (m, 1H), 6.21 - 6.17 (m, 1H), 5.86 - 5.81 (m, 1H), 4.68 - 4.63 (m, 1H), 4.30 - 4.20 (m, 2H), 4.03 - 3.98 (m, 1H), 3.76 - 3.63 (m, 8H), 3.42 (s, 4H), 2.84 - 2.77 (m, 1H), 2.72 - 2.64 (m, 2H), 2.51 - 2.42 (m, 7H), 2.19 - 0.63 (m, 52H) ppm.

$^{13}C$ NMR (176 MHz, $CDCl_3$) δ 217.9, 183.9, 160.9, 159.1, 127.8, 123.2, 106.1, 99.7, 88.6, 76.6, 76.2, 75.7, 73.7, 71.2, 70.3, 68.0, 66.9, 56.8, 55.6, 53.4, 50.9, 49.6, 49.2, 40.8, 38.7, 36.4, 36.0, 35.8, 32.4, 32.4, 32.0, 31.6, 29.0, 28.1, 26.8, 26.5, 23.1, 20.2, 17.7, 15.7, 14.3, 14.1, 13.2, 12.4, 12.1, 11.0, 6.8, 6.6 ppm.

Example FLC-00471-1

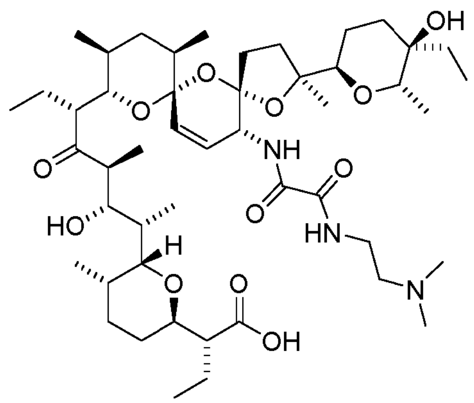

The compound was prepared according to the procedure described in example FLC-00376-1, except that *N*,*N*-dimethylaminoethylamine (excess) was added to the reaction mixture instead of $NH_3$/MeOH. The yield of the obtained

product was 94%.

ESI-MS for $C_{48}H_{81}N_3O_{12}$ (*m/z*): $[M+H]^+$ 893.0, $[M+Na]^+$ 914.9, $[M-H]^-$ 891.0.

$^1H$ NMR (700 MHz, $CDCl_3$) δ 8.48 - 8.41 (m, 1H), 7.71 - 7.64 (m, 1H), 6.22 - 6.17 (m, 1H), 5.86 - 5.83 (m, 1H), 5.64 - 5.42 (m, 1H), 4.69 - 4.63 (m, 1H), 4.28 - 4.22 (m, 2H), 4.05 - 3.99 (m, 1H), 3.77 - 3.74 (m, 1H), 3.68 - 3.65 (m, 1H), 3.45 - 3.42 (m, 1H), 3.40 - 3.31 (m, 2H), 2.83 - 2.79 (m, 1H), 2.72 - 2.66 (m, 2H), 2.43 (s, 2H), 2.23 (s, 6H), 2.16 - 0.62 (m, 55H) ppm. $^{13}C$ NMR (176 MHz, $CDCl_3$) δ 217.9, 183.8, 161.0, 159.1, 127.8, 123.1, 106.1, 99.7, 88.6, 76.6, 76.3, 75.7, 73.7, 71.2, 70.2, 68.0, 57.6, 55.6, 50.8, 49.7, 49.2, 45.2, 40.8, 38.7, 37.1, 36.5, 35.8, 32.4, 32.0, 31.6, 29.0, 28.1, 26.9, 26.4, 23.1, 22.6, 20.2, 19.9, 17.7, 15.7, 14.3, 14.1, 13.2, 12.4, 12.1, 11.0, 6.8, 6.6 ppm.

Example FLC-00477-1

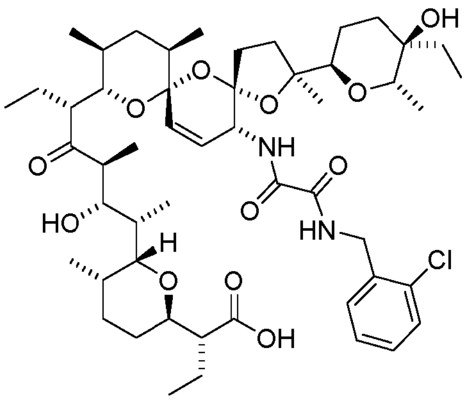

The compound was prepared according to the procedure described in example FLC-00376-1, except that 2-chlorobenzylamine (excess) was added to the reaction mixture instead of $NH_3$/MeOH. The yield of the obtained product was 43%.

ESI-MS for $C_{51}H_{77}ClN_2O_{12}$ (m/z): $[M+Na]^+$ 967.9, $[M-H]^-$ 943.9.

$^1H$ NMR (500 MHz, $CDCl_3$) δ 8.50 (d, *J* = 9.6 Hz, 1H), 7.83 (t, *J* = 6.3 Hz, 1H), 7.35 - 7.30 (m, 2H), 7.21 - 7.16 (m, 2H), 6.17 (dd, *J* = 10.8, 3.0 Hz, 1H), 5.84 (dd, *J* = 10.8, 1.5 Hz, 1H), 5.59 - 5.30 (m, 1H), 4.60 (dd, *J* = 7.1, 4.5 Hz, 1H), 4.55 (dd, *J* = 6.3, 2.5 Hz, 2H), 4.24 (dd, *J* = 16.3, 8.9 Hz, 2H), 3.99 (dd, *J* = 10.7, 4.8 Hz, 1H), 3.73 (dd, *J* = 10.1, 1.7 Hz, 1H), 3.62 (d, *J* = 10.1 Hz, 1H), 3.39 (dd, *J* = 12.1, 1.7 Hz, 1H), 2.79 (d, *J* = 3.3 Hz, 1H), 2.73 - 2.61 (m, 2H), 2.18 - 0.61 (m, 55H) ppm.

$^{13}C$ NMR (126 MHz, $CDCl_3$) δ 217.7, 183.9, 160.8, 159.1, 134.9, 133.6, 130.2, 129.5, 129.0, 127.6, 127.1, 123.2, 106.2, 99.6, 88.6, 76.5, 76.2, 75.7, 73.7, 71.2, 70.2, 67.9, 55.5, 50.8, 49.7, 49.2, 41.4, 40.8, 38.7, 36.5, 35.8, 32.4, 32.3, 32.0, 29.0, 28.1, 26.9, 26.7, 23.2, 20.1, 19.9, 17.7, 15.8, 14.4, 13.2, 12.4, 12.1, 11.0, 6.9, 6.6 ppm.

Example FLC-00478-1

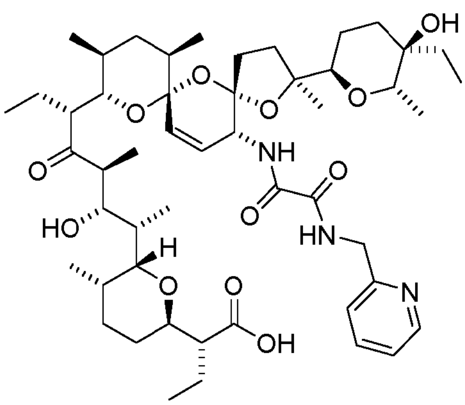

The compound was prepared according to the procedure described in example FLC-00376-1, except that

2-(aminomethyl)pyridine (excess) was added to the reaction mixture instead of $NH_3$/MeOH. The yield of the obtained product was 7%.

ESI-MS for $C_{50}H_{77}N_3O_{12}$ (m/z): $[M+Na]^+$ 934.9; $[M-H]^-$ 910.9.

$^1H$ NMR (500 MHz, $CDCl_3$) δ 8.54 (d, *J* = 3.9 Hz, 1H), 8.44 - 8.37 (m, 1H), 8.32 (s, 1H), 7.64 (s, 1H), 7.26 (s, 1H), 7.19 (s, 1H), 6.15 (s, 1H), 5.85 - 5.75 (m, 1H), 4.59 (d, *J* = 5.6 Hz, 2H), 4.22 - 4.14 (m, 1H), 3.98 (s, 1H), 2.97 - 2.56 (m, 3H), 2.16 - 0.59 (m, 61H) ppm.

$^{13}C$ NMR (126 MHz, $CD_2Cl_2$) δ 219.0, 183.3, 160.4, 159.1, 155.9, 149.1, 136.7, 127.6, 123.2, 122.4, 121.9, 106.0, 99.7, 88.6, 77.0, 76.6, 75.9, 73.0, 70.9, 70.1, 68.3, 55.9, 50.8, 49.5, 49.2, 44.4, 40.7, 38.6, 36.8, 35.6, 32.5, 32.3, 31.9, 28.2, 26.8, 25.3, 22.8, 20.2, 19.9, 17.5, 15.6, 15.4, 14.0, 13.9, 12.9, 12.2, 12.1, 10.9, 6.7, 6.4 ppm.

Example FLC-00479-1

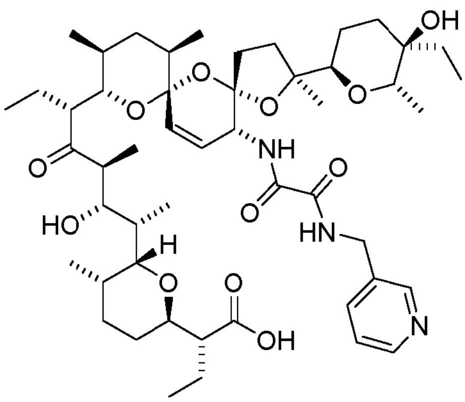

The compound was prepared according to the procedure described in example FLC-00376-1, except that 3-(aminomethyl)pyridine (excess) was added to the reaction mixture instead of $NH_3$/MeOH. The yield of the obtained product was 57%.

ESI-MS for $C_{50}H_{77}N_3O_{12}$ (m/z): $[M+Na]^+$ 934.9, $[M-H]^-$ 911.0.

$^1H$ NMR (500 MHz, $CDCl_3$) δ 8.51 (s, 3H), 8.04 - 7.79 (m, 1H), 7.63 (d, *J* = 7.8 Hz, 1H), 7.23 (dd, *J* = 7.4, 5.0 Hz, 1H), 6.21 - 6.11 (m, 1H), 5.91 - 5.75 (m, 1H), 4.63 - 4.51 (m, 1H), 4.46 (d, *J* = 6.3 Hz, 2H), 4.34 - 4.15 (m, 2H), 3.98 (dd, *J* = 10.6, 4.7 Hz, 1H), 3.76 - 3.57 (m, 2H), 3.45 - 3.29 (m, 1H), 2.90 - 2.78 (m, 1H), 2.65 (d, *J* = 10.2 Hz, 1H), 2.28 - 0.50 (m, 57H) ppm.

Example FLC-00489-1

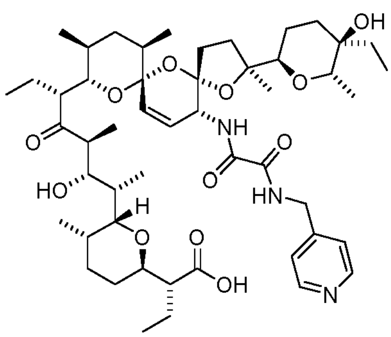

The compound was prepared according to the procedure described in example FLC-00376-1, except that 4-(aminomethyl)pyridine (excess) was added to the reaction mixture instead of $NH_3$/MeOH. The yield of the obtained product was 57%.

ESI-MS for $C_{50}H_{77}N_3O_{12}$ (m/z): $[M+Na]^+$ 934.9, $[M-H]^-$ 911.0.

$^1H$ NMR (500 MHz, $CDCl_3$) δ 8.54 (d, *J* = 5.0 Hz, 2H), 8.05 - 7.92 (m, 1H), 7.19 (d, *J* = 5.7 Hz, 2H), 6.16 (dd, *J* = 10.7, 2.5 Hz, 1H), 5.80 (d, *J* = 10.4 Hz, 1H), 4.58 - 4.40 (m, 3H), 4.16 (d, *J* = 6.8 Hz, 1H), 3.96 (dd, *J* = 10.8, 5.5 Hz, 1H), 3.90 -

3.77 (m, 1H), 3.69 - 3.59 (m, 1H), 2.85 (s, 1H), 2.80 - 2.68 (m, 1H), 2.64 (d, $J$ = 10.2 Hz, 1H), 2.23 - 0.51 (m, 59H) ppm.

Example FLC-00491-1

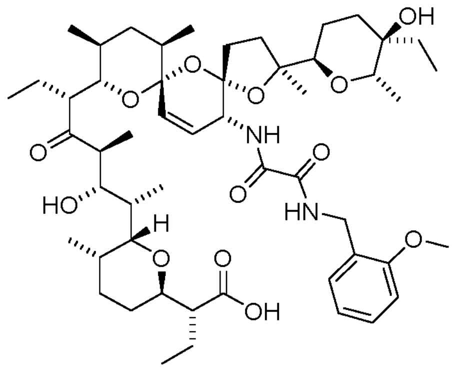

The compound was prepared according to the procedure described in example FLC-00376-1, except that 2-methoxyphenethylamine (excess) was added to the reaction mixture instead of $NH_3$/MeOH. The yield of the obtained product was 35%.

ESI-MS for $C_{53}H_{82}N_2O_{13}$ (m/z): $[M+Na]^+$ 978, $[M-H]^-$ 954.

$^1H$ NMR (500 MHz, $CD_2Cl_2$) δ 8.25 - 8.13 (m, 1H), 7.60 (s, 1H), 7.20 (td, $J$ = 8.1, 1.6 Hz, 1H), 7.12 (dd, $J$ = 7.6, 1.5 Hz, 1H), 6.90 - 6.85 (m, 2H), 6.17 (dd, $J$ = 10.7, 2.3 Hz, 1H), 5.73 (d, $J$ = 10.6 Hz, 1H), 4.61 (d, $J$ = 9.6 Hz, 1H), 4.10 (dd, $J$ = 26.0, 8.6 Hz, 2H), 3.95 - 3.87 (m, 1H), 3.83 (s, 3H), 3.70 (t, $J$ = 9.9 Hz, 2H), 3.52 - 3.43 (m, 3H), 2.84 (t, $J$ = 6.8 Hz, 2H), 2.80 - 2.63 (m, 4H), 2.27 - 0.54 (m, 55H) ppm.

$^{13}C$ NMR (126 MHz, $CD_2Cl_2$) δ 218.9, 217.7, 215.0, 183.3, 160.5, 159.0, 157.6, 130.5, 127.9, 127.1, 123.1, 120.5, 110.2, 106.0, 99.8, 88.5, 77.0, 76.7, 75.9, 72.9, 71.0, 70.2, 68.2, 55.8, 55.2, 50.9, 49.5, 49.3, 40.7, 40.0, 38.6, 36.6, 35.6, 32.5, 32.3, 31.8, 29.9, 28.2, 27.9, 26.8, 25.3, 22.8, 20.2, 19.8, 17.5, 15.5, 13.9, 12.9, 12.3, 12.1, 10.9, 6.7, 6.4 ppm.

Example FLC-00395-1

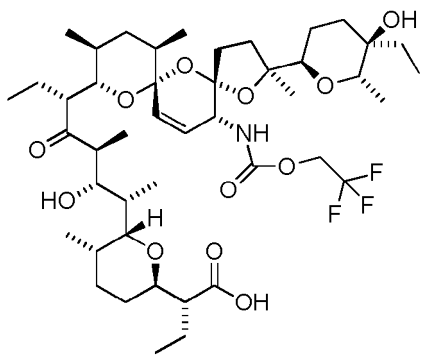

The compound was prepared according to the procedure described in example FLC-00445-1, except that 2,2,2-trifluoroethyl chloroformate (2.2 eq) was added to the reaction mixture instead of 5-chloropentanoyl chloride. The yield of the obtained product was 53%.

ESI-MS for $C_{45}H_{72}F_3NO_{12}$ (m/z): $[M+Na]^+$ 899.0, $[M-H]^-$ 875.1.

$^1H$ NMR (500 MHz, $CDCl_3$) δ 7.00 (d, $J$ = 9.5 Hz, 1H), 6.12 (dd, $J$ = 10.9, 3.0 Hz, 1H), 5.77 (dd, $J$ = 10.8, 1.2 Hz, 1H), 4.58 (dq, $J$ = 12.8, 8.6 Hz, 1H), 4.47 (t, $J$ = 14.1 Hz, 1H), 4.30-4.11 (m, 4H), 3.92 (dd, $J$ = 10.9, 4.3 Hz, 1H), 3.64 (d, $J$ = 10.1 Hz, 1H), 3.56 (dd, $J$ = 15.0, 7.2 Hz, 1H), 3.37 (d, $J$ = 10.7 Hz, 1H), 2.82 (td, $J$ = 10.9, 3.0 Hz, 1H), 2.74 - 2.57 (m, 2H), 2.21 - 2.08 (m, 1H), 2.03 - 0.60 (m, 55H) ppm,

$^{13}C$ NMR (126 MHz, $CDCl_3$) δ 218.0, 184.9, 155.6, 127.4, 123.4, 106.1, 98.8, 88.7, 75.8, 74.6, 71.4, 69.5, 68.3, 67.1, 60.6, 60.3, 55.9, 51.3, 49.4, 49.3, 40.5, 38.6, 37.3, 35.8, 32.4, 32.3, 32.2, 29.3, 28.0, 27.4, 26.8, 23.9, 19.9, 19.8, 17.6, 15.9, 15.4, 14.6, 13.1, 12.1, 11.9, 10.8, 6.7, 6.5 ppm.

Example FLC-00410-1

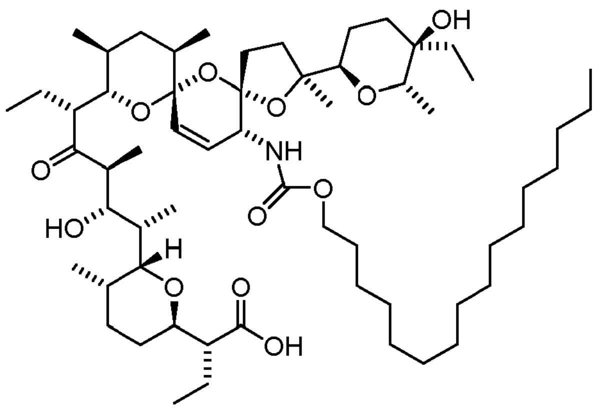

The compound was prepared according to the procedure described in example FLC-00445-1, except that hexadecyl chloroformate (2.2 eq) was added to the reaction mixture instead of 5-chloropentanoyl chloride. The yield of the obtained product was 33%.
ESI-MS for $C_{59}H_{103}NO_{12}$ (m/z): $[M+Na]^+$ 1041.2, $[M-H]^-$ 1017.2.
$^1H$ NMR (500 MHz, $CDCl_3$) δ 6.41 (d, *J* = 9.7 Hz, 1H), 6.09 (dd, *J* = 10.9, 2.9 Hz, 1H), 5,77 (d, *J*= 10.9 Hz, 1H), 4.48 (d, *J* = 9.5 Hz, 1H), 4.28 - 4.15 (m, 3H), 4.05 (dt, *J*= 10.5, 7.1 Hz, 1H), 3.95 - 3.83 (m, 3H), 3.66 (d, *J*= 10.1 Hz, 1H), 3.58 (d, *J* = 10.0 Hz, 1H), 3.36 (d, *J*= 11.0 Hz, 1H), 2.82 (td, *J* = 10.9, 2.9 Hz, 1H), 2.71 - 2.58 (m, 2H), 2.22 - 0.60 (m, 86H) ppm. $^{13}C$ NMR (126 MHz, $CDCl_3$) δ 218.0, 184.7, 157.6, 128.4, 122.8, 106.5, 98.9, 88.4, 75.9, 75.7, 74.7, 71.4, 69.5, 68.1, 65.0, 55.9, 51.3, 49.4, 48.9, 40.6, 38.7, 37.1, 35.9, 32.4, 32.3, 32.0, 30.9, 29.7, 29.7, 29.6, 29.5, 29.4, 29.1, 28.0, 27.5, 26.9, 25.7, 24.0, 22.7, 20.0, 19.8, 17.6, 15.9, 15.4, 14.6, 14.1, 13.1, 12.4, 12.1, 10.8, 6.7, 6.6 ppm.

Example FLC-00424-1

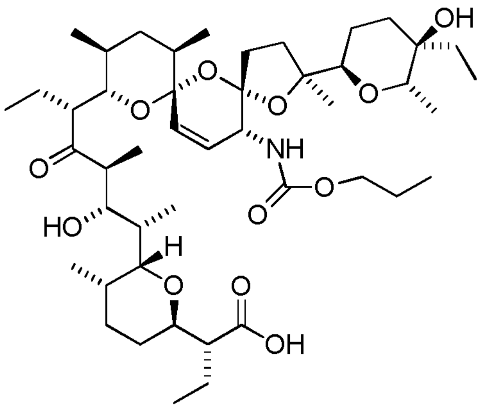

The compound was prepared according to the procedure described in example FLC-00445-1, except that propyl chloroformate (2.2 eq) was added to the reaction mixture instead of 5-chloropentanoyl chloride. The yield of the obtained product was 55%.
ESI-MS for $C_{46}H_{77}NO_{12}$ (m/z): $[M+Na]^+$ 859.1, $[M-H]^-$ 835.1.
$^1H$ NMR (500 MHz, $CDCl_3$) δ 6.41 (d, *J* = 9.7 Hz, 1H), 6.10 (dd, *J* = 10.9, 3.1 Hz, 1H), 5.77 (dd, *J* = 10.8, 1.7 Hz, 1H), 4.50 - 4.46 (m, 1H), 4.26 - 4.15 (m, 2H), 4.07 - 3.99 (m, 1H), 3.93 (dd, *J* = 11.0, 4.6 Hz, 1H), 3.85 (dt, *J*= 10.5, 6.6 Hz, 1H), 3.66 (dd, *J* = 10.1, 1.8 Hz, 1H), 3.59 (d, *J* = 10.1 Hz, 1H), 3.37 (dd, *J* = 12.0, 1.7 Hz, 1H), 2.83 (td, *J* = 11.0, 3.2 Hz, 1H), 2.71 - 2.59 (m, 2H), 2.23 - 0.60 (m, 62H) ppm.
$^{13}C$ NMR (126 MHz, $CDCl_3$) δ 218.0, 184.8, 157.6, 128.3, 122.9, 106.5, 98.9, 88.4, 75.9, 75.7, 74.7, 71.4, 69.5, 68.1, 67.1, 66.5, 55.8, 51.3, 49.4, 48.8, 40.6, 38.7, 37.1, 35.89, 32.4, 32.3, 29.7, 28.0, 27.5, 26.8, 24.0, 22.4, 19.9, 19.8, 17.6, 15.9, 15.4, 14.7, 13.1, 12.4, 12.1, 10.7, 10.4, 6.7, 6.6 ppm.

Example FLC-00425-1

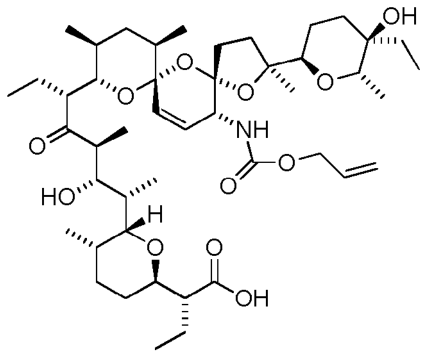

The compound was prepared according to the procedure described in example FLC-00445-1 / JK-B30-f1, except that allyl chloroformate (2.2 eq) was added to the reaction mixture instead of 5-chloropentanoyl chloride. The yield of the obtained product was 56%.
ESI-MS for $C_{46}H_{75}NO_{12}$ (m/z): $[M+Na]^+$ 857.0, $[M-H]^-$ 833.0.
$^1H$ NMR (500 MHz, $CDCl_3$) δ 6.60 (d, *J* = 9.7 Hz, 1H), 6.10 (dd, *J* = 10.9, 3.0 Hz, 1H), 5.88 (ddd, *J* = 22.7, 10.7, 5.4 Hz, 1H), 5.77 (dd, *J* = 10.8, 1.7 Hz, 1H), 5.37 - 5.26 (m, 1H), 5.13 - 5.03 (m, 1H), 4.70 (s, 1H), 4.60 (dd, *J* = 13.5, 5.5 Hz, 1H), 4.51 - 4.46 (m, 1H), 4.39 (dd, *J* = 13.5, 5.4 Hz, 1H), 4.27 - 4.15 (m, 2H), 3.93 (dd, *J* = 10.9, 4.6 Hz, 1H), 3.66 (dd, *J* = 10.1, 1.7 Hz, 1H), 3.58 (d, *J* = 10.1 Hz, 1H), 3.36 (dd, *J* = 12.0, 1.7 Hz, 1H), 2.82 (td, *J* = 11.0, 3.2 Hz, 1H), 2.71 - 2.60 (m, 2H), 2.23 - 0.61 (m, 56H) ppm.
$^{13}C$ NMR (126 MHz, $CDCl_3$) δ 218.0, 184.9, 157.2, 133.4, 128.2, 123.0, 116.9, 106.4, 98.8, 88.4, 75.9, 75.7, 74.7, 71.4, 69.6, 68.1, 67.1, 65.3, 55.8, 51.3, 49.4, 48.9, 40.5, 38.6, 37.2, 35.9, 32.4, 32.3, 29.7, 28.0, 27.5, 26.8, 24.0, 19.9, 19.8, 17.6, 15.9, 15.4, 14.7, 13.1, 12.5, 12.1, 10.7, 6.7, 6.6, 1.1 ppm.

Example FLC-00429-1

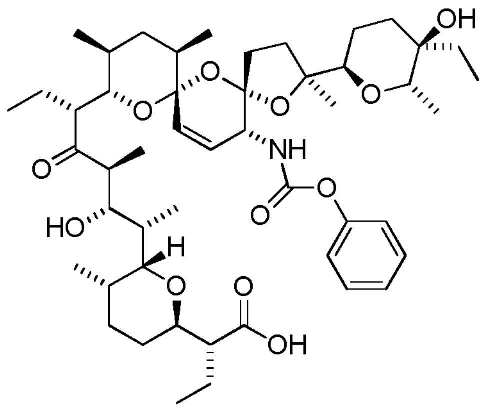

The compound was prepared according to the procedure described in example FLC-00445-1, except that phenyl chloroformate (2.2 eq) was added to the reaction mixture instead of 5-chloropentanoyl chloride. The yield of the obtained product was 93%.
ESI-MS for $C_{49}H_{75}NO_{12}$ (*m/z*): $[M+Na]^+$ = 893. $[M-H]^-$ = 869.
$^1H$ NMR (500 MHz, $CD_2Cl_2$) δ 7.31 (t, J = 7.9 Hz, 2H), 7.20 (d, J = 9.4 Hz, 1H), 7.15 (dd, J = 12.4, 7.5 Hz, 3H), 6.18 (dd, J = 10.8, 3.0 Hz, 1H), 5.76 (dd, J = 10.8, 1.6 Hz, 1H), 4.53 - 4.46 (m, 1H), 4.22 (q, J = 6.7 Hz, 1H), 4.13 (d, J = 10.1 Hz, 1H), 3.83 (dd, J = 11.0, 4.5 Hz, 1H), 3.68 - 3.60 (m, 2H), 3.44 (m, 1H), 2.84 - 2.71 (m, 2H), 2.68 (d, J = 10.2 Hz, 1H), 2.30 - 2.12 (m, 2H), 2.05 - 1.10 (m, 42H), 0.83 (d, J = 7.2 Hz, 3H), 0.80 - 0.72 (m, 6H), 0.70 (d, J = 6.9 Hz, 3H) ppm.
$^{13}C$ NMR (126 MHz, $CD_2Cl_2$) δ 218.9, 184.8, 155.2, 151.4, 128.8, 127.2, 124.8, 123.6, 122.1, 106.4, 98.8, 88.5, 77.0, 75.9, 74.8, 71.2, 69.7, 68.2, 56.0, 51.0, 49.1, 49.0, 40.5, 38.5, 37.5, 35.9, 32.4, 32.3, 32.2, 29.7, 28.0, 27.1, 26.7, 23.8, 19.9, 19.8, 17.2, 15.7, 15.2, 14.5, 12.8, 12.2, 12.1, 10.5, 6.4, 6.3 ppm.

Example FLC-00431-1

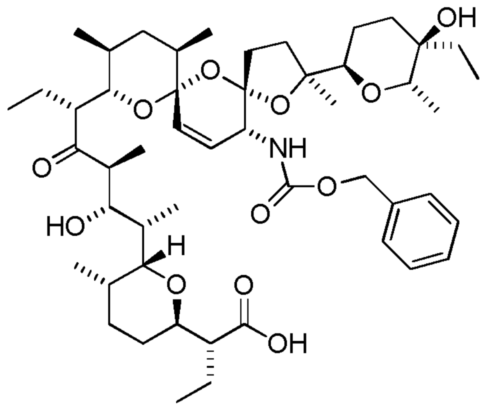

The compound was prepared according to the procedure described in example FLC-00445-1, except that benzyl chloroformate (2.2 eq) was added to the reaction mixture instead of 5-chloropentanoyl chloride. The yield of the obtained product was 60%.

ESI-MS for $C_{50}H_{77}NO_{12}$ (m/z): $[M+Na]^+$ 908.0, $[M-H]^-$ 883.0.

$^1$H NMR (500 MHz, $CD_2Cl_2$) δ 7.30 (ddd, J= 26.8, 23.6, 7.3 Hz, 5H), 6.61 (d, *J* = 9.6 Hz, 1H), 6.11 (dd, *J* = 10.8, 3.0 Hz, 1H), 5.69 (d, *J* = 10.8 Hz, 1H), 5.15 (d, *J* = 12.8 Hz, 1H), 4.90 (d, *J* = 12.8 Hz, 1H), 4.46 (d, *J* = 9.6 Hz, 1H), 4.16 (q, *J* = 6.7 Hz, 1H), 4.08 (d, *J* = 10.5 Hz, 1H), 3.83 (dd, *J* = 10.9, 4.5 Hz, 1H), 3.63 - 3.58 (m, 2H), 3.41 - 3.36 (m, 1H), 2.79 - 2.68 (m, 2H), 2.66 (d, *J* = 10.1 Hz, 1H), 2.24 - 0.60 (m, 57H) ppm.

$^{13}$C NMR (126 MHz, $CD_2Cl_2$) δ 219.0, 184.4, 156.8, 137.5, 128.1, 128.0, 127.7, 127.4, 123.1, 106.4, 99.0, 88.4, 76.9, 76.0, 75.9, 74.3, 71.1, 69.7, 68.3, 67.0, 66.0, 56.0, 50.9, 49.4, 49.1, 40.5, 38.5, 37.2, 35.8, 32.4, 32.2, 29.7, 29.1, 28.1, 26.8, 26.7, 23.6, 19.9, 19.7, 17.3, 15.7, 15.3, 14.4, 12.8, 12.2, 12.1, 10.6, 6.5, 6.4 ppm.

Example FLC-00436-1

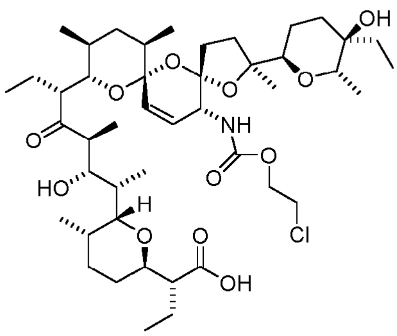

The compound was prepared according to the procedure described in example FLC-00445-1, except that 2-chloroethyl chloroformate (2.2 eq) was added to the reaction mixture instead of 5-chloropentanoyl chloride. The yield of the obtained product was 43%.

ESI-MS for $C_{45}H_{74}ClNO_{12}$ (m/z): $[M+Na]^+$879.0, $[M-H]^-$855.2.

$^1$H NMR (500 MHz, $CD_2Cl_2$) δ 6.72 (d, *J* = 9.5 Hz, 1H), 6.12 (dd, *J* = 10.8, 3.0 Hz, 1H), 5.67 (d, *J* = 10.8 Hz, 1H), 4.43 (d, *J* = 9.5 Hz, 1H), 4.30 (dt, *J* = 11.8, 6.6 Hz, 1H), 4.17 (q, *J* = 6.6 Hz, 1H), 4.12 - 4.03 (m, 2H), 3.84 (dd, *J* = 10.9, 4.3 Hz, 1H), 3.63 - 3.58 (m, 4H), 3.41 (d, *J* = 11.3 Hz, 1H), 2.82 - 2.69 (m, 2H), 2.66 (d, *J* = 10.2 Hz, 1H), 2.26 - 0.64 (m, 57H) ppm,

$^{13}$C NMR (126 MHz, $CD_2Cl_2$) δ 219.3, 184.8, 156.7, 128.0, 123.7, 106.8, 99.2, 88.8, 77.3, 76.4, 76.3, 74.8, 71.6, 70.1, 68.6, 64.3, 56.4, 51.3, 49.6, 49.4, 42.0, 40.9, 38.9, 37.7, 36.2, 32.8, 32.6, 32.5, 29.8, 28.4, 27.2, 27.1, 24.1, 20.3, 20.1, 17.7, 16.1, 15.7, 14.8, 13.2, 12.7, 12.5, 11.0, 6.9, 6.68 ppm.

Example FLC-00437-1

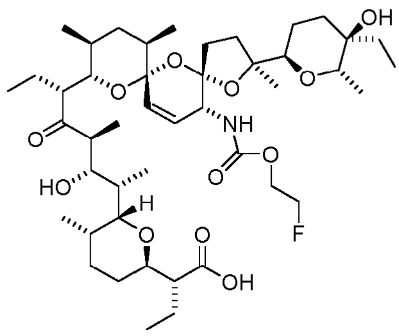

The compound was prepared according to the procedure described in example FLC-00445-1, except that 2,2,2-fluoroethyl chloroformate (2.2 eq) was added to the reaction mixture instead of 5-chloropentanoyl chloride. The yield of the obtained product was 63%.

ESI-MS for $C_{45}H_{74}FNO_{12}$ (m/z): $[M+Na]^+$862.9, $[M-H]^-$839.0.

$^1$H NMR (500 MHz, $CDCl_3$) δ 6.74 (d, *J* = 9.6 Hz, 1H), 6.10 (dd, *J* = 10.9, 3.0 Hz, 1H), 5.75 (dd, *J* = 10.8, 1.5 Hz, 1H), 4.61 - 4.53 (m, 1H), 4.50 - 4.43 (m, 2H), 4.43 - 4.35 (m, 1H), 4.23 (q, *J* = 6.6 Hz, 1H), 4.18 (d, *J* = 10.1 Hz, 1H), 4.07 (dddd, *J* = 28.3, 12.6, 5.0, 3.3 Hz, 1H), 3.91 (dd, *J* = 11.0, 4.5 Hz, 1H), 3.64 (dd, *J* = 10.1, 1.5 Hz, 1H), 3.56 (d, *J* = 10.1 Hz, 1H), 3.35 (d, *J* = 10.6 Hz, 1H), 2.83 (td, *J* = 11.0, 3.1 Hz, 1H), 2.70 - 2.60 (m, 2H), 2.21 - 0.63 (m, 57H) ppm.

$^{13}$C NMR (126 MHz, $CDCl_3$) δ 218.1, 185.0, 157.0, 127.9, 123.2, 106.4, 98.8, 88.5, 82.5, 81.1, 75.9, 75.8, 74.9, 71.5, 69.7, 68.2, 63.44 (d) 55.9, 51.3, 49.5, 48.9, 40.6, 38.7, 37.4, 36.0, 32.5, 32.4, 29.8, 28.1, 27.6, 26.9, 24.1, 20.0, 19.9, 17.6, 16.0, 15.5, 14.8, 13.2, 12.2, 12.2, 10.8, 6.8, 6.64 ppm.

$^{19}$F NMR (471 MHz, $CDCl_3$) δ -77.0 ppm.

Example FLC-00439-1

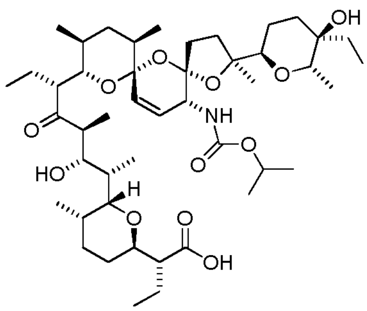

The compound was prepared according to the procedure described in example FLC-00445-1, except that isopropyl chloroformate (2.2 eq) was added to the reaction mixture instead of 5-chloropentanoyl chloride. The yield of the obtained product was 16%.

ESI-MS for $C_{46}H_{77}NO_{12}$ (m/z): $[M+Na]^+$859.0, $[M-H]^-$835.1.

$^1$H NMR (500 MHz, $CD_2Cl_2$) δ 6.25 (d, *J* = 9.4 Hz, 1H), 6.10 (dd, *J* = 10.8, 2.9 Hz, 1H), 5.67 (d, *J* = 10.8 Hz, 1H), 4.89 - 4.76 (m, 1H), 4.44 (d, *J* = 9.4 Hz, 1H), 4.16 (q, *J* = 6.6 Hz, 1H), 4.09 (d, *J* = 10.3 Hz, 1H), 3.83 (dd, *J* = 10.7, 3.9 Hz, 1H), 3.41 (d, *J* = 11.4 Hz, 1H), 2.83 - 2.69 (m, 2H), 2.66 (d, *J* = 10.3 Hz, 1H), 2.28 - 0.66 (m, 65H) ppm.

$^{13}$C NMR (126 MHz, $CD_2Cl_2$) δ 219.4, 184.6, 156.9, 128.5, 123.4, 107.0, 99.3, 88.7, 77.4, 76.4, 76.2, 74.8, 71.6, 70.0, 68.7, 68.0, 56.4, 51.4, 49.5, 49.4, 40.9, 39.0, 37.5, 36.2, 32.9, 32.6, 32.5, 29.7, 28.5, 27.2, 27.1, 24.2, 22.3, 22.2, 20.3, 20.1, 17.7, 16.1, 15.7, 14.8, 13.4, 13.2, 12.5, 11.0, 6.9, 6.70 ppm.

Example FLC-00440-1

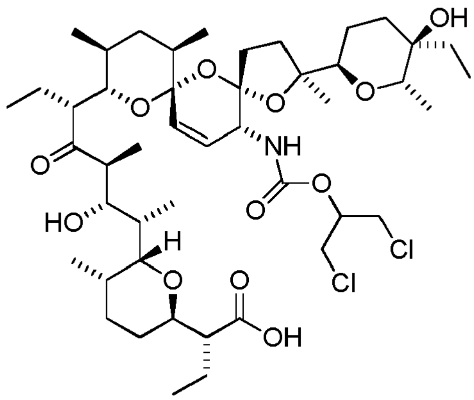

The compound was prepared according to the procedure described in example FLC-00445-1, except that 1,3-dichloroisopropyl chloroformate (2.2 eq) was added to the reaction mixture instead of 5-chloropentanoyl chloride. The yield of the obtained product was 61%.

ESI-MS for $C_{46}H_{75}Cl_2NO_{12}$ (m/z): $[M+Na]^+$927.0, $[M-H]^-$903.1.

$^1$H NMR (500 MHz, $CDCl_3$) δ 6,68 (d, *J* = 9.4 Hz, 1H), 6.11 (dd, *J* = 10.9, 3.0 Hz, 1H), 5.77 (dd, *J* = 10.8, 1.3 Hz, 1H), 5.00 (dq, *J* = 10.4, 5.2 Hz, 1H), 4.47 (d, *J* = 9.4 Hz, 1H), 4.24 (q, *J* = 6.6 Hz, 1H), 4.17 (d, *J* = 10.4 Hz, 1H), 3.92 (dd, *J* = 11.0, 4.4 Hz, 1H), 3.77 - 3.70 (m, , 3H), 3.68 - 3.60 (m, 3H), 3.55 (d, *J* = 10.1 Hz, 1H), 3.37 (d, *J* = 10.9 Hz, 1H), 2.83 (td, *J* = 10.9, 3.1 Hz, 1H), 2.69 - 2.60 (m, 2H), 2.19 - 0.62 (m, 56H) ppm,

$^{13}$C NMR (126 MHz, $CDCl_3$) δ 218.0, 185.1, 156.0, 127.6, 123.4, 106.3, 98.9, 88.6, 76.8, 75.8, 74.9, 73.4, 71.5, 69.8, 68.3, 55.9, 51.2, 49.4, 49.1, 43.1, 42.5, 40.6, 38.7, 37.2, 35.9, 32.5, 32.4, 32.4, 29.8, 29.6, 28.1, 27.7, 26.9, 24.1, 20.0, 17.6, 16.0, 15.5, 14.7, 13.2, 13.0, 12.2, 10.8, 6.8, 6.67 ppm.

Example FLC-00444-1

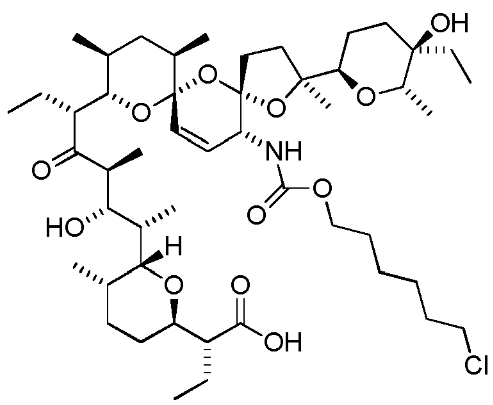

The compound was prepared according to the procedure described in example FLC-00445-1, except that 6-chlorohexyl chloroformate (2.2 eq) was added to the reaction mixture instead of 5-chloropentanoyl chloride. The yield of the obtained product was 61%.

ESI-MS for $C_{49}H_{82}ClNO_{12}$ (m/z): $[M+Na]^+$935.1, $[M-H]^-$911.1.

$^1$H NMR (700 MHz, $CDCl_3$) δ 6.44 (d, *J* = 9.7 Hz, 1H), 6.10 (dd, *J* = 10.9, 3.1 Hz, 1H), 5.76 (dd, *J* = 10.8, 1.8 Hz, 1H), 4.48 (ddd, *J* = 9.7, 2.9, 2.0 Hz, 1H), 4.24 (q, *J* = 6.6 Hz, 1H), 4.19 (dd, *J* = 10.4, 1.5 Hz, 1H), 4.06 (dt, *J* = 10.6, 6.9 Hz, 1H), 3.95 - 3.87 (m, 2H), 3.65 (dd, *J* = 10.1, 2.0 Hz, 1H), 3.58 (d, *J* = 10.1 Hz, 1H), 3.50 (t, *J* = 6.8 Hz, 2H), 3.36 (dd, *J* = 12.1, 2.0 Hz, 1H), 2.83 (td, *J* = 11.0, 3.3 Hz, 1H), 2.68 - 2.60 (m, 2H), 2.21 - 0.63 (m, 65H) ppm.

$^{13}$C NMR (176 MHz, $CDCl_3$) δ 218.1, 184.8, 157.7, 128.4, 123.0, 106.6, 99.0, 88.5, 76.8, 76.0, 75.8, 74.8, 71.5, 69.7, 68.2, 64.8, 56.0, 51.4, 49.5, 49.0, 45.2, 40.7, 38.8, 37.2, 36.0, 32.7, 32.5, 32.5, 32.5, 29.1, 28.1, 27.6, 26.9, 26.8, 25.2, 24.1, 20.0, 19.9, 17.6, 16.0, 15.5, 14.8, 13.2, 12.5, 12.2, 10.8, 6.8, 6.69 ppm.

Example FLC-00446-1

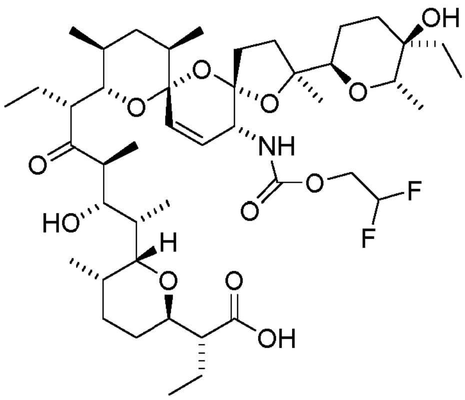

The compound was prepared according to the procedure described in example FLC-00445-1, except that 2,2-difluoroethyl chloroformate (2.2 eq) was added to the reaction mixture instead of 5-chloropentanoyl chloride. The yield of the obtained product was 34%.

ESI-MS for $C_{45}H_{73}F_2NO_{12}$ (m/z): $[M+Na]^+$880.9, $[M-H]^-$857.1.

$^1$H NMR (700 MHz, $CDCl_3$) δ 6.96 (d, *J* = 9.6 Hz, 1H), 6.12 (dd, *J* = 10.9, 3.1 Hz, 1H), 5.96 (tdd, *J* = 8.7, 6.7, 3.9 Hz, 1H), 5.75 (dd, *J* = 10.8, 1.9 Hz, 1H), 4.47 (ddd, *J* = 9.6, 2.9, 2.1 Hz, 1H), 4.42 - 4.35 (m, 1H), 4.25 (q, *J* = 6.7 Hz, 1H), 4.19 (dd, *J* = 10.4, 1.4 Hz, 1H), 3.98 (dtd, *J* = 16.1, 12.3, 3.7 Hz, 1H), 3.92 (dd, *J* = 11.0, 4.8 Hz, 1H), 3.64 (dd, *J* = 10.2, 2.0 Hz, 1H), 3.56 (d, *J* = 9.8 Hz, 1H), 3.36 (dd, *J* = 12.1, 2.0 Hz, 1H), 2.87 - 2.81 (m, 1H), 2.70 - 2.60 (m, 2H), 2.18 - 0.63 (m, 57H) ppm.

$^{13}$C NMR (176 MHz, $CDCl_3$) δ 218.1, 185.2, 156.2, 127.5, 123.6, 113.9, 106.3, 98.8, 88.6, 76.9, 75.9, 75.9, 75.0, 71.5, 69.8, 68.2, 63.2, 56.0, 51.3, 49.4, 49.0, 40.6, 38.7, 37.5, 36.0, 32.5, 32.4, 29.8, 29.8, 28.1, 27.7, 26.9, 24.1, 20.0, 20.0, 17.6, 16.0, 15.5, 14.8, 13.2, 12.2, 12.1, 10.8, 6.8, 6.61 ppm.

Example FLC-00447-1

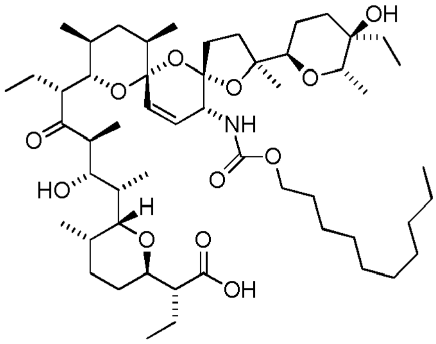

The compound was prepared according to the procedure described in example FLC-00445-1, except that decyl chloroformate (2.2 eq) was added to the reaction mixture instead of 5-chloropentanoyl chloride. The yield of the obtained product was 52%.

ESI-MS for $C_{53}H_{91}NO_{12}$ (*m/z*): $[M+Na]^+$957.0.

$^1$H NMR (700 MHz, $CDCl_3$) δ 6.41 (d, *J* = 9.7 Hz, 1H), 6.09 (dd, *J* = 10.9, 3.1 Hz, 1H), 5.77 (dd, *J* = 10.8, 1.7 Hz, 1H), 4.50 - 4.46 (m, 1H), 4.24 (q, *J* = 6.6 Hz, 1H), 4.19 (d, *J* = 10.4 Hz, 1H), 4.05 (dt, *J* = 10.6, 7.1 Hz, 1H), 3.93 (dd, *J* = 10.9, 4.6 Hz, 1H), 3.88 (dt, *J* = 10.6, 6.8 Hz, 1H), 3.66 (d, *J* = 10.1 Hz, 1H), 3.58 (d, *J* = 10.0 Hz, 1H), 3.38 - 3.35 (m, 1H), 2.82 (td, *J* = 11.1, 3.3 Hz, 1H), 2.71 - 2.65 (m, 1H), 2.62 (d, *J* = 11.0 Hz, 1H), 2.23 - 0.63 (m, 76H) ppm.

$^{13}$C NMR (176 MHz, $CDCl_3$) δ 218.1, 184.8, 157.7, 128.5, 123.0, 106.6, 99.0, 88.5, 76.9, 76.0, 75.8, 74.8, 71.5, 69.6, 68.2, 65.2, 56.0, 51.4, 49.5, 49.0, 40.7, 38.8, 37.2, 36.0, 32.5, 32.5, 32.4, 32.0, 29.9, 29.8, 29.8, 29.7, 29.6, 29.4, 29.2, 28.1, 27.6, 27.0, 25.9, 24.1, 22.8, 20.1, 19.9, 17.7, 16.0, 15.5, 14.8, 14.2, 13.2, 12.5, 12.2, 10.8, 6.8, 6.70 ppm.

Example FLC-00450-1

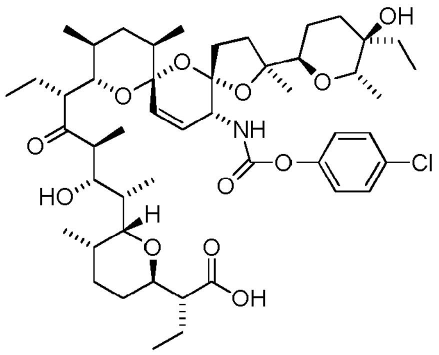

The compound was prepared according to the procedure described in example FLC-00445-1, except that 4-chlorophenyl chloroformate (2.2 eq) was added to the reaction mixture instead of 5-chloropentanoyl chloride. The yield of the obtained product was 83%.

ESI-MS for $C_{49}H_{74}ClNO_{12}$ (*m/z*): $[M+Na]^+$ 927, $[M-H]^-$ 903

$^1H$ NMR (700 MHz, $CDCl_3$) δ 7.42 (d, J = 9.3 Hz, 1H), 7.30 - 7.22 (m, 4H), 6.18 (dd, J = 10.9, 2.9 Hz, 1H), 5.85 (dd, J = 10.8, 1.5 Hz, 1H), 4.54 (m, 1H), 4.30 (q, J = 6.5 Hz, 1H), 4.24 (d, J = 10.3 Hz, 1H), 3.93 (dd, J = 10.9, 4.4 Hz, 1H), 3.68 - 3.64 (m, 1H), 3.59 (d, J = 10.0 Hz, 1H), 3.39 (d, J = 10.9 Hz, 1H), 2.87 (td, J = 11.0, 2.9 Hz, 1H), 2.70 (dd, J = 10.2, 7.3 Hz, 1H), 2.66 (d, J = 10.4 Hz, 1H), 2.23 (dd, J = 21.2, 11.5 Hz, 1H), 2.12 (qd, J = 12.6, 3.9 Hz, 1H), 2.05 - 0.87 (m, 41H), 0.85 (d, J = 7.2 Hz, 3H), 0.77 (t, J = 7.4 Hz, 3H), 0.74 (d, J = 6.6 Hz, 3H), 0.71 (d, J = 6.9 Hz, 3H) ppm.

$^{13}C$ NMR (176 MHz, $CDCl_3$) δ 217.9, 185.3, 155.2, 149.9, 130.0, 128.8, 127.2, 123.7, 123.5, 106.3, 98.6, 88.5, 75.9, 75.7, 75.2, 71.5, 69.7, 68.2, 55.8, 51.4, 49.2, 48.8, 40.4, 38.6, 37.6, 35.9, 32.4, 32.4, 31.9, 29.9, 29.7, 29.7, 29.4, 27.9, 27.7, 26.8, 24.1, 22.7, 20.0, 19.8, 17.5, 15.9, 15.3, 14.7, 14.1, 13.1, 12.2, 12.1, 10.7, 6.6, 6.6 ppm.

Example FLC-00451-1

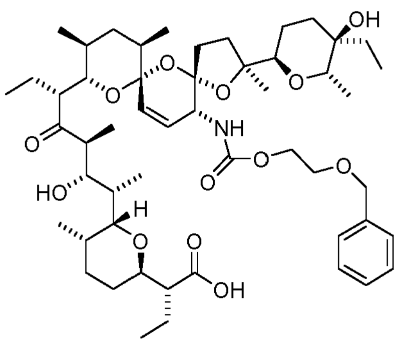

The compound was prepared according to the procedure described in example FLC-00445-1, except that 2-benzyloxyethyl chloroformate (2.2 eq) was added to the reaction mixture instead of 5-chloropentanoyl chloride. The yield of the obtained product was 75%.

ESI-MS for $C_{52}H_{81}NO_{13}$ (*m/z*): $[M+Na]^+$ 951, $[M-H]^-$ 927.

$^1H$ NMR (700 MHz, $CDCl_3$) δ 7.36 - 7.31 (m, 4H), 7.29 - 7.25 (m, 1H), 6.57 (d, J = 9.5 Hz, 1H), 6.12 (dd, J = 10.8, 2.8 Hz, 1H), 5.79 (d, J = 10.6 Hz, 1H), 4.59 - 4.49 (m, 3H), 4.35 (ddd, J = 17.0, 12.4, 6.2 Hz, 1H), 4.28 - 4.22 (m, 1H), 4.21 (d, J = 10.2 Hz, 1H), 4.10 (dt, J = 11.0, 3.9 Hz, 1H), 3.96 (dd, J = 10.6, 4.3 Hz, 1H), 3.72 - 3.57 (m, 4H), 3.35 (d, J = 11.9 Hz, 1H), 2.85 (dd, J = 10.4, 9.2 Hz, 1H), 2.73 - 2.67 (m, 1H), 2.65 (d, J = 10.2 Hz, 1H), 2.23 - 2.17 (m, 1H), 2.10 - 1.10 (m, 32H), 1.01 (dt, J = 7.4, 5.5 Hz, 3H), 0.95 (d, J = 6.9 Hz, 3H), 0.92 - 0.88 (m, 6H), 0.84 (d, J = 7.2 Hz, 3H), 0.77 (t, J = 7.4 Hz, 3H), 0.74 (d, J = 6.6 Hz, 3H), 0.71 (d, J = 6.8 Hz, 3H) ppm.

$^{13}C$ NMR (176 MHz, $CDCl_3$) δ 218.0, 184.7, 157.2, 138.4, 128.3, 128.2, 127.7, 127.5, 122.9, 106.4, 98.9, 88.4, 76.7, 75.9, 75.7, 74.6, 73.0, 71.4, 68.4, 68.2, 63.2, 55.9, 51.1, 49.4, 49.1, 40.5, 38.6, 37.1, 35.9, 32.4, 32.4, 32.2, 29.8, 29.7, 29.7, 28.0, 27.4, 26.8, 23.9, 19.9, 19.8, 17.6, 15.9, 14.6, 13.1, 12.4, 12.1, 10.8, 6.7, 6.6 ppm.

Example FLC-00454-1

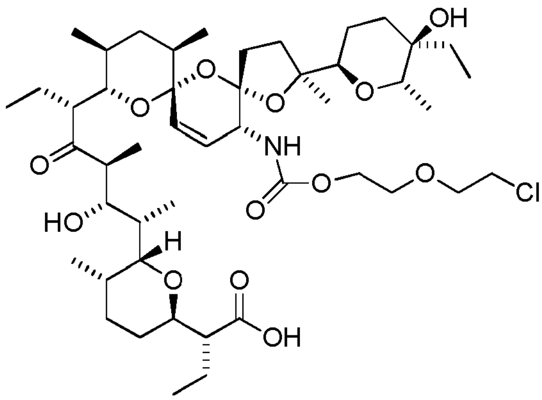

The compound was prepared according to the procedure described in example FLC-00445-1, except that 2-(2-chloroethoxy)ethyl chloroformate (2.2 eq) was added to the reaction mixture instead of 5-chloropentanoyl chloride. The yield of the obtained product was 70%.
ESI-MS for $C_{47}H_{78}ClNO_{13}$ (*m/z*): $[M+Na]^+$ 923.0.
$^1H$ NMR (700 MHz, $CDCl_3$) δ 6.55 (d, *J* = 9.7 Hz, 1H), 6.10 (dd, *J* = 10.9, 3.1 Hz, 1H), 5.76 (dd, *J* = 10.8, 1.8 Hz, 1H), 4.49 - 4.45 (m, 1H), 4.25 (qd, *J* = 11.5, 6.2 Hz, 2H), 4.18 (dd, *J* = 10.4, 1.4 Hz, 1H), 4.06 - 4.01 (m, 1H), 3.93 (dd, *J* = 11.0, 4.9 Hz, 1H), 3.74 - 3.67 (m, 2H), 3.66 - 3.63 (m, 3H), 3.60 - 3.56 (m, 3H), 3.36 (dd, *J* = 12.1, 2.0 Hz, 1H), 2.82 (td, *J*= 11.0, 3.3 Hz, 1H), 2.69 - 2.64 (m, 1H), 2.62 (d, *J* = 9.7 Hz, 1H), 2.20 - 0.64 (m, 57H) ppm,
**[0833]** $^{13}C$ NMR (176 MHz, $CDCl_3$) δ 218.1, 184.8, 157.2, 128.2, 123.1, 106.5, 99.0, 88.5, 76.8, 76.0, 75.8, 74.8, 71.5, 71.2, 69.7, 69.4, 68.2, 63.4, 56.0, 51.3, 49.5, 49.1, 43.0, 40.6, 38.8, 37.2, 36.0, 32.5, 32.5, 32.5, 30.0, 29.8, 28.1, 27.5, 26.9, 24.0, 20.0, 19.9, 17.6, 16.0, 15.5, 14.8, 13.2, 12.5, 12.2, 10.8, 6.8, 6.69 ppm.

Example FLC-00459-1

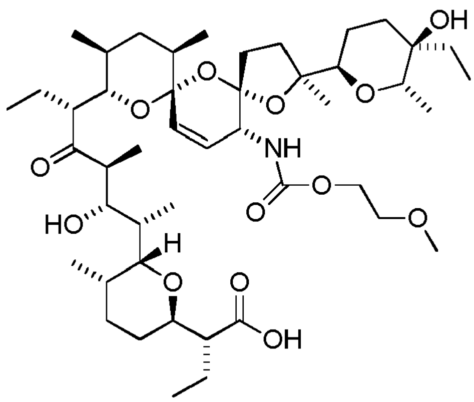

The compound was prepared according to the procedure described in example FLC-00445-1, except that 2-methoxyethyl chloroformate (2.2 eq) was added to the reaction mixture instead of 5-chloropentanoyl chloride. The yield of the obtained product was 45%.
ESI-MS for $C_{46}H_{77}NO_{13}$ (*m/z*): $[M+Na]^+$ 874.9.
$^1H$ NMR (700 MHz, $CDCl_3$) δ 6.53 (d, *J* = 9.6 Hz, 1H), 6.10 (dd, *J* = 10.8, 2.9 Hz, 1H), 5.76 (dd, *J* = 10.7, 1.2 Hz, 1H), 4.47 (d, *J* = 9.6 Hz, 1H), 4.26 - 4.20 (m, 2H), 4.18 (d, *J* = 10.3 Hz, 1H), 4.06 (dt, *J* = 11.3, 5.5 Hz, 1H), 3.93 (dd, *J* = 10.9, 4.6 Hz, 1H), 3.65 (dd, *J* = 10.2, 1.5 Hz, 1H), 3.58 (d, *J*= 10.0 Hz, 1H), 3.56 - 3.49 (m, 2H), 3.37 (dd, *J* = 12.1, 1.7 Hz, 1H), 3.33 (d, *J* = 0.7 Hz, 3H), 2.83 (td, *J* = 11.0, 3.0 Hz, 1H), 2.67 (dq, *J* = 14.6, 7.2 Hz, 1H), 2.62 (d, *J* = 10.0 Hz, 1H), 2.23 - 0.63 (m, 57H) ppm.
$^{13}C$ NMR (176 MHz, $CDCl_3$) δ 218.2, 184.8, 157.3, 128.3, 123.1, 106.5, 99.0, 88.5, 76.8, 76.0, 75.8, 74.8, 71.5, 70.7, 69.7, 68.3, 63.4, 58.9, 56.0, 51.3, 49.5, 49.2, 40.7, 38.8, 37.2, 36.0, 32.5, 32.5, 32.4, 29.8, 28.1, 27.5, 26.9, 24.0, 20.0,

19.9, 17.7, 16.0, 15.5, 14.7, 13.2, 12.5, 12.2, 10.9, 6.8, 6.70 ppm.

Example FLC-00460-1

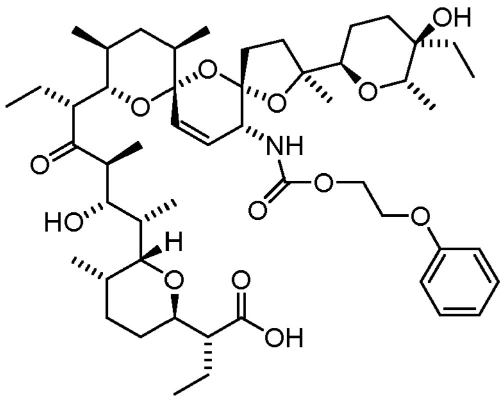

The compound was prepared according to the procedure described in example FLC-00445-1, except that 2-phenoxyethyl chloroformate (2.2 eq) was added to the reaction mixture instead of 5-chloropentanoyl chloride. The yield of the obtained product was 57 %.

ESI-MS for $C_{51}H_{79}NO_{13}$ (m/z): $[M+Na]^+$ 936.9.

$^1H$ NMR (700 MHz, $CDCl_3$) δ 7.26 - 7.23 (m, 2H), 6.93 - 6.90 (m, 1H), 6.88 (dt, *J* = 9.2, 1.7 Hz, 2H), 6.70 (d, *J* = 9.6 Hz, 1H), 6.11 (dd, *J* = 10.9, 3.1 Hz, 1H), 5.77 (dd, *J* = 10.8, 1.9 Hz, 1H), 4.52 - 4.49 (m, 1H), 4.47 (dt, *J* = 11.8, 6.0 Hz, 1H), 4.24 (q, *J* = 6.9 Hz, 1H), 4.22 - 4.17 (m, 2H), 4.14 - 4.09 (m, 2H), 3.92 (dd, *J*= 11.0, 4.8 Hz, 1H), 3.65 (dd, *J*= 10.1, 1.9 Hz, 1H), 3.58 (d, *J* = 10.0 Hz, 1H), 3.33 (dd, *J* = 12.1, 2.0 Hz, 1H), 2.83 (td, *J* = 11.0, 3.3 Hz, 1H), 2.67 (dq, *J* = 10.3, 7.2 Hz, 1H), 2.63 (d, *J* = 9.8 Hz, 1H), 2.23 - 2.17 (m, 1H), 2.10 - 0.64 (m, 56H) ppm.

$^{13}C$ NMR (176 MHz, $CDCl_3$) δ 218.1, 184.9, 158.8, 157.2, 129.5, 128.1, 123.2, 120.8, 114.7, 106.5, 98.9, 88.5, 76.8, 76.0, 75.8, 74.9, 71.5, 69.7, 68.2, 65.9, 62.5, 56.0, 51.3, 49.5, 49.0, 40.6, 38.8, 37.4, 36.0, 32.5, 32.5, 32.4, 29.9, 28.1, 27.6, 26.9, 24.1, 20.0, 19.9, 17.6, 16.0, 15.5, 14.8, 13.2, 12.4, 12.2, 10.8, 6.8, 6.7 ppm.

Example FLC-00461-1

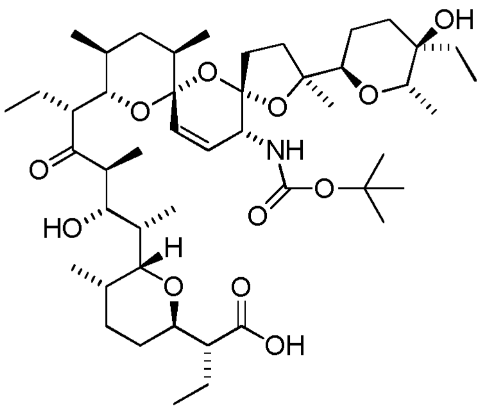

100 mg of C20-amino SAL (1 eq) was mixed with $Boc_2O$ (1.5 eq) and TEA (2 eq) in DCM. The reaction was carried out at RT (TLC and LC-MS control). The post-reaction mixture was suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was 65%.

ESI-MS for $C_{47}H_{79}NO_{12}$ (*m/z*): $[M+Na]^+$ 873.0, $[M-H]^-$ 849.0,

$^1H$ NMR (700 MHz, $CD_2Cl_2$) δ 6.14 (dd, J = 10.9, 3.2 Hz, 1H), 5.98 (d, J = 9.8 Hz, 1H), 5.72 (dd, J = 10.8, 1.8 Hz,

1H), 4.45 (ddd, J = 9.8, 3.0, 1.9 Hz, 1H), 4.20 (q, J = 6.8 Hz, 1H), 4.12 (dd, J = 10.5, 1.7 Hz, 1H), 3.89 (dd, J = 11.0, 4.8 Hz, 1H), 3.71 - 3.68 (m, 1H), 3.67 - 3.66 (m, 1H), 3.47 (dd, J = 12.2, 2.4 Hz, 1H), 2.82 - 2.75 (m, 2H), 2.70 (dd, J = 10.9, 1.4 Hz, 1H), 2.28 - 2.22 (m, 1H), 2.21 - 2.14 (m, 1H), 2.02 - 0.73 (m, 63H) ppm.

$^{13}C$ NMR (176 MHz, $CD_2Cl_2$) δ 219.0, 184.1, 156.0, 129.4, 128.5, 122.8, 106.7, 99.0, 88.4, 78.7, 76.9, 76.1, 75.7, 74.2, 71.1, 69.6, 68.3, 56.1, 50.9, 49.2, 48.8, 40.6, 38.7, 37.0, 35.8, 32.5, 32.3, 32.2, 29.0, 28.1, 26.8, 26.7, 23.6, 19.9, 19.8, 17.3, 15.6, 15.3, 14.3, 12.9, 12.8, 12.1, 10.6, 6.5, 6.3 ppm.

Example FLC-00466-1

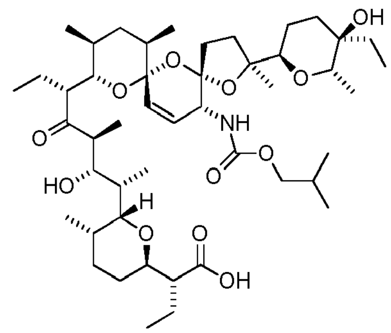

The compound was prepared according to the procedure described in example FLC-00445-1, except that isobutyl chloroformate (2 eq) was added to the reaction mixture instead of 5-chloropentanoyl chloride. The yield of the obtained product was 59%.

ESI-MS for $C_{47}H_{79}NO_{12}$ (m/z): $[M+Na]^+$ 872.9, $[M-H]^-$ 848.9.

$^1H$ NMR (700 MHz, $CD_2Cl_2$) δ 6.35 (d, J = 9.7 Hz, 1H), 6.15 (dd, J = 10.9, 3.1 Hz, 1H), 5.73 (dd, J = 10.8, 1.6 Hz, 1H), 4.48 (d, J = 9.6 Hz, 1H), 4.21 - 4.15 (m, 1H), 4.13 (dd, J = 10.5, 1.7 Hz, 1H), 3.93 - 3.87 (m, 2H), 3.72 - 3.69 (m, 1H), 3.66 - 3.64 (m, 1H), 3.49 - 3.44 (m, 1H), 2.83 - 2.75 (m, 2H), 2.72 - 2.67 (m, 1H), 2.26 - 0.72 (m, 64H) ppm.

$^{13}C$ NMR (176 MHz, $CD_2Cl_2$) δ 219.1, 184.2, 157.1, 128.3, 122.9, 106.5, 99.1, 88.5, 77.0, 76.1, 75.8, 74.2, 71.2, 71.0, 69.7, 68.3, 56.1, 50.9, 49.4, 49.1, 40.6, 38.6, 37.0, 35.8, 32.5, 32.2, 32.1, 28.8, 28.1, 28.1, 26.8, 26.7, 23.6, 19.9, 19.7, 19.0, 18.9, 17.3, 15.7, 15.3, 14.3, 12.8, 12.5, 12.1, 10.6, 6.5, 6.3 ppm.

Example FLC-00480-1

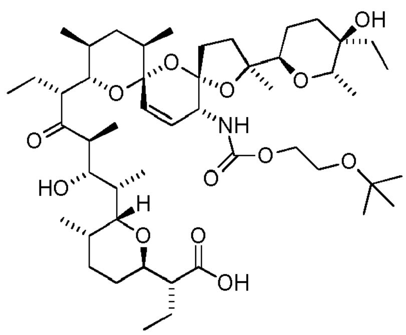

The compound was prepared according to the procedure described in example FLC-00445-1, except that 2-(tert-butoxy)ethyl chloroformate (2.2 eq) was added to the reaction mixture instead of 5-chloropentanoyl chloride. The yield of the obtained product was 53%.

ESI-MS for $C_{49}H_{82}NO_{13}$ (m/z): $[M+Na]^+$ 917.3, $[M-H]^-$ 893.0.

$^1H$ NMR (500 MHz, $CDCl_3$) δ 6.52 (d, *J* = 9.7 Hz, 1H), 6.09 (dd, *J* = 10.9, 3.0 Hz, 1H), 5.75 (dd, *J* = 10.8, 1.4 Hz, 1H), 4.46 (d, *J* = 9.6 Hz, 1H), 4.23 (q, *J* = 6.6 Hz, 1H), 4.17 (d, *J* = 10.2 Hz, 1H), 4.12 (ddd, *J* = 10.5, 7.9, 6.5 Hz, 1H), 4.00 - 3.89 (m, 2H), 3.64 (d, *J* = 9.0 Hz, 1H), 3.56 (d, *J* = 10.1 Hz, 1H), 3.50 - 3.42 (m, 2H), 3.35 (d, *J* = 10.8 Hz, 1H), 2.81 (td, *J* = 11.0, 3.1 Hz, 1H), 2.69 - 2.59 (m, 2H), 2.21 - 2.12 (m, 1H), 2.07 - 0.61 (m, 64H) ppm.

$^{13}C$ NMR (126 MHz, $CDCl_3$) δ 218.1, 184.8, 157.3, 128.2, 123.1, 106.5, 98.9, 88.5, 76.8, 75.9, 75.8, 74.8, 73.2,

71.5, 69.6, 68.2, 64.0, 59.8, 55.9, 51.3, 49.5, 49.0, 40.6, 38.7, 37.2, 36.0, 32.5, 32.4, 29.9, 28.1, 27.6, 27.5, 26.9, 24.1, 20.0, 19.9, 17.6, 16.0, 15.5, 14.8, 13.2, 12.4, 12.2, 10.8, 6.8, 6.69 ppm.

Example FLC-00481-1

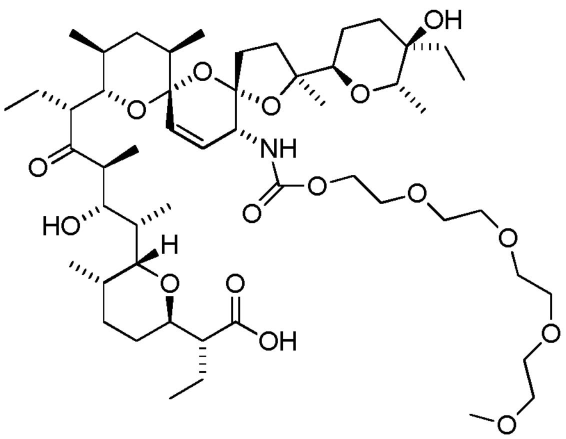

The compound was prepared according to the procedure described in example FLC-00445-1, except that 2,5,8,11-tetraoxytridecan-13-yl chloroformate (2.2 eq) was added to the reaction mixture instead of 5-chloropentanoyl chloride. The yield of the obtained product was 61%.

ESI-MS for $C_{52}H_{89}NO_{16}$ (*m/z*): $[M+Na]^+$ 1007.3, $[M-H]^-$ 983.1.

$^1H$ NMR (500 MHz, $CDCl_3$) δ 6.53 (d, *J* = 9.6 Hz, 1H), 6.09 (dd, *J* = 10.9, 3.0 Hz, 1H), 5.74 (dd, *J* = 10.8, 1.4 Hz, 1H), 4.45 (d, *J* = 9.5 Hz, 1H), 4.20 (ddd, *J* = 21.3, 14.2, 8.6 Hz, 3H), 4.04 (dt, *J* = 11.5, 5.9 Hz, 1H), 3.91 (dd, *J* = 10.9, 4.5 Hz, 1H), 3.66 - 3.51 (m, 18H), 3.35 (d, *J* = 9.0 Hz, 4H), 2.81 (td, *J* = 11.0, 3.0 Hz, 1H), 2.69 - 2.58 (m, 2H), 2.21 - 0.61 (m, 55H) ppm.

$^{13}C$ NMR (126 MHz, $CDCl_3$) δ 218.1, 184.9, 157.2, 128.2, 123.1, 106.4, 98.9, 88.5, 76.7, 75.9, 75.8, 74.8, 72.0, 71.4, 70.7, 70.7, 70.6, 70.5, 69.6, 69.2, 68.2, 63.3, 59.1, 55.9, 51.3, 49.5, 49.0, 40.6, 38.7, 37.2, 35.9, 32.5, 29.9, 28.1, 27.5, 26.9, 24.0, 20.0, 19.8, 17.6, 16.0, 15.5, 14.7, 13.2, 12.5, 12.2, 10.8, 6.8, 6.7 ppm.

Example FLC-00483-1

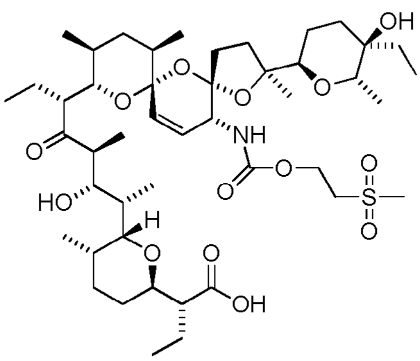

The compound was prepared according to the procedure described in example FLC-00445-1, except that 2-(methylsulphonyl)ethyl chloroformate (2.2 eq) was added to the reaction mixture instead of 5-chloropentanoyl chloride. The yield of the obtained product was 36%.

ESI-MS for $C_{46}H_{77}NO_{14}S$ (*m/z*): $[M+Na]^+$ 922.8, $[M-H]^-$ 899.1.

$^1H$ NMR (500 MHz, $CD_2Cl_2$) δ 6.75 (d, *J* = 9.6 Hz, 1H), 6.13 (dd, *J* = 10.8, 2.9 Hz, 1H), 5.66 (dd, *J* = 10.8, 1.3 Hz, 1H), 4.54 - 4.47 (m, 1H), 4.44 (d, *J* = 9.5 Hz, 1H), 4.25 (dt, *J* = 14.0, 3.9 Hz, 1H), 4.22 - 4.17 (m, 1H), 4.09 (d, *J* = 10.4 Hz, 1H), 3.84 (dd, *J* = 10.9, 4.6 Hz, 1H), 3.61 (d, *J* = 10.1 Hz, 2H), 3.43 (d, *J* = 10.5 Hz, 1H), 3.35 - 3.23 (m, 2H), 2.94 (s, 3H), 2.82 - 2.64 (m, 3H), 2.23 - 0.63 (m, 56H) ppm.

$^{13}C$ NMR (126 MHz, $CD_2Cl_2$) δ 219.2, 184.8, 156.5, 128.0, 123.8, 106.5, 99.3, 88.9, 77.0, 76.3, 76.3, 74.7, 71.5,

70.3, 68.6, 58.7, 56.3, 54.5, 51.2, 49.8, 49.4, 42.5, 40.9, 38.9, 37.6, 36.1, 32.8, 32.6, 29.6, 28.4, 27.3, 27.0, 23.9, 20.2, 20.1, 17.6, 16.0, 15.7, 14.7, 13.2, 13.0, 12.4, 10.9, 6.8, 6.70 ppm.

Example FLC-00484-1

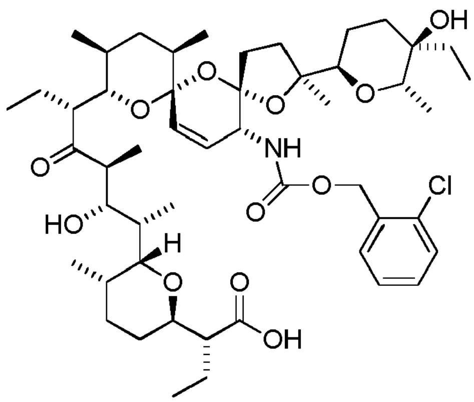

The compound was prepared according to the procedure described in example FLC-00445-1, except that 2-chlorobenzyl chloroformate (2.2 eq) was added to the reaction mixture instead of 5-chloropentanoyl chloride. The yield of the obtained product was 18%.

ESI-MS for $C_{50}H_{76}ClNO_{12}$ (m/z): $[M+Na]^+$ 941.1, $[M-H]^-$ 916.9.

$^1H$ NMR (500 MHz, $CD_2Cl_2$) δ 7.64 (d, *J* = 7.5 Hz, 1H), 7.32 (d, *J* = 7.8 Hz, 1H), 7.24 (dt, *J*= 26.5, 7.2 Hz, 2H), 6.67 (d, *J* = 9.6 Hz, 1H), 6.14 (dd, *J* = 10.8, 2.9 Hz, 1H), 5.73 (d, *J* = 10.8 Hz, 1H), 5.32 (d, *J* = 14.2 Hz, 1H), 4.98 (d, *J* = 14.2 Hz, 1H), 4.50 (d, *J* = 9.6 Hz, 1H), 4.19 (q, *J* = 6.7 Hz, 1H), 4.09 (d, *J* = 10.4 Hz, 1H), 3.84 (dd, *J* = 10.9, 4.3 Hz, 1H), 3.64 (d, *J* = 19.6 Hz, 1H), 3.43 (d, *J* = 10.8 Hz, 1H), 2.79 - 2.70 (m, 2H), 2.67 (d, *J* = 10.5 Hz, 1H), 2.25 - 0.64 (m, 58H) ppm.

$^{13}C$ NMR (126 MHz, $CD_2Cl_2$) δ 219.4, 184.8, 156.9, 135.3, 129.7, 129.3, 129.2, 128.8, 128.3, 127.3, 123.5, 106.7, 99.4, 88.9, 77.2, 76.4, 76.3, 74.6, 71.5, 70.1, 68.7, 63.6, 56.4, 51.2, 49.9, 49.5, 40.9, 39.0, 37.6, 36.2, 32.8, 32.6, 32.6, 30.1, 29.5, 28.5, 27.2, 27.1, 23.9, 20.1, 17.7, 16.1, 15.7, 14.7, 13.2, 12.6, 12.5, 11.0, 6.9, 6.8 ppm.

Example FLC-00485-1

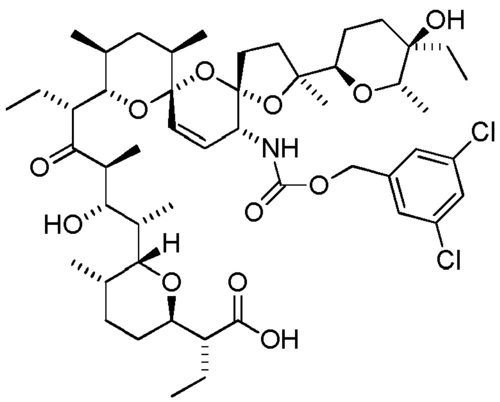

The compound was prepared according to the procedure described in example FLC-00445-1, except that 3,5-dichlorobenzyl chloroformate (2.2 eq) was added to the reaction mixture instead of 5-chloropentanoyl chloride. The yield of the obtained product was 68%.

ESI-MS for $C_{50}H_{75}Cl_2NO_{12}$ (m/z): $[M+Na]^+$ 977.2.

$^1H$ NMR (500 MHz, $CDCl_3$) δ 7.29 (d, *J* = 1.6 Hz, 2H), 7.24 (d, *J* = 1.7 Hz, 1H), 6.73 (d, *J* = 9.7 Hz, 1H), 6.11 (dd, *J* = 10.9, 3.0 Hz, 1H), 5.77 (dd, *J* = 10.8, 1.6 Hz, 1H), 5.04 (d, *J* = 13.0 Hz, 1H), 4.87 (d, *J* = 13.0 Hz, 1H), 4.60 (s, 1H), 4.52 - 4.45 (m, 1H), 4.27 (q, *J* = 6.7 Hz, 1H), 4.18 (d, *J* = 10.3 Hz, 1H), 3.91 (dd, *J* = 11.0, 4.7 Hz, 1H), 3.64 (dd, *J* = 10.1, 1.6 Hz, 1H), 3.56

(d, $J$ = 10.1 Hz, 1H), 3.35 (dd, $J$ = 12.0, 1.6 Hz, 1H), 2.80 (td, $J$ = 11.0, 3.1 Hz, 1H), 2.71 - 2.59 (m, 2H), 2.17 - 0.60 (m, 56H) ppm.

$^{13}C$ NMR (126 MHz, $CDCl_3$) δ 218.1, 185.1, 157.0, 140.6, 134.9, 127.9, 126.5, 123.2, 106.3, 98.9, 88.6, 76.5, 75.9, 75.8, 74.8, 71.5, 69.7, 68.2, 65.2, 55.9, 51.3, 49.5, 49.1, 40.6, 38.7, 37.3, 36.0, 32.5, 32.5, 32.4, 30.1, 28.1, 27.7, 26.9, 24.1, 19.9, 17.6, 16.0, 15.5, 14.8, 13.2, 12.4, 12.2, 10.8, 6.8, 6.7 ppm.

Example FLC-00497-1

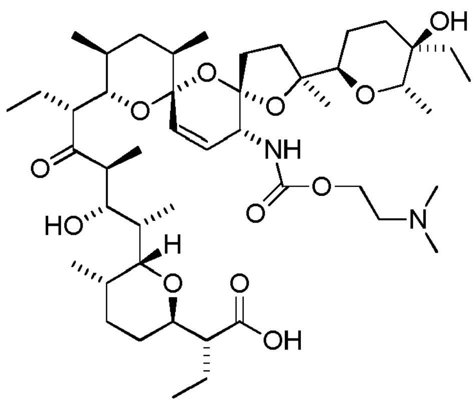

100 mg of C20-amino SAL (1 eq) was mixed with 2-(dimethylamino)ethyl imidazole-1-carboxylate (3.5 eq) in THF. The reaction was carried out at RT (TLC and LC-MS control). The post-reaction mixture was diluted with DCM and washed twice with $H_2O$. The organic layer was suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was 38%.

ESI-MS for $C_{47}H_{80}N_2O_{12}$ (*m/z*): $[M+H]^+$ 865.9, $[M-H]^-$ 864.0.

$^1H$ NMR (500 MHz, $CD_2Cl_2$) δ 6.45 (d, $J$ = 9.4 Hz, 1H), 6.10 (dd, $J$ = 10.8, 2.4 Hz, 1H), 5.66 (d, $J$ = 10.6 Hz, 1H), 4.42 (d, $J$ = 9.0 Hz, 1H), 4.23 - 4.12 (m, 2H), 4.11 - 3.99 (m, 2H), 3.87 - 3.79 (m, 1H), 3.61 (d, $J$ = 9.6 Hz, 2H), 3.40 (d, $J$ = 11.2 Hz, 1H), 2.82 - 2.68 (m, 2H), 2.60 (ddd, $J$ = 20.6, 16.3, 9.1 Hz, 3H), 2.31 (s, 6H), 2.24 - 2.14 (m, 1H), 2.04 - 0.63 (m, 56H) ppm. $^{13}C$ NMR (126 MHz, $CD_2Cl_2$) δ 219.3, 184.6, 174.9, 157.0, 128.3, 123.5, 106.7, 99.3, 88.8, 77.3, 76.3, 74.7, 71.5, 70.1, 68.7, 62.0, 57.5, 56.4, 51.2, 49.7, 49.4, 45.1, 40.9, 38.9, 37.5, 36.1, 32.8, 32.6, 32.5, 30.1, 29.6, 28.5, 27.2, 24.0, 22.1, 20.3, 20.1, 17.7, 16.1, 15.7, 14.8, 13.2, 13.0, 12.5, 11.0, 6.9, 6.7 ppm.

Example FLC-00498-1

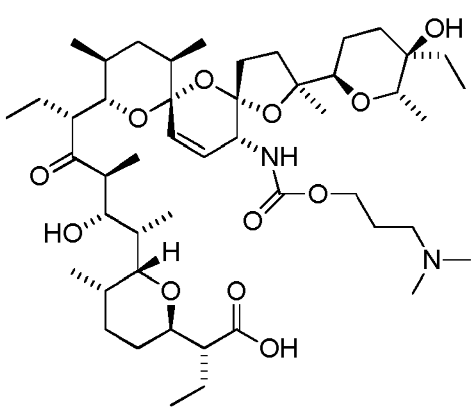

The compound was prepared according to the procedure described in example FLC-00497-1, except that 2-(dimethylamino)propyl imidazole-1-carboxylate (3.5 eq) was added to the reaction mixture instead of 2-(dimethylamino) ethyl imidazole-1-carboxylate. The yield of the obtained product was 34%.

ESI-MS for $C_{48}H_{82}N_2O_{12}$ (*m/z*): $[M+H]^+$ 880.0, $[M-H]^-$ 878.0.

$^1H$ NMR (500 MHz, $CD_2Cl_2$) δ 6.40 (d, *J* = 9.6 Hz, 1H), 6.11 (dd, *J* = 10.8, 3.1 Hz, 1H), 5.67 (dd, *J* = 10.8, 1.3 Hz, 1H), 4.91 - 4.73 (m, 1H), 4.43 (d, *J* = 9.6 Hz, 1H), 4.16 (dd, *J* = 14.2, 7.0 Hz, 1H), 4.11 - 4.02 (m, 3H), 3.90 (dt, *J* = 10.7, 6.6 Hz, 1H), 3.84 (dd, *J* = 10.9, 4.4 Hz, 1H), 3.66 - 3.60 (m, 2H), 3.47 - 3.39 (m, 2H), 2.80 - 2.71 (m, 2H), 2.65 (d, *J* = 10.6 Hz, 1H), 2.36 - 2.29 (m, 1H), 2.27 - 2.20 (m, 2H), 2.17 (s, 6H), 2.10 - 0.64 (m, 55H) ppm.

$^{13}C$ NMR (126 MHz, $CD_2Cl_2$) δ 219.4, 184.7, 157.4, 128.5, 123.4, 106.9, 99.3, 88.8, 77.2, 76.5, 76.2, 74.6, 71.5, 70.0, 68.6, 63.5, 56.4, 51.3, 49.6, 49.5, 45.5, 40.9, 38.9, 37.6, 36.2, 32.8, 32.6, 32.5, 30.1, 29.7, 28.4, 27.6, 27.1, 27.1, 24.0, 20.3, 20.0, 17.7, 16.1, 15.6, 14.8, 13.2, 12.9, 12.5, 11.0, 6.9, 6.7 ppm.

Example FLC-00509-1

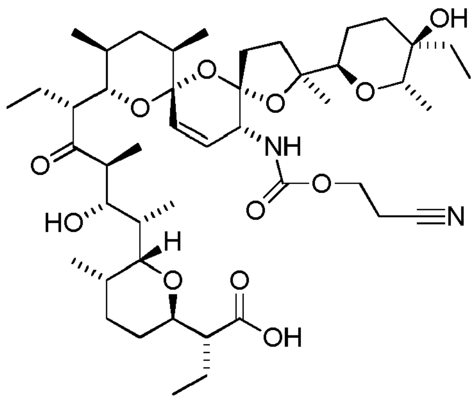

The compound was prepared according to the procedure described in example FLC-00497-1, except that 2-cyanoethyl imidazole-1-carboxylate (3.5 eq) was added to the reaction mixture instead of 2-(dimethylamino)ethyl imidazole-1-carboxylate. The yield of the obtained product was 55%.

ESI-MS for $C_{46}H_{74}N_2O_{12}$ (m/z): $[M+Na]^+$ 869.8, $[M-H]^-$ 846.2.

$^1H$ NMR (500 MHz, $CD_2Cl_2$) δ 6.85 (d, *J*= 9.5 Hz, 1H), 6.13 (dd, *J* = 10.8, 3.0 Hz, 1H), 5.66 (dd, *J* = 10.8, 1.5 Hz, 1H), 4.44 (d, *J* = 9.4 Hz, 1H), 4.30 (dt, *J* = 10.9, 7.1 Hz, 1H), 4.19 (q, *J* = 6.7 Hz, 1H), 4.09 (d, *J* = 10.5 Hz, 1H), 4.04 (dt, *J* = 10.9, 6.8 Hz, 1H), 3.83 (dd, *J* = 10.9, 4.3 Hz, 1H), 3.61 (d, *J* = 10.0 Hz, 2H), 3.41 (d, *J* = 10.5 Hz, 1H), 2.83 - 2.70 (m, 2H), 2.68 (t, *J*= 7.0 Hz, 3H), 2.19 (dt, *J* = 12.8, 9.9 Hz, 1H), 2.02 - 0.64 (m, 56H) ppm.

$^{13}C$ NMR (126 MHz, $CD_2Cl_2$) δ 219.3, 184.9, 156.4, 127.7, 123.9, 117.5, 106.7, 99.2, 88.8, 77.2, 76.3, 76.3, 75.0, 71.6, 70.1, 68.6, 59.4, 56.3, 51.2, 49.4, 40.9, 38.9, 37.8, 36.2, 32.8, 32.6, 32.6, 30.1, 29.9, 28.4, 27.4, 27.1, 24.1, 20.3, 20.2, 18.4, 17.6, 16.1, 15.7, 14.8, 13.2, 12.7, 12.5, 11.0, 6.8, 6.7 ppm.

Example FLC-00530-1

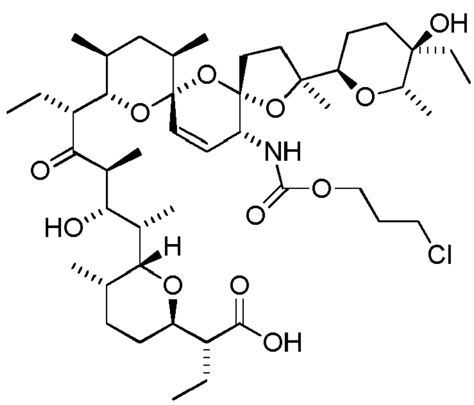

The compound was prepared according to the procedure described in example FLC-00445-1, except that 3-chloropropyl chloroformate (2.2 eq) was added to the reaction mixture instead of 5-chloropentanoyl chloride. The yield of the obtained product was 63%.

ESI-MS for $C_{46}H_{76}ClNO_{12}$ (*m/z*): $[M+Na]^+$ 892.9, $[M-H]^-$ 868.9.

$^1$H NMR (500 MHz, $CD_2Cl_2$) δ 6.45 (d, *J* = 9.6 Hz, 1H), 6.11 (dd, *J* = 10.9, 3.1 Hz, 1H), 5.66 (dd, *J* = 10.8, 1.7 Hz, 1H), 4.49 - 4.42 (m, 1H), 4.25 (ddd, *J* = 11.2, 8.3, 4.2 Hz, 1H), 4.19 (q, *J* = 6.7 Hz. 1H), 4.12 - 4.07 (m, 1H), 3.92 (dt, *J* = 10.7, 5.2 Hz, 1H), 3.83 (dd, *J* = 11.0, 4.5 Hz, 1H), 3.78 (ddd, *J* = 11.0, 7.9, 6.0 Hz, 1H), 3.73 - 3.66 (m, 1H), 3.63 - 3.53 (m, 3H), 3.41 (dd, *J* = 12.0, 1.8 Hz, 1H), 2.81 - 2.70 (m, 2H), 2.67 (d, *J* = 10.0 Hz, 1H), 2.20 - 0.65 (m, 58H) ppm.

$^{13}$C NMR (126 MHz, $CD_2Cl_2$) δ 219.2, 184.9, 157.1, 128.2, 123.6, 106.7, 99.2, 88.8, 77.2, 76.2, 75.0, 71.5, 70.0, 68.6, 61.6, 56.4, 51.3, 49.5, 49.3, 42.4, 40.9, 39.0, 37.5, 36.2, 32.8, 32.6, 32.5, 30.1, 29.6, 28.4, 27.5, 27.1, 24.2, 20.2, 20.0, 17.6, 16.1, 15.7, 14.8, 13.2, 12.8, 12.4, 10.9, 6.8, 6.7 ppm.

Example FLC-00531-1

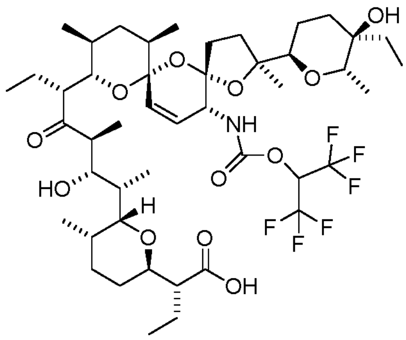

The compound was prepared according to the procedure described in Example FLC-00445-1, except that 1, 1, 1,3,3,3-hexafluoroisopropyl (2.2 eq) was added to the reaction mixture instead of 5-chloropentanoyl chloride. The yield of the obtained product was 19%.

ESI-MS for $C_{46}H_{71}F_6NO_{12}$ (*m/z*): $[M+Na]^+$ 967.2, $[M-H]^-$ 943.1.

$^1$H NMR (500 MHz, $CD_2Cl_2$) δ 7.11 (d, *J* = 8.9 Hz, 1H), 6.16 (dd, *J* = 10.9, 3.1 Hz, 1H), 5.82 - 5.76 (m, 1H), 5.74 (dd, *J* = 10.9, 1.5 Hz, 1H), 4.52 (dd, *J* = 7.0, 2.1 Hz, 1H), 4.14 (q, *J* = 6.7 Hz, 1H), 4.10 (dd, *J* = 10.5, 1.2 Hz, 1H), 3.86 (dd, *J* = 10.8, 4.4 Hz, 1H), 3.61 (dd, *J* = 10.2, 1.9 Hz, 1H), 3.44 (dd, *J* = 12.1, 2.1 Hz, 1H), 2.79 - 2.72 (m, 2H), 2.66 (d, *J* = 9.9 Hz, 1H), 2.21 - 0.66 (m, 58H) ppm.

$^{13}$C NMR (126 MHz, $CD_2Cl_2$) δ 219.5, 184.9, 153.9, 127.0, 124.1, 106.3, 99.5, 89.3, 77.5, 76.5, 74.4, 71.5, 70.0, 69.0, 56.5, 51.3, 51.1, 49.2, 40.9, 39.0, 37.6, 36.1, 32.8, 32.4, 30.1, 28.8, 28.5, 27.1, 26.9, 23.9, 20.3, 19.9, 17.7, 16.0, 15.6, 14.7, 13.2, 12.5, 11.1, 6.9, 6.7 ppm.

Example FLC-00547-1

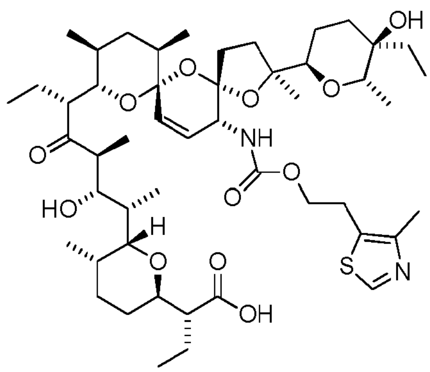

The compound was prepared according to the procedure described in example FLC-00497-1, except that 2-(4-methylthiazol)ethyl 4-nitrophenyl carbonate (3.5 eq) and additionally TEA (8 eq) were added to the reaction mixture

instead of 2-(dimethylamino)ethyl imidazole-1-carboxylate. The yield of the obtained product was 37%.

ESI-MS for $C_{49}H_{78}N_2O_{12}S$ (*m/z*): $[M+H]^+$ 920.1, $[M-H]^-$ 917.9

$^1H$ NMR (500 MHz, $CD_2Cl_2$) δ 8.53 (s, 1H), 6.61 (d, *J* = 9.6 Hz, 1H), 6.12 (dd, *J* = 10.8, 3.0 Hz, 1H), 5.67 (dd, *J* = 10.8, 1.5 Hz, 1H), 4.44 (d, *J* = 9.5 Hz, 1H), 4.17 (dt, *J* = 9.1, 5.9 Hz, 2H), 4.09 (d, *J* = 10.4 Hz, 1H), 4.02 - 3.95 (m, 1H), 3.84 (dd, *J* = 10.9, 4.4 Hz, 1H), 3.41 (dd, *J* = 12.0, 1.6 Hz, 1H), 3.11 - 2.98 (m, 2H), 2.75 (tdd, *J* = 14.4, 10.7, 5.2 Hz, 2H), 2.66 (d, *J* = 10.0 Hz, 1H), 2.36 (s, 3H), 2.27 - 0.64 (m, 59H) ppm.

$^{13}C$ NMR (126 MHz, $CD_2Cl_2$) δ 219.3, 184.8, 157.0, 150.3, 149.7, 128.2, 127.0, 123.7, 106.8, 99.3, 88.8, 77.3, 76.4, 76.3, 74.8, 71.5, 70.0, 68.6, 64.5, 56.4, 51.3, 49.6, 49.4, 40.9, 38.9, 37.7, 36.2, 32.8, 32.6, 32.6, 29.9, 28.4, 27.2, 27.1, 26.5, 24.1, 20.3, 20.1, 17.7, 16.1, 15.6, 15.0, 14.8, 13.2, 12.8, 12.5, 10.9, 6.8, 6.7 ppm.

Example FLC-00551-1

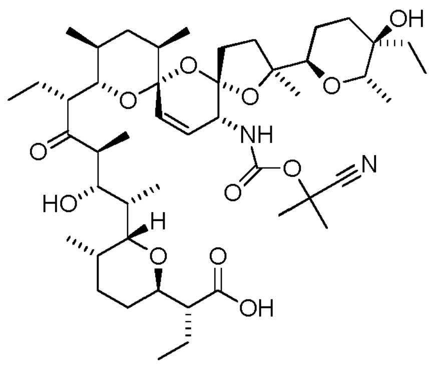

The compound was prepared according to the procedure described in example FLC-00497-1, except that 2-cyanopropan-2-yl imidazole-1-carboxylate (3.5 eq) was added to the reaction mixture instead of 2-(dimethylamino)ethyl imidazole-1-carboxylate. The yield of the obtained product was 34%.

ESI-MS for $C_{47}H_{76}N_2O_{12}$ (m/z): $[M+Na]^+$ 884.3, $[M-H]^-$ 860.2.

$^1H$ NMR (500 MHz, $CD_2Cl_2$) δ 6,69 (d, *J*= 9.5 Hz, 1H), 6.13 (dd, *J* = 10.9, 3.1 Hz, 1H), 5.67 (dd, *J* = 10.8, 1.7 Hz, 1H), 4.50 - 4.44 (m, 1H), 4.17 (q, *J* = 6.7 Hz, 1H), 4.09 (d, *J* = 9.7 Hz, 1H), 3.85 (dd, *J* = 11.0, 4.6 Hz, 1H), 3.64 - 3.59 (m, 2H), 3.43 (dd, *J* = 12.1, 1.8 Hz, 1H), 2.82 - 2.70 (m, 2H), 2.67 (d, *J* = 9.9 Hz, 1H), 2.23 (dt, *J* = 13.0, 9.8 Hz, 1H), 2.07 - 0.65 (m, 62H) ppm.

$^{13}C$ NMR (126 MHz, $CD_2Cl_2$) δ 219.3, 184.9, 154.8, 127.5, 123.9, 120.7, 106.7, 99.2, 88.9, 77.3, 76.3, 76.2, 74.8, 71.5, 70.0, 68.8, 68.7, 56.4, 51.1, 49.4, 49.4, 40.9, 38.9, 37.8, 36.2, 32.8, 32.6, 32.6, 30.1, 29.6, 28.4, 27.6, 27.4, 27.4, 27.1, 24.0, 20.4, 20.2, 17.7, 16.0, 15.6, 14.8, 13.4, 13.2, 12.5, 10.9, 6.8, 6.7 ppm.

Example FLC-00552-1

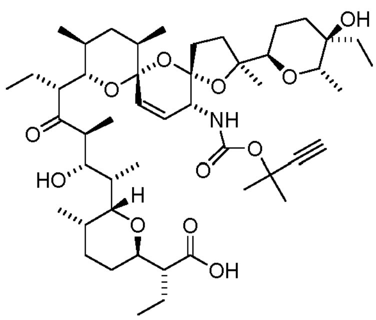

The compound was prepared according to the procedure described in example FLC-00497-1, except that 2-methylbut-3-yn-2-yl imidazole-1-carboxylate (3.5 eq) was added to the reaction mixture instead of 2-(dimethylamino)ethyl imidazole-1-carboxylate. The yield of the obtained product was 23%.

ESI-MS for $C_{48}H_{77}NO_{12}$ (m/z): $[M+Na]^+$ 883.1, $[M-H]^-$ 858.9.

$^1$H NMR (500 MHz, $CD_2Cl_2$) δ 6.24 (d, *J* = 9.6 Hz, 1H), 6.11 (dd, *J* = 10.9, 3.1 Hz, 1H), 5.68 (dd, *J*= 10.8, 1.6 Hz, 1H), 4.48 - 4.40 (m, 1H), 4.14 (q, *J* = 6.8 Hz, 1H), 4.08 (dd, *J* = 10.4, 1.1 Hz, 1H), 3.86 (dd, *J* = 10.9, 4.5 Hz, 1H), 3.66 - 3.61 (m, 2H), 3.43 (dd, *J* = 12.2, 2.0 Hz, 1H), 2.81 - 2.69 (m, 2H), 2.65 (d, *J* = 9.7 Hz, 1H), 2.45 (s, 1H), 2.26 (dt, *J* = 13.1, 9.4 Hz, 1H), 2.16 - 0.64 (m, 62H) ppm.

$^{13}$C NMR (126 MHz, $CDCl_3$) δ 221.4, 186.7, 157.3, 130.3, 125.5, 108.9, 101.3, 90.9, 79.3, 78.5, 78.2, 76.6, 73.5, 73.5, 73.2, 72.0, 70.7, 69.4, 58.5, 53.2, 51.5, 51.4, 42.9, 41.0, 39.6, 38.1, 34.8, 34.6, 34.6, 32.1, 31.7, 31.5, 31.3, 30.5, 29.2, 29.0, 26.0, 22.4, 22.3, 19.7, 18.1, 17.6, 16.7, 15.3, 15.2, 14.5, 13.0, 8.9, 8.7 ppm.

Example FLC-00559-1

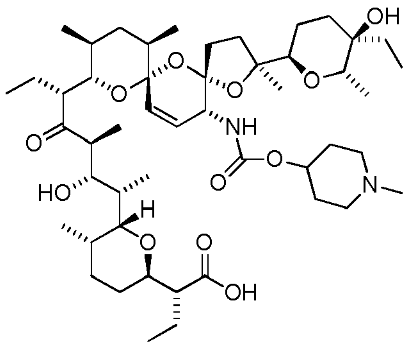

The compound was prepared according to the procedure described in example FLC-00497-1, except that 1-methylpiperidin-4-yl 4-nitrophenyl carbonate (3.5 eq) and additionally TEA (8 eq) were added to the reaction mixture instead of 2-(dimethylamino)ethyl imidazole-1-carboxylate. The yield of the obtained product was 70%.

ESI-MS for $C_{49}H_{82}N_2O_{13}$ (m/z): $[M+H]^+$ 892.1, $[M-H]^-$ 890.0.

$^1$H NMR (500 MHz, $CD_2Cl_2$) δ 6.24 (d, *J* = 9.6 Hz, 1H), 6.11 (dd, *J* = 10.8, 3.1 Hz, 1H), 5.68 (dd, *J* = 10.8, 1.5 Hz, 1H), 4.80 (d, *J* = 3.4 Hz, 1H), 4.56 (s, 1H), 4.47 - 4.43 (m, 1H), 4.17 (q, *J* = 6.7 Hz, 1H), 4.08 (d, *J* = 10.2 Hz, 1H), 3.85 (dd, *J* = 10.9, 4.4 Hz, 1H), 3.42 (dd, *J* = 12.1, 1.8 Hz, 1H), 2.79 - 2.68 (m, 4H), 2.66 (d, *J* = 9.9 Hz, 1H), 2.24 (s, 3H), 2.23 - 0.64 (m, 64H) ppm.

$^{13}$C NMR (126 MHz, $CD_2Cl_2$) δ 219.3, 184.7, 156.7, 128.4, 123.4, 106.9, 99.4, 88.8, 77.2, 76.4, 76.2, 74.6, 71.5, 70.0, 68.7, 56.4, 51.2, 49.6, 49.5, 46.0, 40.9, 39.0, 37.5, 36.2, 32.8, 32.6, 32.6, 31.4, 31.2, 29.7, 28.4, 27.1, 24.0, 20.3, 20.1, 17.7, 16.1, 15.6, 14.7, 13.5, 13.2, 12.4, 11.0, 6.8, 6.73 ppm.

Example FLC-00570-1

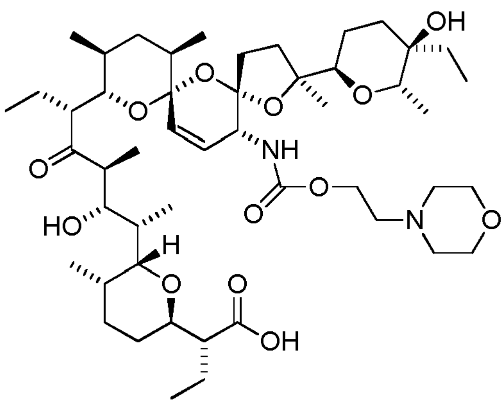

The compound was prepared according to the procedure described in example FLC-00497-1, except that 2-morpholinoethyl 4-nitrophenyl carbonate (3.5 eq) and additionally TEA (8 eq) were added to the reaction mixture instead of 2-(dimethylamino)ethyl imidazole-1-carboxylate.

The yield of the obtained product was 38%.

ESI-MS for $C_{49}H_{82}N_2O_{13}$ (m/z): $[M+H]^+$ 908.6, $[M-H]^-$ 905.9.

$^1$H NMR (500 MHz, $CDCl_3$) δ 6.54 - 6.47 (m, *J* = 9.7 Hz, 1H), 6.10 (dd, *J* = 10.9, 3.0 Hz, 1H), 5.75 (dd, *J* = 10.8, 1.5 Hz, 1H), 4.62 (s, 1H), 4.47 (d, *J* = 9.6 Hz, 1H), 4.24 (dd, *J* = 17.4, 6.8 Hz, 2H), 4.18 (d, *J* = 10.3 Hz, 1H), 4.00 (dt, *J* = 11.1, 6.6 Hz, 1H), 3.91 (dd, *J* = 10.9, 4.5 Hz, 1H), 3.67 (t, *J* = 4.6 Hz, 4H), 3.64 (dd, *J* = 9.4, 1.2 Hz, 1H), 3.36 (d, *J* = 10.6 Hz, 1H), 2.82 (td, *J* = 11.0, 3.1 Hz, 1H), 2.70 - 2.60 (m, 2H), 2.56 (t, *J* = 6.7 Hz, 2H), 2.46 (d, *J* = 4.7 Hz, 4H), 2.23 - 0.60 (m, 57H) ppm.

$^{13}$C NMR (126 MHz, $CDCl_3$) δ 218.1, 184.9, 157.3, 128.1, 123.1, 106.4, 98.9, 88.5, 76.8, 75.9, 75.8, 74.8, 71.5, 69.6, 68.2, 67.2, 61.9, 57.5, 55.9, 54.0, 51.3, 49.5, 49.0, 40.6, 38.7, 37.2, 36.0, 32.5, 30.0, 28.1, 27.6, 26.9, 24.1, 20.0, 19.9, 17.6, 16.0, 15.5, 14.8, 13.2, 12.6, 12.2, 10.8, 6.8, 6.8 ppm.

Example FLC-00571-1

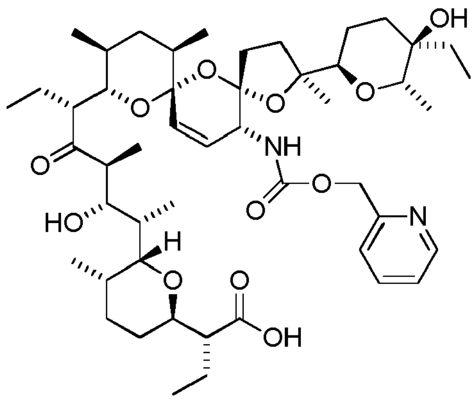

The compound was prepared according to the procedure described in Example FLC-00539-1, except that 2-hydroxymethylpiridine (10 eq) was added to the reaction mixture instead of 3-methoxypropylamine. The yield of the obtained product was 27%.

ESI-MS for $C_{49}H_{76}N_2O_{12}$ (m/z): $[M+H]^+$ 885.8, $[M+Na]^+$ 908.0, $[M-H]^-$ 883.9.

$^1$H NMR (500 MHz, CDCl3) δ 8.47 (d, *J* = 4.4 Hz, 1H), 7.72 (d, *J* = 7.9 Hz, 1H), 7.63 (td, *J* = 7.7, 1.4 Hz, 1H), 7.12 (dd, *J* = 6.8, 5.4 Hz, 1H), 6.69 (d, *J* = 9.7 Hz, 1H), 6.11 (dd, *J* = 10.9, 2.9 Hz, 1H), 5.81 (dd, *J* = 10.8, 1.3 Hz, 1H), 5.42 (d, *J* = 14.6 Hz, 1H), 4.98 (d, *J* = 14.6 Hz, 1H), 4.62 (d, *J* = 36.6 Hz, 2H), 4.54 (d, *J* = 9.7 Hz, 1H), 4.28 (q, *J* = 6.5 Hz, 1H), 4.18 (d, *J* = 10.3 Hz, 1H), 3.91 (dd, *J* = 10.9, 4.4 Hz, 1H), 3.64 (d, *J* = 10.0 Hz, 1H), 3.57 (d, *J* = 10.1 Hz, 1H), 3.35 (dd, *J* = 19.0, 9.0 Hz, 1H), 3.10 (dd, *J* = 13.2, 6.6 Hz, 3H), 2.81 (td, *J* = 11.0, 2.9 Hz, 1H), 2.72 - 2.57 (m, 2H), 2.20 - 0.62 (m, 52H) ppm.

$^{13}$C NMR (126 MHz, $CDCl_3$) δ 218.1, 185.1, 157.4, 157.0, 148.7, 136.8, 128.1, 123.2, 122.2, 121.4, 106.3, 99.0, 88.5, 76.6, 75.8, 74.9, 71.5, 69.7, 68.2, 66.9, 55.9, 51.3, 49.5, 49.1, 42.4, 40.6, 38.7, 37.1, 36.0, 32.6, 32.5, 32.4, 29.9, 28.0, 27.8, 26.9, 24.0, 23.6, 20.0, 19.9, 17.6, 16.0, 15.5, 14.8, 13.2, 12.3, 12.1, 11.5, 10.8, 6.8 ppm.

Example FLC-00577-1

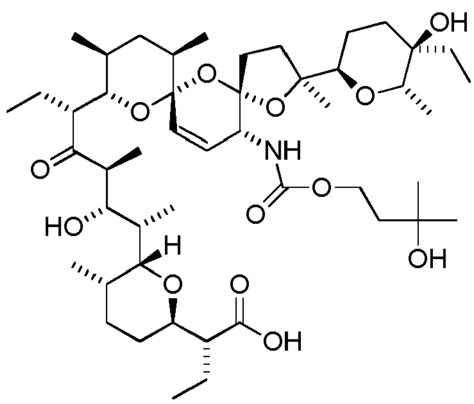

The compound was prepared according to the procedure described in Example FLC-00445-1, except that 3-hydroxy-3-methylbutyl (2.2 eq) was added to the reaction mixture instead of 5-chloropentanoyl chloride. The yield of the obtained product was 49%.

ESI-MS for $C_{48}H_{81}NO_{13}$ (*m/z*): $[M+Na]^+$ 903.0, $[M-H]^-$ 879.2.

$^1H$ NMR (500 MHz, $CDCl_3$) δ 6.48 (d, *J* = 9.7 Hz, 1H), 6.09 (dd, *J* = 10.8, 2.7 Hz, 1H), 5.75 (d, *J* = 10.8 Hz, 1H), 4.68 (s, 1H), 4.47 (d, *J* = 9.6 Hz, 1H), 4.36 - 4.24 (m, 2H), 4.17 (d, *J* = 10.2 Hz, 1H), 4.05 - 3.96 (m, 1H), 3.92 (dd, *J* = 10.8, 4.2 Hz, 1H), 3.65 (d, *J* = 9.9 Hz, 1H), 3.55 (d, *J* = 10.0 Hz, 1H), 3.35 (d, *J* = 11.3 Hz, 1H), 2.84 (td, *J* = 10.8, 2.7 Hz, 1H), 2.65 (dt, *J* = 20.1, 8.5 Hz, 2H), 2.33 - 0.61 (m, 65H) ppm.

$^{13}C$ NMR (126 MHz, $CDCl_3$) δ 218.0, 184.9, 157.7, 128.4, 123.0, 106.3, 98.9, 88.4, 76.4, 75.9, 75.7, 74.9, 71.4, 70.0, 69.5, 68.1, 61.7, 55.9, 51.1, 49.6, 48.8, 42.2, 40.6, 38.7, 37.2, 35.9, 32.5, 32.4, 30.0, 29.8, 29.6, 28.1, 27.8, 26.9, 24.0, 20.0, 17.6, 16.0, 15.5, 14.8, 13.2, 12.8, 12.2, 10.8, 6.8, 6.7 ppm.

Example FLC-00580-1

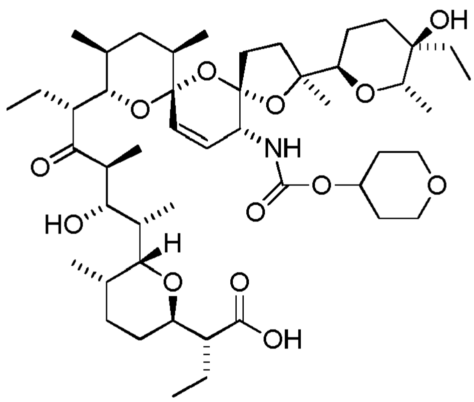

The compound was prepared according to the procedure described in Example FLC-00445-1, except that tetrahydro-2H-piran-4-yl chloroformate (2.2 eq) was added to the reaction mixture instead of 5-chloropentanoyl chloride. The yield of the obtained product was 50%.

ESI-MS for $C_{48}H_{79}NO_{13}$ (*m/z*): $[M+Na]^+$ 901.1, $[M-H]^-$ 877.1.

$^1H$ NMR (500 MHz, $CDCl_3$) δ 6.33 (d, *J* = 9.7 Hz, 1H), 6.10 (dd, *J* = 10.9, 2.9 Hz, 1H), 5.77 (d, *J*= 10.9 Hz, 1H), 4.76 (ddd, *J* = 13.2, 8.8, 4.0 Hz, 1H), 4.69 (s, 1H), 4.57 (s, 1H), 4.49 (d, *J* = 9.6 Hz, 1H), 4.24 (q, *J* = 6.5 Hz, 1H), 4.19 (d, *J* = 10.2 Hz, 1H), 3.97 - 3.86 (m, 3H), 3.63 (d, *J* = 9.9 Hz, 1H), 3.56 (d, *J*= 10.1 Hz, 1H), 3.43 (t, *J*= 10.5 Hz, 2H), 3.36 (d, *J* = 10.9 Hz, 1H), 2.83 (td, *J*= 10.9, 2.9 Hz, 1H), 2.65 (dt, *J* = 20.6, 9.8 Hz, 2H), 2.18 - 0.61 (m, 59H) ppm.

$^{13}C$ NMR (126 MHz, $CDCl_3$) δ 217.8, 184.9, 156.7, 128.0, 123.0, 106.4, 98.9, 88.4, 76.7, 75.7, 75.7, 74.7, 71.4, 69.8, 69.4, 68.1, 65.7, 55.8, 51.3, 49.4, 48.7, 40.5, 38.7, 36.9, 35.9, 32.4, 32.4, 32.2, 32.2, 29.6, 28.0, 27.6, 26.8, 24.1, 19.9, 19.8, 17.5, 15.9, 15.4, 14.6, 13.1, 13.0, 12.1, 10.7, 6.7, 6.6 ppm.

Example FLC-00604-1

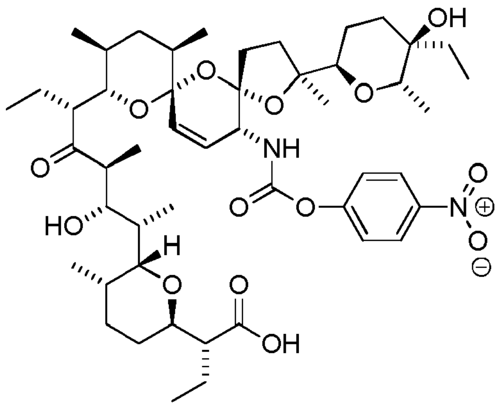

100 mg C20-amino-SAL (1 eq) was mixed with TEA (10 eq) in DCM, cooled in an ice/water bath and *p*-nitrophenyl chloroformate (1.5 eq) was added in DCM. The bath was then removed and the reaction continued at RT (TLC and LC-MS control). The post-reaction mixture was diluted with DCM, washed twice with $H_2O$ and used in the next step after removing the solvent. ~70% yield.

ESI-MS for $C_{49}H_{74}N_2O_{14}$(*m/z*): $[M+Na]^+$ 938.0, $[M-H]^-$ 914.0.

$^1H$ NMR (500 MHz, $CD_2Cl_2$) δ 8.19 - 8.13 (m, 2H), 7.52 (d, J = 9.4 Hz, 1H), 7.48 - 7.42 (m, 2H), 6.19 (dd, J = 10.8, 3.0 Hz, 1H), 5.75 (dd, J = 10.8, 1.8 Hz, 1H), 4.54 - 4.49 (m, 1H), 4.44 (d, J = 3.8 Hz, 1H), 4.23 (q, J = 6.7 Hz, 1H), 4.13 (d, J = 10.8 Hz, 1H), 3.82 (dd, J = 11.0, 4.6 Hz, 1H), 3.60 (d, J = 6.8 Hz, 2H), 3.44 - 3.39 (m, 1H), 2.83 - 2.65 (m, 3H), 2.19 - 0.69 (m, 55H) ppm.

$^{13}C$ NMR (126 MHz, $CD_2Cl_2$) δ 218.9, 185.0, 156.4, 153.8, 144.5, 126.6, 124.6, 124.1, 122.6, 106.2, 98.7, 88.5, 77.1, 76.0, 75.7, 74.9, 71.2, 69.8, 68.2, 56.0, 51.0, 49.0, 49.0, 40.4, 38.4, 37.7, 35.9, 32.4, 32.3, 29.6, 28.0, 27.3, 26.7, 23.9, 19.9, 19.8, 17.2, 15.7, 15.2, 14.6, 12.8, 12.3, 12.1, 10.5, 6.4, 6.3 ppm.

Example FLC-00468-1

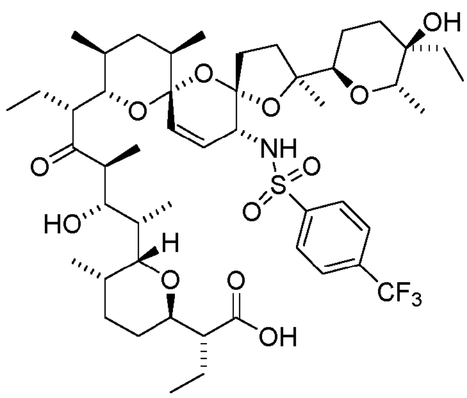

100 mg C20-amino SAL (1 eq) in MeCN with 0.5 M $Na_2CO_3$ (5 eq) was cooled in an ice/water bath and 4-trifluoromethylbenzenesulfonyl chloride (1.5 eq) in THF was added dropwise. Afterwards, the reaction was conducted at RT (TLC and LC-MS control). The post-reaction mixture was diluted with $H_2O$ and extracted twice with DCM. The organic layers were combined, suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was 66%.

ESI-MS for $C_{49}H_{74}F_3NO_{12}S$ (*m/z*): $[M+Na]^+$ 981.0, $[M-H]^-$ 957.0.

$^1H$ NMR (700 MHz, $CD_2Cl_2$) δ 8.13 (d, J = 8.2 Hz, 2H), 7.83 (d, J = 8.3 Hz, 2H), 7.43 (s, 1H), 6.03 (dd, J = 10.9, 3.0 Hz, 1H), 5.30 (dd, J = 10.9, 1.7 Hz, 1H), 4.27 (q, J = 6.5 Hz, 1H), 4.17 (dd, J = 10.4, 1.3 Hz, 1H), 4.03 (s, 1H), 3.89 (dd, J = 10.9, 5.2 Hz, 1H), 3.64 (dd, J = 10.2, 2.1 Hz, 1H), 3.55 (d, J = 10.1 Hz, 1H), 3.46 (dd, J = 12.1, 1.7 Hz, 1H), 2.89 (td, J = 10.7, 3.3 Hz, 1H), 2.77 - 2.68 (m, 2H), 2.36 - 2.29 (m, 1H), 2.28 - 2.21 (m, 1H), 1.99 - 0.69 (m, 54H) ppm. $^{13}C$ NMR (176 MHz, $CD_2Cl_2$) δ 218.6, 184.9, 145.7, 133.5, 127.6, 126.3, 126.1, 126.0, 124.3, 122.8, 105.7, 98.9, 88.9, 77.0, 76.1, 75.7, 75.0, 71.3, 70.0, 68.3, 55.8, 51.6, 51.1, 48.9, 40.5, 38.4, 36.2, 35.8, 32.4, 32.2, 32.1, 29.7, 28.0, 27.4, 26.7, 24.0, 20.0, 19.9, 17.2,

15.5, 15.3, 14.6, 12.7, 12.7, 12.0, 10.5, 6.3, 6.2 ppm.

Example FLC-00502-1

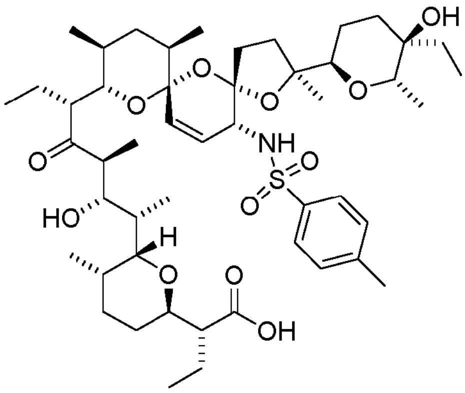

The compound was prepared according to the procedure described in Example FLC-00468-1, except that 4-methylbenzenesulfonyl chloride (1.5 eq) was added to the reaction mixture instead of 4-trifluoromethylbenzenesulfonyl chloride. The yield of the obtained product was 70%.

ESI-MS for $C_{49}H_{77}NO_{12}S$ (*m/z*): $[M+Na]^+$ 926.8, $[M-H]^-$ 903.0.

$^1H$ NMR (500 MHz, $CD_2Cl_2$) δ 7.78 (d, J = 8.2 Hz, 2H), 7.31 (d, J = 8.1 Hz, 2H), 6.76 (d, J = 9.9 Hz, 1H), 5.94 (dd, J = 10.9, 2.9 Hz, 1H), 5.21 (dd, J = 10.8, 1.4 Hz, 1H), 4.22 (d, J = 6.8 Hz, 1H), 4.10 (d, J = 10.4 Hz, 1H), 3.92 (d, J = 9.8 Hz, 1H), 3.84 (dd, J = 11.0, 4.8 Hz, 1H), 3.58 (dd, J = 10.1, 1.7 Hz, 1H), 3.50 (d, J = 10.1 Hz, 1H), 3.44 - 3.37 (m, 1H), 2.83 (td, J = 10.7, 3.3 Hz, 1H), 2.69 - 2.62 (m, 2H), 2.42 (s, 3H), 2.31 - 2.22 (m, 2H), 1.92 - 0.63 (m, 55H) ppm.

$^{13}C$ NMR (126 MHz, $CD_2Cl_2$) δ 218.6, 184.8, 143.0, 139.0, 129.5, 127.0, 126.6, 123.8, 105.7, 98.8, 88.7, 76.9, 75.9, 75.7, 75.0, 71.2, 69.8, 68.2, 55.8, 51.4, 51.0, 49.0, 40.4, 38.5, 36.2, 35.8, 32.4, 32.2, 32.2, 29.8, 29.7, 28.0, 27.4, 26.7, 24.0, 21.3, 20.0, 19.8, 17.2, 15.5, 15.3, 14.6, 12.8, 12.0, 10.5, 6.3, 6.3 ppm.

Example FLC-00503-1

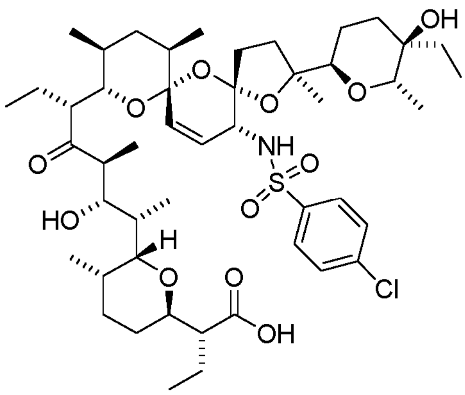

The compound was prepared according to the procedure described in example FLC-00468-1, except that 4-chlorobenzenesulfonyl chloride (1.5 eq) was added to the reaction mixture instead of 4-trifluoromethylbenzenesulfonyl chloride. The yield of the obtained product was 81%. ESI-MS for $C_{48}H_{74}ClNO_{12}S$ (*m/z*): $[M+Na]^+$ 946.9, $[M-H]^-$ 922.8.

$^1H$ NMR (500 MHz, $CD_2Cl_2$) δ 7.88 (d, J = 8.6 Hz, 2H), 7.49 (d, J = 8.6 Hz, 2H), 7.04 (d, J = 9.8 Hz, 1H), 5.97 (dd, J = 10.9, 2.9 Hz, 1H), 5.23 (dd, J = 10.8, 1.6 Hz, 1H), 4.22 (q, J = 6.7 Hz, 1H), 4.10 (d, J = 10.4 Hz, 1H), 3.94 (d, J = 9.2 Hz, 1H), 3.83 (dd, J = 11.0, 4.9 Hz, 1H), 3.57 (dd, J = 10.2, 1.8 Hz, 1H), 3.50 (d, J = 10.1 Hz, 1H), 3.42 - 3.37 (m, 1H), 2.82 (td, J = 10.7, 3.2 Hz, 1H), 2.72 - 2.59 (m, 2H), 2.28 - 2.16 (m, 2H), 1.63 - 0.94 (m, 54H) ppm.

$^{13}C$ NMR (126 MHz, $CD_2Cl_2$) δ 218.6, 184.8, 140.6, 138.4, 129.1, 128.6, 126.2, 124.1, 105.6, 98.8, 88.8, 76.9, 76.1, 75.7, 75.0, 71.2, 69.8, 68.3, 55.8, 51.4, 51.0, 48.9, 40.4, 38.4, 36.3, 35.8, 32.4, 32.2, 32.1, 29.8, 29.7, 28.0, 27.4, 26.7,

24.1, 20.0, 19.8, 17.2, 15.6, 15.2, 14.6, 12.8, 12.0, 10.5, 6.3, 6.3 ppm.

Example FLC-00504-1

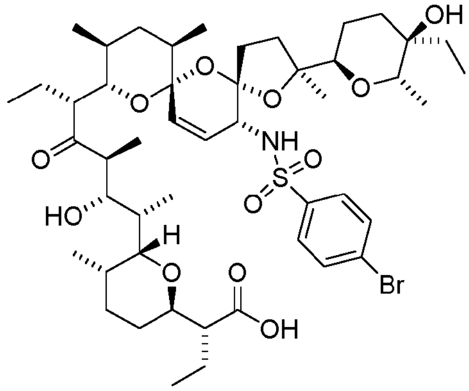

The compound was prepared according to the procedure described in example FLC-00468-1, except that 4-bromobenzenesulfonyl chloride (1.5 eq) was added to the reaction mixture instead of 4-trifluoromethylbenzenesulfonyl chloride. The yield of the obtained product was 60%. ESI-MS for $C_{48}H_{74}BrNO_{12}S$ (*m/z*): $[M+Na]^+$ 990.8, $[M-H]^-$ 967.0.

$^1H$ NMR (500 MHz, $CD_2Cl_2$) δ 7.81 (d, J = 8.6 Hz, 2H), 7.65 (d, J = 8.6 Hz, 2H), 7.07 (d, J = 9.6 Hz, 1H), 5.97 (dd, J = 10.9, 2.9 Hz, 1H), 5.23 (dd, J = 10.8, 1.6 Hz, 1H), 4.22 (q, J = 6.7 Hz, 1H), 4.10 (d, J = 10.5 Hz, 1H), 3.94 (d, J = 9.0 Hz, 1H), 3.83 (dd, J = 11.0, 4.9 Hz, 1H), 3.57 (dd, J = 10.2, 1.8 Hz, 1H), 3.50 (d, J = 10.1 Hz, 1H), 3.43 - 3.38 (m, 1H), 2.82 (td, J = 10.7, 3.2 Hz, 1H), 2.70 - 2.62 (m, 2H), 2.27 - 2.18 (m, 2H), 1.93 - 0.63 (m, 54H) ppm.

$^{13}C$ NMR (126 MHz, $CD_2Cl_2$) δ 218.6, 184.8, 141.1, 132.1, 128.7, 126.9, 126.2, 124.1, 105.6, 98.8, 88.8, 76.9, 76.1, 75.7, 75.1, 71.2, 69.8, 68.3, 55.8, 51.4, 51.0, 48.9, 40.4, 38.4, 36.3, 35.8, 32.4, 32.2, 32.1, 29.82, 29.7, 28.0, 27.4, 26.7, 24.1, 20.0, 19.8, 17.2, 15.6, 15.3, 14.6, 12.8, 12.0, 10.5, 6.3, 6.3 ppm.

Example FLC-00505-1

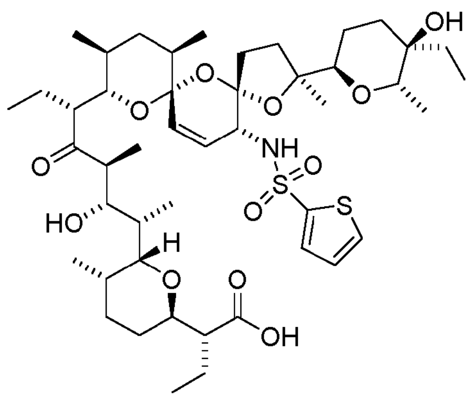

The compound was prepared according to the procedure described in Example FLC-00468-1, except that thiophenesulfonyl chloride (3 eq) was added to the reaction mixture instead of 4-trifluoromethylbenzenesulfonyl chloride. The yield of the obtained product was 53%. ESI-MS for $C_{46}H_{73}NO_{12}S_2$ (*m/z*): $[M+Na]^+$ 919.1, $[M-H]^-$ 894.8.

$_1H$ NMR (500 MHz, $CD_2Cl_2$) δ 7.63 - 7.57 (m, 2H), 7.12 - 7.08 (m, 2H), 5.98 (dd, J = 10.9, 3.0 Hz, 1H), 5.25 (dd, J = 10.8, 1.6 Hz, 1H), 4.21 (q, J = 6.7 Hz, 1H), 4.09 (d, J = 10.4 Hz, 1H), 4.00 (d, J = 3.9 Hz, 1H), 3.83 (dd, J = 11.0, 4.6 Hz, 1H), 3.57 (dd, J = 10.2, 1.8 Hz, 1H), 3.51 (d, J = 10.2 Hz, 1H), 3.44 - 3.39 (m, 1H), 2.82 (td, J = 10.7, 3.3 Hz, 1H), 2.70 - 2.62 (m, 2H), 2.34 - 2.22 (m, 2H), 1.93 - 0.65 (m, 54H) ppm.

$_{13}C$ NMR (126 MHz, $CD_2Cl_2$) δ 218.6, 184.9, 142.9, 131.6, 131.3, 127.4, 126.3, 123.0, 105.7, 98.8, 88.8, 76.9, 76.0, 75.7, 75.0, 71.1, 69.9, 68.2, 55.8, 51.8, 50,9, 49.0, 40.4, 38.5, 36.3, 35.8, 32.4, 32.1, 29.8, 29.7, 28.0, 27.3, 26.7, 24.0, 19.9, 19.8, 17.2, 15.5, 15.3, 14.6, 12.8, 12.8, 12.0, 10.5, 6.4, 6.3 ppm.

Example FLC-00506-1

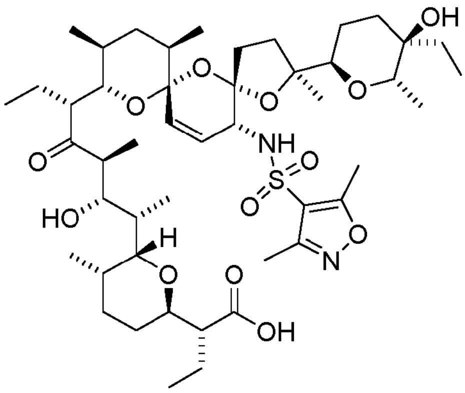

The compound was prepared according to the procedure described in example FLC-00468-1, except that 4-(3,5-dimethylisoxazole)sulfonyl chloride (5 eq) was added to the reaction mixture instead of 4-trifluoromethyl benzenesulfonyl chloride. The yield of the obtained product was 40%.

ESI-MS for $C_{47}H_{76}N_2O_{13}S$ (*m/z*): $[M+Na]^+$ 932.2, $[M-H]^-$ 908.0.

$^1H$ NMR (500 MHz, $CD_2Cl_2$) δ 7.23 (d, J = 9.8 Hz, 1H), 6.05 (dd, J = 10.8, 3.0 Hz, 1H), 5.28 (dd, J= 10.8, 1.5 Hz, 1H), 4.24 (q, J = 6.7 Hz, 1H), 4.11 (d, J = 10.4 Hz, 1H), 3.77 (dd, J = 18.1, 7.0 Hz, 2H), 3.52 (dd, J = 16.6, 6.2 Hz, 2H), 3.41 - 3.37 (m, 1H), 2.75 (t, J = 10.6 Hz, 1H), 2.67 (t, J = 11.0 Hz, 2H), 2.60 (s, 3H), 2.51 (s, 3H), 2.51 - 0.65 (m, 57H) ppm.

$^{13}C$ NMR (126 MHz, $CD_2Cl_2$) δ 218.5, 185.0, 172.6, 157.9, 126.1, 125.0, 117.3, 105.5, 98.9, 88.9, 76.9, 76.2, 75.4, 75.2, 71.3, 69.9, 68.3, 55.8, 51.0, 50.9, 48.8, 40.4, 38.4, 36.1, 35.9, 32.4, 32.2, 32.1, 30.0, 29.7, 27.9, 27.7, 26.7, 24.3, 20.1, 19.9, 17.1, 15.4, 15.3, 14.7, 12.8, 12.6, 12.4, 12.1, 11.1, 10.4, 6.2 ppm.

Example FLC-00514-1

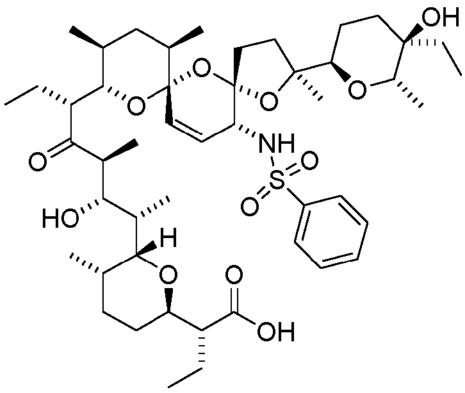

The compound was prepared according to the procedure described in example FLC-00468-1, except that benzenesulfonyl chloride (1.5 eq) was added to the reaction mixture instead of 4-trifluoromethylbenzenesulfonyl chloride. The yield of the obtained product was 73%. ESI-MS for $C_{48}H_{75}NO_{12}S$ (*m/z*): $[M+Na]^+$ 912.9, $[M-H]^-$ 889.0.

$^1H$ NMR (500 MHz, $CD_2Cl_2$) δ 7.91 (d, J = 7.4 Hz, 2H), 7.59 - 7.49 (m, 3H), 6.97 (d, J = 9.9 Hz, 1H), 5.94 (dd, J = 10.9, 2.9 Hz, 1H), 5.21 (dd, J = 10.8, 1.3 Hz, 1H), 4.22 (q, J = 6.6 Hz, 1H), 4.10 (d, J = 10.3 Hz, 1H), 3.94 (d, J = 9.5 Hz, 1H), 3.84 (dd, J = 11.0, 4.7 Hz, 1H), 3.58 (dd, J = 10.1, 1.6 Hz, 1H), 3.49 (d, J = 10.1 Hz, 1H), 3.41 (d, J = 10.7 Hz, 1H), 2.84 (td, J = 10.7, 3.2 Hz, 1H), 2.70 - 2.62 (m, 2H), 2.28 - 2.21 (m, 2H), 1.93 - 0.63 (m, 54H) ppm.

$^{13}C$ NMR (126 MHz, $CD_2Cl_2$) δ 218.6, 184.8, 141.9, 132.2, 128.9, 127.0, 126.6, 123.9, 105.7, 98.8, 88.7, 76.9, 76.0, 75.7, 75.0, 71.2, 69.9, 68.2, 55.8, 51.5, 51.0, 49.0, 40.4, 38.4, 36.2, 35.8, 32.4, 32.1, 29.7, 28.0, 27.4, 26.7, 24.0, 19.9, 19.8, 17.2, 15.5, 15.3, 14.6, 12.8, 12.7, 12.0, 10.5, 6.3, 6.3 ppm.

Example FLC-00536-1

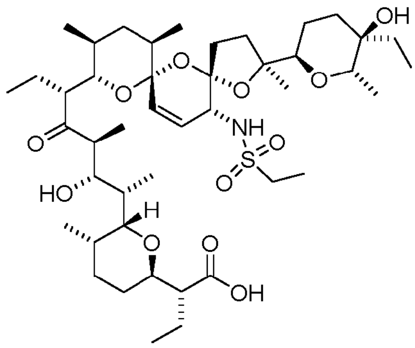

100 mg C20-amino SAL (1 eq) in DCM with TEA (10 eq) was cooled in an ice/water bath and ethanesulfonyl chloride (3 eq) in DCM was added dropwise. Afterwards, the reaction was conducted at RT (TLC and LC-MS control). The post-reaction mixture was diluted with $H_2O$ and extracted twice with DCM. The organic layers were combined, suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was 22%.

ESI-MS for $C_{44}H_{75}NO_{12}S$ (*m/z*): $[M+Na]^+$865.0, $[M-H]^-$ 840.9.

$^1$H NMR (500 MHz, $CD_2Cl_2$) δ 6.33 (d, J = 9.8 Hz, 1H), 6.13 (dd, J = 10.8, 3.0 Hz, 1H), 5.79 (dd, J= 10.8, 1.3 Hz, 1H), 4.19 (q, J = 6.7 Hz, 1H), 4.13 - 4.04 (m, 2H), 3.82 (dd, J = 11.0, 4.3 Hz, 1H), 3.63 - 3.56 (m, 2H), 3.43 (dd, J = 12.2, 1.9 Hz, 1H), 3.00 (dq, J = 14.0, 7.0 Hz, 2H), 2.82 - 2.63 (m, 3H), 2.36 - 2.21 (m, 2H), 2.00 - 0.68 (m, 57H) ppm.

$^{13}$C NMR (126 MHz, $CD_2Cl_2$) δ 218.8, 184.6, 127.7, 124.0, 105.9, 98.9, 88.8, 76.7, 76.0, 75.8, 74.6, 71.2, 69.9, 68.3, 55.9, 51.0, 50.8, 49.0, 48.1, 40.5, 38.5, 36.3, 35.8, 32.4, 32.2, 32.1, 29.7, 29.3, 28.0, 27.2, 26.6, 23.7, 19.9, 19.9, 17.2, 15.6, 15.3, 14.5, 12.8, 12.5, 12.1, 10.6, 7.9, 6.5, 6.2 ppm.

Example FLC-00548-1

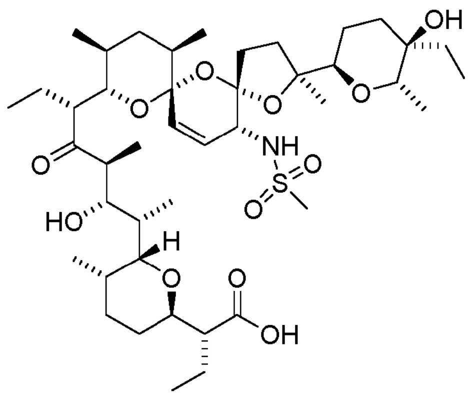

The compound was prepared according to the procedure described in Example FLC-00468-1, except that methanesulfonyl chloride (3 eq) was added to the reaction mixture instead of 4-trifluoromethylbenzenesulfonyl chloride. The yield of the obtained product was 10%.

ESI-MS for $C_{43}H_{73}NO_{12}S$ (*m/z*): $[M+Na]^+$ 850.9, $[M-H]^-$ 826.9.

$^1$H NMR (500 MHz, $CD_2Cl_2$) δ 6.47 (d, J = 9.8 Hz, 1H), 6.14 (dd, J = 10.8, 3.0 Hz, 1H), 5.80 (dd, J = 10.8, 1.4 Hz, 1H), 4.19 (q, J = 6.7 Hz, 1H), 4.14 - 4.06 (m, 2H), 3.83 (dd, J = 11.1, 4.6 Hz, 1H), 3.60 (dd, J = 10.2, 6.2 Hz, 2H), 3.43 (dd, J = 12.1, 1.9 Hz, 1H), 2.94 (s, 3H), 2.81 (td, J= 10.9, 3.3 Hz, 1H), 2.76 - 2.64 (m, 2H), 2.27 (dt, J = 12.5, 8.5 Hz, 2H), 1.98 - 0.68 (m, 54H) ppm.

$^{13}$C NMR (126 MHz, $CD_2Cl_2$) δ 219.2, 185.1, 127.9, 124.5, 106.2, 99.4, 89.2, 77.2, 76.5, 76.3, 75.0, 71.6, 70.4, 68.8, 56.4, 51.5, 51.3, 49.4, 42.1, 40.9, 39.0, 37.0, 36.2, 32.9, 32.6, 32.5, 30.2, 29.7, 28.5, 27.5, 27.1, 24.1, 20.3, 17.7, 16.1,

15.7, 14.9, 13.2, 13.1, 12.5, 11.1, 6.9, 6.7 ppm.

Example FLC-00474-1

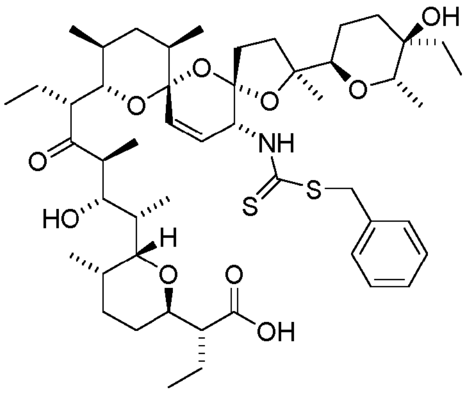

100 mg C20-amino SAL (1 eq) was mixed with TEA (6 eq), $CS_2$ (6 eq) and benzyl bromide (5 eq) in MeCN. The reaction was carried out first at 0 °C, and then at RT (TLC and LC-MS control). The post-reaction mixture was suspended on silica gel and purified using a chromatograph with an ELS detector using a column packed with silica gel. The respective fractions were combined and concentrated, and the residue was dissolved in DCM and washed twice with 0.25 M $Na_2CO_3$. The yield of the obtained product was 72%.

ESI-MS for $C_{50}H_{77}NO_{10}S_2$ (*m*/*z*): $[M+Na]^+$ 938.9.

$^1H$ NMR (700 MHz, $CD_2Cl_2$) δ 8.95 (d, *J* = 8.7 Hz, 1H), 7.41 - 7.20 (m, 5H), 6.21 (dd, *J* = 10.9, 3.1 Hz, 1H), 5.85 - 5.82 (m, 1H), 5.77 (dd, *J* = 10.8, 1.7 Hz, 1H), 4.57 (q, *J* = 13.8 Hz, 2H), 4.22 - 4.18 (m, 1H), 4.14 (dd, *J* = 10.5, 1.7 Hz, 1H), 3.91 (dd, *J* = 10.9, 4.9 Hz, 1H), 3.68 - 3.66 (m, 1H), 3.46 (dd, *J* = 12.2, 2.3 Hz, 1H), 2.83 - 2.75 (m, 3H), 2.71 (d, *J* = 9.6 Hz, 1H), 2.51 (dt, *J*= 13.4, 9.6 Hz, 1H), 2.08 (ddd, *J*= 13.4, 8.5, 5.0 Hz, 1H), 2.01 - 0.66 (m, 55H) ppm. $^{13}C$ NMR (176 MHz, $CD_2Cl_2$) δ 218.9, 199.8, 184.4, 137.3, 129.1, 128.3, 127.0, 126.6, 123.5, 106.4, 99.3, 88.7, 77.0, 76.3, 76.2, 73.7, 71.1, 70.1, 68.6, 67.1, 56.0, 54.4, 50.3, 48.8, 40.6, 39.8, 38.6, 37.3, 35.6, 32.4, 32.1, 31.9, 29.7, 28.7, 28.1, 26.7, 26.5, 23.4, 20.0, 17.4, 15.7, 15.3, 14.2, 12.8, 12.2, 10.8, 6.6, 6.3 ppm.

Example FLC-00475-1

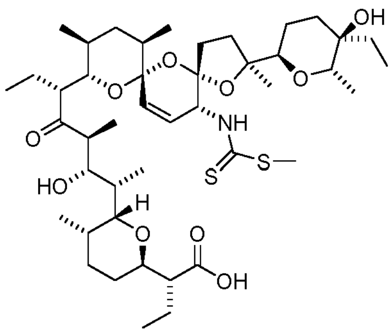

The compound was prepared according to the procedure described in example FLC-00474-1, except that methyl iodide (5 eq) was added to the reaction mixture instead of benzyl bromide. The yield of the obtained product was 93%.

ESI-MS for $C_{44}H_{73}NO_{10}S_2$ (*m*/*z*): $[M+H]^+$ 840.9, $[M-H]^-$ 839.0.

$^1H$ NMR (700 MHz, $CDCl_3$) δ 8.83 (d, *J*= 8.6 Hz, 1H), 6.17 (dd, *J* = 10.8, 3.0 Hz, 1H), 5.86 (d, *J*= 9.0 Hz, 1H), 5.81 (d, *J*= 10.8 Hz, 1H), 4.29 (q, *J*= 6.5 Hz, 1H), 4.23 (d, *J*= 9.9 Hz, 1H), 4.01 (dd, *J* = 10.5, 4.3 Hz, 1H), 3.70 (d, *J* = 14.1 Hz, 4H), 3.62 (d, *J*= 10.0 Hz, 1H), 3.41 (d, *J* = 10.5 Hz, 1H), 2.90 - 2.84 (m, 1H), 2.72 (dq, *J* = 14.5, 7.1 Hz, 1H), 2.67 (d, *J* = 9.9 Hz, 1H), 2.62 (s, 3H), 2.34 (dd, *J*= 23.1, 9.9 Hz, 1H), 2.07 - 0.68 (m, 53H) ppm.

$^{13}C$ NMR (176 MHz, $CDCl_3$) δ 218.1, 202.2, 184.8, 127.0, 123.2, 106.3, 99.3, 88.6, 76.6, 76.0, 75.8, 74.2, 71.3, 70.1, 68.4, 67.1, 55.9, 54.0, 50.5, 49.2, 40.7, 38.7, 37.0, 35.7, 32.4, 32.3, 32.0, 29.7, 29.6, 28.0, 27.3, 26.7, 23.6, 22.7, 20.0,

18.6, 17.6, 15.9, 15.4, 14.6, 13.1, 12.7, 12.2, 6.8, 6.6 ppm.

Example FLC-00476-1

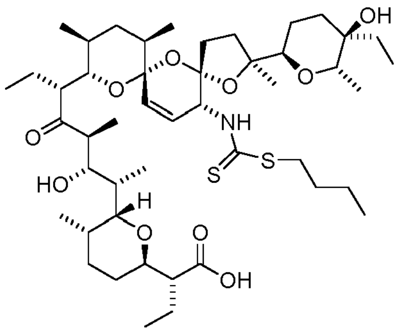

The compound was prepared according to the procedure described in example FLC-00474-1, except that butyl bromide (5 eq) was added to the reaction mixture instead of benzyl bromide. The yield of the obtained product was 38%.

ESI-MS for $C_{47}H_{79}NO_{10}S_2$ (*m/z*): $[M+Na]^+$ 905.0.

$^1H$ NMR (700 MHz, $CDCl_3$) δ 8.69 (d, *J* = 9.3 Hz, 1H), 6.17 (dd, *J* = 10.9, 3.1 Hz, 1H), 5.87 (ddd, *J* = 9.4, 2.9, 1.9 Hz, 1H), 5.81 (dd, *J* = 10.8, 1.8 Hz, 1H), 4.26 (q, *J* = 6.8 Hz, 1H), 4.23 (dd, *J* = 10.4, 1.6 Hz, 1H), 4.01 (dd, *J* = 10.9, 5.0 Hz, 1H), 3.70 (dd, *J* = 10.2, 2.2 Hz, 1H), 3.63 (d, *J* = 10.1 Hz, 1H), 3.41 (dd, *J* = 12.2, 2.3 Hz, 1H), 3.31 - 3.20 (m, 2H), 2.88 - 2.84 (m, 1H), 2.72 (dq, *J* = 10.4, 7.2 Hz, 1H), 2.67 - 2.64 (m, 1H), 2.42 - 2.35 (m, 1H), 2.18 (s, 1H), 2.07 - 0.68 (m, 62H) ppm.

$^{13}C$ NMR (176 MHz, $CDCl_3$) δ 218.1, 201.6, 184.8, 127.0, 123.2, 106.4, 99.2, 88.6, 76.8, 76.0, 74.1, 71.2, 69.9, 68.4, 55.9, 53.7, 50.6, 49.2, 40.6, 38.7, 37.1, 35.8, 35.3, 32.4, 32.3, 32.1, 31.2, 29.7, 29.3, 28.0, 27.2, 26.8, 23.7, 22.0, 20.0, 17.6, 15.9, 15.4, 14.6, 14.1, 13.7, 13.1, 12.9, 12.1, 10.9, 6.7, 6.6 ppm.

Example FLC-00553-1

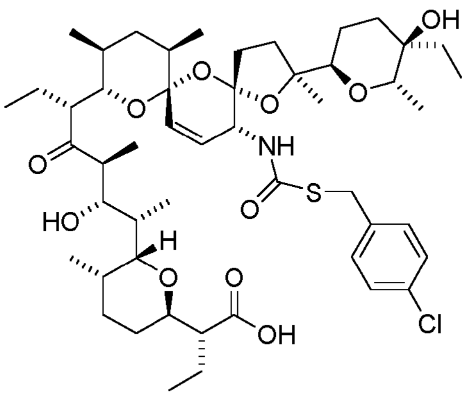

The compound was obtained according to the procedure described in example FLC-00539-1, except that 4-chlorobenzyl mercaptan (3 eq) was added to the reaction mixture instead of 3-methoxypropylamine. The yield of the obtained product was 10% .

ESI-MS for $C_{50}H_{76}ClNO_{11}S$ (*m/z*): $[M+Na]^+$ 957.1, $[M-H]^-$ 933.1.

$^1H$ NMR (500 MHz, $CD_2Cl_2$) δ 7.29 - 7.21 (m, *J*= 6.5, 5.3 Hz, 5H), 6.12 (dd, *J*= 10.9, 3.0 Hz, 1H), 5.65 (dd, *J*= 10.8, 1.4 Hz, 1H), 4.77 (d, *J*= 9.1 Hz, 1H), 4.14 (q, *J*= 6.7 Hz, 1H), 4.09 - 3.99 (m, 3H), 3.86 (dd, *J* = 10.8, 4.4 Hz, 2H), 3.41 (dd, *J* = 12.2, 2.2 Hz, 1H), 2.79 - 2.70 (m, 2H), 2.65 (d, *J* = 9.8 Hz, 1H), 2.17 (dt, *J* = 13.2, 9.5 Hz, 1H), 2.08 - 0.62 (m, 57H) ppm.

$^{13}C$ NMR (126 MHz, $CD_2Cl_2$) δ 219.3, 184.6, 167.4, 138.7, 132.7, 130.6, 128.7, 127.8, 123.8, 106.5, 99.5, 89.1, 77.1, 76.5, 74.1, 71.4, 70.4, 68.8, 56.4, 50.8, 49,7, 49.2, 40.9, 38.9, 37.5, 36.0, 33.6, 32.8, 32.5, 32.3, 30.1, 29.0, 28.5, 27.0, 26.8, 23.8, 20.4, 17.7, 16.0, 15.6, 14.6, 13.4, 13.2, 12.5, 7.0, 6.7 ppm.

Example FLC-00554-1

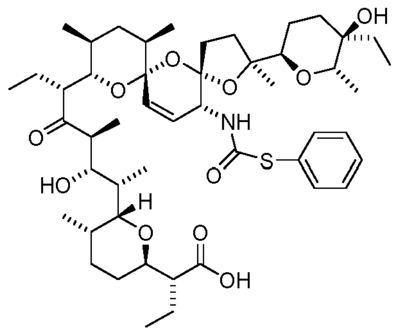

The compound was prepared according to the procedure described in example FLC-00539-1, except that thiophenol (1.5 eq) was added to the reaction mixture instead of 3-methoxypropylamine. The yield of the obtained product was 15%.

ESI-MS for $C_{49}H_{75}NO_{11}S$ (*m/z*): $[M+Na]^+$ 908.9, $[M-H]^-$ 886.1.

$^1H$ NMR (500 MHz, $CD_2Cl_2$) $\delta$ 7.51 - 7.41 (m, 3H), 7.40 - 7.29 (m, 3H), 6.16 (dd, *J* = 10.8, 3.0 Hz, 1H), 5.72 (dd, *J* = 10.8, 1.3 Hz, 1H), 4.74 (d, *J* = 9.1 Hz, 1H), 4.20 (q, *J* = 6.7 Hz, 1H), 4.13 (d, *J* = 10.5 Hz, 1H), 3.90 (dd, *J* = 10.8, 3.9 Hz, 1H), 3.70 - 3.62 (m, 2H), 3.48 (dd, *J* = 12.1, 2.1 Hz, 1H), 2.84 (td, *J* = 10.6, 3.5 Hz, 1H), 2.79 - 2.73 (m, 2H), 2.68 (d, *J* = 9.9 Hz, 1H), 2.27 - 0.65 (m, 56H) ppm.

$^{13}C$ NMR (126 MHz, $CD_2Cl_2$) $\delta$ 219.3, 184.7, 166.6, 135.7, 129.7, 129.1, 129.0, 127.8, 123.9, 106.6, 99.5, 89.1, 77.3, 76.5, 74.3, 71.5, 70.5, 68.9, 56.4, 51.0, 49,8, 49.2, 40.9, 38.9, 37.6, 36.0, 32.8, 32.6, 32.4, 30.1, 29.3, 28.5, 27.1, 26.9, 23.9, 23.1, 20.5, 17.7, 16.0, 15.6, 14.7, 13.4, 13.2, 12.6, 11.2, 7.0, 6.7 ppm.

## Claims

**1.** A compound constituting a C20-aminosalinomycin with the formula FLC-00105 or a salt thereof:

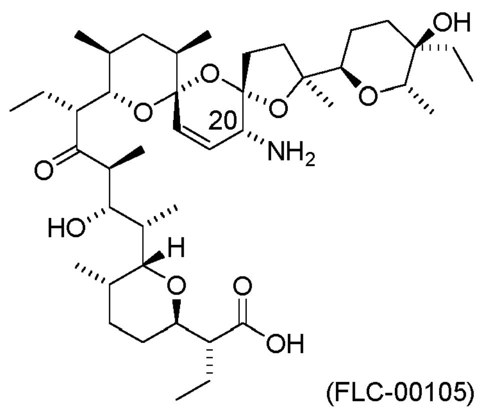

(FLC-00105)

**2.** A method for preparing C20-aminosalinomycin with the formula FLC-00105 or a salt thereof:

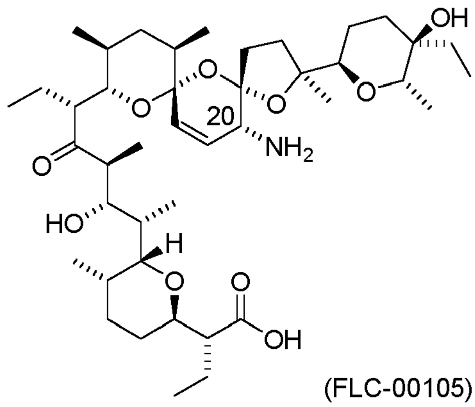

(FLC-00105)

comprising stereoselective reductive amination of C20-ketosalinomycin with the formula FLC-00099 or a salt thereof:

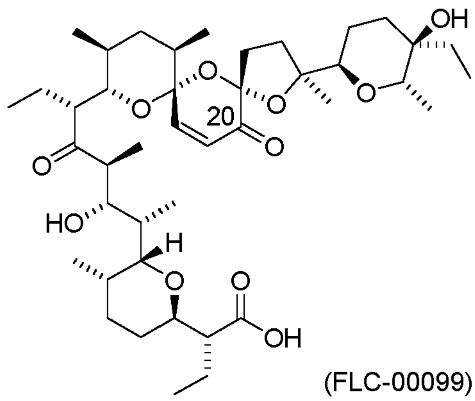

(FLC-00099)

wherein the amine agent used is an alcoholic solution of ammonia and ammonium acetate to obtain *in situ* an imine derivative which is reduced with alkali metal borohydride of sodium, potassium, lithium, or a derivative thereof, such as cyanoborohydride, triacetoxyborohydride, preferably sodium cyanoborohydride.

**3.** A method for preparing C20-aminosalinomycin with the formula FLC-00105 or a salt thereof:

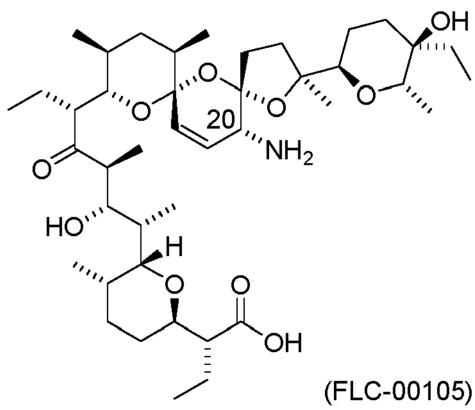

(FLC-00105)

the method comprising the following steps:

a. selective oxidation of the C20-hydroxy group of salinomycin with the formula FLC-00001 or a salt thereof:

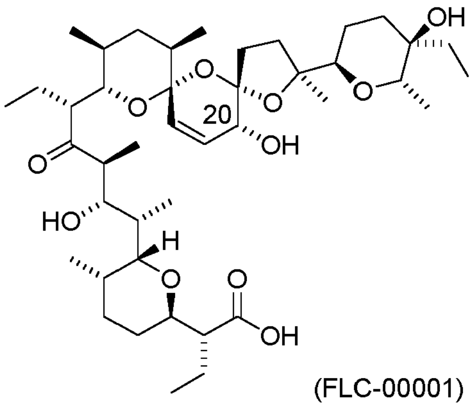

(FLC-00001)

to

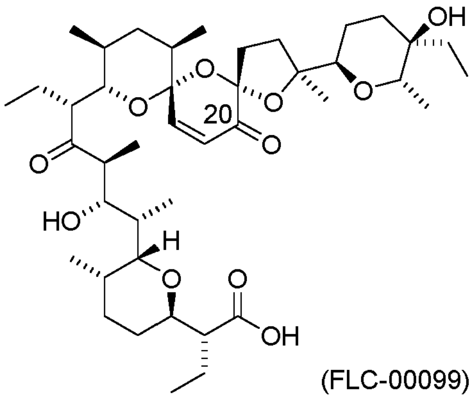

(FLC-00099)

wherein the oxidising agent is selected from the group consisting of: pyridinium or pyrimidinium salt or a derivative thereof, chlorochromic (VI) or dichromic (VI) acid, pyridinium chlorochromate, pyridinium dichromate, chromium (VI) trioxide $CrO_3$, preferably pyridinium dichromate.

b. stereoselective reductive amination of the resulting C20-ketosalinomycin with the formula FLC-00099:

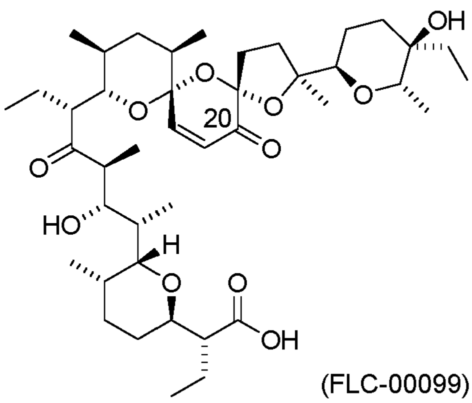

(FLC-00099)

wherein the amine agent used is an alcoholic solution of ammonia and ammonium acetate to obtain *in situ* an imine derivative which is reduced with alkali metal borohydride of sodium, potassium, lithium, or a derivative thereof, such as cyanoborohydride, triacetoxyborohydride, preferably sodium cyanoborohydride.

Europäisches Patentamt
European Patent Office
Office européen des brevets

# EUROPEAN SEARCH REPORT

Application Number
EP 24 18 9896

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2016/038223 A1 (CENTRE NAT RECH SCIENT [FR]; INST NAT SANTE RECH MED [FR] ET AL.) 17 March 2016 (2016-03-17) * compounds oxo-Sal-H and AM8; claims 1, 11; example 1 * ----- | 1-3 | INV. C07D493/20 A61P35/00 A61K31/35 A61K31/422 A61K31/427 A61K31/4433 |
| A,D | LI YU ET AL: "Synthesis and biological evaluation of 20-epi-amino-20-deoxysalinomycin derivatives", EUROPEAN JOURNAL OF MEDICINAL CHEMISTRY, vol. 148, 1 March 2018 (2018-03-01), pages 279-290, XP055788796, AMSTERDAM, NL ISSN: 0223-5234, DOI: 10.1016/j.ejmech.2018.02.004 * scheme 1; page 281; compound 4 * ----- | 1-3 | |
| X,D,P | WO 2021/156461 A1 (FILECLO SP Z O O [PL]) 12 August 2021 (2021-08-12) * example 18; compound 3 * ----- | 1 | TECHNICAL FIELDS SEARCHED (IPC) C07D A61P A61K |

1 The present search report has been drawn up for all claims

| Place of search | Date of completion of the search | Examiner |
|---|---|---|
| Munich | 8 April 2025 | Moriggi, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 18 9896

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2025

| Patent document cited in search report | Publication date | Patent family member(s) | Publication date |
|---|---|---|---|
| WO 2016038223 A1 | 17-03-2016 | CA 2960910 A1 | 17-03-2016 |
| | | CN 107428772 A | 01-12-2017 |
| | | DK 3191493 T3 | 18-11-2019 |
| | | EP 3191493 A1 | 19-07-2017 |
| | | ES 2755424 T3 | 22-04-2020 |
| | | JP 6599444 B2 | 30-10-2019 |
| | | JP 2017526722 A | 14-09-2017 |
| | | PL 3191493 T3 | 28-02-2020 |
| | | US 2017253610 A1 | 07-09-2017 |
| | | WO 2016038223 A1 | 17-03-2016 |
| WO 2021156461 A1 | 12-08-2021 | AU 2021217480 A1 | 11-08-2022 |
| | | CA 3167852 A1 | 12-08-2021 |
| | | CN 115052879 A | 13-09-2022 |
| | | EP 4100411 A1 | 14-12-2022 |
| | | ES 2957260 T3 | 16-01-2024 |
| | | JP 2023514054 A | 05-04-2023 |
| | | KR 20220139946 A | 17-10-2022 |
| | | PL 242212 B1 | 30-01-2023 |
| | | US 2022402932 A1 | 22-12-2022 |
| | | WO 2021156461 A1 | 12-08-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3191493 A
- WO 2021156461 A1
- PL 43285820 A


### Non-patent literature cited in the description


- **MAI et al.** *Nature Chemistry*, 2017, vol. 9, 1025-1033
- **LI et al.** *European Journal of Medicinal Chemistry*, 2018, vol. 148, 279-290
- **VERSINI et al.** *Chemistry A European Journal*, 2020, vol. 26 (33), 7416-7424
- Remington: The Science and Practice of Pharmacy. Mack Publishing Company, 1995
- **WIETRZYK et al.** *Anti-cancer drugs*, 2007, vol. 18, 447-457
- **SKEHAN et al.** *Journal of the National Cancer Institute*, 1990, vol. 82, 1107-1112
- **NEVOZHAY**. *PLoS One*, 2014, vol. 9, e106186